# EUROPEAN PATENT SPECIFICATION

(11) **EP 4 298 092 B1**
(45) Date of publication and mention of the grant of the patent: **26.11.2025**
(21) Application number: 22707455.6
(22) Date of filing: 24.02.2022
(51) Int. Cl.: C07D 403/12, C07D 409/12, C07D 471/04, C07D 495/04, A61P 25/00, A61K 31/437

(54) **NOVEL PYRIMIDIN-2-YL SULFONAMIDE DERIVATIVES**
NEUE PYRIMIDIN-2-YL-SULFONAMIDDERIVATE
NOUVEAUX DÉRIVÉS DE SULFONAMIDE PYRIMIDIN-2-YL

(30) Priority: 26.02.2021 EP 21159452
(43) Date of publication of application: 03.01.2024
(73) Proprietor: F. Hoffmann-La Roche AG, 4070 Basel (CH)
(72) Inventor: GALLEY, Guido, 4070 Basel (CH); GOBBI, Luca, 4070 Basel (CH); GUBA, Wolfgang, 4070 Basel (CH); MAZUNIN, Dmitry, 4070 Basel (CH); PINARD, Emmanuel, 4070 Basel (CH); RICCI, Antonio, 4070 Basel (CH)
(74) Representative: Jochnowitz, Evan
(86) International application number: PCT/EP2022/054586
(87) International publication number: WO 2022/180136

(56) References cited:
- WO-A1-2019/243398

## Description

The present invention relates to organic compounds useful for therapy and/or prophylaxis in a mammal, and in particular to compounds that modulate GPR17 activity.

The present invention provides novel compounds of formula I 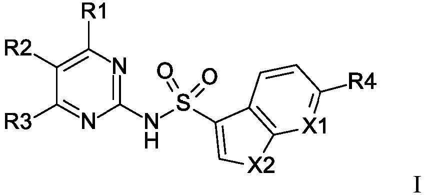 wherein
R¹ is H, halo, alkyl, alkoxy, alkoxyalkyl, alkoxyalkoxy, cyanoalkyl, haloalkyl, haloalkoxy, thiolalkyl, cycloalkyl, cycloalkylalkyl, cycloalkoxy, or heterocycloalkyl;
R² is cyano, halo, alkyl, alkenyl, alkynyl, alkoxy, alkenylalkyl, alkynylalkoxy, alkoxyalkyl, alkoxyalkoxy, haloalkyl, haloalkoxy, haloalkoxyalkyl, (haloalkoxy)alkoxy, cyanoalkyl, cyanoalkoxy, thiolalkyl, halothiolalkyl, halohydroxyalkoxy, alkylsulfonylalkyl, cycloalkyl, cycloalkylalkyl, cycloalkylalkoxy, cycloalkoxy, heterocycloalkyl, heterocycloalkylalkyl, heterocycloalkylalkynyl or heterocycloalkylalkoxy;
R³ is H, halo, alkyl, alkoxy, haloalkoxy, or thiolalkyl;
X₁ is CR⁵ or N;
X₂ is NH, -N-R⁶, S, -S(O)-, or -S(O₂)-;
R⁴ is H, halo, alkyl, hydroxyalkyl, thiolalkyl, cyanoalkyl, alkoxy, haloalkyl, haloalkoxy, cycloalkyl or heterocycloalkyl;
R⁵ is H, halo, cyano, alkyl, alkoxy, cyanoalkyl, cyanyalkoxy, N-alkyl, dialkylamino, haloalkyl, haloalkoxy, halothiolalkyl, alkylsulfinyl, alkylsulfonyl, alkylsulfone, thiolalkyl, hydroxyalkyl, alkoxycarbonyl, heterocycloalkyl, cycloalkyl or cycloalkoxy;
or R⁴ and R⁵ are connected to form a 5 or 6 member cycloalkyl, heterocycloalkyl, substituted heterocycloalkyl comprising one or two substituents independently selected from alkyl and oxo, or a heteroaromatic ring;
R⁶ is H, alkyl, alkylhydroxy, or haloalkyl;
and pharmaceutically acceptable salts.

Furthermore, the invention includes all racemic mixtures, all their corresponding enantiomers and/or optical isomers.

### Background of the Invention

Myelination is a process that occurs robustly during development and despite the abundant presence of oligodendrocyte precursor cells (OPCs) throughout the adult CNS, the transition to myelinating oligodendrocytes and the production of restorative myelin sheaths around denuded axons is impaired in chronic demyelinating diseases. During development, myelination proceeds in a very orderly manner, with OPCs, characterized by expression of markers such as neural/glial antigen 2 (NG2) and platelet-derived growth factor alpha (PDGFRα), differentiating into oligodendrocytes which lose NG2 and PDGFRα expression and gain the expression of markers such as myelin basic protein (MBP) and myelin oligodendrocyte glycoprotein (MOG). The production of myelin by oligodendrocytes is a very tightly regulated process and in the CNS, this can be controlled by interactions with axons, well-understood in the peripheral but not in the central nervous system (Macklin, W.B. (2010). Sci. Signal. 3, pe32-pe32, "The myelin brake: When Enough Is Enough"). Myelination can also be controlled by internal brakes within oligodendrocytes themselves, through the transcription factor EB (TFEB)-PUMA axis or through GPR17 antagonism (Chen, Y., et al. (2009). Nat Neurosci 12, 1398-1406, "The oligodendrocyte-specific G protein-coupled receptor GPR17 is a cell-intrinsic timer of myelination") (Sun, L.O., et al. (2018). Cell 175, 1811-1826.e21, "Spatiotemporal Control of CNS Myelination by Oligodendrocyte Programmed Cell Death through the TFEB-PUMA Axis"). Myelin serves not only to protect axons and facilitate neuronal transmission, but oligodendrocytes have also been shown to play an important role in metabolism of axons as well as in maintaining the electrolyte balance around axons (Schirmer, L., et al. (2014). Ann Neurol 75, 810-828, "Differential loss of KIR4.1 immunoreactivity in multiple sclerosis lesions") (Simons, M., and Nave, K.-A. (2015). Cold Spring Harb Perspect Biol. 22, "Oligodendrocytes: Myelination and Axonal Support").

GPR17 is a Class A orphan G protein-coupled receptor (GPCR). GPCRs are 7 domain transmembrane proteins that couple extracellular ligands with intracellular signaling via their intracellular association with small, heterotrimeric G-protein complexes consisting of G_{α}, G_{β}, G_{γ} subunits. It is the coupling of the GPCR to the G_{α} subunit that confers results in downstream intracellular signaling pathways. GPR17 is known to be coupled directly to G_{α i/o}, which leads to inhibition of adenylate cyclase activity, resulting in a reduction in cyclic AMP production (cAMP). GPR17 has also been shown to couple to G_{q/11}, that targets phospholipase C. Activation of phospholipase C leads to the cleavage of phosphatidylinositol 4,5-bisphosphate which produces inositol triphosphate (IP₃) and diacylglycerol (DAG). IP₃ consequently binds to the IP₃ receptor on the endoplasmic reticulum and causes an increase in intracellular calcium levels (Hanlon, C.D., and Andrew, D.J. (2015). J Cell Sci. 128, 3533-3542, "Outside-in signaling-a brief review of GPCR signaling with a focus on the Drosophila GPCR family") (Inoue, A., et al. (2019), Cell 177, 1933-1947.e25, "Illuminating G-Protein-Coupling Selectivity of GPCRs").

The role of GPR17 in myelination was first identified in a screen of the optic nerves of *Olig1* knockout mice to identify genes regulating myelination. GPR17 expression was found to be expressed only in the myelinating cells of the CNS and absent from the Schwann cells, the peripheral nervous system's myelinating cells. The expression of GPR17 was found to be exclusively expressed in the oligodendrocyte lineage cells and was downregulated in myelinating oligodendrocyte (Chen, Y., et al. (2009)). Specifically, GPR17 expression is found to be present at low levels early on in the OPC and increases in the pre-myelinating oligodendrocyte before the expression is downregulated in the mature, myelinating oligodendrocyte (Boda, E., et al. (2011), Glia 59, 1958-1973, "The GPR17 receptor in NG2 expressing cells: Focus on in vivocell maturation and participation in acute trauma and chronic damage") (Dziedzic, A., et al. (2020). Int. J. Mol. Sci. 21, 1852, "The gpr17 receptor-a promising goal for therapy and a potential marker of the neurodegenerative process in multiple sclerosis") (Fumagalli, M. et al. (2011), J Biol Chem 286, 10593-10604, "Phenotypic changes, signaling pathway, and functional correlates of GPR17-expressing neural precursor cells during oligodendrocyte differentiation"). GPR17 knockout animals were shown to exhibit precocious myelination throughout the CNS and conversely, transgenic mice overexpressing *GPR17* in oligodendrocytes with the CNP-Cre (2', 3' - cyclic-nucleotide 3'-phosphodiesterase) promoter exhibited myelinogenesis defects, in line with what is to be expected of a cell-intrinsic brake on the myelination process (Chen, Y., et al. (2009)). Furthermore, loss of GPR17 enhances remyelination following demyelination with lysophosphatidylcholine-induced demyelination (Lu, C., Dong, et al. (2018), Sci. Rep. 8, 4502, "G-Protein-Coupled Receptor Gpr17 Regulates Oligodendrocyte Differentiation in Response to Lysolecithin-Induced Demyelination"). As such, antagonism of GPR17 that promotes the differentiation of oligodendrocyte lineage cells into mature, myelinating oligodendrocytes would lead to increase in myelination following demyelination.

Multiple sclerosis (MS) is a chronic neurodegenerative disease that is characterized by the loss of myelin, the protective fatty lipid layer surrounding axons, in the central nervous system (CNS). Prevention of myelin loss or remyelination of denuded axons is thought to prevent axonal degeneration and thus prevent progression of the disease (Franklin, R.J. (2002), Nat Rev Neurosci 3, 705-714, "Why does remyelination fail in multiple sclerosis?"). Due to the restorative impact that myelin repair has on the central nervous system, such a treatment will benefit all types of MS namely relapse-remitting, secondary progressive, primary progressive and progressive relapsing MS. Reparation of lost myelin will alleviate neurological symptoms associated with MS due to the neuroprotective effect of preserving axons.

Due to the essential role that myelination plays in functioning of the nervous system, facilitating OPC to oligodendrocyte differentiation has the potential to impact multiple diseases where white matter defects/irregularities due to either loss of myelinating oligodendrocytes or hampered differentiation of OPCs to oligodendrocytes have been observed, due to the disease itself or inflammation. This is in addition to the diseases where GPR17 expression itself is altered.

The diseases that GPR17 antagonism can be thus used to yield a positive disease outcome include, but are not limited to:
Direct damage to myelin sheaths:
   - Metabolic conditions that lead to destruction of central myelin such as central pontine myelinolysis, extra-pontine myelinolysis due to overly-rapid correction of hyponatremia in conditions for instance, but not limited to, alcoholism, liver disease, immunosuppression after transplantation
   - Carbon monoxide poisoning where oligodendrocyte dysfunction and failure to regenerate has been reported in the deep white matter layers of the brain
   - Nutritional deficiency that results in myelin loss or failure to properly generate myelin during development
   - Virus-induced demyelination
Primary demyelinating disorders
   - Multiple Sclerosis (relapse-remitting, secondary progressive, primary progressive and progressive relapsing MS)
   - Acute and multiphasic disseminated encephalomyelitis
   - Neuromyelitis optica spectrum disorders including optic neuritis
   - Transverse myelitis
   - Leukodystrophies such as adrenoleukodystrophy, adrenomyeloneuropathy and other inherited leukodystrophies that result in myelin loss
CNS disorders with associated myelin loss:
   - Alzheimer's Disease
   - Schizophrenia
   - Parkinson's Disease
   - Huntington's disease
   - Amyotrophic lateral
   - Ischemia due to stroke
Other diseases:
   - Inflammation in the CNS for instance following encephalitis, primary angiitis, meningitis

The compounds of formula I bind to and modulates GPR17 activity.

The compounds of formula I are therefore particularly useful in the treatment of diseases related to GPR17 antagonism.

The compounds of formula I are particularly useful in the treatment or prophylaxis of multiple sclerosis (MS), conditions related to direct damage to myelin sheaths such as carbon monoxide poisoning or virus induced demyelination, primary demyelinating disorders such as acute and multiphasic disseminated encephalomyelitis, and other CNS disorders associated with myelin loss such as Alzheimer's disease, schizophrenia, Parkinson's disease and Huntington's disease.

### Summary of the Invention

The present invention provides novel compounds of formula I 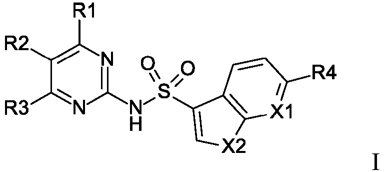 wherein
R¹ is H, halo, alkyl, alkoxy, alkoxyalkyl, alkoxyalkoxy, cyanoalkyl, haloalkyl, haloalkoxy, thiolalkyl, cycloalkyl, cycloalkylalkyl, cycloalkoxy, or heterocycloalkyl;
R² is cyano, halo, alkyl, alkenyl, alkynyl, alkoxy, alkenylalkyl, alkynylalkoxy, alkoxyalkyl, alkoxyalkoxy, haloalkyl, haloalkoxy, haloalkoxyalkyl, (haloalkoxy)alkoxy, cyanoalkyl, cyanoalkoxy, thiolalkyl, halothiolalkyl, halohydroxyalkoxy, alkylsulfonylalkyl, cycloalkyl, cycloalkylalkyl, cycloalkylalkoxy, cycloalkoxy, heterocycloalkyl, heterocycloalkylalkyl, heterocycloalkylalkynyl or heterocycloalkylalkoxy;
R³ is H, halo, alkyl, alkoxy, haloalkoxy, or thiolalkyl;
X₁ is CR⁵ or N;
X₂ is NH, -N-R⁶, S, -S(O)-, or -S(O₂)-;
R⁴ is H, halo, alkyl, hydroxyalkyl, thiolalkyl, cyanoalkyl, alkoxy, haloalkyl, haloalkoxy, cycloalkyl or heterocycloalkyl;
R⁵ is H, halo, cyano, alkyl, alkoxy, cyanoalkyl, cyanyalkoxy, N-alkyl, dialkylamino, haloalkyl, haloalkoxy, halothiolalkyl, alkylsulfinyl, alkylsulfonyl, alkylsulfone, thiolalkyl, hydroxyalkyl, alkoxycarbonyl, heterocycloalkyl, cycloalkyl or cycloalkoxy;
or R⁴ and R⁵ are connected to form a 5 or 6 member cycloalkyl, heterocycloalkyl, substituted heterocycloalkyl comprising one or two substituents independently selected from alkyl and oxo, or a heteroaromatic ring;
R⁶ is H, alkyl, alkylhydroxy, or haloalkyl;
and pharmaceutically acceptable salts.

The term "alkyl" denotes a monovalent linear or branched saturated hydrocarbon group of 1 to 6 carbon atoms. In some embodiments, if not otherwise described, alkyl comprises 1 to 6 carbon atoms (C₁₋₆-alkyl), or 1 to 4 carbon atoms (C₁₋₄-alkyl). Examples of C₁₋₆-alkyl include methyl, ethyl, propyl, isopropyl, n-butyl, iso-butyl, sec-butyl, tert-butyl and pentyl. Particular alkyl groups include methyl, ethyl and propyl. When an alkyl residue having a specific number of carbons is named, all geometric isomers having that number of carbons may be encompassed. Thus, for example, "butyl" can include n-butyl, sec-butyl, isobutyl and t-butyl, and "propyl" can include n-propyl and isopropyl.

The term "alkoxy" denotes a group of the formula -O-R', wherein R' is a C₁₋₆-alkyl group. Examples of C₁₋₆-alkoxy groups include methoxy, ethoxy, n-propoxy, isopropoxy, n-butoxy, isobutoxy and tert-butoxy. Particular examples are methoxy and ethoxy.

The term "alkoxyalkyl" denotes an alkyl group wherein at least one of the hydrogen atoms of the alkyl group has been replaced by an alkoxy group. Exemplary alkoxyalkyl groups include methoxymethyl, ethoxymethyl, methoxyethyl, ethoxyethyl, methoxypropyl and ethoxypropyl. Particular alkoxyalkyl groups include methoxymethyl and methoxyethyl.

The term "alkoxyalkoxy" denotes an alkoxy group wherein at least one of the hydrogen atoms of the alkoxy group has been replaced by another alkoxy group. Examples of alkoxyalkoxy group include methoxymethoxy, ethoxymethoxy, methoxyethoxy, ethoxyethoxy, methoxypropoxy and ethoxypropoxy. A particular alkoxyalkoxy group is methoxyethoxy.

The term "alkoxycarbonyl" means a moiety of the formula -C(O)-R', where R' is alkyl as defined herein. Particular example is methoxycarbonyl.

The term "alkenyl" refers to an unsaturated unbranched or branched univalent hydrocarbon chain having at least one site of a carbon-carbon double bond unsaturation (that is, having at least one moiety of the formula C=C). In some embodiments, unless otherwise specified, alkenyl comprises 2 to 6 carbon atoms, or 2 to 4 carbon atoms. Examples of alkenyl groups include, but are not limited to, ethenyl, propenyl, butenyl. A particular alkenyl group is allyl.

The term "alkenylalkyl" denotes an alkyl group wherein at least one of the hydrogen atoms of the alkyl group is replaced by an alkenyl group.

The term "alkynyl" refers to an unsaturated unbranched or branched univalent hydrocarbon chain having at least one site of acetylenic unsaturation (that is, having at least one moiety of the formula C≡C). In some embodiments, unless otherwise specified, alkynyl comprises 2 to 6 carbon atoms, or 2 to 4 carbon atoms. Examples of alkynyl groups include, but are not limited to, ethynyl (or acetylenyl), prop-1-ynyl, prop-2-ynyl (or propargyl), but-1-ynyl, but-2-ynyl, and but-3-ynyl. Particular example is prop-1-ynyl.

The term "alkynylalkyl" denotes an alkyl group wherein at least one of the hydrogen atoms of the alkyl group is replaced by an alkynyl group.

The term "alkynylalkoxy" denotes an alkoxy group wherein at least one of the hydrogen atoms of the alkyl group is replaced by an alkynyl group. Particular example is but-2-ynoxy.

The term "alkylsulfone" or "alkylsulfonyl" refers to a straight or branched chain lower alkylsulfone of from 1 to 4 carbon atoms is taken to mean a group of the structure, S(O)₂ alkyl, wherein the alkyl moiety is a straight or branched chain alkyl of from 1 to 4 carbon atoms and may be, for example, methyl, ethyl, n-propyl, isopropyl, n-butyl or isobutyl. Particular example is methylsulfonyl.

The term "alkylsulfonylalkyl" denotes an alkyl group wherein at least one of the hydrogen atoms of the alkyl group is replaced by an alkylsulfone group. Particular example of alkylsulfonylalkyl is methylsulfonylethyl.

The term "alkylsulfinyl" denotes a group of the formula -S(O)-R', wherein R' is an alkyl group. Examples of alkylsulfinyl groups include groups of the formula -S(O)-R', wherein R' is methyl, ethyl, n-propyl, isopropyl, n-butyl, isobutyl or tert-butyl. Particular alkylsulfinyl groups include group of the formula -S(O)-R', wherein R' is methyl. Particular example of alkylsulfinyl is methylsulfinyl.

The term "amino" denotes an -NH₂ group.

The term "dialkylamino" an amino group wherein two of the hydrogen atoms of the amino group have been replaced by two alkyl groups. Particular example is dimethylamino.

The term "cycloalkyl" denotes monocyclic or polycyclic saturated or partially unsaturated, non-aromatic hydrocarbon. In some embodiments, unless otherwise described, cycloalkyl comprises 3 to 8 carbon atoms, 3 to 6 carbon atoms, or 3 to 5 carbon atoms. In some embodiments, cycloalkyl is a saturated monocyclic or polycyclic hydrocarbon. In other embodiments, cycloalkyl comprises one or more double bonds (e.g., cycloalkyl fused to an aryl or heteroaryl ring, or a non-aromatic monocyclic hydrocarbon comprising one or two double bonds). Polycyclic cycloalkyl groups may include spiro, fused, or bridged polycyclic moieties wherein each ring is a saturated or partially unsaturated, non-aromatic hydrocarbon. Examples of cycloalkyl groups include cyclopropyl, cyclobutyl, cyclopentyl, cyclohexyl, octahydropentalenyl, spiro[3.3]heptanyl, and the like. Bicyclic means a ring system consisting of two saturated carbocycles having two carbon atoms in common. Examples for monocyclic cycloalkyl are cyclopropyl, cyclobutanyl, cyclopentyl, cyclohexyl or cycloheptyl. Particular example is cyclopropyl.

The term "cycloalkylalkyl" denotes an alkyl group wherein at least one of the hydrogen atoms of the alkyl group is replaced by a cycloalkyl group. Examples of cycloalkylalkyl include cyclopropylmethyl.

The term "cycloalkoxy" denotes a group of the formula -O-R', wherein R' is a cycloalkyl group. Examples of cycloalkoxy group include cyclopropoxy, cyclobutoxy, cyclopentyloxy, cyclohexyloxy, cycloheptyloxy and cyclooctyloxy. Particular cycloalkoxy group is cyclopropoxy.

The term "cycloalkylalkoxy" denotes an alkoxy group wherein at least one of the hydrogen atoms of the alkoxy group is replaced by a cycloalkyl group. Examples of cycloalkylalkoxy include cyclopropylmethoxy, cyclobutylmethoxy, cyclopentylmethoxy, cyclohexylmethoxy, cycloheptylmethoxy and cyclooctylmethoxy.

The term "cyano" denotes a -C≡N group.

"Cyanoalkyl" means a moiety of the formula -R'-R", where R' is alkyl as defined herein and R" is cyano or nitrile. Particular examples are cyanomethyl and cyanoethyl.

"Cyanoalkoxy" denotes a C₁₋₆-alkoxy group wherein at least one of the hydrogen atoms of the C₁₋₆-alkoxy group has been replaced by a cyano group. Particular example is cyanomethoxy.

The term "halogen", "halide" and "halo" are used interchangeably herein and denote fluoro, chloro, bromo or iodo. Particular halogens are fluoro, chloro and bromo.

The term "haloalkyl" denotes a C₁₋₆-alkyl group wherein at least one of the hydrogen atoms of the C₁₋₆-alkyl group has been replaced by the same or different halogen atoms. Particular examples are fluoroethyl, fluoropropyl and difluoromethyl, difluoroethyl, difluoropropyl, trifluoromethyl, trifluoroethyl, trifluoropropyl, and tetrafluoropropyl,

The term "haloalkoxy" denotes a C₁₋₆-alkoxy group wherein at least one of the hydrogen atoms of the C₁₋₆-alkoxy group has been replaced by the same or different halogen atoms. Particular examples are fluoromethoxy, fluoroethoxy, fluoropropoxy, difluoromethoxy, difluoroethoxy, difluoropropoxy, trifluoromethoxy, trifluoroethoxy, trifluoropropoxy and tetrafluoroethoxy.

The term "haloalkoxyalkyl" denotes an alkyl group wherein at least one of the hydrogen atoms of the alkyl group has been replaced by a haloalkoxy group. Examples of haloalkoxyalkyl include fluoromethoxymethyl, difluoromethoxymethyl, trifluoromethoxymethyl, fluoroethoxymethyl, difluoroethoxymethyl, trifluoroethoxymethyl, fluoromethoxyethyl, difluoromethoxyethyl, trifluoromethoxyethyl, fluoroethoxyethyl, difluoroethoxyethyl, trifluoroethoxyethyl, fluoromethoxypropyl, difluoromethoxypropyl, trifluoromethoxypropyl, fluoroethoxypropyl, difluoroethoxypropyl and trifluoroethoxypropyl.

The term "haloalkoxyalkoxy" denotes an alkoxy group wherein at least one of the hydrogen atoms of the alkoxy group has been replaced by a haloalkoxy group. Examples of haloalkoxyalkyl include fluoromethoxymethoxy, difluoromethoxymethoxy, trifluoromethoxymethoxy, fluoroethoxymethoxy, difluoroethoxymethoxy, trifluoroethoxymethyoxy, fluoromethoxyethoxy, difluoromethoxyethoxy, trifluoromethoxyethoxy, fluoroethoxyethoxy, difluoroethoxyethoxy, trifluoroethoxyethoxy, fluoromethoxypropoxy, difluoromethoxypropoxy, trifluoromethoxypropoxy, fluoroethoxypropoxy, difluoroethoxypropoxy and trifluoroethoxypropoxy. Particular example is difluoromethoxyethoxy.

The term "halohydroxyalkoxy" denotes an alkoxy group wherein at least one of the hydrogen atoms of the alkoxy group has been replaced by same or different halogen atoms and at least one of the hydrogen atoms of the alkoxy group has been replaced by a hydroxyl. Particular example is 1,1-difluoro-2-hydroxy-ethoxy.

The term "heterocycloalkyl" denotes a monovalent saturated or partly unsaturated mono- or bicyclic ring system of 4 to 9 ring atoms, comprising 1, 2, or 3 ring heteroatoms selected from N, O and S, the remaining ring atoms being carbon. Bicyclic means consisting of two cycles having two ring atoms in common, i.e. the bridge separating the two rings is either a single bond or a chain of one or two ring atoms. Examples for monocyclic saturated heterocycloalkyl are oxetanyl, azetidinyl, pyrrolidinyl, tetrahydrofuranyl, pyrazolidinyl, imidazolidinyl, oxazolidinyl, isoxazolidinyl, thiazolidinyl, piperidinyl, tetrahydropyranyl, tetrahydrothiopyranyl, or piperazinyl. Examples for partly unsaturated heterocycloalkyl are dihydrofuryl, imidazolinyl, dihydro-oxazolyl, tetrahydro-pyridinyl, or dihydropyranyl. Particular examples of heterocycloalkyl groups are oxetane, morpholino, tetrahydrofuranyl, and tetrahydropyranyl.

The term "heterocycloalkoxy" denotes a group of the formula -O-R', wherein R' is a heterocycloalkyl group. Examples of heterocycloalkoxy groups include tetrahydropyranyloxy, tetrahydrofuranyloxy and oxetanyloxy.

The term "heterocycloalkylalkoxy" denotes an alkoxy group wherein at least one of the hydrogen atoms of the alkoxy group is replaced by a heterocycloalkyl group. Examples of heterocycloalkylalkoxy include tetrahydropyranylmethoxy, tetrahydrofuranylmethoxy, oxetanylmethoxy, tetrahydropyranylethoxy, tetrahydrofuranylethoxy and oxetanylethoxy. Particular example heterocycloalkylalkoxy is oxetanylmethoxy.

"Heterocycloalkylalkyl" means a heterocycloalkyl moiety as defined above linked via an alkyl moiety (defined above) to a parent core. Non-limiting examples of suitable heterocycloalkylalkyls include piperidinylmethyl, piperazinylmethyl, and oxetanylmethyl. Particular example heterocycloalkylalkyl is oxetanylethyl.

"Heterocycloalkylalkynyl" means a heterocycloalkyl moiety as defined above linked via an alkyn moiety (defined above) to a parent core. Particular example of heterocycloalkylalkynyl is oxetanylethynyl.

A "heteroaromatic ring" refers a monocyclic or polycyclic group comprising at least one aromatic ring, wherein the aromatic ring comprises at least one ring heteroatom. In some embodiments, the heteroatom is independently selected from the group consisting of N, O, and S. Unless otherwise specified, a heteroaromatic ring may comprise 5, 6, 7, 8, 9, 10, 11, or 12 ring atoms, comprising 1, 2, 3 or 4 heteroatoms selected from N, O and S, where ring atoms refer to the sum of carbon and heteroatoms in the one or more rings (e.g., be a 5-membered, 6-membered, 7-membered, 8-membered, 9-membered, 10-membered, 11-membered, or 12-membered heteroaryl). Examples of heteroaryl group include pyrrolyl, furanyl, oxazolyl, pyrazinyl, pyridazinyl, oxadiazolyl, isooxazol, pyrazol, triazolyl, and pyrimidinyl. Particular examples of heteroaromatic rings include pyridinyn and thiazole.

The term "hydroxy" denotes a -OH group.

The term "oxo" denotes a refers to a doubly bonded oxygen (=O), i.e. a carbonyl group.

The term "hydroxyalkyl" denotes an alkyl group wherein at least one of the hydrogen atoms of the alkyl group has been replaced by a hydroxy group. Examples of hydroxyalkyl include hydroxymethyl, hydroxyethyl, hydroxypropyl, hydroxymethylpropyl and dihydroxypropyl. Particular example is 1-hydroxy-1-methyl-ethyl.

"Thiolalkyl" group is an alkyl group as defined above with the indicated number of carbon atoms covalently bound to the group it substitutes by a sulfur bridge (-S-). Particular example is methylsulfanyl (-S-CH₃).

"Halothiolalkyl" group is a thiolalkyl group wherein at least one of the hydrogen atoms of the thiolalkyl group has been replaced by the same or different halogen atoms. Particular examples is difluoromethylthio or difluoromethylsulfanyl.

The term "pharmaceutically acceptable salts" refers to those salts which retain the biological effectiveness and properties of the free bases or free acids, which are not biologically or otherwise undesirable. The salts are formed with inorganic acids such as hydrochloric acid, hydrobromic acid, sulfuric acid, nitric acid, phosphoric acid, particularly hydrochloric acid, and organic acids such as formic acid, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, N-acetylcystein. In addition these salts may be prepared from addition of an inorganic base or an organic base to the free acid. Salts derived from an inorganic base include, but are not limited to, the sodium, potassium, lithium, ammonium, calcium, magnesium salts. Salts derived from organic bases include, but are not limited to salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, lysine, arginine, N-ethylpiperidine, piperidine, polyamine resins. The compound of formula I can also be present in the form of zwitterions. Particularly preferred pharmaceutically acceptable salts of compounds of formula I are the salts formed with formic acid and the salts formed with hydrochloric acid yielding a hydrochloride, dihydrochloride or trihydrochloride salt.

The abbreviation uM means microMolar and is equivalent to the symbol µM.

The abbreviation uL means microliter and is equivalent to the symbol µL.

The abbreviation ug means microgram and is equivalent to the symbol µg.

The compounds of formula I can contain several asymmetric centers and can be present in the form of optically pure enantiomers, mixtures of enantiomers such as, for example, racemates, optically pure diastereoisomers, mixtures of diastereoisomers, diastereoisomeric racemates or mixtures of diastereoisomeric racemates.

According to the Cahn-Ingold-Prelog Convention the asymmetric carbon atom can be of the "R" or "S" configuration.

Also an embodiment of the present invention provides compounds according to formula I as described herein and pharmaceutically acceptable salts or esters thereof, in particular compounds according to formula I as described herein and pharmaceutically acceptable salts thereof, more particularly compounds according to formula I as described herein.

An embodiment of the present invention provides compounds according to formula I as described herein, wherein R¹ is H, halo, haloalkyl, haloalkoxy, alkyl, alkoxy, alkoxyalkoxy, alkoxyalkyl, thiolalkyl, cyanoalkyl, cycloalkoxy, cycloalkyl, cycloalkylalkyl, or heterocycloalkyl.

A particular embodiment of the present invention provides compounds according to formula I as described herein, wherein R¹ is H, alkoxy, cycloalkyl or haloalkoxy.

An embodiment of the present invention provides compounds according to formula I as described herein, wherein R² is alkoxy, alkoxyalkoxy, alkoxyalkyl, alkylsulfonylalkyl, alkyl, alkenyl, alkynyl, alkynylalkoxy, cyano, cyanoalkyl, cycloalkyl, cycloalkoxy, cycloalkylalkyl, cyanoalkoxy, halo, haloalkoxy, (haloalkoxy)alkoxy, thiolalkyl, halothiolalkyl, halohydroxyalkoxy, haloalkyl, heterocycloalkyl, heterocycloalkylalkyl, heterocycloalkylalkynyl or heterocycloalkylalkoxy.

A particular embodiment of the present invention provides compounds according to formula I as described herein, wherein R² is alkyl, alkenyl, alkynyl, alkoxyalkyl, alkynyl, cyanoalkoxy, halo, haloalkoxy, haloalkyl or cycloalkyl.

An embodiment of the present invention provides compounds according to formula I as described herein, wherein R³ is H or alkoxy.

An embodiment of the present invention provides compounds according to formula I as described herein, wherein X₂ is NH or S.

An embodiment of the present invention provides compounds according to formula I as described herein, wherein R⁴ is H, halo, alkyl, alkoxy, thiolalkyl, cyanoalkyl, cycloalkyl, haloalkoxy, haloalkyl, heterocycloalkyl, or hydroxyalkyl, and R⁵ is H, halo, haloalkyl, haloalkoxy, halothiolalkyl, alkoxy, alkoxycarbonyl, alkyl, hydroxyalkyl, thiolalkyl, alkylsulfino, alkylsulfano, cyano, cyanoalkyl, cyanoalkoxy, dialkylamino, cycloalkyl, cycloalkoxy, or heterocycloalkyl, or R⁴ and R⁵ are connected to form a 5-membered heterocycloalkyl comprising a single O heteroatom, a 6-membered heterocycloalkyl comprising a single N heteroatom substituted by oxo and alkyl, a 5-membered heteroaromatic ring comprising one N heteroatom and one S heteroatom, or a 6-membered heteroaromatic ring comprising a single N heteroatom.

An embodiment of the present invention provides compounds according to formula I as described herein, wherein R⁴ is halo, alkyl or haloalkyl, and R⁵ is H, halo, haloalkyl, haloalkoxy, halothiolalkyl, alkoxy, alkyl, thiolalkyl, cyano, cyanoalkyl, cyanoalkoxy, dialkylamino or cycloalkoxy, or R⁴ and R⁵ are connected to form a 5-membered heterocycloalkyl comprising a single O heteroatom, or a 6-membered heteroaromatic ring comprising a single N heteroatom.

An embodiment of the present invention provides compounds according to formula I as described herein, wherein R⁴ is H, halo, alkyl, alkoxy, thiolalkyl, cyanoalkyl, cycloalkyl, haloalkoxy, haloalkyl, heterocycloalkyl, or hydroxyalkyl.

An embodiment of the present invention provides compounds according to formula I as described herein, wherein R⁴ is halo, alkyl, or haloalkyl.

A particular embodiment of the present invention provides compounds according to formula I as described herein, wherein R⁵ is H, halo, haloalkyl, haloalkoxy, halothiolalkyl, alkoxy, alkyl, hydroxyalkyl, alkoxycarbonyl, thiolalkyl, alkylsulfino, alkylsulfano, cyano, cyanoalkyl, cyanoalkoxy, dialkylamino, cycloalkyl, cycloalkoxy, heterocycloalkyl, or heterocycloalkyl substituted with alkyl and oxo.

A particular embodiment of the present invention provides compounds according to formula I as described herein, wherein R⁵ is H, halo, haloalkyl, haloalkoxy, halothiolalkyl, alkoxy, alkyl, thiolalkyl, cyano, cyanoalkyl, cyanoalkoxy, dialkylamino or cycloalkoxy.

An embodiment of the present invention provides compounds according to formula I as described herein, wherein
R¹ is H, halo, haloalkyl, haloalkoxy, alkyl, alkoxy, alkoxyalkoxy, alkoxyalkyl, thiolalkyl, cyanoalkyl, cycloalkoxy, cycloalkyl, cycloalkylalkyl, or heterocycloalkyl;
R² is alkoxy, alkoxyalkoxy, alkoxyalkyl, alkylsulfonylalkyl, alkyl, alkenyl, alkynyl, alkynylalkoxy, cyano, cyanoalkyl, cycloalkyl, cycloalkoxy, cycloalkylalkyl, cyanoalkoxy, halo, haloalkoxy, (haloalkoxy)alkoxy, thiolalkyl, halothiolalkyl, halohydroxyalkoxy, haloalkyl, heterocycloalkyl, heterocycloalkylalkyl, heterocycloalkylalkynyl or heterocycloalkylalkoxy;
R³ is H, halo, alkyl, alkoxy, haloalkoxy, or thiolalkyl;
X₁ is CR⁵ or N;
X₂ is NH or S;
R⁴ is H, halo, alkyl, alkoxy, thiolalkyl, cyanoalkyl, cycloalkyl, haloalkoxy, haloalkyl, heterocycloalkyl, or hydroxyalkyl, and R⁵ is H, halo, haloalkyl, haloalkoxy, halothiolalkyl, alkoxy, alkyl, alkoxycarbonyl, hydroxyalkyl, thiolalkyl, alkylsulfino, alkylsulfano, cyano, cyanoalkyl, cyanoalkoxy, dialkylamino, cycloalkyl, cycloalkoxy, or heterocycloalkyl, or R⁴ and R⁵ are connected to form a 5-membered heterocycloalkyl comprising a single O heteroatom, a 6-membered heterocycloalkyl comprising a single N heteroatom substituted with alkyl and oxo, a 5-membered heteroaromatic ring comprising one N heteroatom and one S heteroatom, or a 6-membered heteroaromatic ring comprising a single N heteroatom;
and pharmaceutically acceptable salts.

A particular embodiment of the present invention provides compounds according to formula I as described herein, wherein
R¹ is H, alkoxy, cycloalkyl or haloalkoxy;
R² is alkyl, alkenyl, alkynyl, alkoxyalkyl, alkynyl, cyanoalkoxy, halo, haloalkoxy, haloalkyl or cycloalkyl;
R³ is H or alkoxy;
X₁ is CR⁵ or N;
X₂ is NH or S;
R⁴ is halo, alkyl, or haloalkyl, and R⁵ is H, halo, haloalkyl, haloalkoxy, halothiolalkyl, alkoxy, alkyl, thiolalkyl, cyano, cyanoalkyl, cyanoalkoxy dialkylamino or cycloalkoxy, or R⁴ and R⁵ are connected to form a 5-membered heterocycloalkyl comprising a single O heteroatom or a 6-membered heteroaromatic ring comprising a single N heteroatom;
and pharmaceutically acceptable salts.

An embodiment of the present invention provides compounds according to formula I as described herein, wherein R¹ is H, alkyl, alkoxy or cycloalkyl.

A particular embodiment of the present invention provides compounds according to formula I as described herein, wherein R¹ is H, alkoxy or cycloalkyl.

An embodiment of the present invention provides compounds according to formula I as described herein, wherein R² is alkoxy, alkoxyalkoxy, alkyl, alkynyl, cyano, cycloalkyl, halo, haloalkoxy, (haloalkoxy)alkoxy, or haloalkyl.

A particular embodiment of the present invention provides compounds according to formula I as described herein, wherein R² is alkyl, alkynyl, halo, haloalkoxy, haloalkyl.

An embodiment of the present invention provides compounds according to formula I as described herein, wherein R³ is H, alkoxy or haloalkoxy.

An embodiment of the present invention provides compounds according to formula I as described herein, wherein X₂ is NH or S.

An embodiment of the present invention provides compounds according to formula I as described herein, wherein R⁴ is halo.

An embodiment of the present invention provides compounds according to formula I as described herein, wherein R⁵ is H, halo, haloalkyl or cycloalkoxy.

A particular embodiment of the present invention provides compounds according to formula I as described herein, wherein R⁵ is H, halo, or haloalkyl.

An embodiment of the present invention provides compounds according to formula I as described herein, wherein
R¹ is H, halo, alkyl, alkoxy, alkoxyalkyl, alkoxyalkoxy, cyanoalkyl, haloalkyl, haloalkoxy, thiolalkyl, cycloalkyl, cycloalkylalkyl, cycloalkylalkoxy, or heterocycloalkyl;
R² is cyano, halo, alkyl, alkynyl, alkoxy, alkenylalkyl, alkoxyalkyl, alkoxyalkoxy, haloalkyl, haloalkoxy, haloalkoxyalkyl, (haloalkoxy)alkoxy, cyanoalkyl, thiolalkyl, halothiolalkyl, cycloalkyl, cycloalkylalkyl, cycloalkylalkoxy, heterocycloalkyl, heterocycloalkylalkyl, or heterocycloalkylalkoxy;
R³ is H, halo, alkyl, alkoxy, haloalkoxy, or thiolalkyl;
X₁ is CR⁵ or N;
X₂ is NH, -N-R⁶, S, -S(O)-, or -S(O₂)-;
R⁴ is halo, alkyl, alkoxy, haloalkyl, haloalkoxy, cycloalkyl or heterocycloalkyl;
R⁵ is H, halo, cyano, alkyl, alkoxy, N-alkyl, haloalkyl, alkylsulfone, cycloalkyl or cycloalkoxy;
or R⁴ and R⁵ are connected to form a 5 or 6 member cycloalkyl, heterocycloalkyl or heteroaromatic ring comprising O, N or S heteroatom;
R⁶ is H, alkyl, alkylhydroxy, or haloalkyl;
and pharmaceutically acceptable salts.

An embodiment of the present invention provides compounds according to formula I as described herein, wherein
R¹ is H, alkyl, alkoxy or cycloalkyl;
R² is cyano, halo, alkyl, alkynyl, alkoxy, alkoxyalkoxy, haloalkyl, haloalkoxy, (haloalkoxy)alkoxy, or cycloalkyl;
R³ is H, alkoxy or haloalkoxy;
X₁ is CR⁵ or N;
X₂ is NH or S;
R⁴ is halo;
R⁵ is H, halo, haloalkyl or cycloalkoxy;
and pharmaceutically acceptable salts.

A particular embodiment of the present invention provides compounds according to formula I as described herein, wherein
R¹ is H, alkoxy or cycloalkyl;
R² is alkyl, alkynyl, halo, haloalkoxy, haloalkyl;
R³ is H, alkoxy or haloalkoxy;
X₁ is CR⁵ or N;
X₂ is NH or S;
R⁴ is halo;
R⁵ is H, halo, or haloalkyl;
and pharmaceutically acceptable salts.

Particular examples of compounds of formula I as described herein are selected from
6-chloro-N-[5-(2-fluoroethoxy)-4-methoxy-pyrimidin-2-yl]-1H-indole-3-sulfonamide;
6-chloro-7-fluoro-N-[5-(2-fluoroethoxy)-4-methoxy-pyrimidin-2-yl]-1H-indole-3-sulfonamide;
6,7-dichloro-N-[5-(2-fluoroethoxy)-4-methoxy-pyrimidin-2-yl]-1H-indole-3-sulfonamide;
6-chloro-7-(difluoromethyl)-N-[5-(2-fluoroethoxy)-4-methoxy-pyrimidin-2-yl]-1H-indole-3-sulfonamide;
6-chloro-7-(cyclopropoxy)-N-[5-(2-fluoroethoxy)-4-methoxy-pyrimidin-2-yl]-1H-indole-3-sulfonamide;
6-chloro-N-(5-ethoxy-4-methoxy-pyrimidin-2-yl)-1H-indole-3-sulfonamide;
6-chloro-N-[4-methoxy-5-(2-methoxyethoxy)pyrimidin-2-yl]-1H-indole-3-sulfonamide;
6-chloro-N-[5-(difluoromethoxy)-4-methoxy-pyrimidin-2-yl]-1H-indole-3-sulfonamide;
6-chloro-N-[5-[2-(difluoromethoxy)ethoxy]-4-methoxy-pyrimidin-2-yl]-1H-indole-3-sulfonamide;
6-chloro-N-[5-(2-fluoroethoxy)-4-methoxy-pyrimidin-2-yl]-1H-pyrrolo[2,3-b]pyridine-3-sulfonamide;
6-chloro-N-(5-chloro-4,6-dimethoxy-pyrimidin-2-yl)-1H-indole-3-sulfonamide;
N-(5-bromo-4,6-dimethoxy-pyrimidin-2-yl)-6-chloro-1H-indole-3-sulfonamide;
N-(5-bromo-4-ethoxy-6-methoxy-pyrimidin-2-yl)-6-chloro-1H-indole-3-sulfonamide;
N-[5-bromo-4-(2-fluoroethoxy)-6-methoxy-pyrimidin-2-yl]-6-chloro-1H-indole-3-sulfonamide;
6-chloro-N-(5-ethyl-4-methoxy-pyrimidin-2-yl)-1H-indole-3-sulfonamide;
6-chloro-N-(5-ethyl-4,6-dimethoxy-pyrimidin-2-yl)-1H-indole-3-sulfonamide;
6-chloro-N-(4,6-dimethoxy-5-methyl-pyrimidin-2-yl)-1H-indole-3-sulfonamide;
N-[5-bromo-4-(difluoromethoxy)-6-methoxy-pyrimidin-2-yl]-6-chloro-1H-indole-3-sulfonamide;
6-chloro-N-[4-cyclopropyl-5-(2,2-difluoroethoxy)-6-methoxy-pyrimidin-2-yl]-1H-indole-3-sulfonamide;
6-chloro-N-[5-(2,2-difluoroethoxy)-4-methoxy-6-methyl-pyrimidin-2-yl]-1H-indole-3-sulfonamide;
6-chloro-N-[5-(3-fluoropropyl)-4-methoxy-pyrimidin-2-yl]-1H-indole-3-sulfonamide;
N-(5-bromo-4-methoxy-pyrimidin-2-yl)-6-chloro-1H-indole-3-sulfonamide;
6-chloro-N-(5-cyano-4-methoxy-pyrimidin-2-yl)-1H-indole-3-sulfonamide;
6-chloro-N-[5-(2-fluoroethoxy)-4-methoxy-pyrimidin-2-yl]benzothiophene-3-sulfonamide;
6-bromo-N-[5-(2-fluoroethoxy)-4-methoxy-pyrimidin-2-yl]-1H-indole-3-sulfonamide;
6-chloro-N-(4-methoxy-5-methyl-pyrimidin-2-yl)-1H-indole-3-sulfonamide;
6-chloro-N-[5-(2-fluoroethoxy)-4-methoxy-pyrimidin-2-yl]thieno[2,3-b]pyridine-3-sulfonamide;
6-chloro-N-(5-cyclopropyl-4-methoxy-pyrimidin-2-yl)-1H-indole-3-sulfonamide;
6-chloro-N-(4-methoxy-5-prop-1-ynyl-pyrimidin-2-yl)-1H-indole-3-sulfonamide;
6-bromo-N-(5-bromo-4,6-dimethoxy-pyrimidin-2-yl)-1H-pyrrolo[2,3-b]pyridine-3-sulfonamide;
N-(5-bromo-4,6-dimethoxy-pyrimidin-2-yl)-6-chloro-1H-pyrrolo[2,3-b]pyridine-3-sulfonamide;
6-chloro-N-(5-cyano-4,6-dimethoxy-pyrimidin-2-yl)-1H-indole-3-sulfonamide;
and pharmaceutically acceptable salts thereof.

Also particular examples of compounds of formula I as described herein are selected from
N-(5-bromo-4,6-dimethyl-pyrimidin-2-yl)-6-chloro-1H-indole-3-sulfonamide;
6-chloro-N-[5-(2,2-difluoroethoxy)pyrimidin-2-yl]-1H-indole-3-sulfonamide;
6-chloro-N-[5-(2,2-difluoroethoxy)-4-methylsulfanyl-pyrimidin-2-yl]-1H-indole-3-sulfonamide;
6-chloro-N-(4-methoxy-5-methylsulfanyl-pyrimidin-2-yl)-1H-indole-3-sulfonamide;
6-chloro-N-[5-(difluoromethylthio)-4-methoxy-pyrimidin-2-yl]-1H-indole-3-sulfonamide;
6-chloro-N-[5-(cyclopropoxy)-4-methoxy-pyrimidin-2-yl]-1H-indole-3-sulfonamide;
6-chloro-N-[5-(3,3-difluoropropoxy)-4-methoxy-pyrimidin-2-yl]-1H-indole-3-sulfonamide;
6-chloro-N-[4-methoxy-5-(3,3,3-trifluoropropoxy)pyrimidin-2-yl]-1H-indole-3-sulfonamide;
6-chloro-N-[4-methoxy-5-(1,1,2-trifluoroethoxy)pyrimidin-2-yl]-1H-indole-3-sulfonamide;
6-chloro-N-[5-(1,1-difluoroethoxy)-4-methoxy-pyrimidin-2-yl]-1H-indole-3-sulfonamide;
6-chloro-N-[4-methoxy-5-(1,1,2,2-tetrafluoroethoxy)pyrimidin-2-yl]-1H-indole-3-sulfonamide;
6-chloro-N-[5-(2-chloro-1,1,2-trifluoro-ethoxy)-4-methoxy-pyrimidin-2-yl]-1H-indole-3-sulfonamide;
6-chloro-N-(4-methoxy-5-propyl-pyrimidin-2-yl)-1H-indole-3-sulfonamide;
6-chloro-N-[5-(cyclopropylmethyl)-4-methoxy-pyrimidin-2-yl]-1H-indole-3-sulfonamide;
6-chloro-N-[5-(2,2-difluoroethyl)-4-methoxy-pyrimidin-2-yl]-1H-indole-3-sulfonamide;
6-chloro-N-[5-(3,3-difluoropropyl)-4-methoxy-pyrimidin-2-yl]-1H-indole-3-sulfonamide;
6-chloro-N-[4-methoxy-5-(2-methoxyethyl)pyrimidin-2-yl]-1H-indole-3-sulfonamide;
6-chloro-N-[4-methoxy-5-(oxetan-3-yl)pyrimidin-2-yl]-1H-indole-3-sulfonamide;
6-chloro-N-[5-(fluoromethoxy)-4,6-dimethoxy-pyrimidin-2-yl]-1H-indole-3-sulfonamide;
6-chloro-N-[5-(difluoromethoxy)-4,6-dimethoxy-pyrimidin-2-yl]-1H-indole-3-sulfonamide;
6-chloro-N-[5-(2-fluoroethoxy)-4,6-dimethoxy-pyrimidin-2-yl]-1H-indole-3-sulfonamide;
6-chloro-N-[5-(2,2-difluoroethoxy)-4,6-dimethoxy-pyrimidin-2-yl]-1H-indole-3-sulfonamide;
6-chloro-N-[4,6-dimethoxy-5-(1,1,2-trifluoroethoxy)pyrimidin-2-yl]-1H-indole-3-sulfonamide;
N-(5-allyl-4,6-dimethoxy-pyrimidin-2-yl)-6-chloro-1H-indole-3-sulfonamide;
6-chloro-N-(4,6-dimethoxy-5-prop-1-ynyl-pyrimidin-2-yl)-1H-indole-3-sulfonamide;
6-chloro-N-(4,6-dimethoxy-5-propyl-pyrimidin-2-yl)-1H-indole-3-sulfonamide;
6-chloro-N-[5-(2-fluoroethyl)-4,6-dimethoxy-pyrimidin-2-yl]-1H-indole-3-sulfonamide;
6-chloro-N-[5-(2,2-difluoroethyl)-4,6-dimethoxy-pyrimidin-2-yl]-1H-indole-3-sulfonamide;
6-chloro-N-[5-(3-fluoropropyl)-4,6-dimethoxy-pyrimidin-2-yl]-1H-indole-3-sulfonamide;
6-chloro-N-[5-(3,3-difluoropropyl)-4,6-dimethoxy-pyrimidin-2-yl]-1H-indole-3-sulfonamide;
6-chloro-N-[5-(2,2-difluoropropyl)-4,6-dimethoxy-pyrimidin-2-yl]-1H-indole-3-sulfonamide;
6-chloro-N-[4,6-dimethoxy-5-(3,3,3-trifluoropropyl)pyrimidin-2-yl]-1H-indole-3-sulfonamide;
6-chloro-N-[4,6-dimethoxy-5-(methoxymethyl)pyrimidin-2-yl]-1H-indole-3-sulfonamide;
6-chloro-N-[4,6-dimethoxy-5-(2-methoxyethyl)pyrimidin-2-yl]-1H-indole-3-sulfonamide;
6-chloro-N-[5-(cyanomethyl)-4,6-dimethoxy-pyrimidin-2-yl]-1H-indole-3-sulfonamide;
6-chloro-N-[5-(2-cyanoethyl)-4,6-dimethoxy-pyrimidin-2-yl]-1H-indole-3-sulfonamide;
6-chloro-N-(5-cyclopropyl-4,6-dimethoxy-pyrimidin-2-yl)-1H-indole-3-sulfonamide;
N-[5-bromo-4-methoxy-6-(2-methoxyethoxy)pyrimidin-2-yl]-6-chloro-1H-indole-3-sulfonamide;
N-[5-bromo-4-(cyclopropoxy)-6-methoxy-pyrimidin-2-yl]-6-chloro-1H-indole-3-sulfonamide;
6-chloro-N-[4-(difluoromethoxy)-6-methoxy-5-methyl-pyrimidin-2-yl]-1H-indole-3-sulfonamide;
N-(5-bromo-4-fluoro-6-methoxy-pyrimidin-2-yl)-6-chloro-1H-indole-3-sulfonamide;
6-chloro-N-[5-(2,2-difluoroethoxy)-4-fluoro-6-methoxy-pyrimidin-2-yl]-1H-indole-3-sulfonamide;
6-chloro-N-(4-chloro-5,6-dimethoxy-pyrimidin-2-yl)-1H-indole-3-sulfonamide;
6-chloro-N-[4-chloro-5-(2,2-difluoroethoxy)pyrimidin-2-yl]-1H-indole-3-sulfonamide;
6-chloro-N-[5-(2,2-difluoroethoxy)-4-ethyl-6-methoxy-pyrimidin-2-yl]-1H-indole-3-sulfonamide;
6-chloro-N-[5-(2,2-difluoroethyl)-4-methoxy-6-methyl-pyrimidin-2-yl]-1H-indole-3-sulfonamide;
6-chloro-N-[4-cyclopropyl-5-(2,2-difluoroethyl)-6-methoxy-pyrimidin-2-yl]-1H-indole-3-sulfonamide;
N-[5-bromo-4-(cyclopropylmethyl)-6-methoxy-pyrimidin-2-yl]-6-chloro-1H-indole-3-sulfonamide;
N-[5-bromo-4-methoxy-6-(methoxymethyl)pyrimidin-2-yl]-6-chloro-1H-indole-3-sulfonamide;
N-[5-bromo-4-(1,1-difluoroethyl)-6-methoxy-pyrimidin-2-yl]-6-chloro-1H-indole-3-sulfonamide;
N-[5-bromo-4-(fluoromethoxy)-6-methoxy-pyrimidin-2-yl]-6-chloro-1H-indole-3-sulfonamide;
N-[5-bromo-4-(difluoromethyl)-6-methoxy-pyrimidin-2-yl]-6-chloro-1H-indole-3-sulfonamide;
N-[5-bromo-4-(cyanomethyl)-6-methoxy-pyrimidin-2-yl]-6-chloro-1H-indole-3-sulfonamide;
6-chloro-N-[5-(difluoromethoxy)-4-methoxy-6-methylsulfanyl-pyrimidin-2-yl]-1H-indole-3-sulfonamide;
N-[4,6-dimethoxy-5-(1,1,2-trifluoroethoxy)pyrimidin-2-yl]-6-fluoro-1H-indole-3-sulfonamide;
6-bromo-N-[5-(2,2-difluoroethyl)-4-methoxy-pyrimidin-2-yl]-1H-indole-3-sulfonamide;
6-bromo-N-[5-(2-cyanoethyl)-4-methoxy-pyrimidin-2-yl]-1H-indole-3-sulfonamide;
6-bromo-N-[5-(difluoromethoxy)-4,6-dimethoxy-pyrimidin-2-yl]-1H-indole-3-sulfonamide;
6-bromo-N-[5-(2-fluoroethoxy)-4,6-dimethoxy-pyrimidin-2-yl]-1H-indole-3-sulfonamide;
6-bromo-N-[5-(2,2-difluoroethoxy)-4,6-dimethoxy-pyrimidin-2-yl]-1H-indole-3-sulfonamide;
6-bromo-N-[5-(2-cyanoethyl)-4,6-dimethoxy-pyrimidin-2-yl]-1H-indole-3-sulfonamide;
N-[5-(difluoromethoxy)-4,6-dimethoxy-pyrimidin-2-yl]-6-(difluoromethyl)-1H-pyrrolo[2,3-b]pyridine-3-sulfonamide;
6-(difluoromethyl)-N-[5-(2-fluoroethoxy)-4,6-dimethoxy-pyrimidin-2-yl]-1H-pyrrolo[2,3-b]pyridine-3-sulfonamide;
N-[5-(2,2-difluoroethoxy)-4,6-dimethoxy-pyrimidin-2-yl]-6-(difluoromethyl)-1H-pyrrolo[2,3-b]pyridine-3-sulfonamide;
6-(difluoromethyl)-N-[4,6-dimethoxy-5-(1,1,2-trifluoroethoxy)pyrimidin-2-yl]-1H-pyrrolo[2,3-b]pyridine-3-sulfonamide;
6-(difluoromethyl)-N-[5-(3,3-difluoropropyl)-4,6-dimethoxy-pyrimidin-2-yl]-1H-pyrrolo[2,3-b]pyridine-3-sulfonamide;
N-[5-(cyanomethoxy)-4,6-dimethoxy-pyrimidin-2-yl]-6-(difluoromethyl)-1H-pyrrolo[2,3-b]pyridine-3-sulfonamide;
6-(difluoromethyl)-N-[4-methoxy-5-(1,1,2-trifluoroethoxy)pyrimidin-2-yl]-1H-indole-3-sulfonamide;
N-[5-(difluoromethoxy)-4,6-dimethoxy-pyrimidin-2-yl]-6-(difluoromethyl)-1H-indole-3-sulfonamide;
N-[5-(2,2-difluoroethoxy)-4,6-dimethoxy-pyrimidin-2-yl]-6-(difluoromethyl)-1H-indole-3-sulfonamide;
N-[5-(2-cyanoethyl)-4,6-dimethoxy-pyrimidin-2-yl]-6-(difluoromethyl)-1H-indole-3-sulfonamide;
N-[5-(2,2-difluoroethyl)-4-methoxy-pyrimidin-2-yl]-6-(difluoromethoxy)-1H-indole-3-sulfonamide;
6-chloro-N-[5-(2-cyanoethyl)-4-methoxy-pyrimidin-2-yl]-7-(difluoromethylthio)-1H-indole-3-sulfonamide;
6-chloro-7-(difluoromethylthio)-N-[5-(2-fluoroethoxy)-4,6-dimethoxy-pyrimidin-2-yl]-1H-indole-3-sulfonamide;
6-chloro-N-[5-(2-cyanoethyl)-4,6-dimethoxy-pyrimidin-2-yl]-7-(difluoromethylthio)-1H-indole-3-sulfonamide;
7-chloro-N-[5-(2,2-difluoroethyl)-4-methoxy-pyrimidin-2-yl]-1H-indole-3-sulfonamide;
6-bromo-N-[5-(2-fluoroethoxy)-4,6-dimethoxy-pyrimidin-2-yl]-1H-pyrrolo[2,3-b]pyridine-3-sulfonamide;
6-bromo-N-[4,6-dimethoxy-5-(1,1,2-trifluoroethoxy)pyrimidin-2-yl]-1H-pyrrolo[2,3-b]pyridine-3-sulfonamide;
6-bromo-N-[5-(2,2-difluoroethyl)-4,6-dimethoxy-pyrimidin-2-yl]-1H-pyrrolo[2,3-b]pyridine-3-sulfonamide;
6-bromo-N-[5-(3,3-difluoropropyl)-4,6-dimethoxy-pyrimidin-2-yl]-1H-pyrrolo[2,3-b]pyridine-3-sulfonamide;
6-bromo-N-[5-(2-cyanoethyl)-4,6-dimethoxy-pyrimidin-2-yl]-1H-pyrrolo[2,3-b]pyridine-3-sulfonamide;
6-bromo-N-[5-(cyanomethoxy)-4,6-dimethoxy-pyrimidin-2-yl]-1H-pyrrolo[2,3-b]pyridine-3-sulfonamide;
6-chloro-N-[5-(2,2-difluoroethyl)-4,6-dimethoxy-pyrimidin-2-yl]-7-(dimethylamino)-1H-indole-3-sulfonamide;
N-[5-(difluoromethoxy)-4,6-dimethoxy-pyrimidin-2-yl]-1H-pyrrolo[3,2-h]quinoline-3-sulfonamide;
N-[5-(2-fluoroethoxy)-4,6-dimethoxy-pyrimidin-2-yl]-1H-pyrrolo[3,2-h]quinoline-3-sulfonamide;
6-chloro-N-(4,6-dimethoxy-5-methyl-pyrimidin-2-yl)-1H-pyrrolo[2,3-b]pyridine-3-sulfonamide;
6-chloro-N-[5-(difluoromethoxy)-4-methoxy-pyrimidin-2-yl]-1H-pyrrolo[2,3-b]pyridine-3-sulfonamide;
6-chloro-N-[5-(2,2-difluoroethyl)-4,6-dimethoxy-pyrimidin-2-yl]-1H-pyrrolo[2,3-b]pyridine-3-sulfonamide;
6-chloro-N-[5-(3,3-difluoropropyl)-4,6-dimethoxy-pyrimidin-2-yl]-1H-pyrrolo[2,3-b]pyridine-3-sulfonamide;
6-chloro-N-[5-(2-cyanoethyl)-4,6-dimethoxy-pyrimidin-2-yl]-1H-pyrrolo[2,3-b]pyridine-3-sulfonamide;
6-chloro-N-[5-(2,2-difluoroethoxy)-4-methoxy-pyrimidin-2-yl]-7-fluoro-1H-indole-3-sulfonamide;
6-chloro-N-[5-(2,2-difluoroethyl)-4-methoxy-pyrimidin-2-yl]-7-fluoro-1H-indole-3-sulfonamide;
6-chloro-N-[5-(difluoromethoxy)-4,6-dimethoxy-pyrimidin-2-yl]-7-fluoro-1H-indole-3-sulfonamide;
6-chloro-N-[5-(difluoromethoxy)-4-methoxy-pyrimidin-2-yl]-7-fluoro-1H-indole-3-sulfonamide;
6-chloro-7-(cyclopropoxy)-N-[5-(2,2-difluoroethyl)-4-methoxy-pyrimidin-2-yl]-1H-indole-3-sulfonamide;
7-bromo-N-[5-(2,2-difluoroethyl)-4-methoxy-pyrimidin-2-yl]-6-fluoro-1H-indole-3-sulfonamide;
7-bromo-N-[5-(2,2-difluoroethyl)-4-methoxy-pyrimidin-2-yl]-1H-indole-3-sulfonamide;
7-chloro-N-[5-(2,2-difluoroethyl)-4-methoxy-pyrimidin-2-yl]-6-fluoro-1H-indole-3-sulfonamide;
N-[5-(2-fluoroethoxy)-4,6-dimethoxy-pyrimidin-2-yl]-6-methyl-1H-pyrrolo[2,3-b]pyridine-3-sulfonamide;
N-[5-(2,2-difluoroethyl)-4,6-dimethoxy-pyrimidin-2-yl]-6-methyl-1H-pyrrolo[2,3-b]pyridine-3-sulfonamide;
6-chloro-N-[5-(2,2-difluoroethyl)-4,6-dimethoxy-pyrimidin-2-yl]-7-morpholino-1H-indole-3-sulfonamide;
7-bromo-N-[5-(2,2-difluoroethyl)-4,6-dimethoxy-pyrimidin-2-yl]-1H-indole-3-sulfonamide;
6-chloro-N-[5-(2,2-difluoroethoxy)-4-methoxy-pyrimidin-2-yl]-1H-indole-3-sulfonamide;
6-chloro-N-[5-(1,1-difluoroethyl)-4-methoxy-pyrimidin-2-yl]-1H-indole-3-sulfonamide;
6-chloro-N-(4-ethyl-6-methoxy-5-methyl-pyrimidin-2-yl)-1H-indole-3-sulfonamide;
N-(5-bromo-4-methoxy-6-tetrahydropyran-4-yl-pyrimidin-2-yl)-6-chloro-1H-indole-3-sulfonamide;
6-chloro-N-[5-(2,2-difluoroethyl)-4-methoxy-pyrimidin-2-yl]thieno[2,3-b]pyridine-3-sulfonamide;
N-[5-(2,2-difluoroethyl)-4-methoxy-pyrimidin-2-yl]-6-fluoro-1H-indole-3-sulfonamide;
7-bromo-6-chloro-N-[5-(2-fluoroethoxy)-4-methoxy-pyrimidin-2-yl]-1H-indole-3-sulfonamide;
7-bromo-6-chloro-N-[5-(2,2-difluoroethyl)-4-methoxy-pyrimidin-2-yl]-1H-indole-3-sulfonamide;
6-bromo-N-[5-(2,2-difluoroethoxy)-4-methoxy-pyrimidin-2-yl]-1H-indole-3-sulfonamide;
6-bromo-N-(4,6-dimethoxy-5-methyl-pyrimidin-2-yl)thieno[2,3-b]pyridine-3-sulfonamide;
6-bromo-N-[5-(2,2-difluoroethoxy)-4-methoxy-pyrimidin-2-yl]thieno[2,3-b]pyridine-3-sulfonamide;
6-bromo-N-[5-(2-fluoroethoxy)-4-methoxy-pyrimidin-2-yl]thieno[2,3-b]pyridine-3-sulfonamide;
6-bromo-N-[4-methoxy-5-(1,1,2-trifluoroethoxy)pyrimidin-2-yl]thieno[2,3-b]pyridine-3-sulfonamide;
6-bromo-N-[5-(2,2-difluoroethyl)-4-methoxy-pyrimidin-2-yl]thieno[2,3-b]pyridine-3-sulfonamide;
6-bromo-N-[5-(2-cyanoethyl)-4-methoxy-pyrimidin-2-yl]thieno[2,3-b]pyridine-3-sulfonamide;
6-bromo-N-[5-(2,2-difluoroethyl)-4,6-dimethoxy-pyrimidin-2-yl]thieno[2,3-b]pyridine-3-sulfonamide;
6-bromo-N-[5-(2-cyanoethyl)-4,6-dimethoxy-pyrimidin-2-yl]thieno[2,3-b]pyridine-3-sulfonamide;
N-[5-(2,2-difluoroethyl)-4-methoxy-pyrimidin-2-yl]-6-ethyl-1H-indole-3-sulfonamide;
N-[5-(2,2-difluoroethyl)-4-methoxypyrimidin-2-yl]-6-propyl-1H-indole-3-sulfonamide;
6-chloro-N-[5-(2,2-difluoroethyl)-4-methoxy-pyrimidin-2-yl]benzothiophene-3-sulfonamide;
6-chloro-N-[5-(3,3-difluoropropyl)-4-methoxy-pyrimidin-2-yl]benzothiophene-3-sulfonamide;
6-chloro-N-[5-(2-cyanoethyl)-4-methoxy-pyrimidin-2-yl]benzothiophene-3-sulfonamide;
6-bromo-N-[5-(2,2-difluoroethoxy)-4-methoxy-pyrimidin-2-yl]benzothiophene-3-sulfonamide;
6-bromo-N-[5-(2-fluoroethoxy)-4-methoxy-pyrimidin-2-yl]benzothiophene-3-sulfonamide;
N-[5-(2-fluoroethoxy)-4-methoxy-pyrimidin-2-yl]-6-methyl-benzothiophene-3-sulfonamide;
6-bromo-N-(4,6-dimethoxy-5-methyl-pyrimidin-2-yl)-1H-pyrrolo[2,3-b]pyridine-3-sulfonamide;
6-bromo-N-[5-(2,2-difluoroethoxy)-4-methoxy-pyrimidin-2-yl]-1H-pyrrolo[2,3-b]pyridine-3-sulfonamide;
6-chloro-N-[5-(2,2-difluoroethyl)-4-methoxy-pyrimidin-2-yl]-7-fluoro-benzothiophene-3-sulfonamide;
6-chloro-N-[5-(2-cyanoethyl)-4-methoxy-pyrimidin-2-yl]-7-fluoro-benzothiophene-3-sulfonamide;
N-[5-(2,2-difluoroethyl)-4-methoxy-pyrimidin-2-yl]-6,7-difluoro-1H-indole-3-sulfonamide;
6-(difluoromethyl)-N-[5-(2-fluoroethoxy)-4-methoxy-pyrimidin-2-yl]thieno[2,3-b]pyridine-3-sulfonamide;
N-[5-(2,2-difluoroethyl)-4-methoxy-pyrimidin-2-yl]-6-(difluoromethyl)thieno[2,3-b]pyridine-3-sulfonamide;
N-[5-(2,2-difluoroethyl)-4,6-dimethoxy-pyrimidin-2-yl]-6-(difluoromethyl)thieno[2,3-b]pyridine-3-sulfonamide;
N-[5-(2,2-difluoroethoxy)-4,6-dimethoxy-pyrimidin-2-yl]-1H-pyrrolo[3,2-h]quinoline-3-sulfonamide;
N-[5-(2,2-difluoroethyl)-4,6-dimethoxy-pyrimidin-2-yl]-1H-pyrrolo[3,2-h]quinoline-3-sulfonamide;
6-chloro-N-[5-(2,2-difluoroethoxy)-4-methoxy-pyrimidin-2-yl]-1H-pyrrolo[2,3-b]pyridine-3-sulfonamide;
6-chloro-N-[4,6-dimethoxy-5-(1,1,2-trifluoroethoxy)pyrimidin-2-yl]-1H-pyrrolo[2,3-b]pyridine-3-sulfonamide;
6-bromo-N-[5-(difluoromethoxy)-4,6-dimethoxy-pyrimidin-2-yl]-1H-pyrrolo[2,3-b]pyridine-3-sulfonamide;
6-chloro-N-(4-chloro-5-methoxy-pyrimidin-2-yl)-1H-indole-3-sulfonamide;
6-chloro-N-[5-(3-fluoropropoxy)-4-methoxy-pyrimidin-2-yl]-1H-indole-3-sulfonamide;
N-(5-but-2-ynoxy-4-methoxy-pyrimidin-2-yl)-6-chloro-1H-indole-3-sulfonamide;
6-chloro-N-[5-(2,3-difluoropropyl)-4-methoxy-pyrimidin-2-yl]-1H-indole-3-sulfonamide;
6-chloro-N-[4-methoxy-5-(2,2,2-trifluoroethyl)pyrimidin-2-yl]-1H-indole-3-sulfonamide;
6-chloro-N-[4-methoxy-5-(3,3,3-trifluoropropyl)pyrimidin-2-yl]-1H-indole-3-sulfonamide;
6-chloro-N-[5-(2-cyanoethyl)-4-methoxy-pyrimidin-2-yl]-1H-indole-3-sulfonamide;
6-chloro-N-[4-methoxy-5-[2-(oxetan-3-yl)ethynyl]pyrimidin-2-yl]-1H-indole-3-sulfonamide;
6-chloro-N-[4-methoxy-5-[2-(oxetan-3-yl)ethyl]pyrimidin-2-yl]-1H-indole-3-sulfonamide;
N-[5-(2,2-difluoroethyl)-4-methoxy-pyrimidin-2-yl]-6-methyl-1H-indole-3-sulfonamide;
7-bromo-6-chloro-N-[5-(3,3-difluoropropyl)-4-methoxy-pyrimidin-2-yl]-1H-indole-3-sulfonamide;
6-bromo-N-[5-(2,2-difluoroethyl)-4-methoxy-pyrimidin-2-yl]-7-methyl-1H-indole-3-sulfonamide;
6-chloro-N-[5-(2,2-difluoroethyl)-4-methoxy-pyrimidin-2-yl]-7-methoxy-1H-indole-3-sulfonamide;
6-chloro-N-[5-(3,3-difluoropropyl)-4-methoxy-pyrimidin-2-yl]-7-methoxy-1H-indole-3-sulfonamide;
6-chloro-N-[5-(2,2-difluoroethyl)-4-methoxy-pyrimidin-2-yl]-7-(difluoromethoxy)-1H-indole-3-sulfonamide;
6-bromo-N-[5-(difluoromethoxy)-4-methoxy-pyrimidin-2-yl]-1H-indole-3-sulfonamide;
N-[5-(2,2-difluoroethyl)-4-methoxy-pyrimidin-2-yl]-6-(trifluoromethyl)-1H-indole-3-sulfonamide;
6-bromo-N-[5-(2,2-difluoroethyl)-4-methoxy-pyrimidin-2-yl]-7-fluoro-1H-indole-3-sulfonamide;
6-chloro-N-[5-(2,2-difluoroethyl)-4-methoxy-pyrimidin-2-yl]-7-methyl-1H-indole-3-sulfonamide;
N-[5-(2,2-difluoroethyl)-4-methoxy-pyrimidin-2-yl]-6-(methylthio)-1H-indole-3-sulfonamide;
N-[5-(2,2-difluoroethyl)-4,6-dimethoxy-pyrimidin-2-yl]-6-(difluoromethyl)-1H-pyrrolo[2,3-b]pyridine-3-sulfonamide;
N-[5-(2,2-difluoroethyl)-4-methoxy-pyrimidin-2-yl]-6-(difluoromethyl)-1H-indole-3-sulfonamide;
6-bromo-N-[5-(2,2-difluoroethyl)-4-methoxy-pyrimidin-2-yl]-7-methoxy-1H-indole-3-sulfonamide;
6-(cyanomethyl)-N-[5-(2,2-difluoroethyl)-4-methoxy-pyrimidin-2-yl]-1H-indole-3-sulfonamide;
6-chloro-N-[5-(2,2-difluoroethyl)-4-methoxy-pyrimidin-2-yl]-7-(fluoromethoxy)-1H-indole-3-sulfonamide;
6-chloro-N-[5-(2,2-difluoroethyl)-4-methoxy-pyrimidin-2-yl]-7-(difluoromethylsulfanyl)-1H-indole-3-sulfonamide;
6-bromo-N-[5-(2,2-difluoroethyl)-4-methoxy-pyrimidin-2-yl]-1H-pyrrolo[2,3-b]pyridine-3-sulfonamide;
6-bromo-N-[5-(3,3-difluoropropyl)-4-methoxy-pyrimidin-2-yl]-1H-pyrrolo[2,3-b]pyridine-3-sulfonamide;
6-bromo-N-[5-(2,2-difluoroethoxy)-4,6-dimethoxy-pyrimidin-2-yl]-1H-pyrrolo[2,3-b]pyridine-3-sulfonamide;
6-chloro-N-[5-(2,2-difluoroethyl)-4-methoxy-pyrimidin-2-yl]-1H-pyrrolo[2,3-b]pyridine-3-sulfonamide;
6-chloro-N-[5-(3,3-difluoropropyl)-4-methoxy-pyrimidin-2-yl]-1H-pyrrolo[2,3-b]pyridine-3-sulfonamide;
6-chloro-N-[5-(difluoromethoxy)-4,6-dimethoxy-pyrimidin-2-yl]-1H-pyrrolo[2,3-b]pyridine-3-sulfonamide;
6-chloro-N-[5-(2,2-difluoroethoxy)-4,6-dimethoxy-pyrimidin-2-yl]-1H-pyrrolo[2,3-b]pyridine-3-sulfonamide;
6-chloro-N-[5-(3,3-difluoropropyl)-4-methoxy-pyrimidin-2-yl]-7-fluoro-1H-indole-3-sulfonamide;
6-chloro-N-[5-(2,2-difluoroethoxy)-4,6-dimethoxy-pyrimidin-2-yl]-7-fluoro-1H-indole-3-sulfonamide;
6-chloro-7-(difluoromethyl)-N-[5-(3,3-difluoropropyl)-4-methoxy-pyrimidin-2-yl]-1H-indole-3-sulfonamide;
6,7-dichloro-N-[5-(2,2-difluoroethyl)-4-methoxy-pyrimidin-2-yl]-1H-indole-3-sulfonamide;
6,7-dichloro-N-[5-(3,3-difluoropropyl)-4-methoxy-pyrimidin-2-yl]-1H-indole-3-sulfonamide;
6-chloro-7-(cyclopropoxy)-N-[5-(3,3-difluoropropyl)-4-methoxy-pyrimidin-2-yl]-1H-indole-3-sulfonamide;
6-bromo-7-fluoro-N-[5-(2-fluoroethoxy)-4-methoxy-pyrimidin-2-yl]-1H-indole-3-sulfonamide;
6-chloro-N-[5-(2,2-difluoroethyl)-4-methoxy-pyrimidin-2-yl]-7-(difluoromethyl)-1H-indole-3-sulfonamide;
6-chloro-7-fluoro-N-[4-methoxy-5-(1,1,2-trifluoroethoxy)pyrimidin-2-yl]-1H-indole-3-sulfonamide;
6-chloro-N-[5-(2-cyanoethyl)-4-methoxy-pyrimidin-2-yl]-7-fluoro-1H-indole-3-sulfonamide;
6-chloro-N-[5-(2,2-difluoroethyl)-4,6-dimethoxy-pyrimidin-2-yl]-7-fluoro-1H-indole-3-sulfonamide;
6-chloro-N-[5-(2-cyanoethyl)-4,6-dimethoxy-pyrimidin-2-yl]-7-fluoro-1H-indole-3-sulfonamide;
6-chloro-N-[5-(cyanomethoxy)-4,6-dimethoxy-pyrimidin-2-yl]-7-fluoro-1H-indole-3-sulfonamide;
6-chloro-N-[4-methoxy-5-(1,1,2-trifluoroethoxy)pyrimidin-2-yl]-1H-pyrrolo[2,3-b]pyridine-3-sulfonamide;
6-chloro-N-[5-(2-fluoroethoxy)-4,6-dimethoxy-pyrimidin-2-yl]-1H-pyrrolo[2,3-b]pyridine-3-sulfonamide;
N-[5-(2,2-difluoroethyl)-4-methoxy-pyrimidin-2-yl]-1H-pyrrolo[3,2-h]quinoline-3-sulfonamide;
6-chloro-7-cyano-N-[5-(2,2-difluoroethyl)-4-methoxy-pyrimidin-2-yl]-1H-indole-3-sulfonamide;
6-chloro-7-cyano-N-[5-(difluoromethoxy)-4,6-dimethoxy-pyrimidin-2-yl]-1H-indole-3-sulfonamide;
6-chloro-7-cyano-N-[5-(2-fluoroethoxy)-4,6-dimethoxy-pyrimidin-2-yl]-1H-indole-3-sulfonamide;
6-chloro-7-cyano-N-[5-(2,2-difluoroethoxy)-4,6-dimethoxy-pyrimidin-2-yl]-1H-indole-3-sulfonamide;
6-chloro-7-cyano-N-[5-(2,2-difluoroethyl)-4,6-dimethoxy-pyrimidin-2-yl]-1H-indole-3-sulfonamide;
N-[5-(2,2-difluoroethyl)-4-methoxy-pyrimidin-2-yl]-6,8-dihydro-1H-fur[3,4-g]indole-3-sulfonamide;
N-[5-(2,2-difluoroethyl)-4,6-dimethoxy-pyrimidin-2-yl]-6,8-dihydro-1H-fur[3,4-g]indole-3-sulfonamide;
6-bromo-N-[5-(2-fluoroethoxy)-4-methoxy-pyrimidin-2-yl]-1H-pyrrolo[2,3-b]pyridine-3-sulfonamide;
6-bromo-N-[4-methoxy-5-(1,1,2-trifluoroethoxy)pyrimidin-2-yl]-1H-pyrrolo[2,3-b]pyridine-3-sulfonamide;
6-chloro-7-(cyanomethyl)-N-[5-(2,2-difluoroethyl)-4-methoxy-pyrimidin-2-yl]-1H-indole-3-sulfonamide;
6-chloro-7-cyclopropyl-N-[5-(2,2-difluoroethyl)-4-methoxy-pyrimidin-2-yl]-1H-indole-3-sulfonamide;
6-chloro-N-[5-(2,2-difluoroethyl)-4-methoxy-pyrimidin-2-yl]-7-(methylthio)-1H-indole-3-sulfonamide;
6-(difluoromethyl)-N-[5-(2-fluoroethoxy)-4-methoxy-pyrimidin-2-yl]-1H-indole-3-sulfonamide;
N-[5-(2,2-difluoroethyl)-4,6-dimethoxy-pyrimidin-2-yl]-6-(difluoromethyl)-1H-indole-3-sulfonamide;
N-[5-(cyanomethoxy)-4,6-dimethoxy-pyrimidin-2-yl]-6-(difluoromethyl)-1H-indole-3-sulfonamide;
6-(difluoromethyl)-N-[4-methoxy-5-(1,1,2-trifluoroethoxy)pyrimidin-2-yl]-1H-pyrrolo[2,3-b]pyridine-3-sulfonamide;
N-[5-(2-cyanoethyl)-4,6-dimethoxy-pyrimidin-2-yl]-6-(difluoromethyl)-1H-pyrrolo[2,3-b]pyridine-3-sulfonamide;
6-bromo-N-[5-(2-cyanoethyl)-4-methoxy-pyrimidin-2-yl]-7-fluoro-1H-indole-3-sulfonamide;
6-bromo-N-[5-(difluoromethoxy)-4,6-dimethoxy-pyrimidin-2-yl]-7-fluoro-1H-indole-3-sulfonamide;
6-bromo-N-[5-(2,2-difluoroethoxy)-4,6-dimethoxy-pyrimidin-2-yl]-7-fluoro-1H-indole-3-sulfonamide;
6-bromo-N-[5-(2-cyanoethyl)-4,6-dimethoxy-pyrimidin-2-yl]-7-fluoro-1H-indole-3-sulfonamide;
6-bromo-N-[5-(cyanomethoxy)-4,6-dimethoxy-pyrimidin-2-yl]-7-fluoro-1H-indole-3-sulfonamide;
6-bromo-N-[5-(2,2-difluoroethyl)-4,6-dimethoxy-pyrimidin-2-yl]-1H-indole-3-sulfonamide;
6-bromo-N-[5-(cyanomethoxy)-4,6-dimethoxy-pyrimidin-2-yl]-1H-indole-3-sulfonamide;
6-(difluoromethyl)-7-fluoro-N-[5-(2-fluoroethoxy)-4-methoxy-pyrimidin-2-yl]-1H-indole-3-sulfonamide;
N-[5-(2,2-difluoroethyl)-4-methoxy-pyrimidin-2-yl]-6-(difluoromethyl)-7-fluoro-1H-indole-3-sulfonamide;
N-[5-(difluoromethoxy)-4,6-dimethoxy-pyrimidin-2-yl]-6-(difluoromethyl)-7-fluoro-1H-indole-3-sulfonamide;
N-[5-(2,2-difluoroethoxy)-4,6-dimethoxy-pyrimidin-2-yl]-6-(difluoromethyl)-7-fluoro-1H-indole-3-sulfonamide;
N-[5-(2,2-difluoroethyl)-4,6-dimethoxy-pyrimidin-2-yl]-6-(difluoromethyl)-7-fluoro-1H-indole-3-sulfonamide;
N-[5-(2-cyanoethyl)-4,6-dimethoxy-pyrimidin-2-yl]-6-(difluoromethyl)-7-fluoro-1H-indole-3-sulfonamide;
N-[5-(cyanomethoxy)-4,6-dimethoxy-pyrimidin-2-yl]-6-(difluoromethyl)-7-fluoro-1H-indole-3-sulfonamide;
6-cyclopropyl-N-[5-(2,2-difluoroethyl)-4-methoxy-pyrimidin-2-yl]-1H-indole-3-sulfonamide;
7-bromo-6-chloro-N-[5-(2-cyanoethyl)-4-methoxy-pyrimidin-2-yl]-1H-indole-3-sulfonamide;
N-[5-(difluoromethoxy)-4,6-dimethoxy-pyrimidin-2-yl]-6-fluoro-1H-indole-3-sulfonamide;
N-[5-(2,2-difluoroethoxy)-4,6-dimethoxy-pyrimidin-2-yl]-6-fluoro-1H-indole-3-sulfonamide;
N-[5-(2,2-difluoroethyl)-4,6-dimethoxy-pyrimidin-2-yl]-6-fluoro-1H-indole-3-sulfonamide;
N-[5-(2-cyanoethyl)-4,6-dimethoxy-pyrimidin-2-yl]-6-fluoro-1H-indole-3-sulfonamide;
6-chloro-N-[5-(2,2-difluoropropyl)-4-methoxy-pyrimidin-2-yl]-1H-indole-3-sulfonamide;
6-chloro-N-[4-methoxy-5-(2,2,3,3-tetrafluoropropyl)pyrimidin-2-yl]-1H-indole-3-sulfonamide;
6-chloro-N-[5-(difluoromethoxymethyl)-4-methoxy-pyrimidin-2-yl]-1H-indole-3-sulfonamide;
6-chloro-N-[4,6-dimethoxy-5-(oxetan-3-ylmethoxy)pyrimidin-2-yl]-1H-indole-3-sulfonamide;
6-chloro-N-[5-(2,3-difluoropropyl)-4,6-dimethoxy-pyrimidin-2-yl]-1H-indole-3-sulfonamide;
6-chloro-N-[5-(cyanomethoxy)-4,6-dimethoxy-pyrimidin-2-yl]-1H-indole-3-sulfonamide;
6-chloro-N-[4-methoxy-5-(2,3,3-trifluoropropyl)pyrimidin-2-yl]-1H-indole-3-sulfonamide;
6-chloro-N-[5-(cyanomethoxy)-4-methoxy-pyrimidin-2-yl]-1H-indole-3-sulfonamide;
6-bromo-N-[5-(difluoromethoxy)-4-methoxy-pyrimidin-2-yl]benzothiophene-3-sulfonamide;
6-bromo-N-[5-(difluoromethoxy)-4,6-dimethoxy-pyrimidin-2-yl]thieno[2,3-b]pyridine-3-sulfonamide;
6-bromo-N-[5-(3,3-difluoropropyl)-4-methoxy-pyrimidin-2-yl]thieno[2,3-b]pyridine-3-sulfonamide;
6-chloro-N-[5-(difluoromethoxy)-4,6-dimethoxy-pyrimidin-2-yl]thieno[2,3-b]pyridine-3-sulfonamide;
N-[5-(2,2-difluoroethyl)-4-methoxy-pyrimidin-2-yl]-6-(oxetan-3-yl)-1H-indole-3-sulfonamide;
N-(4,6-dimethoxy-5-methyl-pyrimidin-2-yl)-6-methoxy-1H-indole-3-sulfonamide;
N-[5-(2,2-difluoroethyl)-4-methoxy-pyrimidin-2-yl]-6-(1-hydroxy-1-methyl-ethyl)-1H-indole-3-sulfonamide;
6-chloro-N-[5-(1,1-difluoro-2-hydroxy-ethoxy)-4,6-dimethoxy-pyrimidin-2-yl]-1H-indole-3-sulfonamide;
6-chloro-N-[5-(2,2-difluoroethyl)-4-methoxy-pyrimidin-2-yl]-7-methylsulfinyl-1H-indole-3-sulfonamide;
6-chloro-N-[5-(2,2-difluoroethyl)-4-methoxy-pyrimidin-2-yl]-7-mesyl-1H-indole-3-sulfonamide;
6-chloro-N-[5-(2-fluoroethoxy)-4,6-dimethoxy-pyrimidin-2-yl]-7-methylsulfinyl-1H-indole-3-sulfonamide;
6-chloro-N-[5-(2,2-difluoroethyl)-4,6-dimethoxy-pyrimidin-2-yl]-7-methylsulfinyl-1H-indole-3-sulfonamide;
6-chloro-7-cyano-N-[5-(2-fluoroethoxy)-4-methoxy-pyrimidin-2-yl]-1H-indole-3-sulfonamide;
6-chloro-N-[4,6-dimethoxy-5-(2-methylsulfonylethyl)pyrimidin-2-yl]-1H-indole-3-sulfonamide;
N-[5-(2,2-difluoroethyl)-4-methoxy-pyrimidin-2-yl]-1H-pyrrolo[2,3-e][1,2]benzothiazole-3-sulfonamide;
7-bromo-6-chloro-N-[5-(2,2-difluoroethyl)-4,6-dimethoxy-pyrimidin-2-yl]-1H-indole-3-sulfonamide;
N-[5-(cyanomethoxy)-4,6-dimethoxy-pyrimidin-2-yl]-6-methyl-1H-pyrrolo[2,3-b]pyridine-3-sulfonamide;
N-[5-(2,2-difluoroethoxy)-4,6-dimethoxy-pyrimidin-2-yl]-6-methyl-1H-pyrrolo[2,3-b]pyridine-3-sulfonamide;
N-[5-(difluoromethoxy)-4,6-dimethoxy-pyrimidin-2-yl]-6-methyl-1H-pyrrolo[2,3-b]pyridine-3-sulfonamide;
6-(difluoromethyl)-N-[5-(2,3-difluoropropyl)-4,6-dimethoxy-pyrimidin-2-yl]-1H-pyrrolo[2,3-b]pyridine-3-sulfonamide;
6-bromo-N-[5-(2,3-difluoropropyl)-4,6-dimethoxy-pyrimidin-2-yl]-1H-pyrrolo[2,3-b]pyridine-3-sulfonamide;
6-chloro-N-[5-(2,3-difluoropropyl)-4,6-dimethoxy-pyrimidin-2-yl]-1H-pyrrolo[2,3-b]pyridine-3-sulfonamide;
N-[5-(2,2-difluoroethyl)-4-methoxy-pyrimidin-2-yl]-8H-pyrrolo[2,3-e][1,3]benzothiazole-6-sulfonamide;
3-[[5-(2,2-difluoroethyl)-4-methoxy-pyrimidin-2-yl]sulfamoyl]-6-methyl-1H-indole-7-carboxylic acid methyl ester;
N-[5-(2,2-difluoroethyl)-4-methoxy-pyrimidin-2-yl]-6-methyl-7-methylol-1H-indole-3-sulfonamide;
N-[5-(2-fluoroethoxy)-4-methoxy-pyrimidin-2-yl]-9-keto-8-methyl-6,7-dihydro-1H-pyrrol[3,2-h]isoquinoline-3-sulfonamide;
6-chloro-7-(cyanomethoxy)-N-[5-(2,2-difluoroethyl)-4,6-dimethoxy-pyrimidin-2-yl]-1H-indole-3-sulfonamide;
and pharmaceutically acceptable salts thereof.

Further particular examples of formula I as described herein are selected from
6-chloro-N-[5-(2-fluoroethoxy)-4-methoxy-pyrimidin-2-yl]-1H-indole-3-sulfonamide;
6-chloro-7-fluoro-N-[5-(2-fluoroethoxy)-4-methoxy-pyrimidin-2-yl]-1H-indole-3-sulfonamide;
6,7-dichloro-N-[5-(2-fluoroethoxy)-4-methoxy-pyrimidin-2-yl]-1H-indole-3-sulfonamide;
6-chloro-7-(difluoromethyl)-N-[5-(2-fluoroethoxy)-4-methoxy-pyrimidin-2-yl]-1H-indole-3-sulfonamide;
6-chloro-N-[5-(2-fluoroethoxy)-4-methoxy-pyrimidin-2-yl]-1H-pyrrolo[2,3-b]pyridine-3-sulfonamide;
6-chloro-N-(5-chloro-4,6-dimethoxy-pyrimidin-2-yl)-1H-indole-3-sulfonamide;
N-(5-bromo-4,6-dimethoxy-pyrimidin-2-yl)-6-chloro-1H-indole-3-sulfonamide;
6-chloro-N-(5-ethyl-4,6-dimethoxy-pyrimidin-2-yl)-1H-indole-3-sulfonamide;
6-chloro-N-(4,6-dimethoxy-5-methyl-pyrimidin-2-yl)-1H-indole-3-sulfonamide;
N-[5-bromo-4-(difluoromethoxy)-6-methoxy-pyrimidin-2-yl]-6-chloro-1H-indole-3-sulfonamide;
6-chloro-N-[4-cyclopropyl-5-(2,2-difluoroethoxy)-6-methoxy-pyrimidin-2-yl]-1H-indole-3-sulfonamide;
6-chloro-N-[5-(3-fluoropropyl)-4-methoxy-pyrimidin-2-yl]-1H-indole-3-sulfonamide;
6-chloro-N-[5-(2-fluoroethoxy)-4-methoxy-pyrimidin-2-yl]benzothiophene-3-sulfonamide;
6-bromo-N-[5-(2-fluoroethoxy)-4-methoxy-pyrimidin-2-yl]-1H-indole-3-sulfonamide;
6-chloro-N-[5-(2-fluoroethoxy)-4-methoxy-pyrimidin-2-yl]thieno[2,3-b]pyridine-3-sulfonamide;
6-chloro-N-(4-methoxy-5-prop-1-ynyl-pyrimidin-2-yl)-1H-indole-3-sulfonamide;
6-bromo-N-(5-bromo-4,6-dimethoxy-pyrimidin-2-yl)-1H-pyrrolo[2,3-b]pyridine-3-sulfonamide;
N-(5-bromo-4,6-dimethoxy-pyrimidin-2-yl)-6-chloro-1H-pyrrolo[2,3-b]pyridine-3-sulfonamide;
and pharmaceutically acceptable salts thereof.

Also further particular examples of formula I as described herein are selected from
6-chloro-7-fluoro-N-[5-(2-fluoroethoxy)-4-methoxy-pyrimidin-2-yl]-1H-indole-3-sulfonamide;
6,7-dichloro-N-[5-(2-fluoroethoxy)-4-methoxy-pyrimidin-2-yl]-1H-indole-3-sulfonamide;
6-chloro-7-(difluoromethyl)-N-[5-(2-fluoroethoxy)-4-methoxy-pyrimidin-2-yl]-1H-indole-3-sulfonamide;
N-(5-bromo-4,6-dimethoxy-pyrimidin-2-yl)-6-chloro-1H-indole-3-sulfonamide;
6-chloro-N-(5-ethyl-4,6-dimethoxy-pyrimidin-2-yl)-1H-indole-3-sulfonamide;
6-chloro-N-(4,6-dimethoxy-5-methyl-pyrimidin-2-yl)-1H-indole-3-sulfonamide;
6-chloro-N-[4-cyclopropyl-5-(2,2-difluoroethoxy)-6-methoxy-pyrimidin-2-yl]-1H-indole-3-sulfonamide;
6-chloro-N-[5-(3-fluoropropyl)-4-methoxy-pyrimidin-2-yl]-1H-indole-3-sulfonamide;
6-bromo-N-[5-(2-fluoroethoxy)-4-methoxy-pyrimidin-2-yl]-1H-indole-3-sulfonamide;
6-bromo-N-(5-bromo-4,6-dimethoxy-pyrimidin-2-yl)-1H-pyrrolo[2,3-b]pyridine-3-sulfonamide;
6-chloro-N-[4-methoxy-5-(1,1,2-trifluoroethoxy)pyrimidin-2-yl]-1H-indole-3-sulfonamide;
6-chloro-N-[5-(2,2-difluoroethyl)-4-methoxy-pyrimidin-2-yl]-1H-indole-3-sulfonamide;
6-chloro-N-[5-(3,3-difluoropropyl)-4-methoxy-pyrimidin-2-yl]-1H-indole-3-sulfonamide;
6-chloro-N-[5-(difluoromethoxy)-4,6-dimethoxy-pyrimidin-2-yl]-1H-indole-3-sulfonamide;
6-chloro-N-[5-(2-fluoroethoxy)-4,6-dimethoxy-pyrimidin-2-yl]-1H-indole-3-sulfonamide;
6-chloro-N-[5-(2,2-difluoroethoxy)-4,6-dimethoxy-pyrimidin-2-yl]-1H-indole-3-sulfonamide;
6-chloro-N-[4,6-dimethoxy-5-(1,1,2-trifluoroethoxy)pyrimidin-2-yl]-1H-indole-3-sulfonamide;
N-(5-allyl-4,6-dimethoxy-pyrimidin-2-yl)-6-chloro-1H-indole-3-sulfonamide;
6-chloro-N-(4,6-dimethoxy-5-prop-1-ynyl-pyrimidin-2-yl)-1H-indole-3-sulfonamide;
6-chloro-N-(4,6-dimethoxy-5-propyl-pyrimidin-2-yl)-1H-indole-3-sulfonamide;
6-chloro-N-[5-(2-fluoroethyl)-4,6-dimethoxy-pyrimidin-2-yl]-1H-indole-3-sulfonamide;
6-chloro-N-[5-(2,2-difluoroethyl)-4,6-dimethoxy-pyrimidin-2-yl]-1H-indole-3-sulfonamide;
6-chloro-N-[5-(3-fluoropropyl)-4,6-dimethoxy-pyrimidin-2-yl]-1H-indole-3-sulfonamide;
6-chloro-N-[5-(3,3-difluoropropyl)-4,6-dimethoxy-pyrimidin-2-yl]-1H-indole-3-sulfonamide;
6-chloro-N-[4,6-dimethoxy-5-(3,3,3-trifluoropropyl)pyrimidin-2-yl]-1H-indole-3-sulfonamide;
6-chloro-N-[4,6-dimethoxy-5-(2-methoxyethyl)pyrimidin-2-yl]-1H-indole-3-sulfonamide;
6-chloro-N-[5-(2-cyanoethyl)-4,6-dimethoxy-pyrimidin-2-yl]-1H-indole-3-sulfonamide;
6-chloro-N-(5-cyclopropyl-4,6-dimethoxy-pyrimidin-2-yl)-1H-indole-3-sulfonamide;
6-chloro-N-[4-(difluoromethoxy)-6-methoxy-5-methyl-pyrimidin-2-yl]-1H-indole-3-sulfonamide;
6-bromo-N-[5-(2,2-difluoroethyl)-4-methoxy-pyrimidin-2-yl]-1H-indole-3-sulfonamide;
6-bromo-N-[5-(difluoromethoxy)-4,6-dimethoxy-pyrimidin-2-yl]-1H-indole-3-sulfonamide;
6-bromo-N-[5-(2-fluoroethoxy)-4,6-dimethoxy-pyrimidin-2-yl]-1H-indole-3-sulfonamide;
6-bromo-N-[5-(2,2-difluoroethoxy)-4,6-dimethoxy-pyrimidin-2-yl]-1H-indole-3-sulfonamide;
6-bromo-N-[5-(2-cyanoethyl)-4,6-dimethoxy-pyrimidin-2-yl]-1H-indole-3-sulfonamide;
N-[5-(2,2-difluoroethoxy)-4,6-dimethoxy-pyrimidin-2-yl]-6-(difluoromethyl)-1H-pyrrolo[2,3-b]pyridine-3-sulfonamide;
6-(difluoromethyl)-N-[4,6-dimethoxy-5-(1,1,2-trifluoroethoxy)pyrimidin-2-yl]-1H-pyrrolo[2,3-b]pyridine-3-sulfonamide;
6-(difluoromethyl)-N-[5-(3,3-difluoropropyl)-4,6-dimethoxy-pyrimidin-2-yl]-1H-pyrrolo[2,3-b]pyridine-3-sulfonamide;
N-[5-(difluoromethoxy)-4,6-dimethoxy-pyrimidin-2-yl]-6-(difluoromethyl)-1H-indole-3-sulfonamide;
N-[5-(2,2-difluoroethoxy)-4,6-dimethoxy-pyrimidin-2-yl]-6-(difluoromethyl)-1H-indole-3-sulfonamide;
N-[5-(2-cyanoethyl)-4,6-dimethoxy-pyrimidin-2-yl]-6-(difluoromethyl)-1H-indole-3-sulfonamide;
6-chloro-N-[5-(2-cyanoethyl)-4-methoxy-pyrimidin-2-yl]-7-(difluoromethylthio)-1H-indole-3-sulfonamide;
6-chloro-7-(difluoromethylthio)-N-[5-(2-fluoroethoxy)-4,6-dimethoxy-pyrimidin-2-yl]-1H-indole-3-sulfonamide;
6-chloro-N-[5-(2-cyanoethyl)-4,6-dimethoxy-pyrimidin-2-yl]-7-(difluoromethylthio)-1H-indole-3-sulfonamide;
6-bromo-N-[5-(2-fluoroethoxy)-4,6-dimethoxy-pyrimidin-2-yl]-1H-pyrrolo[2,3-b]pyridine-3-sulfonamide;
6-bromo-N-[4,6-dimethoxy-5-(1,1,2-trifluoroethoxy)pyrimidin-2-yl]-1H-pyrrolo[2,3-b]pyridine-3-sulfonamide;
6-bromo-N-[5-(2,2-difluoroethyl)-4,6-dimethoxy-pyrimidin-2-yl]-1H-pyrrolo[2,3-b]pyridine-3-sulfonamide;
6-bromo-N-[5-(3,3-difluoropropyl)-4,6-dimethoxy-pyrimidin-2-yl]-1H-pyrrolo[2,3-b]pyridine-3-sulfonamide;
6-bromo-N-[5-(2-cyanoethyl)-4,6-dimethoxy-pyrimidin-2-yl]-1H-pyrrolo[2,3-b]pyridine-3-sulfonamide;
6-bromo-N-[5-(cyanomethoxy)-4,6-dimethoxy-pyrimidin-2-yl]-1H-pyrrolo[2,3-b]pyridine-3-sulfonamide;
6-chloro-N-[5-(2,2-difluoroethyl)-4,6-dimethoxy-pyrimidin-2-yl]-7-(dimethylamino)-1H-indole-3-sulfonamide;
N-[5-(difluoromethoxy)-4,6-dimethoxy-pyrimidin-2-yl]-1H-pyrrolo[3,2-h]quinoline-3-sulfonamide;
N-[5-(2-fluoroethoxy)-4,6-dimethoxy-pyrimidin-2-yl]-1H-pyrrolo[3,2-h]quinoline-3-sulfonamide;
6-chloro-N-(4,6-dimethoxy-5-methyl-pyrimidin-2-yl)-1H-pyrrolo[2,3-b]pyridine-3-sulfonamide;
6-chloro-N-[5-(2,2-difluoroethyl)-4,6-dimethoxy-pyrimidin-2-yl]-1H-pyrrolo[2,3-b]pyridine-3-sulfonamide;
6-chloro-N-[5-(3,3-difluoropropyl)-4,6-dimethoxy-pyrimidin-2-yl]-1H-pyrrolo[2,3-b]pyridine-3-sulfonamide;
6-chloro-N-[5-(2-cyanoethyl)-4,6-dimethoxy-pyrimidin-2-yl]-1H-pyrrolo[2,3-b]pyridine-3-sulfonamide;
6-chloro-N-[5-(2,2-difluoroethyl)-4-methoxy-pyrimidin-2-yl]-7-fluoro-1H-indole-3-sulfonamide;
6-chloro-N-[5-(difluoromethoxy)-4,6-dimethoxy-pyrimidin-2-yl]-7-fluoro-1H-indole-3-sulfonamide;
6-chloro-7-(cyclopropoxy)-N-[5-(2,2-difluoroethyl)-4-methoxy-pyrimidin-2-yl]-1H-indole-3-sulfonamide;
N-[5-(2,2-difluoroethyl)-4,6-dimethoxy-pyrimidin-2-yl]-6-methyl-1H-pyrrolo[2,3-b]pyridine-3-sulfonamide;
N-[5-(2,2-difluoroethyl)-4-methoxy-pyrimidin-2-yl]-6-fluoro-1H-indole-3-sulfonamide;
7-bromo-6-chloro-N-[5-(2-fluoroethoxy)-4-methoxy-pyrimidin-2-yl]-1H-indole-3-sulfonamide;
7-bromo-6-chloro-N-[5-(2,2-difluoroethyl)-4-methoxy-pyrimidin-2-yl]-1H-indole-3-sulfonamide;
6-bromo-N-(4,6-dimethoxy-5-methyl-pyrimidin-2-yl)thieno[2,3-b]pyridine-3-sulfonamide;
6-bromo-N-[5-(2,2-difluoroethyl)-4-methoxy-pyrimidin-2-yl]thieno[2,3-b]pyridine-3-sulfonamide;
6-bromo-N-[5-(2,2-difluoroethyl)-4,6-dimethoxy-pyrimidin-2-yl]thieno[2,3-b]pyridine-3-sulfonamide;
6-bromo-N-[5-(2-cyanoethyl)-4,6-dimethoxy-pyrimidin-2-yl]thieno[2,3-b]pyridine-3-sulfonamide;
6-chloro-N-[5-(2,2-difluoroethyl)-4-methoxy-pyrimidin-2-yl]benzothiophene-3-sulfonamide;
6-bromo-N-[5-(2-fluoroethoxy)-4-methoxy-pyrimidin-2-yl]benzothiophene-3-sulfonamide;
6-bromo-N-(4,6-dimethoxy-5-methyl-pyrimidin-2-yl)-1H-pyrrolo[2,3-b]pyridine-3-sulfonamide;
N-[5-(2,2-difluoroethoxy)-4,6-dimethoxy-pyrimidin-2-yl]-1H-pyrrolo[3,2-h]quinoline-3-sulfonamide;
N-[5-(2,2-difluoroethyl)-4,6-dimethoxy-pyrimidin-2-yl]-1H-pyrrolo[3,2-h]quinoline-3-sulfonamide;
6-bromo-N-[5-(difluoromethoxy)-4,6-dimethoxy-pyrimidin-2-yl]-1H-pyrrolo[2,3-b]pyridine-3-sulfonamide;
6-chloro-N-[5-(2,3-difluoropropyl)-4-methoxy-pyrimidin-2-yl]-1H-indole-3-sulfonamide;
6-chloro-N-[5-(2-cyanoethyl)-4-methoxy-pyrimidin-2-yl]-1H-indole-3-sulfonamide;
7-bromo-6-chloro-N-[5-(3,3-difluoropropyl)-4-methoxy-pyrimidin-2-yl]-1H-indole-3-sulfonamide;
6-bromo-N-[5-(2,2-difluoroethyl)-4-methoxy-pyrimidin-2-yl]-7-methyl-1H-indole-3-sulfonamide;
6-chloro-N-[5-(2,2-difluoroethyl)-4-methoxy-pyrimidin-2-yl]-7-methoxy-1H-indole-3-sulfonamide;
6-chloro-N-[5-(3,3-difluoropropyl)-4-methoxy-pyrimidin-2-yl]-7-methoxy-1H-indole-3-sulfonamide;
6-chloro-N-[5-(2,2-difluoroethyl)-4-methoxy-pyrimidin-2-yl]-7-(difluoromethoxy)-1H-indole-3-sulfonamide;
6-bromo-N-[5-(2,2-difluoroethyl)-4-methoxy-pyrimidin-2-yl]-7-fluoro-1H-indole-3-sulfonamide;
6-chloro-N-[5-(2,2-difluoroethyl)-4-methoxy-pyrimidin-2-yl]-7-methyl-1H-indole-3-sulfonamide;
N-[5-(2,2-difluoroethyl)-4,6-dimethoxy-pyrimidin-2-yl]-6-(difluoromethyl)-1H-pyrrolo[2,3-b]pyridine-3-sulfonamide;
6-bromo-N-[5-(2,2-difluoroethyl)-4-methoxy-pyrimidin-2-yl]-7-methoxy-1H-indole-3-sulfonamide;
6-chloro-N-[5-(2,2-difluoroethyl)-4-methoxy-pyrimidin-2-yl]-7-(fluoromethoxy)-1H-indole-3-sulfonamide;
6-chloro-N-[5-(2,2-difluoroethyl)-4-methoxy-pyrimidin-2-yl]-7-(difluoromethylsulfanyl)-1H-indole-3-sulfonamide;
6-bromo-N-[5-(3,3-difluoropropyl)-4-methoxy-pyrimidin-2-yl]-1H-pyrrolo[2,3-b]pyridine-3-sulfonamide;
6-bromo-N-[5-(2,2-difluoroethoxy)-4,6-dimethoxy-pyrimidin-2-yl]-1H-pyrrolo[2,3-b]pyridine-3-sulfonamide;
6-chloro-N-[5-(3,3-difluoropropyl)-4-methoxy-pyrimidin-2-yl]-1H-pyrrolo[2,3-b]pyridine-3-sulfonamide;
6-chloro-N-[5-(difluoromethoxy)-4,6-dimethoxy-pyrimidin-2-yl]-1H-pyrrolo[2,3-b]pyridine-3-sulfonamide;
6-chloro-N-[5-(3,3-difluoropropyl)-4-methoxy-pyrimidin-2-yl]-7-fluoro-1H-indole-3-sulfonamide;
6-chloro-N-[5-(2,2-difluoroethoxy)-4,6-dimethoxy-pyrimidin-2-yl]-7-fluoro-1H-indole-3-sulfonamide;
6,7-dichloro-N-[5-(2,2-difluoroethyl)-4-methoxy-pyrimidin-2-yl]-1H-indole-3-sulfonamide;
6,7-dichloro-N-[5-(3,3-difluoropropyl)-4-methoxy-pyrimidin-2-yl]-1H-indole-3-sulfonamide;
6-chloro-7-(cyclopropoxy)-N-[5-(3,3-difluoropropyl)-4-methoxy-pyrimidin-2-yl]-1H-indole-3-sulfonamide;
6-bromo-7-fluoro-N-[5-(2-fluoroethoxy)-4-methoxy-pyrimidin-2-yl]-1H-indole-3-sulfonamide;
6-chloro-N-[5-(2,2-difluoroethyl)-4-methoxy-pyrimidin-2-yl]-7-(difluoromethyl)-1H-indole-3-sulfonamide;
6-chloro-7-fluoro-N-[4-methoxy-5-(1,1,2-trifluoroethoxy)pyrimidin-2-yl]-1H-indole-3-sulfonamide;
6-chloro-N-[5-(2-cyanoethyl)-4-methoxy-pyrimidin-2-yl]-7-fluoro-1H-indole-3-sulfonamide;
6-chloro-N-[5-(2,2-difluoroethyl)-4,6-dimethoxy-pyrimidin-2-yl]-7-fluoro-1H-indole-3-sulfonamide;
6-chloro-N-[5-(2-cyanoethyl)-4,6-dimethoxy-pyrimidin-2-yl]-7-fluoro-1H-indole-3-sulfonamide;
6-chloro-N-[5-(cyanomethoxy)-4,6-dimethoxy-pyrimidin-2-yl]-7-fluoro-1H-indole-3-sulfonamide;
6-chloro-N-[4-methoxy-5-(1,1,2-trifluoroethoxy)pyrimidin-2-yl]-1H-pyrrolo[2,3-b]pyridine-3-sulfonamide;
6-chloro-N-[5-(2-fluoroethoxy)-4,6-dimethoxy-pyrimidin-2-yl]-1H-pyrrolo[2,3-b]pyridine-3-sulfonamide;
6-chloro-7-cyano-N-[5-(2,2-difluoroethyl)-4-methoxy-pyrimidin-2-yl]-1H-indole-3-sulfonamide;
6-chloro-7-cyano-N-[5-(difluoromethoxy)-4,6-dimethoxy-pyrimidin-2-yl]-1H-indole-3-sulfonamide;
6-chloro-7-cyano-N-[5-(2-fluoroethoxy)-4,6-dimethoxy-pyrimidin-2-yl]-1H-indole-3-sulfonamide;
6-chloro-7-cyano-N-[5-(2,2-difluoroethoxy)-4,6-dimethoxy-pyrimidin-2-yl]-1H-indole-3-sulfonamide;
6-chloro-7-cyano-N-[5-(2,2-difluoroethyl)-4,6-dimethoxy-pyrimidin-2-yl]-1H-indole-3-sulfonamide;
N-[5-(2,2-difluoroethyl)-4,6-dimethoxy-pyrimidin-2-yl]-6,8-dihydro-1H-fur[3,4-g]indole-3-sulfonamide;
6-bromo-N-[5-(2-fluoroethoxy)-4-methoxy-pyrimidin-2-yl]-1H-pyrrolo[2,3-b]pyridine-3-sulfonamide;
6-bromo-N-[4-methoxy-5-(1,1,2-trifluoroethoxy)pyrimidin-2-yl]-1H-pyrrolo[2,3-b]pyridine-3-sulfonamide;
6-chloro-7-(cyanomethyl)-N-[5-(2,2-difluoroethyl)-4-methoxy-pyrimidin-2-yl]-1H-indole-3-sulfonamide;
6-chloro-N-[5-(2,2-difluoroethyl)-4-methoxy-pyrimidin-2-yl]-7-(methylthio)-1H-indole-3-sulfonamide;
6-(difluoromethyl)-N-[5-(2-fluoroethoxy)-4-methoxy-pyrimidin-2-yl]-1H-indole-3-sulfonamide;
N-[5-(2,2-difluoroethyl)-4,6-dimethoxy-pyrimidin-2-yl]-6-(difluoromethyl)-1H-indole-3-sulfonamide;
N-[5-(cyanomethoxy)-4,6-dimethoxy-pyrimidin-2-yl]-6-(difluoromethyl)-1H-indole-3-sulfonamide;
N-[5-(2-cyanoethyl)-4,6-dimethoxy-pyrimidin-2-yl]-6-(difluoromethyl)-1H-pyrrolo[2,3-b]pyridine-3-sulfonamide;
6-bromo-N-[5-(2-cyanoethyl)-4-methoxy-pyrimidin-2-yl]-7-fluoro-1H-indole-3-sulfonamide;
6-bromo-N-[5-(difluoromethoxy)-4,6-dimethoxy-pyrimidin-2-yl]-7-fluoro-1H-indole-3-sulfonamide;
6-bromo-N-[5-(2,2-difluoroethoxy)-4,6-dimethoxy-pyrimidin-2-yl]-7-fluoro-1H-indole-3-sulfonamide;
6-bromo-N-[5-(2-cyanoethyl)-4,6-dimethoxy-pyrimidin-2-yl]-7-fluoro-1H-indole-3-sulfonamide;
6-bromo-N-[5-(cyanomethoxy)-4,6-dimethoxy-pyrimidin-2-yl]-7-fluoro-1H-indole-3-sulfonamide;
6-bromo-N-[5-(2,2-difluoroethyl)-4,6-dimethoxy-pyrimidin-2-yl]-1H-indole-3-sulfonamide;
6-bromo-N-[5-(cyanomethoxy)-4,6-dimethoxy-pyrimidin-2-yl]-1H-indole-3-sulfonamide;
6-(difluoromethyl)-7-fluoro-N-[5-(2-fluoroethoxy)-4-methoxy-pyrimidin-2-yl]-1H-indole-3-sulfonamide;
N-[5-(difluoromethoxy)-4,6-dimethoxy-pyrimidin-2-yl]-6-(difluoromethyl)-7-fluoro-1H-indole-3-sulfonamide;
N-[5-(2,2-difluoroethoxy)-4,6-dimethoxy-pyrimidin-2-yl]-6-(difluoromethyl)-7-fluoro-1H-indole-3-sulfonamide;
N-[5-(2,2-difluoroethyl)-4,6-dimethoxy-pyrimidin-2-yl]-6-(difluoromethyl)-7-fluoro-1H-indole-3-sulfonamide;
N-[5-(2-cyanoethyl)-4,6-dimethoxy-pyrimidin-2-yl]-6-(difluoromethyl)-7-fluoro-1H-indole-3-sulfonamide;
N-[5-(cyanomethoxy)-4,6-dimethoxy-pyrimidin-2-yl]-6-(difluoromethyl)-7-fluoro-1H-indole-3-sulfonamide;
7-bromo-6-chloro-N-[5-(2-cyanoethyl)-4-methoxy-pyrimidin-2-yl]-1H-indole-3-sulfonamide;
N-[5-(2,2-difluoroethyl)-4,6-dimethoxy-pyrimidin-2-yl]-6-fluoro-1H-indole-3-sulfonamide;
N-[5-(2-cyanoethyl)-4,6-dimethoxy-pyrimidin-2-yl]-6-fluoro-1H-indole-3-sulfonamide;
6-chloro-N-[5-(difluoromethoxymethyl)-4-methoxy-pyrimidin-2-yl]-1H-indole-3-sulfonamide;
6-chloro-N-[5-(2,3-difluoropropyl)-4,6-dimethoxy-pyrimidin-2-yl]-1H-indole-3-sulfonamide;
6-chloro-N-[5-(cyanomethoxy)-4,6-dimethoxy-pyrimidin-2-yl]-1H-indole-3-sulfonamide;
6-chloro-N-[5-(cyanomethoxy)-4-methoxy-pyrimidin-2-yl]-1H-indole-3-sulfonamide;
7-bromo-6-chloro-N-[5-(2,2-difluoroethyl)-4,6-dimethoxy-pyrimidin-2-yl]-1H-indole-3-sulfonamide;
6-chloro-7-(cyanomethoxy)-N-[5-(2,2-difluoroethyl)-4,6-dimethoxy-pyrimidin-2-yl]-1H-indole-3-sulfonamide;
and pharmaceutically acceptable salts thereof.

Also further particular examples of formula I as described herein are selected from
6-chloro-N-[4-methoxy-5-(1,1,2-trifluoroethoxy)pyrimidin-2-yl]-1H-indole-3-sulfonamide;
6-chloro-N-[5-(2,2-difluoroethyl)-4-methoxy-pyrimidin-2-yl]-1H-indole-3-sulfonamide;
6-chloro-N-[5-(3,3-difluoropropyl)-4-methoxy-pyrimidin-2-yl]-1H-indole-3-sulfonamide;
6-chloro-N-[5-(difluoromethoxy)-4,6-dimethoxy-pyrimidin-2-yl]-1H-indole-3-sulfonamide;
6-chloro-N-[5-(2-fluoroethoxy)-4,6-dimethoxy-pyrimidin-2-yl]-1H-indole-3-sulfonamide;
6-chloro-N-[5-(2,2-difluoroethoxy)-4,6-dimethoxy-pyrimidin-2-yl]-1H-indole-3-sulfonamide;
6-chloro-N-[4,6-dimethoxy-5-(1,1,2-trifluoroethoxy)pyrimidin-2-yl]-1H-indole-3-sulfonamide;
N-(5-allyl-4,6-dimethoxy-pyrimidin-2-yl)-6-chloro-1H-indole-3-sulfonamide;
6-chloro-N-(4,6-dimethoxy-5-prop-1-ynyl-pyrimidin-2-yl)-1H-indole-3-sulfonamide;
6-chloro-N-(4,6-dimethoxy-5-propyl-pyrimidin-2-yl)-1H-indole-3-sulfonamide;
6-chloro-N-[5-(2-fluoroethyl)-4,6-dimethoxy-pyrimidin-2-yl]-1H-indole-3-sulfonamide;
6-chloro-N-[5-(2,2-difluoroethyl)-4,6-dimethoxy-pyrimidin-2-yl]-1H-indole-3-sulfonamide;
6-chloro-N-[5-(3-fluoropropyl)-4,6-dimethoxy-pyrimidin-2-yl]-1H-indole-3-sulfonamide;
6-chloro-N-[5-(3,3-difluoropropyl)-4,6-dimethoxy-pyrimidin-2-yl]-1H-indole-3-sulfonamide;
6-chloro-N-[4,6-dimethoxy-5-(3,3,3-trifluoropropyl)pyrimidin-2-yl]-1H-indole-3-sulfonamide;
6-chloro-N-[4,6-dimethoxy-5-(2-methoxyethyl)pyrimidin-2-yl]-1H-indole-3-sulfonamide;
6-chloro-N-[5-(2-cyanoethyl)-4,6-dimethoxy-pyrimidin-2-yl]-1H-indole-3-sulfonamide;
6-chloro-N-(5-cyclopropyl-4,6-dimethoxy-pyrimidin-2-yl)-1H-indole-3-sulfonamide;
6-chloro-N-[4-(difluoromethoxy)-6-methoxy-5-methyl-pyrimidin-2-yl]-1H-indole-3-sulfonamide;
6-bromo-N-[5-(2,2-difluoroethyl)-4-methoxy-pyrimidin-2-yl]-1H-indole-3-sulfonamide;
6-bromo-N-[5-(difluoromethoxy)-4,6-dimethoxy-pyrimidin-2-yl]-1H-indole-3-sulfonamide;
6-bromo-N-[5-(2-fluoroethoxy)-4,6-dimethoxy-pyrimidin-2-yl]-1H-indole-3-sulfonamide;
6-bromo-N-[5-(2,2-difluoroethoxy)-4,6-dimethoxy-pyrimidin-2-yl]-1H-indole-3-sulfonamide;
6-bromo-N-[5-(2-cyanoethyl)-4,6-dimethoxy-pyrimidin-2-yl]-1H-indole-3-sulfonamide;
N-[5-(2,2-difluoroethoxy)-4,6-dimethoxy-pyrimidin-2-yl]-6-(difluoromethyl)-1H-pyrrolo[2,3-b]pyridine-3-sulfonamide;
6-(difluoromethyl)-N-[4,6-dimethoxy-5-(1,1,2-trifluoroethoxy)pyrimidin-2-yl]-1H-pyrrolo[2,3-b]pyridine-3-sulfonamide;
6-(difluoromethyl)-N-[5-(3,3-difluoropropyl)-4,6-dimethoxy-pyrimidin-2-yl]-1H-pyrrolo[2,3-b]pyridine-3-sulfonamide;
N-[5-(difluoromethoxy)-4,6-dimethoxy-pyrimidin-2-yl]-6-(difluoromethyl)-1H-indole-3-sulfonamide;
N-[5-(2,2-difluoroethoxy)-4,6-dimethoxy-pyrimidin-2-yl]-6-(difluoromethyl)-1H-indole-3-sulfonamide;
N-[5-(2-cyanoethyl)-4,6-dimethoxy-pyrimidin-2-yl]-6-(difluoromethyl)-1H-indole-3-sulfonamide;
6-chloro-N-[5-(2-cyanoethyl)-4-methoxy-pyrimidin-2-yl]-7-(difluoromethylthio)-1H-indole-3-sulfonamide;
6-chloro-7-(difluoromethylthio)-N-[5-(2-fluoroethoxy)-4,6-dimethoxy-pyrimidin-2-yl]-1H-indole-3-sulfonamide;
6-chloro-N-[5-(2-cyanoethyl)-4,6-dimethoxy-pyrimidin-2-yl]-7-(difluoromethylthio)-1H-indole-3-sulfonamide;
6-bromo-N-[5-(2-fluoroethoxy)-4,6-dimethoxy-pyrimidin-2-yl]-1H-pyrrolo[2,3-b]pyridine-3-sulfonamide;
6-bromo-N-[4,6-dimethoxy-5-(1,1,2-trifluoroethoxy)pyrimidin-2-yl]-1H-pyrrolo[2,3-b]pyridine-3-sulfonamide;
6-bromo-N-[5-(2,2-difluoroethyl)-4,6-dimethoxy-pyrimidin-2-yl]-1H-pyrrolo[2,3-b]pyridine-3-sulfonamide;
6-bromo-N-[5-(3,3-difluoropropyl)-4,6-dimethoxy-pyrimidin-2-yl]-1H-pyrrolo[2,3-b]pyridine-3-sulfonamide;
6-bromo-N-[5-(2-cyanoethyl)-4,6-dimethoxy-pyrimidin-2-yl]-1H-pyrrolo[2,3-b]pyridine-3-sulfonamide;
6-bromo-N-[5-(cyanomethoxy)-4,6-dimethoxy-pyrimidin-2-yl]-1H-pyrrolo[2,3-b]pyridine-3-sulfonamide;
6-chloro-N-[5-(2,2-difluoroethyl)-4,6-dimethoxy-pyrimidin-2-yl]-7-(dimethylamino)-1H-indole-3-sulfonamide;
N-[5-(difluoromethoxy)-4,6-dimethoxy-pyrimidin-2-yl]-1H-pyrrolo[3,2-h]quinoline-3-sulfonamide;
N-[5-(2-fluoroethoxy)-4,6-dimethoxy-pyrimidin-2-yl]-1H-pyrrolo[3,2-h]quinoline-3-sulfonamide;
6-chloro-N-(4,6-dimethoxy-5-methyl-pyrimidin-2-yl)-1H-pyrrolo[2,3-b]pyridine-3-sulfonamide;
6-chloro-N-[5-(2,2-difluoroethyl)-4,6-dimethoxy-pyrimidin-2-yl]-1H-pyrrolo[2,3-b]pyridine-3-sulfonamide;
6-chloro-N-[5-(3,3-difluoropropyl)-4,6-dimethoxy-pyrimidin-2-yl]-1H-pyrrolo[2,3-b]pyridine-3-sulfonamide;
6-chloro-N-[5-(2-cyanoethyl)-4,6-dimethoxy-pyrimidin-2-yl]-1H-pyrrolo[2,3-b]pyridine-3-sulfonamide;
6-chloro-N-[5-(2,2-difluoroethyl)-4-methoxy-pyrimidin-2-yl]-7-fluoro-1H-indole-3-sulfonamide;
6-chloro-N-[5-(difluoromethoxy)-4,6-dimethoxy-pyrimidin-2-yl]-7-fluoro-1H-indole-3-sulfonamide;
6-chloro-7-(cyclopropoxy)-N-[5-(2,2-difluoroethyl)-4-methoxy-pyrimidin-2-yl]-1H-indole-3-sulfonamide;
N-[5-(2,2-difluoroethyl)-4,6-dimethoxy-pyrimidin-2-yl]-6-methyl-1H-pyrrolo[2,3-b]pyridine-3-sulfonamide;
N-[5-(2,2-difluoroethyl)-4-methoxy-pyrimidin-2-yl]-6-fluoro-1H-indole-3-sulfonamide;
7-bromo-6-chloro-N-[5-(2-fluoroethoxy)-4-methoxy-pyrimidin-2-yl]-1H-indole-3-sulfonamide;
7-bromo-6-chloro-N-[5-(2,2-difluoroethyl)-4-methoxy-pyrimidin-2-yl]-1H-indole-3-sulfonamide;
6-bromo-N-(4,6-dimethoxy-5-methyl-pyrimidin-2-yl)thieno[2,3-b]pyridine-3-sulfonamide;
6-bromo-N-[5-(2,2-difluoroethyl)-4-methoxy-pyrimidin-2-yl]thieno[2,3-b]pyridine-3-sulfonamide;
6-bromo-N-[5-(2,2-difluoroethyl)-4,6-dimethoxy-pyrimidin-2-yl]thieno[2,3-b]pyridine-3-sulfonamide;
6-bromo-N-[5-(2-cyanoethyl)-4,6-dimethoxy-pyrimidin-2-yl]thieno[2,3-b]pyridine-3-sulfonamide;
6-chloro-N-[5-(2,2-difluoroethyl)-4-methoxy-pyrimidin-2-yl]benzothiophene-3-sulfonamide;
6-bromo-N-[5-(2-fluoroethoxy)-4-methoxy-pyrimidin-2-yl]benzothiophene-3-sulfonamide;
6-bromo-N-(4,6-dimethoxy-5-methyl-pyrimidin-2-yl)-1H-pyrrolo[2,3-b]pyridine-3-sulfonamide;
N-[5-(2,2-difluoroethoxy)-4,6-dimethoxy-pyrimidin-2-yl]-1H-pyrrolo[3,2-h]quinoline-3-sulfonamide;
N-[5-(2,2-difluoroethyl)-4,6-dimethoxy-pyrimidin-2-yl]-1H-pyrrolo[3,2-h]quinoline-3-sulfonamide;
6-bromo-N-[5-(difluoromethoxy)-4,6-dimethoxy-pyrimidin-2-yl]-1H-pyrrolo[2,3-b]pyridine-3-sulfonamide;
6-chloro-N-[5-(2,3-difluoropropyl)-4-methoxy-pyrimidin-2-yl]-1H-indole-3-sulfonamide;
6-chloro-N-[5-(2-cyanoethyl)-4-methoxy-pyrimidin-2-yl]-1H-indole-3-sulfonamide;
7-bromo-6-chloro-N-[5-(3,3-difluoropropyl)-4-methoxy-pyrimidin-2-yl]-1H-indole-3-sulfonamide;
6-bromo-N-[5-(2,2-difluoroethyl)-4-methoxy-pyrimidin-2-yl]-7-methyl-1H-indole-3-sulfonamide;
6-chloro-N-[5-(2,2-difluoroethyl)-4-methoxy-pyrimidin-2-yl]-7-methoxy-1H-indole-3-sulfonamide;
6-chloro-N-[5-(3,3-difluoropropyl)-4-methoxy-pyrimidin-2-yl]-7-methoxy-1H-indole-3-sulfonamide;
6-chloro-N-[5-(2,2-difluoroethyl)-4-methoxy-pyrimidin-2-yl]-7-(difluoromethoxy)-1H-indole-3-sulfonamide;
6-bromo-N-[5-(2,2-difluoroethyl)-4-methoxy-pyrimidin-2-yl]-7-fluoro-1H-indole-3-sulfonamide;
6-chloro-N-[5-(2,2-difluoroethyl)-4-methoxy-pyrimidin-2-yl]-7-methyl-1H-indole-3-sulfonamide;
N-[5-(2,2-difluoroethyl)-4,6-dimethoxy-pyrimidin-2-yl]-6-(difluoromethyl)-1H-pyrrolo[2,3-b]pyridine-3-sulfonamide;
6-bromo-N-[5-(2,2-difluoroethyl)-4-methoxy-pyrimidin-2-yl]-7-methoxy-1H-indole-3-sulfonamide;
6-chloro-N-[5-(2,2-difluoroethyl)-4-methoxy-pyrimidin-2-yl]-7-(fluoromethoxy)-1H-indole-3-sulfonamide;
6-chloro-N-[5-(2,2-difluoroethyl)-4-methoxy-pyrimidin-2-yl]-7-(difluoromethylsulfanyl)-1H-indole-3-sulfonamide;
6-bromo-N-[5-(3,3-difluoropropyl)-4-methoxy-pyrimidin-2-yl]-1H-pyrrolo[2,3-b]pyridine-3-sulfonamide;
6-bromo-N-[5-(2,2-difluoroethoxy)-4,6-dimethoxy-pyrimidin-2-yl]-1H-pyrrolo[2,3-b]pyridine-3-sulfonamide;
6-chloro-N-[5-(3,3-difluoropropyl)-4-methoxy-pyrimidin-2-yl]-1H-pyrrolo[2,3-b]pyridine-3-sulfonamide;
6-chloro-N-[5-(difluoromethoxy)-4,6-dimethoxy-pyrimidin-2-yl]-1H-pyrrolo[2,3-b]pyridine-3-sulfonamide;
6-chloro-N-[5-(3,3-difluoropropyl)-4-methoxy-pyrimidin-2-yl]-7-fluoro-1H-indole-3-sulfonamide;
6-chloro-N-[5-(2,2-difluoroethoxy)-4,6-dimethoxy-pyrimidin-2-yl]-7-fluoro-1H-indole-3-sulfonamide;
6,7-dichloro-N-[5-(2,2-difluoroethyl)-4-methoxy-pyrimidin-2-yl]-1H-indole-3-sulfonamide;
6,7-dichloro-N-[5-(3,3-difluoropropyl)-4-methoxy-pyrimidin-2-yl]-1H-indole-3-sulfonamide;
6-chloro-7-(cyclopropoxy)-N-[5-(3,3-difluoropropyl)-4-methoxy-pyrimidin-2-yl]-1H-indole-3-sulfonamide;
6-bromo-7-fluoro-N-[5-(2-fluoroethoxy)-4-methoxy-pyrimidin-2-yl]-1H-indole-3-sulfonamide;
6-chloro-N-[5-(2,2-difluoroethyl)-4-methoxy-pyrimidin-2-yl]-7-(difluoromethyl)-1H-indole-3-sulfonamide;
6-chloro-7-fluoro-N-[4-methoxy-5-(1,1,2-trifluoroethoxy)pyrimidin-2-yl]-1H-indole-3-sulfonamide;
6-chloro-N-[5-(2-cyanoethyl)-4-methoxy-pyrimidin-2-yl]-7-fluoro-1H-indole-3-sulfonamide;
6-chloro-N-[5-(2,2-difluoroethyl)-4,6-dimethoxy-pyrimidin-2-yl]-7-fluoro-1H-indole-3-sulfonamide;
6-chloro-N-[5-(2-cyanoethyl)-4,6-dimethoxy-pyrimidin-2-yl]-7-fluoro-1H-indole-3-sulfonamide;
6-chloro-N-[5-(cyanomethoxy)-4,6-dimethoxy-pyrimidin-2-yl]-7-fluoro-1H-indole-3-sulfonamide;
6-chloro-N-[4-methoxy-5-(1,1,2-trifluoroethoxy)pyrimidin-2-yl]-1H-pyrrolo[2,3-b]pyridine-3-sulfonamide;
6-chloro-N-[5-(2-fluoroethoxy)-4,6-dimethoxy-pyrimidin-2-yl]-1H-pyrrolo[2,3-b]pyridine-3-sulfonamide;
6-chloro-7-cyano-N-[5-(2,2-difluoroethyl)-4-methoxy-pyrimidin-2-yl]-1H-indole-3-sulfonamide;
6-chloro-7-cyano-N-[5-(difluoromethoxy)-4,6-dimethoxy-pyrimidin-2-yl]-1H-indole-3-sulfonamide;
6-chloro-7-cyano-N-[5-(2-fluoroethoxy)-4,6-dimethoxy-pyrimidin-2-yl]-1H-indole-3-sulfonamide;
6-chloro-7-cyano-N-[5-(2,2-difluoroethoxy)-4,6-dimethoxy-pyrimidin-2-yl]-1H-indole-3-sulfonamide;
6-chloro-7-cyano-N-[5-(2,2-difluoroethyl)-4,6-dimethoxy-pyrimidin-2-yl]-1H-indole-3-sulfonamide;
N-[5-(2,2-difluoroethyl)-4,6-dimethoxy-pyrimidin-2-yl]-6,8-dihydro-1H-fur[3,4-g]indole-3-sulfonamide;
6-bromo-N-[5-(2-fluoroethoxy)-4-methoxy-pyrimidin-2-yl]-1H-pyrrolo[2,3-b]pyridine-3-sulfonamide;
6-bromo-N-[4-methoxy-5-(1,1,2-trifluoroethoxy)pyrimidin-2-yl]-1H-pyrrolo[2,3-b]pyridine-3-sulfonamide;
6-chloro-7-(cyanomethyl)-N-[5-(2,2-difluoroethyl)-4-methoxy-pyrimidin-2-yl]-1H-indole-3-sulfonamide;
6-chloro-N-[5-(2,2-difluoroethyl)-4-methoxy-pyrimidin-2-yl]-7-(methylthio)-1H-indole-3-sulfonamide;
6-(difluoromethyl)-N-[5-(2-fluoroethoxy)-4-methoxy-pyrimidin-2-yl]-1H-indole-3-sulfonamide;
N-[5-(2,2-difluoroethyl)-4,6-dimethoxy-pyrimidin-2-yl]-6-(difluoromethyl)-1H-indole-3-sulfonamide;
N-[5-(cyanomethoxy)-4,6-dimethoxy-pyrimidin-2-yl]-6-(difluoromethyl)-1H-indole-3-sulfonamide;
N-[5-(2-cyanoethyl)-4,6-dimethoxy-pyrimidin-2-yl]-6-(difluoromethyl)-1H-pyrrolo[2,3-b]pyridine-3-sulfonamide;
6-bromo-N-[5-(2-cyanoethyl)-4-methoxy-pyrimidin-2-yl]-7-fluoro-1H-indole-3-sulfonamide;
6-bromo-N-[5-(difluoromethoxy)-4,6-dimethoxy-pyrimidin-2-yl]-7-fluoro-1H-indole-3-sulfonamide;
6-bromo-N-[5-(2,2-difluoroethoxy)-4,6-dimethoxy-pyrimidin-2-yl]-7-fluoro-1H-indole-3-sulfonamide;
6-bromo-N-[5-(2-cyanoethyl)-4,6-dimethoxy-pyrimidin-2-yl]-7-fluoro-1H-indole-3-sulfonamide;
6-bromo-N-[5-(cyanomethoxy)-4,6-dimethoxy-pyrimidin-2-yl]-7-fluoro-1H-indole-3-sulfonamide;
6-bromo-N-[5-(2,2-difluoroethyl)-4,6-dimethoxy-pyrimidin-2-yl]-1H-indole-3-sulfonamide;
6-bromo-N-[5-(cyanomethoxy)-4,6-dimethoxy-pyrimidin-2-yl]-1H-indole-3-sulfonamide;
6-(difluoromethyl)-7-fluoro-N-[5-(2-fluoroethoxy)-4-methoxy-pyrimidin-2-yl]-1H-indole-3-sulfonamide;
N-[5-(difluoromethoxy)-4,6-dimethoxy-pyrimidin-2-yl]-6-(difluoromethyl)-7-fluoro-1H-indole-3-sulfonamide;
N-[5-(2,2-difluoroethoxy)-4,6-dimethoxy-pyrimidin-2-yl]-6-(difluoromethyl)-7-fluoro-1H-indole-3-sulfonamide;
N-[5-(2,2-difluoroethyl)-4,6-dimethoxy-pyrimidin-2-yl]-6-(difluoromethyl)-7-fluoro-1H-indole-3-sulfonamide;
N-[5-(2-cyanoethyl)-4,6-dimethoxy-pyrimidin-2-yl]-6-(difluoromethyl)-7-fluoro-1H-indole-3-sulfonamide;
N-[5-(cyanomethoxy)-4,6-dimethoxy-pyrimidin-2-yl]-6-(difluoromethyl)-7-fluoro-1H-indole-3-sulfonamide;
7-bromo-6-chloro-N-[5-(2-cyanoethyl)-4-methoxy-pyrimidin-2-yl]-1H-indole-3-sulfonamide;
N-[5-(2,2-difluoroethyl)-4,6-dimethoxy-pyrimidin-2-yl]-6-fluoro-1H-indole-3-sulfonamide;
N-[5-(2-cyanoethyl)-4,6-dimethoxy-pyrimidin-2-yl]-6-fluoro-1H-indole-3-sulfonamide;
6-chloro-N-[5-(difluoromethoxymethyl)-4-methoxy-pyrimidin-2-yl]-1H-indole-3-sulfonamide;
6-chloro-N-[5-(2,3-difluoropropyl)-4,6-dimethoxy-pyrimidin-2-yl]-1H-indole-3-sulfonamide;
6-chloro-N-[5-(cyanomethoxy)-4,6-dimethoxy-pyrimidin-2-yl]-1H-indole-3-sulfonamide;
6-chloro-N-[5-(cyanomethoxy)-4-methoxy-pyrimidin-2-yl]-1H-indole-3-sulfonamide;
7-bromo-6-chloro-N-[5-(2,2-difluoroethyl)-4,6-dimethoxy-pyrimidin-2-yl]-1H-indole-3-sulfonamide;
6-chloro-7-(cyanomethoxy)-N-[5-(2,2-difluoroethyl)-4,6-dimethoxy-pyrimidin-2-yl]-1H-indole-3-sulfonamide
and pharmaceutically acceptable salts thereof.

Processes for the manufacture of compounds of formula I as described herein are an object of the invention.

The present compounds of formula I and their pharmaceutically acceptable salts can be prepared by methods known in the art, for example, by processes described below, which process comprises
a)reacting a compound of formula II 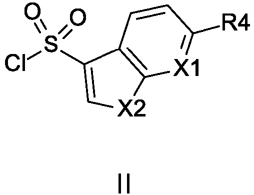
with a compound of formula III

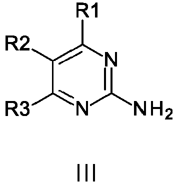

in the presence of a base selected from N,N-diisopropylethylamine or pyridine to provide a compound of formula I

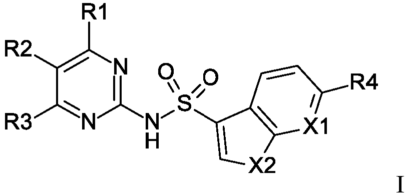

Wherein the substituents R¹, R², R³, R⁴ and X₁ and X₂ are as defined above, or
a) by reacting a compound of formula IV 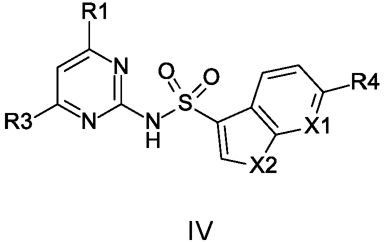
   with an halogenating agent like N-bromosuccinimide
   to provide a compound of formula I 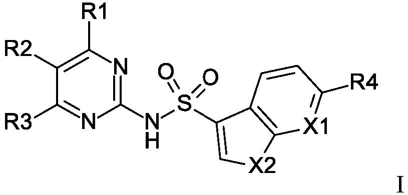
   wherein R² is a halogen like Br and the other substituents are as defined above.
b) By reacting a compound of formula I 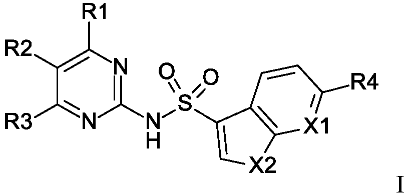
   wherein R² is a halogen with an cyanating agent like zinc cyanide in the presence of palladium catalyst like tetrakis(triphenylphosphine)palladium to provide a compound of formula I 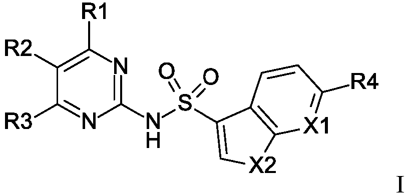
   wherein R² is a cyano group and the other substituents are as defined above.

### General Synthetic Schemes

The compounds of formula I may be prepared in accordance with the process variant described above and with the following scheme 1. The starting materials are commercially available or may be prepared in accordance with known methods.

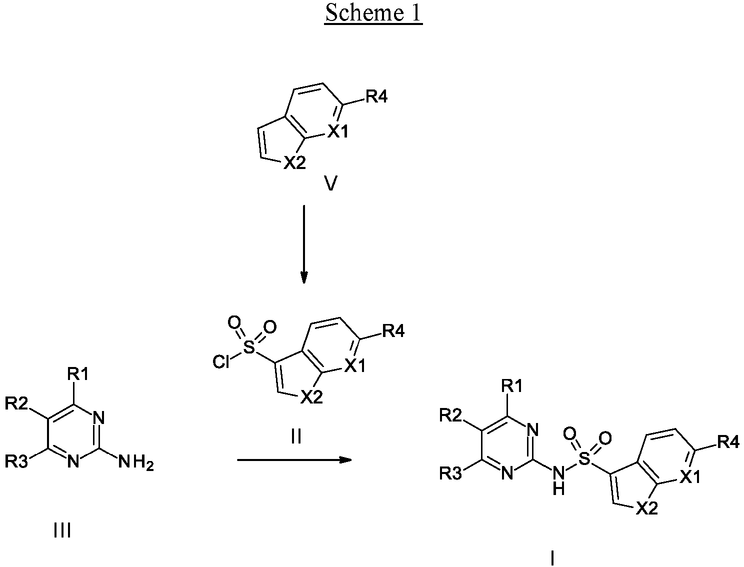

Compounds of general formula I can be prepared by reacting sulfonylchloride II with 2-amino-pyrimidine III in the presence of a base like N,N-diisopropylethylamine, pyridine, potassium phosphate or sodium hydride. II can be prepared from intermediate V in the presence of chlorosulfonylating agent like chlorosulfonic acid, or in the presence of sulfonylating agent like sulfur trioxide N,N-dimethylformamide complex, followed by chlorination of the intermediate sulfonic acid with a chlorinating agent like thionylchloride.

2-Amino-pyrimidines of formula III are commercially available or may be prepared in accordance with known methods or may be prepared in accordance with the process variant described in the following scheme 2-7. The starting materials are commercially available or may be prepared in accordance with known methods.

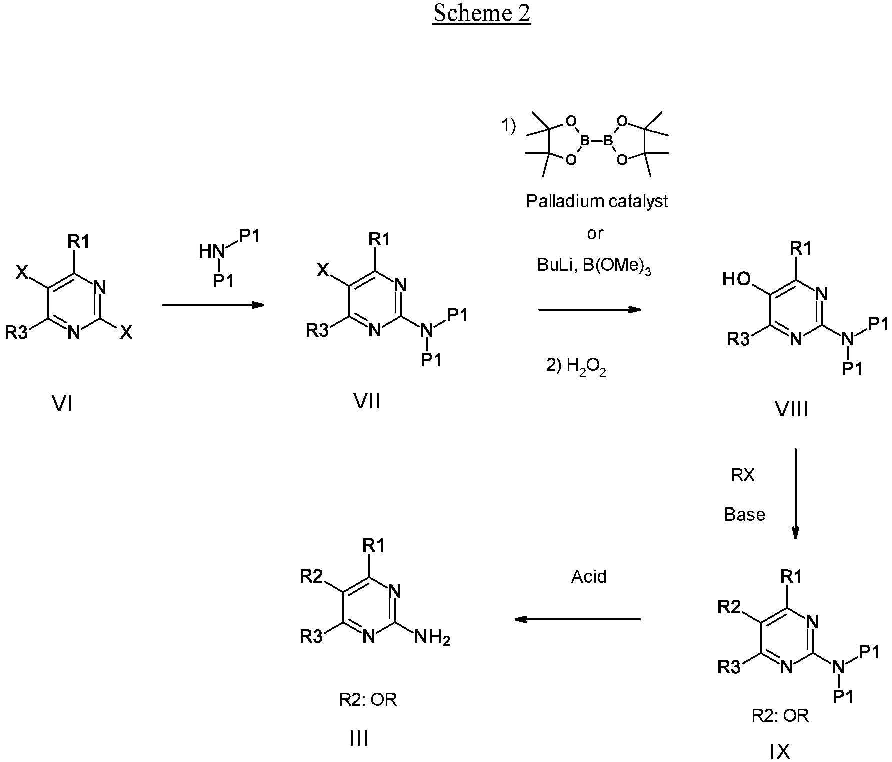

2-Amino-pyrimidine of formula III wherein R² is an alkoxy group can be prepared by deprotection of intermediate IX in the presence of an acid like trifluoro acetic acid wherein P1 is a protective group like p-methoxy-benzyl, 3-4-dimethoxybenzyl or a Boc group. IX can be obtained by alkylation of alcohol VIII in the presence of a base like cesium- or potassium-carbonate or sodium- or potassium-hydroxide and an alkylating agent RX. Alcohol VIII can be prepared from dihalogenated starting material VI, by reacting VI with a protected amine to provide monohalogenated intermediate VII which is first transformed into a boronic ester that is then oxidized in the presence of an oxidant like hydrogen peroxide.

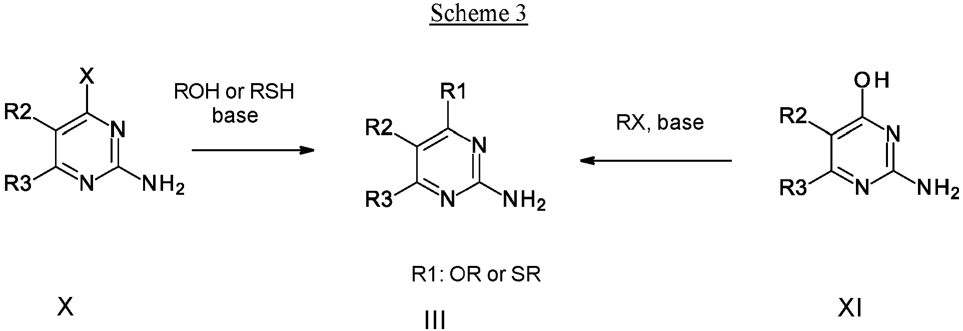

2-Amino-pyrimidine of formula III wherein R¹ is an alkoxy group or a thiolalkyl group can be prepared by reaction of halogenated starting material X in the presence of an alcohol or a thiol and a base like sodium hydride or alternatively III wherein R¹ is an alkoxy group can be prepared by alkylation of alcohol XI.

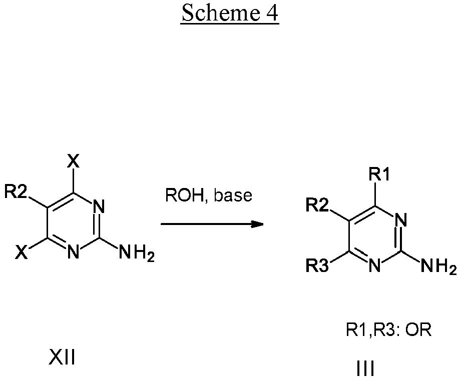

2-Amino-pyrimidine of formula III wherein R¹ and R³ are alkoxy groups can be prepared by reaction of di-halogenated starting material XII in the presence of an alcohol and a base like sodium hydride.

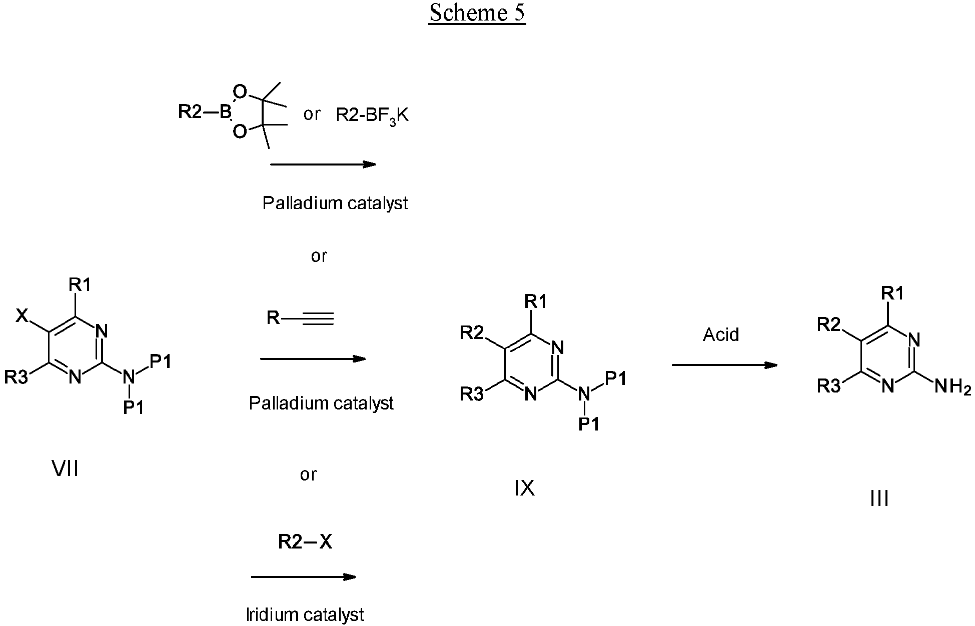

2-Amino-pyrimidine of formula III wherein R² is an alkyl, alkenylalkyl, alkynyl, cyanoalkyl, cycloalkyl, heterocycloalkyl can be prepared by deprotection of intermediate IX in the presence of an acid like trifluoro acetic acid wherein P1 is a protective group like p-methoxy-benzyl or a Boc group. IX can be obtained from monohalogenated intermediate VII under well known metal-catalyzed cross coupling reactions conditions.

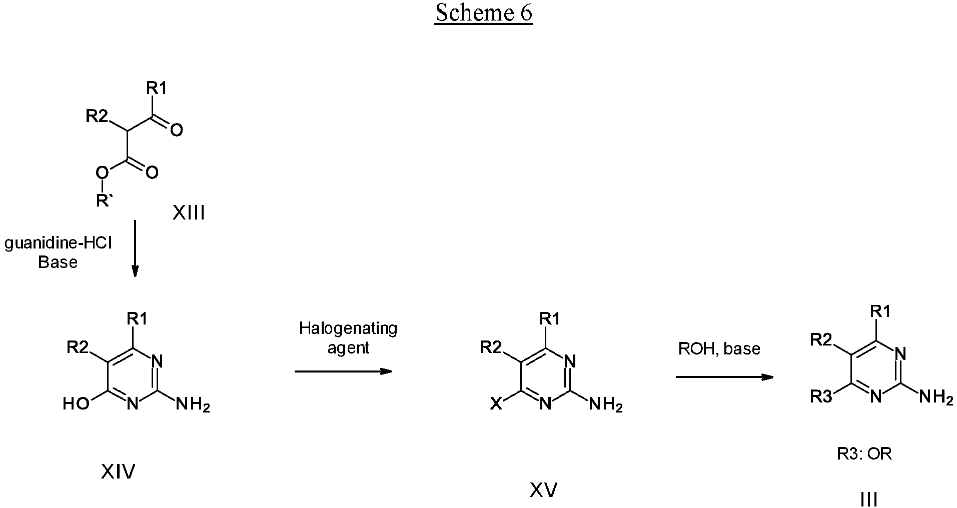

2-Amino-pyrimidine of formula III wherein R³ is an alkoxy group can be prepared by reaction of halogenated starting material XV in the presence of an alcohol and a base like sodium hydride. XV can be prepared from keto-ester XIII by its reaction with guanidine-hydrochloride salt in the presence of a base like sodium methoxide to provide alcohol intermediate XIV which is then reacted with an halogenating agent like phosphorus oxychloride.

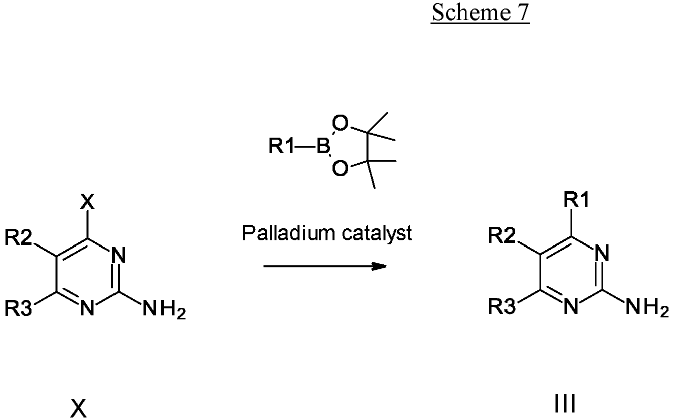

2-Amino-pyrimidine of formula III wherein R¹ is alkyl, cycloalkyl, heterocycloalkyl group can be prepared by reaction of halogenated starting material X under Suzuki reaction conditions in the presence of a boronic-ester and a palladium catalyst.

Intermediate of formula V are commercially available or may be prepared in accordance with known methods or in accordance with the process variant described in the following scheme 8-9. The starting materials are commercially available or may be prepared in accordance with known methods.

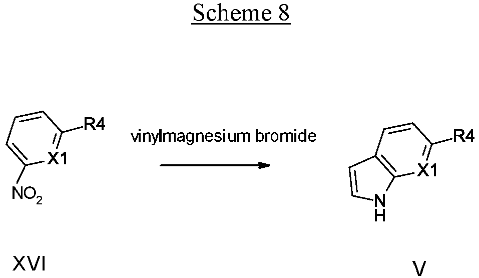

Intermediate of formula V wherein X2 is NH can be prepared by reaction of a nitro starting material XVI under Bartoli reaction conditions, in the presence of vinylmagnesium bromide.

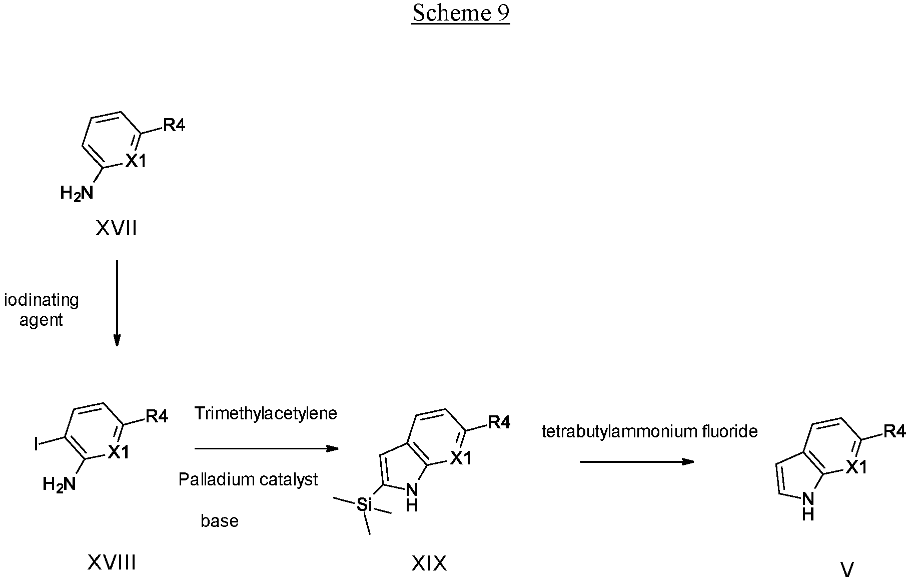

Intermediate of formula V wherein X2 is NH can be prepared following Larock synthesis route starting from amine XVII which is transformed into iodo intermediate XVIII in the presence of a iodinating agent like N-iodosuccinimide. XVIII is then tranformed into trimethylsilylated intermediate XIX in the presence of trimethyl silyl acetylene, a palladium catalyst like tetrakis(triphenylphosphine)palladium and a base like potassium carbonate. XIX is reacted with tetrabutylammonium fluoride to provide intermediate of formula V.

The invention thus relates to a compound according to the invention when manufactured according to a process of the invention.

An embodiment of the present invention is a process to prepare a compound of formula I as defined above comprising the reaction of a compound of II 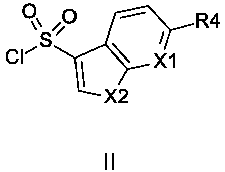
with a compound of formula III 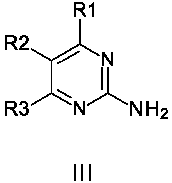
in the presence of a base selected from N,N-diisopropylethylamine or pyridine, wherein R¹, R², R³, R⁴, X¹ and X² are as defined above.

Another embodiment of the invention provides a pharmaceutical composition or medicament containing a compound of the invention and a therapeutically inert carrier, diluent or excipient, as well as a method of using the compounds of the invention to prepare such composition and medicament. In one example, the compound of formula I may be formulated by mixing at ambient temperature at the appropriate pH, and at the desired degree of purity, with physiologically acceptable carriers, i.e., carriers that are non-toxic to recipients at the dosages and concentrations employed into a galenical administration form. The pH of the formulation depends mainly on the particular use and the concentration of compound, but preferably ranges anywhere from about 3 to about 8. In one example, a compound of formula I is formulated in an acetate buffer, at pH 5. In another embodiment, the compound of formula I is sterile. The compound may be stored, for example, as a solid or amorphous composition, as a lyophilized formulation or as an aqueous solution.

Compositions are formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners.

The compounds of the invention may be administered by any suitable means, including oral, topical (including buccal and sublingual), rectal, vaginal, transdermal, parenteral, subcutaneous, intraperitoneal, intrapulmonary, intradermal, intrathecal and epidural and intranasal, and, if desired for local treatment, intralesional administration. Parenteral infusions include intramuscular, intravenous, intraarterial, intraperitoneal, or subcutaneous administration.

The compounds of the present invention may be administered in any convenient administrative form, e.g., tablets, powders, capsules, solutions, dispersions, suspensions, syrups, sprays, suppositories, gels, emulsions, patches, etc. Such compositions may contain components conventional in pharmaceutical preparations, e.g., diluents, carriers, pH modifiers, sweeteners, bulking agents, and further active agents.

A typical formulation is prepared by mixing a compound of the present invention and a carrier or excipient. Suitable carriers and excipients are well known to those skilled in the art and are described in detail in, e.g., Ansel, Howard C., et al., Ansel's Pharmaceutical Dosage Forms and Drug Delivery Systems. Philadelphia: Lippincott, Williams & Wilkins, 2004; Gennaro, Alfonso R., et al. Remington: The Science and Practice of Pharmacy. Philadelphia: Lippincott, Williams & Wilkins, 2000; and Rowe, Raymond C. Handbook of Pharmaceutical Excipients. Chicago, Pharmaceutical Press, 2005. The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents, diluents and other known additives to provide an elegant presentation of the drug (i.e., a compound of the present invention or pharmaceutical composition thereof) or aid in the manufacturing of the pharmaceutical product (i.e., medicament).

The compounds of formula I and their pharmaceutically acceptable salts can be processed with pharmaceutically inert, inorganic or organic adjuvants for the production of tablets, coated tablets, dragées,hard gelatin capsules, injection solutions or topical formulations Lactose, corn starch or derivatives thereof, talc, stearic acid or its salts etc. can be used, for example, as such adjuvants for tablets, dragées and hard gelatin capsules.

Suitable adjuvants for soft gelatin capsules, are, for example, vegetable oils, waxes, fats, semi-solid substances and liquid polyols, etc.

Suitable adjuvants for the production of solutions and syrups are, for example, water, polyols, saccharose, invert sugar, glucose, etc.

Suitable adjuvants for injection solutions are, for example, water, alcohols, polyols, glycerol, vegetable oils, etc.

Suitable adjuvants for suppositories are, for example, natural or hardened oils, waxes, fats, semi-solid or liquid polyols, etc.

Suitable adjuvants for topical ocular formulations are, for example, cyclodextrins, mannitol or many other carriers and excipients known in the art.

Moreover, the pharmaceutical preparations can contain preservatives, solubilizers, viscosity-increasing substances, stabilizers, wetting agents, emulsifiers, sweeteners, colorants, flavorants, salts for varying the osmotic pressure, buffers, masking agents or antioxidants. They can also contain still other therapeutically valuable substances.

The dosage can vary in wide limits and will, of course, be fitted to the individual requirements in each particular case. In general, in the case of oral administration a daily dosage of about 0.1 mg to 20 mg per kg body weight, preferably about 0.5 mg to 4 mg per kg body weight (e.g. about 300 mg per person), divided into preferably 1-3 individual doses, which can consist, for example, of the same amounts, should it be appropriate. In the case of topical administration, the formulation can contain 0.001% to 15% by weight of medicament and the required dose, which can be between 0.1 and 25 mg in can be administered either by single dose per day or per week, or by multiple doses (2 to 4) per day, or by multiple doses per week It will, however, be clear that the upper or lower limit given herein can be exceeded when this is shown to be indicated.

The references to methods of treatment in the subsequent paragraphs of this description are to be interpreted as references to the compounds, pharmaceutical compositions and medicaments of the present invention for use in a method for treatment of the human (or animal) body by therapy (or for diagnosis). The invention also relates in particular to:
A compound of formula I for use as therapeutically active substance;
A compound of formula I for use in the treatment of a disease modulated by GPR17;
Likewise an object of the present invention is a pharmaceutical composition comprising a compound according to formula I as described herein and a therapeutically inert carrier.

The use of a compound of formula I for the treatment or prophylaxis of conditions resulting from direct damage to myelin sheaths (including but not limited central pontine and extra-pontine myelinolysis, carbon monoxide poisoning, nutritional deficiency, and virus-induced demyelination), demyelinating disorders (including but not limited to multiple sclerosis, acute and multiphasic disseminated encephalomyelitis, neuromyelitis optica spectrum disorders, and leukodystrophies), CNS disorders associated with myelin loss (including but not limited to Alzheimer's disease, schizophrenia, Parkinson's disease, Huntington's disease, Amyotrophic lateral sclerosis, and Ischemia due to stroke), and Inflammation in the CNS for instance following encephalitis, primary angiitis, meningitis and obesity.

An embodiment of the present invention is the use of a compound of formula I for the treatment or prophylaxis of multiple sclerosis, Alzheimer's disease, Parkinson's disease, or Huntington's disease.

A particular embodiment of the invention is the use of a compound of formula I for the treatment or prophylaxis of multiple sclerosis.

The use of a compound of formula I for the preparation of a medicament for the treatment or prophylaxis of conditions resulting from direct damage to myelin sheaths (including but not limited central pontine and extra-pontine myelinolysis, carbon monoxide poisoning, nutritional deficiency, and virus-induced demyelination), demyelinating disorders (including but not limited to multiple sclerosis, acute and multiphasic disseminated encephalomyelitis, neuromyelitis optica spectrum disorders, and leukodystrophies), CNS disorders associated with myelin loss (including but not limited to Alzheimer's disease, schizophrenia, Parkinson's disease, Huntington's disease, Amyotrophic lateral sclerosis, and Ischemia due to stroke), and Inflammation in the CNS for instance following encephalitis, primary angiitis, meningitis and obesity.

An embodiment of the present invention is the use of a compound of formula I for the preparation of a medicament for the treatment or prophylaxis of multiple sclerosis, Alzheimer's disease, Parkinson's disease, or Huntington's disease.

A particular embodiment of the invention is the use of a compound of formula I for the preparation of a medicament for the treatment or prophylaxis of multiple sclerosis.

A compound according to formula I for use in the treatment or prophylaxis of conditions resulting from direct damage to myelin sheaths (including but not limited central pontine and extra-pontine myelinolysis, carbon monoxide poisoning, nutritional deficiency, and virus-induced demyelination), demyelinating disorders (including but not limited to multiple sclerosis, acute and multiphasic disseminated encephalomyelitis, neuromyelitis optica spectrum disorders, and leukodystrophies), CNS disorders associated with myelin loss (including but not limited to Alzheimer's disease, schizophrenia, Parkinson's disease, Huntington's disease, Amyotrophic lateral sclerosis, and Ischemia due to stroke), and Inflammation in the CNS for instance following encephalitis, primary angiitis, meningitis and obesity.

An embodiment of the present invention is a compound of formula I for use in the treatment or prophylaxis of multiple sclerosis, Alzheimer's disease, Parkinson's disease, or Huntington's disease.

A particular embodiment of the invention is a compound according to formula I for use in the treatment or prophylaxis of multiple sclerosis.

A method for the treatment or propylaxis of conditions resulting from direct damage to myelin sheaths (including but not limited central pontine and extra-pontine myelinolysis, carbon monoxide poisoning, nutritional deficiency, and virus-induced demyelination), demyelinating disorders (including but not limited to multiple sclerosis, acute and multiphasic disseminated encephalomyelitis, neuromyelitis optica spectrum disorders, and leukodystrophies), CNS disorders associated with myelin loss (including but not limited to Alzheimer's disease, schizophrenia, Parkinson's disease, Huntington's disease, Amyotrophic lateral sclerosis, and Ischemia due to stroke), and Inflammation in the CNS for instance following encephalitis, primary angiitis, meningitis and obesity, which method comprises administering an effective amount of a compound of formula I to a patient in need thereof.

An embodiment of the present invention is a method for the treatment or prophylaxis of multiple sclerosis, Alzheimer's disease, Parkinson's disease, or Huntington's disease, which method comprises administering an effective amount of a compound of formula I to a patient in need thereof.

A particular embodiment of the invention is a method for the treatment or prophylaxis of multiple sclerosis, which method comprises administering an effective amount of a compound of formula I to a patient in need thereof.

Also an embodiment of the present invention provides compounds of formula I as described herein, when manufactured according to any one of the described processes.

### Assay Procedures

### GPR17 cAMP Assay Protocol:

CHO-K1 cells stably expressing vector containing untagged human GPR17 short isoform (Roche) were cultured at 37°C / 5% CO2 in DMEM (Dulbecco's Modified Eagle Medium):F-12 (1:1) supplemented with 10% foetal bovine serum and 400 µg/ml Geneticin.

Changes in intracellular cyclic adenosine monophosphate (cAMP) levels were quantified using the Nano-TRF Detection Assay kit (Roche Diagnostics, Cat. No. 05214386001). This assay allows for direct cAMP quantification in a homogeneous solution. cAMP is detected based on time-resolved fluorescence energy transfer (TR-FRET) and competitive binding of ruthenylated cAMP and endogenous cAMP to an anti-cAMP monoclonal antibody labeled with AlexaFluor-700. The Ruthenium complex serves as the FRET donor and transfers energy to AlexaFluor-700. The FRET signal is inversely proportional to the cAMP concentration.

CHO-GPR17S cells were detached with Accutase and resuspended in assay buffer consisting of Hank's Balanced Salt Solution (HBSS), 10mM HEPES (4-(2-hydroxyethyl) piperazine-1-ethanesulfonic acid solution) and 0.1% bovine serum albumin (pH 7.4). The cells were seeded in black 384-well plates (Corning) at a density of 10'000 cells / 20µl assay buffer until the addition of compounds.

Test antagonist compounds were serially diluted in dimethyl sulfoxide (DMSO) and spotted in 384-well plates. The compounds were then diluted in HBSS buffer supplemented with an EC80 concentration of MDL29,951 (3-(2-Carboxy-4,6-dichloroindol-3-yl)propionic acid) (GPR17 agonist) plus 3-Isobutyl-1-methylxanthine (IBMX) (0.5mM final concentration) and added to the cells at room temperature. Forskolin (15µM final concentration) was added 5 minutes after the test compounds and the cells were incubated at room temperature for 30 minutes. The assay was stopped by adding cAMP detection mix (containing detergents for cell lysis) for 90 minutes at room temperature.

Cellular cAMP was measured using a Paradigm reader (Molecular Devices). The raw data was used to calculate the FRET signal based on the assay's P-factor as per cAMP kit instructions. The data was normalized to the maximal activity of a reference antagonist and dose response curves were fitted to the percent activity of the test compounds using a sigmoidal dose response model (Genedata Screener).

Results in the hGPR17 cAMP assay are provided for compounds of formula I in Table 1

**Table 1:**

| **Example number** | **hGPR17 cAMP IC50 [µM]** |
|---|---|
| 1 | 0.069 |
| 2 | 0.036 |
| 3 | 0.050 |
| 4 | 0.034 |
| 5 | 0.103 |
| 6 | 0.129 |
| 7 | 0.403 |
| 8 | 0.182 |
| 9 | 0.218 |
| 10 | 0.096 |
| 11 | 0.091 |
| 12 | 0.020 |
| 13 | 0.236 |
| 14 | 0.215 |
| 15 | 0.132 |
| 16 | 0.023 |
| 17 | 0.019 |
| 18 | 0.083 |
| 19 | 0.049 |
| 20 | 0.399 |
| 21 | 0.012 |
| 22 | 0.515 |
| 23 | 0.256 |
| 24 | 0.051 |
| 25 | 0.023 |
| 26 | 0.809 |
| 27 | 0.083 |
| 28 | 0.653 |
| 29 | 0.080 |
| 30 | 0.043 |
| 31 | 0.076 |
| 32 | 0.232 |
| 33 | 0.569 |
| 34 | 0.924 |
| 35 | 0.479 |
| 36 | 0.119 |
| 37 | 0.068 |
| 38 | 2.675 |
| 39 | 0.287 |
| 40 | 0.628 |
| 41 | 0.021 |
| 42 | 0.194 |
| 43 | 0.060 |
| 44 | 0.131 |
| 45 | 0.082 |
| 46 | 0.918 |
| 47 | 0.019 |
| 48 | 0.026 |
| 49 | 0.809 |
| 50 | 2.791 |
| 51 | 0.094 |
| 52 | 0.032 |
| 53 | 0.011 |
| 54 | 0.017 |
| 55 | 0.017 |
| 56 | 0.013 |
| 57 | 0.034 |
| 58 | 0.017 |
| 59 | 0.013 |
| 60 | 0.008 |
| 61 | 0.007 |
| 62 | 0.012 |
| 63 | 0.182 |
| 64 | 0.039 |
| 65 | 0.061 |
| 66 | 0.019 |
| 67 | 0.065 |
| 68 | 0.009 |
| 69 | 0.014 |
| 70 | 2.741 |
| 71 | 11.456 |
| 72 | 0.040 |
| 73 | 0.124 |
| 74 | 0.142 |
| 75 | 0.946 |
| 76 | 0.916 |
| 77 | 0.148 |
| 78 | 0.244 |
| 79 | 0.128 |
| 80 | 20.833 |
| 81 | 5.705 |
| 82 | 0.752 |
| 83 | 0.078 |
| 84 | 0.521 |
| 85 | 0.623 |
| 86 | 0.117 |
| 87 | 0.066 |
| 88 | 0.033 |
| 89 | 0.212 |
| 90 | 0.029 |
| 91 | 0.030 |
| 92 | 0.018 |
| 93 | 0.010 |
| 94 | 0.076 |
| 95 | 0.068 |
| 96 | 0.029 |
| 97 | 0.050 |
| 98 | 0.025 |
| 99 | 0.102 |
| 100 | 0.160 |
| 101 | 0.029 |
| 102 | 0.014 |
| 103 | 0.011 |
| 104 | 0.209 |
| 105 | 0.014 |
| 106 | 0.007 |
| 107 | 0.012 |
| 108 | 0.642 |
| 109 | 0.036 |
| 110 | 0.025 |
| 111 | 0.013 |
| 112 | 0.016 |
| 113 | 0.021 |
| 114 | 0.045 |
| 115 | 0.026 |
| 116 | 0.026 |
| 117 | 0.030 |
| 118 | 0.033 |
| 119 | 0.161 |
| 120 | 0.040 |
| 121 | 0.016 |
| 122 | 0.036 |
| 123 | 0.091 |
| 124 | 0.034 |
| 125 | 0.023 |
| 126 | 0.082 |
| 127 | 0.030 |
| 128 | 0.446 |
| 129 | 0.961 |
| 130 | 0.386 |
| 131 | 0.116 |
| 132 | 0.039 |
| 133 | 0.076 |
| 134 | 0.124 |
| 135 | 0.187 |
| 136 | 0.747 |
| 137 | 0.442 |
| 138 | 20.833 |
| 139 | 0.106 |
| 140 | 0.050 |
| 141 | 0.014 |
| 142 | 0.029 |
| 143 | 0.109 |
| 144 | 0.050 |
| 145 | 0.152 |
| 146 | 0.089 |
| 147 | 0.104 |
| 148 | 0.049 |
| 149 | 0.147 |
| 150 | 0.039 |
| 151 | 0.029 |
| 152 | 0.104 |
| 153 | 0.353 |
| 154 | 0.045 |
| 155 | 0.116 |
| 156 | 0.092 |
| 157 | 0.176 |
| 158 | 0.047 |
| 159 | 0.218 |
| 160 | 0.011 |
| 161 | 0.072 |
| 162 | 0.248 |
| 163 | 0.216 |
| 164 | 0.126 |
| 165 | 1.107 |
| 166 | 1.155 |
| 167 | 0.259 |
| 168 | 0.026 |
| 169 | 0.020 |
| 170 | 0.162 |
| 171 | 0.089 |
| 172 | 0.015 |
| 173 | 0.877 |
| 174 | 0.484 |
| 175 | 0.073 |
| 176 | 0.047 |
| 177 | 0.162 |
| 178 | 0.108 |
| 179 | 0.031 |
| 180 | 0.305 |
| 181 | 16.815 |
| 182 | 0.101 |
| 183 | 0.023 |
| 184 | 0.029 |
| 185 | 0.089 |
| 186 | 0.013 |
| 187 | 0.018 |
| 188 | 0.028 |
| 189 | 0.167 |
| 190 | 0.106 |
| 191 | 0.033 |
| 192 | 0.020 |
| 193 | 0.096 |
| 194 | 0.025 |
| 195 | 0.054 |
| 196 | 0.016 |
| 197 | 0.981 |
| 198 | 0.036 |
| 199 | 0.008 |
| 200 | 0.066 |
| 201 | 0.030 |
| 202 | 0.028 |
| 203 | 0.127 |
| 204 | 0.029 |
| 205 | 0.049 |
| 206 | 0.057 |
| 207 | 0.014 |
| 208 | 0.025 |
| 209 | 0.068 |
| 210 | 0.018 |
| 211 | 0.030 |
| 212 | 0.045 |
| 213 | 0.034 |
| 214 | 0.038 |
| 215 | 0.028 |
| 216 | 0.043 |
| 217 | 0.017 |
| 218 | 0.014 |
| 219 | 0.018 |
| 220 | 0.027 |
| 221 | 0.065 |
| 222 | 0.291 |
| 223 | 0.040 |
| 224 | 0.025 |
| 225 | 0.032 |
| 226 | 0.035 |
| 227 | 0.022 |
| 228 | 0.161 |
| 229 | 0.027 |
| 230 | 0.041 |
| 231 | 0.021 |
| 232 | 0.035 |
| 233 | 0.202 |
| 234 | 0.016 |
| 235 | 0.032 |
| 236 | 0.014 |
| 237 | 0.034 |
| 238 | 0.383 |
| 239 | 0.046 |
| 240 | 0.048 |
| 241 | 0.021 |
| 242 | 0.043 |
| 243 | 0.009 |
| 244 | 0.016 |
| 245 | 0.027 |
| 246 | 0.019 |
| 247 | 0.042 |
| 248 | 0.367 |
| 249 | 0.036 |
| 250 | 0.035 |
| 251 | 0.022 |
| 252 | 0.014 |
| 253 | 0.020 |
| 254 | 0.075 |
| 255 | 0.038 |
| 256 | 0.215 |
| 257 | 0.084 |
| 258 | 0.037 |
| 259 | 0.025 |
| 260 | 0.488 |
| 261 | 0.091 |
| 262 | 0.040 |
| 263 | 2.061 |
| 264 | 0.023 |
| 265 | 0.014 |
| 266 | 0.090 |
| 267 | 0.049 |
| 268 | 0.165 |
| 269 | 0.138 |
| 270 | 0.072 |
| 271 | 0.290 |
| 272 | 1.177 |
| 273 | 0.128 |
| 274 | 0.237 |
| 275 | 0.128 |
| 276 | 0.136 |
| 277 | 0.718 |
| 278 | 0.229 |
| 279 | 0.069 |
| 280 | 0.062 |
| 281 | 0.237 |
| 282 | 0.110 |
| 283 | 0.013 |
| 284 | 0.273 |
| 285 | 0.117 |
| 286 | 0.203 |
| 287 | 0.114 |
| 288 | 0.057 |
| 289 | 0.069 |
| 290 | 0.405 |
| 291 | 0.842 |
| 292 | 0.433 |
| 293 | 0.240 |
| 294 | 0.024 |

### Microsome Clearance Assay Protocol:

Incubations at a test compound of 1 mM in microsomes (0.5 mg/mL) plus cofactor NADPH are performed in 96 well plates at 37°C on a TECAN (Tecan Group Ltd, Switzerland) automated liquid handling system. The final concentration of the test compound in the incubation is 1 microM. After a 10 minutes preincubation step of the test compound with the microsomes, the enzymatic reaction is started by the addition of cofactors. At 1, 3, 6, 9, 15, 25, 35 and 45 minutes, aliquots of the incubations are removed and quenched with 1:3 (v/v) acetonitrile containing internal standards. Samples are then cooled and centrifuged before analysis of the supernatant by LC-MS/MS.

Reference examples RE-A, RE-B, RE-C, RE-D, RE-E, and RE-F have been prepared as described herein.

Reference compounds were tested against exemplified compounds for their Microsome Clearance. The results are shown in Table 2 below.

**Table 2.**

| **Compound** | **Microsomal Clearance human, mouse, rat (µL/min/mg protein)** |
|---|---|
| **Example 1** | 16, 10, 29 |
| 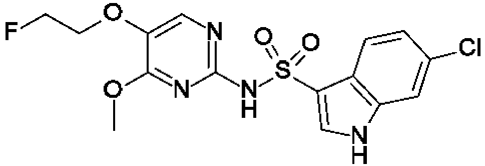 | |
| **Example 123** | 10, 10, 10 |
| 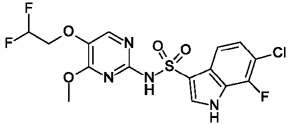 | |
| **Example 47** | 10, 10, 110 |
| 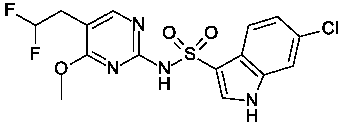 | |
| **Example 179** | 10, 10, 33 |
| 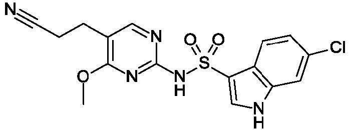 | |
| **Example 48** | 10, 29 |
| 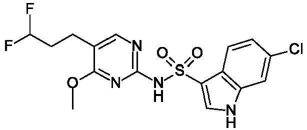 | |
| **Example 22** | 10, 10, 10 |
| 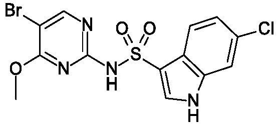 | |
| 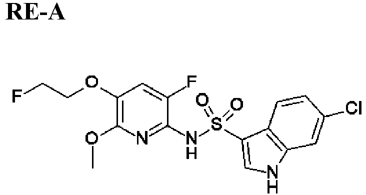 | 121, 77, 357 |
| 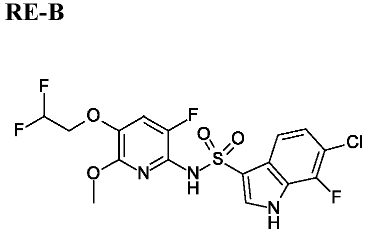 | 11, 26, 65 |
| 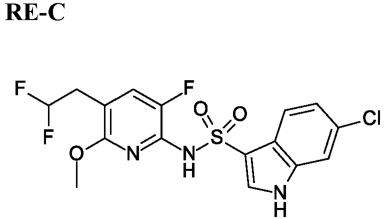 | 35, 81, 286 |
| 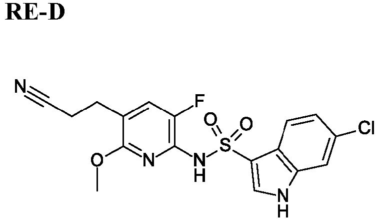 | 103, 121, 869 |
| 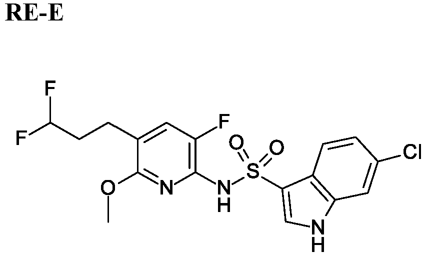 | 153, 385 |
| 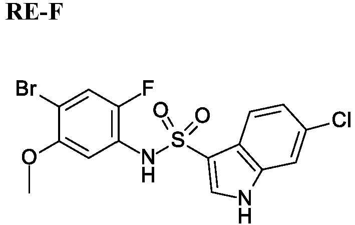 | 29, 129, 166 |

The invention will now be illustrated by the following examples which have no limiting character.

In case the preparative examples are obtained as a mixture of enantiomers, the pure enantiomers can be obtained by methods described herein or by methods known to those skilled in the art, such as e.g. chiral chromatography or crystallization.

### Examples

All examples and intermediates were prepared under nitrogen atmosphere if not specified otherwise.

### Intermediates A

### Intermediate A1: 6-chloro-1H-indole-3-sulfonyl chloride

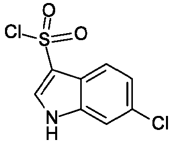

### Intermediate A1 is commercial (CAS: 1216060-79-5)

### Intermediate A2: 6-bromo-1H-indole-3-sulfonyl chloride

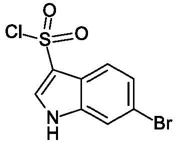

### Intermediate A2 is commercial (CAS: 2137914-35-1)

### Intermediates A3-A9

The intermediates A3-A9 are known and have been prepared following procedures described in the indicated patent applications:

| Intermediate number | Structure | Name (CAS number) | Patent application number |
|---|---|---|---|
| A3 | 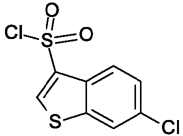 | 6-chlorobenzothiophene-3-sulfonyl chloride (1593831-66-3) | WO2018122232 |
| A4 | 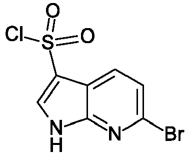 | 6-bromo-1H-pyrrolo[2,3-b]pyridine-3-sulfonyl chloride (2231234-27-6) | WO2018122232 |
| A5 | 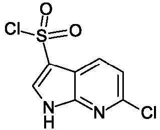 | 6-chloro-1H-pyrrolo[2,3-b]pyridine-3-sulfonyl chloride (2231234-21-0) | WO2018122233 |
| A6 | 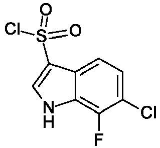 | 6-chloro-7-fluoro-1H-indole-3-sulfonyl chloride (2231234-36-7) | WO2018122233 |
| A7 | 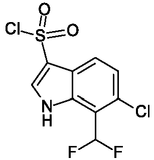 | 6-chloro-7-(difluoromethyl)-1H-indole-3-sulfonyl chloride (2404661-51-2) | WO2019243398 |
| A8 | 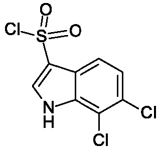 | 6,7-dichloro-1H-indole-3-sulfonyl chloride (1780326-24-0) | WO2019243398 |
| A9 | 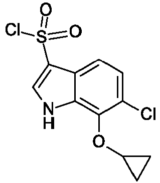 | 6-chloro-7-(cyclopropoxy)-1H-indole-3-sulfonyl chloride (2404661-50-1) | WO2019243398 |

### Intermediate A10: 6-chlorothieno[2,3-b]pyridine-3-sulfonyl chloride

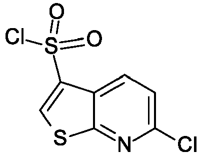

6-chlorothieno[2,3-b]pyridine (50 mg, 0.295 mmol, CAS: 62226-18-0) was dissolved in chlorosulfonic acid (708.12 mg, 406.97 uL, 5.9 mmol) at room temperature. The mixture was stirred at 60°C for 2 hr and then added to a stirring mixture of icewater (10 ml) / ethyl acetate (10 ml). Both layers were quickly separated. The aqueous phase was extracted with ethyl acetate. The combined organic phases were dried over anhydrous sodium sulfate, filtered and concentrated in vacuo to provide the title compound as a dark brown solid (80 mg, 96 % yield). MS (ESI): m/z=268.0 [M+H]+

### Intermediates A11-A20

The intermediates A11-A20 are known and have been prepared following procedures described in the indicated patent applications:

| Intermediate number | Structure | Name (CAS number) | Patent application number |
|---|---|---|---|
| A11 | 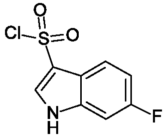 | 6-fluoro-1H-indole-3-sulfonyl chloride (1216026-07-1) | WO2018122232 |
| A12 | 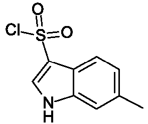 | 6-methyl-1H-indole-3-sulfonyl chloride (1367937-31-2) | WO2018122232 |
| A13 | 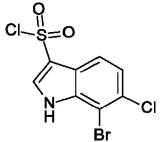 | 7-bromo-6-chloro-1H-indole-3-sulfonyl chloride (2231234-40-3) | WO2018122232 |
| A14 | 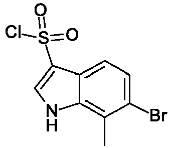 | 6-bromo-7-methyl-1H-indole-3-sulfonyl chloride (2231234-52-7) | WO2018122232 |
| A15 | 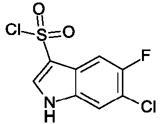 | 6-chloro-5-fluoro-1H-indole-3-sulfonyl chloride (2231234-44-7) | WO2018122232 |
| A16 | 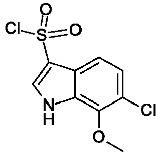 | 6-chloro-7-methoxy-1H-indole-3-sulfonyl chloride (2231234-35-6) | WO2018122232 |
| A17 | 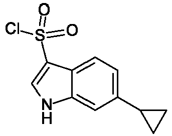 | 6-cyclopropyl-1H-indole-3-sulfonyl chloride (2231234-20-9) | WO2018122232 |
| A18 | 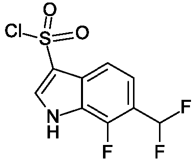 | 6-(difluoromethyl)-7-fluoro-1H-indole-3-sulfonyl chloride (2404661-52-3) | WO2019243398 |
| A19 | 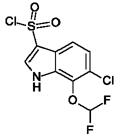 | 6-chloro-7-(difluoromethoxy)-1H-indole-3-sulfonyl chloride (2231234-59-4) | WO2018122232 |
| A20 | 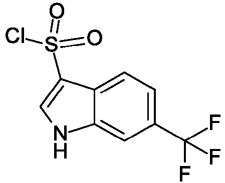 | 6-(trifluoromethyl)-1H-indole-3-sulfonyl chloride (1784173-91-6) | WO2018122232 |

### Intermediates A21-A31

The intermediates A21-A31 have been prepared in analogy to sulfonylchloride intermediate A10, by chlorosulfonylation of their respective non-sulfonylated precursors in the presence of chlorosulfonic acid. The precursors are either commercial or described in the indicated patent applications:

| Intermediate number | Structure | Name | Name of non-sulfonylated staring material (CAS number, patent application number) |
|---|---|---|---|
| A21 | 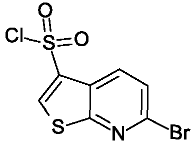 | 6-bromothieno[2,3-b]pyridine-3-sulfonyl chloride | 6-bromothieno[2,3-b]pyridine (CAS: 1836214-86-8, commercial) |
| A22 | 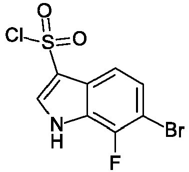 | 6-bromo-7-fluoro-1H-indole-3-sulfonyl chloride | 6-bromo-7-fluoro-1H-indole (CAS: 936901-94-9, commercial) |
| A23 | 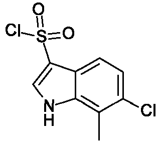 | 6-chloro-7-methyl-1H-indole-3-sulfonyl chloride | 6-chloro-7-methyl-1H-indole (CAS: 57817-09-1, commercial) |
| A24 | 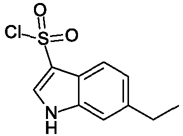 | 6-ethyl-1H-indole-3-sulfonyl chloride | 6-ethyl-1H-indole (CAS: 4765-24-6, commercial) |
| A25 | 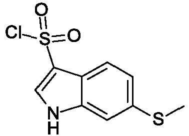 | 6-methylsulfanyl-1H-indole-3-sulfonyl chloride | 6-(methylthio)-1H-indole (CAS: 202584-22-3, commercial) |
| A26 | 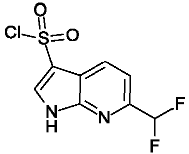 | 6-(difluoromethyl)-1H-pyrrolo[2,3-b]pyridine-3-sulfonyl chloride | 6-(difluoromethyl)-1H-pyrrolo[2,3-b]pyridine (CAS: 1261488-22-5, WO2018122232) |
| A27 | 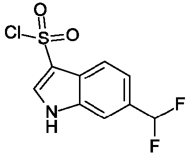 | 6-(difluoromethyl)-1H-indole-3-sulfonyl chloride | 6-(difluoromethyl)-1H-indole (CAS: 127956-27-8, commercial) |
| A28 | 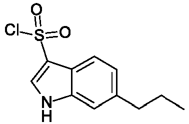 | 6-propyl-1H-indole-3-sulfonyl chloride | 6-propyl-1H-indole (CAS: 1043602-17-0, WO2015110092) |
| A29 | 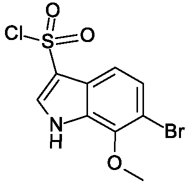 | 6-bromo-7-methoxy-1H-indole-3-sulfonyl chloride | 6-bromo-7-methoxy-1H-indole (CAS:938061-65-5, US20070123527) |
| A30 | 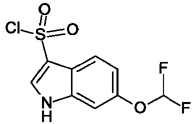 | 6-(difluoromethoxy)-1H-indole-3-sulfonyl chloride | 6-(difluoromethoxy)-1H-indole (CAS: 200207-21-2, commercial) |
| A31 | 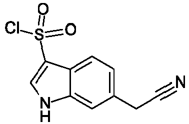 | 6-(cyanomethyl)-1H-indole-3-sulfonyl chloride | 2-(1H-indol-6-yl)acetonitrile (CAS:39689-57-1, WO2021111124) |

### Intermediate A32: 6-chloro-7-methylsulfanyl-1H-indole-3-sulfonyl chloride

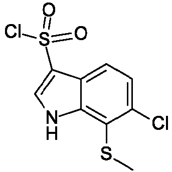

### Step 1: 1-chloro-2-(methylthio)-3-nitro-benzene

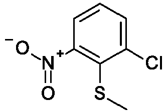

To a solution of 1-chloro-2-fluoro-3-nitro-benzene (600 mg, 402.68 uL, 3.42 mmol, commercial) in N,N-dimethylformamide, extra dry (7 mL) under argon, NaSMe (311.44 mg, 4.44 mmol) was added. The brown reaction was stirred at room temperature for 3.5 hours, another 0.5 equivalent of NaSMe was added. The reaction mixture was stirred for another 30 min, quenched with bleach and extracted 3 times with ethylacetate. The organic layers were dried over sodiumsulfate, filtered and concentrated in vacuo. The residue was purified by flash chromatography over silica gel using a gradient ethylacetate/heptane 0-5% to provide the title compound as a orange oil (311 mg, 44 % yield).

### Step 2: 6-chloro-7-methylsulfanyl-1H-indole

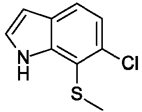

Under argon a solution of 1-chloro-2-(methylthio)-3-nitro-benzene (346 mg, 1.53 mmol) in THF, extra dry (5 mL) was cooled to -78°C. 1 M vinylmagnesium bromide (4.59 mL, 4.59 mmol) was added within 8 min, the dark orange solution was stirred for 4.5h. 1 M vinylmagnesium bromide (0.76 mL, 0.765 mmol) was added. After 5.5 h, the reaction mixture was quenched with a saturated solution of ammonim chloride. The aqueous phase was extracted twice with ethylacetate, dried over sodiumsulfate, filtered and concentrated in vacuo. The residue was purified by flash chromatography over silica gel using a gradient ethylacetate/heptane 0-5% to provide the title compound as a orange oil (150 mg, 50 % yield). MS (ESI): m/z= 198.1 [M+H]⁺

### Step 3: 6-chloro-7-methylsulfanyl-1H-indole-3-sulfonyl chloride

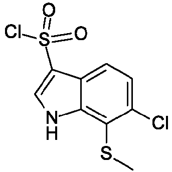

The title compound was prepared in analogy to intermediate A10 from 6-chloro-7-methylsulfanyl-1H-indole as a light yellow oil. MS (ESI): m/z= 294.0 [M+H]⁺

### Intermediate A33: 6-chloro-7-(fluoromethoxy)-1H-indole-3-sulfonyl chloride

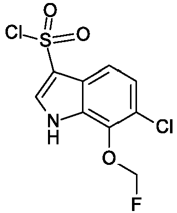

### Step 1: 1-chloro-2-(fluoromethoxy)-3-nitro-benzene

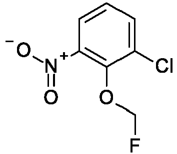

To a solution of 2-chloro-6-nitrophenol (1.8 g, 10.37 mmol, commercial) in DMF (40 mL) was added NaH (622.82 mg, 15.56 mmol) at 0°C. The mixture was stirred at 0°C for 0.5 hour and fluoro(iodo)methane (2.5 g, 15.56 mmol) was added. The reaction mixture was stirred at 25°C for 12 hours, quenched with water (50 mL) and then extracted twice with ethylacetate (50mL). The combined organic phases were washed with brine, dried over sodiumsulfate, filtered and concentrated in vacuo. The residue was purified by flash chromatography over silica gel using a gradient ethylacetate/ether 0-5% to provide the title compound as a light yellow oil (1.6 g, 75 % yield). 1H NMR (400 MHz, CDCl₃) δ = 7.79 (dd, J = 1.6, 8.2 Hz, 1H), 7.71 (dd, J = 1.6, 8.1 Hz, 1H), 7.32 (t, J = 8.2 Hz, 1H), 5.77 (s, 1H), 5.63 (s, 1H)

### Step 2: 6-chloro-7-(fluoromethoxy)-1H-indole

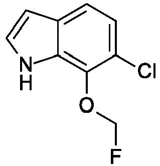

The title compound was prepared in analogy to intermediate A32, step 2 from 1-chloro-2-(fluoromethoxy)-3-nitro-benzene as a brown oil. 1H NMR (400 MHz, CDCl₃) δ ppm 8.41 - 8.84 (m, 1 H) 7.39 - 7.43 (m, 1 H) 7.23 - 7.26 (m, 1 H) 7.10 - 7.14 (m, 1 H) 6.54 - 6.58 (m, 1 H) 5.67 - 5.85 (m, 2 H)

### Step 3: 6-chloro-7-(fluoromethoxy)-1H-indole-3-sulfonyl chloride

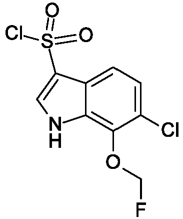

The title compound was prepared in analogy to intermediate A10 from 6-chloro-7-(fluoromethoxy)-1H-indole as a grey oil.

### Intermediate A34: 6-chloro-7-(difluoromethylsulfanyl)-1H-indole-3-sulfonyl chloride

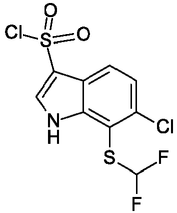

### Step 1: 2-chloro-6-nitro-benzenethiol

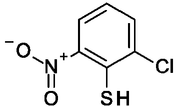

To a solution of 2,3-dichloronitrobenzene (20.0 g, 104.17 mmol, commercial) in DMSO (300 mL) was added sodium sulfide (8.53 g, 109.38 mmol) at 25 °C. The mixture was stirred at 25 °C for 18 hours. The reaction mixture was poured into water (1000 mL), and 3M HCl was added to the reaction mixture until the pH adjust to 4, extracted with ethylacetate (3x 300 mL), the combined organic layers was dried over anhydrous sodiumsulfate, filtered and concentrated in vacuo. The residue was purified by flash chromatography over silica gel using a gradient ethylacetate/ether 0-30% to provide the title compound as a brown solid (12 g, 61 % yield).

### Step 2: 1-chloro-2-(difluoromethylsulfanyl)-3-nitro-benzene

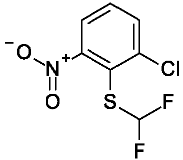

To a 30°C mixture of 2-chloro-6-nitro-benzenethiol (6.0 g, 31.64 mmol) in acetonitrile (300 mL) was added KOH (17.75 g, 316.42 mmol) followed by diethyl (bromodifluoromethyl)phosphonate (33.8 g, 126.57 mmol). The reaction mixture was stirred at 30 °C for 12 hours, poured into water (500 mL), extracted with ethylacetate (3x200 mL). The combined organic layers were dried over anhydrous sodiumsulfate, filtered and concentrated in vacuo. The residue was purified by flash chromatography over silica gel using a gradient ethylacetate/ether 0-30% to provide the title compound as a brown solid (5 g, 66 % yield). 1H NMR (400 MHz, DMSO-d6) δ = 8.06 - 7.98 (m, 2H), 7.85 - 7.78 (m, 1H), 7.59 - 7.28 (m, 1H)

### Step 3: 6-chloro-7-(fluoromethylsulfanyl)-1H-indole

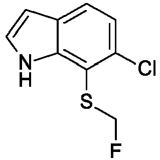

The title compound was prepared in analogy to intermediate A32, step 2 from 1-chloro-2-(difluoromethylsulfanyl)-3-nitro-benzene as a yellow oil. 1H NMR (400 MHz, DMSO-d6) δ = 11.50 (br s, 1H), 7.72 (d, J = 8.3 Hz, 1H), 7.54 - 7.36 (m, 2H), 7.25 (d, J = 8.4 Hz, 1H), 6.57 (dd, J = 1.9, 3.1 Hz, 1H)

### Step 4: 6-chloro-7-(fluoromethylsulfanyl)-1H-indole-3-sulfonyl chloride

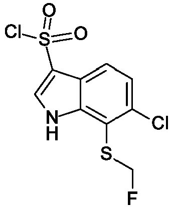

The title compound was prepared in analogy to intermediate A10 from 6-chloro-7-(fluoromethylsulfanyl)-1H-indole as a light red solid.

### Intermediate A35: 6-chloro-7-cyclopropyl-1H-indole-3-sulfonyl chloride

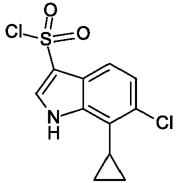

### Step 1: 6-chloro-7-cyclopropyl-1H-indole

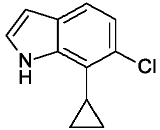

To a solution of 7-bromo-6-chloro-1H-indole (6.50 g, 28.2 mmol, commercial) and cyclopropyl-boronic acid (3.63 g, 42.3 mmol, 3.59 mL) in dioxane (100 mL) and in water (10.0 mL) was added PdCl2(dppf) (2.30 g, 2.80 mmol) and potassium phosphate (11.9 g, 56.4 mmol) in one portion at 25 °C under nitrogen. The mixture was stirred at 100 °C for 16 hours. The mixture was filtered and concentrated in vacuo. The residue was extracted with dichloromethane (2x50 mL) and the combined organic phases were washed with brine (2x50.0 mL), dried over sodium sulfate, filtered and concentrated in vacuo. The residue was purified by revered phase chromatography (column: YMC Triart C18 250 × 50 mm × 7 um) using a gradient water (10 mM NH4HCO3) - ACN 48-68% to provide the title compound as a yellow solid (5 g, 92.6 % yield). MS (ESI): m/z= 191.9 [M+H]⁺

### Step 2: 6-chloro-7-methylsulfanyl-1H-indole-3-sulfonyl chloride

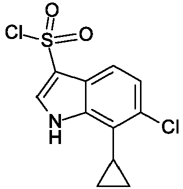

The title compound was prepared in analogy to intermediate A10 from 6-chloro-7-cyclopropyl-1H-indole as a white solid. MS (ESI): m/z= 288.1 [M+H]⁺

### Intermediate A36: 6-chloro-7-(cyanomethyl)-1H-indole-3-sulfonyl chloride

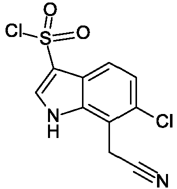

### Step 1: 2-(6-chloro-1H-indol-7-yl)acetonitrile

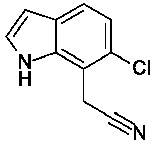

To a mixture of 7-bromo-6-chloro-1H-indole (12.7 g, 55.1 mmol, commercial) and 4-(4,4,5,5-tetramethyl-1,3,2-dioxaborolan-2-yl)isoxazole (13.9 g, 71.6 mmol, commercial) in DMSO (380 mL) and H₂O (190 mL) was added KF (9.60 g, 165 mmol, 3.87 mL) in one portion at 25 °C under N₂, then Pd(dppf)Cl₂ (8.06 g, 11.0 mmol) was added to the mixture. The mixture was warmed slowly to 120 °C and stirred for 12 hrs. The mixture was quenched with H₂O (950 mL), extracted with ethyl acetate (3x100 mL). The combined organic phases were washed with brine (2x50.0 mL), dried over sodium sulfate, filtered and concentrated in vacuo. The residue was purified by flash chromatography over silica gel using a gradient ethylacetate/ether 0-100% to provide the title compound as a grey solid (1.3 g, 12 % yield). MS (ESI): m/z= 191.0 [M+H]⁺

### Step 2: 6-chloro-7-(cyanomethyl)-1H-indole-3-sulfonyl chloride

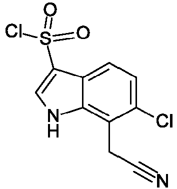

The title compound was prepared in analogy to intermediate A10 from 2-(6-chloro-1H-indol-7-yl)acetonitrile as a off-white solid. MS (ESI): m/z= 287.1 [M-H]⁻

### Intermediate A37: 6-bromobenzothiophene-3-sulfonyl chloride

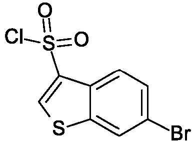

To a suspension of sulfur trioxide n,n-dimethylformamide complex (539.06 mg, 3.52 mmol) in 1,2-dichloroethane (2.5 mL) was added at 22 °C, 6-bromobenzothiophene (500 mg, 2.35 mmol, CAS: 17347-32-9) and stirred at 22 °C for 1 hour and then heated to 70 °C for 18 hours. The reaction mixture was allowed to cool to room temperature and thionyl chloride (614.14 mg, 376.77 uL, 5.16 mmol) was added. The mixture was stirred at 22 °C for 30 min, heated to 80 °C for 1 h and concentrated. The residue was extracted twice with ethylacetate and washed twice with water. The organic layers were combined, dried over sodium sulfate, filtered and concentrated in vacuo. The residue was purified by flash chromatography over silica gel using heptane as eluent to provide the title compound as an off-white solid (282 mg, 38 % yield). MS (ESI): m/z= 292.9 [M-H]⁻ (sulfonic acid)

### Intermediate A38: 6-methylbenzothiophene-3-sulfonyl chloride

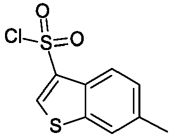

The title compound was prepared in analogy to intermediate A37 from 6-methylbenzothiophene (CAS: 16587-47-6, commercial) as a off-white solid . MS (ESI): m/z= 227.1 [M-H]⁻ (sulfonic acid)

### Intermediate A39: 7-chloro-1H-indole-3-sulfonyl chloride

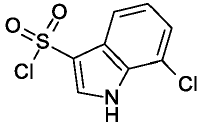

Intermediate A39 is commercial (CAS: 1369230-76-1)

### Intermediate A40: 6-chloro-7-fluoro-benzothiophene-3-sulfonyl chloride

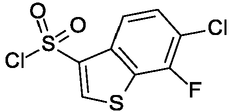

### Step 1: methyl 6-chloro-7-fluoro-benzothiophene-2-carboxylate

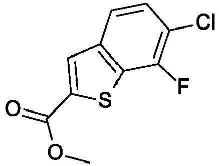

To a mixture of methyl thioglycolate (4.57 g, 43.05 mmol) in DMF (90 mL) was added 4-chloro-2,3-difluoro-benzaldehyde (7.6 g, 43.05 mmol, CAS: 1160573-23-8) and potassium carbonate (11.87 g, 86.09 mmol). The mixture was stirred at 40 °C for 16 hours, diluted with water and extracted three times with ethyl acetate. The organic layers were dried over sodium sulfate, filtered and concentrated in vacuo. The residue was purified by flash chromatography over silica gel using a gradient ethylacetate/petroleum ether 0-5% to provide the title compound as a white solid (8.7 g, 83 % yield). ¹H NMR (400 MHz, CHLOROFORM-d) δ = 8.03 (d, J = 3.5 Hz, 1H), 7.60 (d, J = 8.5 Hz, 1H), 7.42 (dd, J = 6.6, 8.5 Hz, 1H), 3.97 (s, 4H)

### Step 2: 6-chloro-7-fluoro-benzothiophene-2-carboxylic acid

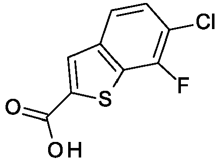

To a mixture of methyl 6-chloro-7-fluoro-benzothiophene-2-carboxylate (8700.0 mg, 35.56 mmol) in methanol (100 mL) and tetrahydrofuran (50 mL) was added 5 M aqueous NaOH (50.0 mL, 250 mmol) dropwise. The reaction mixture was stirred at 25 °C for 16 hours. 5 M HCl was added to the reaction mixture to adjust the pH to 1-2. The mixture was diluted with water and extracted three times with ethyl acetate. The organic layers were dried over sodium sulfate, filtered and concentrated in vacuo to provide the title compound as a yellow solid (8 g, 98 % yield). ¹H NMR (400 MHz, DMSO-d6) δ = 8.13 (d, J = 3.7 Hz, 1H), 7.87 (d, J = 8.6 Hz, 1H), 7.63 (dd, J = 7.0, 8.5 Hz, 1H)

### Step 3: 6-chloro-7-fluoro-benzothiophene

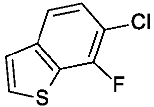

To a mixture of 6-chloro-7-fluoro-benzothiophene-2-carboxylic acid (1.0 g, 4.34 mmol) in DMF (40 mL) was added copper(I) chloride (1697.88 mg, 17.34 mmol). The reaction mixture was stirred at 120 °C for 16 hours, diluted with water and extracted three times with ethyl acetate. The organic layers were dried over sodium sulfate, filtered and concentrated in vacuo. The residue was purified by flash chromatography over silica gel using a gradient ethylacetate/petroleum ether 0-1% to provide the title compound as a yellow solid (600 mg, 74 % yield). ¹H NMR (400 MHz, CHLOROFORM-d) δ = 7.54 (d, J = 8.4 Hz, 1H), 7.49 (d, J = 5.3 Hz, 1H), 7.41 - 7.32 (m, 2H)

### Step 4: 6-chloro-7-fluoro-benzothiophene-3-sulfonyl chloride

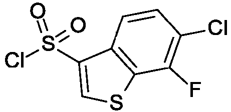

To a solution of 6-chloro-7-fluoro-benzothiophene (100 mg, 0.536 mmol) in acetonitrile, extra dry (5 mL) was added chlorosulfonic acid (202.92 mg, 115.75 uL, 1.74 mmol) dropwise at 0 °C. The mixture was stirred at 22 °C overnight. Phosphorus oxychloride (361.5 mg, 219.75 uL, 2.36 mmol) was added and the mixture was stirred at 50 °C for 2 hours and for 22 hours at 70 °C. The mixture was dropped into ice (25 mL) and extracted twice with ethyl acetate. The organic layers were dried over sodium sulfate, filtered and concentrated in vacuo to provide the title compound as a light brown solid (152 mg, 100 % yield). MS (ESI): m/z= 265.0 [M-H]⁻ (sulfonic acid)

### Intermediate A41: 6,7-difluoro-1H-indole-3-sulfonyl chloride

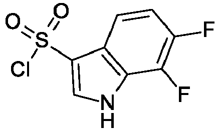

The title compound was prepared in analogy to intermediate A10 from 6,7-difluoro-1H-indole (CAS: 271780-84-8) as a red solid. MS (ESI): m/z= 250.0 [M-H]⁻

### Intermediate A42: 6,8-dihydro-1H-furo[3,4-g]indole-3-sulfonyl chloride

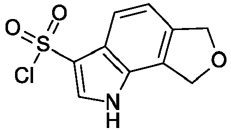

### Step 1: 5,7-diiodo-1,3-dihydroisobenzofuran-4-amine

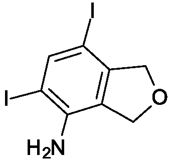

To a stirred suspension of 1,3-dihydroisobenzofuran-4-amine (1 g, 7.18 mmol, CAS: 98475-10-6) in toluene (10 mL) was added acetic acid (452.52 mg, 430.97 uL, 7.54 mmol), followed by n-iodosuccinimide (1.75 g, 7.54 mmol). The reaction mixture was stirred at room temperature for 30 min. The reaction mixture was poured into water and extracted twice with ethyl acetate. The organic layers were dried over sodium sulfate, filtered and concentrated in vacuo. The residue was purified by flash chromatography over silica gel using a gradient ethylacetate/heptane 0-50%, followed by flash chromatography on a C18 RediSepRf Gold column using a gradient acetonitrile/water 10-100% to provide the title compound as an off-white solid (273 mg, 10 % yield). MS (ESI): m/z= 388.0 [M+H]⁺

### Step 2: (5-iodo-6,8-dihydro-1H-furo[3,4-g]indol-2-yl)-trimethyl-silane

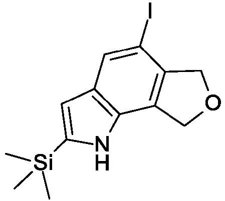

In a sealed tube, a mixture of 5,7-diiodo-1,3-dihydroisobenzofuran-4-amine (1 g, 2.58 mmol), potassium carbonate (721.53 mg, 5.17 mmol), tetrakis(triphenylphosphine)palladium(0) (301.64 mg, 0.258 mmol), XPhos (246.39 mg, 0.517 mmol) and trimethylsilylacetylene (310.81 mg, 438.37 uL, 3.1 mmol) in tetrahydrofuran (40 mL) was heated to 75 °C and stirred for 15 hours. The reaction mixture was poured into water and extracted twice with ethyl acetate. The organic layers were dried over sodium sulfate, filtered and concentrated in vacuo. The residue was purified by flash chromatography over silica gel using a gradient ethylacetate/heptane 0-20%, followed by flash chromatography on a C18 RediSepRf Gold column using a gradient acetonitrile/water 10-100% to provide the title compound as a white solid (341 mg, 37 % yield). MS (ESI): m/z= 358.0 [M+H]⁺

### Step 3: 5-iodo-6,8-dihydro-1H-furo[3,4-g]indole

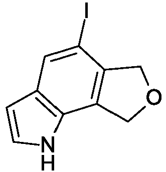

To a stirred solution of (5-iodo-6,8-dihydro-1H-fur[3,4-g]indol-2-yl)-trimethyl-silane (339 mg, 0.949 mmol) in tetrahydrofuran (7 mL) was added 1 M tetrabutylammonium fluoride solution in THF (4.74 mL, 4.74 mmol) dropwise at room temperature. The reaction mixture was heated to 50 °C and stirred for 16 hours. The reaction mixture was poured into sat. NaHCO3 and extracted twice with ethyl acetate. The organic layers were dried over sodium sulfate, filtered and concentrated in vacuo. The residue was triturated with dichloromethane, filtered and dried in vacuo to give 220 mg of the desired product. The filtrate was concentrated in vacuo. The residue was purified by flash chromatography over silica gel using a gradient ethylacetate/heptane 0-30% to give 24 mg of the desired product. Overall the process provided the title compound as an orange solid (244 mg, 90 % yield). MS (ESI): m/z= 286.0 [M+H]⁺

### Step 4: 6,8-dihydro-1H-furo[3,4-g]indole

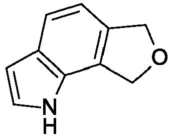

A suspension of 5-iodo-6,8-dihydro-1H-fur[3,4-g]indole (250 mg, 0.877 mmol) and triethylamine (97.61 mg, 134.45 uL, 0.965 mmol) in ethanol (15 mL) was degassed with argon and palladium on activated charcoal (93.32 mg, 0.088 mmol) was added. The reaction mixture was stirred at room temperature under 1 atm. of hydrogen for 1 hour, filtered through a pad of celite and concentrated in vacuo. The residue was purified by flash chromatography over silica gel using a gradient ethylacetate/heptane 0-50% to provide the title compound as a white solid (125 mg, 90 % yield). MS (ESI): m/z= 160.1 [M+H]⁺

### Step 5: 6,8-dihydro-1H-furo[3,4-g]indole-3-sulfonyl chloride

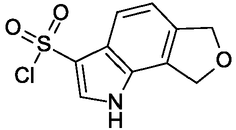

A solution of 6,8-dihydro-1H-fur[3,4-g]indole (65 mg, 0.408 mmol) in acetonitrile (1.5 mL) was cooled to 0 °C. Chlorosulfonic acid (123.71 mg, 71.1 uL, 1.06 mmol) was added dropwise. The reaction mixture was stirred at 0 °C for 30 min, at room temperature for 3 hours. The reaction mixture was poured into ice/ethyl acetate and extracted twice with ethyl acetate. The organic layers were dried over sodium sulfate, filtered and concentrated in vacuo to provide the title compound as a light brown solid (44 mg, 42 % yield). MS (ESI): m/z= 256.0 [M+H]⁺

### Intermediate A43: 6-chloro-7-cyano-1H-indole-3-sulfonyl chloride

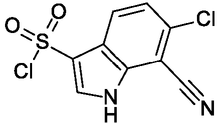

### Step 1: 6-chloro-1H-indole-7-carbonitrile

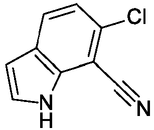

A suspension of 7-bromo-6-chloro-1H-indole (377 mg, 1.6 mmol, CAS: 1427439-04-0), zinc cyanide (768.39 mg, 6.41 mmol) and tetrakis[triphenylphosphine]palladium(0) (333.04 mg, 0.285 mmol) in N,N-dimethylacetamide (10 mL) was heated in a microwave at 160 °C for 20 min. The reaction mixture was diluted with ethyl acetate and washed twice with brine. The organic layer was dried over sodium sulfate, filtered and concentrated in vacuo. The residue was purified by flash chromatography over silica gel using a gradient ethylacetate/heptane 0-20% to provide the title compound as an off-white solid (233 mg, 82 % yield). MS (ESI): m/z= 175.0 [M-H]⁻

### Step 2: 6-chloro-7-cyano-1H-indole-3-sulfonyl chloride

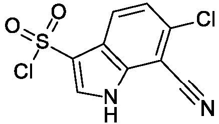

The title compound was prepared in analogy to intermediate A10 from 6-chloro-1H-indole-7-carbonitrile as a off-white solid. MS (ESI): m/z= 273.0 [M-H]⁻

### Intermediate A44: 6-(difluoromethyl)thieno[2,3-b]pyridine-3-sulfonyl chloride

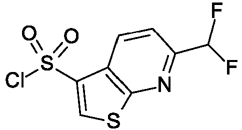

### Step 1: 7-oxidothieno[2,3-b]pyridin-7-ium

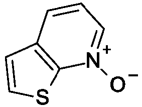

To a solution of thieno[2,3-b]pyridine (2 g, 1.54 mL, 14.79 mmol, CAS: 272-23-4) in dichloromethane (20 mL) and ethyl acetate (20 mL) was added m-CPBA (4.38 g, 17.75 mmol) portionwise at 0 °C over 20 min. The reaction mixture was allowed to warm to room temperature and stirred for 2 hours. The solvent was removed under reduced pressure. The residue was diluted with dichloromethane and washed with three times with sat. NaHCO3. The combined aqueous layers were extracted twice with dichloromethane. The organic layers were dried over sodium sulfate, filtered and concentrated in vacuo to provide the title compound as a yellow oil (2.2 g, 96 % yield). MS (ESI): m/z= 152.1 [M+H]⁺

### Step 2: 6-bromothieno[2,3-b]pyridine

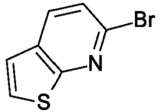

7-oxidothieno[2,3-b]pyridin-7-ium (2.2 g, 14.13 mmol) was dissolved in N,N-dimethylformamide, extra dry (12.54 mL) and ethylene glycol dimethyl ether (75.24 mL) under argon. The stirring solution was cooled to 0 °C and tetrabutylammonium bromide (5.47 g, 16.96 mmol) was added in one portion. Methanesulfonic anhydride (4.92 g, 28.27 mmol) was added portionswise over 15 min at 0 °C. The brown solution was stirred for 2 hours at 0 °C and was then allowed to warm up to room temperature and stirred for another 3 hours. The solvent was removed under reduced pressure. The residue was dissolved in dichloromethane (80mL) and water (40 mL). The pH of the mixture was carefully set to 7 with 2 N NaOH. The aqueous layer was extracted with three times with dichloromethane. The organic layers were washed with brine, dried over sodium sulfate, filtered and concentrated in vacuo. The residue was purified by flash chromatography over silica gel using a gradient ethylacetate/heptane 0-70% to afford a mixture of regioisomers, which were separated by SFC to provide the title compound as a light yellow solid (900 mg, 29 % yield). MS (ESI): m/z= 213.9 [M+H]⁺

### Step 3: thieno[2,3-b]pyridine-6-carbaldehyde

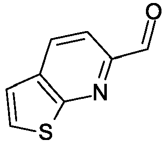

6-bromothieno[2,3-b]pyridine (994 mg, 4.64 mmol) was laid in an autoclave and was dissolved in acetonitrile (47.16 g, 60 mL, 1148.84 mmol). Pd(dppp)Cl2 (758.35 mg, 0.929 mmol, CAS: 59831-02-6), triethylsilane (809.85 mg, 1.11 mL, 6.96 mmol) and sodium acetate (761.75 mg, 9.29 mmol) were added. The reactor was closed, rinsed 5 times with 10 bar of CO and was then pushed to 30 bar CO. The reaction mixture was stirred for 24 hours at 60 °C and 30 bar. The mixture was filtered, poured into water and a small amount of brine and extracted three times with ethyl acetate. The organic layers were dried over sodium sulfate, filtered and concentrated in vacuo. The residue was purified by flash chromatography over silica gel using a gradient ethylacetate/heptane 0-20% to provide the title compound as a white solid (473 mg, 52 % yield). MS (ESI): m/z= 163.9 [M+H]⁺

### Step 4: 6-(difluoromethyl)thieno[2,3-b]pyridine

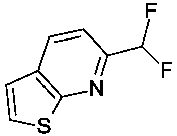

To a solution of thieno[2,3-b]pyridine-6-carbaldehyde (470 mg, 2.42 mmol) in dichloromethane, extra dry (13.16 mL) was added Deoxo-fluor ^{®}, 2.7M (50 wt.%) solution in toluene (1.34 mL, 3.63 mmol) dropwise at 0 °C. The reaction mixture was stirred for 1 hour at 0 °C and was then allowed to warm to room temperature and stirred overnight. The mixture was diluted with sat. NaHCO3 and then extracted three times with dichloromethane. The organic layers were dried over sodium sulfate, filtered and concentrated in vacuo. The residue was purified by flash chromatography over silica gel using a gradient ethylacetate/heptane 0-40% to provide the title compound as a colorless liquid (185 mg, 41 % yield). MS (ESI): m/z= 186.0 [M+H]⁺

### Step 5: 6-(difluoromethyl)thieno[2,3-b]pyridine-3-sulfonyl chloride

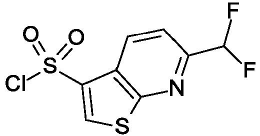

The title compound was prepared in analogy to intermediate A10 from 6-(difluoromethyl)thieno[2,3-b]pyridine as a light brown solid. MS (ESI): m/z= 283.9 [M+H]⁺

### Intermediate A45: 6-chloro-7-(dimethylamino)-1H-indole-3-sulfonyl chloride

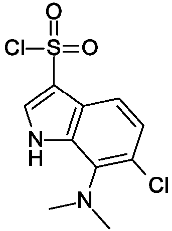

### Step 1: 6-chloro-N,N-dimethyl-1H-indol-7-amine

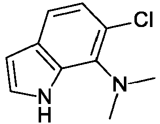

To a solution of (2-chloro-6-nitro-phenyl)-dimethyl-amine (600 mg, 2.99 mmol, CAS: 96994-75-1) in tetrahydrofuran, extra dry (9.97 mL) was added 1 M vinylmagnesium bromide solution in THF (11.96 mL, 11.96 mmol, 4 eq) dropwise at -78 °C. The reaction was stirred for 45 min, allowed to warm to 0 °C, quenched by addition of a saturated solution of ammonium chloride and extracted three times with ethyl acetate. The organic layers were washed with brine, dried over sodium sulfate, filtered and concentrated in vacuo. The residue was purified by flash chromatography over silica gel using a gradient ethylacetate/heptane 0-50% to provide the title compound as a yellow oil (353.5 mg, 59 % yield). MS (ESI): m/z= 195.0 [M+H]⁺

### Step 2: 6-chloro-7-(dimethylamino)-1H-indole-3-sulfonyl chloride

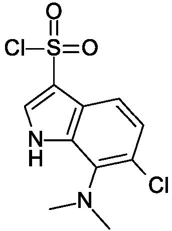

To a solution of 6-chloro-N,N-dimethyl-1H-indol-7-amine (100 mg, 0.514 mmol) in acetonitrile (2 mL) was added chlorosulfonic acid (119.72 mg, 68.8 uL, 1.03 mmol) dropwise at 0 °C. The reaction was stirred at 0 °C for 2 hours, at room temperature for 1 hour, at 60 °C for 2 hours and then at 80 °C overnight. Phosphorus oxychloride (329 mg, 200 uL, 2.15 mmol) was added dropwise at room temperature. The reaction mixture was stirred at 60 °C overnight and cooled to room temperature. Phosphorus oxychloride (329 mg, 200 uL, 2.15 mmol) was added. The reaction mixture was stirred at 80 °C for 2 days, poured into ice/ethylacetate and extracted twice with ethyl acetate. The organic layers were dried over sodium sulfate, filtered and concentrated in vacuo to provide the title compound as a dark brown solid (150 mg, 100 % yield). MS (ESI): m/z= 293.0 [M+H]⁺

### Intermediate A46: 1H-pyrrolo[3,2-h]quinoline-3-sulfonyl chloride

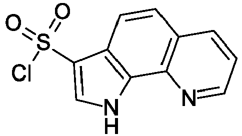

The intermediate A46 is known and has been prepared following procedures described in WO2018122232 from 1H-pyrrolo[3,2-h]quinoline (CAS: 233-88-5).

### Intermediate A47: 6-methyl-1H-pyrrolo[2,3-b]pyridine-3-sulfonyl chloride

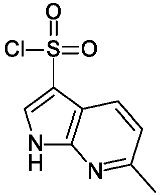

To a solution of 6-methyl-1H-pyrrolo[2,3-b]pyridine (0.500 g, 3.78 mmol, CAS: 824-51-1) in acetonitrile, extra dry (12.5 mL) was added chlorosulfonic acid (1.43 g, 823.39 uL, 12.3 mmol) dropwise at 0 °C. The mixture was stirred at 0 °C for 1 hour and at room temperature for 2 hours. Phosphorus oxychloride (2.55 g, 1.55 mL, 16.65 mmol) was added and the mixture was stirred at 70 °C overnight. The mixture was dropped into ice/ethyl acetate and extracted three times with ethyl acetate. The organic layers were dried over sodium sulfate, filtered and concentrated in vacuo. The residue was purified by flash chromatography over silica gel using a gradient ethylacetate/heptane 0-70% to provide the title compound as a white powder (642 mg, 66 % yield). MS (ESI): m/z= 231.0 [M+H]⁺

### Intermediate A48: 7-chloro-6-fluoro-1H-indole-3-sulfonyl chloride

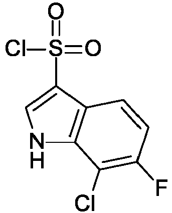

The intermediate A48 is known and has been prepared following procedures described in WO2018122232 from 7-chloro-6-fluoro-1H-indole (CAS: 259860-04-3).

### Intermediate A49: 7-bromo-6-fluoro-1H-indole-3-sulfonyl chloride

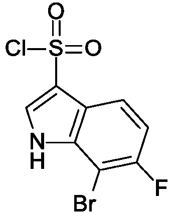

To a solution of 7-bromo-6-fluoro-1H-indole (0.250 g, 1.17 mmol, CAS: 1000339-62-7) in acetonitrile, extra dry (4 mL) was added chlorosulfonic acid (442.33 mg, 254.21 uL, 3.25 mmol) dropwise at 0 °C. The mixture was stirred at 0 °C for 1 hour, at room temperature for 2 hours, poured into ice/ethyl acetate and extracted three times with ethyl acetate. The organic layers were dried over sodium sulfate, filtered and concentrated in vacuo to provide the title compound as an off-white solid (304 mg, 83 % yield). MS (ESI): m/z= 312.0 [M+H]⁺

### Intermediate A50: 7-bromo-1H-indole-3-sulfonyl chloride

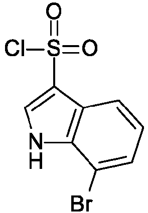

Intermediate A50 is commercial (CAS: 2137914-27-1)

### Intermediate A51: 6-chloro-7-morpholino-1H-indole-3-sulfonyl chloride

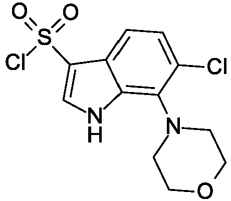

### Step 1: 4-(2-chloro-6-nitro-phenyl)morpholine

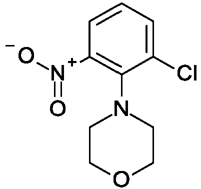

To a solution of morpholine (744.45 mg, 744.45 uL, 8.55 mmolq) and triethylamine (1.15 g, 1.59 mL, 11.39 mmol) in tetrahydrofuran (20 mL) was added 1-chloro-2-fluoro-3-nitro-benzene (1 g, 666.67 uL, 5.7 mmol, CAS: 2106-49-2) at room temperature. The reaction mixture was then heated to 60 °C and stirred overnight. Morpholine (248.15 mg, 248.15 uL, 2.85 mmol) was added again. The mixture was heated to reflux and after a few hours, morpholine (248.15 mg, 248.15 uL, 2.85 mmol) and triethylamine (1.15 g, 1.59 mL, 11.39 mmol) were added again. The reaction was stirred at 70 °C overnight, cooled to room temperature, diluted with water and extracted three times with dichloromethane. The organic layers were dried over sodium sulfate, filtered and concentrated in vacuo to provide the title compound as an orange solid (1.36 g, 93 % yield). MS (ESI): m/z= 243.0 [M+H]⁺

### Step 2: 4-(6-chloro-1H-indol-7-yl)morpholine

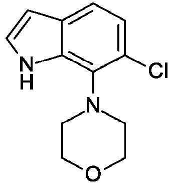

To a yellow solution of 4-(2-chloro-6-nitro-phenyl)morpholine (1.27 g, 5.22 mmol) in tetrahydrofuran, extra dry (20 mL) was added 1 M vinylmagnesium bromide solution in THF (20.49 g, 20.89 mL, 20.89 mmol) dropwise at -78 °C. After 1 hour, the mixture was allowed to warm to 0 °C and quenched with a saturated solution of ammonium chloride. The aqueous layer was extracted three times with ethyl acetate. The organic layers were dried over sodium sulfate, filtered and concentrated in vacuo. The residue was purified by flash chromatography over silica gel using a gradient ethylacetate/heptane 0-70%, followed by flash chromatography on a C18 RediSepRf Gold column using a gradient acetonitrile/water 10-100% to provide the title compound as a brown solid (120 mg, 9 % yield). MS (ESI): m/z= 237.1 [M+H]⁺

### Step 3: 6-chloro-7-morpholino-1H-indole-3-sulfonyl chloride

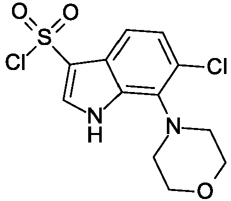

To a stirred solution of 4-(6-chloro-1H-indol-7-yl)morpholine (120 mg, 0.492 mmol) in acetonitrile, extra dry (2.4 mL) was added chlorosulfonic acid (143.25 mg, 82.33 uL, 1.23 mmol) dropwise at 0 °C. After 30 min, the reaction was allowed to warm up to room temperature. Phosphorus oxychloride (301.61 mg, 183.35 uL, 1.97 mmol) was added dropwise at room temperature. The mixture was heated to 70 °C, stirred overnight, cooled to room temperature, poured into ice/ethyl acetate and extracted three times with ethyl acetate. The organic layers were dried over sodium sulfate, filtered and concentrated in vacuo to provide the title compound as a dark red waxy solid (164.8 mg, 100 % yield). MS (ESI): m/z= 335.0 [M+H]⁺

### Intermediate A52: methyl 3-chlorosulfonyl-6-methyl-1H-indole-7-carboxylate

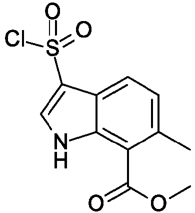

### Step 1: methyl 6-methyl-1H-indole-7-carboxylate

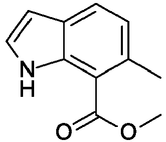

In a reaction vessel, 7-bromo-6-methyl-1H-indole (370 mg, 1.76 mmol, CAS: 1360885-93-3), [1,1'-bis(diphenylphosphino)ferrocene]dichloropalladium(II) (4.32 mg, 0.005 mol), 1,1'-bis(diphenylphosphino)ferrocene (8.95 mg, 0.016 mmol) and triethylamine (178.23 mg, 245 µl, 1.76 mmol) were combined with methanol (7.4 ml). The reaction vessel was sealed and flushed with carbon monoxide (5 x 10 atm), press on carbon monoxide (40 atm), placed in a shaker, heated to 130 °C and shaken for 48 hours. The procedure was repeated two times. The reaction mixture was concentrated in vacuo and the residue was purified by flash chromatography on silica gel using a gradient ethylacetate/heptane to provide the title compound as a yellow solid (210 mg, 49 % yield). MS (ESI): m/z= 190.1 [M+H]⁺

### Step 2: methyl 3-chlorosulfonyl-6-methyl-1H-indole-7-carboxylate

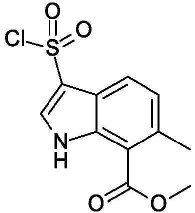

To a solution of methyl 6-methyl-1H-indole-7-carboxylate (187 mg, 0.939 mmol) in acetonitrile (6 mL) was added chlorosulfonic acid (287.33 mg, 164.19 uL, 2.44 mmol) at 0 °C. The mixture was stirred at 0 °C for 1 hour, at room temperature for 30 min, poured into ice/ethyl acetate and extracted twice with ethyl acetate. The organic layers were dried over sodium sulfate, filtered and concentrated in vacuo to provide the title compound as an orange solid (221 mg, 82 % yield). MS (ESI): m/z= 285.9 [M-H]⁻

### Intermediate A53: 8-methyl-9-oxo-6,7-dihydro-1H-pyrrolo[3,2-h]isoquinoline-3-sulfonyl chloride

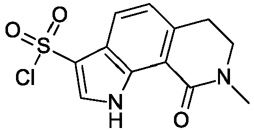

### Step 1: methyl 2-[2-(tert-butoxycarbonylamino)ethyl]-6-nitro-benzoate

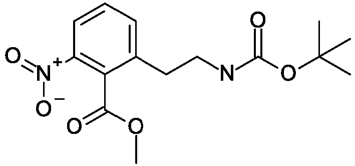

A mixture of 2-bromo-6-nitro-benzoic acid methyl ester (1.5 g, 5.65 mmol, CAS: 135484-76-3), potassium (2-((tert-butoxycarbonyl)amino)ethyl)trifluoroborate (2.24 g, 8.48 mmol), cesium carbonate (6.6 g, 20.07 mmol), palladium(II) acetate (76.15 mg, 0.339 mmol) and RuPhos (333.21 mg, 0.678 mmol) in toluene (30 mL) and water (10 mL) was heated to 95 °C and stirred for 15 hours. The mixture was poured into water and extracted twice with ethyl acetate. The organic layers were dried over sodium sulfate, filtered and concentrated in vacuo. The residue was purified by flash chromatography over silica gel using a gradient ethylacetate/heptane 0-40% to provide the title compound as a yellow viscous oil (1.69 g, 87 % yield). MS (ESI): m/z= 225.1 [M+H-BOC]⁺

### Step 2: 8-nitro-3,4-dihydro-2H-isoquinolin-1-one

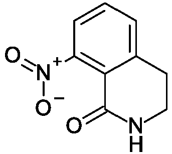

To a stirred solution of methyl 2-[2-(tert-butoxycarbonylamino)ethyl]-6-nitro-benzoate (1.69 g, 4.9 mmol) in dichloromethane (15 mL) was added trifluoroacetic acid (5.58 g, 3.75 mL, 48.98 mmol). The reaction mixture was stirred at room temperature for 30 min and concentrated in vacuo. The residue was diluted with 1,4-dioxane (40 mL) and n-ethyldiisopropylamine (6.46 g, 8.54 mL, 48.98 mmol) was added. The reaction mixture was heated to 80 °C, stirred for 15 hours and concentrated in vacuo. The residue was purified by flash chromatography over silica gel using a gradient ethylacetate/heptane 0-100% to provide the title compound as an off-white solid (743 mg, 79 % yield). MS (ESI): m/z= 193.0 [M+H]⁺

### Step 3: 2-methyl-8-nitro-3,4-dihydroisoquinolin-1-one

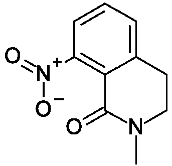

A solution of 8-nitro-3,4-dihydro-2H-isoquinolin-1-one (741 mg, 3.86 mmol) in N,N-dimethylformamide (20 mL) was cooled to 0 °C. Sodium hydride (169.65 mg, 4.24 mmol) was added. After stirring at 0 °C for 30 min, iodomethane (656.77 mg, 289.33 uL, 4.63 mmol) was added. The reaction mixture was stirred at 0 °C for 30 min, quenched with a saturated solution of ammonium chloride, poured into brine and extracted twice with ethyl acetate. The organic layers were dried over sodium sulfate, filtered and concentrated in vacuo. The residue was purified by flash chromatography over silica gel using a gradient ethylacetate/heptane 0-100% to provide the title compound as an off-white solid (760 mg, 96 % yield). MS (ESI): m/z= 207.1 [M+H]⁺

### Step 4: 8-amino-2-methyl-3,4-dihydroisoquinolin-1-one

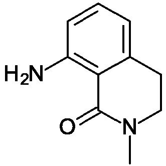

A suspension of 2-methyl-8-nitro-3,4-dihydroisoquinolin-1-one (758 mg, 3.68 mmol) in ethyl acetate (24 mL) was degassed with argon and palladium on activated charcoal (391.2 mg, 0.368 mmol) was added. The reaction mixture was stirred at room temperature under 1 atm. of hydrogen for 4 hours, filtered through a pad of celite and concentrated in vacuo to provide the title compound as an off-white solid (629 mg, 94 % yield). MS (ESI): m/z= 177.0 [M+H]⁺

### Step 5: 8-amino-5,7-diiodo-2-methyl-3,4-dihydroisoquinolin-1-one

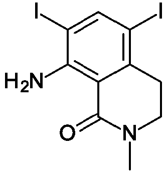

To a stirred solution of 8-amino-2-methyl-3,4-dihydroisoquinolin-1-one (627 mg, 3.45 mmol) and acetic acid (455.97 mg, 434.25 uL, 7.59 mmol) in toluene (10 mL) was added n-iodosuccinimide (1.76 g, 7.59 mmol). The reaction mixture was stirred at room temperature for 15 hours, diluted with ethyl acetate and washed once with water. The organic layer was dried over sodium sulfate, filtered and concentrated in vacuo. The residue was purified by flash chromatography over silica gel using a gradient ethylacetate/heptane 0-20% to provide the title compound as a light yellow solid (1.41 g, 95 % yield). MS (ESI): m/z= 428.9 [M+H]⁺

### Step 6: 5-iodo-8-methyl-2-trimethylsilyl-6,7-dihydro-1H-pyrrolo[3,2-h]isoquinolin-9-one

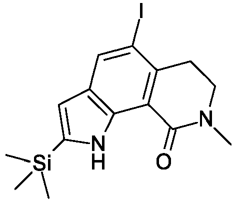

In a sealed tube, a mixture of 8-amino-5,7-diiodo-2-methyl-3,4-dihydroisoquinolin-1-one (1.4 g, 3.27 mmol), potassium carbonate (913.26 mg, 6.54 mmol), tetrakis(triphenylphosphine)palladium (381.8 mg, 0.327 mmol), XPhos (321.51 mg, 0.654 mmol) and trimethylsilylacetylene (393.4 mg, 554.86 uL, 3.93 mmol) in tetrahydrofuran (34 mL) was heated to 75 °C and stirred for 15 hours. The reaction mixture was poured into water and extracted twice with ethyl acetate. The organic layers were dried over sodium sulfate, filtered and concentrated in vacuo. The residue was purified by flash chromatography over silica gel using a gradient ethylacetate/heptane 0-20% to provide the title compound as a yellow viscous oil (552 mg, 40 % yield). MS (ESI): m/z= 399.0 [M+H]⁺

### Step 7: 5-iodo-8-methyl-6,7-dihydro-1H-pyrrolo[3,2-h]isoquinolin-9-one

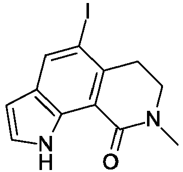

To a stirred solution of 5-iodo-8-methyl-2-trimethylsilyl-6,7-dihydro-1H-pyrrol[3,2-h]isoquinolin-9-one (550 mg, 1.31 mmol) in tetrahydrofuran (8 mL) was added 1 M tetrabutylammonium fluoride solution in THF (6.56 mL, 6.56 mmol) at room temperature. The reaction mixture was heated to 50 °C, stirred for 1 hour, poured into sat. NaHCO3 and extracted twice with ethyl acetate. The organic layers were dried over sodium sulfate, filtered and concentrated in vacuo. The residue was purified by flash chromatography over silica gel using a gradient ethylacetate/heptane 0-40% to provide the title compound as a yellow solid (359 mg, 76 % yield). MS (ESI): m/z= 327.0 [M+H]⁺

### Step 8: 8-methyl-6,7-dihydro-1H-pyrrolo[3,2-h]isoquinolin-9-one

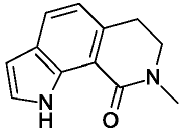

A suspension of 5-iodo-8-methyl-6,7-dihydro-1H-pyrrol[3,2-h]isoquinolin-9-one (357 mg, 0.985 mmol) and triethylamine (109.66 mg, 151.05 uL, 1.08 mmol) in ethanol (15 mL) was degassed with argon and palladium on activated charcoal (104.84 mg, 0.099 mmol) was added. The reaction mixture was stirred at room temperature under 1 atm. of hydrogen for 3.5 hours, filtered through a pad of celite and concentrated in vacuo. The residue was purified by flash chromatography over silica gel using a gradient ethylacetate/heptane 0-60%, followed by flash chromatography on a C18 RediSepRf Gold column using a gradient acetonitrile/water 10-60% to provide the title compound as a white solid (150 mg, 76 % yield). MS (ESI): m/z= 201.1 [M+H]⁺

### Step 9: 8-methyl-9-oxo-6,7-dihydro-1H-pyrrolo[3,2-h]isoquinoline-3-sulfonyl chloride

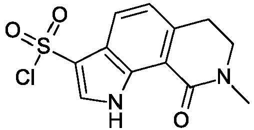

A solution of 8-methyl-6,7-dihydro-1H-pyrrol[3,2-h]isoquinolin-9-one (114 mg, 0.569 mmol) in acetonitrile (5 mL) was cooled to 0 °C. Chlorosulfonic acid (174.22 mg, 99.39 uL, 1.48 mmol) was added and the reaction mixture was stirred at 0 °C for 30 min. Then phosphorus oxychloride (436.47 mg, 264.53 uL, 2.85 mmol) was added. The reaction mixture was heated to 70 °C and stirred for 5.5 hours. The reaction mixture was poured into ice/ethyl acetate and extracted twice with ethyl acetate. The organic layers were dried over sodium sulfate, filtered and concentrated in vacuo to provide the title compound as a light red solid (232 mg, 100 % yield). MS (ESI): m/z= 298.9 [M+H]⁺

### Intermediate A54: 6-chloro-7-(cyanomethoxy)-1H-indole-3-sulfonyl chloride

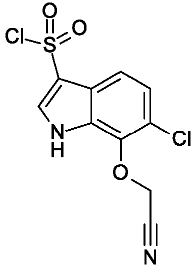

### Step 1: 2-benzyloxy-1-chloro-3-nitro-benzene

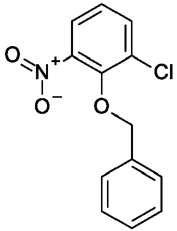

To a mixture of 2-chloro-6-nitrophenol (10 g, 57.6 mmol, CAS: 603-86-1) and potassium carbonate (15.927 g, 115.2 mmol) in dry acetonitrile (150 mL) was added benzyl bromide (9.85 g, 6.85 mL, 57.6 mmol). The reaction mixture was stirred at 70 °C overnight, poured into water and extracted twice with ethyl acetate. The organic layers were washed with brine, dried over sodium sulfate, filtered and concentrated in vacuo to provide the title compound as a brown waxy solid (15 g, 89 % yield), which was directly used in the next step without further purification.

### Step 2: 7-benzyloxy-6-chloro-1H-indole

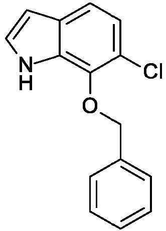

A stirred solution of vinylmagnesium chloride (284.44 mL, 284.44 mmol) in dry tetrahydrofuran (500 mL) was cooled to -40 °C and 2-benzyloxy-1-chloro-3-nitro-benzene (15.0 g, 56.89 mmol) was added. The reaction mixture was stirred at this temperature for 30 min, quenched with a saturated solution of ammonium chloride and extracted twice with ethyl acetate. The organic layers were washed with brine, dried over sodium sulfate, filtered and concentrated in vacuo. The residue was purified by flash chromatography over silica gel to provide the title compound as a light brown waxy solid (5.5 g, 36 % yield). 1H NMR (500 MHz, DMSO-d6) δ = 11.44 (br s, 1H), 7.59 (br d, J = 7.3 Hz, 2H), 7.44 - 7.30 (m, 5H), 7.01 (dd, J = 2.1, 8.4 Hz, 1H), 6.54 - 6.41 (m, 1H), 5.11 (s, 2H)

### Step 3: 6-chloro-1H-indol-7-ol

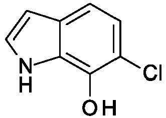

A solution of 7-benzyloxy-6-chloro-1H-indole (4.0 g, 15.52 mmol) in dry ethyl acetate (100 mL) was degassed with argon and palladium on activated charcoal (350 mg) was added. The reaction mixture was stirred at room temperature under 1 atm. of hydrogen overnight, filtered through a pad of celite and concentrated in vacuo to provide the title compound as a brown oil (3 g, 98 % yield). MS (ESI): m/z= 168.0 [M+H]⁺

### Step 4: 2-[(6-chloro-1H-indol-7-yl)oxy]acetonitrile

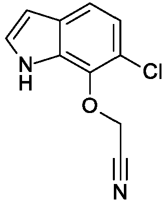

To a stirred solution of 6-chloro-1H-indol-7-ol (4.0 g, 20.29 mmol) and potassium carbonate (5.61 g, 40.58 mmol) in dry acetonitrile (40 mL) was added bromoacetonitrile (2.68 g, 22.32 mmol). The reaction mixture was stirred at room temperature overnight. The precipitate was filtered off. The filtrate was concentrated in vacuo. The residue was diluted with water and extracted twice with ethyl acetate. The organic layers were washed with brine, dried over sodium sulfate, filtered and concentrated in vacuo. The residue was purified by flash chromatography over silica gel using a gradient TBME/hexane to provide the title compound as a light brown solid (1.9 g, 45 % yield). MS (ESI): m/z= 207.0 [M+H]⁺

### Step 5: 6-chloro-7-(cyanomethoxy)-1H-indole-3-sulfonyl chloride

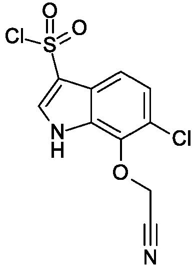

A solution of 2-[(6-chloro-1H-indol-7-yl)oxy]acetonitrile (100 mg, 0.484 mmol) in acetonitrile (4 mL) was cooled to 0 °C. Chlorosulfonic acid (148.1 mg, 84.48 uL, 1.26 mmol) was added dropwise. The reaction mixture was stirred at 0 °C for 1 hour, at room temperature for 16 hours, poured into ice/ethyl acetate and extracted twice with ethyl acetate. The organic layers were dried over sodium sulfate, filtered and concentrated in vacuo to provide the title compound as a light red gum (123 mg, 83 % yield). MS (ESI): m/z= 302.9 [M-H]⁻

### Intermediates B

### Intermediate B1: 5-bromo-4-methoxy-pyrimidin-2-amine

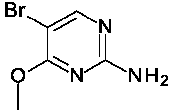

Intermediate B1 is commercial (CAS: 36082-45-8)

### Intermediate B2: 2-amino-4-methoxy-pyrimidine-5-carbonitrile

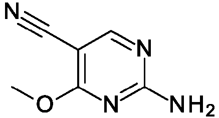

Intermediate B2 is commercial (CAS: 81066-95-7)

### Intermediate B3: 5-(2-fluoroethoxy)-4-methoxy-pyrimidin-2-amine

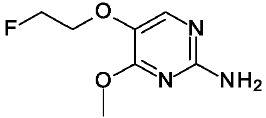

### Step 1: (5-bromo-4-methoxy-pyrimidin-2-yl)-bis(p-anisyl)amine

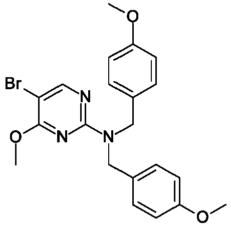

A suspension of 5-bromo-2-chloro-4-methoxy-pyrimidine (1.02 g, 4.48 mmol, CAS: 57054-92-9), bis(p-anisyl)amine (1.29 g, 4.92 mmol) and n-ethyldiisopropylamine (858 uL, 4.92 mmol) in acetonitrile (20 mL) was heated at 70 °C for 2 days. The resulting solution was poured into a saturated aqueous sodium bicarbonate solution and extracted twice with ethylacetate. The organic layers were dried over sodium sulfate, filtered and dried in vacuo. The residue was purified by flash chromatography over silica gel using a gradient ethylacetate/heptane 0-20% to provide the title compound as a colorless viscous oil (998 mg, 50 % yield). MS (ESI): m/z= 446.2 [M+H]⁺

### Step 2: [4-methoxy-5-(4,4,5,5-tetramethyl-1,3,2-dioxaborolan-2-yl)pyrimidin-2-yl]-bis(p-anisyl)amine

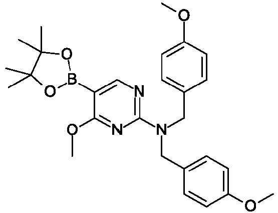

A suspension of (5-bromo-4-methoxy-pyrimidin-2-yl)-bis(p-anisyl)amine (500 mg, 1.13 mmol), bis(pinacolato)diboron (354 mg, 1.35 mmol) and potassium acetate (335 mg, 3.38 mmol) in 1,4-dioxane (10 mL) was purged with argon for 5 min. dichloro[1,1'-bis(diphenylphosphino)ferrocene]palladium(ii) dichloromethane adduct (91.9 mg, 0.113 mmol) was added. The reaction mixture was heated to 90 °C and stirred for 16 hours. The resulting dark suspension was poured into ethylacetate and washed once with sat. NaCl. The organic layer was dried over sodium sulfate, filtered and concentrated in vacuo. The residue was purified by column chromatography on silica gel using a gradient ethylacetate/heptane 0-30% to provide the title compound as a colorless viscous oil (157 mg, 29 % yield). MS (ESI): m/z= 492.4 [M+H]+

### Step 3: 2-[bis(p-anisyl)amino]-4-methoxy-pyrimidin-5-ol

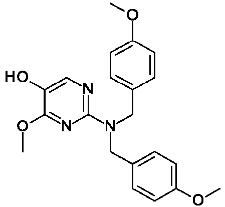

A solution of [4-methoxy-5-(4,4,5,5-tetramethyl-1,3,2-dioxaborolan-2-yl)pyrimidin-2-yl]-bis(p-anisyl)amine (130 mg, 0.265 mmol) in tetrahydrofuran (2.5 mL) was cooled to 0°C. Hydrogen peroxide 35% (500 uL, 5.71 mmol) was added. The reaction mixture was stirred at 0 °C for 15 min, allowed to warm to rt and stirred for 3 hours. The reaction mixture was poured into cold 0.1 N sodiumsulfite solution and extracted twice with EtOAc. The organic layers were washed twice with brine, dried over sodiumsulfate, filtered and concentrated in vacuo to provide the title compound as a light yellow viscous oil (103 mg, 100 % yield). MS (ESI): m/z= 382.3 [M+H]+

### Step 4: [5-(2-fluoroethoxy)-4-methoxy-pyrimidin-2-yl]-bis(p-anisyl)amine

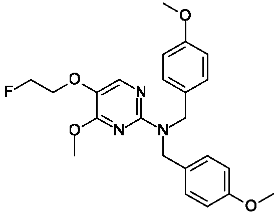

A suspension of 2-[bis(p-anisyl)amino]-4-methoxy-pyrimidin-5-ol (100 mg, 0.236 mmol), potassium carbonate (98.82 mg, 0.708 mmol) and 1-bromo-2-fluoroethane (61.14 mg, 35.75 uL, 0.472 mmol) in acetonitrile (2.5 mL) was stirred at room temperature for 15 min and at 80°C for 6 hours. The reaction mixture was poured into water and extracted twice with EtOAc. The organic layers were dried over sodium sulfate, filtered and concentrated in vacuo. The residue was purified by flash chromatography on silica gel using a gradient ethylacetate/heptane 0-30% to provide the title compound as a colorless viscous oil (22 mg, 22 % yield). MS (ESI): m/z= 428.3 [M+H]+

### Step 5: [5-(2-fluoroethoxy)-4-methoxy-pyrimidin-2-yl]amine

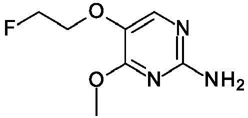

A solution of [5-(2-fluoroethoxy)-4-methoxy-pyrimidin-2-yl]-bis(p-anisyl)amine (87 mg, 0.204 mmol) in dichloromethane (500 uL) was cooled to 0 °C. Trifluoroacetic acid (1.41 g, 944.56 uL, 12.21 mmol) was added. The reaction mixture was allowed to warm to room temperature and stirred for 16 hours and at 55°C for two additional hours. The resulting purple solution was poured into a sat. aqueous sodium bicarbonate solution and extracted twice with EtOAc. The organic layers were dried over sodium sulfate, filtered and concentrated in vacuo. The residue was purified by flash chromatography on silica gel using a gradient ethylacetate/heptane 0-100% to provide the title compound as an off-white solid (27 mg, 71 % yield). MS (ESI): m/z= 188.1 [M+H]+

### Intermediate B4-B7

The intermediates B4-B7 have been prepared in analogy to intermediate B3, by alkylation of 2-[bis(p-anisyl)amino]-4-methoxy-pyrimidin-5-ol (intermediate B3, step 3) using the base and alkylating agent indicated in the following table, followed by deprotection of the bis(p-anisyl) protecting group with TFA (intermediate B3, step 5):

| Int. number | Structure | Name | Alkylating agent | base | MS (ESI): m/z, [M+H] ⁺ |
|---|---|---|---|---|---|
| B4 | 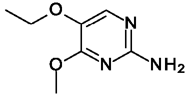 | (5-ethoxy-4-methoxy-pyrimidin-2-yl)amine | Bromoetha ne | K₂CO₃ | 170.2 |
| B5 | 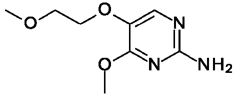 | [4-methoxy-5-(2-methoxyethoxy)py rimidin-2-yl]amine | 1-Bromo-2-methoxyethane | K₂CO₃ | 200.2 |
| B6 | 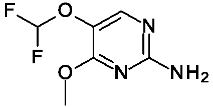 | [5-(difluoromethoxy)-4-methoxy-pyrimidin-2-yl]amine | Sodium chlorodiflu oroacetate | Cs₂CO₃ | 192.1 |
| B7 | 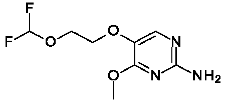 | 5-[2-(difluoromethoxy) ethoxy]-4-methoxy-pyrimidin-2-amine | 1-bromo-2-(difluorom ethoxy)etha ne | K₂CO₃ | 236.2 |

### Intermediate B8: (4-methoxy-5-prop-1-ynyl-pyrimidin-2-yl)amine

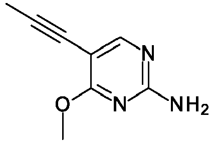

### Step 1: (4-methoxy-5-prop-1-ynyl-pyrimidin-2-yl)-bis(p-anisyl)amine

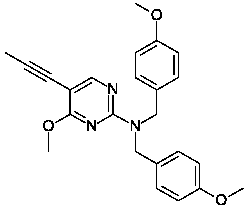

(5-bromo-4-methoxy-pyrimidin-2-yl)-bis(p-anisyl)amine (500 mg, 1.13 mmol, intermediate B3, step 1) was dissolved in N,N-dimethylformamide (5 mL) and triethylamine (455.48 mg, 627.39 uL, 4.5 mmol) was added at room temperature. The reaction mixture was purged with argon and 1 M prop-1-yne 1M in DMF (1.35 mL, 1.35 mmol), bis(triphenylphosphine)palladium (II) chloride (78.99 mg, 0.113 mmol) and copper (I) iodide (32.15 mg, 0.169 mmol) were added. The mixture was stirred at 50°C for 72 hr. The reaction mixture was diluted with a saturated sodium bicarbonate solution and extracted two times with EtOAc. The organic layers were washed with water and brine, dried over sodium sulfate and concentrated in vacuo. The residue was purified by flash chromatography over silica gel using a gradient ethylacetate/heptane 0-100% to provide the title compound as a light yellow oil (130 mg, 24 % yield). MS (ESI): m/z= 404.2 [M+H]+

### Step 2: (4-methoxy-5-prop-1-ynyl-pyrimidin-2-yl)amine

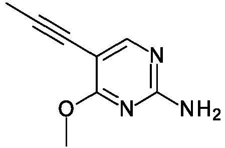

The title compound was prepared in analogy to intermediate B3 step 5 from (4-methoxy-5-prop-1-ynyl-pyrimidin-2-yl)-bis(p-anisyl)amine as a light yellow solid. MS (ESI): m/z= 164.1 [M+H]⁺

### Intermediate B9: (5-ethyl-4-methoxy-pyrimidin-2-yl)amine

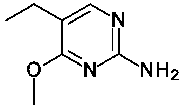

### Step 1: 2-chloro-5-ethyl-4-methoxy-pyrimidine

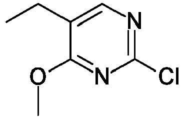

To a suspension of 2,4-dichloro-5-ethyl-pyrimidine (274.3 mg, 1.55 mmol, CAS: 34171-40-9) in MeOH (7 mL) was added NaH (65.07 mg, 1.63 mmol, 1.05 eq) at RT. The reaction mixture was stirred at room temperature for 1 hour, the solvent was removed under reduced pressure. The residue was dissolved in EtOAC and extracted 3 times. The combined organic layers were washed 3 times with brine, dried over sodium sulfate, filtered and concentrated in vacuo. The residue was purified by flash chromatography over silica gel using a gradient ethylacetate/heptane 0-50% to provide the title compound as a colorless oil (154 mg, 57 % yield). MS (ESI): m/z= 173.1 [M+H]⁺

### Step 2: (5-ethyl-4-methoxy-pyrimidin-2-yl)amine

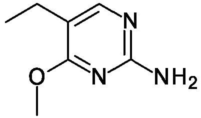

In an autoclave was added in an argon amosphere under a glove box, 2-chloro-5-ethyl-4-methoxy-pyrimidine (130.4 mg, 0.77 mmol), sodium tert-butoxide (82.33 mg, 0.8 mmol) and catalyst [Pd(Allyl)(tBuBrettPhos)]OTf; (11.8 mg, 0.01.5 mmol) and 1,4-dioxane (3.26 mL). The autoclave was closed and ammonia was introduced (15 mmol) at room temperature using a ammonia gas bomb. The reaction mixture stirred at 100°C for 20 hours The reaction mixture was filtered and washed with EtOAc, the solvent was removed under vacuo. The residue was purified by flash chromatography over silica gel using a gradient ethylacetate/heptane 30-100% to provide the title compound as a white powder (78 mg, 64 % yield). MS (ESI): m/z= 154.1 [M+H]⁺

### Intermediate B10: (4-methoxy-5-methyl-pyrimidin-2-yl)amine

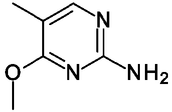

The title compound was prepared in analogy to intermediate B9 step 2 from 2-chloro-4-methoxy-5-methyl-pyrimidine as a white solid. MS (ESI): m/z= 140.1 [M+H]⁺

### Intermediate B11: (5-cyclopropyl-4-methoxy-pyrimidin-2-yl)amine

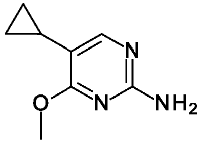

### Step 1: 5-cyclopropyl-4-methoxy-N,N-bis[(4-methoxyphenyl)methyl]pyrimidin-2-amine

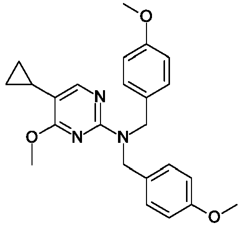

In a glastube, a solution of (5-bromo-4-methoxy-pyrimidin-2-yl)-bis(p-anisyl)amine (500 mg, 1.13 mmol, intermediate B3, step 1), 2-cyclopropyl-4,4,5,5-tetramethyl-1,3,2-dioxaborolane (945.49 mg, 5.63 mmol) and tripotassium phosphate (716.61 mg, 3.38 mmol) in 1,4-dioxane (9.18 mL) and water (2.3 mL) was degassed under argon. 1,1'-bis(diphenylphosphino)ferrocene-palladium(II)dichloride dichloromethane complex (93.03 mg, 0.113 mmol) was added and the mixture was degassed with argon. The reaction mixture was stirred at 80°C for 1 hr. The reaction mixture was diluted with a saturated sat. sodium bicarbonate solution and extracted two times with EtOAc. The organic layers were washed with water and brine, dried over sodium sulfate and concentrated in vacuo. The residue was purified by flash chromatography over silica gel using a gradient ethylacetate/heptane 0-50% to provide the title compound as a colorless oil (417 mg, 91 % yield). MS (ESI): m/z= 406.3 [M+H]⁺

### Step 2: (5-cyclopropyl-4-methoxy-pyrimidin-2-yl)amine

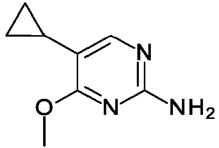

The title compound was prepared in analogy to intermediate B3 step 5 from 5-cyclopropyl-4-methoxy-N,N-bis[(4-methoxyphenyl)methyl]pyrimidin-2-amine as a white solid. MS (ESI): m/z= 166.1 [M+H]⁺

### Intermediate B12: (5-chloro-4,6-dimethoxy-pyrimidin-2-yl)amine

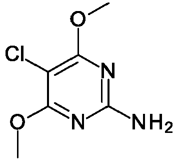

To a stirred solution of (4,6-dimethoxypyrimidin-2-yl)amine (100 mg, 0.632 mmol, CAS: 36315-01-2) in acetonitrile (2 mL) was added N-chlorosuccinimide (109.65 mg, 0.821 mmol) at room temperature. The reaction mixture was stirred at room temperature and the obtained light yellow solution was diluted with EtOAc and washed with water. The organic layer was dried over sodium sulfate, filtered and concentrated in vacuo. The residue was purified by flash chromatography over silica gel using a gradient ethylacetate/heptane 0-50% to provide the title compound as an off-white solid (87 mg, 71 % yield). MS (ESI): m/z= 190.1 [M+H]+

### Intermediate B13: (5-bromo-4,6-dimethoxy-pyrimidin-2-yl)amine

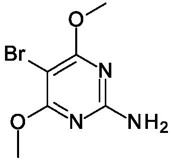

The title compound was prepared in analogy to intermediate B12 from (4,6-dimethoxypyrimidin-2-yl)amine (CAS: 36315-01-2) using N-bromosuccinimide as brominating agent. White solid. MS (ESI): m/z= 234.1 [M+H]⁺

### Intermediate B14: (5-bromo-4-ethoxy-6-methoxy-pyrimidin-2-yl)amine

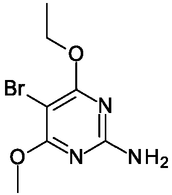

### Step 1: (5-bromo-4-chloro-6-methoxy-pyrimidin-2-yl)amine

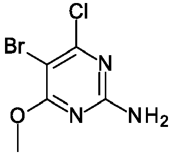

The title compound was prepared in analogy to intermediate B12 from (4-chloro-6-methoxy-pyrimidin-2-yl)amine (CAS: 5734-64-5) using N-bromosuccinimide as brominating agent. White solid. MS (ESI): m/z= 238.1 [M+H]⁺

### Step 2: (5-bromo-4-ethoxy-6-methoxy-pyrimidin-2-yl)amine

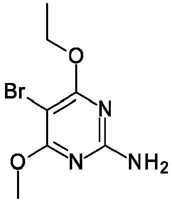

To a suspension of NaH (22.81 mg, 0.570 mmol) in 0.5 mL DMF was added ethanol (28.9 mg, 36.63 uL, 0.627 mmol) and stirred for 15 min at rt. To this reaction mixture a solution of (5-bromo-4-chloro-6-methoxy-pyrimidin-2-yl)amine (68 mg, 0.285 mmol) in 1 mL DMF was added. The resulting reaction mixture was warmed at 80°C. After 1 hour, the reaction mixture was cooled to room temperature, quenched with a saturated solution of ammonium chloride, extracted with ethylacetate. The combined organic layers were dried over sodium sulfate, and concentrated in vacuo. The residue was purified by flash chromatography over silica gel using a gradient ethylacetate/heptane 0-15% to provide the title compound as a white solid (30 mg, 30 % yield). MS (ESI): m/z= 248.1 [M+H]+

### Intermediate B15: 5-bromo-4-(2-fluoroethoxy)-6-methoxy-pyrimidin-2-amine

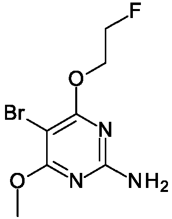

The title compound was prepared in analogy to intermediate B14 step 2 from (5-bromo-4-chloro-6-methoxy-pyrimidin-2-yl)amine using 2-fluroro-ethanol as alcohol and THF as solvent. White solid. 1H NMR (300 MHz, CHLOROFORM-d) δ = 4.86 - 4.72 (m, 3H), 4.69 - 4.58 (m, 2H), 4.55 - 4.48 (m, 1H), 3.94 (s, 3H); 19F NMR (282 MHz, CHLOROFORM-d) δ = -224.63 (s, 1F)

### Intermediate B16: [5-bromo-4-(difluoromethoxy)-6-methoxy-pyrimidin-2-yl]amine

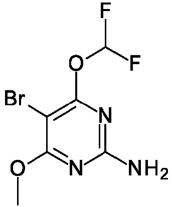

### Step 1: [4-(difluoromethoxy)-6-methoxy-pyrimidin-2-yl]amine

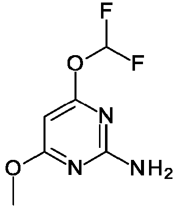

To a suspension of 2-amino-6-methoxy-pyrimidin-4-ol (20 mg, 0.135 mmol, CAS: 59081-28-6) in acetonitrile (1.5 mL) was added 5 M potassium hydroxide (538.51 uL, 2.69 mmol) dropwise at 0 °C, followed by dropwise addition of bromodifluoromethyl diethylphosphonate (71.89 mg, 47.83 uL, 0.269 mmol) in acetonitrile (200 uL) at 0 °C. After 15 min stirring at 0 °C, the reaction mixture was allowed to warm to room temperature and stirred for 2 hours. The resulting yellow biphasic solution was quenched with water and extracted twice with EtOAc. The organic layers were washed with water, dried over sodium sulfate, filtered and concentrated in vacuo. The residue was purified by flash chromatography over silica gel using a gradient ethylacetate/heptane 0-50% to provide the title compound as a white solid (8 mg, 31 % yield). MS (ESI): m/z= 192.1 [M+H]+

### Step 2: [5-bromo-4-(difluoromethoxy)-6-methoxy-pyrimidin-2-yl]amine

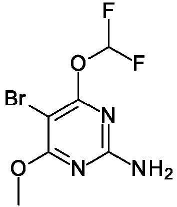

The title compound was prepared in analogy to intermediate B12 from [4-(difluoromethoxy)-6-methoxy-pyrimidin-2-yl]amine using N-bromosuccinimide as brominating agent. White solid. MS (ESI): m/z= 272.0 [M+H]+

### Intermediate B17: 5-(3-fluoropropyl)-4-methoxy-pyrimidin-2-amine

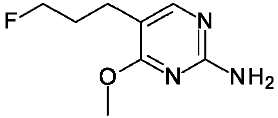

### Step 1: (E)-3-(2-amino-4-methoxy-pyrimidin-5-yl)acrylic acid ethyl ester

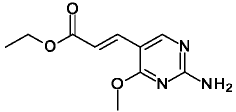

In a sealed tube, a mixture of (5-bromo-4-methoxy-pyrimidin-2-yl)amine (200 mg, 0.961 mmol, CAS: 36082-45-8), potassium tert-butylate (165. mg, 1.44 mmol), tetrakis(triphenylphosphine)palladium(0) (112.14 mg, 0.096 mmol) and (E)-3-(4,4,5,5-tetramethyl-1,3,2-dioxaborolan-2-yl)acrylic acid ethyl ester (260.63 mg, 258.05 uL, 1.15 mmol) in 1,4-dioxane (4 mL) and water (4 mL) was heated to 90 °C and stirred for 15 hours. The reaction mixture was poured into water and extracted twice with EtOAc. The organic layers were dried over sodium sulfate, filtered and concentrated in vacuo. The residue was purified by flash chromatography over silica gel using a gradient ethylacetate/heptane 0-50% to provide the title compound as a light yellow solid (43 mg, 20 % yield). MS (ESI): m/z= 224.2 [M+H]+

### Step 2: 3-(2-amino-4-methoxy-pyrimidin-5-yl)propionic acid ethyl ester

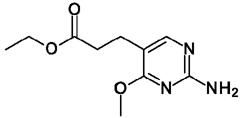

A suspension of (E)-3-(2-amino-4-methoxy-pyrimidin-5-yl)acrylic acid ethyl ester (41 mg, 0.184 mmol) in methanol (2 mL) was purged with argon for 5 min. Palladium on activated charcoal (19.55 mg, 0.018 mmol) was added. The reaction mixture was purged with hydrogen and stirred under 1 atm. of hydrogen at room temperature for 15 hours. The reaction mixture was filtered through a pad of celite and concentrated in vacuo to provide the title compound as a light grey solid (36 mg, 74 % yield). MS (ESI): m/z= 226.2 [M+H]+

### Step 3: 3-(2-amino-4-methoxy-pyrimidin-5-yl)propan-1-ol

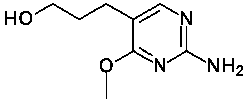

A solution of 3-(2-amino-4-methoxy-pyrimidin-5-yl)propionic acid ethyl ester (35 mg, 0.132 mmol) in tetrahydrofuran (400 uL) was cooled to 0 °C. A 1 M solution of LiAlH₄ in THF (264.16 uL, 0.264 mmol) was added dropwise. The reaction mixture was stirred at 0 °C for 1 hour. The reaction mixture was carefully quenched with a saturated solution of ammonium chloride and extracted twice with EtOAc. The organic layers were washed with brine, dried over sodium sulfate, filtered and concentrated in vacuo. The residue was purified by flash chromatography over silica gel using a gradient ethylacetate/heptane 0-100% the with 10% MeOH to provide the title compound as a white solid (11 mg, 46 % yield). MS (ESI): m/z= 184.1 [M+H]+

### Step 4: 5-(3-fluoropropyl)-4-methoxy-pyrimidin-2-amine

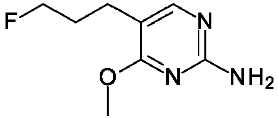

A suspension of 3-(2-amino-4-methoxy-pyrimidin-5-yl)propan-1-ol (70 mg, 0.352 mmol) in dichloromethane (1.38 mL) was cooled to 0°C. Deoxo-fluor ^{®}, 2.7M (50 wt.%) solution in toluene (622.15 mg, 518.46 uL, 1.41 mmol) was added dropwise. The reaction mixture was allowed to warm to room temperature and stirred for 3 days. The resulting yellow solution was diluted with EtOAc and carefully quenched with sat. NaHCO₃. The organic layer was separated, washed once with water, dried over sodium sulfate and concentrated in vacuo. The residue was purified by flash chromatography over silica gel using a gradient ethylacetate/heptane 0-100% to provide the title compound as a light yellow solid (9 mg, 14 % yield). MS (ESI): m/z= 186.1 [M+H]+

### Intermediate B18: (5-ethyl-4,6-dimethoxy-pyrimidin-2-yl)amine

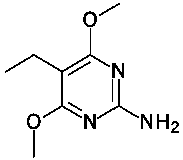

A suspension of (4,6-dichloro-5-ethyl-pyrimidin-2-yl)amine (100 mg, 0.521 mmol, CAS: 6343-68-6) and sodium methylate (296.11 mg, 5.21 mmol) in methanol (2.5 mL) was heated to 100 °C and stirred for 15 hours. Sodium methylate (148.04 mg, 2.6 mmol) was again added and the stirring was continued at 100 °C for 20 hours. The reaction mixture was concentrated in vacuo. The residue was poured into water and extracted twice with EtOAc. The organic layers were dried over sodium sulfate, filtered and concentrated in vacuo to provide the title compound as a white solid (88 mg, 90 % yield). MS (ESI): m/z= 183.8 [M+H]+

### Intermediate B19: (4,6-dimethoxy-5-methyl-pyrimidin-2-yl)amine

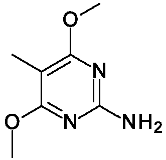

The title compound was prepared in analogy to intermediate B18 from (4,6-dichloro-5-methyl-pyrimidin-2-yl)amine (CAS: 7153-13-1) as a white solid. MS (ESI): m/z= 169.8 [M+H]+

### Intermediate B20: [5-(2,2-difluoroethoxy)-4-methoxy-6-methyl-pyrimidin-2-yl]amine

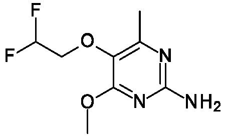

### Step 1: (5-bromo-4-methoxy-6-methyl-pyrimidin-2-yl)amine

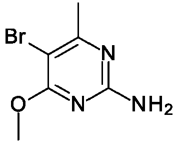

The title compound, a white solid, was prepared in analogy to intermediate B12 from (4-methoxy-6-methyl-pyrimidin-2-yl)amine (CAS: 7749-47-5) using N-bromosuccinimide as a brominating agent. MS (ESI): m/z=218.0 [M+H]+

### Step 2: (5-bromo-4-methoxy-6-methyl-pyrimidin-2-yl)amine

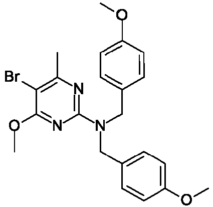

A solution of (5-bromo-4-methoxy-6-methyl-pyrimidin-2-yl)amine (700 mg, 3.21 mmol) in N,N-dimethylacetamide (8 mL) was cooled to 0 °C. Sodium hydride (385.2 mg, 9.63 mmol) was added portionwise (3x128 mg). The stirring was continued at 0 °C for 30 min. 4-methoxybenzyl chloride (1.03 g, 884.52 uL, 6.42 mmol) was added dropwise. The reaction mixture was allowed to warm to room temperature and stirred for 1.5 hours. The reaction mixture was carefully quenched with sat. ammonium chloride solution, poured into water and extracted twice with EtOAc. The organic layers were dried over sodium sulfate, filtered and concentrated in vacuo. The residue was purified by flash chromatography over silica gel using a gradient ethylacetate/heptane 0-20% to provide the title compound as a white solid (1.24 g, 84 % yield). MS (ESI): m/z= 460.2 [M+H]+

### Step 3: 2-[bis(p-anisyl)amino]-4-methoxy-6-methyl-pyrimidin-5-ol

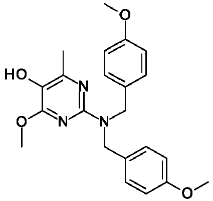

To a colorless solution of (5-bromo-4-methoxy-6-methyl-pyrimidin-2-yl)-bis(p-anisyl)amine (290 mg, 0.633 mmol) in tetrahydrofuran (2 mL) was added 1.6 M n-butyllithium in hexanes (442.1 mg, 514.07 uL, 0.823 mmol) dropwise at -78 °C. The resulting yellow solution was stirred at -78 °C for 30 min. Trimethyl borate (98.62 mg, 105.81 uL, 0.949 mmol) was added dropwise and the stirring was continued at -78 °C for 1.5 hour. The reaction mixture was allowed to warm to 0 °C and acetic acid (75.99 mg, 72.42 uL, 1.27 mmol) was added dropwise, followed by hydrogen peroxide 35% (92.23 mg, 83.09 uL, 0.949 mmol). The stirring was continued at 0 °C for 45 min. The resulting light yellow suspension was poured into 0.1 N sodium sulfite and extracted twice with EtOAc. The organic layers were washed twice with brine, dried over sodium sulfate, filtered and concentrated in vacuo. The residue was purified by flash chromatography over silica gel using a gradient ethylacetate/heptane 0-30% to provide the title compound as a colorless gum (104 mg, 42 % yield). MS (ESI): m/z= 396.3 [M+H]+

### Step 4: [5-(2,2-difluoroethoxy)-4-methoxy-6-methyl-pyrimidin-2-yl]-bis(p-anisyl)amine

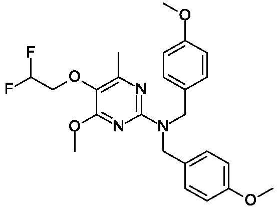

To a stirred suspension of 2-[bis(p-anisyl)amino]-4-methoxy-6-methyl-pyrimidin-5-ol (46 mg, 0.116 mmol) and cesium carbonate (114.85 mg, 0.349 mmol) in N,N-dimethylformamide (1.5 mL) was added trifluoromethanesulfonic acid 2,2-difluoroethyl ester (27.96 mg, 17.36 uL, 0.128 mmol). The reaction mixture was stirred at room temperature for 30 min. The reaction mixture was poured into water and extracted twice with EtOAc. The organic layers were washed twice with brine, dried over sodium sulfate, filtered and concentrated in vacuo to provide the title compound as a light yellow gum (30 mg, 53 % yield). MS (ESI): m/z= 460.4 [M+H]+

### Step 5: [5-(2,2-difluoroethoxy)-4-methoxy-6-methyl-pyrimidin-2-yl]amine

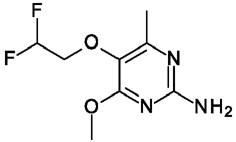

The title compound was prepared in analogy to intermediate B3, step 5 from [5-(2,2-difluoroethoxy)-4-methoxy-6-methyl-pyrimidin-2-yl]-bis(p-anisyl)amine as a white solid. MS (ESI): m/z=220.2 [M+H]+

### Intermediate B21: 4-cyclopropyl-5-(2,2-difluoroethoxy)-6-methoxy-pyrimidin-2-amine

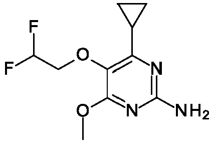

### Step 1: (4-cyclopropyl-6-methoxy-pyrimidin-2-yl)amine

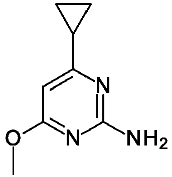

In a sealed tube, a suspension of (4-chloro-6-cyclopropyl-pyrimidin-2-yl)amine (300 mg, 1.77 mmol, CAS:21573-09-1) and sodium methylate (502.92 mg, 8.84 mmol) in methanol (10.39 mL) was heated at 85°C for 20 hours. The resulting light yellow suspension was concentrated in vacuo. The residue was diluted with ethyl acetate. Water was added and both layers were separated. The aqueous one was extracted twice with ethyl acetate. The combined organic layers were dried over sodium sulfate and concentrated in vacuo to provide the title compound as a light yellow solid (259 mg, 89 % yield). MS (ESI): m/z= 282.1 [M-H]

### Step 2: (5-bromo-4-cyclopropyl-6-methoxy-pyrimidin-2-yl)amine

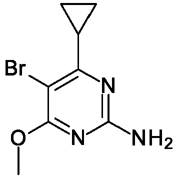

The title compound was prepared in analogy to intermediate B20, step 1 from (4-cyclopropyl-6-methoxy-pyrimidin-2-yl)amine as a white solid. MS (ESI): m/z=244.1 [M+H]+

### Step 3: (5-bromo-4-cyclopropyl-6-methoxy-pyrimidin-2-yl)-bis(p-anisyl)amine

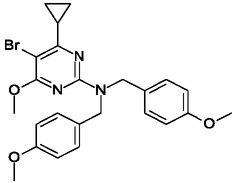

The title compound was prepared in analogy to intermediate B20, step 2 from (5-bromo-4-cyclopropyl-6-methoxy-pyrimidin-2-yl)amine as a white solid. MS (ESI): m/z=486.3 [M+H]+

### Step 4: 2-[bis(p-anisyl)amino]-4-cyclopropyl-6-methoxy-pyrimidin-5-ol

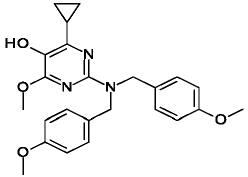

The title compound was prepared in analogy to intermediate B20, step 3 from (5-bromo-4-cyclopropyl-6-methoxy-pyrimidin-2-yl)-bis(p-anisyl)amine as a white solid. MS (ESI): m/z=422.3 [M+H]+

### Step 5: [4-cyclopropyl-5-(2,2-difluoroethoxy)-6-methoxy-pyrimidin-2-yl]-bis(p-anisyl)amine

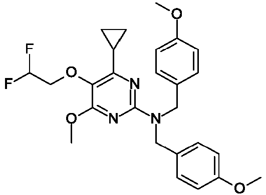

The title compound was prepared in analogy to intermediate B20, step 4 from 2-[bis(p-anisyl)amino]-4-cyclopropyl-6-methoxy-pyrimidin-5-ol as a white solid. MS (ESI): m/z=486.4 [M+H]+

### Step 6: 4-cyclopropyl-5-(2,2-difluoroethoxy)-6-methoxy-pyrimidin-2-amine

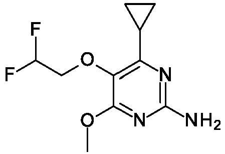

The title compound was prepared in analogy to intermediate B3, step 5 from [4-cyclopropyl-5-(2,2-difluoroethoxy)-6-methoxy-pyrimidin-2-yl]-bis(p-anisyl)amine as a off-white solid. MS (ESI): m/z=246.1 [M+H]+

### Intermediate B22: 5-bromo-4,6-dimethyl-pyrimidin-2-amine

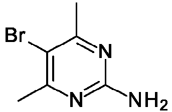

Intermediate B22 is commercial (CAS: 4214-57-7)

### Intermediate B23: 4-chloro-5-methoxy-pyrimidin-2-amine

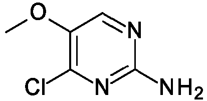

Intermediate B23 is commercial (CAS: 4763-36-4)

### Intermediate B24: 5-(2,2-difluoroethoxy)pyrimidin-2-amine

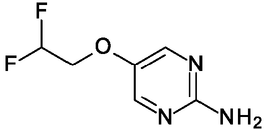

### Step 1: 2-chloro-5-(2,2-difluoroethoxy)pyrimidine

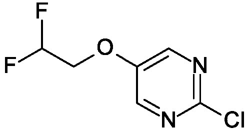

To a stirred suspension of 2-chloropyrimidin-5-ol (100 mg, 0.776 mmol, CAS:4983-28-2) and potassium carbonate (158.83 mg, 1.15 mmol) in acetone (2.7 mL) was added trifluoromethanesulfonic acid 2,2-difluoroethyl ester (188.64 mg, 0.881 mmol). The reaction mixture was stirred at room temperature for 1.5 hours, then diluted with diethylether, filtered and concentrated in vacuo. Dichloromethane was added to the residue. The mixture was sonicated for 5 min, filtered and concentrated in vacuo. The residue was purified by flash chromatography over silica gel using a gradient ethylacetate/heptane 0-15% to provide the title compound as a white solid (86 mg, 57 % yield). MS (ESI): m/z= 195.1 [M+H]+

### Step 2: 5-(2,2-difluoroethoxy)pyrimidin-2-amine

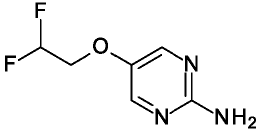

In a glass tube, a mixture of 2-chloro-5-(2,2-difluoroethoxy)pyrimidine (20 mg, 0.101 mmol), sodium tert. butoxide (10.65 mg, 0.111 mmol) and [tBuBrettPhos Pd(allyl)]Otf (2.36 mg, 0.003 mmol, CAS:1798782-15-6) was flushed with argon and a 0.5 M solution of ammonia in dioxane (2.42 mL, 1.21 mmol) was added. The reaction mixture was stirred at 30 °C for 2 hours and then concentrated in vacuo. Dichloromethane was added to the residue. The mixture was filtered and concentrated in vacuo to provide the title compound as a light yellow oil (23 mg, 100 % yield). MS (ESI): m/z= 176.1 [M+H]+

### Intermediate B25: 5-(2,2-difluoroethoxy)-4-methylsulfanyl-pyrimidin-2-amine

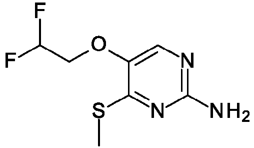

### Step 1: ethyl 2-(2,2-difluoroethoxy)acetate

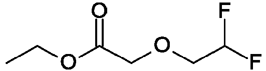

To a mixture of sodium hydride, 60 % in oil (5.0 g, 208.33 mmol) in tetrahydrofuran (500 mL) at 0° C was added dropwise 2,2-difluoroethanol (12.96 mL, 204.75 mmol). After 5 min, the reaction was allowed to warm to room temperature and ethyl bromoacetate (21.78 mL, 196.41 mmol) was added dropwise. After stirring at 22 °C for 24 hours, the mixture was poured into an aqueous solution of NaHSO4 and extracted twice with ethyl acetate. The organic layers were washed with brine, dried over sodium sulfate, filtered and carefully evaporated to provide the title compound as a dark brown oil (32 g, 74 % yield). ¹H NMR (400 MHz, DMSO-d6) δ = 6.12 (tt, J = 3.6, 54.9 Hz, 1H), 4.19 (s, 2H), 4.13 - 4.06 (m, 2H), 3.79 - 3.68 (m, 2H), 1.16 (t, J = 1.0 Hz, 3H)

### Step 2: ethyl (Z)-2-(2,2-difluoroethoxy)-3-(dimethylamino)prop-2-enoate

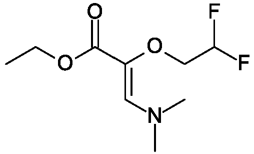

A mixture of ethyl 2-(2,2-difluoroethoxy)acetate (32 g, 190.32 mmol) and tert-butoxy bis(dimethylamino)methane (168.8 g, 200 mL, 968.6 mmol) was heated to 140 °C and stirred for 20 hours. The reaction mixture was concentrated in vacuo to provide the title compound as a dark brown oil (52 g, 86 % yield), which was directly used in the next step without further purification.

### Step 3: 2-amino-5-(2,2-difluoroethoxy)-1H-pyrimidin-6-one

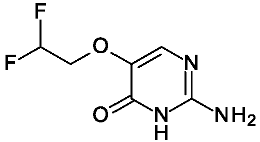

To a stirred solution of ethyl (Z)-2-(2,2-difluoroethoxy)-3-(dimethylamino)prop-2-enoate (52 g, 232.95 mmol) in N-methyl-2-pyrrolidinone (200 mL) was added guanidine carbonate (41.97 g, 232.95 mmol) and potassium carbonate (64.39 g, 465.91 mmol). The reaction mixture was stirred at 150 °C for 24 hours and then concentrated in vacuo. The residue was purified by flash chromatography over silica gel to provide the title compound as a white solid (16.3 g, 29 % yield). MS (ESI): m/z= 192.0 [M+H]+

### Step 4: 4-chloro-5-(2,2-difluoroethoxy)pyrimidin-2-amine

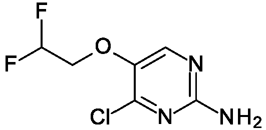

A solution of 2-amino-5-(2,2-difluoroethoxy)-1H-pyrimidin-6-one (3.0 g, 15.7 mmol) in phosphorus oxychloride (29.26 mL, 313.91 mmol) was heated at reflux for 16 hours. After that, the reaction mixture was evaporated to dryness and water was added (10 mL). The resulting mixture was stirred at room temperature for 30 minutes and a 6% aqueous solution of ammonium hydroxide was added (10 mL). The resulting precipitate was filtered off. The filtrate was diluted with brine and extracted three times with ethyl acetate. The organic layers were dried over sodium sulfate, filtered and concentrated in vacuo to provide the title compound as a brown solid (2.5 g, 61 % yield). ¹H NMR (500 MHz, DMSO-d6) δ = 8.21 (s, 1H), 6.33 (tt, J = 3.4, 54.3 Hz, 1H), 4.29 (dt, J = 3.5, 14.7 Hz, 2H)

### Step 5: 5-(2,2-difluoroethoxy)-4-methylsulfanyl-pyrimidin-2-amine

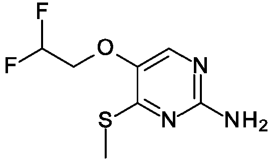

To a stirred solution of 4-chloro-5-(2,2-difluoroethoxy)pyrimidin-2-amine (1.1 g, 5.25 mmol) in N,N-dimethylformamide (30 mL) was added a sodium thiomethoxide solution 21 % in water (2.63 mL, 2.63 g, 7.87 mmol) dropwise at 0 °C. The reaction mixture was stirred at 0 °C for 1 hour and at room temperature overnight, then poured into water and extracted twice with ethyl acetate. The organic layers were washed twice with brine, dried over sodium sulfate and concentrated in vacuo to provide the title compound as an orange oil (330 mg, 23 % yield). MS (ESI): m/z= 222.0 [M+H]+

### Intermediate B26: 4-methoxy-5-methylsulfanyl-pyrimidin-2-amine

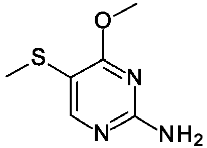

### Step 1: 4-methoxy-N,N-bis[(4-methoxyphenyl)methyl]-5-methylsulfanyl-pyrimidin-2-amine

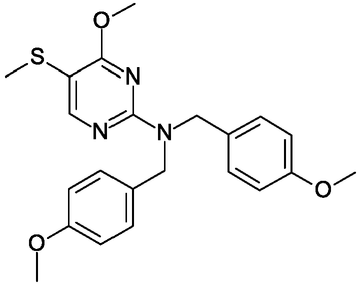

To a solution of 5-bromo-4-methoxy-N,N-bis[(4-methoxyphenyl)methyl]pyrimidin-2-amine (800.0 mg, 1.8 mmol, intermediate B3, step 1) in tetrahydrofuran (10 mL) at -78 °C, was added a 2.5 M solution of n-butyllithium in hexanes (0.94 mL, 2.34 mmol) dropwise. The mixture was stirred at -60 °C for 1 hour before methyl methanethiosulfonate (681.67 mg, 5.4 mmol) was added and the mixture was allowed to warm up to room temperature. After stirring for 16 hours, the mixture was quenched with a saturated solution of ammonium chloride and concentrated in vacuo. The residue was diluted with water and extracted with ethyl acetate. The organic phase was dried over sodium sulfate and concentrated in vacuo. The residue was purified by preparative HPLC to provide the title compound (117 mg, 16 % yield). MS (ESI): m/z= 412.2 [M+H]+

### Step 2: 4-methoxy-5-methylsulfanyl-pyrimidin-2-amine

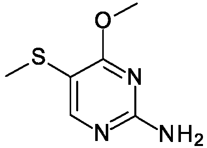

To a solution of 4-methoxy-N,N-bis[(4-methoxyphenyl)methyl]-5-methylsulfanyl-pyrimidin-2-amine (117.0 mg, 0.280 mmol) in dichloromethane (10 mL), was added trifluoroacetic acid (1.95 g, 1.31 mL, 17.06 mmol) was added. The mixture was heated at 50 °C for 15 hours and concentrated in vacuo. The residue was dissolved in aqueous sodium carbonate and extracted with ethyl acetate. The organic phase was washed with water, brine, dried over sodium sulfate and concentrated in vacuo to provide the title compound (42 mg, 86 % yield). MS (ESI): m/z= 172.0 [M+H]+

### Intermediate B27: 5-(difluoromethylsulfanyl)-4-methoxy-pyrimidin-2-amine

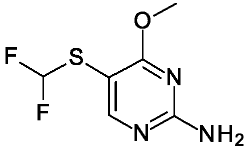

### Step 1: S-(2-chloro-4-methoxy-pyrimidin-5-yl) ethanethioate

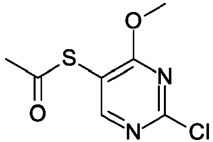

A mixture of 5-bromo-2-chloro-4-methoxy-pyrimidine (1 g, 4.39 mmol, CAS: 57054-92-9), tris(dibenzylideneacetone)dipalladium(0) (211.37 mg, 0.219 mmol), xantphos (258.94 mg, 0.439 mmol), n-ethyldiisopropylamine (1.74 g, 2.29 mL, 13.16 mmol) and potassium thioacetate (766.65 mg, 6.58 mmol) in 1,4-dioxane (20 mL) was heated in a microwave at 120 °C for 30 min. The reaction mixture was poured into brine and extracted twice with ethyl acetate. The organic layers were dried over sodium sulfate, filtered and concentrated in vacuo. The residue was purified by flash chromatography over silica gel using a gradient ethylacetate/heptane 0-20% to provide the title compound as an orange oil (254 mg, 26 % yield). MS (ESI) m/z= 219.0 [M+H]+

### Step 2: 2-chloro-5-(difluoromethylsulfanyl)-4-methoxy-pyrimidine

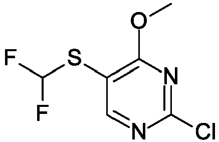

To a solution of S-(2-chloro-4-methoxy-pyrimidin-5-yl) ethanethioate (190 mg, 0.869 mmol) in acetonitrile (2 mL) was added a 5 M solution of potassium hydroxide in water (3.48 mL, 17.38 mmol). The reaction mixture was stirred at room temperature for 2 hours. Bromodifluoromethyl diethylphosphonate (464.02 mg, 308.73 uL, 1.74 mmol) in acetonitrile (600 uL) was added dropwise and the stirring was continued at room temperature for 30 min. The reaction mixture was poured into water and extracted twice with ethyl acetate. The organic layers were dried over sodium sulfate, filtered and concentrated in vacuo. The residue was purified by flash chromatography over silica gel using a gradient ethylacetate/heptane 0-20% to provide the title compound as a colorless viscous oil (57 mg, 29 % yield). MS (ESI) m/z= 227.0 [M+H]+

### Step 3: 5-(difluoromethylsulfanyl)-4-methoxy-N,N-bis[(4-methoxyphenyl)methyl]pyrimidin-2-amine

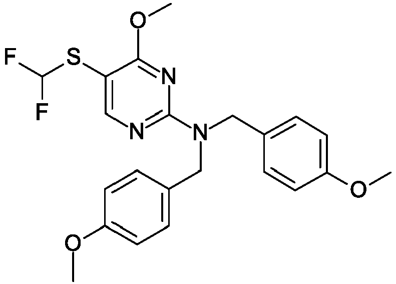

A colorless solution of 2-chloro-5-(difluoromethylsulfanyl)-4-methoxy-pyrimidine (68 mg, 0.300 mmol), bis(p-anisyl)amine (77.21 mg, 0.300 mmol) and n-ethyldiisopropylamine (79.14 mg, 104.68 uL, 0.600 mmol) in N-methyl-2-pyrrolidinone (1.4 mL) was heated to 80 °C and stirred for 4.5 hours. The resulting light red solution was diluted with ethyl acetate and washed twice with brine. The organic layer was dried over sodium sulfate, filtered and concentrated in vacuo. The residue was purified by flash chromatography over silica gel using a gradient ethylacetate/heptane 0-20% to provide the title compound as a colorless gum (99 mg, 74 % yield). MS (ESI) m/z= 448.3 [M+H]+

### Step 4: 5-(difluoromethylsulfanyl)-4-methoxy-pyrimidin-2-amine

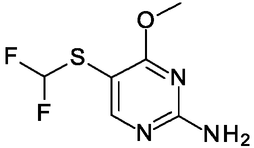

A solution of 5-(difluoromethylsulfanyl)-4-methoxy-N,N-bis[(4-methoxyphenyl)methyl]pyrimidin-2-amine (98 mg, 0.219 mmol) in dichloromethane (300 uL) was cooled to 0 °C. Trifluoroacetic acid (1.5 g, 1.01 mL, 13.14 mmol) was added. The reaction mixture was stirred at 0 °C for 30 min, at room temperature for 2 days, at 60 °C for 40 hours and concentrated in vacuo. The residue was poured into sat. NaHCO3 and extracted twice with ethyl acetate. The organic layers were dried over sodium sulfate, filtered and concentrated in vacuo. The residue was purified by flash chromatography over silica gel using a gradient ethylacetate/heptane 0-60% to provide the title compound as a light yellow solid (38 mg, 84 % yield). MS (ESI) m/z= 208.1 [M+H]+

### Intermediate B28: 5-(cyclopropoxy)-4-methoxy-pyrimidin-2-amine

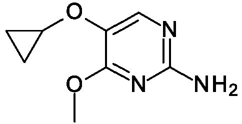

### Step 1: 5-(cyclopropoxy)-4-methoxy-N,N-bis[(4-methoxyphenyl)methyl]pyrimidin-2-amine

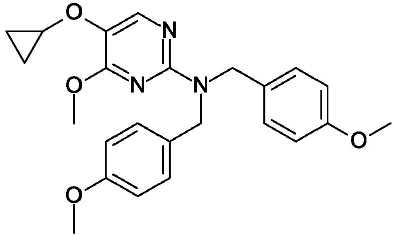

A suspension of 2-[bis(p-anisyl)amino]-4-methoxy-pyrimidin-5-ol (200 mg, 0.524 mmol, intermediate B3, step 3), cesium carbonate (341.68 mg, 1.05 mmol) and bromocyclopropane (317.17 mg, 210.05 uL, 2.62 mmol) in N-methyl-2-pyrrolidinone (3 mL) was stirred at 130 °C for 5 hours and at 140 °C for 2.5 hours. The reaction mixture was allowed to cool to room temperature, poured into water and extracted twice with ethyl acetate. The organic layers were dried over sodium sulfate, filtered and concentrated in vacuo. The residue was purified by preparative HPLC to provide the title compound as an orange viscous oil (49 mg, 19 % yield). MS (ESI) m/z= 422.4 [M+H]+

### Step 2: 5-(cyclopropoxy)-4-methoxy-pyrimidin-2-amine

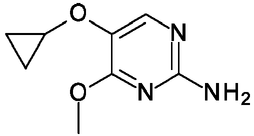

To a solution of 5-(cyclopropoxy)-4-methoxy-N,N-bis[(4-methoxyphenyl)methyl]pyrimidin-2-amine (48.9 mg, 0.116 mmol) in dichloromethane (100 uL) was added trifluoroacetic acid (737 mg, 536.28 uL, 6.96 mmol). The reaction mixture was stirred at room temperature for 23 hours, then poured into water and extracted twice with ethyl acetate. The organic layers were dried over sodium sulfate, filtered and concentrated in vacuo. The residue was purified by flash chromatography over silica gel using a gradient ethylacetate/heptane 0-60% to provide the title compound as a white powder (18 mg, 76 % yield). MS (ESI) m/z= 182.1 [M+H]+

### Intermediate B29: 5-(2,2-difluoroethoxy)-4-methoxy-pyrimidin-2-amine

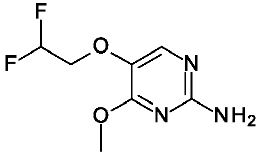

### Step 1: 5-(2,2-difluoroethoxy)-4-methoxy-N,N-bis[(4-methoxyphenyl)methyl]pyrimidin-2-amine

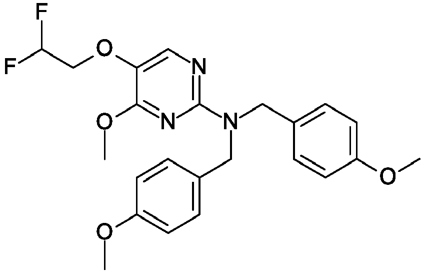

To a solution of 2-[bis(p-anisyl)amino]-4-methoxy-pyrimidin-5-ol (120 mg, 0.315 mmol, intermediate B3, step 3) in N,N-dimethylformamide (3 mL) was added cesium carbonate (307.51 mg, 0.944 mmol) and trifluoromethanesulfonic acid 2,2-difluoroethyl ester (75.61 mg, 46.96 uL, 0.346 mmol). The reaction mixture was stirred at room temperature for 15 min, poured into water and extracted twice with ethyl acetate. The organic layers were dried over sodium sulfate, filtered and concentrated in vacuo to provide the title compound as a light yellow viscous oil (113 mg, 81 % yield). MS (ESI) m/z= 446.3 [M+H]+

### Step 2: 5-(2,2-difluoroethoxy)-4-methoxy-pyrimidin-2-amine

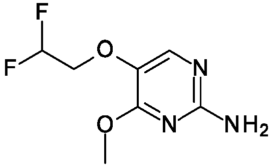

A solution of 5-(2,2-difluoroethoxy)-4-methoxy-N,N-bis[(4-methoxyphenyl)methyl]pyrimidin-2-amine (111 mg, 0.249 mmol) in dichloromethane (600 uL) was cooled to 0 °C. Trifluoroacetic acid (1.77 g, 1.19 mL, 15.36 mmol) was added. After stirring at 0 °C for 30 min, the reaction mixture was allowed to warm to room temperature and the stirring was continued for 2 days. The resulting red solution was poured into sat. NaHCO3 and extracted twice with ethyl acetate. The organic layers were dried over sodium sulfate, filtered and concentrated in vacuo. The residue was purified by flash chromatography over silica gel using a gradient ethylacetate/heptane 0-100% to provide the title compound as a white solid (36 mg, 70 % yield). MS (ESI) m/z= 206.1 [M+H]+

### Intermediate B30: 5-(3-fluoropropoxy)-4-methoxy-pyrimidin-2-amine

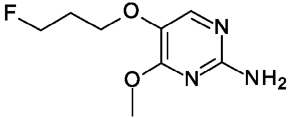

The title compound was prepared in analogy to intermediate B29 by alkylation of 2-[bis(p-anisyl)amino]-4-methoxy-pyrimidin-5-ol (intermediate B3, step 3) using cesium carbonate as base and 4-methylbenzenesulfonic acid 3-fluoropropyl ester as alkylating agent followed by deprotection of the bis(p-anisyl) protecting group with TFA as yellow solid. MS (ESI): m/z= 202.1 [M+H]+

### Intermediate B31: 5-but-2-ynoxy-4-methoxy-pyrimidin-2-amine

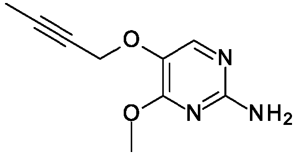

The title compound was prepared in analogy to intermediate B29 by alkylation of 2-[bis(p-anisyl)amino]-4-methoxy-pyrimidin-5-ol (intermediate B3, step 3) using cesium carbonate as base and 1-bromobut-2-yne as alkylating agent followed by deprotection of the bis(p-anisyl) protecting group with TFA as white solid. MS (ESI): m/z= 194.1 [M+H]+

### Intermediate B32: 5-(3,3-difluoropropoxy)-4-methoxy-pyrimidin-2-amine

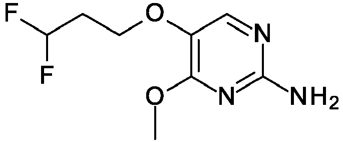

### Step 1: 3,3-difluoropropyl 4-methylbenzenesulfonate

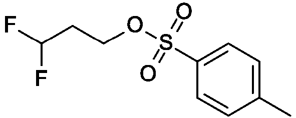

To a solution of 3,3-difluoropropan-1-ol (1.3 g, 13.53 mmol) in dichloromethane (40 mL) under nitrogen, was added triethylamine (3.42 g, 4.7 mL, 33.83 mmol), followed by p-toluenesulfonyl chloride (3.13 g, 16.24 mmol). The reaction was stirred at room temperature for 4 hours, then poured into sat. NaHCO3 and extracted twice with ethyl acetate. The organic layers were dried over sodium sulfate, filtered and concentrated in vacuo. The residue was purified by flash chromatography over silica gel using a gradient ethylacetate/heptane 0-30% to provide the title compound as a colorless liquid (3 g, 84 % yield). ¹H NMR (300 MHz, CHLOROFORM-d) δ = 7.79 (d, J = 7.7 Hz, 2H), 7.37 (d, J = 8.1 Hz, 2H), 5.89 (tt, J = 4.5, 56.0 Hz, 1H), 4.18 (t, J = 6.1 Hz, 2H), 2.46 (s, 3H), 2.29 - 2.13 (m, 2H)

### Step 2: 5-(3,3-difluoropropoxy)-4-methoxy-N,N-bis[(4-methoxyphenyl)methyl]pyrimidin-2-amine

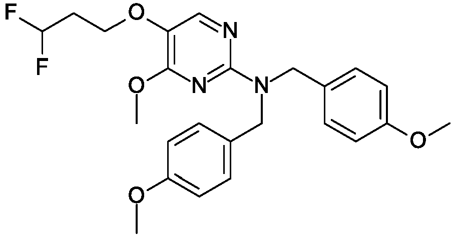

To a solution of 2-[bis(p-anisyl)amino]-4-methoxy-pyrimidin-5-ol (317 mg, 0.790 mmol, intermediate B3, step 3) in N,N-dimethylformamide (3.95 mL) was added cesium carbonate (771.73 mg, 2.37 mmol). The mixture was stirred at room temperature for 30 min. 3,3-difluoropropyl 4-methylbenzenesulfonate (310.49 mg, 0.868 mmol) was added and the reaction mixture was stirred at room temperature for 1 hour. The mixture was poured into water and extracted twice with ethyl acetate. The organic layers were washed twice with brine, dried over sodium sulfate, filtered and concentrated in vacuo. The residue was purified by flash chromatography over silica gel using a gradient ethylacetate/heptane 0-70% to provide the title compound as a yellow oil (126 mg, 30 % yield). MS (ESI) m/z= 460.3 [M+H]+

### Step 3: 5-(3,3-difluoropropoxy)-4-methoxy-pyrimidin-2-amine

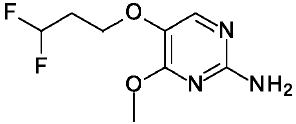

To a stirred solution of 5-(3,3-difluoropropoxy)-4-methoxy-N,N-bis[(4-methoxyphenyl)methyl]pyrimidin-2-amine (126.2 mg, 0.233 mmol) in dichloromethane (399 uL) was added trifluoroacetic acid (1.6 g, 1.08 mL, 14.01 mmol). The reaction mixture was stirred at 50 °C for 15 hours and then concentrated in vacuo. The residue was poured into sat. NaHCO3 and extracted twice with ethyl acetate. The organic layers were dried over sodium sulfate, filtered and concentrated in vacuo. The residue was purified by flash chromatography over silica gel using a gradient methanol/dichloromethane 0-10% to provide the title compound as a white solid (32 mg, 59 % yield). MS (ESI) m/z= 220.1 [M+H]+

### Intermediate B33: 4-methoxy-5-(3,3,3-trifluoropropoxy)pyrimidin-2-amine

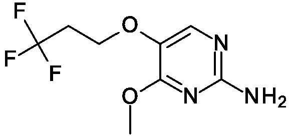

The title compound was prepared in analogy to intermediate B29 by alkylation of 2-[bis(p-anisyl)amino]-4-methoxy-pyrimidin-5-ol (intermediate B3, step 3) using cesium carbonate as base and trifluoromethanesulfonic acid 3,3,3-trifluoropropyl ester as alkylating agent followed by deprotection of the bis(p-anisyl) protecting group with TFA as light yellow solid. MS (ESI): m/z= 238.1 [M+H]+

### Intermediate B34: 4-methoxy-5-(1,1,2-trifluoroethoxy)pyrimidin-2-amine

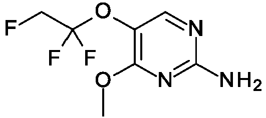

### Step 1: 5-(2,2-dichloro-1,1,2-trifluoro-ethoxy)-4-methoxy-N,N-bis[(4-methoxyphenyl)methyl]pyrimidin-2-amine

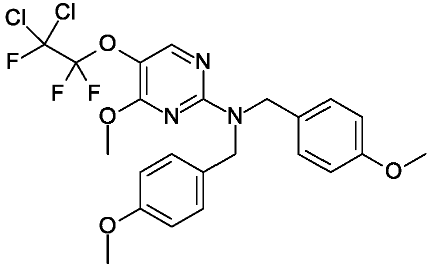

A mixture of 2-[bis(p-anisyl)amino]-4-methoxy-pyrimidin-5-ol (1100.0 mg, 2.62 mmol, intermediate B3, step 3), 1,1,2-trichlorotrifluoroethane (1081 mg, 5.80 mmol) and cesium carbonate (1409 mg, 4.3 mmol) in dimethyl sulfoxide (25 mL) was heated to 50 °C and stirred for 12 hours under nitrogen. The reaction mixture was poured into water and extracted three times with ethyl acetate. The organic layers were dried over sodium sulfate, filtered and concentrated in vacuo. The residue was purified by flash chromatography over silica gel using a gradient ethylacetate/petroleum ether 0-30% to provide the title compound as a yellow oil (800 mg, 11 % yield). MS (ESI) m/z= 498.2 [M+H]+

### Step 2: 5-(2,2-dichloro-1,1,2-trifluoro-ethoxy)-4-methoxy-pyrimidin-2-amine

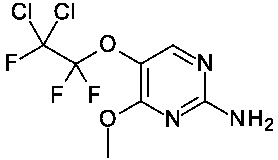

A mixture of 5-(2,2-dichloro-1,1,2-trifluoro-ethoxy)-4-methoxy-N,N-bis[(4-methoxyphenyl)methyl]pyrimidin-2-amine (1200 mg, 2.25 mmol) in trifluoroacetic acid (55 mL) was stirred at 60 °C for 12 hours. The reaction mixture was poured into sat. NaHCO3 to adjust pH to 7-8 and extracted twice with ethyl acetate. The organic layers were dried over sodium sulfate, filtered and concentrated in vacuo to provide the title compound as a yellow semisolid (660 mg, 100 % yield). MS (ESI) m/z= 291.6 [M+H]+

### Step 3: 4-methoxy-5-(1,1,2-trifluoroethoxy)pyrimidin-2-amine

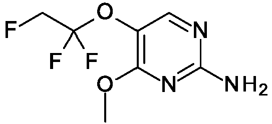

To a stirred suspension of 5-(2,2-dichloro-1,1,2-trifluoro-ethoxy)-4-methoxy-pyrimidin-2-amine (3000.0 mg, 10.27 mmol) in triethylsilane (40.0 mL) was added triethylamine (4.3 mL, 30.82 mmol) and a catalytic amount of palladium dichloride (182.16 mg, 1.03 mmol) under nitrogen at room temperature. The mixture was stirred at 80 °C for 48 hours, poured into water and extracted four times with ethyl acetate. The organic layers were washed with brine, dried over sodium sulfate, filtered and concentrated in vacuo. The residue was purified by preparative HPLC to provide the title compound as an off-white solid (790 mg, 34 % yield). MS (ESI) m/z= 224.0 [M+H]+

### Intermediate B35: 5-(1,1-difluoroethoxy)-4-methoxy-pyrimidin-2-amine

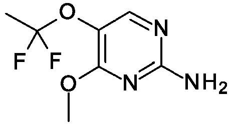

### Step 1: 5-[(Z)-1,2-dichlorovinyloxy]-4-methoxy-N,N-bis[(4-methoxyphenyl)methyl]pyrimidin-2-amine

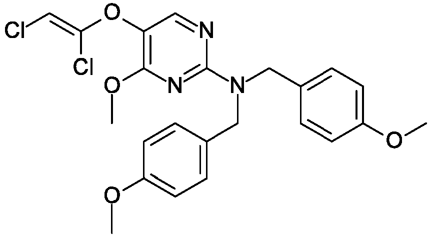

To a solution of trichloroethylene (620.04 mg, 4.72 mmol) in N,N-dimethylformamide (10 mL) was added sodium hydride (103.82 mg, 2.6 mmol) at 20 °C. The reaction mixture was stirred at 20 °C for 1 hour. A solution of 2-[bis(p-anisyl)amino]-4-methoxy-pyrimidin-5-ol (900.0 mg, 2.36 mmol, intermediate B3, step 3) in N,N-dimethylformamide (10 mL) was added to the reaction mixture. The mixture was stirred at 50 °C for 16 hours and directly purified by flash chromatography over silica gel using a gradient ethylacetate/petroleum ether 0-13% to provide the title compound as a colorless oil (900 mg, 80 % yield).

### Step 2: 5-ethynoxy-4-methoxy-N,N-bis[(4-methoxyphenyl)methyl]pyrimidin-2-amine

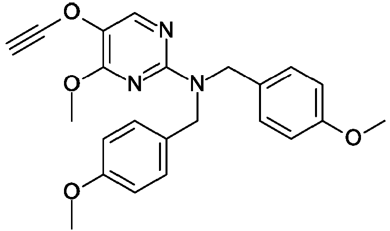

To a solution of 5-[(Z)-1,2-dichlorovinyloxy]-4-methoxy-N,N-bis[(4-methoxyphenyl)methyl]pyrimidin-2-amine (630.0 mg, 1.32 mmol) in dry diethyl ether (30 mL) was added a 2.5 M n-butyllithium solution (2.65 mL, 6.61 mmol) at -78 °C under nitrogen atmosphere. The mixture was stirred at -78° C for 1 hour. Then the mixture was warmed to -40 °C and stirred at -40 °C for another 2 hours. The reaction mixture was quenched with a saturated solution of ammonium chloride and extracted three times with ethyl acetate. The organic layers were washed with brine, dried over sodium sulfate, filtered and concentrated in vacuo. The residue was purified by flash chromatography over silica gel using a gradient ethylacetate/petroleum ether 0-15% to provide the title compound as a colorless oil (89 mg, 17 % yield). MS (ESI) m/z= 405.9 [M+H]+

### Step 3: 5-(1,1-difluoroethoxy)-4-methoxy-N,N-bis[(4-methoxyphenyl)methyl]pyrimidin-2-amine

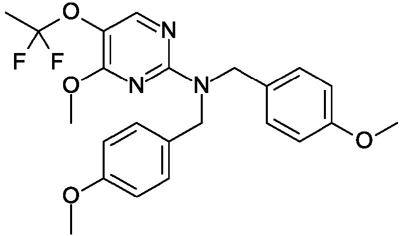

5-ethynoxy-4-methoxy-N,N-bis[(4-methoxyphenyl)methyl]pyrimidin-2-amine (130.0 mg, 0.320 mmol) was added into a Teflon flask and dissolved into dichloromethane (6 mL). The solution was cooled to -78 °C and stirred for 15 min. Hydrogen fluoride-pyridine (220.0 mg, 1.44 mmol) was added. The reaction mixture was stirred for 18 hours, left to reach 25 °C, then poured into sat. NaHCO3 and extracted twice with ethyl acetate. The organic layers were dried over sodium sulfate, filtered and concentrated in vacuo to provide the title compound as a light brown oil (142.3 mg, 100 % yield). MS (ESI) m/z= 445.8 [M+H]+

### Step 4: 5-(1,1-difluoroethoxy)-4-methoxy-pyrimidin-2-amine

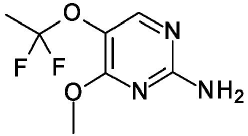

To a mixture of 5-(1,1-difluoroethoxy)-4-methoxy-N,N-bis[(4-methoxyphenyl)methyl]pyrimidin-2-amine (150.0 mg, 0.340 mmol) in dichloromethane (0.500 mL) was added trifluoroacetic acid (1.5 mL, 19.47 mmol) at 25 °C. The reaction mixture was stirred for 5 hours at 60 °C, then poured into sat. NaHCO3 and extracted three times with ethyl acetate. The organic layers were washed with brine, dried over sodium sulfate, filtered and concentrated in vacuo. The residue was purified by flash chromatography over silica gel using a gradient ethylacetate/petroleum ether 0-80% to provide the title compound as an off-white semisolid (50 mg, 72 % yield). MS (ESI) m/z= 206.0 [M+H]+

### Intermediate B36: 4-methoxy-5-(1,1,2,2-tetrafluoroethoxy)pyrimidin-2-amine

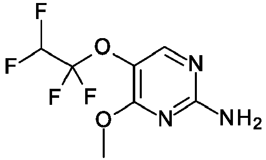

### Step 1: 5-(2-bromo-1,1,2,2-tetrafluoro-ethoxy)-4-methoxy-N,N-bis[(4-methoxyphenyl)methyl]pyrimidin-2-amine

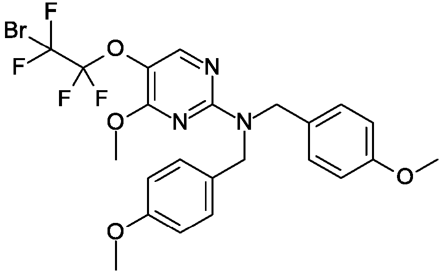

A mixture of 2-[bis(p-anisyl)amino]-4-methoxy-pyrimidin-5-ol (400 mg, 1.05 mmol, intermediate B3, step 3), 1,2-dibromotetrafluoroethane (0.25 mL, 2.1 mmol) and cesium carbonate (512.52 mg, 1.57 mmol) in dimethyl sulfoxide (10 mL) was heated to 50 °C and stirred for 2 hours under nitrogen. The reaction mixture was extracted three times with ethyl acetate. The organic layers were washed with brine, dried over sodium sulfate, filtered and concentrated in vacuo. The residue was purified by flash chromatography over silica gel using a gradient ethylacetate/petroleum ether 0-20% to provide the title compound as a colorless oil (341 mg, 58 % yield). MS (ESI) m/z= 559.9 [M+H]+

### Step 2: 4-methoxy-5-(1,1,2,2-tetrafluoroethoxy)pyrimidin-2-amine

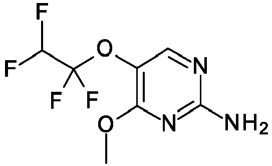

A mixture of 5-(2-bromo-1,1,2,2-tetrafluoro-ethoxy)-4-methoxy-N,N-bis[(4-methoxyphenyl)methyl]pyrimidin-2-amine (300 mg, 0.540 mmol) in methanol (30 mL) was purged with nitrogen. Palladium hydroxide (300 mg) was added. The mixture was purged with hydrogen and then stirred at 80 °C for 16 hours under 2.7 atm. of hydrogen. The reaction mixture was filtered and the filtrate was concentrated in vacuo. The residue was purified by flash chromatography over silica gel using a gradient ethylacetate/petroleum ether 0-100% to provide the title compound as a colorless oil (50 mg, 38 % yield). MS (ESI) m/z= 241.7 [M+H]+

### Intermediate B37: 5-(2-chloro-1,1,2-trifluoro-ethoxy)-4-methoxy-pyrimidin-2-amine

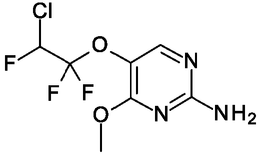

### Step 1: 5-(2-chloro-1,1,2-trifluoro-ethoxy)-4-methoxy-N,N-bis[(4-methoxyphenyl)methyl]pyrimidin-2-amine

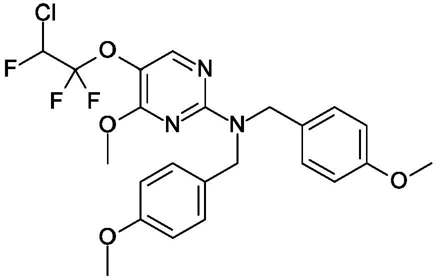

A mixture of 2-[bis(p-anisyl)amino]-4-methoxy-pyrimidin-5-ol (1100 mg, 2.62 mmol, intermediate B3, step 3), 1,1,2-trichlorotrifluoroethane (1081 mg, 5.80 mmol) and cesium carbonate (1409 mg, 4.3 mmol) in dimethyl sulfoxide (25 mL) was heated to 50 °C and stirred for 12 hours under nitrogen. The reaction mixture was poured into water and extracted three times with ethyl acetate. The organic layers were washed with brine, dried over sodium sulfate, filtered and concentrated in vacuo. The residue was purified by flash chromatography over silica gel using a gradient ethylacetate/petroleum ether 0-30% to provide the title compound as a yellow oil (800 mg, 11 % yield). MS (ESI) m/z= 498.2 [M+H]+

### Step 2: 5-(2-chloro-1,1,2-trifluoro-ethoxy)-4-methoxy-pyrimidin-2-amine

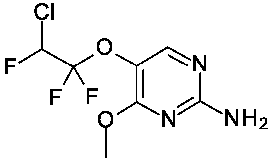

A mixture of 5-(2-chloro-1,1,2-trifluoro-ethoxy)-4-methoxy-N,N-bis[(4-methoxyphenyl)methyl]pyrimidin-2-amine (720.0 mg, 1.45 mmol) in trifluoroacetic acid (7 mL) was stirred at 80 °C for 12 hours. The reaction mixture was quenched by addition of sat. NaHCO3 and extracted three times with ethyl acetate. The organic layers were washed with brine, dried over sodium sulfate, filtered and concentrated in vacuo to provide the title compound as a dark brown solid (373 mg, 100 % yield). MS (ESI) m/z= 258.1 [M+H]+

### Intermediate B38: 4-methoxy-5-propyl-pyrimidin-2-amine

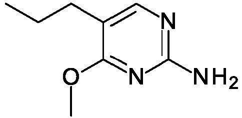

### Step 1: 5-allyl-4-methoxy-N,N-bis[(4-methoxyphenyl)methyl]pyrimidin-2-amine

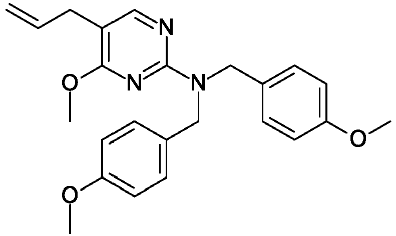

A mixture of (5-bromo-4-methoxy-pyrimidin-2-yl)-bis(p-anisyl)amine (400 mg, 0.900 mmol, intermediate B3, step 1), 2-allyl-4,4,5,5-tetramethyl-1,3,2-dioxaborolane (302.56 mg, 338.43 uL, 1.8 mmol), potassium carbonate (373.27 mg, 2.7 mmol) and dichloro[1,1'-bis(diphenylphosphino)ferrocene]palladium(II) dichloromethane adduct (22.06 mg, 0.027 mmol) in 1,4-dioxane (4.19 mL) and water (1.05 mL) was heated to 90 °C and stirred for 6 hours. The reaction mixture was poured into water and extracted with ethyl acetate. The organic layers were washed with brine, dried over sodium sulfate, filtered and concentrated in vacuo. The residue was purified by flash chromatography over silica gel using a gradient ethylacetate/heptane 0-7% to provide the title compound as a colorless waxy solid (200 mg, 27 % yield). MS (ESI) m/z= 406.3 [M+H]+

### Step 2: 4-methoxy-N,N-bis[(4-methoxyphenyl)methyl]-5-propyl-pyrimidin-2-amine

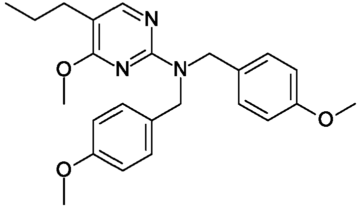

A suspension of 5-allyl-4-methoxy-N,N-bis[(4-methoxyphenyl)methyl]pyrimidin-2-amine (200 mg, 0.493 mmol) in methanol (2 mL) and palladium on activated charcoal (26.24 mg, 0.025 mmol) was stirred under 1 atm. of hydrogen at room temperature for 5 hours. The reaction mixture was filtered through a pad of celite and concentrated in vacuo to provide the title compound as a colorless waxy solid (200 mg, 80 % yield). MS (ESI) m/z= 408.3 [M+H]+

### Step 3: 4-methoxy-5-propyl-pyrimidin-2-amine

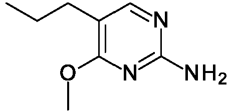

A mixture of 4-methoxy-N,N-bis[(4-methoxyphenyl)methyl]-5-propyl-pyrimidin-2-amine (200 mg, 0.491 mmol) in trifluoroacetic acid (1.99 mL) was stirred at 50 °C for 17 hours and concentrated in vacuo to provide the title compound as a colorless solid (80 mg, 93 % yield). MS (ESI) m/z=168.1 [M+H]+

### Intermediate B39: 5-(cyclopropylmethyl)-4-methoxy-pyrimidin-2-amine

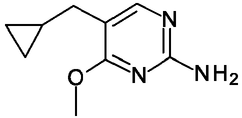

### Step 1: 5-bromo-N,N-bis[(2,4-dimethoxyphenyl)methyl]-4-methoxy-pyrimidin-2-amine

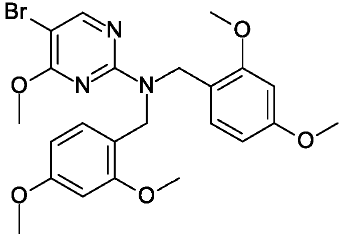

A solution of 5-bromo-2-chloro-4-methoxy-pyrimidine (5.0 g, 22.38 mmol, CAS: 57054-92-9), n-ethyldiisopropylamine (8.68 g, 11.72 mL, 67.13 mmol) and bis(2,4-dimethoxybenzyl)amine (10.65 g, 33.56 mmol) in acetonitrile (45 mL) was stirred at 80 °C for 23 hours, then cooled to room temperature and concentrated in vacuo. The residue was purified by flash chromatography over silica gel using a gradient ethylacetate/heptane 0-25% to provide the title compound as a light yellow amorphous (9.18 g, 77 % yield). MS (ESI) m/z= 506.2 [M+H]+

### Step 2: 2-[bis[(2,4-dimethoxyi)henyl)methyl]amino]-4-methoxy-pyrimidine-5-carbaldehyde

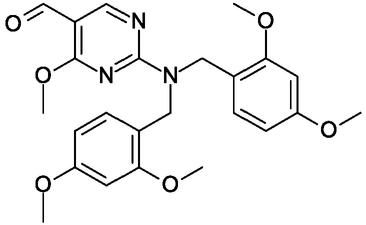

A light yellow solution of 5-bromo-N,N-bis[(2,4-dimethoxyphenyl)methyl]-4-methoxy-pyrimidin-2-amine (1.52 g, 2.86 mmol) in tetrahydrofuran (25 mL) was cooled to -72 °C. A 1.6 M n-butyllithium solution in hexanes (1.54 g, 1.79 mL, 2.86 mmol) was added dropwise. The reaction mixture was stirred at -72 °C for 15 min and N,N-dimethylformamide (313.9 mg, 332.17 uL, 4.29 mmol) was added. The stirring was continued at -72 °C for 45 min. The reaction mixture was quenched with a saturated solution of ammonium chloride and extracted twice with ethyl acetate. The organic layers were dried over sodium sulfate, filtered and concentrated in vacuo. The residue was purified by flash chromatography over silica gel using a gradient ethylacetate/heptane 0-40% to provide the title compound as a colorless viscous oil (730 mg, 56 % yield). MS (ESI) m/z= 454.3 [M+H]+

### Step 3: [2-[bis[(2,4-dimethoxyphenyl)methyl]amino]-4-methoxy-pyrimidin-5-yl]-cyclopropyl-methanol

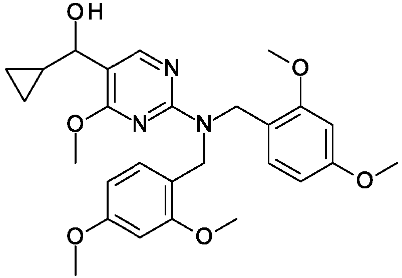

1 M cyclopropylmagnesium bromide solution in 2-methyltetrahydrofuran (441.02 uL, 0.441 mmol) was added dropwise to a solution of 2-[bis[(2,4-dimethoxyphenyl)methyl]amino]-4-methoxy-pyrimidine-5-carbaldehyde (100 mg, 0.221 mmol) in tetrahydrofuran (4 mL) at 0 °C. The reaction mixture was stirred at 0 °C for 15 min, then quenched with a saturated solution of ammonium chloride and extracted twice with ethyl acetate. The organic layers were dried over sodium sulfate, filtered and concentrated in vacuo. The residue was purified by flash chromatography over silica gel using a gradient ethylacetate/heptane 0-50% to provide the title compound as a white solid (94 mg, 86 % yield). MS (ESI) m/z= 496.3 [M+H]+

### Step 4: 5-(cyclopropylmethyl)-N,N-bis[(2,4-dimethoxyphenyl)methyl]-4-methoxy-pyrimidin-2-amine

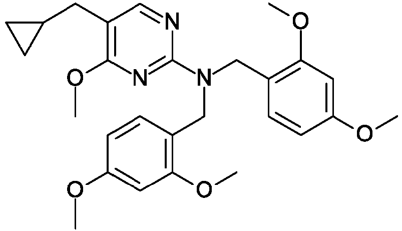

A solution of [2-[bis[(2,4-dimethoxyphenyl)methyl]amino]-4-methoxy-pyrimidin-5-yl]-cyclopropyl-methanol (85 mg, 0.172 mmol) in dichloromethane (1 mL) was cooled to 0 °C. Triethylsilane (201.46 mg, 276.73 uL, 1.72 mmol) was added dropwise, followed by trifluoroacetic acid (58.67 mg, 39.38 uL, 0.515 mmol). The reaction mixture was stirred at 0 °C for 20 min, at room temperature for 20 hours, then poured into sat. NaHCO3 and extracted twice with ethyl acetate. The organic layers were dried over sodium sulfate, filtered and concentrated in vacuo. The residue was purified by flash chromatography over silica gel using a gradient ethylacetate/heptane 0-20% to provide the title compound as a white solid (57 mg, 69 % yield). MS (ESI) m/z= 480.3 [M+H]+

### Step 5: 5-(cyclopropylmethyl)-4-methoxy-pyrimidin-2-amine

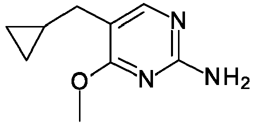

A colorless solution of 5-(cyclopropylmethyl)-N,N-bis[(2,4-dimethoxyphenyl)methyl]-4-methoxy-pyrimidin-2-amine (57 mg, 0.119 mmol) in dichloromethane (100 uL) was cooled to 0 °C. Trifluoroacetic acid (813.14 mg, 545.73 uL, 7.13 mmol) was added. The reaction mixture was allowed to warm to room temperature and stirred for 2 hours. The resulting purple mixture was poured into sat. NaHCO3 and extracted twice with ethyl acetate. The organic layers were dried over sodium sulfate, filtered and concentrated in vacuo to provide the title compound as a white solid (22 mg, 100 % yield). MS (ESI) m/z= 180.2 [M+H]+

### Intermediate B40: 5-(2,2-difluoroethyl)-4-methoxy-pyrimidin-2-amine

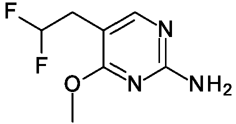

### Step 1: 5-[(E)-2-ethoxyvinyl]-4-methoxy-N,N-bis[(4-methoxyphenyl)methyl]pyrimidin-2-amine

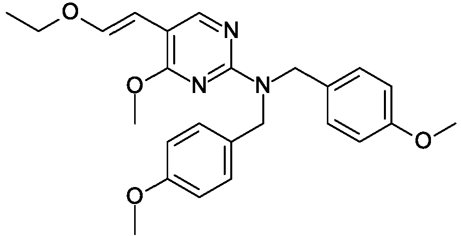

In a 250 mL round-bottomed flask were added one after the other, (5-bromo-4-methoxy-pyrimidin-2-yl)-bis(p-anisyl)amine (11.1 g, 23.73 mmol, intermediate B3, step 1), 2-[(E)-2-ethoxyvinyl]-4,4,5,5-tetramethyl-1,3,2-dioxaborolane (6.11 g, 30.85 mmol), 1,4-dioxane (97 mL) / water (16 mL), dichloro[1,1'-bis(diphenylphosphino)ferrocene]palladium(II) dichloromethane adduct (1.94 g, 2.37 mmol) and cesium carbonate (23.2 g, 71.2 mmol) (vacuum/argon after each addition). The reaction mixture was stirred at 110 °C for 15 hours, then poured into water and extracted three times with ethyl acetate. The organic layers were washed with brine, dried over sodium sulfate, filtered and concentrated in vacuo. The residue was purified by flash chromatography over silica gel using a gradient ethylacetate/heptane 0-30% to provide the title compound as a light yellow oil (4.2 g, 39 % yield). MS (ESI) m/z= 436.3 [M+H]+

### Step 2: 2-[2-[bis[(4-methoxyphenyl)methyl]amino]-4-methoxy-pyrimidin-5-yl]acetaldehyde

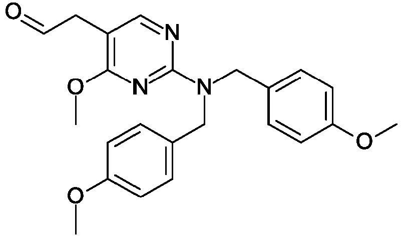

5-[(E)-2-ethoxyvinyl]-4-methoxy-N,N-bis[(4-methoxyphenyl)methyl]pyrimidin-2-amine (1 g, 2.3 mmol) in formic acid (3.17 g, 2.64 mL, 68.88 mmol) was stirred at 60 °C for 30 min, then poured into sat. NaHCO3 under ice cooling and extracted twice with ethyl acetate. The organic layers were washed with water and brine, dried over sodium sulfate, filtered and concentrated in vacuo to provide the title compound as a light yellow oil (1.01g, 97 % yield). MS (ESI) m/z= 408.3 [M+H]+

### Step 3: 5-(2,2-difluoroethyl)-4-methoxy-N,N-bis[(4-methoxyphenyl)methyl]pyrimidin-2-amine

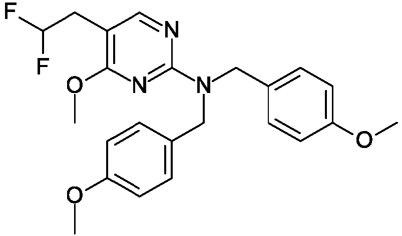

A solution of 2-[2-[bis[(4-methoxyphenyl)methyl]amino]-4-methoxy-pyrimidin-5-yl]acetaldehyde (1.01 g, 2.23 mmol) in dichloromethane, extra dry (10 mL) was cooled to 0 °C. Deoxo-fluor ^{®}, 2.7M (50 wt.%) solution in toluene (1.98 g, 1.65 mL, 4.46 mmol) was added dropwise. The reaction mixture was stirred at 0 °C for 30 min, then poured into sat. NaHCO3 and extracted twice with ethyl acetate. The organic layers were dried over sodium sulfate, filtered and concentrated in vacuo. The residue was purified by flash chromatography over silica gel using a gradient ethylacetate/heptane 0-30% to provide the title compound as a colorless oil (763 mg, 72 % yield). MS (ESI) m/z= 430.3 [M+H]+

### Step 4: 5-(2,2-difluoroethyl)-4-methoxy-pyrimidin-2-amine

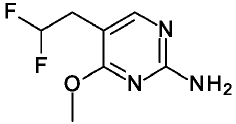

To a stirred solution of 5-(2,2-difluoroethyl)-4-methoxy-N,N-bis[(4-methoxyphenyl)methyl]pyrimidin-2-amine (763 mg, 1.6 mmol) in dichloromethane (8 mL) was added trifluoroacetic acid (10.94 g, 7.39 mL, 95.94 mmol). The reaction mixture was stirred at 50 °C for 2 days, then concentrated in vacuo. The residue was poured into sat. NaHCO3 and extracted twice with ethyl acetate. The organic layers were dried over sodium sulfate, filtered and concentrated in vacuo. The residue was purified by flash chromatography over silica gel using a gradient ethylacetate/heptane 0-100% to provide the title compound as a light yellow solid (220 mg, 73 % yield). MS (ESI) m/z= 190.1 [M+H]+

### Intermediate B41: 5-(2,2-difluoropropyl)-4-methoxy-pyrimidin-2-amine

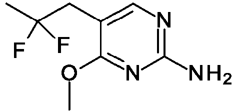

### Step 1: 1-[2-[bis[(2,4-dimethoxyphenyl)methyl]amino]-4-methoxy-pyrimidin-5-yl]-2,2-difluoro-propan-1-ol

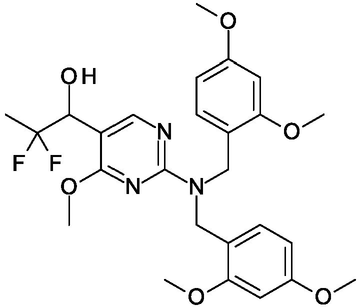

To a solution of 5-bromo-N,N-bis[(2,4-dimethoxyphenyl)methyl]-4-methoxy-pyrimidin-2-amine (1.5 g, 2.97 mmol, intermediate B39, step 1) in tetrahydrofuran, extra dry (10 mL) was added 1.6 M n-butyllithium in hexanes (2.42 mL, 3.87 mmol) dropwise at -78 °C. After 1 hour, 2,2-difluoropropionic acid ethyl ester (2.05 g, 14.87 mmol) was added dropwise and the reaction mixture was stirred for an additional hour at -78 °C before warming to 0 °C. After 45 min, methanol (2 mL) was added, followed by sodium borohydride (112.51 mg, 2.97 mmol). The reaction mixture was quenched with a saturated solution of ammonium chloride and extracted with ethyl acetate. The organic layers were dried over sodium sulfate, filtered and concentrated in vacuo. The residue was purified by flash chromatography over silica gel using a gradient ethylacetate/heptane 0-35% to provide the title compound as a white foam (805 mg, 42 % yield). MS (ESI) m/z= 520.4 [M+H]+

### Step 2: 5-(2,2-difluoropropyl)-N,N-bis[(2,4-dimethoxyphenyl)methyl]-4-methoxy-pyrimidin-2-amine

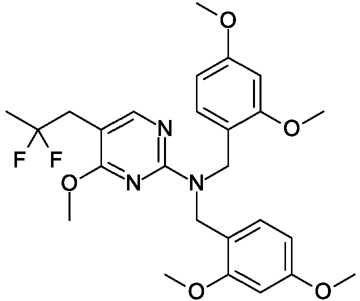

1-[2-[bis[(2,4-dimethoxyphenyl)methyl]amino]-4-methoxy-pyrimidin-5-yl]-2,2-difluoro-propan-1-ol (580 mg, 1.12 mmol) and 1,1'-thiocarbonyldiimidazole (298.42 mg, 1.67 mmol) were mixed in tetrahydrofuran (8 mL) and stirred at 70 °C for 5.5 hours. The reaction mixture was cooled to room temperature, diluted with ethyl acetate and washed with brine. The organic layer was dried over sodium sulfate, filtered and concentrated in vacuo. The residue was dissolved in toluene (11.6 mL) and degassed. 2,2'-azobis(2-methylpropionitrile) (36.66 mg, 0.223 mmol) and tri-n-butyltin hydride (1.62 g, 1.49 mL, 5.58 mmol) were added. The reaction mixture was stirred at 85 °C for 3 hours, cooled to room temperature, put on silica gel, concentrated in vacuo and purified by flash chromatography using a gradient ethylacetate/heptane 0-20% to provide the title compound as a colorless amorphous (242 mg, 34 % yield). MS (ESI) m/z= 504.4 [M+H]+

### Step 3: 5-(2,2-difluoropropyl)-4-methoxy-pyrimidin-2-amine

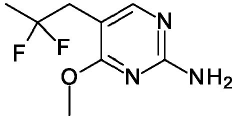

5-(2,2-difluoropropyl)-N,N-bis[(2,4-dimethoxyphenyl)methyl]-4-methoxy-pyrimidin-2-amine (242 mg, 0.384 mmol) was dissolved in trifluoroacetic acid (4.44 g, 3 mL, 38.94 mmol). The reaction mixture was stirred at room temperature for 15 hours and concentrated in vacuo. The residue was poured into sat. NaHCO3 and extracted with ethyl acetate. The organic layers were dried over sodium sulfate, filtered and concentrated in vacuo. The residue was purified by flash chromatography over silica gel using a gradient ethylacetate/heptane 0-70% to provide the title compound as a colorless solid (52 mg, 63 % yield). MS (ESI) m/z= 204.2 [M+H]+

### Intermediate B42: 5-(3,3-difluoropropyl)-4-methoxy-pyrimidin-2-amine

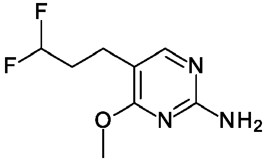

### Step 1: tert-butyl N-(5-bromo-4-methoxy-pyrimidin-2-yl)-N-tert-butoxycarbonyl-carbamate

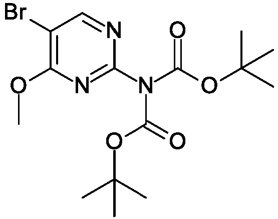

(5-bromo-4-methoxy-pyrimidin-2-yl)amine (3 g, 14.7 mmol, CAS: 36082-45-8) was suspended in dichloromethane (72 mL) and n-ethyldiisopropylamine (2.28 g, 3.08 mL, 17.65 mmol) was added. The mixture was cooled to 5 °C and a solution of di-tert-butyl dicarbonate (6.42 g, 6.83 mL, 29.41 mmol) in dichloromethane (36 mL) and 4-dimethylaminopyridine (179.64 mg, 1.47 mmol) was added dropwise. After the addition, the cooling bath was removed and the mixture was stirred at room temperature for 5 hours, then poured into water and extracted three times with dichloromethane. The organic layers were washed with brine, dried over sodium sulfate, filtered and concentrated in vacuo. The residue was purified by flash chromatography over silica gel using a gradient ethylacetate/heptane 0-50% to provide the title compound as a light yellow oil (4.68 g, 79 % yield). MS (ESI) m/z= 204.0 [M+H-2 BOC]+

### Step 2: ethyl (E)-3-[2-[bis(tert-butoxycarbonyl)amino]-4-methoxy-pyrimidin-5-yl]prop-2-enoate

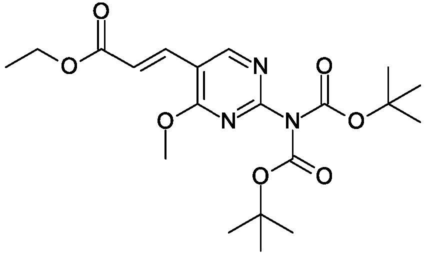

In a round-bottomed flask were added one after the other, tert-butyl N-(5-bromo-4-methoxy-pyrimidin-2-yl)-N-tert-butoxycarbonyl-carbamate (4.68 g, 11.58 mmol), (e)-3-(4,4,5,5-tetramethyl-1,3,2-dioxaborolane-2-yl) acrylic acid ethyl ester (3.4 g, 3.37 mL, 15.05 mmol), 1,4-dioxane, extra dry (30 mL), water (5 mL) and 1,1'-bis(diphenylphosphino)ferrocene-palladium(II) dichloride (847.07 mg, 1.16 mmol) (3 vacuum-argon cycles after each addition). The reaction mixture was heated to 110 °C. After 1 hour of stirring at 110 °C, cesium carbonate (11.32 g, 34.73 mmol) was added. The reaction mixture was stirred at 110 °C for 20 hours, then poured into water and extracted three times with ethyl acetate. The organic layers were dried over sodium sulfate, filtered and concentrated in vacuo. The residue was purified by flash chromatography over silica gel using a gradient ethylacetate/heptane 0-30% to provide the title compound as a light yellow oil (2.72 g, 55 % yield). MS (ESI) m/z= 424.2 [M+H]+

### Step 3: ethyl 3-[2-[bis(tert-butoxycarbonyl)amino]-4-methoxy-pyrimidin-5-yl]propanoate

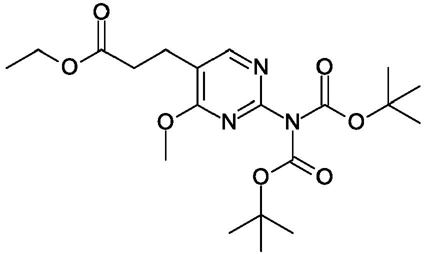

A solution of ethyl (E)-3-[2-[bis(tert-butoxycarbonyl)amino]-4-methoxy-pyrimidin-5-yl]prop-2-enoate (2.72 g, 6.42 mmol) in ethyl acetate (24.8 mL) was purged with argon for 5 min. Palladium on activated charcoal (683.56 mg, 0.642 mmol) was added. The reaction mixture was purged with hydrogen and stirred under 1 atm. of hydrogen at room temperature for 2 hours. The reaction mixture was filtered through a pad of celite and concentrated in vacuo to provide the title compound as a light grey oil (2.54 g, 90 % yield). MS (ESI): m/z= 426.2 [M+H]+

### Step 4: tert-butyl N-tert-butoxycarbonyl-N-[5-(3-hydroxypropyl)-4-methoxy-pyrimidin-2-yl]carbamate

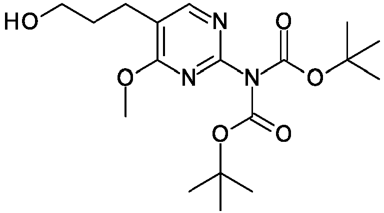

Ethyl 3-[2-[bis(tert-butoxycarbonyl)amino]-4-methoxy-pyrimidin-5-yl]propanoate (2.54 g, 5.79 mmol) was dissolved in tetrahydrofuran, extra dry (25.62 mL) under argon. The solution was cooled to 0 °C and 1 M DIBAL-H in tetrahydrofuran (11.58 mL, 11.58 mmol) was added dropwise. After addition, the reaction mixture was allowed to warm to room temperature, stirred for 2 hours and carefully quenched by adding a saturated solution of sodium tartrate slowly at 0 °C. The mixture was then poured into water and extracted three times with dichloromethane. The organic layers were washed with brine, dried over sodium sulfate, filtered and concentrated in vacuo. Dichloromethane was added to the residue, unsolubles were filtered off and the filtrate was purified by flash chromatography over silica gel using a gradient ethylacetate/heptane 0-60% to provide the title compound as a colorless oil (1.02 g, 44 % yield). MS (ESI) m/z= 384.2 [M+H]+

### Step 5: tert-butyl N-tert-butoxycarbonyl-N-[4-methoxy-5-(3-oxopropyl)pyrimidin-2-yl]carbamate

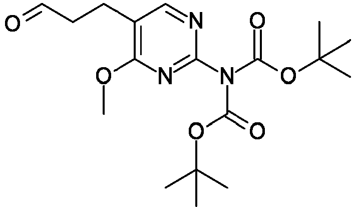

Tert-butyl N-tert-butoxycarbonyl-N-[5-(3-hydroxypropyl)-4-methoxy-pyrimidin-2-yl]carbamate (483 mg, 1.26 mmol) was dissoved in dichloromethane, extra dry (3.91 mL). The mixture was degassed by sonicating in a ultrasonic bath under argon atmosphere. 1,1,1-tris(acetyloxy)-1,1-dihydro-1,2-benzodioxol-3-(1H)-one (641.12 mg, 1.51 mmol) was added in 2 portions. The mixture was stirred for 1 hour at room temperature, then poured into sat. NaHCO3 and extracted with ethyl acetate. The organic layers were dried over sodium sulfate, filtered and concentrated in vacuo. The residue was purified by flash chromatography over silica gel using a gradient ethylacetate/heptane 0-60% to provide the title compound as a colorless oil (419.3 mg, 86 % yield). MS (ESI) m/z= 382.2 [M+H]+

### Step 6: tert-butyl N-tert-butoxycarbonyl-N-[5-(3,3-difluoropropyl)-4-methoxy-pyrimidin-2-yl]carbamate

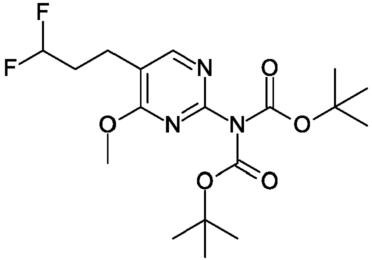

To a solution of tert-butyl N-tert-butoxycarbonyl-N-[4-methoxy-5-(3-oxopropyl)pyrimidin-2-yl]carbamate (419.3 mg, 1.08 mmol) in dichloromethane (4.73 mL) was added dropwise Deoxo-fluor ^{®}, 2.7M (50 wt.%) solution in toluene (399.01 uL, 1.08 mmol) at 0 °C under argon atmosphere. The reaction mixure was stirred for 2 hours at 0 °C, then poured into sat. NaHCO3 and extracted with ethyl acetate. The organic layers were dried over sodium sulfate, filtered and concentrated in vacuo to provide the title compound as a yellow liquid (434.2 mg, 97 % yield). MS (ESI) m/z= 404.3 [M+H]+

### Step 7: 5-(3,3-difluoropropyl)-4-methoxy-pyrimidin-2-amine

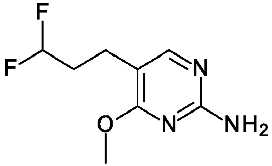

A mixture of tert-butyl N-tert-butoxycarbonyl-N-[5-(3,3-difluoropropyl)-4-methoxy-pyrimidin-2-yl]carbamate (434.2 mg, 1.04 mmol) and a 4 M HCl solution in 1,4-dioxane (3.92 mL, 15.66 mmol) was stirred at room temperature for 15 hours, then poured into sat. NaHCO3 and extracted with ethyl acetate. The organic layers were dried over sodium sulfate, filtered and concentrated in vacuo. The residue was purified by flash chromatography over silica gel using a gradient ethylacetate/heptane 0-80% to provide the title compound as a light yellow solid (170.9 mg, 79 % yield). MS (ESI) m/z= 203.9 [M+H]+

### Intermediate B43: 5-(2,3-difluoropropyl)-4-methoxy-pyrimidin-2-amine

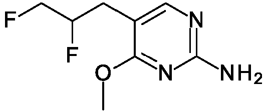

### Step 1: 5-allyl-4-methoxy-N,N-bis[(4-methoxyphenyl)methyl]pyrimidin-2-amine

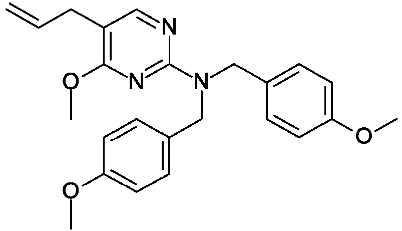

To a solution of (5-bromo-4-methoxy-pyrimidin-2-yl)-bis(p-anisyl)amine (1000.0 mg, 2.25 mmol, intermediate B3, step 1) in 1,4-dioxane (20 mL) and water (4 mL) was added allylboronic acid pinacol ester (567.29 mg, 3.38 mmol), cesium carbonate (1466.6 mg, 4.5 mmol) and 1,1'-bis(diphenylphosphino)ferrocene-palladium(II) dichloride dichloromethane complex (82.34 mg, 0.110 mmol). The mixture was evacuated and backfilled with nitrogen (three times), and then stirred for 12 hours at 100 °C. The reaction mixture was extracted three times with ethyl acetate. The organic layers were washed with brine, dried over sodium sulfate, filtered and concentrated in vacuo. The residue was purified by flash chromatography over silica gel using a gradient ethylacetate/petroleum ether 0-10% to provide the title compound as a colorless oil (700 mg, 77 % yield). MS (ESI) m/z= 406.1 [M+H]+

### Step 2: 3-[2-[bis[(4-methoxyphenyl)methyl]lamino]-4-methoxy-pyrimidin-5-yl|propane-1,2-diol

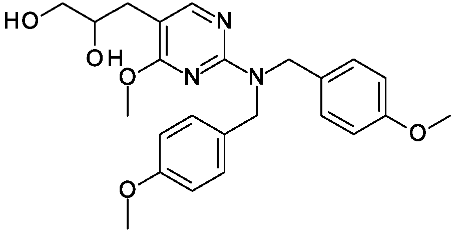

To a solution of 5-allyl-4-methoxy-N,N-bis[(4-methoxyphenyl)methyl]pyrimidin-2-amine (630.0 mg, 1.55 mmol) and N-methylmorpholine-N-oxide (364.03 mg, 3.11 mmol) in acetone (84 mL) and water (21 mL) was added osmium tetroxide (40.0 mg, 0.160 mmol) at room temperature. The reaction mixture was stirred at room temperature for 12 hours. A solution of sodium sulfite (50 mL) was added and the stirring was continued at room temperature for 1 hour. The reaction mixture was diluted with ethyl acetate and successively washed with water and brine. The organic layer was dried over sodium sulfate, filtered and concentrated in vacuo. The residue was purified by flash chromatography over silica gel using a gradient ethylacetate/petroleum ether 0-100% to provide the title compound as a colorless oil (500 mg, 73 % yield). MS (ESI) m/z= 440.2 [M+H]+

### Step 3: 5-(2,3-difluoropropyl)-4-methoxy-N,N-bis[(4-methoxyphenyl)methyl]pyrimidin-2-amine

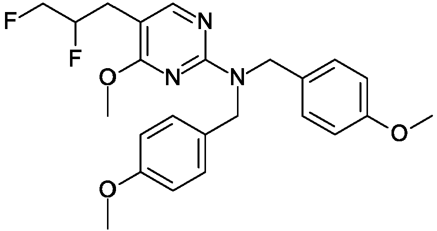

To a solution of 3-[2-[bis[(4-methoxyphenyl)methyl]amino]-4-methoxy-pyrimidin-5-yl]propane-1,2-diol (300.0 mg, 0.680 mmol) in dichloromethane (6 mL) was added diethylaminosulfur trifluoride (0.45 mL, 3.41 mmol) at 0 °C. The reaction mixture was allowed to warm to room temperature, stirred for 4 hours, and then slowly added to ice/water. The pH was adjusted to 7 with sat. NaHCO3. The mixture was extracted twice with ethyl acetate. The organic layers were washed with brine, dried over sodium sulfate, filtered and concentrated in vacuo. The residue was purified by preparative TLC (eluent: ethylacetate/petroleum ether 1/5) to provide the title compound as a white solid (60 mg, 20 % yield). MS (ESI) m/z= 444.1 [M+H]+

### Step 4: 5-(2,3-difluoropropyl)-4-methoxy-pyrimidin-2-amine

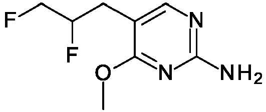

A solution of 5-(2,3-difluoropropyl)-4-methoxy-N,N-bis[(4-methoxyphenyl)methyl]pyrimidin-2-amine (60.0 mg, 0.140 mmol) in trifluoroacetic acid (2.0 mL, 26.93 mmol) was stirred for 2 hours at 60 °C, then poured into sat. NaHCO3 and extracted twice with ethyl acetate. The organic layers were dried over sodium sulfate, filtered and concentrated in vacuo. The residue was purified by preparative TLC (eluent: ethylacetate/petroleum ether 1/2) to provide the title compound as a colorless gum (20 mg, 73 % yield). MS (ESI) m/z= 203.9 [M+H]+

### Intermediate B44: 4-methoxy-5-(2,2,2-trifluoroethyl)pyrimidin-2-amine

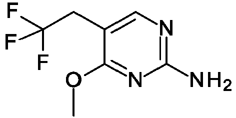

### Step 1: dimethyl 2-(2,2,2-trifluoroethyl)propanedioate

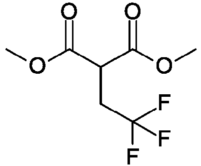

To a suspension of sodium hydride (296.72 mg, 7.42 mmol) in tetrahydrofuran, extra dry (20 mL) under argon, was added dimethyl malonate (1 g, 869.57 uL, 7.42 mmol) dropwise within 5 min at 0 °C. After 20 min, trifluoromethanesulfonic acid 2,2,2-trifluoroethyl ester (1.72 g, 1.07 mL, 7.42 mmol) was added at room temperature. The resulting light yellow solution was heated to 50 °C and stirred for 6 hours. Trifluoromethanesulfonic acid 2,2,2-trifluoroethyl ester (1.72 g, 1.07 mL, 7.42 mmol) was added again and the stirring was continued at 50 °C for 1 hour. The reaction mixture was allowed to cool to room temperature, poured into water and extracted twice with ethyl acetate. The organic layers were dried over sodium sulfate, filtered and concentrated in vacuo to provide the title compound as an orange solid (1.19 g, 60 % yield), which was directly used in the next step without further purification. ¹H NMR (300 MHz, DMSO-d6) δ = 3.88 (t, J = 7.1 Hz, 1H), 3.71 (s, 6H), 2.87 (dq, J = 7.2, 11.0 Hz, 2H)

### Step 2: 2-amino-5-(2,2,2-trifluoroethyl)pyrimidine-4,6-diol

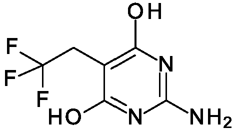

To an orange solution of dimethyl 2-(2,2,2-trifluoroethyl)propanedioate (1.19 g, 4.45 mmol) in ethanol (11 mL) was added guanidine hydrochloride (424.7 mg, 4.45 mmol). The suspension was stirred at 75 °C for 5 hours, then diluted with water (5 mL) and acetic acid (902.34 mg, 860.19 uL, 15.03 mmol). The mixture was heated at 80 °C for 10 min, cooled to room temperature, concentrated in vacuo and recrystallized from ethanol to provide the title compound as a brown solid (240 mg, 25 % yield). MS (ESI) m/z= 208.1 [M-H]-

### Step 3: 4,6-dichloro-5-(2,2,2-trifluoroethyl)pyrimidin-2-amine

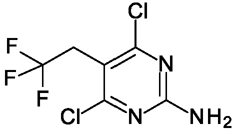

2-amino-5-(2,2,2-trifluoroethyl)pyrimidine-4,6-diol (240 mg, 1.09 mmol) was suspended in phosphorus oxychloride (2.01 g, 1.22 mL, 13.08 mmol) and heated at 100 °C for 16 hours. The reaction mixture was cooled to room temperature, poured into ice/water and extracted with ethyl acetate. The organic layers were dried over sodium sulfate, filtered and concentrated in vacuo. The residue was purified by flash chromatography over silica gel using a gradient ethylacetate/heptane 0-17% to provide the title compound as a light brown solid (108 mg, 38 % yield). MS (ESI) m/z= 246.1 [M+H]+

### Step 4: 4-chloro-6-methoxy-5-(2,2,2-trifluoroethyl)pyrimidin-2-amine

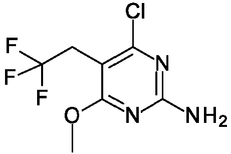

A solution of 4,6-dichloro-5-(2,2,2-trifluoroethyl)pyrimidin-2-amine (108 mg, 0.439 mmol) and sodium methoxide (23.71 mg, 0.439 mmol) in methanol (2 mL) was heated to 45 °C and stirred for 23 hours. The reaction mixture was poured into water and extracted with ethyl acetate. The organic layers were dried over sodium sulfate, filtered and concentrated in vacuo. The residue was purified by flash chromatography over silica gel using a gradient ethylacetate/heptane 0-20% to provide the title compound as a colorless solid (83 mg, 74 % yield). MS (ESI) m/z= 242.1 [M+H]+

### Step 5: 4-methoxy-5-(2,2,2-trifluoroethyl)pyrimidin-2-amine

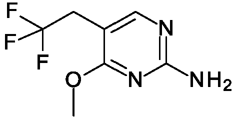

A suspension of 4-chloro-6-methoxy-5-(2,2,2-trifluoroethyl)pyrimidin-2-amine (83 mg, 0.326 mmol) and palladium on activated charcoal (70 mg) in ethyl acetate (3 mL) was stirred under hydrogen and stirred under 1 atm. of hydrogen at room temperature for 9 hours. The reaction mixture was filtered through a pad of celite and concentrated in vacuo. The residue was purified by flash chromatography over silica gel using a gradient ethylacetate/heptane 0-80% to provide the title compound as a colorless solid (35 mg, 49 % yield). MS (ESI): m/z= 208.2 [M+H]+

### Intermediate B45: 4-methoxy-5-(3,3,3-trifluoropropyl)pyrimidin-2-amine

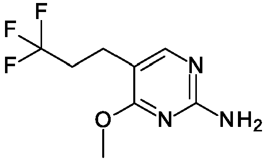

### Step 1: 4-methoxy-N,N-bis[(4-methoxyphenyl)methyl]-5-(3,3,3-trifluoropropyl)pyrimidin-2-amine

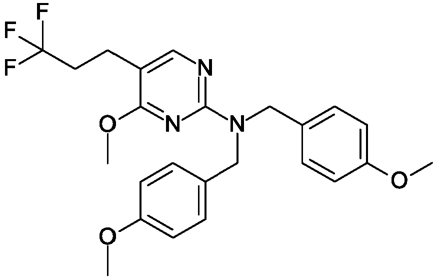

To a solution of (5-bromo-4-methoxy-pyrimidin-2-yl)-bis(p-anisyl)amine (200.0 mg, 0.450 mmol, intermediate B3, step 1) in 1,4-dioxane (10 mL) and water (2 mL) was added potassium 3,3,3-trifluoropropane-1-trifluoroborate (459.04 mg, 2.25 mmol), cesium carbonate (1466.6 mg, 4.5 mmol) and 1,1'-bis(diphenylphosphino)ferrocene-palladium(II) dichloride dichloromethane complex (16.47 mg, 0.020 mmol). The mixture was stirred for 12 hours at 100 °C, then poured into water and extracted twice with ethyl acetate. The organic layers were washed with successively with water and brine, dried over sodium sulfate, filtered and concentrated in vacuo. The residue was purified by flash chromatography over silica gel using a gradient ethylacetate/petroleum ether 0-20% to provide the title compound as a colorless oil (150 mg, 46 % yield). MS (ESI) m/z= 462.1 [M+H]+

### Step 2: 4-methoxy-5-(3,3,3-trifluoropropyl)pyrimidin-2-amine

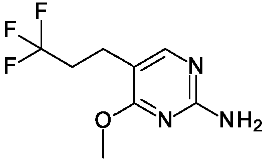

A solution of 4-methoxy-N,N-bis[(4-methoxyphenyl)methyl]-5-(3,3,3-trifluoropropyl)pyrimidin-2-amine (130.0 mg, 0.280 mmol) in trifluoroacetic acid (3.0 mL, 40.39 mmol) was stirred for 2 hours at 60 °C, then poured into sat. NaHCO3 and extracted twice with ethyl acetate. The organic layers were successively washed with water and brine, dried over sodium sulfate, filtered and concentrated in vacuo. The residue was purified by preparative TLC (eluent: ethylacetate/petroleum ether 1/1) to provide the title compound as a white solid (20 mg, 32 % yield). MS (ESI) m/z= 222.4 [M+H]+

### Intermediate B46: 5-(1,1-difluoroethyl)-4-methoxy-pyrimidin-2-amine

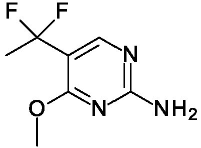

### Step 1: 1-(2-chloro-4-methoxy-pyrimidin-5-yl)ethanone

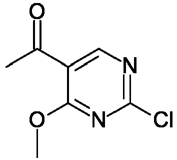

To a solution of tributyl(1-ethoxyvinyl)tin (4.85 g, 13.43 mmol) and 5-bromo-2-chloro-4-methoxy-pyrimidine (3.0 g, 13.43 mmol, CAS: 57054-92-9) in N,N-dimethylformamide (50 mL) was added bis(triphenylphosphine)palladium(II) dichloride (0.94 g, 1.34 mmol) under nitrogen atmosphere. The mixture was stirred at 120 °C for 3 hours, then poured into water and extracted three times with ethylacetate. The organic layers were concentrated in vacuo. Ethyl acetate (100 mL) and a 2 M HCl solution (200 mL) were added to the residue. The mixture was stirred at room temperature for 20 min and extracted three times with ethyl acetate. The organic layers were dried over sodium sulfate, filtered and concentrated in vacuo. The residue was purified by flash chromatography over silica gel using a gradient ethylacetate/petroleum ether 0-10% to provide the title compound as a white solid (1.32 g, 53 % yield). MS (ESI) m/z= 186.7 [M+H]+

### Step 2: 2-chloro-5-(1,1-difluoroethyl)-4-methoxy-pyrimidine

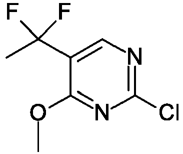

To a solution of 1-(2-chloro-4-methoxy-pyrimidin-5-yl)ethanone (1.32 g, 7.07 mmol) in dichloromethane (20 mL) was added diethylaminosulfur trifluoride (5.7 g, 35.37 mmol) at -78 °C under nitrogen atmosphere. The reaction mixture was stirred at 20 °C for 12 hours under nitrogen. Four times was added diethylaminosulfur trifluoride (5.7 g, 35.37 mmol) at -78 °C under nitrogen and the reaction mixture stirred at 20 °C for 12 hours under nitrogen. The reaction was then poured into sat. NaHCO3 and extracted twice with dichloromethane. The organic layers were washed with brine, dried over sodium sulfate, filtered and concentrated in vacuo. The residue was purified by flash chromatography over silica gel using a gradient ethylacetate/petroleum ether 0-13% to provide the title compound as a yellow oil (1 g, 68 % yield). MS (ESI) m/z= 209.0 [M+H]+

### Step 3: 5-(1,1-difluoroethyl)-4-methoxy-pyrimidin-2-amine

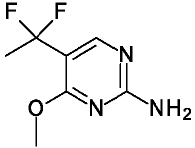

A mixture of 2-chloro-5-(1,1-difluoroethyl)-4-methoxy-pyrimidine (300.0 mg, 1.44 mmol) and a solution of NH3 in tetrahydrofuran (5.0 mL, 25 mmol) was stirred at 60 °C for 16 hours and concentrated in vacuo. The residue was purified by flash chromatography over silica gel using a gradient ethylacetate/petroleum ether 0-43% to provide the title compound as a white solid (100 mg, 26 % yield). MS (ESI) m/z= 190.0 [M+H]+

### Intermediate B47: 4-methoxy-5-(2,2,3,3-tetrafluoropropyl)pyrimidin-2-amine

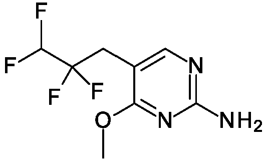

### Step 1: 1-[2-[bis[(2,4-dimethoxyphenyl)methyl]amino]-4-methoxy-pyrimidin-5-yl]-2,2,3,3-tetrafluoro-propan-1-ol

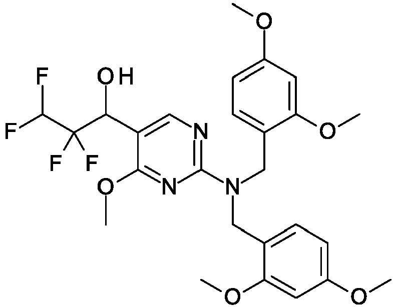

To a solution of 5-bromo-N,N-bis[(2,4-dimethoxyphenyl)methyl]-4-methoxy-pyrimidin-2-amine (1.8 g, 3.57 mmol, intermediate B39, step 1) in tetrahydrofuran (12 mL) was added a 1.6 M n-butyllithium solution in hexanes (2.9 mL, 4.64 mmol) dropwise at -78 °C. After 1 hour, 2,2,3,3-tetrafluoropropionic acid ethyl ester (1.86 g, 10.71 mmol) was added dropwise and the reaction mixture was stirred for an additional hour at -78 °C before warming to 0 °C. After 20 min, methanol (2.4 mL) was added, followed by sodium borohydride (135.01 mg, 3.57 mmol). The reaction mixture was quenched with a saturated solution of ammonium chloride and extracted with ethyl acetate. The organic layers were dried over sodium sulfate, filtered and concentrated in vacuo. The residue was purified by flash chromatography over silica gel using a gradient ethylacetate/heptane 0-15% to provide the title compound as a colorless solid (1.25 g, 54 % yield). MS (ESI) m/z= 556.4 [M+H]+

### Step 2: N,N-bis[(2,4-dimethoxyphenyl)methyl]-4-methoxy-5-(2,2,3,3-tetrafluoropropyl)pyrimidin-2-amine

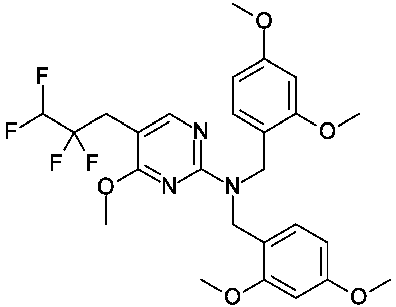

1-[2-[bis[(2,4-dimethoxyphenyl)methyl]amino]-4-methoxy-pyrimidin-5-yl]-2,2,3,3-tetrafluoro-propan-1-ol (745 mg, 1.14 mmol) and 1,1'-thiocarbonyldiimidazole (304.72 mg, 1.71 mmol) were mixed in tetrahydrofuran (12.42 mL) and stirred at 70 °C for 5 hours. The reaction mixture was cooled to room temperature, diluted with ethyl acetate and washed with brine. The organic layer was dried over sodium sulfate, filtered and concentrated in vacuo. The residue was dissolved in toluene (8 mL) and 2,2'-azobis(2-methylpropionitrile) (37.44 mg, 0.228 mmol) followed by tri-n-butyltin hydride (1.66 g, 1.52 mL, 5.7 mmol) were added. The reaction mixture was stirred at 85 °C for 2 hours, cooled to room temperature, put on silica gel, concentrated in vacuo and purified by flash chromatography using a gradient ethylacetate/heptane 0-22% to provide the title compound as a colorless viscous oil (99 mg, 16 % yield). MS (ESI) m/z= 540.4 [M+H]+

### Step 3: 4-methoxy-5-(2,2,3,3-tetrafluoropropyl)pyrimidin-2-amine

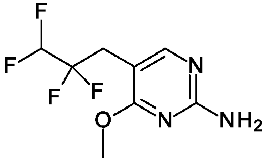

N,N-bis[(2,4-dimethoxyphenyl)methyl]-4-methoxy-5-(2,2,3,3-tetrafluoropropyl)pyrimidin-2-amine (99 mg, 0.184 mmol) was dissolved in trifluoroacetic acid (2.96 g, 2 mL, 141.48 mmol). The reaction mixture was stirred at room temperature for 18 hours and concentrated in vacuo. The residue was poured into sat. NaHCO3 and extracted with ethyl acetate. The organic layers were dried over sodium sulfate, filtered and concentrated in vacuo. The residue was purified by flash chromatography over silica gel using a gradient ethylacetate/heptane 0-10% to provide the title compound as a colorless solid (31 mg, 67 % yield). MS (ESI) m/z= 240.2 [M+H]+

### Intermediate B48: 3-(2-amino-4-methoxy-pyrimidin-5-yl)propanenitrile

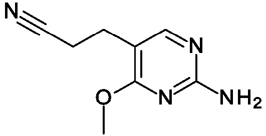

### Step 1: 3-[2-[bis[(4-methoxyphenyl)methyl]amino]-4-methoxy-pyrimidin-5-yl]propanenitrile

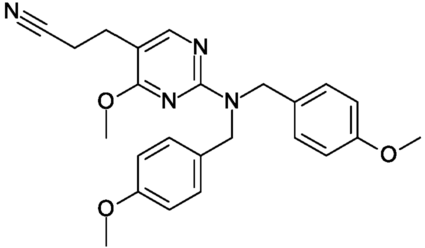

To a solution of (5-bromo-4-methoxy-pyrimidin-2-yl)-bis(p-anisyl)amine (4000.0 mg, 9 mmol, intermediate B3, step 1) in methoxycyclopentane (20 mL) was added 3-(4,4,5,5-tetramethyl-1,3,2-dioxaborolan-2-yl)propanenitrile (1955.78 mg, 10.8 mmol), cesium carbonate (8799.6 mg, 27.01 mmol) and cataCXium^{®} A Pd G3 (655.63 mg, 0.9 mmol, CAS: 1651823-59-4) under nitrogen. The mixture was purged three times with nitrogen, stirred for 12 hours at 90 °C, then poured into water and extracted three times with ethyl acetate. The organic layers were dried over sodium sulfate, filtered and concentrated in vacuo. The residue was purified by flash chromatography over silica gel using a gradient ethylacetate/petroleum ether 0-30% to provide the title compound as a yellow oil (3100 mg, 82 % yield). MS (ESI) m/z= 418.9 [M+H]+

### Step 2: 3-(2-amino-4-methoxy-pyrimidin-5-yl)propanenitrile

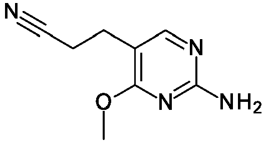

A mixture of 3-[2-[bis[(4-methoxyphenyl)methyl]amino]-4-methoxy-pyrimidin-5-yl]propanenitrile (2000.0 mg, 4.78 mmol) in trifluoroacetic acid (21 mL) was stirred at room temperature for 48 hours and concentrated in vacuo. The reaction mixture was quenched with sat. NaHCO3 and extracted three times with ethyl acetate. The organic layers were dried over sodium sulfate, filtered and concentrated in vacuo. The residue was purified by flash chromatography over silica gel using a gradient ethylacetate/petroleum ether 0-100% to provide the title compound as a yellow solid (674 mg, 78 % yield). MS (ESI) m/z= 179.0 [M+H]+

### Intermediate B49: 4-methoxy-5-(2-methoxyethyl)pyrimidin-2-amine

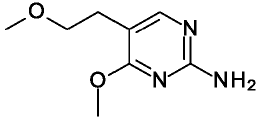

### Step 1: 2-[2-[bis[(4-methoxyphenyl)methyl]amino]-4-methoxy-pyrimidin-5-yl]ethanol

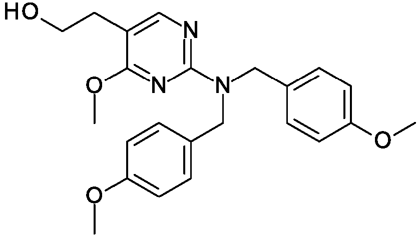

2-[2-[bis[(4-methoxyphenyl)methyl]amino]-4-methoxy-pyrimidin-5-yl]acetaldehyde (1.07 g, 2.22 mmol, intermediate B40, step 2) was dissolved in tetrahydrofuran, extra dry (12 mL) under argon and cooled to -70 °C. 1 M DIBAL-H in tetrahydrofuran (2.67 mL, 2.67 mmol) was added dropwise. After addition, the reaction mixture was allowed to warm to 0 °C, stirred for 1 hour and quenched by adding a saturated solution of sodium tartrate in water. The mixture was then poured into water and extracted with dichloromethane. The organic layers were washed with brine, dried over sodium sulfate, filtered and concentrated in vacuo. The residue was purified by flash chromatography over silica gel using a gradient ethylacetate/heptane 0-80% to provide the title compound as a light yellow oil (342 mg, 34 % yield). MS (ESI) m/z= 410.3 [M+H]+

### Step 2: 4-methoxy-5-(2-methoxyethyl)-N,N-bis[(4-methoxyphenyl)methyl]pyrimidin-2-amine

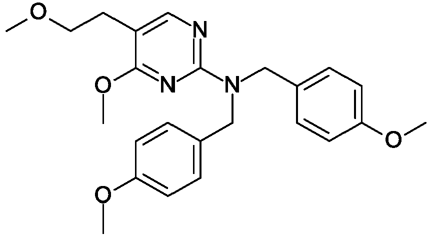

A solution of 2-[2-[bis[(4-methoxyphenyl)methyl]amino]-4-methoxy-pyrimidin-5-yl]ethanol (342 mg, 0.835 mmol) in N,N-dimethylformamide, extra dry (3 mL) was added dropwise at 0 °C to a suspension of sodium hydride (36.75 mg, 0.919 mmol) in N,N-dimethylformamide, extra dry (1 mL). The reaction mixture was stirred for 30 min at 0 °C. Methyl iodide (130.4 mg, 57.19 uL, 0.919 mmol) was then added and the reaction mixture was stirred at room temperature for 3 hours, poured into water and extracted twice with ethyl acetate. The organic layers were dried over sodium sulfate, filtered and concentrated in vacuo. The residue was purified by flash chromatography over silica gel using a gradient ethylacetate/heptane 0-80% to provide the title compound as a colorless oil (225 mg, 61 % yield). MS (ESI) m/z= 424.3 [M+H]+

### Step 3: 4-methoxy-5-(2-methoxyethyl)pyrimidin-2-amine

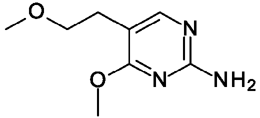

To a stirred solution of 4-methoxy-5-(2-methoxyethyl)-N,N-bis[(4-methoxyphenyl)methyl]pyrimidin-2-amine (225 mg, 0.510 mmol) in dichloromethane, extra dry (5 mL) was added trifluoroacetic acid (3.49 g, 2.36 mL, 30.6 mmol). The reaction mixture was stirred at 50 °C for 40 hours and concentrated in vacuo. The residue was poured into sat. NaHCO3 and extracted twice with ethyl acetate. The organic layers were dried over sodium sulfate, filtered and concentrated in vacuo. The residue was purified by flash chromatography over silica gel using a gradient ethylacetate/heptane 0-100% to provide the title compound as a white solid (48 mg, 51 % yield). MS (ESI) m/z= 184.1 [M+H]+

### Intermediate B50: 5-(difluoromethoxymethyl)-4-methoxy-pyrimidin-2-amine

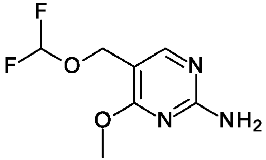

### Step 1: [2-[bis[(2,4-dimethoxyphenyl)methyl]amino]-4-methoxy-pyrimidin-5-yl]methanol

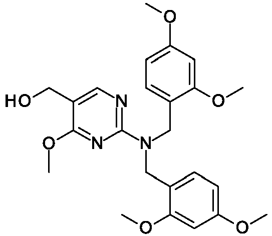

To a solution of 5-bromo-N,N-bis[(2,4-dimethoxyphenyl)methyl]-4-methoxy-pyrimidin-2-amine (2.1 g, 4.16 mmol, intermediate B39, step 1) in tetrahydrofuran (14 mL) was added a 1.6 M n-butyllithium solution in hexanes (3.38 mL, 5.41 mmol) dropwise at -78 °C. After 1.5 hours, N,N-dimethylformamide (1.52 g, 1.61 mL, 20.82 mmol) was added and the reaction mixture was stirred for an additional hour at -78 °C before warming to 0 °C. After 15 min, methanol (2.8 mL) was added, followed by sodium borohydride (157.51 mg, 4.16 mmol). The reaction mixture was stirred at 0 °C for 15 min, quenched with a saturated solution of ammonium chloride and extracted with ethyl acetate. The organic layers were dried over sodium sulfate, filtered and concentrated in vacuo. The residue was purified by flash chromatography over silica gel using a gradient ethylacetate/heptane 0-45% to provide the title compound as a colorless amorphous (1.44 g, 72 % yield). MS (ESI) m/z= 456.4 [M+H]+

### Step 2: 5-(difluoromethoxymethyl)-N,N-bis[(2,4-dimethoxyphenyl)methyl]-4-methoxy-pyrimidin-2-amine

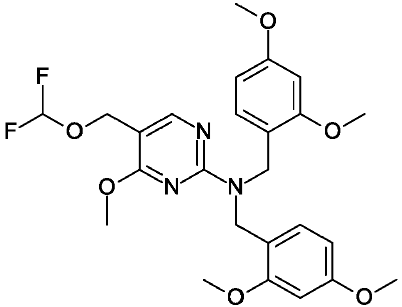

To a colorless solution of [2-[bis[(2,4-dimethoxyphenyl)methyl]amino]-4-methoxy-pyrimidin-5-yl]methanol (825 mg, 1.72 mmol) in dichloromethane (7 mL) and water (7 mL) was added at 0 °C, potassium acetate (1.01 g, 10.32 mmol) and (bromodifluoromethyl)trimethylsilane (698.92 mg, 533.53 uL, 3.44 mmol) dropwise. (bromodifluoromethyl)trimethylsilane (698.92 mg, 533.53 uL, 3.44 mmol) was added three times. Then the reaction mixture was quenched with sat. NaHCO3 and extracted three times with ethyl acetate. The organic layers were dried over sodium sulfate, filtered and concentrated in vacuo. The residue was purified by flash chromatography over silica gel using a gradient ethylacetate/heptane 0-25% to provide the title compound as a colorless viscous oil (107.7 mg, 12 % yield). MS (ESI) m/z= 506.3 [M+H]+

### Step 3: 5-(difluoromethoxymethyl)-4-methoxy-pyrimidin-2-amine

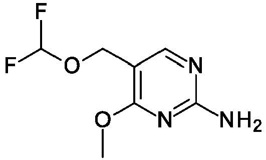

To a colorless solution of 5-(difluoromethoxymethyl)-N,N-bis[(2,4-dimethoxyphenyl)methyl]-4-methoxy-pyrimidin-2-amine (107.7 mg, 0.209 mmol) in dichloromethane, extra dry (2 mL) was added trifluoroacetic acid (2.96 g, 2 mL, 25.96 mmol). The pink solution was stirred at 50 °C for 50 min and concentrated in vacuo. The residue was poured into sat. NaHCO3 and extracted twice with ethyl acetate. The organic layers were washed with brine, dried over sodium sulfate, filtered and concentrated in vacuo. The residue was purified by flash chromatography over silica gel using a gradient ethylacetate/heptane 0-37% to provide the title compound as a white solid (29 mg, 64 % yield). MS (ESI) m/z= 206.1 [M+H]+

### Intermediate B51: 4-methoxy-5-(oxetan-3-yl)pyrimidin-2-amine

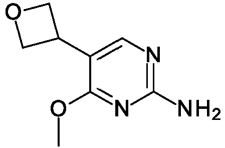

[4,4'-bis(1,1-dimethylethyl)-2,2'-bipyridine-n1,n1']bis[3,5-difluoro-2-[5-(trifluoromethyl)-2-pyridinyl-n]phenyl-c]iridium(III) hexafluorophosphate (8.25 mg, 0.007 mmol, CAS: 870987-63-6) was added to the reaction vial, followed by sodium carbonate (154.4 mg, 1.47 mmol), (5-bromo-4-methoxy-pyrimidin-2-yl)amine (150 mg, 0.735 mmol, CAS: 36082-45-8), 3-bromooxetane (201.42 mg, 122. uL, 1.47 mmol) and tris(trimethylsilyl)silane (244.98 mg, 303.95 uL, 0.985 mmol). Ethylene glycol dimethyl ether, extra dry (11.86 mL) was added and the reaction mixture was degassed by bubbling argon through the mixture for 2 min. Finally nickel(II) chloride ethylene glycol dimethyl ether complex (807.72 ug, 0.004 mmol, 0.005 eq) and 4,4'-bis(tert-butyl)-2,2'-bipyridine (986.66 ug, 0.004 mmol, 0.005 eq) were added and the reaction was degassed again for 5 min. The vial was placed in a photoreactor and irradiated with 450 nm for 14 hours (stirred at 900 rpm, LED 100 % power). The reaction mixture was filtered and concentrated in vacuo. The residue was purified by flash chromatography over silica gel using a gradient ethylacetate/heptane 0-100% then methanol/ethylacetate 0-10% to provide the title compound as a light yellow solid (28.8 mg, 21 % yield). MS (ESI) m/z= 182.1 [M+H]+

### Intermediate B52: 4-methoxy-5-[2-(oxetan-3-yl)ethynyl]pyrimidin-2-amine

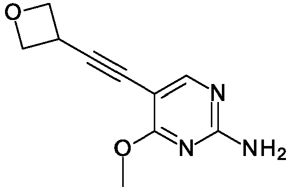

To a solution of 5-iodo-4-methoxy-pyrimidin-2-amine (300.0 mg, 1.2 mmol, CAS: 89322-66-7) and 3-ethynyloxetane (117.74 mg, 1.43 mmol) in N,N-dimethylformamide (3 mL) was added bis(triphenylphosphine)palladium(II) dichloride (83.88 mg, 0.120 mmol), copper(I) iodide (68.05 mg, 0.360 mmol) and triethylamine (362.79 mg, 3.59 mmol) under nitrogen atmosphere. The mixture was stirred at 30 °C for 12 hours, poured into water and extracted three times with ethyl acetate. The organic layers were dried over sodium sulfate, filtered and concentrated in vacuo. The residue was purified by flash chromatography over silica gel using a gradient ethylacetate/petroleum ether 0-50% to provide the title compound as a yellow solid (150 mg, 61 % yield). MS (ESI) m/z= 206.0 [M+H]+

### Intermediate B53: 4-methoxy-5-[2-(oxetan-3-yl)ethyl]pyrimidin-2-amine

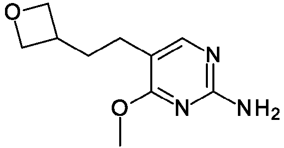

To a solution of 4-methoxy-5-[2-(oxetan-3-yl)ethynyl]pyrimidin-2-amine (60 mg, 0.290 mmol, intermediate B52) in methanol (4 mL) was added palladium on activated charcoal (6 mg, 0.056 mmol). The reaction mixture was purged with hydrogen and stirred under 1 atm. of hydrogen at room temperature for 12 hours. The reaction mixture was filtered through a pad of celite and concentrated in vacuo to provide the title compound as a light yellow solid (30 mg, 34 % yield). MS (ESI): m/z= 210.1 [M+H]+

### Intermediate B54: 5-(fluoromethoxy)-4,6-dimethoxy-pyrimidin-2-amine

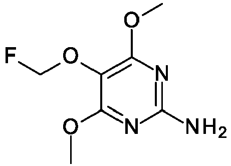

### Step 1: 5-bromo-4,6-dimethoxy-pyrimidin-2-amine

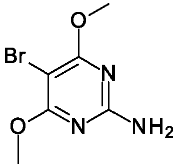

To a stirred solution of (4,6-dimethoxypyrimidin-2-yl)amine (7 g, 44.22 mmol, CAS: 36315-01-2) in acetonitrile (100 mL) was added a solution of N-bromosuccinimide (10.33 g, 57.48 mmol) in acetonitrile (100 mL) dropwise at room temperature. The reaction mixture was stirred at room temperature for 30 min. The resulting white suspension was diluted with ethyl acetate and washed with water. The organic layer was dried over sodium sulfate, filtered, diluted with heptane and concentrated in vacuo. The precipitate was filtered off and washed with heptane to provide the title compound as a white solid (9.26 g, 87 % yield). MS (ESI) m/z= 234.1 [M+H]+

### Step 2: 5-bromo-4,6-dimethoxy-N,N-bis[(4-methoxyphenyl)methyl]pyrimidin-2-amine

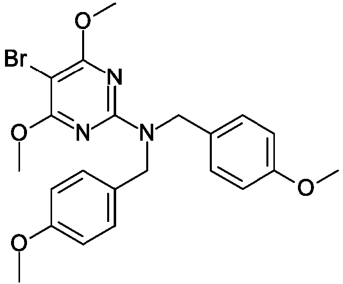

A solution of 5-bromo-4,6-dimethoxy-pyrimidin-2-amine (517 mg, 2.21 mmol) in N,N-dimethylacetamide (9 mL) was cooled to 0 °C. Sodium hydride (265.05 mg, 6.63 mmol) was added portionwise (3 x 88 mg). The stirring was continued at 0 °C for 30 min. 4-methoxybenzyl chloride (706. mg, 608.62 uL, 4.42 mmol) was added dropwise. The reaction mixture was allowed to warm to room temperature, stirred for 1 hour, carefully quenched with a saturated ammonium chloride solution, poured into water and extracted twice with ethyl acetate. The organic layers were dried over sodium sulfate, filtered and concentrated in vacuo. The residue was purified by flash chromatography over silica gel using a gradient ethylacetate/heptane 0-20% to provide the title compound as a white solid (1.11 g, 100 % yield). MS (ESI) m/z= 476.2 [M+H]+

### Step 3: 2-[bis[(4-methoxyphenyl)methyl]amino]-4,6-dimethoxy-pyrimidin-5-ol

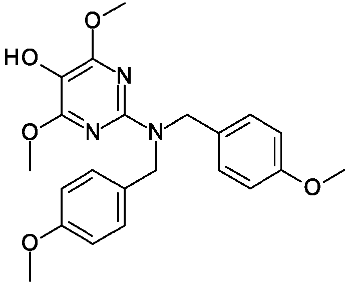

To a colorless solution of 5-bromo-4,6-dimethoxy-N,N-bis[(4-methoxyphenyl)methyl]pyrimidin-2-amine (500 mg, 1 mmol) in tetrahydrofuran (3.5 mL) was added a 1.6 M n-butyllithium solution in hexanes (699.71 mg, 813.61 uL, 1.3 mmol) dropwise at -78 °C. The resulting yellow solution was stirred at -78 °C for 30 min. Trimethyl borate (156.08 mg, 167.47 uL, 1.5 mmol) was added dropwise and the stirring was continued at -78 °C for 1.5 hours. The reaction mixture was allowed to warm to 0 °C and acetic acid (120.27 mg, 114.62 uL, 2 mmol) was added dropwise, followed by hydrogen peroxide 35% (145.98 mg, 131.51 uL, 1.5 mmol). The stirring was continued at 0 °C for 1.5 hours. The resulting pink suspension was poured into a 0.1 N sodium thiosulfate solution and extracted twice with ethyl acetate. The organic layers were washed with brine, dried over sodium sulfate, filtered and concentrated in vacuo. The residue was purified by flash chromatography over silica gel using a gradient ethylacetate/heptane 0-30% to provide the title compound as a light yellow viscous oil (194 mg, 47 % yield). MS (ESI) m/z= 412.3 [M+H]+

### Step 4: 5-(fluoromethoxy)-4,6-dimethoxy-N,N-bis[(4-methoxyphenyl)methyl]pyrimidin-2-amine

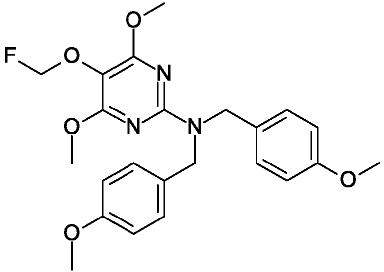

To a solution of 2-[bis[(4-methoxyphenyl)methyl]amino]-4,6-dimethoxy-pyrimidin-5-ol (300.0 mg, 0.730 mmol) in N,N-dimethylformamide (10 mL), sodium hydride (26.25 mg, 1.09 mmol) was added and the mixture was heated at 50 °C until gas evolution ceased (15 min). The reaction mixture was cooled to room temperature and fluoro(iodo)methane (233.22 mg, 1.46 mmol) was added. The resulting mixture was stirred at room temperature for 16 hours, diluted with water and extracted with ethyl acetate. The organic layer was washed twice with water, once with brine, dried over sodium sulfate, filtered and concentrated in vacuo to provide the title compound as a brown oil (250 mg, 77 % yield). MS (ESI) m/z= 444.4 [M+H]+

### Step 5: 5-(fluoromethoxy)-4,6-dimethoxy-pyrimidin-2-amine

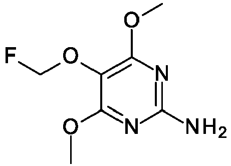

5-(fluoromethoxy)-4,6-dimethoxy-N,N-bis[(4-methoxyphenyl)methyl]pyrimidin-2-amine (250.0 mg, 0.560 mmol) was dissolved in trifluoroacetic acid (2.61 mL, 33.82 mmol) and heated at 50 °C for 15 hours. The mixture was concentrated in vacuo. The residue was poured into a sodium carbonate solution and extracted with ethyl acetate. The organic layer was dried over sodium sulfate, filtered and concentrated in vacuo to provide the title compound (200 mg, 63 % yield). MS (ESI) m/z= 204.2 [M+H]+

### Intermediate B55: 5-(difluoromethoxy)-4,6-dimethoxy-pyrimidin-2-amine

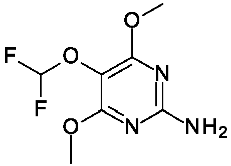

### Step 1: 5-(difluoromethoxy)-4,6-dimethoxy-N,N-bis[(4-methoxyphenyl)methyl]pyrimidin-2-amine

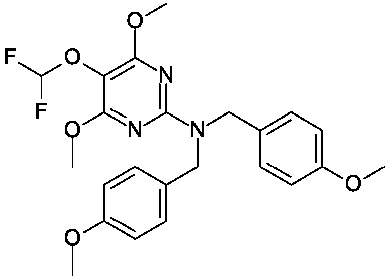

To a solution of 2-[bis[(4-methoxyphenyl)methyl]amino]-4,6-dimethoxy-pyrimidin-5-ol (130 mg, 0.316 mmol, intermediate B54, step 3) in acetonitrile (5 mL) was added a 5 M potassium hydroxide solution (1.26 mL, 6.32 mmol) dropwise at 0 °C, followed by addition of bromodifluoromethyl diethylphosphonate (168.72 mg, 112.26 uL, 0.632 mmol) in acetonitrile (1 mL) dropwise at 0 °C. The reaction mixture was stirred at 0 °C for 10 min. The resulting light yellow biphasic mixture was poured into water and extracted twice with ethyl acetate. The organic layers were dried over sodium sulfate, filtered and concentrated in vacuo. The residue was purified by flash chromatography over silica gel using a gradient ethylacetate/heptane 0-30% to provide the title compound as a white solid (57 mg, 39 % yield). MS (ESI) m/z= 462.3 [M+H]+

### Step 2: 5-(difluoromethoxy)-4,6-dimethoxy-pyrimidin-2-amine

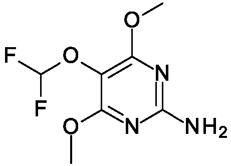

To a stirred solution of 5-(difluoromethoxy)-4,6-dimethoxy-N,N-bis[(4-methoxyphenyl)methyl]pyrimidin-2-amine (56 mg, 0.121 mmol) in dichloromethane (100 uL) was added trifluoroacetic acid (838.62 mg, 563.21 uL, 7.28 mmol). The reaction mixture was stirred at room temperature for 40 hours, at 50 °C for 6 hours and concentrated in vacuo. The residue was poured into sat. NaHCO3 and extracted twice with ethyl acetate. The organic layers were dried over sodium sulfate, filtered and concentrated in vacuo. The residue was purified by flash chromatography over silica gel using a gradient ethylacetate/heptane 0-100% to provide the title compound as a white solid (24 mg, 89 % yield). MS (ESI) m/z= 222.1 [M+H]+

### Intermediate B56: 5-(2-fluoroethoxy)-4,6-dimethoxy-pyrimidin-2-amine

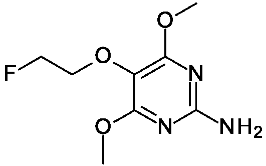

### Step 1: 5-(2-fluoroethoxy)-4,6-dimethoxy-N,N-bis[(4-methoxyphenyl)methyl]pyrimidin-2-amine

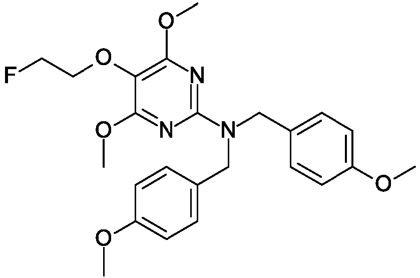

A suspension of 2-[bis[(4-methoxyphenyl)methyl]amino]-4,6-dimethoxy-pyrimidin-5-ol (2 g, 4.52 mmol, intermediate B54, step 3), potassium carbonate (1.89 g, 13.56 mmol) and 1-bromo-2-fluoroethane (1.76 g, 1.03 mL, 13.56 mmol) in N,N-dimethylformamide (45 mL) was heated to 80 °C and stirred for 2.5 hours. The reaction mixture was poured into brine and extracted twice with ethyl acetate. The organic layers were dried over sodium sulfate, filtered and concentrated in vacuo. The residue was purified by flash chromatography over silica gel using a gradient ethylacetate/heptane 0-30% to provide the title compound as a white solid (1.79 g, 85 % yield). MS (ESI) m/z= 458.3 [M+H]+

### Step 2: 5-(2-fluoroethoxy)-4,6-dimethoxy-pyrimidin-2-amine

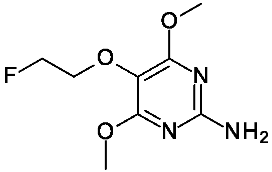

To a stirred solution of 5-(2-fluoroethoxy)-4,6-dimethoxy-N,N-bis[(4-methoxyphenyl)methyl]pyrimidin-2-amine (1.79 g, 3.83 mmol) in dichloromethane (4 mL) was added trifluoroacetic acid (26.23 g, 17.62 mL, 230.06 mmol). The reaction mixture was stirred at 50 °C for 3 hours, at room temperature for 15 hours and concentrated in vacuo. The residue was poured into sat. NaHCO3 and extracted twice with ethyl acetate. The organic layers were dried over sodium sulfate, filtered and concentrated in vacuo. The residue was purified by flash chromatography over silica gel using a gradient ethylacetate/heptane 0-60% to provide the title compound as an off-white solid (885 mg, 100 % yield). MS (ESI) m/z= 218.1 [M+H]+

### Intermediate B57: 5-(2,2-difluoroethoxy)-4,6-dimethoxy-pyrimidin-2-amine

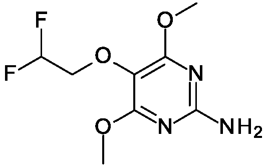

### Step 1: 5-(2,2-difluoroethoxy)-4,6-dimethoxy-N,N-bis[(4-methoxyl)henyl)methyl]rimidin-2-amine

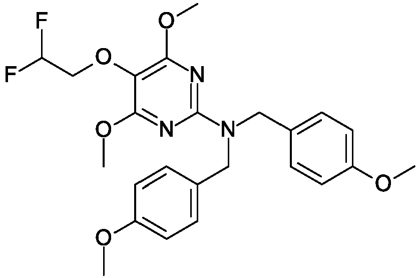

To a solution of 2-[bis[(4-methoxyphenyl)methyl]amino]-4,6-dimethoxy-pyrimidin-5-ol (100 mg, 0.214 mmol, intermediate B54, step 3) in N,N-dimethylformamide (1.75 mL) was added potassium carbonate (88.68 g, 0.642 mmol) and 1,1-difluoro-2-iodoethane (123.16 mg, 56.5 uL, 0.642 mmol). The reaction mixture was stirred at 80 °C for 1.5 hours, cooled to room temperature, poured into water and extracted twice with ethyl acetate. The organic layers were dried over sodium sulfate, filtered and concentrated in vacuo. The residue was purified by flash chromatography over silica gel using a gradient ethylacetate/heptane 0-20% to provide the title compound as an off-white solid (92 mg, 85 % yield). MS (ESI) m/z= 476.2 [M+H]+

### Step 1: 5-(2,2-difluoroethoxy)-4,6-dimethoxy-pyrimidin-2-amine

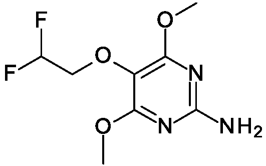

To a stirred solution of 5-(2,2-difluoroethoxy)-4,6-dimethoxy-N,N-bis[(4-methoxyphenyl)methyl]pyrimidin-2-amine (92 mg, 0.193 mmol) in dichloromethane (0.340 mL) was added trifluoroacetic acid (1.34 g, 897.97 uL, 11.61 mmol) at 0 °C. The reaction mixture was stirred at room temperature for 18 hours and at 50 °C for 4 hours. The resulting red solution was concentrated in vacuo, poured into sat. NaHCO3 and extracted twice with ethyl acetate. The organic layers were dried over sodium sulfate, filtered and concentrated in vacuo. The residue was purified by flash chromatography over silica gel using a gradient ethylacetate/heptane 0-30% to provide the title compound as a light yellow solid (41 mg, 87 % yield). MS (ESI) m/z= 236.2 [M+H]+

### Intermediate B58: 4,6-dimethoxy-5-(1,1,2-trifluoroethoxy)pyrimidin-2-amine

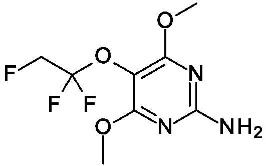

### Step 1: ethyl 2-[2-[bis[(4-methoxyi)henyl)methyl]mino]-4,6-dimethoxy-pyrimidin-5-yl]oxy-2,2-difluoro-acetate

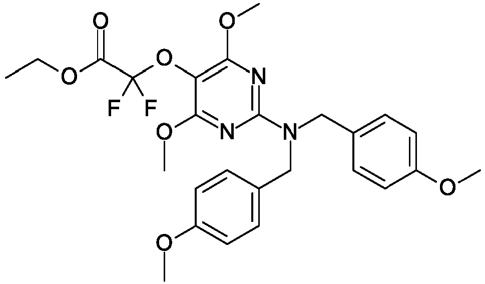

A mixture of 2-[bis[(4-methoxyphenyl)methyl]amino]-4,6-dimethoxy-pyrimidin-5-ol (1 g, 2.26 mmol, intermediate B54, step 3), potassium carbonate (946.62 mg, 6.78 mmol) and ethyl bromodifluoroacetate (1.42 g, 903.79 uL, 6.78 mmol) in N,N-dimethylformamide (20 mL) was stirred at 80 °C for 20 hours, poured into brine and extracted twice with ethyl acetate. The organic layers were dried over sodium sulfate, filtered and concentrated in vacuo. The residue was purified by flash chromatography over silica gel using a gradient ethylacetate/heptane 0-20% to provide the title compound as a colorless viscous oil (449 mg, 34 % yield). MS (ESI) m/z= 534.3 [M+H]+

### Step 2: 2-[2-[bis[(4-methoxyphenyl)methyl]amino]-4,6-dimethoxy-pyrimidin-5-yl]oxy-2,2-difluoro-ethanol

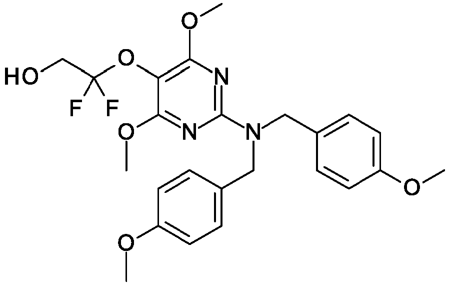

A solution of ethyl 2-[2-[bis[(4-methoxyphenyl)methyl]amino]-4,6-dimethoxy-pyrimidin-5-yl]oxy-2,2-difluoro-acetate (447 mg, 0.771 mmol) in tetrahydrofuran (11 mL) was cooled to 0 °C. 1 M lithium aluminium hydride solution in THF (1.16 mL, 1.16 mmol) was added dropwise. The reaction mixture was stirred at 0 °C for 30 min, carefully quenched with a saturated ammonium chloride solution, poured into water and extracted twice with ethyl acetate. The organic layers were dried over sodium sulfate, filtered and concentrated in vacuo. The residue was purified by flash chromatography over silica gel using a gradient ethylacetate/heptane 0-40% to provide the title compound as a colorless viscous oil (374 mg, 99 % yield). MS (ESI) m/z= 492.4 [M+H]+

### Step 3: 4,6-dimethoxy-N,N-bis[(4-methoxyphenyl)methyl]-5-(1,1,2-trifluoroethoxy)pyrimidin-2-amine

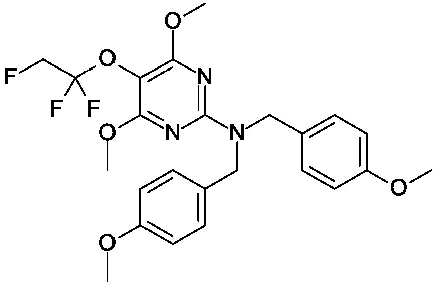

To a solution of 2-[2-[bis[(4-methoxyphenyl)methyl]amino]-4,6-dimethoxy-pyrimidin-5-yl]oxy-2,2-difluoro-ethanol (372 mg, 0.757 mmol) in tetrahydrofuran (8 mL) were added triethylamine (919.09 mg, 1.27 mL, 9.08 mmol), perfluorobutanesulfonyl fluoride (914.61 mg, 531.75 uL, 3.03 mmol) and triethylamine trihydrofluoride (498.04 mg, 503.57 uL, 3.03 mmol). The reaction mixture was stirred at 60 °C and for 56 hours, cooled to rt, poured into sat. NaHCO3 extracted with ethyl acetate. The organic layer was dried over sodium sulfate, filtered and concentrated in vacuo. The residue was purified by flash chromatography over silica gel using a gradient ethylacetate/heptane 0-20% to provide the title compound as an orange gum (110 mg, 28 % yield). MS (ESI) m/z= 494.4 [M+H]+

### Step 4: 4,6-dimethoxy-5-(1,1,2-trifluoroethoxy)pyrimidin-2-amine

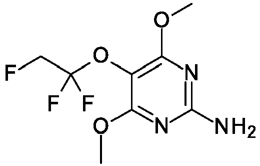

To a stirred solution of 4,6-dimethoxy-N,N-bis[(4-methoxyphenyl)methyl]-5-(1,1,2-trifluoroethoxy)pyrimidin-2-amine (109 mg, 0.210 mmol) in dichloromethane (400 uL) was added trifluoroacetic acid (1.44 g, 963.47 uL, 12.59 mmol) dropwise at room temperature. The reaction mixture was stirred at room temperature for 15 hours, at 60 °C for 4 hours and concentrated in vacuo. The residue was poured into sat. NaHCO3 and extracted twice with ethyl acetate. The organic layers were dried over sodium sulfate, filtered and concentrated in vacuo. The residue was purified by flash chromatography over silica gel using a gradient ethylacetate/heptane 0-50% to provide the title compound as a yellow solid (53 mg, 100 % yield). MS (ESI) m/z= 254.1 [M+H]+

### Intermediate B59: 5-allyl-4,6-dimethoxy-pyrimidin-2-amine

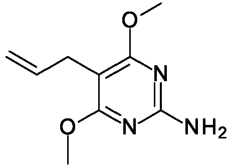

To a solution of 5-bromo-4,6-dimethoxy-pyrimidin-2-amine (5000 mg, 21.4 mmol, intermediate B54, step 1), allylboronic acid pinacol ester (4307.8 mg, 25.64 mmol) and potassium carbonate (8857.47 mg, 64.09 mmol) in 1,4-dioxane (125 mL) and water (25 mL) was added 1,1'-bis(diphenylphosphino)ferrocene-palladium(II)dichloride dichloromethane complex (1743.22 mg, 2.14 mmol) under nirogen. The mixture was stirred for 12 hours at 110 °C and extracted three times with ethyl acetate. The organic layers were washed with brine, dried over sodium sulfate, filtered and concentrated in vacuo. The residue was purified by preparative HPLC and freeze-dried to provide the title compound as an off-white solid (835 mg, 14 % yield). MS (ESI) m/z= 195.8 [M+H]+

### Intermediate B60: 4,6-dimethoxy-5-prop-1-ynyl-pyrimidin-2-amine

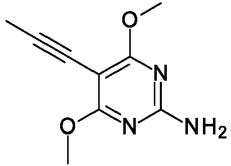

### Step 1: 5-iodo-4,6-dimethoxy-pyrimidin-2-amine

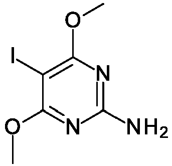

To a solution of 2-amino-4,6-dimethoxypyrimidine (1.0 g, 6.45 mmol, CAS: 10272-07-8) in acetonitrile (20 mL) was N-iodosuccinimide (1.6 g, 7.09 mmol). The mixture was stirred at room temperature, diluted with 200 mL of water and extracted with ethyl acetate. The organic layer was dried over sodium sulfate, filtered and concentrated in vacuo to provide the title compound (1.4 g, 77 % yield).

### Step 2: 4,6-dimethoxy-5-prop-1-ynyl-pyrimidin-2-amine

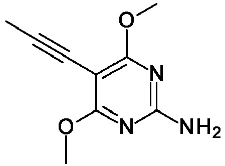

To a degassed mixture of 5-iodo-4,6-dimethoxy-pyrimidin-2-amine (0.5 g, 1.78 mmol), triethylamine (0.74 mL, 5.34 mmol), 1-(trimethylsilyl)-1-propyne (0.32 mL, 2.13 mmol), copper(I) iodide (67.76 mg, 0.360 mmol) and tetrakis(triphenylphosphine)palladium(0) (205.58 mg, 0.180 mmol) in N,N-dimethylformamide (25 mL) under argon was added potassium fluoride (124.03 mg, 2.13 mmol) in one portion. The mixture was heated at 80 °C overnight under argon, poured into water and extracted with ethyl acetate. The organic layers were washed twice with water, once with brine, dried over sodium sulfate, filtered and concentrated in vacuo. The residue was purified by flash chromatography over silica gel using a gradient chloroform/acetonitrile 0-10% to provide the title compound as a light brown solid (120 mg, 35 % yield). MS (ESI) m/z= 194.2 [M+H]+

### Intermediate B61: 4,6-dimethoxy-5-propyl-pyrimidin-2-amine

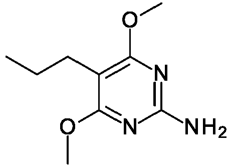

To a solution of 5-allyl-4,6-dimethoxy-pyrimidin-2-amine (100.0 mg, 0.510 mmol, intermediate B59) in methanol (2 mL) was added palladium on activated charcoal (10 mg, 0.094 mmol). The reaction mixture was purged with hydrogen and stirred under 1 atm. of hydrogen at room temperature for 4 hours. The reaction mixture was filtered through a pad of celite and concentrated in vacuo to provide the title compound as a white solid (100 mg, 99 % yield). MS (ESI) m/z= 197.8 [M+H]+

### Intermediate B62: 5-(2-fluoroethyl)-4,6-dimethoxy-pyrimidin-2-amine

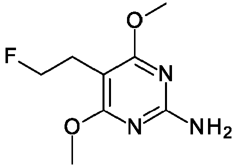

### Step 1: tert-butyl N-(5-bromo-4,6-dimethoxy-pyrimidin-2-yl)-N-tert-butoxycarbonyl-carbamate

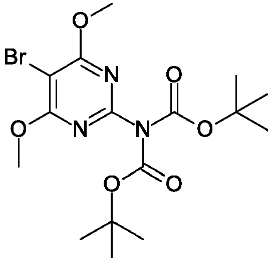

5-bromo-4,6-dimethoxy-pyrimidin-2-amine (8.9 g, 38.03 mmol, intermediate B54, step 1) was suspended in dichloromethane (200 mL) and N,N-diisopropylethylamine (5.9 g, 7.97 mL, 45.63 mmol) was added. The mixture was cooled to 5 °C and a solution of di-tert-butyl dicarbonate (9.13 g, 9.71 mL, 41.83 mmol) in dichloromethane (100 mL) was added dropwise. After the addition, the cooling bath was removed and the mixture was stirred at room temperature for 2.5 hours. 4-dimethylaminopyridine (464.56 mg, 3.8 mmol) was added and the reaction mixture was further stirred at room temperature for 2 hours. Di-tert-butyl dicarbonate (9.13 g, 9.71 mL, 41.83 mmol) was added. The reaction mixture was stirred at room temperature overnight, poured into water and extracted twice with dichloromethane. The organic layers were washed with brine, dried over sodium sulfate, filtered and concentrated in vacuo. The residue was purified by flash chromatography over silica gel using a gradient ethylacetate/heptane 0-50% to provide the title compound as a colorless oil (14.88 g, 86 % yield). MS (ESI) m/z= 336.1 [M+H-BOC]+

### Step 2: tert-butyl N-tert-butoxycarbonyl-N-(4,6-dimethoxy-5-vinyl-pyrimidin-2-yl)carbamate

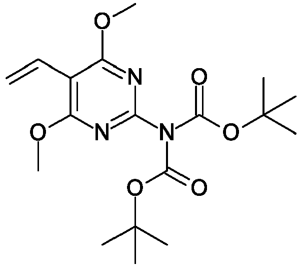

tert-butyl N-(5-bromo-4,6-dimethoxy-pyrimidin-2-yl)-N-tert-butoxycarbonyl-carbamate (1.02 g, 2.35 mmol), potassium vinyltrifluoroborate (440.45 mg, 3.29 mmol), isopropanol (4 mL), 1,1'-bis(diphenylphosphino)ferrocene-palladium(II)dichloride dichloromethane complex (95.9 mg, 0.117 mmol) and triethylamine (475.33 mg, 654.73 uL, 4.7 mmol) were added under argon. The reaction mixture was stirred for 22 hours at 100 °C, poured into water and extracted twice with ethyl acetate. The organic layers were washed with brine, dried over sodium sulfate, filtered and concentrated in vacuo. The residue was purified by flash chromatography over silica gel using a gradient ethylacetate/heptane 0-50% to provide the title compound as a colorless oil (600 mg, 47 % yield). MS (ESI) m/z= 382.3 [M+H]+

### Step 3: tert-butylN-[5-(2-hydroxyethyl)-4,6-dimethoxy-pyrimidin-2-yl]carbamate

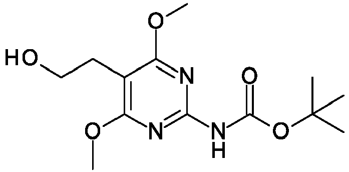

To a solution of tert-butyl N-tert-butoxycarbonyl-N-(4,6-dimethoxy-5-vinyl-pyrimidin-2-yl)carbamate (598 mg, 1.57 mmol) in tetrahydrofuran (8 mL) was added a 0.5 M 9-borabicyclo[3.3.1]nonane solution solution in THF (5.02 mL, 2.51 mmol) dropwise at 0 °C. The mixture was warmed to room temperature and stirred overnight. After cooling to 0 °C, water (508.54 mg, 508.54 uL, 28.22 mmol), a 4 M NaOH solution (3.92 mL, 15.68 mmol) and hydrogen peroxide (1.83 g, 1.65 mL, 18.81 mmol) were added. The reaction mixture was stirred at 50 °C for 2.5 hours, poured into water/sodium thiosulfate and extracted twice with ethyl acetate. The organic layers were washed with brine, dried over sodium sulfate, filtered and concentrated in vacuo. The residue was purified by flash chromatography over silica gel using a gradient ethylacetate/heptane 0-50% to provide the title compound as a colorless oil (213 mg, 43 % yield). MS (ESI) m/z= 300.2 [M+H]+

### Step 4: tert-butyl N-[5-(2-fluoroethyl)-4,6-dimethoxy-pyrimidin-2-yl]carbamate

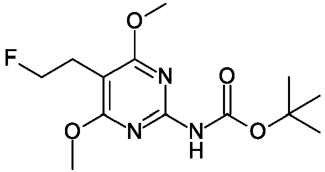

A solution of tert-butyl N-[5-(2-hydroxyethyl)-4,6-dimethoxy-pyrimidin-2-yl]carbamate (213 mg, 0.712 mmol) in dichloromethane (6 mL) was cooled to 0 °C. Diethylaminosulfur trifluoride (149.12 mg, 122.23 uL, 0.925 mmol) was added. The reaction mixture was further stirred at 0 °C, carefully poured into sat. NaHCO3, stirred for 20 min and extracted twice with dichloromethane. The organic layers were dried over sodium sulfate, filtered and concentrated in vacuo. The residue was purified by flash chromatography over silica gel using a gradient ethylacetate/heptane 0-30% to provide the title compound as a white semisolid (78 mg, 35 % yield). MS (ESI) m/z= 302.2 [M+H]+

### Step 5: 5-(2-fluoroethyl)-4,6-dimethoxy-pyrimidin-2-amine

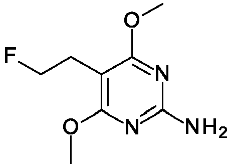

A solution of tert-butyl N-[5-(2-fluoroethyl)-4,6-dimethoxy-pyrimidin-2-yl]carbamate (76 mg, 0.252 mmol) in dichloromethane (2 mL) was cooled to 0 °C. Trifluoroacetic acid (230.08 mg, 155.46 uL, 2.02 mmol) was added. The reaction mixture was stirred 1 hour at 0 °C, 6 hours at room temperature, poured into sat. NaHCO3 and extracted twice with dichloromethane. The organic layers were dried over sodium sulfate, filtered and concentrated in vacuo to provide the title compound as a white solid (45 mg, 84 % yield). MS (ESI) m/z= 202.1 [M+H]+

### Intermediate B63: 5-(2,2-difluoroethyl)-4,6-dimethoxy-pyrimidin-2-amine

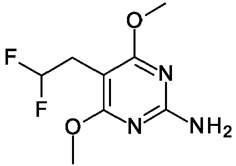

### Step 1: diethyl 2-(2,2-difluoroethyl)propanedioate

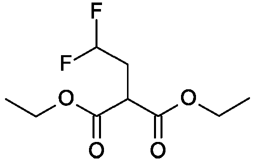

Diethyl malonate (75.83 mL, 499.47 mmol) was combined with tetrahydrofuran (450 mL). Sodium ethoxide (prepared from ethanol (150 mL) and sodium (11.48 g, 499.47 mmol)) was added at room temperature and the reaction mixture was stirred 15 min at room temperature. A solution of 2,2-difluoroethyl trifluoromethanesulfonate (75.83 mL, 499.47 mmol) in tetrahydrofuran (10 mL) was added slowly. The reaction mixture was stirred for 18 hours at 20 °C, then cooled to 0 °C, quenched with a saturated ammonium chloride solution and extracted twice with ethyl acetate. The organic layers were washed with brine, dried over sodium sulfate, filtered and concentrated in vacuo to provide the title compound (100.5 g, 90 % yield). MS (ESI) m/z= 225.0 [M+H]+

### Step 2: 2-amino-5-(2,2-difluoroethyl)pyrimidine-4,6-diol

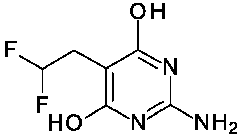

To a stirred solution of diethyl 2-(2,2-difluoroethyl)propanedioate (46.75 g, 208.52 mmol) in ethanol (5 mL) was added guanidine hydrochloride (19.92 g, 208.52 mmol), followed by sodium ethoxide (prepared from ethanol and sodium (14.38 g, 625.56 mmol)). The resulting orange suspension was heated to 80 °C and stirred for 4 hours. The reaction mixture was concentrated by half, 50 mL of water was added, followed by acetic acid (42.57 g, 708.97 mmol). The mixture was heated to 80 °C and stirred for 10 min, then cooled to room temperature. The solid product was filtered off, washed successively with water, ethanol and methyl tert-butyl ether to provide the title compound (22.3 g, 50 % yield). MS (ESI) m/z= 192.0 [M+H]+

### Step 3: 4,6-dichloro-5-(2,2-difluoroethyl)pyrimidin-2-amine

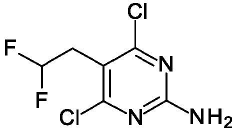

2-amino-5-(2,2-difluoroethyl)pyrimidine-4,6-diol (13.2 g, 69.06 mmol) was suspended in phosphorus oxychloride (80.46 mL, 863.24 mmol). The reaction mixture was heated to 100 °C and stirred for 18 hours and concentrated in vacuo. The residue was diluted with ethyl acetate and carefully poured into ice/sat. NaHCO3. The resulting biphasic mixture was stirred at room temperature for 5 min and extracted twice with ethyl acetate. The organic layers were dried over sodium sulfate, filtered and concentrated in vacuo. The residue was purified by flash chromatography over silica gel to provide the title compound (7.35 g, 47 % yield). MS (ESI) m/z= 227.8 [M+H]+

### Step 4: 5-(2,2-difluoroethyl)-4,6-dimethoxy-pyrimidin-2-amine

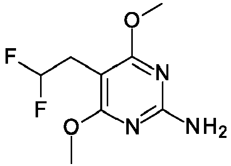

In a sealed tube, a mixture of 4,6-dichloro-5-(2,2-difluoroethyl)pyrimidin-2-amine (7.6 g, 33.33 mmol) and sodium methylate (prepared from sodium (7662.33 mg, 333.29 mmol) in methanol (50 mL)) was heated to 75 °C and stirred for 18 hours. The reaction mixture was quenched with water and extracted twice with ethyl acetate. The organic layers were dried over sodium sulfate, filtered and concentrated in vacuo to provide the title compound as a light yellow solid (6.604 g, 86 % yield). MS (ESI) m/z= 220.0 [M+H]+

### Intermediate B64: 5-(3-fluoropropyl)-4,6-dimethoxy-pyrimidin-2-amine

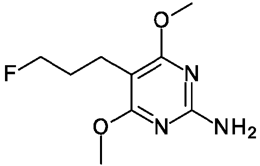

### Step 1: diethyl 2-(3-fluoropropyl)propanedioate

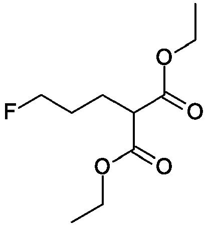

To a suspension of potassium carbonate (7719.77 mg, 55.86 mmol) in acetone (50 mL) at 25 °C was added diethyl malonate (2.04 mL, 13.41 mmol), followed by 1-fluoro-3-iodo-propane (2100.0 mg, 11.17 mmol). The mixture was then stirred at 25 °C for 24 hours and extracted three times with ethyl acetate. The organic layers were washed with brine, dried over sodium sulfate, filtered and concentrated in vacuo. The residue was purified by flash chromatography over silica gel using a gradient ethylacetate/petroleum ether 0-30% to provide the title compound as a yellow oil (2200 mg, 89 % yield).

### Step 2: 2-amino-5-(3-fluoropropyl)pyrimidine-4,6-diol

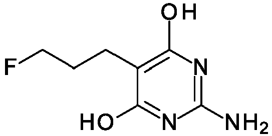

To a stirred solution of guanidine hydrochloride (858.83 mg, 8.99 mmol) in methanol (40 mL) was added a 5 M sodium methoxide solution in methanol (3.76 mL, 18.8 mmol), followed by diethyl 2-(3-fluoropropyl)propanedioate (1800.0 mg, 8.17 mmol) at 25 °C under nitrogen atmosphere. Then the reaction mixture was heated to 40 °C and stirred for 2 hours. Methanol was evaporated and water (80 mL) was added to the residue. After stirring, the product was almost dissolved. The obtained mixture was subsequently neutralized by dropwise addition of acetic acid, resulting in immediate and quantitative precipitation of the product in the form of a fine solid. This mixture was subsequently heated under reflux for 10 min and then cooled to 25 °C. The solid product was filtered off, washed with water and ethanol. The product was dried under high vacuum at 60 °C to provide the title compound as an off-white solid (700 mg, 46 % yield). MS (ESI) m/z= 187.7 [M+H]+

### Step 3: 4,6-dichloro-5-(3-fluoropropyl)pyrimidin-2-amine

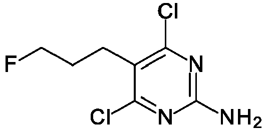

To a solution of 2-amino-5-(3-fluoropropyl)pyrimidine-4,6-diol (330.0 mg, 1.76 mmol) in phosphoryl chloride (5.0 mL) was added triethylamine (0.75 mL, 5.38 mmol). The reaction mixture was stirred at 60 °C for 2 hours, cooled to 25 °C and quenched by addition of water (15 mL) and the pH was adjusted to 7-8 with 10 % NaOH (aq., 35mL). Then the mixture was extracted three times with ethyl acetate. The organic layers were washed with brine, dried over sodium sulfate, filtered and concentrated in vacuo to provide the title compound as an off-white solid (400 mg, 81 % yield). MS (ESI) m/z= 223.7 [M+H]+

### Step 4: 5-(3-fluoropropyl)-4,6-dimethoxy-pyrimidin-2-amine

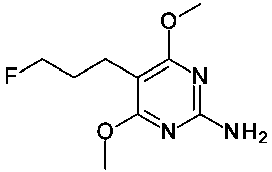

To a stirred suspension of 4,6-dichloro-5-(3-fluoropropyl)pyrimidin-2-amine (200.0 mg, 0.890 mmol) in tetrahydrofuran (5 mL) was added a 5 M sodium methoxide solution in methanol (0.54 mL, 2.68 mmol) at 20 °C under nitrogen atmosphere. The reaction mixture was stirred at 30 °C for 3 hours, at 60 °C for 6 hours and at 80 °C for 1 hour, then cooled to 25 °C, quenched by addition of a saturated ammonium chloride solution (10 mL) and extracted with three times with ethyl acetate. The organic layers were washed with brine, dried over sodium sulfate, filtered and concentrated in vacuo to provide the title compound as an off-white solid (180 mg, 94 % yield). MS (ESI) m/z= 216.4 [M+H]+

### Intermediate B65: 5-(3,3-difluoropropyl)-4,6-dimethoxy-pyrimidin-2-amine

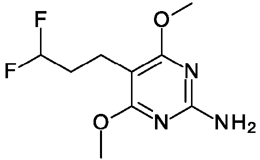

### Step 1: diethyl 2-(3,3-difluoropropyl)propanedioate

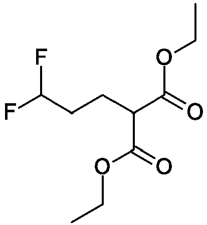

Sodium hydride (538.92 mg, 13.47 mmol) was supended in tetrahydrofuran, extra dry (30 mL) and the mixture was cooled to 0 °C. Diethyl malonate (1.92 g, 1.83 mL, 11.99 mmol) was added slowly. The mixture was stirred 10 min at 0 °C then a solution of 3,3-difluoropropyl 4-methylbenzenesulfonate (3 g, 11.99 mmol, intermediate B32, step 1) in tetrahydrofuran (5 mL) was added slowly. The reaction mixture was stirred 5 min at 0 °C, 20 hours at 60 °C, then cooled to 0 °C and quenched with a saturated ammonium chloride solution, poured into water and extracted twice with ethyl acetate. The organic layers were washed with brine, dried over sodium sulfate, filtered and concentrated in vacuo. The residue was purified by flash chromatography over silica gel using a gradient ethylacetate/heptane 0-30% to provide the title compound as a colorless liquid (2.16 g, 50 % yield). MS (ESI) m/z= 239.2 [M+H]+

### Step 2: 2-amino-5-(3,3-difluoropropyl)pyrimidine-4,6-diol

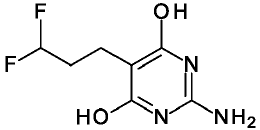

Diethyl 2-(3,3-difluoropropyl)propanedioate (1.98 g, 8.31 mmol) was combined with ethanol (16 mL) to give a colorless solution. Guanidine hydrochloride (793.98 mg, 8.31 mmol) was added followed by sodium ethoxide 21% in ethanol (8.08 g, 9.31 mL, 24.93 mmol) and the orange suspension was stirred at 75 °C for 4 hours. The reaction mixture was concentrated by half, 5 mL of water were added, followed by acetic acid (1.69 g, 1.61 mL, 28.1 mmol). The mixture was heated at 80 °C for 10 min then cooled to room temperature. The solid was filtered off and successively washed with water, ethanol and heptane to afford 460 mg of the product as a light brown solid. The filtrate was washed with ethyl acetate and concentrated by half. The precipitated was filtered off, successively washed with water, ethanol and heptane, combined with the previously obtained solid and dried on high vacuo to provide the title compound as a light brown solid (568 mg, 28 % yield). MS (ESI) m/z= 206.1 [M+H]+

### Step 3: 4,6-dichloro-5-(3,3-difluoropropyl)pyrimidin-2-amine

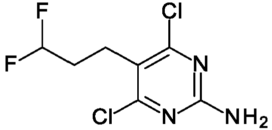

2-amino-5-(3,3-difluoropropyl)pyrimidine-4,6-diol (746 mg, 3.64 mmol) was suspended in phosphorus oxychloride (9.9 g, 6.02 mL, 64.54 mmol) and stirred at 100 °C for 4 hours. The excess of phosphorus oxychloride was removed under reduced pressure. The residue was diluted in dichloromethane, poured into an ice cooled NaHCO3 solution and stirred for 10 min at room temperature. After extraction, the organic layer was washed with brine, dried over sodium sulfate, filtered and concentrated in vacuo. The residue was purified by flash chromatography over silica gel using a gradient ethylacetate/heptane 0-50% to provide the title compound as an off-white solid (432 mg, 44 % yield). MS (ESI) m/z= 242.1 [M+H]+

### Step 4: 5-(3,3-difluoropropyl)-4,6-dimethoxy-pyrimidin-2-amine

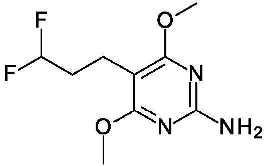

4,6-dichloro-5-(3,3-difluoropropyl)pyrimidin-2-amine (432 mg, 1.78 mmol) was dissolved in tetrahydrofuran, extra dry (15 mL). Sodium methoxide 25% in methanol (1.93 g, 2.04 mL, 8.92 mmol) was added. The reaction mixture was stirred at 60 °C for 5 hours, cooled to 0 °C, quenched with a saturated ammonium chloride solution and extracted twice with ethyl acetate. The organic layers were washed with brine, dried over sodium sulfate, filtered and concentrated in vacuo. The residue was purified by flash chromatography over silica gel using a gradient ethylacetate/heptane 0-30% to provide the title compound as an off-white solid (328 mg, 76 % yield). MS (ESI) m/z= 234.2 [M+H]+

### Intermediate B66: 5-(2,2-difluoropropyl)-4,6-dimethoxy-pyrimidin-2-amine

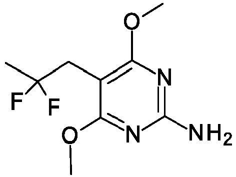

### Step 1: dimethyl 2-(2,2-difluoropropyl)propanedioate

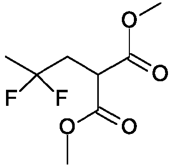

A mixture of dimethyl acetonylmalonate (4.0 g, 21.26 mmol, CAS: 24889-15-4) and diethylaminosulfur trifluoride (13.71 g, 85.02 mmol) was stirred at 40 °C for 72 hours, then cooled to room temperature, slowly poured into ice/water and extracted with ethyl acetate. The organic layer was successively washed with water and brine, dried over sodium sulfate, filtered and concentrated in vacuo to provide the title compound as a dark brown oil, which was directly used in the next step without further purification.

### Step 2: 2-amino-5-(2,2-difluoropropyl)pyrimidine-4,6-diol

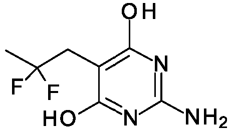

A mixture of dimethyl 2-(2,2-difluoropropyl)propanedioate (1.1 g, 5.23 mmol), guanidine hydrochloride (0.5 g, 5.23 mmol) and potassium tert-butoxide (1.17 g, 10.5 mmol) in dry 1,4-dioxane (15 mL) was stirred at 115 °C for 30 hours, and then concentrated in vacuo. The residue was triturated with tert-butyl methyl ether (20 mL). The precipitate was filtered, washed twice with acetonitrile (10 mL), poured into water (5 mL) and acidified to pH 4-5 with acetic acid. The resulting precipitate was filtered and dried in vacuo to provide the title compound as a brown solid (180 mg, 15 % yield). 1H NMR (400 MHz, DMSO-d6) δ = 6.67 (br s, 2H), 2.82 - 2.65 (m, 2H), 1.48 (br t, J = 18.8 Hz, 3H)

### Step 3: 4,6-dichloro-5-(2,2-difluoropropyl)pyrimidin-2-amine

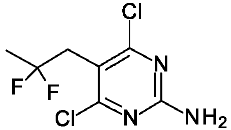

To a stirred solution of N,N-dimethylformamide (8.3 mL, 107.23 mmol) in dry chloroform (20 mL) was slowly added phosphorus oxychloride (11.99 mL, 128.68 mmol). The reaction mixture was stirred at room temperature for 1 hour. Then 2-amino-5-(2,2-difluoropropyl)pyrimidine-4,6-diol (2.2 g, 10.72 mmol) was added. The reaction mixture was stirred at 60 °C for 15 hours, and then concentrated in vacuo. The residue was diluted with ethanol (20 mL) and a 12 M aqueous HCl solution (5 mL), stirred at 50 °C for 2 hours and concentrated in vacuo. The residue was diluted with aqueous NaHCO3 (pH 8-9) and extracted three times with ethyl acetate. The organic layers were washed with brine, dried over sodium sulfate, filtered and concentrated in vacuo to provide the title compound as a brown solid (800 mg, 28 % yield). MS (ESI) m/z= 244.0 [M+H]+

### Step 4: 5-(2,2-difluoropropyl)-4,6-dimethoxy-pyrimidin-2-amine

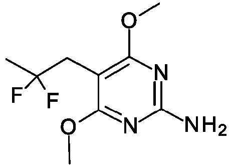

Sodium (156.72 mg, 6.82 mmol) was dissolved in dry methanol (30 mL) and 4,6-dichloro-5-(2,2-difluoropropyl)pyrimidin-2-amine (750.0 mg, 3.1 mmol) was added. The mixture was stirred at reflux for 15 hours, then diluted with toluene (40 mL) and stirred at 100 °C overnight. The solvents were evaporated under reduced pressure and the residue was diluted with water and extracted three times with dichloromethane. The organic layers were dried over sodium sulfate, filtered and concentrated in vacuo to provide the title compound as a light brown semisolid (250 mg, 35 % yield). MS (ESI) m/z= 234.1 [M+H]+

### Intermediate B67: 4,6-dimethoxy-5-(3,3,3-trifluoropropyl)pyrimidin-2-amine

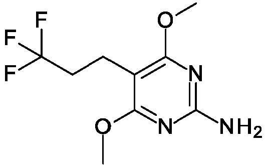

### Step 1: diethyl 2-(3,3,3-trifluoropropyl)propanedioate

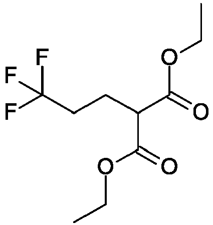

Diethyl malonate (650.75 mg, 619.77 uL, 4.06 mmol) was combined with tetrahydrofuran (7 mL). Sodium ethoxide 21% in ethanol (1.32 g, 1.52 mL, 4.06 mmol) was added at room temperature and the reaction mixture was stirred 15 min. A solution of trifluoromethanesulfonic acid 3,3,3-trifluoropropyl ester (1 g, 4.06 mmol) in tetrahydrofuran (5 mL) was added slowly within 25 min. The reaction mixture was stirred 1 hour at room temperature, cooled to 0 °C, quenched with a saturated ammonium chloride solution and extracted twice with ethyl acetate. The organic layers were washed with brine, dried over sodium sulfate, filtered and concentrated in vacuo. The residue was purified by flash chromatography over silica gel using a gradient ethylacetate/heptane 0-30% to provide the title compound as a colorless liquid (645 mg, 59 % yield). MS (ESI) m/z= 257.2 [M+H]+

### Step 2: 2-amino-5-(3,3,3-trifluoropropyl)pyrimidine-4,6-diol

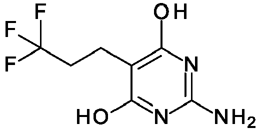

Diethyl 2-(3,3,3-trifluoropropyl)propanedioate (1.05 g, 4.1 mmol) was combined with ethanol (10 mL) to give a colorless solution. Guanidine hydrochloride (391.49 mg, 4.1 mmol) and sodium ethoxide 21% in ethanol (3.98 g, 4.59 mL, 12.29 mmol) were added. The orange suspension was stirred at 75 °C for 4 hours and concentrated by half. 5 mL of water was added followed by acetic acid (839.2 mg, 800 uL, 13.98 mmol). The mixture was heated at 80 °C for 10 min and cooled to room temperature. The precipitate was filtered off, washed with water, ethanol and heptane then dried on high vacuo to provide the title compound as an off-white solid (386 mg,40 % yield). MS (ESI) m/z= 224.0 [M+H]+

### Step 3: 4,6-dichloro-5-(3,3,3-trifluoropropyl)pyrimidin-2-amine

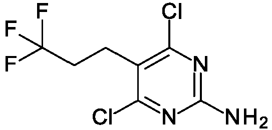

2-amino-5-(3,3,3-trifluoropropyl)pyrimidine-4,6-diol (386 mg, 1.73 mmol) was added to phosphorus oxychloride (3.29 g, 2 mL, 21.46 mmol) and the mixture was stirred at 100 °C for 7 hours. The excess of phosphorus oxychloride was removed under reduced pressure. The residue was diluted with dichloromethane, poured into an ice cold NaHCO3 solution and stirred for 10 min at room temperature. After extraction, the combined organic layers were washed with brine, dried over sodium sulfate, filtered and concentrated in vacuo. The residue was purified by flash chromatography over silica gel using a gradient ethylacetate/heptane 0-100% to provide the title compound as an off-white solid (250 mg, 50 % yield). MS (ESI) m/z= 260.1 [M+H]+

### Step 4: 4,6-dimethoxy-5-(3,3,3-trifluoropropyl)pyrimidin-2-amine

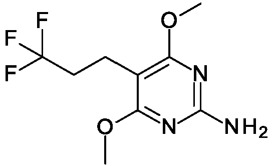

4,6-dichloro-5-(3,3,3-trifluoropropyl)pyrimidin-2-amine (250 mg, 0.961 mmol) was combined with tetrahydrofuran (5 mL) to give a light yellow solution. Sodium methoxide 25% in methanol (1.04 g, 1.1 mL, 4.81 mmol) was added. The reaction mixture was stirred at 60 °C for 2 hours, cooled to 0 °C, quenched with a saturated ammonium chloride solution and extracted with ethyl acetate. The organic layers were dried over sodium sulfate, filtered and concentrated in vacuo. The residue was purified by flash chromatography over silica gel using a gradient ethylacetate/heptane 0-50% to provide the title compound as a white solid (155 mg, 61 % yield). MS (ESI) m/z= 252.2 [M+H]+

### Intermediate B68: 4,6-dimethoxy-5-(methoxymethyl)pyrimidin-2-amine

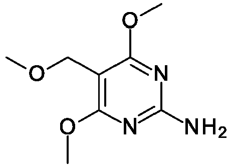

### Step 1: (2-amino-4,6-dimethoxy-pyrimidin-5-yl)methanol

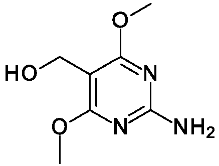

A suspension of (2-amino-4,6-dichloro-pyrimidin-5-yl)methanol (613 mg, 3.16 mmol, CAS: 850554-81-3) in methanol (1 mL) was cooled to 0 °C. A 0.5 M sodium methoxide solution in methanol (18.96 mL, 9.48 mmol) was added at this temperature. The ice-bath was removed and the reaction mixture was stirred at room temperature overnight. Sodium methoxide 0.5M in methanol (12.64 mL, 6.32 mmol) was added and the stirring was continued at room temperature for 4 hours. Sodium methoxide 25 % in methanol (2.73 g, 2.89 mL, 12.64 mmol) was added. The reaction mixture was stirred at 60 °C for 6 hours, poured into a saturated ammonium chloride solution and extracted four times with dichloromethane/methanol 9:1. The organic layers were dried over sodium sulfate, filtered and concentrated in vacuo. The residue was purified by flash chromatography over silica gel using a gradient ethylacetate/heptane 30-100% to provide the title compound as a white solid (295 mg, 47 % yield). MS (ESI) m/z= 186.1 [M+H]+

### Step 2: 4,6-dimethoxy-5-(methoxymethyl)pyrimidin-2-amine

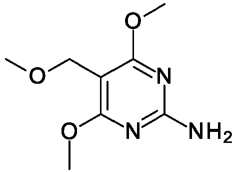

To a solution of (2-amino-4,6-dimethoxy-pyrimidin-5-yl)methanol (83.1 mg, 0.422 mmol) and iodomethane (131.72 mg, 58.03 uL, 0.928 mmol) in tetrahydrofuran (2100 uL) was added sodium hydride (33.75 mg, 0.844 mmol) in portions. The mixture was allowed to stir at 23 °C for 48 hours. Iodomethane (131.72 mg, 58.03 uL, 0.928 mmol) was added. The reaction mixture was stirred at 23 °C for 7.5 hours, quenched with brine, poured into water and extracted three times with ethyl acetate. The organic layers were dried over sodium sulfate, filtered and concentrated in vacuo. The residue was purified by flash chromatography over silica gel using a gradient ethylacetate/heptane 0-100% to provide the title compound as a light yellow solid (40.3 mg, 46 % yield). MS (ESI) m/z= 200.1 [M+H]+

### Intermediate B69: 4,6-dimethoxy-5-(2-methoxyethyl)pyrimidin-2-amine

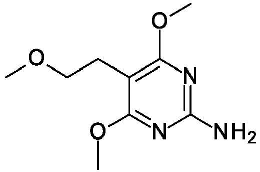

### Step 1: 2-amino-5-(2-methoxyethyl)pyrimidine-4,6-diol

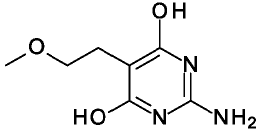

1,3-diethyl 2-(2-methoxyethyl)propanedioate (6 g, 27.49 mmol, CAS: 6335-02-0) was combined with ethanol (60 mL) to give a colorless solution. Guanidine hydrochloride (2.63 g, 27.49 mmol) and sodium ethoxide 21 % in ethanol (26.73 g, 30.79 mL, 82.47 mmol) were added and the orange suspension was stirred at 75 °C for 5 hours and concentrated by half. 25 mL of water was added, followed by acetic acid (5.61 g, 5.35 mL, 93.47 mmol) and the mixture was concentrated in vacuo, diluted with toluene and concentrated in vacuo until precipitation occured. The solid was filtered off and washed with toluene to provide the title compound as a brown solid (2.38 g, 37 % yield). MS (ESI) m/z= 186.1 [M+H]+

### Step 2: 4,6-dichloro-5-(2-methoxyethyl)pyrimidin-2-amine

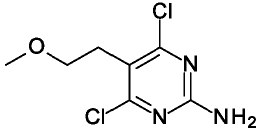

2-amino-5-(2-methoxyethyl)pyrimidine-4,6-diol (1.6 g, 8.64 mmol) was carefully added to stirred phosphorus oxychloride (14.81 g, 9 mL, 96.56 mmol). The mixture was stirred for 4 hours at 100 °C. The excess of phosphorus oxychloride was removed under reduced pressure. The residue was diluted with dichloromethane, poured into an ice cold NaHCO3 solution and stirred for 10 min at room temperature. After extraction, the combined organic layers were washed with brine, dried over sodium sulfate, filtered and concentrated in vacuo. The residue was purified by flash chromatography over silica gel using a gradient ethylacetate/heptane 0-35% to provide the title compound as a light yellow semisolid (267 mg, 13 % yield). MS (ESI) m/z= 222.1 [M+H]+

### Step 3: 4,6-dimethoxy-5-(2-methoxyethyl)pyrimidin-2-amine

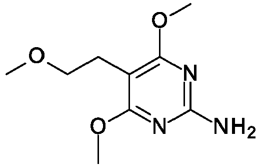

4,6-dichloro-5-(2-methoxyethyl)pyrimidin-2-amine (350 mg, 1.58 mmol) was combined with tetrahydrofuran (7 mL) to give a light yellow solution. Sodium methoxide 25 % in methanol (1.7 g, 1.8 mL, 7.88 mmol) was added. The reaction mixture was stirred at 60 °C for 2 hours, cooled to 0 °C, quenched with a saturated ammonium chloride solution and extracted with ethyl acetate. The organic layers were dried over sodium sulfate, filtered and concentrated in vacuo. The residue was purified by flash chromatography over silica gel using a gradient ethylacetate/heptane 0-50% to provide the title compound as a white solid (223 mg, 50 % yield). MS (ESI) m/z= 214.2 [M+H]+

### Intermediate B70: 2-(2-amino-4,6-dimethoxy-pyrimidin-5-yl)acetonitrile

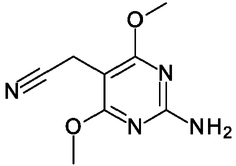

### Step 1: tert-butyl N-tert-butoxycarbonyl-N-(5-isoxazol-4-yl-4,6-dimethoxy-pyrimidin-2-yl)carbamate

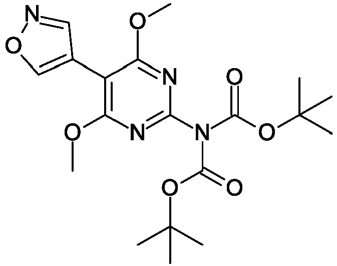

In a round-bottomed flask were added one after the other, tert-butyl N-(5-bromo-4,6-dimethoxy-pyrimidin-2-yl)-N-tert-butoxycarbonyl-carbamate (1 g, 2.3 mmol, intermediate B62, step 1), 4-(4,4,5,5-tetramethyl-1,3,2-dioxaborolan-2-yl)isoxazole (583.78 mg, 2.99 mmol), 1,4-dioxane (36 mL), water (6 mL), 1,1'-bis(diphenylphosphino)ferrocene-palladium(II)dichloride dichloromethane complex (190.37 mg, 0.230 mmol) and cesium carbonate (2.25 g, 6.91 mmol) (vacuum/argon after each addition). The reaction mixture was stirred at 100 °C for 2 hours. 1,1'-bis(diphenylphosphino)ferrocene-palladium(II)dichloride dichloromethane complex (190.37 mg, 0.230 mmol) was added again. The reaction mixture was stirred at 100 °C overnight, poured into water and extracted four times with ethyl acetate. The organic layers were filtered through a Agilent Chem Elut column and concentrated in vacuo. The residue was purified by flash chromatography over silica gel using a gradient ethylacetate/heptane 0-30% to provide the title compound as an off-white solid (130 mg, 13 % yield). MS (ESI) m/z= 323.1 [M+H-BOC]+

### Step 2: tert-butyl N-[5-(cyanomethyl)-4,6-dimethoxy-pyrimidin-2-yl]carbamate

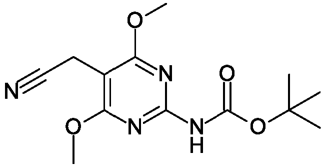

A mixture of tert-butyl N-tert-butoxycarbonyl-N-(5-isoxazol-4-yl-4,6-dimethoxy-pyrimidin-2-yl)carbamate (130 mg, 0.292 mmol) and a 1 M potassium fluoride solution in water (877.07 uL, 0.877 mmol) in N,N-dimethylformamide (2 mL) was stirred at 90 °C overnight, poured into water and extracted twice with ethyl acetate. The organic layers were washed with brine, dried over sodium sulfate, filtered and concentrated in vacuo. The residue was purified by flash chromatography over silica gel using a gradient ethylacetate/heptane 0-50% to provide the title compound as a colorless oil (69 mg, 68 % yield). MS (ESI) m/z= 295.1 [M+H]+

### Step 3: 2-(2-amino-4,6-dimethoxy-pyrimidin-5-yl)acetonitrile

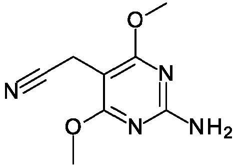

Tert-butyl N-[5-(cyanomethyl)-4,6-dimethoxy-pyrimidin-2-yl]carbamate (69 mg, 0.199 mmol) was combined with a 4 M HCl solution in dioxane (597.84 mg, 498.2 uL, 1.99 mmol). The reaction mixture was stirred at room temperature for 1 hour, poured into NaHCO3 solution and water and extracted twice with ethyl acetate. The organic layers were washed with brine, dried over sodium sulfate, filtered and concentrated in vacuo to provide the title compound as a white solid (44 mg, 97 % yield). MS (ESI) m/z= 195.1 [M+H]+

### Intermediate B71: 3-(2-amino-4,6-dimethoxy-pyrimidin-5-yl)propanenitrile

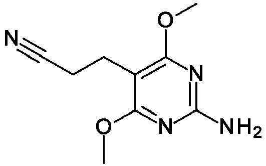

### Step 1: 3-[2-[bis[(4-methoxyphenyl)methyl]amino]-4,6-dimethoxy-pyrimidin-5-yl]propanenitrile

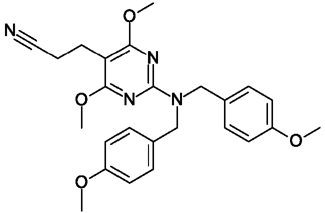

To a solution of 5-bromo-4,6-dimethoxy-N,N-bis[(4-methoxyphenyl)methyl]pyrimidin-2-amine (5000.0 mg, 10.54 mmol, intermediate B54, step 2) in methoxycyclopentane (75.0 mL) was added 3-(4,4,5,5-tetramethyl-1,3,2-dioxaborolan-2-yl)propanenitrile (2289.95 mg, 12.65 mmol), cesium carbonate (10303.15 mg, 31.62 mmol) and cataCXium^{®} A Pd G3 (767.65 mg, 1.05 mmol, CAS: 1651823-59-4) under nitrogen. The mixture was purged three times with nitrogen, stirred at 90 °C for 12 hours under nitrogen, poured into NaHCO3 solution and extracted three times with ethyl acetate. The organic layers were washed with dried over sodium sulfate, filtered and concentrated in vacuo. The residue was purified by flash chromatography over silica gel using a gradient ethylacetate/petroleum ether 0-30% to provide the title compound as a yellow solid (2.9 g, 61 % yield). MS (ESI) m/z= 449.0 [M+H]+

### Step 2: 3-(2-amino-4,6-dimethoxy-pyrimidin-5-yl)propanenitrile

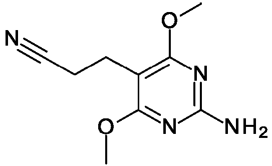

A mixture of 3-[2-[bis[(4-methoxyphenyl)methyl]amino]-4,6-dimethoxy-pyrimidin-5-yl]propanenitrile (2300.0 mg, 5.13 mmol) in trifluoroacetic acid (23 mL) was stirred at 25 °C for 48 hours, quenched with ice/sat. NaHCO3 and extracted three times with ethyl acetate. The organic layers were dried over sodium sulfate, filtered and concentrated in vacuo. The residue was purified by flash chromatography over silica gel using a gradient ethylacetate/petroleum ether 0-100% to provide the title compound as a yellow solid (1000 mg, 89 % yield). MS (ESI) m/z= 209.0 [M+H]+

### Intermediate B72: 5-cyclopropyl-4,6-dimethoxy-pyrimidin-2-amine

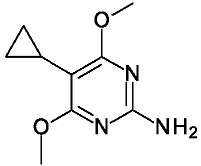

5-bromo-4,6-dimethoxy-pyrimidin-2-amine (570.0 mg, 2.44 mmol, intermediate B54, step 1), cyclopropylboronic acid (2.09 g, 24.35 mmol), potassium phosphate tribasic (2.02 mL, 24.35 mmol) were dissolved in 1,4-dioxane (10 mL) and water (10 mL). The flask was evacuated and backfilled with argon, then cataCXium^{®} A Pd G3 (177.61 mg, 0.240 mmol, CAS: 1651823-59-4) and X-Phos (116.1 mg, 0.240 mmol) were added. The mixture was evacuated and backfilled with argon, stirred at 100 °C for 15 hours, diluted with water and extracted with ethyl acetate. The organic layer was successively washed with water and brine, dried over sodium sulfate, filtered and concentrated in vacuo. The residue was purified by preparative HPLC to provide the title compound as an off-white solid (208 mg, 44 % yield). MS (ESI) m/z= 196.2 [M+H]+

### Intermediate B73: 5-bromo-4-methoxy-6-(2-methoxyethoxy)pyrimidin-2-amine

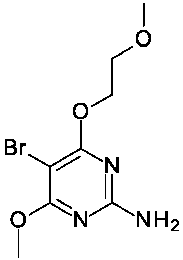

To a suspension of sodium hydride (25.16 mg, 0.629 mmol) in tetrahydrofuran (2.21 mL) was added 2-methoxyethanol (38.29 mg, 39.68 uL, 0.503 mmol) and stirred for 15 min at room temperature. To this mixture (5-bromo-4-chloro-6-methoxy-pyrimidin-2-yl)amine (100 mg, 0.419 mmol, intermediate B14, step 1) was added. The reaction mixture was stirred at 70°C for 3 hours, cooled to room temperature, quenched with acetic acid (30 uL) and concentrated in vacuo. The residue was purified by flash chromatography over silica gel using a gradient ethylacetate/heptane 0-50% to provide the title compound as a colorless solid (118 mg, 96 % yield). MS (ESI) m/z= 278.2 [M+H]+

### Intermediate B74: 5-bromo-4-(cyclopropoxy)-6-methoxy-pyrimidin-2-amine

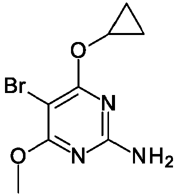

The title compound was prepared in analogy to intermediate B73 from (5-bromo-4-chloro-6-methoxy-pyrimidin-2-yl)amine (intermediate B14, step 1) using cyclopropanol as alcohol. Colorless solid.

### Intermediate B75: 4-(difluoromethoxy)-6-methoxy-5-methyl-pyrimidin-2-amine

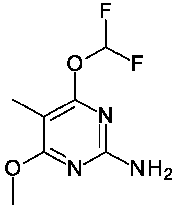

### Step 1: 4-benzyloxy-6-chloro-5-methyl-pyrimidin-2-amine

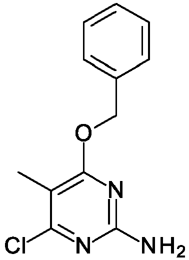

To a solution of benzyl alcohol (0.61 mL, 5.9 mmol) in dry 1,4-dioxane (5 mL) was added sodium tert-butoxide (566.82 mg, 5.9 mmol) at 20 °C. The reaction mixture was stirred at room temperature for 1 hour. 4,6-dichloro-5-methyl-pyrimidin-2-amine (500.0 mg, 2.81 mmol, CAS: 39906-04-2) was added and the reaction mixture was stirred at room temperature for 12 hours, and then concentrated in vacuo. The residue was diluted with ethyl acetate and successively washed with 1 N aqueous ammonium chloride and brine. The organic layers were dried over magnesium sulfate, filtered and concentrated in vacuo to provide the title compound as a yellow solid (510 mg, 51 % yield). MS (ESI) m/z= 250.0 [M+H]+

### Step 2: 4-benzyloxy-6-methoxy-5-methyl-pyrimidin-2-amine

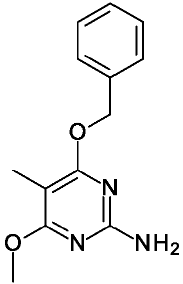

A mixture of 4-benzyloxy-6-chloro-5-methyl-pyrimidin-2-amine (3.1 g, 12.41 mmol) in 1,4-dioxane (50 mL) was cooled to 10 °C with an ice bath and a 1 M sodium methoxide solution (26.07 mL, 26.07 mmol) was added dropwise while maintaining the temperature. Then the reaction mixture was stirred at 90 °C overnight and concentrated in vacuo. The residue was diluted with ethyl acetate and successively washed with water and brine. The organic layers were dried over magnesium sulfate, filtered and concentrated in vacuo. The residue was purified by flash chromatography over silica gel to provide the title compound as a light yellow solid (245 mg, 14 % yield). MS (ESI) m/z= 246.2 [M+H]+

### Step 3: 2-amino-6-methoxy-5-methyl-pyrimidin-4-ol

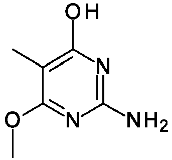

To a solution of 4-benzyloxy-6-methoxy-5-methyl-pyrimidin-2-amine (245.0 mg, 1 mmol) in dry methanol (50 mL) was added palladium on activated charcoal (200 mg, 1.88 mmol). The reaction mixture was purged with hydrogen and stirred under 1 atm. of hydrogen at room temperature for 12 hours. The reaction mixture was filtered through a pad of celite and concentrated in vacuo to provide the title compound as a white solid (150 mg, 68 % yield). 1H NMR (500 MHz, DMSO-d6) δ = 10.47 (br s, 1H), 6.41 (br s, 2H), 3.73 (s, 3H), 1.61 (s, 3H)

### Step 4: 4-(difluoromethoxy)-6-methoxy-5-methyl-pyrimidin-2-amine

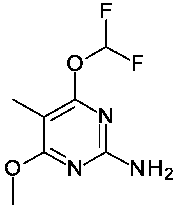

To a suspension of 2-amino-6-methoxy-5-methyl-pyrimidin-4-ol (0.15 g, 0.970 mmol) in acetonitrile (8.8 mL) was added 5 M potassium hydroxide (3.87 mL, 19.34 mmol) at 0 °C followed by dropwise addition of diethyl (bromodifluoromethyl)phosphonate (0.52 g, 1.93 mmol) in acetonitrile (1.5 mL) at 0 °C. The reaction mixture was allowed to warm to room temperature over 15 hours, poured into water and extracted twice with ethyl acetate. The organic layers were dried over magnesium sulfate, filtered and concentrated in vacuo to provide the title compound as an orange solid (150 mg, 45 % yield). MS (ESI) m/z= 206.0 [M+H]+

### Intermediate B76: 5-bromo-4-fluoro-6-methoxy-pyrimidin-2-amine

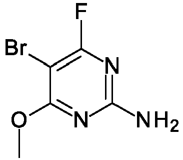

To a stirred suspension of (4-fluoro-6-methoxy-pyrimidin-2-yl)amine (850 mg, 5.64 mmol, CAS: 130687-25-1) in acetonitrile (45 mL) was added N-bromosuccinimide (1.01 g, 5.64 mmol). The reaction mixture was stirred at room temperature for 30 min, diluted with ethyl acetate and washed once with sat. NaHCO3 and once with brine. The organic layers were dried over sodium sulfate, filtered and concentrated in vacuo to provide the title compound as a light yellow solid (1.31 g, 99 % yield). MS (ESI) m/z= 222.0 [M+H]+

### Intermediate B77: 5-(2,2-difluoroethoxy)-4-fluoro-6-methoxy-pyrimidin-2-amine

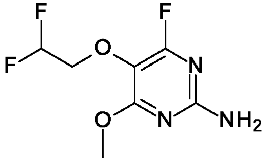

### Step 1: 5-bromo-4-fluoro-6-methoxy-N,N-bis[(4-methoxyphenyl)methyl]pyrimidin-2-amine

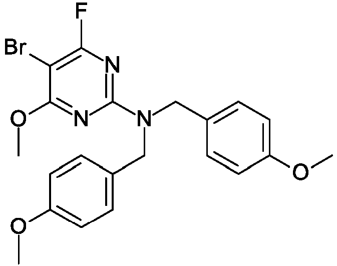

A solution of (5-bromo-4-fluoro-6-methoxy-pyrimidin-2-amine (704 mg, 3.01 mmol, intermediate B76) in N,N-dimethylacetamide (10 mL) was cooled to 0 °C. Sodium hydride (361.45 mg, 9.04 mmol) was added in 3 portions. The reaction mixture was stirred at 0 °C for 15 min. 4-methoxybenzyl chloride (962.78 mg, 829.98 uL, 6.02 mmol) was added. The reaction mixture was stirred at 0 °C for 30 min, carefully quenched with a saturated solution of ammonium chloride, poured into water and extracted twice with ethyl acetate. The organic layers were dried over sodium sulfate, filtered and concentrated in vacuo. The residue was purified by flash chromatography over silica gel using a gradient ethylacetate/heptane 0-15% to provide the title compound as a colorless viscous oil (702 mg, 49 % yield). MS (ESI) m/z= 464.1 [M+H]+

### Step 2: 5-(2,2-difluoroethoxy)-4-fluoro-6-methoxy-N,N-bis[(4-methoxyphenyl)methyl]pyrimidin-2-amine

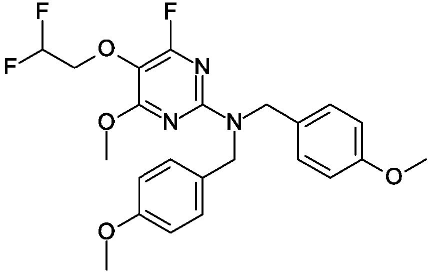

A colorless solution of 5-bromo-4-fluoro-6-methoxy-N,N-bis[(4-methoxyphenyl)methyl]pyrimidin-2-amine (160 mg, 0.336 mmol) in tetrahydrofuran (3 mL) was cooled to -72 °C. A 1.6 M n-butyllithium solution in hexanes (234.57 mg, 272.76 uL, 0.436 mmol) was added dropwise. The resulting light yellow solution was stirred at -72 °C for 30 min. Trimethyl borate (59.9 mg, 64.27 uL, 0.571 mmol) in tetrahydrofuran (300 uL) was added dropwise and the stirring was continued at -72 °C for 15 min. The reaction mixture was allowed to warm to 0 °C. Acetic acid (40.32 mg, 38.43 uL, 0.671 mmol) was added, followed by hydrogen peroxide (48.94 mg, 44.09 uL, 0.504 mmol). The reaction mixture was stirred at 0 °C for 45 min, allowed to warm to room temperature and stirred for 80 min. N-ethyldiisopropylamine (132.82 mg, 175.69 uL, 1.01 mmol) was added, followed by trifluoromethanesulfonic acid 2,2-difluoroethyl ester (80.68 mg, 50.17 uL, 0.369 mmol). The stirring was continued at room temperature for 5 min. Cesium carbonate (334.84 mg, 1.01 mmol) was added followed by trifluoromethanesulfonic acid 2,2-difluoroethyl ester (80.68 mg, 50.17 uL, 0.369 mmol). The reaction mixture was stirred at room temperature for 15 hours, poured into brine and extracted twice with ethyl acetate. The organic layers were dried over sodium sulfate, filtered and concentrated in vacuo. The residue was purified by flash chromatography over silica gel using a gradient ethylacetate/heptane 0-30% to provide the title compound as a colorless gum (94 mg, 60 % yield). MS (ESI) m/z= 464.2 [M+H]+

### Step 3: 5-(2,2-difluoroethoxy)-4-fluoro-6-methoxy-pyrimidin-2-amine

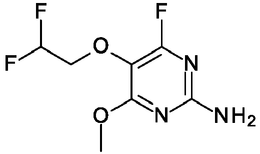

A solution of 5-(2,2-difluoroethoxy)-4-fluoro-6-methoxy-N,N-bis[(4-methoxyphenyl)methyl]pyrimidin-2-amine (92 mg, 0.199 mmol) in dichloromethane (300 uL) was cooled to 0 °C. Trifluoroacetic acid (1.37 g, 921.3 uL, 11.91 mmol) was added. The reaction mixture was allowed to warm to room temperature, stirred for 16 hours and concentrated in vacuo. The residue was poured into sat. NaHCO3 and extracted twice with ethyl acetate. The organic layers were dried over sodium sulfate, filtered and concentrated in vacuo. The residue was purified by flash chromatography over silica gel using a gradient ethylacetate/heptane 0-50% to provide the title compound as a white solid (41 mg, 88 % yield). MS (ESI) m/z= 224.1 [M+H]+

### Intermediate B78: 4-chloro-5,6-dimethoxy-pyrimidin-2-amine

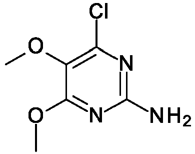

A suspension of (4,6-dichloro-5-methoxy-pyrimidin-2-yl)amine (100 mg, 0.490 mmol, CAS: 13428-25-6) and sodium methylate (27.86 mg, 0.490 mmol) in methanol (2.5 mL) was stirred at 65 °C for 17 hours, at 80 °C for 1.5 hours and concentrated in vacuo. The residue was purified by flash chromatography over silica gel using a gradient ethylacetate/heptane 0-50% to provide the title compound as a white solid (30 mg, 32 % yield). MS (ESI) m/z= 190.1 [M+H]+

### Intermediate B79: 4-chloro-5-(2,2-difluoroethoxy)pyrimidin-2-amine

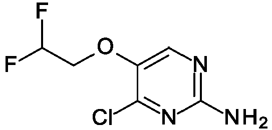

### Step 1: ethyl 2-(2,2-difluoroethoxy)acetate

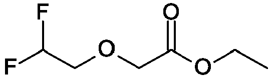

Sulfuric acid (10.0 mL, 183.52 mmol) was dissolved in ethanol (150 mL). 2-(2,2-difluoroethoxy)acetic acid (10.0 g, 71.38 mmol) was added. The reaction mixture was stirred at reflux for 3 hours, poured in water and extracted twice with diethylether. The organic layers were washed once with sat. NaHCO3, four times with brine, dried over sodium sulfate, filtered and evaporated carefully without vacuum to provide the title compound as a light brown liquid (9.3 g, 62 % yield). 1H NMR (400 MHz, CHLOROFORM-d) δ = 5.88 (tt, J = 4.1, 55.3 Hz, 1H), 4.17 (d, J = 7.2 Hz, 2H), 4.12 - 4.11 (m, 2H), 3.73 (d, J = 4.0 Hz, 2H), 1.26 - 1.20 (m, 3H)

### Step 2: ethyl (Z)-2-(2,2-difluoroethoxy)-3-(dimethylamino)prop-2-enoate

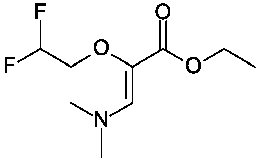

A mixture of ethyl 2-(2,2-difluoroethoxy)acetate (4.8 g, 28.55 mmol) and tert-butoxy bis(dimethylamino)methane (35.0 mL, 169.5 mmol) was heated at 140 °C for 20 hours and concentrated in vacuo to provide the title compound as a dark brown oil (5.25 g, 58 % yield). 1H NMR (400 MHz, CHLOROFORM-d) δ = 6.82 - 6.79 (m, 1H), 6.02 (tt, J = 4.1, 55.5 Hz, 1H), 4.19 - 4.08 (m, 2H), 3.95 - 3.83 (m, 2H), 3.02 (s, 6H), 2.51 (s, 0H), 1.25 (s, 3H)

### Step 3: 2-amino-5-(2,2-difluoroethoxy)-1H-pyrimidin-6-one

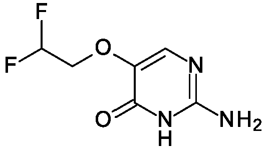

To a solution of ethyl (Z)-2-(2,2-difluoroethoxy)-3-(dimethylamino)prop-2-enoate (5.25 g, 23.52 mmol) in N-methyl-2-pyrrolidinone (60 mL) was added guanidine carbonate (8.47 g, 47.04 mmol) and potassium carbonate (6.5 g, 47.04 mmol). The reaction mixture was stirred at 150 °C for 24 hours and concentrated in vacuo. The residue was purified by flash chromatography over silica gel to provide the title compound as a light yellow solid (950 mg, 17 % yield). MS (ESI) m/z= 192.2 [M+H]+

### Step 4: 4-chloro-5-(2,2-difluoroethoxy)pyrimidin-2-amine

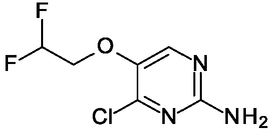

A solution of 2-amino-5-(2,2-difluoroethoxy)-1H-pyrimidin-6-one (3.0 g, 15.7 mmol) in phosphorus oxychloride (29.26 mL, 313.91 mmol) was heated at reflux for 16 hours. After that, the reaction mixture was evaporated to dryness and water was added (10 mL). The resulting mixture was stirred at room temperature for 30 minutes and a 6 % aqueous solution of ammonium hydroxide was added (10 mL). The resulting precipitate was filtered off. The filtrate was diluted with brine and extracted three times with ethyl acetate. The organic layers were dried over sodium sulfate, filtered and concentrated in vacuo to provide the title compound as a brown solid (2.5 g, 61 % yield). 1H NMR (500 MHz, DMSO-d6) δ = 8.21 (s, 1H), 6.48 - 6.18 (m, 1H), 4.29 (dt, J = 3.5, 14.7 Hz, 2H)

### Intermediate B80: 4-ethyl-6-methoxy-5-methyl-pyrimidin-2-amine

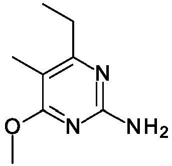

### Step 1: 4-ethyl-6-methoxy-pyrimidin-2-amine

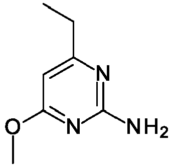

To a solution of 4-chloro-6-ethyl-pyrimidin-2-amine (1000.0 mg, 6.35 mmol, CAS: 5764-67-8) in tetrahydrofuran (10 mL) was added a 5 M sodium methoxide solution (3.17 mL, 15.86 mmol). The mixture was stirred at 60 °C for 3 hours, poured into water and extracted three times with ethyl acetate. The organic layers were dried over sodium sulfate, filtered and concentrated in vacuo to provide the title compound as a yellow oil (940 mg, 97 % yield). MS (ESI) m/z= 154.0 [M+H]+

### Step 2: 5-bromo-4-ethyl-6-methoxy-pyrimidin-2-amine

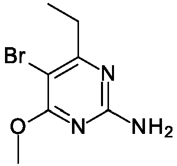

To a solution of 4-ethyl-6-methoxy-pyrimidin-2-amine (890.0 mg, 5.81 mmol) in acetonitrile (10 mL) was added N-bromosuccinimide (1240.91 mg, 6.97 mmol). The mixture was stirred at 25 °C for 12 hours and concentrated in vacuo. The residue was purified by flash chromatography over silica gel using a gradient ethylacetate/petroleum ether 0-50% to provide the title compound as a yellow solid (1100 mg, 82 % yield). MS (ESI) m/z= 232.0 [M+H]+

### Step 3: 4-ethyl-6-methoxy-5-methyl-pyrimidin-2-amine

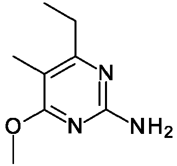

To a solution of 5-bromo-4-ethyl-6-methoxy-pyrimidin-2-amine (100.0 mg, 0.430 mmol) and trimethylboroxine (216.36 mg, 0.860 mmol) in 1,4-dioxane (2 mL) and water (0.400 mL) was added potassium carbonate (178.65 mg, 1.29 mmol) and, 1,1'-bis(diphenylphosphino)ferrocene-palladium(II)dichloride dichloromethane complex (35.19 mg, 0.040 mmol) under nitrogen. The reaction mixture was stirred at 100 °C for 12 hours, filtered and concentrated in vacuo. The residue was purified by preparative TLC (eluent: ethylacetate/petroleum ether 1/1) to provide the title compound as a colorless oil (40 mg, 28 % yield). MS (ESI) m/z= 168.1 [M+H]+

### Intermediate B81: 5-(2,2-difluoroethoxy)-4-ethyl-6-methoxy-pyrimidin-2-amine

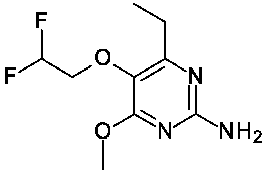

### Step 1: 5-bromo-4-ethyl-6-methoxy-N,N-bis[(4-methoxyphenyl)methyl]pyrimidin-2-amine

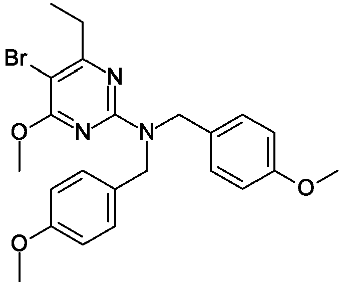

To a solution of 4-methoxybenzylchloride (4.05 mL, 29.86 mmol) in N,N-dimethylformamide (45 mL) was added sodium hydride (1706.31 mg, 42.66 mmol) at 0 °C. The mixture was stirred at 25 °C for 0.5 hour, then 5-bromo-4-ethyl-6-methoxy-pyrimidin-2-amine (3300.0 mg, 14.22 mmol, intermediate B80, step 2) was added. The mixture was stirred at 25 °C for 12 hours, quenched with a saturated solution of ammonium chloride and extracted three times with ethyl acetate. The organic layers were dried over sodium sulfate, filtered and concentrated in vacuo. The residue was purified by flash chromatography over silica gel using a gradient ethylacetate/petroleum ether 0-50% to provide the title compound as a white solid (5500 mg, 82 % yield). MS (ESI) m/z= 474.1 [M+H]+

### Step 2: 2-[bis[(4-methoxyphenyl)methyl]amino]-4-ethyl-6-methoxy-pyrimidin-5-ol

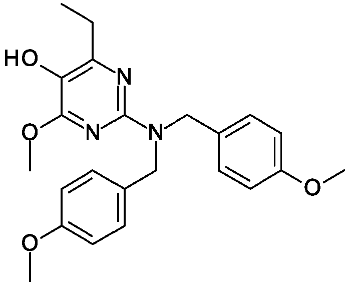

To a stirred solution of 5-bromo-4-ethyl-6-methoxy-N,N-bis[(4-methoxyphenyl)methyl]pyrimidin-2-amine (2000.0 mg, 4.23 mmol) in 25 mL of anhydrous tetrahydrofuran was added and a 2.5 M n-butyllithium solution (2.2 mL, 5.5 mmol). The reaction mixture was stirred at -78 °C for 30 min. Then trimethyl borate (1319.73 mg, 12.7 mmol) in tetrahydrofuran (6 mL) was added dropwise. The reaction mixture was stirred at -78 °C for 1.5 hours. The mixture was warm to 0 °C and hydrogen peroxide (9599.82 mg, 84.68 mmol) was added. The reaction mixture was stirred at 25 °C for 1 hour, poured into 0.1 N sodium sulfite and extracted three times with ethyl acetate. The organic layers were dried over sodium sulfate, filtered and concentrated in vacuo. The residue was purified by flash chromatography over silica gel using a gradient ethylacetate/petroleum ether 0-50% to provide the title compound as a white solid (1300 mg, 75 % yield). MS (ESI) m/z= 410.2 [M+H]+

### Step 3: 5-(2,2-difluoroethoxy)-4-ethyl-6-methoxy-N,N-bis[(4-methoxyphenyl)methyl]pyrimidin-2-amine

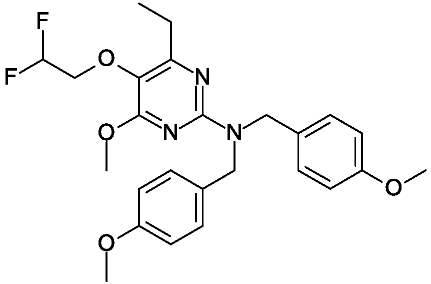

To a suspension of 2-[bis[(4-methoxyphenyl)methyl]amino]-4-ethyl-6-methoxy-pyrimidin-5-ol (400.0 mg, 0.980 mmol) and cesium carbonate (477.39 mg, 1.47 mmol) in acetonitrile (5 mL) was added 2,2-difluoroethyl trifluoromethanesulfonate (250.98 mg, 1.17 mmol). The mixture was stirred at 30 °C for 12 hours, poured into water and extracted twice with ethyl acetate. The organic layers were dried over sodium sulfate, filtered and concentrated in vacuo to provide the title compound as a yellow solid (400 mg, 86 % yield). MS (ESI) m/z= 474.2 [M+H]+

### Step 4: 5-(2,2-difluoroethoxy)-4-ethyl-6-methoxy-pyrimidin-2-amine

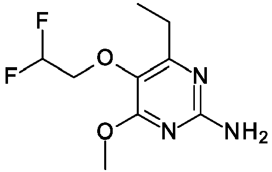

A mixture of 5-(2,2-difluoroethoxy)-4-ethyl-6-methoxy-N,N-bis[(4-methoxyphenyl)methyl]pyrimidin-2-amine (400.0 mg, 0.840 mmol) in trifluoroacetic acid (5.0 mL) was stirred at 25 °C for 12 hours, quenched with water (50 mL), filtered and purified by preparative HPLC to provide the title compound as a white solid (197 mg, 100 % yield). MS (ESI) m/z= 234.1 [M+H]+

### Intermediate B82: 5-(2,2-difluoroethyl)-4-methoxy-6-methyl-pyrimidin-2-amine

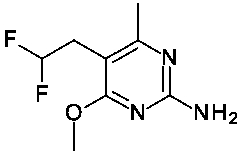

### Step 1: ethyl 2-acetyl-4,4-difluoro-butanoate

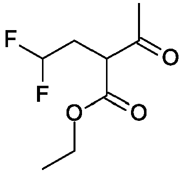

To a solution of ethyl acetoacetate (1.96 mL, 15.37 mmol) in tetrahydrofuran (50 mL) was added sodium hydride (737.67 mg, 18.44 mmol) at 0 °C under nitrogen. The mixture was stirred at 0 °C for 20 min, then a solution of 2,2-difluoroethyl trifluoromethanesulfonate (3.62 g, 16.9 mmol) in tetrahydrofuran (5 mL) was added. The reaction mixture was stirred at 20 °C under nitrogen for 16 hours, quenched with a saturated solution of ammonium chloride, poured into water and extracted three times with ethyl acetate. The organic layers were washed with brine, dried over sodium sulfate, filtered and concentrated in vacuo to provide the title compound as a yellow semisolid (5 g, 100 % yield). MS (ESI) m/z= 194.7 [M+H]+

### Step 2: 2-amino-5-(2,2-difluoroethyl)-6-methyl-pyrimidin-4-ol

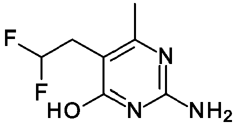

To a stirred solution of ethyl 2-acetyl-4,4-difluoro-butanoate (2300.0 mg, 7.11 mmol) in methanol (50 mL) was added a 5 M sodium methoxide solution (3.27 mL, 16.35 mmol), followed by guanidine hydrochloride (746.8 mg, 7.82 mmol) at 25 °C under nitrogen atmosphere. Then the reaction mixture was heated to 40 °C, stirred for 4 hours and quenched by addition of 1 N HCl (12 mL). The pH was adjusted to 7 with sat. NaHCO3. The mixture was poured into water and extracted three times with ethyl acetate. The organic layers were washed with brine, dried over sodium sulfate, filtered and concentrated in vacuo. The residue was purified by flash chromatography over silica gel using a gradient methanol/ethylacetate 0-8% to provide the title compound as a yellow solid (300 mg, 22 % yield). MS (ESI) m/z= 189.7 [M+H]+

### Step 3: 4-chloro-5-(2,2-difluoroethyl)-6-methyl-pyrimidin-2-amine

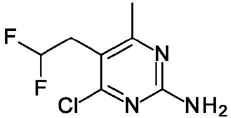

To a mixture of 2-amino-5-(2,2-difluoroethyl)-6-methyl-pyrimidin-4-ol (230.0 mg, 1.22 mmol) in acetonitrile (3 mL) was added phosphorus oxychloride (0.68 mL, 7.3 mmol), followed by triethylamine (0.17 mL, 1.22 mmol). The reaction mixture was stirred at 30 °C for 16 hours, quenched with water and extracted three times with ethyl acetate. The organic layers were washed with brine, dried over sodium sulfate, filtered and concentrated in vacuo. The residue was purified by preparative TLC (eluent: ethylacetate/petroleum ether 1/1 + 0.1% triethylamine) to provide the title compound as a white solid (80 mg, 32 % yield). MS (ESI) m/z= 207.7 [M+H]+

### Step 4: 5-(2,2-difluoroethyl)-4-methoxy-6-methyl-pyrimidin-2-amine

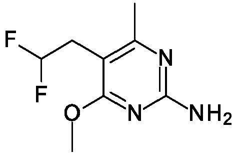

To a stirred suspension of 4-chloro-5-(2,2-difluoroethyl)-6-methyl-pyrimidin-2-amine (80.0 mg, 0.390 mmol) in tetrahydrofuran (6 mL) was added a 5 M sodium methoxide solution (0.1 mL, 0.520 mmol) at 20 °C under nitrogen atmosphere. The reaction mixture was stirred for 3 hours at 20 °C. Three times was added a 5 M sodium methoxide solution (0.02 mL, 0.080 mmol). The reaction mixture was quenched with water and extracted three times with ethyl acetate. The organic layers were washed with brine, dried over sodium sulfate, filtered and concentrated in vacuo. The residue was purified by preparative TLC (eluent: ethylacetate/petroleum ether 1/1 + 0.1% triethylamine) to provide the title compound as a white solid (80 mg, 32 % yield). MS (ESI) m/z= 207.7 [M+H]+

### Intermediate B83: 4-cyclopropyl-5-(2,2-difluoroethyl)-6-methoxy-pyrimidin-2-amine

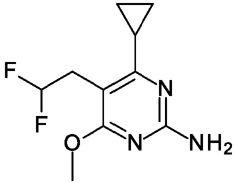

### Step 1: ethyl 2-(cyclopropanecarbonyl)-4,4-difluoro-butanoate

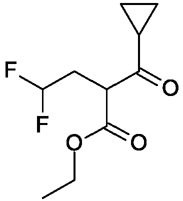

To a solution of methyl 3-cyclopropyl-3-oxopropanoate (2.0 g, 14.07 mmol) in tetrahydrofuran (50 mL) was added sodium hydride (675.34 mg, 16.88 mmol) at 0 °C under nitrogen. The mixture was stirred at 0 °C for 20 min, then a solution of 2,2-difluoroethyl trifluoromethanesulfonate (3.31 g, 15.48 mmol) in tetrahydrofuran (5 mL) was added. The reaction mixture was stirred at 20 °C under nitrogen for 16 hours, quenched with a saturated solution of ammonium chloride, poured into water and extracted three times with ethyl acetate. The organic layers were washed with brine, dried over sodium sulfate, filtered and concentrated in vacuo to provide the title compound as a yellow oil (2.9 g, 100 % yield). MS (ESI) m/z= 206.8 [M+H]+

### Step 2: 2-amino-6-cyclopropyl-5-(2,2-difluoroethyl)pyrimidin-4-ol

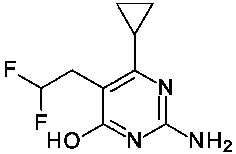

To a stirred solution of methyl 2-(cyclopropanecarbonyl)-4,4-difluoro-butanoate (3.1 g, 15.03 mmol) in methanol (80 mL) was added a 5 M sodium methoxide solution (6.92 mL, 34.58 mmol), followed by guanidine hydrochloride (1.58 g, 16.54 mmol) at 25 °C under nitrogen atmosphere. Then the reaction mixture was heated to 40 °C, stirred for 12 hours and quenched by addition of 2 N HCl (12 mL). The pH was adjusted to 7 with sat. NaHCO3. The mixture was poured into water and extracted three times with ethyl acetate. The organic layers were washed with brine, dried over sodium sulfate, filtered and concentrated in vacuo. The residue was purified by flash chromatography over silica gel using a gradient methanol/ethylacetate 0-1% to provide the title compound as a yellow solid (530 mg, 16 % yield). MS (ESI) m/z= 215.8 [M+H]+

### Step 3: 4-chloro-6-cyclopropyl-5-(2,2-difluoroethyl)pyrimidin-2-amine

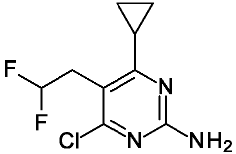

To a mixture of 2-amino-6-cyclopropyl-5-(2,2-difluoroethyl)pyrimidin-4-ol (210.0 mg, 0.980 mmol) in acetonitrile (10 mL) was added phosphorus oxychloride (0.27 mL, 2.93 mmol), followed by triethylamine (0.14 mL, 0.980 mmol). The reaction mixture was stirred at 30 °C for 20 hours, poured into water and extracted three times with ethyl acetate. The organic layers were washed with brine, dried over sodium sulfate, filtered and concentrated in vacuo. The residue was purified by flash chromatography over silica gel using a gradient ethylacetate/petroleum ether 0-30% to provide the title compound as a yellow solid (30 mg, 13 % yield). MS (ESI) m/z= 233.7 [M+H]+

### Step 4: 4-cyclopropyl-5-(2,2-difluoroethyl)-6-methoxy-pyrimidin-2-amine

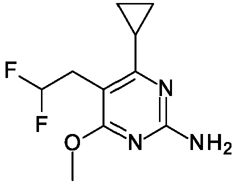

To a stirred suspension of 4-chloro-6-cyclopropyl-5-(2,2-difluoroethyl)pyrimidin-2-amine (47.0 mg, 0.200 mmol) in tetrahydrofuran (3 mL) was added a 5 M sodium methoxide solution (0.05 mL, 0.270 mmol) at 20 °C under nitrogen atmosphere. The reaction mixture was stirred for 5 hours at 20 °C, quenched with water and extracted three times with ethyl acetate. The organic layers were washed with brine, dried over sodium sulfate, filtered and concentrated in vacuo. The residue was purified by preparative TLC (eluent: ethylacetate/petroleum ether 3/1) to provide the title compound as a yellow solid (19 mg, 41 % yield). MS (ESI) m/z= 230.1 [M+H]+

### Intermediate B84: 5-bromo-4-(cyclopropylmethyl)-6-methoxy-pyrimidin-2-amine

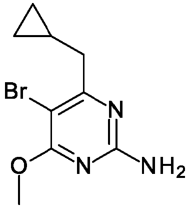

### Step 1: 2-amino-6-(cyclopropylmethyl)pyrimidin-4-ol

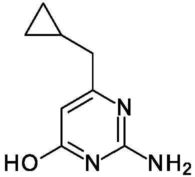

Ethyl 4-cyclopropyl-3-oxobutanoate (890.87 mg, 5.23 mmol) was dissolved in ethanol, extra dry (10 mL) and guanidine hydrochloride (500 mg, 5.23 mmol) and sodium ethoxide (5.09 g, 5.85 mL, 15.7 mmol) were added. The mixture was stirred for 16 hours at 75 °C. 4 N HCl (2.5 mL) was added and the mixture was concentrated in vacuo to provide the title compound as a light brown solid (864.6 mg, 100 % yield), which was used in the next step without further purification. MS (ESI) m/z= 166.1 [M+H]+

### Step 2: 4-chloro-6-(cyclopropylmethyl)pyrimidin-2-amine

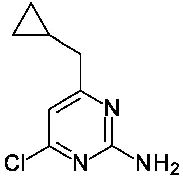

2-amino-6-(cyclopropylmethyl)pyrimidin-4-ol (2.22 g, 5.39 mmol) was mixed with neat phosphorus oxychloride (7.9 g, 4.8 mL, 51.5 mmol). The reaction mixture was stirred at 100 °C for 2 hours and concentrated in vacuo. The residue was poured into sat. NaHCO3 and ethyl acetate. The mixture was stirred at room temperature for 10 min and extracted three times with ethyl acetate. The organic layers were dried over sodium sulfate, filtered and concentrated in vacuo. The residue was purified by flash chromatography over silica gel using a gradient ethylacetate/heptane 0-70% to provide the title compound as a yellow solid (667.6 mg, 65 % yield). MS (ESI) m/z= 184.1 [M+H]+

### Step 3: 4-(cyclopropylmethyl)-6-methoxy-pyrimidin-2-amine

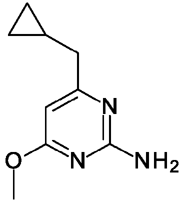

To a suspension of 4-chloro-6-(cyclopropylmethyl)pyrimidin-2-amine (639.2 mg, 3.48 mmol) in methanol (16.03 mL) was added sodium hydride (220.18 mg, 5.5 mmol) at room temperature. The reaction mixture was stirred at 80 °C for 2 hours and concentrated in vacuo. The residue was poured into water and extracted three times with ethyl acetate. The organic layers were washed three times with brine, dried over sodium sulfate, filtered and concentrated in vacuo. The residue was purified by flash chromatography over silica gel using a gradient ethylacetate/heptane 0-100% to provide the title compound as a yellow oil (600.4 mg, 87 % yield). MS (ESI) m/z= 180.2 [M+H]+

### Step 4: 5-bromo-4-(cyclopropylmethyl)-6-methoxy-pyrimidin-2-amine

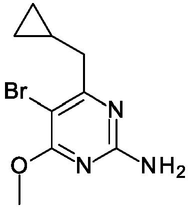

To a solution of 4-(cyclopropylmethyl)-6-methoxy-pyrimidin-2-amine (104 mg, 0.580 mmol) in ethyl acetate (3 mL) was added N-bromosuccinimide (103.28 mg, 0.580 mmol) at room temperature. The reaction mixture was then stirred 23 °C for 2 hours, poured into water and extracted three times with ethyl acetate. The organic layers were washed three times with brine, dried over sodium sulfate, filtered and concentrated in vacuo to provide the title compound as a yellow solid (124.2 mg, 80 % yield). MS (ESI) m/z= 258.2 [M+H]+

### Intermediate B85: 5-bromo-4-methoxy-6-(methoxymethyl)pyrimidin-2-amine

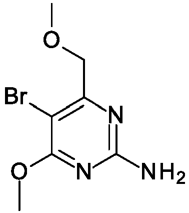

### Step 1: 2-amino-6-(methoxymethyl)pyrimidin-4-ol

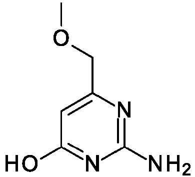

Guanidine hydrochloride (1.33 g, 13.92 mmol) was dissolved in ethanol, extra dry (26.6 mL) and sodium ethoxide (13.53 g, 15.59 mL, 41.77 mmol) and methyl 4-methoxyacetoacetate (2.03 g, 1.8 mL, 13.92 mmol) were added. The mixture was stirred for 18 hours at 80 °C. 4 N HCl (10.5 mL) was added and, after stirring at room temperature for 20 min, concentrated in vacuo to provide the title compound as a dark red solid (2.78 g, 51 % yield), which was directly used in the next step without further purification. MS (ESI) m/z= 156.1 [M+H]+

### Step 2: 4-chloro-6-(methoxymethyl)pyrimidin-2-amine

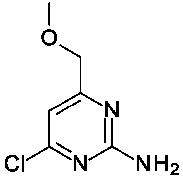

2-amino-6-(methoxymethyl)pyrimidin-4-ol (2.2 g, 5.67 mmol) was mixed with neat phosphorus oxychloride (7.27 g, 4.42 mL, 47.44 mmol). The reaction mixture was stirred at 100 °C for 2 hours and concentrated in vacuo. The residue was poured into sat. NaHCO3 and ethyl acetate. The mixture was stirred at room temperature for 10 min and extracted three times with ethyl acetate. The organic layers were dried over sodium sulfate, filtered and concentrated in vacuo. The residue was purified by flash chromatography over silica gel using a gradient ethylacetate/heptane 0-70% to provide the title compound as a yellow solid (590.5 mg, 56 % yield). MS (ESI) m/z= 174.1 [M+H]+

### Step 3: 4-methoxy-6-(methoxymethyl)pyrimidin-2-amine

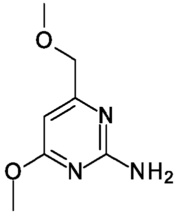

To a suspension of 4-chloro-6-(methoxymethyl)pyrimidin-2-amine (576.1 mg, 3.32 mmol) in methanol (15.28 mL) was added sodium hydride (209.92 mg, 5.25 mmol) at room temperature. The reaction mixture was stirred at 80 °C for 1 hour and concentrated in vacuo. The residue was poured into water and extracted three times with ethyl acetate. The organic layers were washed three times with brine, dried over sodium sulfate, filtered and concentrated in vacuo. The residue was purified by SFC to provide the title compound as a white powder (247 mg, 44 % yield). MS (ESI) m/z= 170.1 [M+H]+

### Step 4: 5-bromo-4-methoxy-6-(methoxymethyl)pyrimidin-2-amine

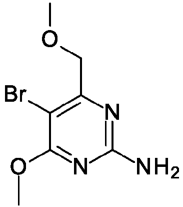

To a solution of 4-methoxy-6-(methoxymethyl)pyrimidin-2-amine (191.6 mg, 1.13 mmol) in ethyl acetate (5.85 mL) was added N-bromosuccinimide (201.57 mg, 1.13 mmol) at room temperature. The reaction mixture was then stirred 23 °C for 2 hours, poured into water and extracted three times with ethyl acetate. The organic layers were washed three times with brine, dried over sodium sulfate, filtered and concentrated in vacuo to provide the title compound as a white powder (254.5 mg, 89 % yield). MS (ESI) m/z= 248.1 [M+H]+

### Intermediate B86: 5-bromo-4-methoxy-6-tetrahydropyran-4-yl-pyrimidin-2-amine

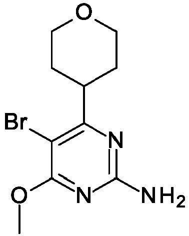

### Step 1: 4-(3,6-dihydro-2H-pyran-4-yl)-6-methoxy-pyrimidin-2-amine

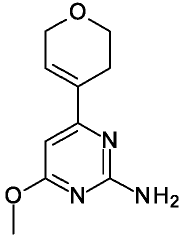

To a solution 2-amino-4-chloro-6-methoxypyrimidine (5.0 g, 31.33 mmol, CAS: 5734-64-5) in 1,4-dioxane (150 mL) and water (15 mL) were 3,6-dihydro-2H-pyran-4-boronic acid pinacol ester (9.87 g, 47 mmol), tetrakis(triphenylphosphine)palladium(0) (3.63 g, 3.13 mmol) and sodium carbonate (4.98 g, 47 mmol) under argon. The reaction mixture was stirred at 100 °C for 12 hours and concentrated in vacuo. The residue was diluted with ethyl acetate and washed twice with water and once with brine. The organic layer was dried over magnesium sulfate, filtered and concentrated in vacuo. The residue was purified by flash chromatography over silica gel to provide the title compound as a light yellow solid (4 g, 55 % yield). 1H NMR (500 MHz, DMSO-d6) δ = 6.79 (br s, 1H), 6.43 (br s, 2H), 6.04 (s, 1H), 4.21 (br s, 2H), 3.78 (s, 3H), 3.74 (br t, J = 5.1 Hz, 2H), 2.42 - 2.28 (m, 2H)

### Step 2: 4-methoxy-6-tetrahydropyran-4-yl-pyrimidin-2-amine

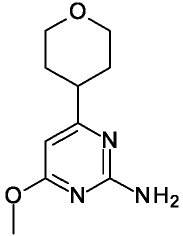

To a solution of 4-(3,6-dihydro-2H-pyran-4-yl)-6-methoxy-pyrimidin-2-amine (2.1 g, 8.61 mmol) in methanol (50 mL) and tetrahydrofuran (50 mL) was added platinum on carbon (500 mg). The reaction mixture was purged with hydrogen and stirred under 1 atm. of hydrogen at room temperature for 12 hours. The reaction mixture was filtered through a pad of celite and concentrated in vacuo to provide the title compound as a light yellow solid (1.8 g, 80 % yield). MS (ESI): m/z= 210.1 [M+H]+

### Step 3: 5-bromo-4-methoxy-6-tetrahydropyran-4-yl-pyrimidin-2-amine

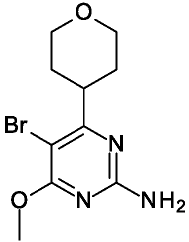

To a solution of 4-methoxy-6-tetrahydropyran-4-yl-pyrimidin-2-amine (1.0 g, 4.78 mmol) in N,N-dimethylformamide (20 mL) was added N-bromosuccinimide (1.11 g, 6.21 mmol) at 25 °C. The reaction mixture was then stirred at room temperature for 6 hours and concentrated in vacuo. The residue was diluted with ethyl acetate and washed twice with water and once with brine. The organic layer was dried over magnesium sulfate, filtered and concentrated in vacuo to provide the title compound as a light yellow solid (900 mg, 53 % yield). MS (ESI) m/z= 290.0 [M+H]+

### Intermediate B87: 4,6-dimethoxy-5-(oxetan-3-ylmethoxy)pyrimidin-2-amine

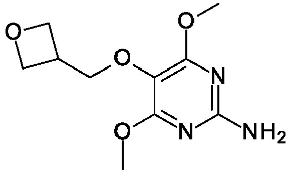

### Step 1: 4,6-dimethoxy-N,N-bis[(4-methoxyphenyl)methyl]-5-foxetan-3-ylmethoxy)pyrimidin-2-amine

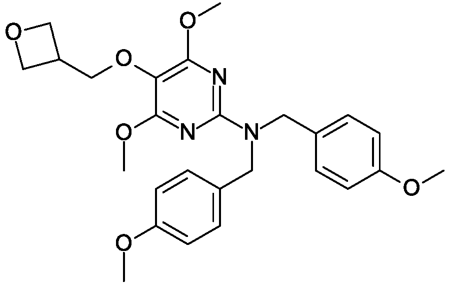

A mixture of 2-[bis[(4-methoxyphenyl)methyl]amino]-4,6-dimethoxy-pyrimidin-5-ol (410 mg, 0.996 mmol, intermediate B54, step 3), 3-(bromomethyl)oxetane (451.4 mg, 2.99 mmol) and potassium carbonate (413.17 mg, 2.99 mmol) in N,N-dimethylformamide (8 mL) was stirred at 80 °C for 4.5 hours. The reaction mixture was poured into water and extracted twice with ethyl acetate. The organic layers were washed with brine, dried over sodium sulfate, filtered and concentrated in vacuo. The residue was purified by flash chromatography over silica gel using a gradient ethylacetate/heptane 0-40% to provide the title compound as a colorless oil (444 mg, 83 % yield). MS (ESI) m/z= 482.5 [M+H]+

### Step 2: 4,6-dimethoxy-5-(oxetan-3-ylmethoxy)pyrimidin-2-amine

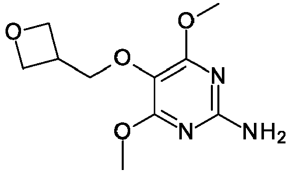

4,6-dimethoxy-N,N-bis[(4-methoxyphenyl)methyl]-5-(oxetan-3-ylmethoxy)pyrimidin-2-amine (349 mg, 0.725 mmol) was dissolved in methanol (50 mL). Palladium hydroxide on carbon (101.78 mg, 0.036 mmol) was added under argon. The reaction mixture was purged and backfilled with hydrogen and then stirred under 1 atm. of hydrogen for 5 days (after 4 days, palladium hydroxide on carbon (101.78 mg, 0.036 mmol) was added again). The reaction mixture was filtered through a pad of celite and concentrated in vacuo. The residue was purified by flash chromatography over silica gel using a gradient ethylacetate/heptane 0-55% to provide the title compound as a white semisolid (153 mg, 85 % yield). MS (ESI) m/z= 242.2 [M+H]+

### Intermediate B88: 5-(2,3-difluoropropyl)-4,6-dimethoxy-pyrimidin-2-amine

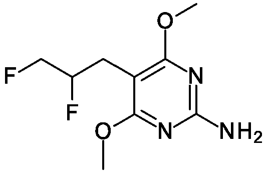

### Step 1: 5-allyl-4,6-dimethoxy-N,N-bis[(4-methoxyphenyl)methyllpyrimidin-2-amine

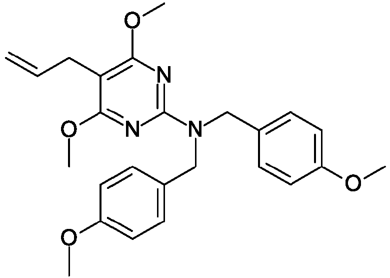

To a solution of 5-allyl-4,6-dimethoxy-pyrimidin-2-amine (1.8 g, 9.22 mmol, intermediate B59) in N,N-dimethylformamide (20 mL) was added sodium hydride (1.48 g, 36.88 mmol) at 0 °C under nitrogen and the mixture was stirred at room temperature for 10 min. Then 4-methoxybenzylchloride (2.5 mL, 18.44 mmol) was added. The reaction mixture was stirred at room temperature overnight. The reaction mixture was poured into water and extracted three times with ethyl acetate. The organic layers were washed successively with brine and a saturated solution of ammonium chloride, dried over sodium sulfate, filtered and concentrated in vacuo. The residue was purified by flash chromatography over silica gel using a gradient ethylacetate/petroleum ether 30-50% to provide the title compound as a white solid (3.3 g, 72 % yield). MS (ESI) m/z= 436.3 [M+H]+

### Step 2: 3-[2-[bis[(4-methoxyphenyl)methyl]amino]-4,6-dimethoxy-pyrimidin-5-yl]propane-1,2-diol

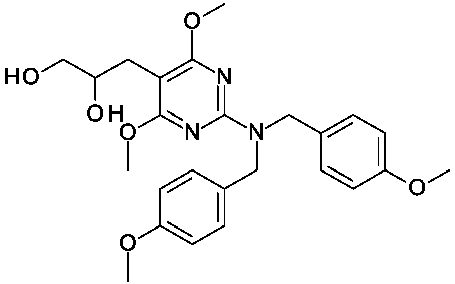

To a stirring solution of 5-allyl-4,6-dimethoxy-N,N-bis[(4-methoxyphenyl)methyl]pyrimidin-2-amine (3.3 g, 7.58 mmol) in THF (25 mL) and water (5 mL) was added N-Methylmorpholine N-oxide (1.78 g, 15.15 mmol) and then osmium tetroxide (385.27 mg, 1.52 mmol). The reaction mixture was stirred at 25 °C for 16 hours. 50 mL of an aqueous solution of Na2S2O3 (10 g) was added to the mixture. After stirring for a few minutes, the reaction mixture was extracted three times with ethyl acetate. The organic layers were washed with brine, dried over sodium sulfate, filtered and concentrated in vacuo to provide the title compound as a white solid (1.5 g, 42 % yield). MS (ESI) m/z= 470.3 [M+H]+

### Step 3: 5-(2,3-difluoropropyl)-4,6-dimethoxy-N,N-bis[(4-methoxyphenyl)methyl]pyrimidin-2-amine

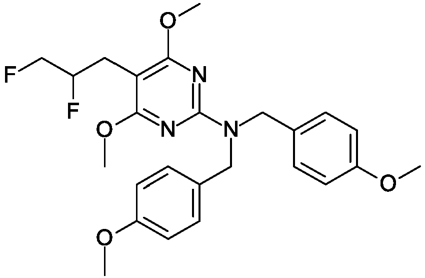

To a solution of diethylaminosulfur trifluoride (0.84 mL, 6.39 mmol) in dichloromethane (10 mL) was added 3-[2-[bis[(4-methoxyphenyl)methyl]amino]-4,6-dimethoxy-pyrimidin-5-yl]propane-1,2-diol (600.0 mg, 1.28 mmol) in dichloromethane (10 mL) at 0-10 °C under nitrogen. The reaction mixture was stirred at 0 °C for 1 hour, quenched with sat. NaHCO3 and extracted three times with dichloromethane. The organic layers were dried over sodium sulfate, filtered and concentrated in vacuo. The residue was purified by flash chromatography over silica gel using a gradient ethylacetate/petroleum ether 10-20% to provide the title compound as a white waxy solid (605 mg, 100 % yield). MS (ESI) m/z= 474.3 [M+H]+

### Step 4: 5-(2,3-difluoropropyl)-4,6-dimethoxy-pyrimidin-2-amine

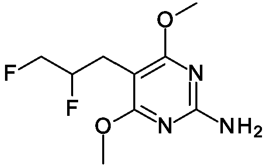

A solution of 5-(2,3-difluoropropyl)-4,6-dimethoxy-N,N-bis[(4-methoxyphenyl)methyl]pyrimidin-2-amine (900.0 mg, 1.9 mmol) in trifluoroactic acid (12.86 mL, 173.1 mmol) was stirred for 2 hours at 60 °C, poured into sat. NaHCO3 and extracted twice with ethyl acetate. The organic layers were washed with brine, dried over sodium sulfate, filtered and concentrated in vacuo. The residue was purified by flash chromatography over silica gel using a gradient ethylacetate/petroleum ether 30-50%, followed by preparative HPLC to provide the title compound as a white solid (85 mg, 19 % yield). MS (ESI) m/z= 234.2 [M+H]+

### Intermediate B89: 2-(2-amino-4,6-dimethoxy-pyrimidin-5-yl)oxyacetonitrile

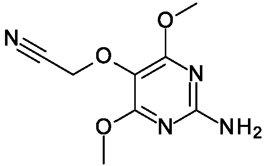

### Step 1: 2-[2-[bis[(4-methoxyphenyl)methyl]amino]-4,6-dimethoxy-pyrimidin-5-yl]oxyacetonitrile

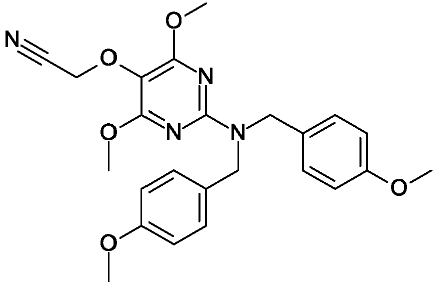

A mixture of 2-[bis[(4-methoxyphenyl)methyl]amino]-4,6-dimethoxy-pyrimidin-5-ol (1 g, 2.43 mmol, intermediate B54, step 3), iodoacetonitrile (1.22 g, 529.25 uL, 7.29 mmol) and potassium carbonate (1.01 g, 7.29 mmol) in N,N-dimethylformamide (16 mL) was stirred at 80 °C overnight. The reaction mixture was poured into water and extracted twice with ethyl acetate. The organic layers were washed with brine, dried over sodium sulfate, filtered and concentrated in vacuo. The residue was purified by flash chromatography over silica gel using a gradient ethylacetate/heptane 0-20% to provide the title compound as a white solid (855 mg, 77 % yield). MS (ESI) m/z= 451.3 [M+H]+

### Step 2: 2-(2-amino-4,6-dimethoxy-pyrimidin-5-yl)oxyacetonitrile

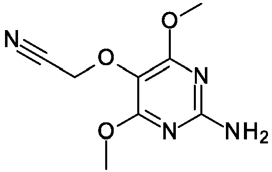

A mixture of 2-[2-[bis[(4-methoxyphenyl)methyl]amino]-4,6-dimethoxy-pyrimidin-5-yl]oxyacetonitrile (855 mg, 1.9 mmol) and trifluoroacetic acid (6.49 g, 4.39 mL, 56.94 mmol) in dichloromethane (6 mL) was stirred at 50 °C overnight. The reaction mixture was concentrated in vacuo, poured into sat. NaHCO3 and extracted twice with ethyl acetate. The organic layers were washed with brine, dried over sodium sulfate, filtered and concentrated in vacuo. The residue was purified by flash chromatography over silica gel using a gradient ethylacetate/heptane 0-39% to provide the title compound as an off-white solid (406 mg, 92 % yield). MS (ESI) m/z= 211.1 [M+H]+

### Intermediate B90: 4-methoxy-5-(2,3,3-trifluoropropyl)pyrimidin-2-amine

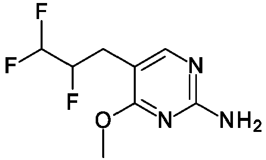

### Step 1: 1-[2-[bis[(4-methoxyphenyl)methyl]amino]-4-methoxy-pyrimidin-5-yl]-2,2,3,3-tetrafluoro-propan-1-one

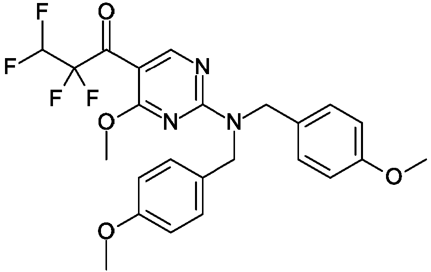

To a solution of ethyl 2,2,3,3-tetrafluoropropanoate (4936.83 mg, 28.36 mmol) in THF (40 mL) was added a 2.5 M n-butyllithium solution (4.92 mL, 12.29 mmol) dropwise at -78 °C. The mixture was stirred at -78 °C for 1.15 hours. 5-bromo-4-methoxy-N,N-bis[(4-methoxyphenyl)methyl]pyrimidin-2-amine (4200.0 mg, 9.45 mmol, intermediate B3, step 1) was added to the reaction. The reaction mixture was stirred at -78 °C for 1 hour, quenched with a saturated solution of ammonium chloride and extracted three times with ethyl acetate. The organic layers were dried over sodium sulfate, filtered and concentrated in vacuo. The residue was purified by flash chromatography over silica gel using a gradient ethylacetate/petroleum ether 0-30% to provide the title compound as a yellow solid (3400 mg, 73 % yield). MS (ESI) m/z= 494.0 [M+H]+

### Step 2: 1-[2-[bis[(4-methoxyphenyl)methyl]amino]-4-methoxy-pyrimidin-5-yl]-2,2,3,3-tetrafluoro-propan-1-ol

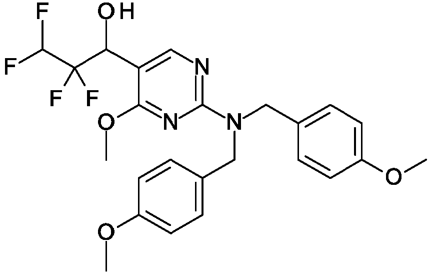

To a solution of 1-[2-[bis[(4-methoxyphenyl)methyl]amino]-4-methoxy-pyrimidin-5-yl]-2,2,3,3-tetrafluoro-propan-1-one (2250.0 mg, 4.56 mmol) in methanol (40 mL) was added sodium borohydride (862.47 mg, 22.8 mmol) at 0 °C. The reaction mixture was stirred at 25 °C for 1 hour, poured into a saturated solution of ammonium chloride and extracted three times with ethyl acetate. The organic layers were dried over sodium sulfate, filtered and concentrated in vacuo. The residue was purified by flash chromatography over silica gel using a gradient ethylacetate/petroleum ether 0-30% to provide the title compound as a light yellow solid (2259 mg, 100 % yield). MS (ESI) m/z= 496.0 [M+H]+

### Step 3: 1-[2-[bis[(4-methoxyphenyl)methyl]amino]-4-methoxy-pyrimidin-5-yl]-2,3,3-trifluoro-prop-2-en-1-ol

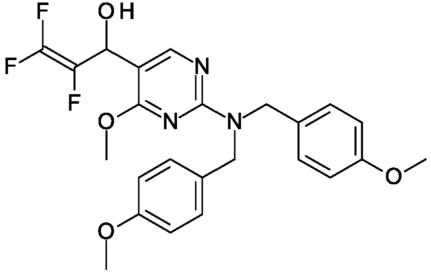

To a solution of 1-[2-[bis[(4-methoxyphenyl)methyl]amino]-4-methoxy-pyrimidin-5-yl]-2,2,3,3-tetrafluoro-propan-1-ol (2200.0 mg, 4.44 mmol) in THF (60 mL) was added a 2 M lithium diisopropylamide solution (17.76 mL, 35.52 mmol) at 0 °C. The mixture was stirred at 0 °C for 2 hours, poured into a saturated solution of ammonium chloride and extracted three times with ethyl acetate. The organic layers were dried over sodium sulfate, filtered and concentrated in vacuo. The residue was purified by flash chromatography over silica gel using a gradient ethylacetate/petroleum ether 0-30% to provide the title compound as a light yellow solid (1200 mg, 57 % yield). MS (ESI) m/z= 476.0 [M+H]+

### Step 4: 5-(1-chloro-2,3,3-trifluoro-allyl)-4-methoxy-N,N-bis[(4-methoxyphenyl)methyl]pyrimidin-2-amine

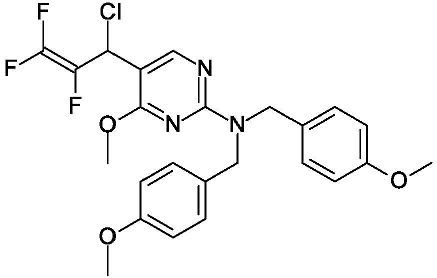

A mixture of 1-[2-[bis[(4-methoxyphenyl)methyl]amino]-4-methoxy-pyrimidin-5-yl]-2,3,3-trifluoro-prop-2-en-1-ol (450.0 mg, 0.950 mmol) in thionyl chloride (5.0 mL) was stirred at 25 °C for 1 hour and concentrated in vacuo to provide the title compound as an off-white solid (450 mg, 96 % yield), which was directly used in the next step. MS (ESI) m/z= 494.0 [M+H]+

### Step 5: 4-methoxy-N,N-bis[(4-methoxyphenyl)methyl]-5-(2,3,3-trifluoropropyl)pyrimidin-2-amine

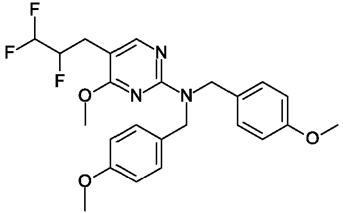

A solution of 5-(1-chloro-2,3,3-trifluoro-allyl)-4-methoxy-N,N-bis[(4-methoxyphenyl)methyl]pyrimidin-2-amine (450.0 mg, 0.910 mmol) was dissolved in ethyl acetate (20 mL). Palladium on activated charcoal (450 mg) was added under nitrogen. The reaction mixture was purged and backfilled three times with hydrogen and then stirred under 1 atm. of hydrogen for 2 hours. The reaction mixture was filtered through a pad of celite and concentrated in vacuo. The residue was purified by preparative HPLC to provide the title compound as a light yellow oil (230 mg, 55 % yield). MS (ESI) m/z= 462.0 [M+H]+

### Step 6: 4-methoxy-5-(2,3,3-trifluoropropyl)pyrimidin-2-amine

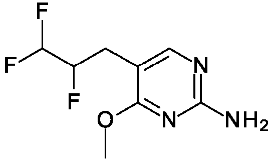

A mixture of 4-methoxy-N,N-bis[(4-methoxyphenyl)methyl]-5-(2,3,3-trifluoropropyl)pyrimidin-2-amine (230.0 mg, 0.500 mmol) in trifluoroacetic acid (3.0 mL) was stirred at 25° C for 24 hours, quenched with sat. NaHCO3 and extracted three times with ethyl acetate. The organic layers were dried over sodium sulfate, filtered and concentrated in vacuo. The residue was purified by preparative TLC (eluent: ethylacetate/petroleum ether 1/1) to provide the title compound as a light yellow solid (65 mg, 59 % yield). MS (ESI) m/z= 222.0 [M+H]+

### Intermediate B91: 5-bromo-4-(1,1-difluoroethyl)-6-methoxy-pyrimidin-2-amine

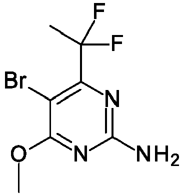

### Step 1: methyl 2-[bis[(4-methoxyphenyl)methyl]amino]-6-methoxy-pyrimidine-4-carboxylate

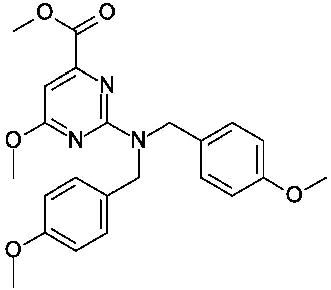

A colorless solution of 2-chloro-6-methoxy-pyrimidine-4-carboxylic acid methyl ester (300 mg, 1.48 mmol, CAS: 127861-30-7), bis-(4-methoxybenzyl)-amine (419.15 mg, 1.63 mmol) and n-ethyldiisopropylamine (390.56 mg, 516.62 uL, 2.96 mmol) in N-methyl-2-pyrrolidinone (6 mL) was heated to 80 °C and stirred for 22 hours, then diluted with ethyl acetate and washed twice with brine. The organic layer was dried over sodium sulfate, filtered and concentrated in vacuo. The residue was purified by flash chromatography over silica gel using a gradient ethylacetate/heptane 0-30% to provide the title compound as a white solid (454 mg, 71 % yield). MS (ESI) m/z= 424.2 [M+H]+

### Step 2: 2-[bis[(4-methoxyphenyl)methyl]amino]-6-methoxy-pyrimidine-4-carboxylic acid

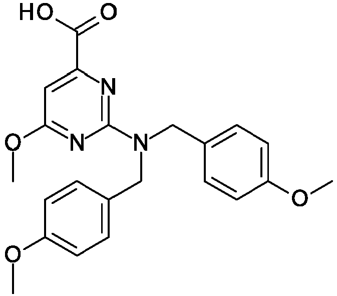

To a stirred solution of methyl 2-[bis[(4-methoxyphenyl)methyl]amino]-6-methoxy-pyrimidine-4-carboxylate (453 mg, 1.05 mmol) in tetrahydrofuran (5 mL) and water (1 mL) was added lithium hydroxide monohydrate (109.98 mg, 2.62 mmol). The reaction mixture was stirred at room temperature for 1.5 hours, poured into 1 N HCl and extracted twice with ethyl acetate. The organic layers were dried over sodium sulfate, filtered and concentrated in vacuo to provide the title compound as a light yellow gum (435 mg, 99 % yield). MS (ESI) m/z= 410.2 [M+H]+

### Step 3: 2-[bis[(4-methoxyphenyl)methyl]amino]-N,6-dimethoxy-N-methyl-pyrimidine-4-carboxamide

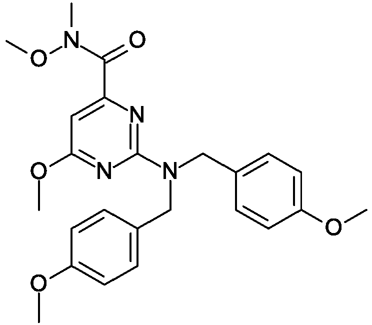

A solution of 2-[bis[(4-methoxyphenyl)methyl]amino]-6-methoxy-pyrimidine-4-carboxylic acid (433 mg, 1.04 mmol), n,o-dimethylhydroxylamine hydrochloride (123.79 mg, 1.24 mmol) and 4-methylmorpholine (127.06 mg, 138.11 uL, 1.24 mmol) in dichloromethane (8 mL) was cooled to 0 °C. 1-(3-dimethylaminopropyl)-3-ethylcarbodiimide hydrochloride (238.41 mg, 1.24 mmol) was added. The reaction mixture was stirred at 0 °C for 2 hours, diluted with dichloromethane and successively washed with 1 N HCl, sat. NaHCO3 and water. The organic layer was dried over sodium sulfate, filtered and concentrated in vacuo to provide the title compound as a light yellow gum (433 mg, 92 % yield). MS (ESI) m/z= 453.3 [M+H]+

### Step 4: 1-[2-[bis[(4-methoxyphenyl)methyl]amino]-6-methoxy-pyrimidin-4-yl]ethanone

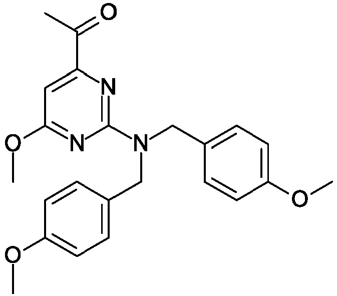

A solution of 2-[bis[(4-methoxyphenyl)methyl]amino]-N,6-dimethoxy-N-methyl-pyrimidine-4-carboxamide (431 mg, 0.952 mmol) in tetrahydrofuran (8 mL) was cooled to 0 °C. A 3 M methylmagnesium bromide solution in diethyl ether (407.66 mg, 476.24 uL, 1.43 mmol) was added dropwise. The reaction mixture was stirred at 0 °C for 30 min, carefully quenched with a saturation solution of ammonium chloride and extracted twice with ethyl acetate. The organic layers were dried over sodium sulfate, filtered and concentrated in vacuo to provide the title compound as a light yellow gum (381 mg, 98 % yield). MS (ESI) m/z= 408.3 [M+H]+

### Step 5: 4-(1,1-difluoroethyl)-6-methoxy-N,N-bis[(4-methoxyphenyl)methyl]pyrimidin-2-amine

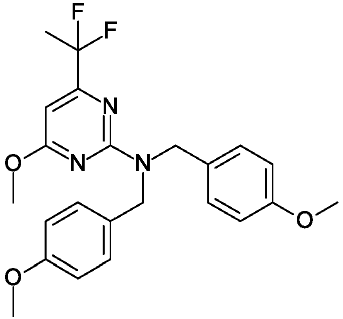

To a solution of 1-[2-[bis[(4-methoxyphenyl)methyl]amino]-6-methoxy-pyrimidin-4-yl]ethanone (298 mg, 0.731 mmol) in toluene (3 mL) was added Deoxo-fluor ^{®}, 2.7M (50 wt.%) solution in toluene (3.24 g, 2.7 mL). The reaction mixture was heated to 80 °C, stirred for 4.5 hours, then cooled to room temperature, poured into sat. NaHCO3 and extracted twice with ethyl acetate. The organic layers were dried over sodium sulfate, filtered and concentrated in vacuo. The residue was purified by flash chromatography over silica gel using a gradient ethylacetate/heptane 0-15% to provide the title compound as a light yellow viscous oil (166 mg, 50 % yield). MS (ESI) m/z= 430.3 [M+H]+

### Step 6: 4-(1,1-difluoroethyl)-6-methoxy-pyrimidin-2-amine

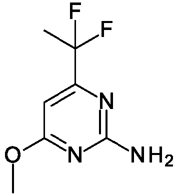

To a stirred solution of 4-(1,1-difluoroethyl)-6-methoxy-N,N-bis[(4-methoxyphenyl)methyl]pyrimidin-2-amine (80 mg, 0.175 mmol) in dichloromethane (100 uL) was added trifluoroacetic acid (1.2 g, 804. uL, 10.51 mmol). The reaction mixture was heated to 60 °C, stirred for 20 hours and concentrated in vacuo. The residue was poured into sat. NaHCO3 and extracted twice with ethyl acetate. The organic layers were dried over sodium sulfate, filtered and concentrated in vacuo. The residue was purified by flash chromatography over silica gel using a gradient ethylacetate/heptane 0-50% to provide the title compound as a white solid (34 mg, 98 % yield). MS (ESI) m/z= 190.0 [M+H]+

### Step 7: 5-bromo-4-(1,1-difluoroethyl)-6-methoxy-pyrimidin-2-amine

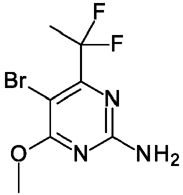

To a stirred solution of [4-(1,1-difluoroethyl)-6-methoxy-pyrimidin-2-yl]amine (33 mg, 0.166 mmol) in acetonitrile (2 mL) was added n-bromosuccinimide (29.8 mg, 0.166 mmol). The reaction mixture was stirred at room temperature for 30 min, diluted with ethyl acetate and successively washed with sat. NaHCO3 and brine. The organic layer was dried over sodium sulfate, filtered and concentrated in vacuo. The residue was purified by flash chromatography over silica gel using a gradient ethylacetate/heptane 0-30% to provide the title compound as a white solid (34 mg, 74 % yield). MS (ESI) m/z= 270.0 [M+H]+

### Intermediate B92: 5-bromo-4-(fluoromethoxy)-6-methoxy-pyrimidin-2-amine

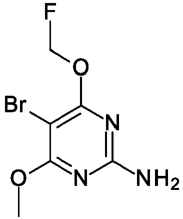

### Step 1: 4-(fluoromethoxy)-6-methoxy-pyrimidin-2-amine

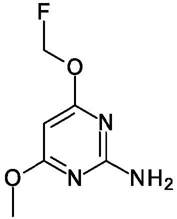

To a solution of 2-amino-6-methoxy-pyrimidin-4-ol (400 mg, 2.69 mmol, CAS: 59081-28-6) in N,N-dimethylformamide (24.48 mL) was added potassium carbonate (372.14 mg, 2.69 mmol) and then fluoro(iodo)methane (430.62 mg, 181.7 uL, 2.69 mmol). The reaction mixture was stirred at room temperature for 45 min in a sealed tube, then poured into water and extracted three times with ethyl acetate. The organic layers were dried over sodium sulfate, filtered and concentrated in vacuo. The residue was purified by flash chromatography over silica gel using a gradient ethylacetate/heptane 0-70% to provide the title compound as a yellow oil (92.9 mg, 17 % yield). MS (ESI) m/z= 174.1 [M+H]+

### Step 2: 5-bromo-4-(fluoromethoxy)-6-methoxy-pyrimidin-2-amine

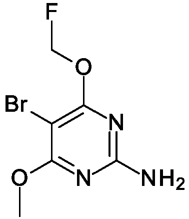

To 4-(fluoromethoxy)-6-methoxy-pyrimidin-2-amine (40 mg, 0.231 mmol) in acetonitrile (1.54 mL) was added n-bromosuccinimide (53.45 mg, 0.300 mmol). The reaction mixture was stirred at room temperature for 20 min, then diluted with ethyl acetate and washed once with water. The organic layer was dried over sodium sulfate, filtered and concentrated in vacuo. The residue was purified by flash chromatography over silica gel using a gradient ethylacetate/heptane 0-100% to provide the title compound as a white solid (27 mg, 46 % yield). MS (ESI) m/z= 252.1 [M+H]+

### Intermediate B93: 5-bromo-4-(difluoromethyl)-6-methoxy-pyrimidin-2-amine

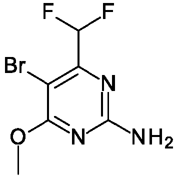

### Step 1: [2-[bis[(4-methoxyphenyl)methyl]amino]-6-methoxy-pyrimidin-4-yl]methanol

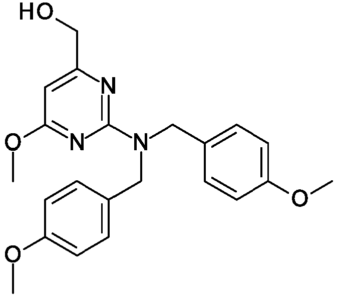

A solution of methyl 2-[bis[(4-methoxyphenyl)methyl]amino]-6-methoxy-pyrimidine-4-carboxylate (440 mg, 1.01 mmol, intermediate B91, step 1) in tetrahydrofuran (12 mL) was cooled to 0 °C. 1 M lithium aluminium hydride solution in THF (1.51 mL, 1.51 mmol) was added dropwise. The reaction mixture was stirred at 0 °C for 30 min, then carefully quenched with a saturated solution of ammonium chloride, poured into water and extracted twice with ethyl acetate. The organic layers were washed with brine, dried over sodium sulfate, filtered and concentrated in vacuo. The residue was purified by flash chromatography over silica gel using a gradient ethylacetate/heptane 0-40% to provide the title compound as a white solid (257 mg, 64 % yield). MS (ESI) m/z= 396.3 [M+H]+

### Step 2: 2-[bis[(4-methoxyphenyl)methyl]amino]-6-methoxy-pyrimidine-4-carbaldehyde

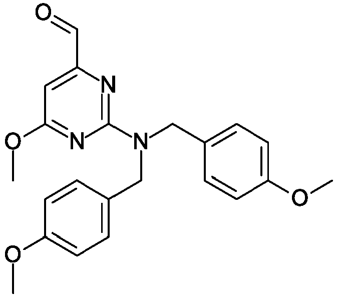

A solution of [2-[bis[(4-methoxyphenyl)methyl]amino]-6-methoxy-pyrimidin-4-yl]methanol (125 mg, 0.316 mmol) in dichloromethane (3 mL) was cooled to 0 °C. Dess-martin periodinane (160.88 mg, 0.379 mmol) was added. The reaction mixture was stirred at 0 °C for 30 min, at room temperature and for 16 hours, poured into sat. NaHCO3 and extracted twice with dichloromethane. The organic layers were dried over sodium sulfate, filtered and concentrated in vacuo. The residue was purified by flash chromatography over silica gel using a gradient ethylacetate/heptane 0-30% to provide the title compound as a yellow gum (100 mg, 79 % yield). MS (ESI) m/z= 394.3 [M+H]+

### Step 3: 4-(difluoromethyl)-6-methoxy-N,N-bis[(4-methoxyphenyl)methyl]pyrimidin-2-amine

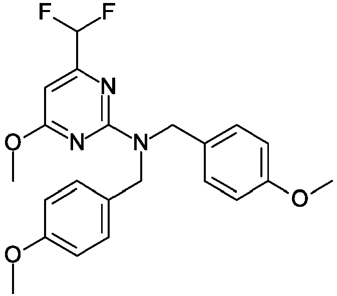

A solution of 4-(difluoromethyl)-6-methoxy-N,N-bis[(4-methoxyphenyl)methyl]pyrimidin-2-amine (98 mg, 0.244 mmol) in dichloromethane (1 mL) was cooled to 0 °C. Deoxo-fluor ^{®}, 2.7M (50 wt.%) solution in toluene (162.74 mg, 135.61 uL, 0.366 mmol) was added dropwise. The reaction mixture was stirred at 0 °C for 1 hour, poured into sat. NaHCO3 and extracted twice with dichloromethane. The organic layers were dried over sodium sulfate, filtered and concentrated in vacuo. The residue was purified by flash chromatography over silica gel using a gradient ethylacetate/heptane 0-20% to provide the title compound as a colorless gum (83 mg, 82 % yield). MS (ESI) m/z= 416.3 [M+H]+

### Step 4: 4-(difluoromethyl)-6-methoxy-pyrimidin-2-amine

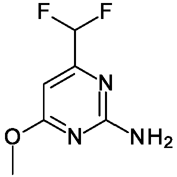

To a solution of 4-(difluoromethyl)-6-methoxy-N,N-bis[(4-methoxyphenyl)methyl]pyrimidin-2-amine (82 mg, 0.197 mmol) in dichloromethane (100 uL) was added trifluoroacetic acid (1.35 g, 906.31 uL, 11.84 mmol). The reaction mixture was heated to 60 °C and stirred for 20 hours. Trifluoroacetic acid (675.18 mg, 453.14 uL, 5.92 mmol) was added and the stirring was continued at 60 °C for 5 hours. The reaction mixture was concentrated in vacuo. The residue was diluted with ethyl acetate, poured into sat. NaHCO3 and extracted twice with ethyl acetate. The organic layers were dried over sodium sulfate, filtered and concentrated in vacuo. The residue was purified by flash chromatography over silica gel using a gradient ethylacetate/heptane 0-50% to provide the title compound as a white solid (36 mg, 100 % yield). MS (ESI) m/z= 176.1 [M+H]+

### Step 5: 5-bromo-4-(difluoromethyl)-6-methoxy-pyrimidin-2-amine

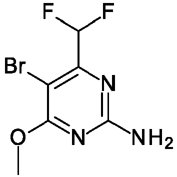

To a stirred solution of 4-(difluoromethyl)-6-methoxy-pyrimidin-2-amine (35 mg, 0.192 mmol) in acetonitrile (2 mL) was added n-bromosuccinimide (34.49 mg, 0.192 mmol). The reaction mixture was stirred at room temperature for 30 min, then diluted with ethyl acetate and successively washed with sat. NaHCO3 and brine. The organic layer was dried over sodium sulfate, filtered and concentrated in vacuo. The residue was purified by flash chromatography over silica gel using a gradient ethylacetate/heptane 0-30% to provide the title compound as a white solid (38 mg, 78 % yield). MS (ESI) m/z= 254.1 [M+H]+

### Intermediate B94: 2-(2-amino-5-bromo-6-methoxy-pyrimidin-4-yl)acetonitrile

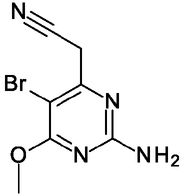

### Step 1: 4-(chloromethyl)-6-methoxy-N,N-bis[(4-methoxyphenyl)methyl]pyrimidin-2-amine

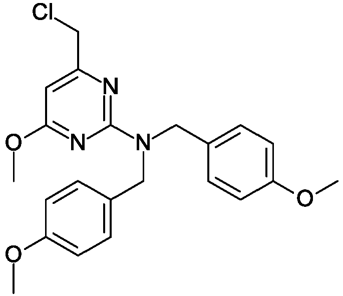

To a stirred solution of [2-[bis[(4-methoxyphenyl)methyl]amino]-6-methoxy-pyrimidin-4-yl]methanol (125 mg, 0.316 mmol, intermediate B93, step 1) in dichloromethane (1.5 mL) was added thionyl chloride (75.21 mg, 45.86 uL, 0.632 mmol). The reaction mixture was stirred at room temperature for 1 hour and concentrated in vacuo to provide the title compound as a light yellow solid (131 mg, 100 % yield). MS (ESI) m/z= 414.3 [M+H]+

### Step 2: 2-[2-[bis[(4-methoxyphenyl)methyl]amino]-6-methoxy-pyrimidin-4-yl]acetonitrile

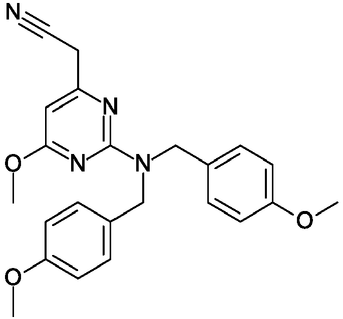

To a stirred solution of 4-(chloromethyl)-6-methoxy-N,N-bis[(4-methoxyphenyl)methyl]pyrimidin-2-amine (130 mg, 0.314 mmol) in dichloromethane (700 uL) was added tetrabutylammonium bromide (10.23 mg, 0.031 mmol), followed by a solution of sodium cyanide (19.04 mg, 0.377 mmol) in water (130 uL). The reaction mixture was stirred at room temperature for 1.5 hours. Sodium cyanide (19.04 mg, 0.377 mmol) was added and the stirring was continued at room temperature for 17 hours. Sodium cyanide (19.04 mg, 0.377 mmol) was added again and the stirring was continued at room temperature for 20 hours. Sodium cyanide (19.04 mg, 0.377 mmol) was added again and the stirring was continued at room temperature for 3 hours. The reaction mixture diluted with dichloromethane and washed twice with water. The organic layer was dried over sodium sulfate, filtered and concentrated in vacuo. The residue was purified by flash chromatography over silica gel using a gradient ethylacetate/heptane 0-30% to provide the title compound as a colorless gum (77 mg, 61 % yield). MS (ESI) m/z= 405.3 [M+H]+

### Step 3: 2-(2-amino-6-methoxy-pyrimidin-4-yl)acetonitrile

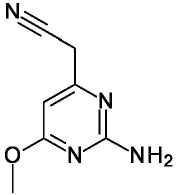

To a solution of 2-[2-[bis[(4-methoxyphenyl)methyl]amino]-6-methoxy-pyrimidin-4-yl]acetonitrile (71 mg, 0.176 mmol) in dichloromethane (100 uL) was added trifluoroacetic acid (1.2 g, 806.01 uL, 10.53 mmol). The reaction mixture was heated to 60 °C and stirred for 25 hours. Trifluoroacetic acid (675.18 mg, 453.14 uL) was added and the stirring was continued at 60 °C for 5 hours. The reaction mixture was concentrated in vacuo. The residue was diluted with ethyl acetate, poured into sat. NaHCO3 and extracted twice with ethyl acetate. The organic layers were dried over sodium sulfate, filtered and concentrated in vacuo. The residue was purified by flash chromatography over silica gel using a gradient ethylacetate/heptane 0-100% to provide the title compound as an orange solid (22 mg, 76 % yield). MS (ESI) m/z= 165.1 [M+H]+

### Step 4: 2-(2-amino-5-bromo-6-methoxy-pyrimidin-4-yl)acetonitrile

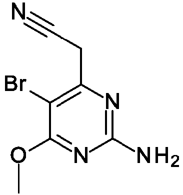

To a stirred solution of 2-(2-amino-6-methoxy-pyrimidin-4-yl)acetonitrile (21 mg, 0.128 mmol) in acetonitrile (1.5 mL) was added n-bromosuccinimide (23 mg, 0.128 mmol). The reaction mixture was stirred at room temperature for 20 min, then diluted with ethyl acetate and successively washed with sat. NaHCO3 and brine. The organic layer was dried over sodium sulfate, filtered and concentrated in vacuo. The residue was purified by flash chromatography over silica gel using a gradient ethylacetate/heptane 0-50% to provide the title compound as a white solid (29 mg, 93 % yield). MS (ESI) m/z= 243.1 [M+H]+

### Intermediate B95: 2-(2-amino-4-methoxy-pyrimidin-5-yl)oxyacetonitrile

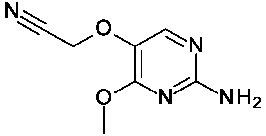

### Step 1: 2-[2-[bis[(4-methoxyphenyl)methyl]amino]-4-methoxy-pyrimidin-5-yl]oxyacetonitrile

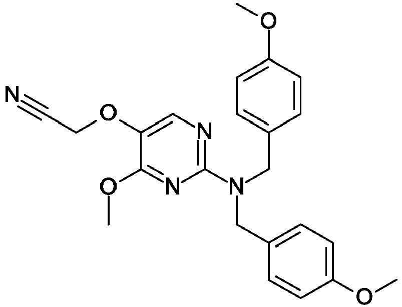

A mixture of 2-[bis[(4-methoxyphenyl)methyl]amino]-4-methoxy-pyrimidin-5-ol (500.0 mg, 1.31 mmol, intermediate B3, step 3) and bromoacetonitrile (314.47 mg, 2.62 mmol) in N,N-dimethylformamide (8 mL) was added potassium carbonate (543.52 mg, 3.93 mmol). The reaction mixture was stirred at 20 °C for 3 hours, diluted with water (30 mL) and extracted twice with ethyl acetate. The organic layers were washed with brine, dried over sodium sulfate, filtered and concentrated in vacuo. The residue was purified by flash chromatography over silica gel using a gradient ethylacetate/petroleum ether 0-50% to provide the title compound as an orange oil (372 mg, 67 % yield). MS (ESI) m/z= 421.2 [M+H]+

### Step 2: 2-(2-amino-4-methoxy-pyrimidin-5-yl)oxyacetonitrile

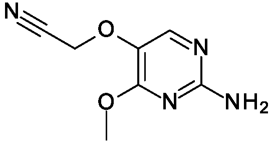

A mixture of 2-[2-[bis[(4-methoxyphenyl)methyl]amino]-4-methoxy-pyrimidin-5-yl]oxyacetonitrile (370.0 mg, 0.880 mmol) in trifluoroacetic acid (5.0 mL) was stirred at 60 °C for 6 hours, filtered and concentrated in vacuo. The residue was poured into ice/water. The pH was adjusted to 7 with NaHCO3. The mixture was diluted with water (30 mL) and extracted twice with ethyl acetate. The organic layers were washed with brine, dried over sodium sulfate, filtered and concentrated in vacuo. The residue was purified by flash chromatography over silica gel using a gradient ethylacetate/petroleum ether 0-50% to provide the title compound as an off-white powder (129 mg, 81 % yield). MS (ESI) m/z= 181.2 [M+H]+

### Intermediate B96: 5-(difluoromethoxy)-4-methoxy-6-methylsulfanyl-pyrimidin-2-amine

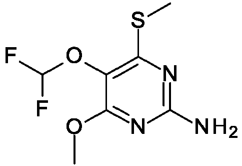

### Step 1: 4-chloro-6-methoxy-N,N-bis[(4-methoxyphenyl)methyl]pyrimidin-2-amine

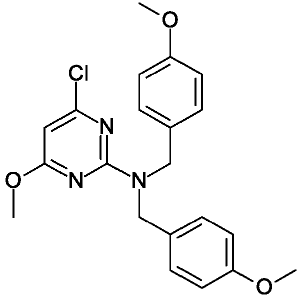

To a solution of 2-amino-4-chloro-6-methoxypyrimidine (500.0 mg, 3.13 mmol, CAS: 5734-64-5) in N,N-dimethylformamide (10 mL) was added sodium hydride (501.35 mg, 12.53 mmol) in portions at 0 °C. The mixture was stirred at 0 °C for 30 min under nitrogen. Then 4-methoxybenzyl chloride (977.82 mg, 6.27 mmol) was dropwise added to the mixture at 0 °C. The mixture was warmed to 15 °C, stirred for 2 hours, slowly poured ino a saturated solution of ammonium chloride, stirred for 10 min at 0 °C and extracted three times with ethyl acetate. The organic layers were washed with brine, dried over sodium sulfate, filtered and concentrated in vacuo. The residue was purified by flash chromatography over silica gel using a gradient ethylacetate/petroleum ether 0-10% to provide the title compound as a white solid (1100 mg, 88 % yield). MS (ESI) m/z= 400.2 [M+H]+

### Step 2: 5-bromo-4-chloro-6-methoxy-N,N-bis[(4-methoxyphenyl)methyl]pyrimidin-2-amine

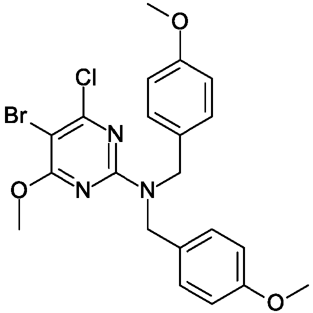

To a solution of 4-chloro-6-methoxy-N,N-bis[(4-methoxyphenyl)methyl]pyrimidin-2-amine (1000.0 mg, 2.5 mmol) in acetonitrile (20 mL) was added N-bromosuccinimide (534.11 mg, 3 mmol). The reaction mixture was stirred at 15 °C for 2 hours and concentrated in vacuo. The residue was purified by flash chromatography over silica gel using a gradient ethylacetate/petroleum ether 0-10% to provide the title compound as a white solid (1200 mg, 100 % yield). MS (ESI) m/z= 480.1 [M+H]+

### Step 3: 2-[bis[(4-methoxyphenyl)methyl]amino]-4-chloro-6-methoxy-pyrimidin-5-ol

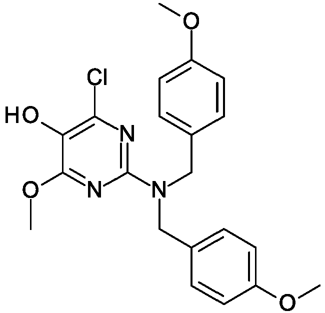

To a solution of 5-bromo-4-chloro-6-methoxy-N,N-bis[(4-methoxyphenyl)methyl]pyrimidin-2-amine (700.0 mg, 1.46 mmol) in tetrahydrofuran (25 mL) was added a 2.5 M solution of n-BuLi (0.76 mL, 1.9 mmol) at -70 °C. The mixture was stirred at -70 °C for 0.5 hour. Then trimethyl borate (0.25 mL, 2.19 mmol) was added at -70 °C and the mixture was stirred at -70 °C for 2 hours. Then the mixture was warmed to 0 °C and acetic acid (0.25 mL, 4.39 mmol) and hydrogen peroxide (0.45 mL, 4.39 mmol) were added at 0 °C. The mixture was warmed to 10 °C, stirred for 16 hours, quenched with a 0.1 M solution of sodium thiosulfate (60 mL) and stirred at 20 °C for 30 min. The mixture was extracted twice with ethyl acetate. The organic layers were washed with brine, dried over sodium sulfate, filtered and concentrated in vacuo. The residue was purified by flash chromatography over silica gel using a gradient ethylacetate/petroleum ether 0-20% to provide the title compound as a colorless oil (350 mg, 58 % yield). MS (ESI) m/z= 416.2 [M+H]+

### Step 4: 4-chloro-5-(difluoromethoxy)-6-methoxy-N,N-bis[(4-methoxyphenyl)methyl]pyrimidin-2-amine

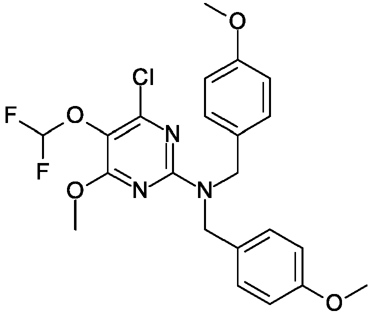

To a mixture of 2-[bis[(4-methoxyphenyl)methyl]amino]-4-chloro-6-methoxy-pyrimidin-5-ol (400.0 mg, 0.960 mmol) in acetonitrile (20 mL) was added a solution of potassium hydroxide (539.69 mg, 9.62 mmol) in water (4.8 mL) at 30 °C, followed by diethyl (bromodifluoromethyl)phosphonate (1540.92 mg, 5.77 mmol). The reaction mixture was stirred at 30 °C for 16 hours, poured into water and extracted with ethyl acetate. The organic layer was washed with brine, dried over sodium sulfate, filtered and concentrated in vacuo. The residue was purified by preparative HPLC to provide the title compound as a white solid (280 mg, 62 % yield). MS (ESI) m/z= 466.2 [M+H]+

### Step 5: 5-(difluoromethoxy)-4-methoxy-N,N-bis[(4-methoxyphenyl)methyl]-6-methylsulfanyl-pyrimidin-2-amine

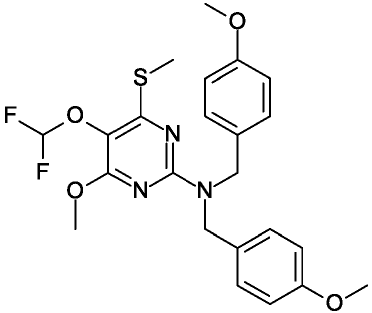

To a solution of 4-chloro-5-(difluoromethoxy)-6-methoxy-N,N-bis[(4-methoxyphenyl)methyl]pyrimidin-2-amine (280.0 mg, 0.600 mmol) in N-methyl-2-pyrrolidinone (6 mL) was added sodium methanethiolate (126.19 mg, 1.8 mmol). The reaction mixture was stirred at 15 °C for 2 hours, poured into water and extracted three times with ethyl acetate. The organic layers were washed with brine, dried over sodium sulfate, filtered and concentrated in vacuo. The residue was purified by flash chromatography over silica gel using a gradient ethylacetate/petroleum ether 0-20% to provide the title compound as a white solid (230 mg, 80 % yield). MS (ESI) m/z= 478.2 [M+H]+

### Step 6: 5-(difluoromethoxy)-4-methoxy-6-methylsulfanyl-pyrimidin-2-amine

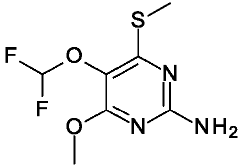

A mixture of 5-(difluoromethoxy)-4-methoxy-N,N-bis[(4-methoxyphenyl)methyl]-6-methylsulfanyl-pyrimidin-2-amine (180.0 mg, 0.380 mmol) in trifluoroacetic acid (4.0 mL) was stirred at 60 °C for 3 hours and concentrated in vacuo. The residue was poured into sat. NaHCO3 and extracted three times with ethyl acetate. The organic layers were washed with brine, dried over sodium sulfate, filtered and concentrated in vacuo. The residue was purified by preparative HPLC to provide the title compound as a white solid (70 mg, 78 % yield). MS (ESI) m/z= 238.1 [M+H]+

### Intermediate B97: 4,6-dimethoxy-5-(2-methylsulfonylethyl)pyrimidin-2-amine

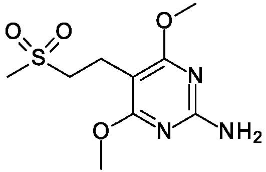

### Step 1: diethyl 2-(2-methylsulfonylethyl)propanedioate

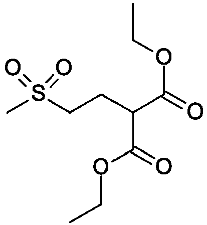

To ethanol (30 mL) was added sodium hydride (124.87 mg, 3.12 mmol) and the mixture was stirred at 20 °C for 0.5 hour. Then methyl vinyl sulfone (3.31 g, 31.22 mmol) and diethyl malonate (4.74 mL, 31.22 mmol) was added. The mixture was stirred at 30 °C for 15.5 hours, quenched with a saturated solution of ammonium chloride and extracted twice with ethyl acetate. The organic layers were washed twice with brine, dried over sodium sulfate, filtered and concentrated in vacuo. The residue was purified by flash chromatography over silica gel using a gradient ethylacetate/petroleum ether 40-50% to provide the title compound as a colorless oil (2.3 g, 28 % yield). 1H NMR (400 MHz, DMSO-d6) δ = 4.15 (dq, J = 3.2, 7.2 Hz, 4H), 3.69 (t, J = 7.2 Hz, 1H), 3.19 - 3.09 (m, 2H), 3.00 (s, 3H), 2.22 - 2.11 (m, 2H), 1.19 (t, J = 7.2 Hz, 6H)

### Step 2: 2-amino-5-(2-methylsulfonylethyl)pyrimidine-4,6-diol

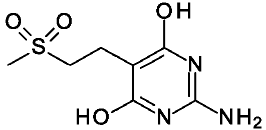

Sodium (362.74 mg, 15.77 mmol) was dissolved in absolute ethanol (100 mL) under argon while being intensively stirred with a mechanical stirrer. Guanidine hydrochloride (591.88 mg, 6.2 mmol) was added under intensive stirring, followed by diethyl 2-(2-methylsulfonylethyl)propanedioate (1.5 g, 5.63 mmol). The reaction mixture was further intensively stirred for 16 hours. Absolute ethanol (100 mL) was added and the reaction mixture was refluxed for 1 hour. Afterward, ethanol was evaporated and water (100 mL) was added to the reaction mixture. After stirring, the product (in the form of a sodium salt) was almost dissolved. The obtained mixture was subsequently neutralized by dropwise addition of acetic acid (50 mL), resulting in immediate and quantitative precipitation of the desired product in the form of a fine solid. This mixture was subsequently heated under reflux for 10 min and then cooled to laboratory temperature. This heating and cooling was repeated twice to get a well-filterable solid product. The solid product was filtered off, washed twice with water (100 mL), twice with ethanol (100 mL), and twice with acetone (100 mL). The product was suspended in toluene and concentrated under high vacuum at 60 °C (2 times) to provide the title compound as a colorless oil (400 mg, 30 % yield). MS (ESI) m/z= 234.0 [M+H]+

### Step 3: 4,6-dichloro-5-(2-methylsulfonylethyl)pyrimidin-2-amine

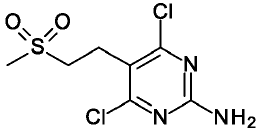

To a stirred solution of 2-amino-5-(2-methylsulfonylethyl)pyrimidine-4,6-diol (300.0 mg, 1.29 mmol) in phosphorus oxychloride (30 mL) was stirred at 100 °C for 3 hours. The reaction mixture was concentrated in vacuo. The residue was diluted with ice cold water (100 mL), basified with sat. NaHCO3 till pH 8 and extracted twice with ethyl acetate. The organic layers were washed three times with brine, dried over sodium sulfate, filtered and concentrated in vacuo to provide the title compound as a dark brown powder (347 mg, 100 % yield). MS (ESI): m/z= 270.0 [M+H]+

### Step 4: 4,6-dimethoxy-5-(2-methylsulfonylethyl)pyrimidin-2-amine

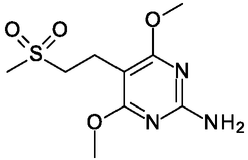

A solution of sodium hydride (666.32 mg, 16.66 mmol) in methanol (40 mL) was cooled to 0 °C under nitrogen. The mixture was stirred for 15 min at 0 °C. Then, 4,6-dichloro-5-(2-methylsulfonylethyl)pyrimidin-2-amine (450.0 mg, 1.67 mmol) was added to the solution. The reaction mixture was stirred at 80 °C for 16 hours, poured into water and extracted three times with ethyl acetate. The organic layers were washed three times with brine, dried over sodium sulfate, filtered and concentrated in vacuo. The residue was purified by flash chromatography over silica gel using a gradient ethylacetate/petroleum ether 30-50% to provide the title compound as a white solid (200 mg, 44 % yield). MS (ESI) m/z= 262.2 [M+H]+

### Examples

### Example 1: 6-chloro-N-[5-(2-fluoroethoxy)-4-methoxy-pyrimidin-2-yl]-1H-indole-3-sulfonamide

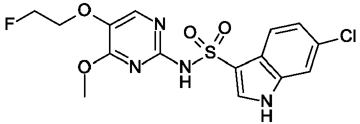

To a stirred solution of [5-(2-fluoroethoxy)-4-methoxy-pyrimidin-2-yl]amine (25 mg, 0.134 mmol, intermediate B3) and N-ethyldiisopropylamine (35.23 mg, 46.54 uL, 0.267 mmol) in dichloromethane (500 uL) was added 6-chloro-1H-indole-3-sulfonyl chloride (50.11 mg, 0.200 mmol, Intermediate A1). The reaction mixture was stirred at room temperature for 10 min. The resulting yellow suspension was poured into water and extracted twice with EtOAc. The organic layers were dried over sodium sulfate, filtered and concentrated in vacuo. The residue was purified by flash chromatography over silica gel using a gradient ethylacetate/heptane 0-100% to provide the title compound as a colorless gum (9 mg, 16 % yield). MS (ESI): m/z= 401.1 [M+H]+

The following examples 2-23 were prepared in analogy to example 1 by coupling the indicated sulfonylchloride intermediates A and amine intermediates B.

| Ex. | Structure | Name | Int. A | Int. B | MS (ESI): m/z, [M+H] ⁺ |
|---|---|---|---|---|---|
| 2 | 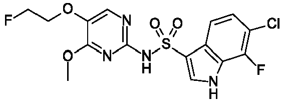 | 6-chloro-7-fluoro-N-[5-(2-fluoroethoxy)-4-methoxy-pyrimidin-2-yl]-1H-indole-3-sulfonamide | A6 | B3 | 419.2 |
| 3 | 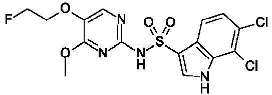 | 6,7-dichloro-N-[5-(2-fluoroethoxy)-4-methoxy-pyrimidin-2-yl]-1H-indole-3-sulfonamide | A8 | B3 | 435.2 |
| 4 | 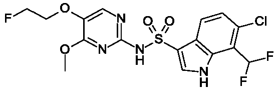 | 6-chloro-7-(difluoromethyl)-N-[5-(2-fluoroethoxy)-4-methoxy-pyrimidin-2-yl]-1H-indole-3-sulfonamide | A7 | B3 | 451.2 |
| 5 | 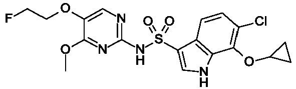 | 6-chloro-7-(cyclopropoxy)-N-[5-(2-fluoroethoxy)-4-methoxy-pyrimidin-2-yl]-1H-indole-3-sulfonamide | A9 | B3 | 457.3 |
| 6 | 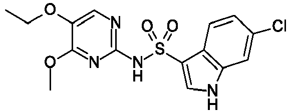 | 6-chloro-N-(5-ethoxy-4-methoxy-pyrimidin-2-yl)-1H-indole-3-sulfonamide | A1 | B4 | 383.2 |
| 7 | 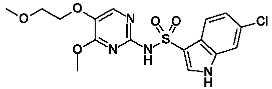 | 6-chloro-N-[4-methoxy-5-(2-methoxyethoxy)pyrimidin-2-yl]-1H-indole-3-sulfonamide | A1 | B5 | 413.3 |
| 8 | 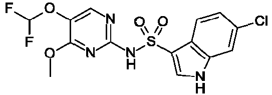 | 6-chloro-N-[5-(difluoromethoxy)-4-methoxy-pyrimidin-2-yl]-1H-indole-3-sulfonamide | A1 | B6 | 405.2 |
| 9 | 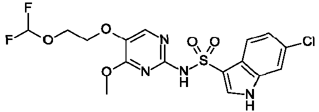 | 6-chloro-N-[5-[2-(difluoromethoxy)ethoxy]-4-methoxy-pyrimidin-2-yl]-1H-indole-3-sulfonamide | A1 | B7 | 449.2 |
| 10 | 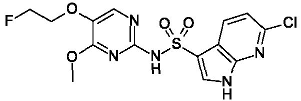 | 6-chloro-N-[5-(2-fluoroethoxy)-4-methoxy-pyrimidin-2-yl]-1H-pyrrolo[2,3-b]pyridine-3-sulfonamide | A5 | B3 | 402.2 |
| 11 | 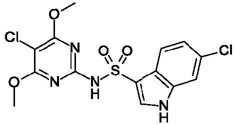 | 6-chloro-N-(5-chloro-4,6-dimethoxy-pyrimidin-2-yl)-1H-indole-3-sulfonamide | A1 | B12 | 403.2 |
| 12 | 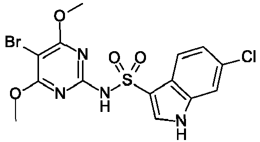 | N-(5-bromo-4,6-dimethoxy-pyrimidin-2-yl)-6-chloro-1H-indole-3-sulfonamide | A1 | B13 | 449.1 |
| 13 | 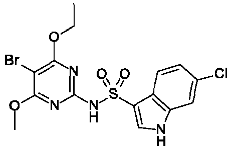 | N-(5-bromo-4-ethoxy-6-methoxy-pyrimidin-2-yl)-6-chloro-1H-indole-3-sulfonamide | A1 | B14 | 461.2 |
| 14 | 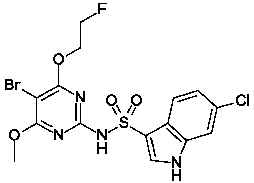 | N-[5-bromo-4-(2-fluoroethoxy)-6-methoxy-pyrimidin-2-yl]-6-chloro-1H-indole-3-sulfonamide | A1 | B15 | 479.2 |
| 15 | 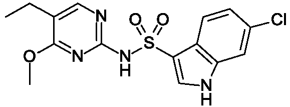 | 6-chloro-N-(5-ethyl-4-methoxy-pyrimidin-2-yl)-1H-indole-3-sulfonamide | A1 | B9 | 367.1 |
| 16 | 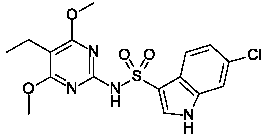 | 6-chloro-N-(5-ethyl-4,6-dimethoxy-pyrimidin-2-yl)-1H-indole-3-sulfonamide | A1 | B18 | 397.2 |
| 17 | 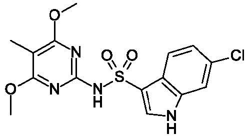 | 6-chloro-N-(4,6-dimethoxy-5-methyl-pyrimidin-2-yl)-1H-indole-3-sulfonamide | A1 | B19 | 383.1 |
| 18 | 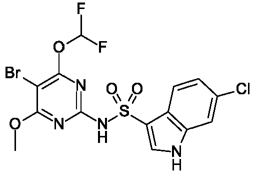 | N-[5-bromo-4-(difluoromethoxy)-6-methoxy-pyrimidin-2-yl]-6-chloro-1H-indole-3-sulfonamide | A1 | B16 | 485.1 |
| 19 | 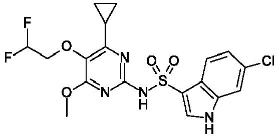 | 6-chloro-N-[4-cyclopropyl-5-(2,2-difluoroethoxy)-6-methoxy-pyrimidin-2-yl]-1H-indole-3-sulfonamide | A1 | B21 | 459.2 |
| 20 | 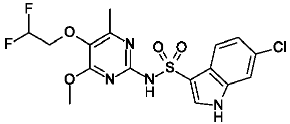 | 6-chloro-N-[5-(2,2-difluoroethoxy)-4-methoxy-6-methyl-pyrimidin-2-yl]-1H-indole-3-sulfonamide | A1 | B20 | 433.2 |
| 21 | 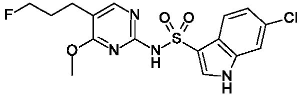 | 6-chloro-N-[5-(3-fluoropropyl)-4-methoxy-pyrimidin-2-yl]-1H-indole-3-sulfonamide | A1 | B17 | 399.1 |
| 22 | 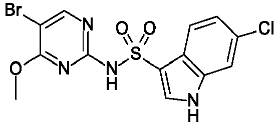 | N-(5-bromo-4-methoxy-pyrimidin-2-yl)-6-chloro-1H-indole-3-sulfonamide | A1 | B1 | 419.1 |
| 23 | 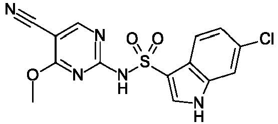 | 6-chloro-N-(5-cyano-4-methoxy-pyrimidin-2-yl)-1H-indole-3-sulfonamide | A1 | B2 | 364.1 |

### Example 24: 6-chloro-N-[5-(2-fluoroethoxy)-4-methoxy-pyrimidin-2-yl]benzothiophene-3-sulfonamide

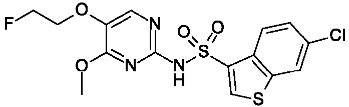

To 6-chlorobenzothiophene-3-sulfonyl chloride (85.64 mg, 0.321 mmol, intermediate A3) was added [5-(2-fluoroethoxy)-4-methoxy-pyrimidin-2-yl]amine (30 mg, 0.160 mmol, intermediate B3), followed by pyridine, extra dry (1 mL). The reaction mixture was stirred room temperature for 2 hours. The solvent was evaporated. The residue was purified by flash chromatography over silica gel using a gradient ethylacetate/heptane 0-100% to provide the title compound as a white powder (28 mg, 39 % yield). MS (ESI): m/z= 418.0 [M+H]+

The following examples 25-27 were prepared in analogy to example 24 by coupling the indicated sulfonylchloride intermediates A and amine intermediates B.

| Ex. | Structure | Name | Int. A | Int. B | MS (ESI): m/z, [M+H] ⁺ |
|---|---|---|---|---|---|
| 25 | 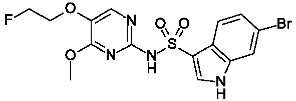 | 6-bromo-N-[5-(2-fluoroethoxy)-4-methoxy-pyrimidin-2-yl]-1H-indole-3-sulfonamide | A2 | B3 | 447.0 |
| 26 | 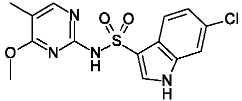 | 6-chloro-N-(4-methoxy-5-methyl-pyrimidin-2-yl)-1H-indole-3-sulfonamide | A1 | B10 | 353.1 |
| 27 | 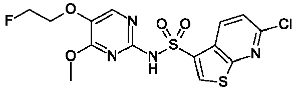 | 6-chloro-N-[5-(2-fluoroethoxy)-4-methoxy-pyrimidin-2-yl]thieno[2,3-b]pyridine-3-sulfonamide | A10 | B3 | 419.2 |

### Example 28: 6-chloro-N-(5-cyclopropyl-4-methoxy-pyrimidin-2-yl)-1H-indole-3-sulfonamide

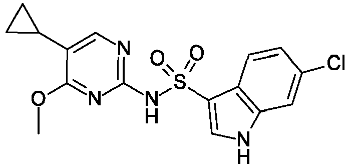

6-chloro-1H-indole-3-sulfonyl chloride (44.97 mg, 0.180 mmol, intermediate A1), (5-cyclopropyl-4-methoxy-pyrimidin-2-yl)amine (15 mg, 0.090 mmol, intermediate B11) and 4-dimethylaminopyridine (1.1 mg, 0.009 mmol) were introduced in a flask under argon. Pyridine, extra dry (0.5 mL) was added and the reaction mixture was stirred at 20°C for 30 min and at 100°C for 1 hour. The reaction mixture was poured in water and extracted twice with EtOAc. The organic layers were dried over sodium sulfate, filtered and concentrated in vacuo. The residue was purified by flash chromatography over silica gel using a gradient ethylacetate/heptane 0-100% to provide the title compound as a white powder (5 mg, 13 % yield). MS (ESI): m/z= 379.1 [M+H]+

### Example 29: 6-chloro-N-(4-methoxy-5-prop-1-ynyl-pyrimidin-2-yl)-1H-indole-3-sulfonamide

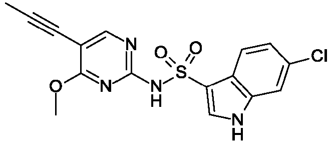

The title compound was prepared in analogy to Example 28 from intermediate A1 and intermediate B8 as an off-white solid. MS (ESI): m/z= 377.1 [M+H]+

### Example 30: 6-bromo-N-(5-bromo-4,6-dimethoxy-pyrimidin-2-yl)-1H-pyrrolo[2,3-b]pyridine-3-sulfonamide

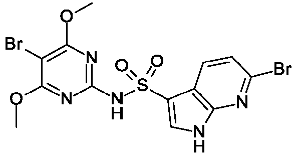

### Step 1: 6-bromo-N-(4,6-dimethoxypyrimidin-2-yl)-1H-pyrrolo[2,3-b]pyridine-3-sulfonamide

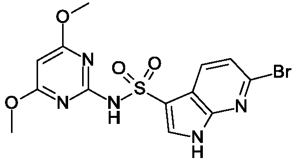

The title compound was prepared in analogy to example 1 from intermediate A4 and (4,6-dimethoxypyrimidin-2-yl)amine (CAS: 36315-01-2) as an off-white solid. MS (ESI): m/z= 416.1 [M+H]+

### Step 2: 6-bromo-N-(5-bromo-4,6-dimethoxy-pvrimidin-2-yl)-1H-pyrrolo[2,3-b]pyridine-3-sulfonamide

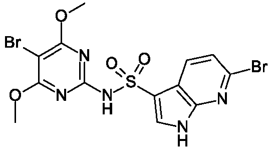

The title compound was prepared in analogy to intermediate B12 from 6-bromo-N-(4,6-dimethoxypyrimidin-2-yl)-1H-pyrrolo[2,3-b]pyridine-3-sulfonamide using N-bromosuccinimide as brominating agent. White solid. MS (ESI): m/z=492.0 [M+H]+

### Example 31: N-(5-bromo-4,6-dimethoxy-pyrimidin-2-yl)-6-chloro-1H-pyrrolo[2,3-b]pyridine-3-sulfonamide

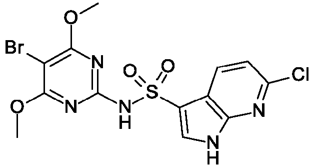

### Step 1: 6-chloro-N-(4,6-dimethoxypyrimidin-2-yl)-1H-pyrrolo[2,3-b]pyridine-3-sulfonamide

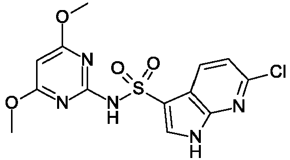

The title compound was prepared in analogy to example 1 from intermediate A5 and (4,6-dimethoxypyrimidin-2-yl)amine (CAS: 36315-01-2) as an off-white solid. MS (ESI): m/z= 370.1 [M+H]+

### Step 2: N-(5-bromo-4,6-dimethoxy-pyrimidin-2-yl)-6-chloro-1H-pyrrolo[2,3-b]pyridine-3-sulfonamide

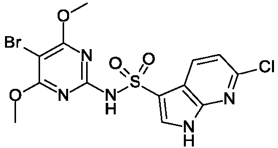

The title compound was prepared in analogy to intermediate B12 from 6-chloro-N-(4,6-dimethoxypyrimidin-2-yl)-1H-pyrrolo[2,3-b]pyridine-3-sulfonamide using N-bromosuccinimide as brominating agent. White solid. MS (ESI): m/z=450.0 [M+H]+

### Example 32: 6-chloro-N-(5-cyano-4,6-dimethoxy-pyrimidin-2-yl)-1H-indole-3-sulfonamide

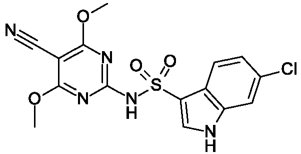

A microwave tube was charged with a solution of N-(5-bromo-4,6-dimethoxy-pyrimidin-2-yl)-6-chloro-1H-indole-3-sulfonamide (20 mg, 0.045 mmol, example 12) in N,N-dimethylacetamide (0.209 mL). Then tetrakis(triphenylphosphine)palladium(0) (10.32 mg, 0.009 mmol) and zinc cyanide (5.25 mg, 0.045 mmol) were added and argon was bubbled through the mixture for 1 min. The tube was sealed and reacted in a microwave oven for 15 min at 160 °C. The reaction mixture was directly purified with flash column chromatography over silica gel using a gradient ethylacetate/heptane 0-50% to provide the title compound as a white solid (4 mg, 23 % yield). MS (ESI): m/z= 394.1 [M+H]+

The following examples 33-134 were prepared in analogy to example 1 by coupling the indicated sulfonylchloride intermediates A and amine intermediates B.

| Ex. | Structure | Name | Int. A | Int. B | MS (ESI): m/z, [M+H] ⁺ |
|---|---|---|---|---|---|
| 33 | 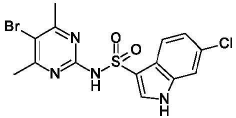 | N-(5-bromo-4,6-dimethyl-pyrimidin-2-yl)-6-chloro-1H-indole-3-sulfonamide | A1 | B22 | 417.0 |
| 34 | 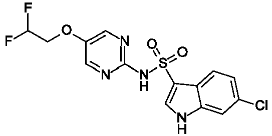 | 6-chloro-N-[5-(2,2-difluoroethoxy)pyrimidin-2-yl]-1H-indole-3-sulfonamide | A1 | B24 | 389.2 |
| 35 | 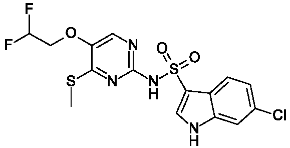 | 6-chloro-N-[5-(2,2-difluoroethoxy)-4-methylsulfanyl-pyrimidin-2-yl]-1H-indole-3-sulfonamide | A1 | B25 | 435.0 |
| 36 | 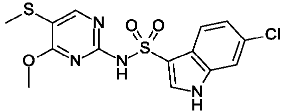 | 6-chloro-N-(4-methoxy-5-methylsulfanyl-pyrimidin-2-yl)-1H-indole-3-sulfonamide | A1 | B26 | 385.2 |
| 37 | 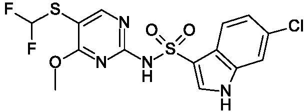 | 6-chloro-N-[5-(difluoromethylthio)-4-methoxy-pyrimidin-2-yl]-1H-indole-3-sulfonamide | A1 | B27 | 421.1 |
| 38 | 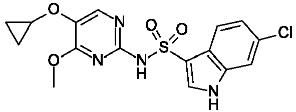 | 6-chloro-N-[5-(cyclopropoxy)-4-methoxy-pyrimidin-2-yl]-1H-indole-3-sulfonamide | A1 | B28 | 395.2 |
| 39 | 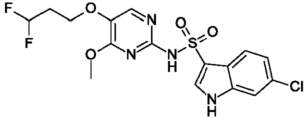 | 6-chloro-N-[5-(3,3-difluoropropoxy)-4-methoxy-pyrimidin-2-yl]-1H-indole-3-sulfonamide | A1 | B32 | 433.1 |
| 40 | 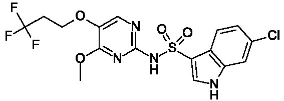 | 6-chloro-N-[4-methoxy-5-(3,3,3-trifluoropropoxy)pyrimidin-2-yl]-1H-indole-3-sulfonamide | A1 | B33 | 451.2 |
| 41 | 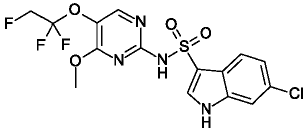 | 6-chloro-N-[4-methoxy-5-(1,1,2-trifluoroethoxy)pyrimidin-2-yl]-1H-indole-3-sulfonamide | A1 | B34 | 436.7 |
| 42 | 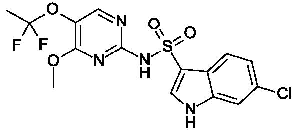 | 6-chloro-N-[5-(1,1-difluoroethoxy)-4-methoxy-pyrimidin-2-yl]-1H-indole-3-sulfonamide | A1 | B35 | 418.8 |
| 43 | 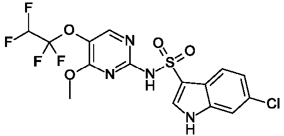 | 6-chloro-N-[4-methoxy-5-(1,1,2,2-tetrafluoroethoxy)pyrimidin -2-yl]-1H-indole-3-sulfonamide | A1 | B36 | 454.8 |
| 44 | 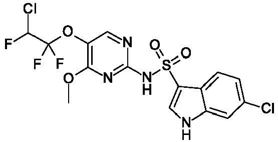 | 6-chloro-N-[5-(2-chloro-1,1,2-trifluoro-ethoxy)-4-methoxy-pyrimidin-2-yl]-1H-indole-3-sulfonamide | A1 | B37 | 470.8 |
| 45 | 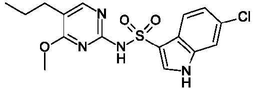 | 6-chloro-N-(4-methoxy-5-propyl-pyrimidin-2-yl)-1H-indole-3-sulfonamide | A1 | B38 | 381.0 |
| 46 | 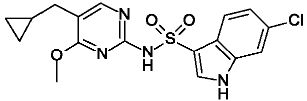 | 6-chloro-N-[5-(cyclopropylmethyl)-4-methoxy-pyrimidin-2-yl]-1H-indole-3-sulfonamide | A1 | B39 | 393.1 |
| 47 | 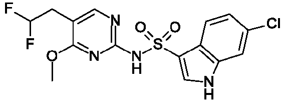 | 6-chloro-N-[5-(2,2-difluoroethyl)-4-methoxy-pyrimidin-2-yl]-1H-indole-3-sulfonamide | A1 | B40 | 403.2 |
| 48 | 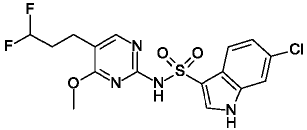 | 6-chloro-N-[5-(3,3-difluoropropyl)-4-methoxy-pyrimidin-2-yl]-1H-indole-3-sulfonamide | A1 | B42 | 417.2 |
| 49 | 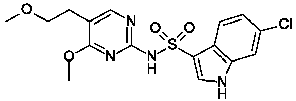 | 6-chloro-N-[4-methoxy-5-(2-methoxyethyl)pyrimidin-2-yl]-1H-indole-3-sulfonamide | A1 | B49 | 397.2 |
| 50 | 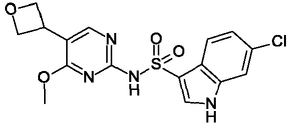 | 6-chloro-N-[4-methoxy-5-(oxetan-3-yl)pyrimidin-2-yl]-1H-indole-3-sulfonamide | A1 | B51 | 395.0 |
| 51 | 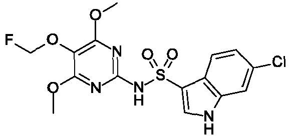 | 6-chloro-N-[5-(fluoromethoxy)-4,6-dimethoxy-pyrimidin-2-yl]-1H-indole-3-sulfonamide | A1 | B54 | 417.2 |
| 52 | 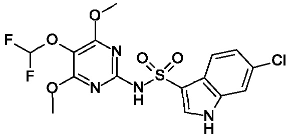 | 6-chloro-N-[5-(difluoromethoxy)-4,6-dimethoxy-pyrimidin-2-yl]-1H-indole-3-sulfonamide | A1 | B55 | 435.2 |
| 53 | 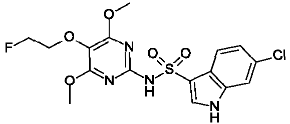 | 6-chloro-N-[5-(2-fluoroethoxy)-4,6-dimethoxy-pyrimidin-2-yl]-1H-indole-3-sulfonamide | A1 | B56 | 431.1 |
| 54 | 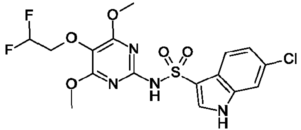 | 6-chloro-N-[5-(2,2-difluoroethoxy)-4,6-dimethoxy-pyrimidin-2-yl]-1H-indole-3-sulfonamide | A1 | B57 | 447.1 |
| 55 | 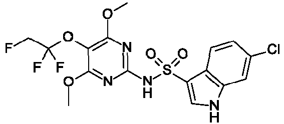 | 6-chloro-N-[4,6-dimethoxy-5-(1,1,2-trifluoroethoxy)pyrimidin-2-yl]-1H-indole-3-sulfonamide | A1 | B58 | 465.3 |
| 56 | 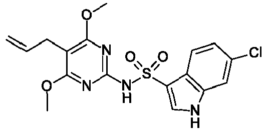 | N-(5-allyl-4,6-dimethoxy-pyrimidin-2-yl)-6-chloro-1H-indole-3-sulfonamide | A1 | B59 | 408.9 |
| 57 | 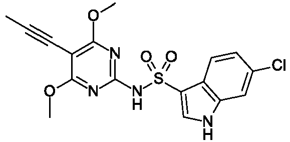 | 6-chloro-N-(4,6-dimethoxy-5-prop-1-ynyl-pyrimidin-2-yl)-1H-indole-3-sulfonamide | A1 | B60 | 407.2 |
| 58 | 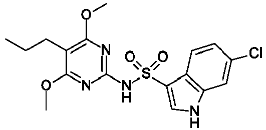 | 6-chloro-N-(4,6-dimethoxy-5-propyl-pyrimidin-2-yl)-1H-indole-3-sulfonamide | A1 | B61 | 410.8 |
| 59 | 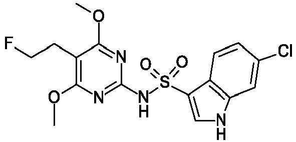 | 6-chloro-N-[5-(2-fluoroethyl)-4,6-dimethoxy-pyrimidin-2-yl]-1H-indole-3-sulfonamide | A1 | B62 | 415.1 |
| 60 | 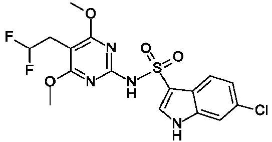 | 6-chloro-N-[5-(2,2-difluoroethyl)-4,6-dimethoxy-pyrimidin-2-yl]-1H-indole-3-sulfonamide | A1 | B63 | 433.2 |
| 61 | 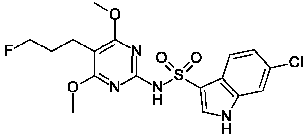 | 6-chloro-N-[5-(3-fluoropropyl)-4,6-dimethoxy-pyrimidin-2-yl]-1H-indole-3-sulfonamide | A1 | B64 | 428.9 |
| 62 | 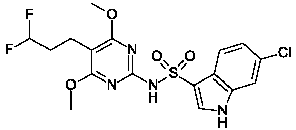 | 6-chloro-N-[5-(3,3-difluoropropyl)-4,6-dimethoxy-pyrimidin-2-yl]-1H-indole-3-sulfonamide | A1 | B65 | 447.2 |
| 63 | 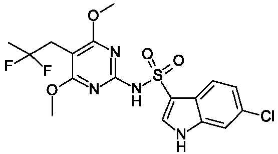 | 6-chloro-N-[5-(2,2-difluoropropyl)-4,6-dimethoxy-pyrimidin-2-yl]-1H-indole-3-sulfonamide | A1 | B66 | 447.0 |
| 64 | 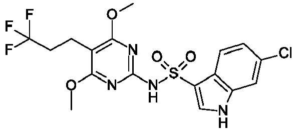 | 6-chloro-N-[4,6-dimethoxy-5-(3,3,3-trifluoropropyl)pyrimidin-2-yl]-1H-indole-3-sulfonamide | A1 | B67 | 465.1 |
| 65 | 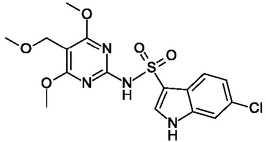 | 6-chloro-N-[4,6-dimethoxy-5-(methoxymethyl)pyrimidin-2-yl]-1H-indole-3-sulfonamide | A1 | B68 | 413.2 |
| 66 | 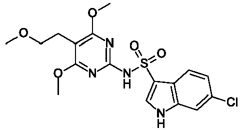 | 6-chloro-N-[4,6-dimethoxy-5-(2-methoxyethyl)pyrimidin-2-yl]-1H-indole-3-sulfonamide | A1 | B69 | 427.1 |
| 67 | 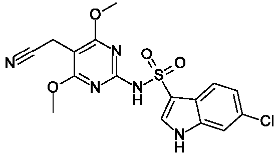 | 6-chloro-N-[5-(cyanomethyl)-4,6-dimethoxy-pyrimidin-2-yl]-1H-indole-3-sulfonamide | A1 | B70 | 408.1 |
| 68 | 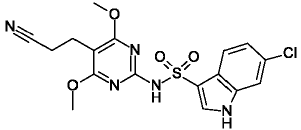 | 6-chloro-N-[5-(2-cyanoethyl)-4,6-dimethoxy-pyrimidin-2-yl]-1H-indole-3-sulfonamide | A1 | B71 | 422.2 |
| 69 | 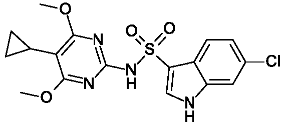 | 6-chloro-N-(5-cyclopropyl-4,6-dimethoxy-pyrimidin-2-yl)-1H-indole-3-sulfonamide | A1 | B72 | 409.2 |
| 70 | 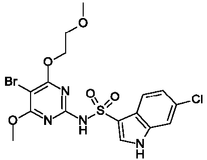 | N-[5-bromo-4-methoxy-6-(2-methoxyethoxy)pyrimidin-2-yl]-6-chloro-1H-indole-3-sulfonamide | A1 | B73 | 491.2 |
| 71 | 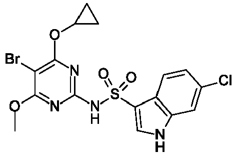 | N-[5-bromo-4-(cyclopropoxy)-6-methoxy-pyrimidin-2-yl]-6-chloro-1H-indole-3-sulfonamide | A1 | B74 | 473.2 |
| 72 | 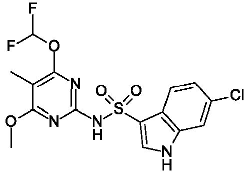 | 6-chloro-N-[4-(difluoromethoxy)-6-methoxy-5-methyl-pyrimidin-2-yl]-1H-indole-3-sulfonamide | A1 | B75 | 419.0 |
| 73 | 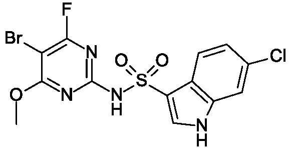 | N-(5-bromo-4-fluoro-6-methoxy-pyrimidin-2-yl)-6-chloro-1H-indole-3-sulfonamide | A1 | B76 | 436.9 |
| 74 | 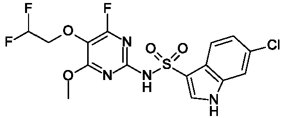 | 6-chloro-N-[5-(2,2-difluoroethoxy)-4-fluoro-6-methoxy-pyrimidin-2-yl]-1H-indole-3-sulfonamide | A1 | B77 | 435.0 |
| 75 | 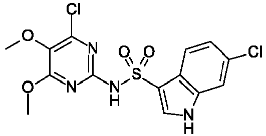 | 6-chloro-N-(4-chloro-5,6-dimethoxy-pyrimidin-2-yl)-1H-indole-3-sulfonamide | A1 | B78 | 403.1 |
| 76 | 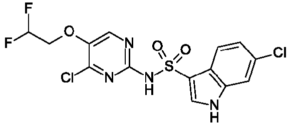 | 6-chloro-N-[4-chloro-5-(2,2-difluoroethoxy)pyrimidin-2-yl]-1H-indole-3-sulfonamide | A1 | B79 | 422.8 |
| 77 | 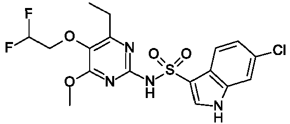 | 6-chloro-N-[5-(2,2-difluoroethoxy)-4-ethyl-6-methoxy-pyrimidin-2-yl]-1H-indole-3-sulfonamide | A1 | B81 | 447.1 |
| 78 | 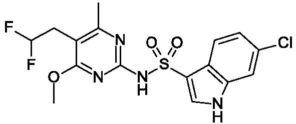 | 6-chloro-N-[5-(2,2-difluoroethyl)-4-methoxy-6-methyl-pyrimidin-2-yl]-1H-indole-3-sulfonamide | A1 | B82 | 416.8 |
| 79 | 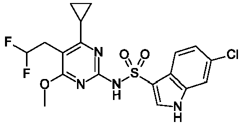 | 6-chloro-N-[4-cyclopropyl-5-(2,2-difluoroethyl)-6-methoxy-pyrimidin-2-yl]-1H-indole-3-sulfonamide | A1 | B83 | 442.8 |
| 80 | 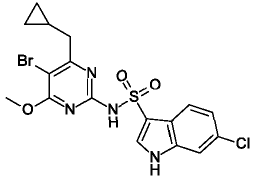 | N-[5-bromo-4-(cyclopropylmethyl)-6-methoxy-pyrimidin-2-yl]-6-chloro-1H-indole-3-sulfonamide | A1 | B84 | 473.1 |
| 81 | 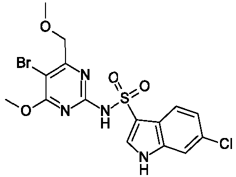 | N-[5-bromo-4-methoxy-6-(methoxymethyl)pyrimidin-2-yl]-6-chloro-1H-indole-3-sulfonamide | A1 | B85 | 463.1 |
| 82 | 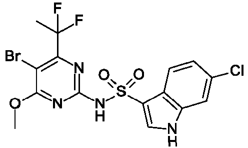 | N-[5-bromo-4-(1,1-difluoroethyl)-6-methoxy-pyrimidin-2-yl]-6-chloro-1H-indole-3-sulfonamide | A1 | B91 | 481.0 |
| 83 | 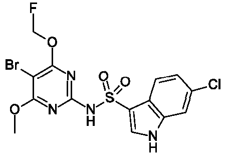 | N-[5-bromo-4-(fluoromethoxy)-6-methoxy-pyrimidin-2-yl]-6-chloro-1H-indole-3-sulfonamide | A1 | B92 | 465.1 |
| 84 | 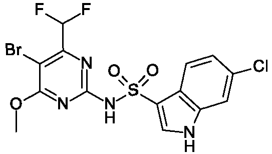 | N-[5-bromo-4-(difluoromethyl)-6-methoxy-pyrimidin-2-yl]-6-chloro-1H-indole-3-sulfonamide | A1 | B93 | 467.1 |
| 85 | 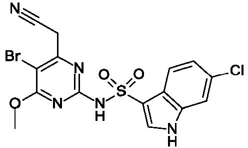 | N-[5-bromo-4-(cyanomethyl)-6-methoxy-pyrimidin-2-yl]-6-chloro-1H-indole-3-sulfonamide | A1 | B94 | 456.1 |
| 86 | 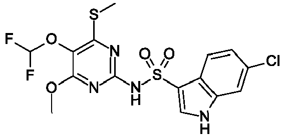 | 6-chloro-N-[5-(difluoromethoxy)-4-methoxy-6-methylsulfanyl-pyrimidin-2-yl]-1H-indole-3-sulfonamide | A1 | B96 | 451.1 |
| 87 | 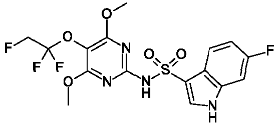 | N-[4,6-dimethoxy-5-(1,1,2-trifluoroethoxy)pyrimidin-2-yl]-6-fluoro-1H-indole-3-sulfonamide | A11 | B58 | 449.2 |
| 88 | 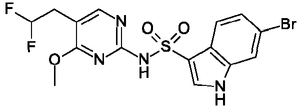 | 6-bromo-N-[5-(2,2-difluoroethyl)-4-methoxy-pyrimidin-2-yl]-1H-indole-3-sulfonamide | A2 | B40 | 447.1 |
| 89 | 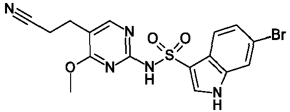 | 6-bromo-N-[5-(2-cyanoethyl)-4-methoxy-pyrimidin-2-yl]-1H-indole-3-sulfonamide | A2 | B48 | 438.0 |
| 90 | 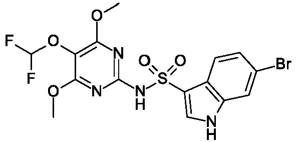 | 6-bromo-N-[5-(difluoromethoxy)-4,6-dimethoxy-pyrimidin-2-yl]-1H-indole-3-sulfonamide | A2 | B55 | 481.1 |
| 91 | 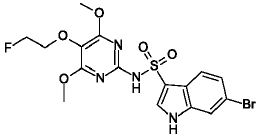 | 6-bromo-N-[5-(2-fluoroethoxy)-4,6-dimethoxy-pyrimidin-2-yl]-1H-indole-3-sulfonamide | A2 | B56 | 477.0 |
| 92 | 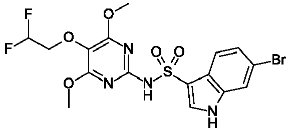 | 6-bromo-N-[5-(2,2-difluoroethoxy)-4,6-dimethoxy-pyrimidin-2-yl]-1H-indole-3-sulfonamide | A2 | B57 | 495.3 |
| 93 | 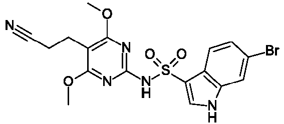 | 6-bromo-N-[5-(2-cyanoethyl)-4,6-dimethoxy-pyrimidin-2-yl]-1H-indole-3-sulfonamide | A2 | B71 | 468.2 |
| 94 | 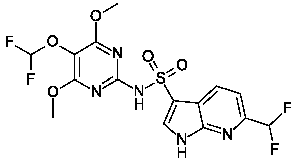 | N-[5-(difluoromethoxy)-4,6-dimethoxy-pyrimidin-2-yl]-6-(difluoromethyl)-1H-pyrrolo[2,3-b]pyridine-3-sulfonamide | A26 | B55 | 452.2 |
| 95 | 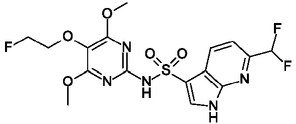 | 6-(difluoromethyl)-N-[5-(2-fluoroethoxy)-4,6-dimethoxy-pyrimidin-2-yl]-1H-pyrrolo[2,3-b]pyridine-3-sulfonamide | A26 | B56 | 446.1 |
| 96 | 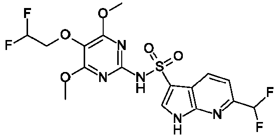 | N-[5-(2,2-difluoroethoxy)-4,6-dimethoxy-pyrimidin-2-yl]-6-(difluoromethyl)-1H-pyrrolo[2,3-b]pyridine-3-sulfonamide | A26 | B57 | 464.1 |
| 97 | 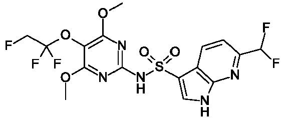 | 6-(difluoromethyl)-N-[4,6-dimethoxy-5-(1,1,2-trifluoroethoxy)pyrimidin-2-yl]-1H-pyrrolo[2,3-b]pyridine-3-sulfonamide | A26 | B58 | 482.1 |
| 98 | 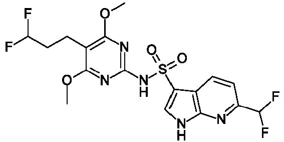 | 6-(difluoromethyl)-N-[5-(3,3-difluoropropyl)-4,6-dimethoxy-pyrimidin-2-yl]-1H-pyrrolo[2,3-b]pyridine-3-sulfonamide | A26 | B65 | 464.2 |
| 99 | 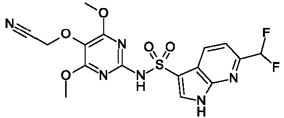 | N-[5-(cyanomethoxy)-4,6-dimethoxy-pyrimidin-2-yl]-6-(difluoromethyl)-1H-pyrrolo[2,3-b]pyridine-3-sulfonamide | A26 | B89 | 441.1 |
| 100 | 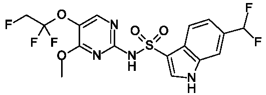 | 6-(difluoromethyl)-N-[4-methoxy-5-(1,1,2-trifluoroethoxy)pyrimidin-2-yl]-1H-indole-3-sulfonamide | A27 | B34 | 453.1 |
| 101 | 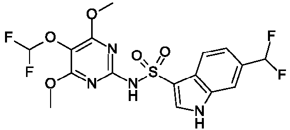 | N-[5-(difluoromethoxy)-4,6-dimethoxy-pyrimidin-2-yl]-6-(difluoromethyl)-1H-indole-3-sulfonamide | A27 | B55 | 449.2 |
| 102 | 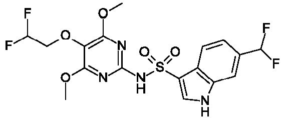 | N-[5-(2,2-difluoroethoxy)-4,6-dimethoxy-pyrimidin-2-yl]-6-(difluoromethyl)-1H-indole-3-sulfonamide | A27 | B57 | 463.2 |
| 103 | 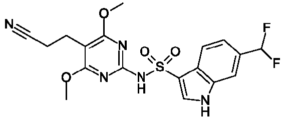 | N-[5-(2-cyanoethyl)-4,6-dimethoxy-pyrimidin-2-yl]-6-(difluoromethyl)-1H-indole-3-sulfonamide | A27 | B71 | 438.2 |
| 104 | 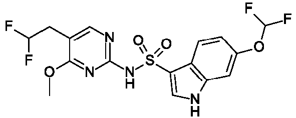 | N-[5-(2,2-difluoroethyl)-4-methoxy-pyrimidin-2-yl]-6-(difluoromethoxy)-1H-indole-3-sulfonamide | A30 | B40 | 435.4 |
| 105 | 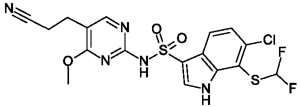 | 6-chloro-N-[5-(2-cyanoethyl)-4-methoxy-pyrimidin-2-yl]-7-(difluoromethylthio)-1H-indole-3-sulfonamide | A34 | B48 | 474.1 |
| 106 | 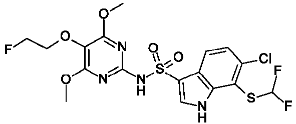 | 6-chloro-7-(difluoromethylthio)-N-[5-(2-fluoroethoxy)-4,6-dimethoxy-pyrimidin-2-yl]-1H-indole-3-sulfonamide | A34 | B56 | 513.1 |
| 107 | 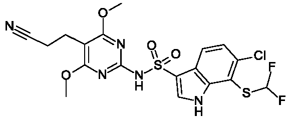 | 6-chloro-N-[5-(2-cyanoethyl)-4,6-dimethoxy-pyrimidin-2-yl]-7-(difluoromethylthio)-1H-indole-3-sulfonamide | A34 | B71 | 504.1 |
| 108 | 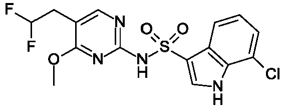 | 7-chloro-N-[5-(2,2-difluoroethyl)-4-methoxy-pyrimidin-2-yl]-1H-indole-3-sulfonamide | A39 | B40 | 403.1 |
| 109 | 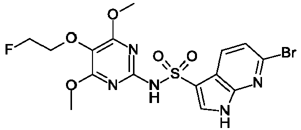 | 6-bromo-N-[5-(2-fluoroethoxy)-4,6-dimethoxy-pyrimidin-2-yl]-1H-pyrrolo[2,3-b]pyridine-3-sulfonamide | A4 | B56 | 476.1 |
| 110 | 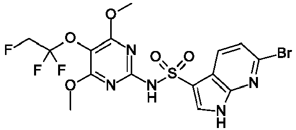 | 6-bromo-N-[4,6-dimethoxy-5-(1,1,2-trifluoroethoxy)pyrimidin-2-yl]-1H-pyrrolo[2,3-b]pyridine-3-sulfonamide | A4 | B58 | 514.1 |
| 111 | 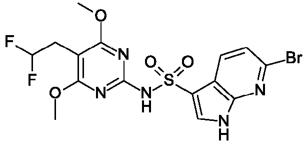 | 6-bromo-N-[5-(2,2-difluoroethyl)-4,6-dimethoxy-pyrimidin-2-yl]-1H-pyrrolo[2,3-b]pyridine-3-sulfonamide | A4 | B63 | 478.0 |
| 112 | 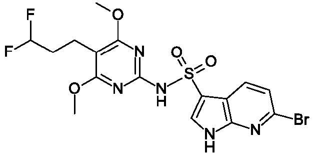 | 6-bromo-N-[5-(3,3-difluoropropyl)-4,6-dimethoxy-pyrimidin-2-yl]-1H-pyrrolo[2,3-b]pyridine-3-sulfonamide | A4 | B65 | 494.2 |
| 113 | 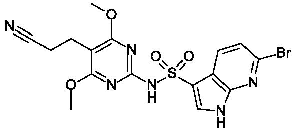 | 6-bromo-N-[5-(2-cyanoethyl)-4,6-dimethoxy-pyrimidin-2-yl]-1H-pyrrolo[2,3-b]pyridine-3-sulfonamide | A4 | B71 | 467.1 |
| 114 | 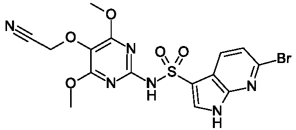 | 6-bromo-N-[5-(cyanomethoxy)-4,6-dimethoxy-pyrimidin-2-yl]-1H-pyrrolo[2,3-b]pyridine-3-sulfonamide | A4 | B89 | 471.1 |
| 115 | 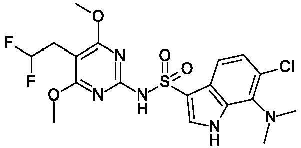 | 6-chloro-N-[5-(2,2-difluoroethyl)-4,6-dimethoxy-pyrimidin-2-yl]-7-(dimethylamino)-1H-indole-3-sulfonamide | A45 | B63 | 476.2 |
| 116 | 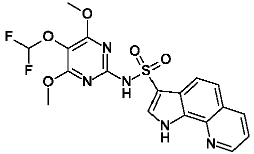 | N-[5-(difluoromethoxy)-4,6-dimethoxy-pyrimidin-2-yl]-1H-pyrrolo[3,2-h]quinoline-3-sulfonamide | A46 | B55 | 452.1 |
| 117 | 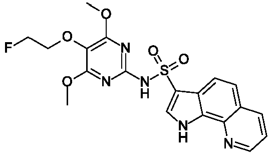 | N-[5-(2-fluoroethoxy)-4,6-dimethoxy-pyrimidin-2-yl]-1H-pyrrolo[3,2-h]quinoline-3-sulfonamide | A46 | B56 | 448.1 |
| 118 | 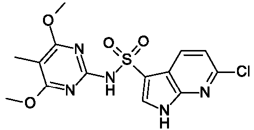 | 6-chloro-N-(4,6-dimethoxy-5-methyl-pyrimidin-2-yl)-1H-pyrrolo[2,3-b]pyridine-3-sulfonamide | A5 | B19 | 384.2 |
| 119 | 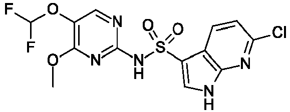 | 6-chloro-N-[5-(difluoromethoxy)-4-methoxy-pyrimidin-2-yl]-1H-pyrrolo[2,3-b]pyridine-3-sulfonamide | A5 | B6 | 406.1 |
| 120 | 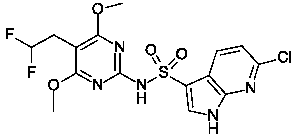 | 6-chloro-N-[5-(2,2-difluoroethyl)-4,6-dimethoxy-pyrimidin-2-yl]-1H-pyrrolo[2,3-b]pyridine-3-sulfonamide | A5 | B63 | 434.2 |
| 121 | 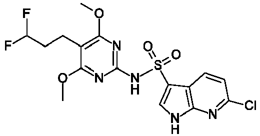 | 6-chloro-N-[5-(3,3-difluoropropyl)-4,6-dimethoxy-pyrimidin-2-yl]-1H-pyrrolo[2,3-b]pyridine-3-sulfonamide | A5 | B65 | 448.2 |
| 122 | 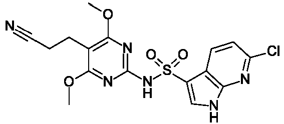 | 6-chloro-N-[5-(2-cyanoethyl)-4,6-dimethoxy-pyrimidin-2-yl]-1H-pyrrolo[2,3-b]pyridine-3-sulfonamide | A5 | B71 | 423.1 |
| 123 | 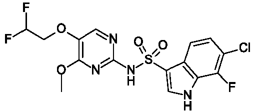 | 6-chloro-N-[5-(2,2-difluoroethoxy)-4-methoxy-pyrimidin-2-yl]-7-fluoro-1H-indole-3-sulfonamide | A6 | B29 | 437.2 |
| 124 | 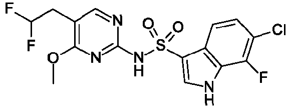 | 6-chloro-N-[5-(2,2-difluoroethyl)-4-methoxy-pyrimidin-2-yl]-7-fluoro-1H-indole-3-sulfonamide | A6 | B40 | 421.1 |
| 125 | 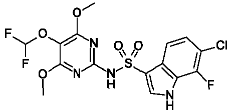 | 6-chloro-N-[5-(difluoromethoxy)-4,6-dimethoxy-pyrimidin-2-yl]-7-fluoro-1H-indole-3-sulfonamide | A6 | B55 | 453.1 |
| 126 | 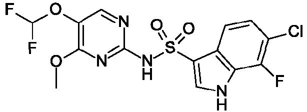 | 6-chloro-N-[5-(difluoromethoxy)-4-methoxy-pyrimidin-2-yl]-7-fluoro-1H-indole-3-sulfonamide | A6 | B6 | 423.1 |
| 127 | 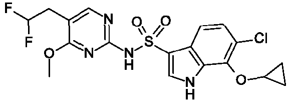 | 6-chloro-7-(cyclopropoxy)-N-[5-(2,2-difluoroethyl)-4-methoxy-pyrimidin-2-yl]-1H-indole-3-sulfonamide | A9 | B40 | 459.1 |
| 128 | 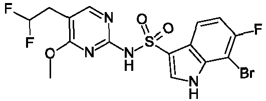 | 7-bromo-N-[5-(2,2-difluoroethyl)-4-methoxy-pyrimidin-2-yl]-6-fluoro-1H-indole-3-sulfonamide | A49 | B40 | 467.0 |
| 129 | 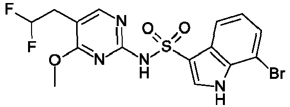 | 7-bromo-N-[5-(2,2-difluoroethyl)-4-methoxy-pyrimidin-2-yl]-1H-indole-3-sulfonamide | A50 | B40 | 449.0 |
| 130 | 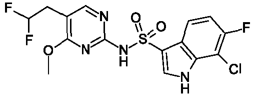 | 7-chloro-N-[5-(2,2-difluoroethyl)-4-methoxy-pyrimidin-2-yl]-6-fluoro-1H-indole-3-sulfonamide | A48 | B40 | 421.0 |
| 131 | 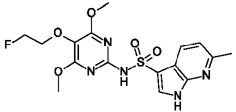 | N-[5-(2-fluoroethoxy)-4,6-dimethoxy-pyrimidin-2-yl]-6-methyl-1H-pyrrolo[2,3-b]pyridine-3-sulfonamide | A47 | B56 | 412.2 |
| 132 | 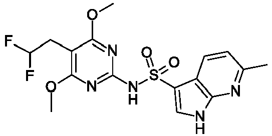 | N-[5-(2,2-difluoroethyl)-4,6-dimethoxy-pyrimidin-2-yl]-6-methyl-1H-pyrrolo[2,3-b]pyridine-3-sulfonamide | A47 | B63 | 414.2 |
| 133 | 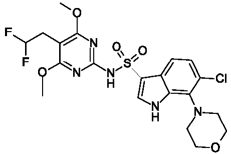 | 6-chloro-N-[5-(2,2-difluoroethyl)-4,6-dimethoxy-pyrimidin-2-yl]-7-morpholino-1H-indole-3-sulfonamide | A51 | B63 | 518.1 |
| 134 | 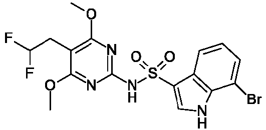 | 7-bromo-N-[5-(2,2-difluoroethyl)-4,6-dimethoxy-pyrimidin-2-yl]-1H-indole-3-sulfonamide | A50 | B63 | 479.1 |

The following examples 135-171 were prepared in analogy to example 24 by coupling the indicated sulfonylchloride intermediates A and amine intermediates B.

| Ex. | Structure | Name | Int. A | Int. B | MS (ESI): m/z, [M+H] + |
|---|---|---|---|---|---|
| 135 | 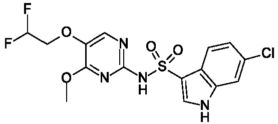 | 6-chloro-N-[5-(2,2-difluoroethoxy)-4-methoxy-pyrimidin-2-yl]-1H-indole-3-sulfonamide | A1 | B29 | 419.1 |
| 136 | 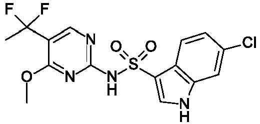 | 6-chloro-N-[5-(1,1-difluoroethyl)-4-methoxy-pyrimidin-2-yl]-1H-indole-3-sulfonamide | A1 | B46 | 402.9 |
| 137 | 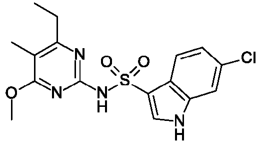 | 6-chloro-N-(4-ethyl-6-methoxy-5-methyl-pyrimidin-2-yl)-1H-indole-3-sulfonamide | A1 | B80 | 381.0 |
| 138 | 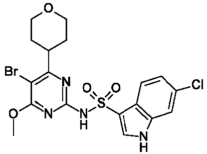 | N-(5-bromo-4-methoxy-6-tetrahydropyran-4-yl-pyrimidin-2-yl)-6-chloro-1H-indole-3-sulfonamide | A1 | B86 | 502.8 |
| 139 | 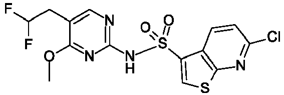 | 6-chloro-N-[5-(2,2-difluoroethyl)-4-methoxy-pyrimidin-2-yl]thieno[2,3-b]pyridine-3-sulfonamide | A10 | B40 | 421.1 |
| 140 | 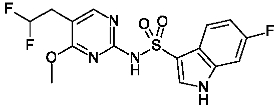 | N-[5-(2,2-difluoroethyl)-4-methoxy-pyrimidin-2-yl]-6-fluoro-1H-indole-3-sulfonamide | A11 | B40 | 387.1 |
| 141 | 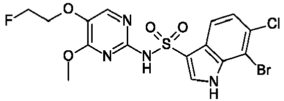 | 7-bromo-6-chloro-N-[5-(2-fluoroethoxy)-4-methoxy-pyrimidin-2-yl]-1H-indole-3-sulfonamide | A13 | B3 | 481.1 |
| 142 | 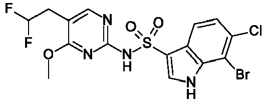 | 7-bromo-6-chloro-N-[5-(2,2-difluoroethyl)-4-methoxy-pyrimidin-2-yl]-1H-indole-3-sulfonamide | A13 | B40 | 483.1 |
| 143 | 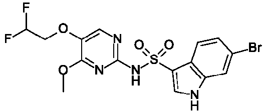 | 6-bromo-N-[5-(2,2-difluoroethoxy)-4-methoxy-pyrimidin-2-yl]-1H-indole-3-sulfonamide | A2 | B29 | 465.1 |
| 144 | 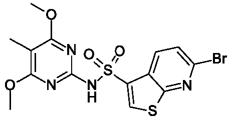 | 6-bromo-N-(4,6-dimethoxy-5-methyl-pyrimidin-2-yl)thieno[2,3-b]pyridine-3-sulfonamide | A21 | B19 | 447.0 |
| 145 | 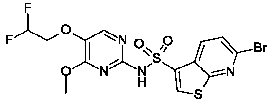 | 6-bromo-N-[5-(2,2-difluoroethoxy)-4-methoxy-pyrimidin-2-yl]thieno[2,3-b]pyridine-3-sulfonamide | A21 | B29 | 483.0 |
| 146 | 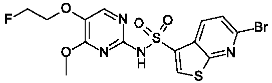 | 6-bromo-N-[5-(2-fluoroethoxy)-4-methoxy-pyrimidin-2-yl]thieno[2,3-b]pyridine-3-sulfonamide | A21 | B3 | 465.0 |
| 147 | 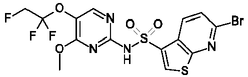 | 6-bromo-N-[4-methoxy-5-(1,1,2-trifluoroethoxy)pyrimidin-2-yl]thieno[2,3-b]pyridine-3-sulfonamide | A21 | B34 | 501.1 |
| 148 | 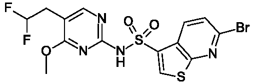 | 6-bromo-N-[5-(2,2-difluoroethyl)-4-methoxy-pyrimidin-2-yl]thieno[2,3-b]pyridine-3-sulfonamide | A21 | B40 | 467.1 |
| 149 | 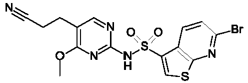 | 6-bromo-N-[5-(2-cyanoethyl)-4-methoxy-pyrimidin-2-yl]thieno[2,3-b]pyridine-3-sulfonamide | A21 | B48 | 456.0 |
| 150 | 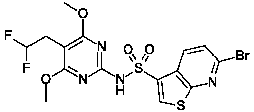 | 6-bromo-N-[5-(2,2-difluoroethyl)-4,6-dimethoxy-pyrimidin-2-yl]thieno[2,3-b]pyridine-3-sulfonamide | A21 | B63 | 497.1 |
| 151 | 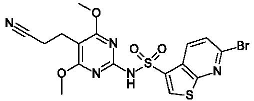 | 6-bromo-N-[5-(2-cyanoethyl)-4,6-dimethoxy-pyrimidin-2-yl]thieno[2,3-b]pyridine-3-sulfonamide | A21 | B71 | 486.1 |
| 152 | 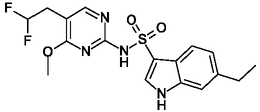 | N-[5-(2,2-difluoroethyl)-4-methoxy-pyrimidin-2-yl]-6-ethyl-1H-indole-3-sulfonamide | A24 | B40 | 397.2 |
| 153 | 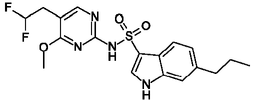 | N-[5-(2,2-difluoroethyl)-4-methoxypyrimidin-2-yl]-6-propyl-1H-indole-3-sulfonamide | A28 | B40 | 411.1 |
| 154 | 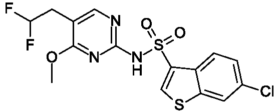 | 6-chloro-N-[5-(2,2-difluoroethyl)-4-methoxy-pyrimidin-2-yl]benzothiophene-3-sulfonamide | A3 | B40 | 420.0 |
| 155 | 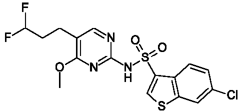 | 6-chloro-N-[5-(3,3-difluoropropyl)-4-methoxy-pyrimidin-2-yl]benzothiophene-3-sulfonamide | A3 | B42 | 433.9 |
| 156 | 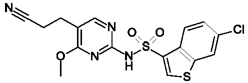 | 6-chloro-N-[5-(2-cyanoethyl)-4-methoxy-pyrimidin-2-yl]benzothiophene-3-sulfonamide | A3 | B48 | 409.2 |
| 157 | 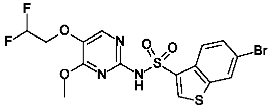 | 6-bromo-N-[5-(2,2-difluoroethoxy)-4-methoxy-pyrimidin-2-yl]benzothiophene-3-sulfonamide | A37 | B29 | 480.0 |
| 158 | 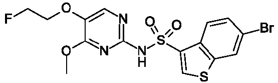 | 6-bromo-N-[5-(2-fluoroethoxy)-4-methoxy-pyrimidin-2-yl]benzothiophene-3-sulfonamide | A37 | B3 | 462.1 |
| 159 | 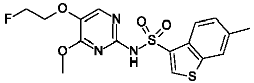 | N-[5-(2-fluoroethoxy)-4-methoxy-pyrimidin-2-yl]-6-methyl-benzothiophene-3-sulfonamide | A38 | B3 | 398.2 |
| 160 | 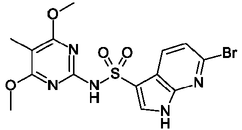 | 6-bromo-N-(4,6-dimethoxy-5-methyl-pyrimidin-2-yl)-1H-pyrrolo[2,3-b]pyridine-3-sulfonamide | A4 | B19 | 428.1 |
| 161 | 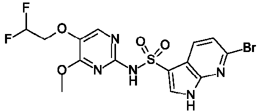 | 6-bromo-N-[5-(2,2-difluoroethoxy)-4-methoxy-pyrimidin-2-yl]-1H-pyrrolo[2,3-b]pyridine-3-sulfonamide | A4 | B29 | 466.2 |
| 162 | 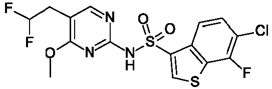 | 6-chloro-N-[5-(2,2-difluoroethyl)-4-methoxy-pyrimidin-2-yl]-7-fluoro-benzothiophene-3-sulfonamide | A40 | B40 | 438.2 |
| 163 | 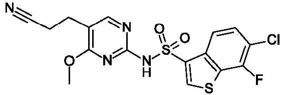 | 6-chloro-N-[5-(2-cyanoethyl)-4-methoxy-pyrimidin-2-yl]-7-fluoro-benzothiophene-3-sulfonamide | A40 | B48 | 427.1 |
| 164 | 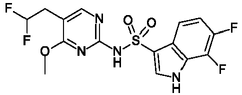 | N-[5-(2,2-difluoroethyl)-4-methoxy-pyrimidin-2-yl]-6,7-difluoro-1H-indole-3-sulfonamide | A41 | B40 | 405.1 |
| 165 | 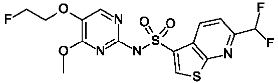 | 6-(difluoromethyl)-N-[5-(2-fluoroethoxy)-4-methoxy-pyrimidin-2-yl]thieno[2,3-b]pyridine-3-sulfonamide | A44 | B3 | 435.1 |
| 166 | 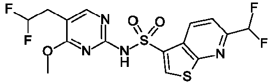 | N-[5-(2,2-difluoroethyl)-4-methoxy-pyrimidin-2-yl]-6-(difluoromethyl)thieno[2,3-b]pyridine-3-sulfonamide | A44 | B40 | 437.1 |
| 167 | 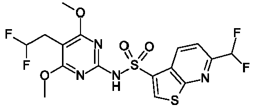 | N-[5-(2,2-difluoroethyl)-4,6-dimethoxy-pyrimidin-2-yl]-6-(difluoromethyl)thieno[2,3-b]pyridine-3-sulfonamide | A44 | B63 | 467.1 |
| 168 | 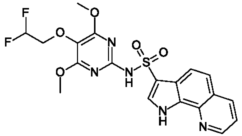 | N-[5-(2,2-difluoroethoxy)-4,6-dimethoxy-pyrimidin-2-yl]-1H-pyrrolo[3,2-h]quinoline-3-sulfonamide | A46 | B57 | 466.1 |
| 169 | 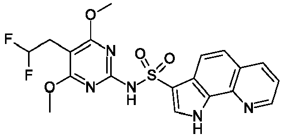 | N-[5-(2,2-difluoroethyl)-4,6-dimethoxy-pyrimidin-2-yl]-1H-pyrrolo[3,2-h]quinoline-3-sulfonamide | A46 | B63 | 450.1 |
| 170 | 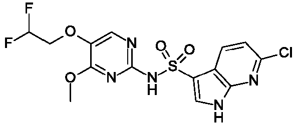 | 6-chloro-N-[5-(2,2-difluoroethoxy)-4-methoxy-pyrimidin-2-yl]-1H-pyrrolo[2,3-b]pyridine-3-sulfonamide | A5 | B29 | 420.1 |
| 171 | 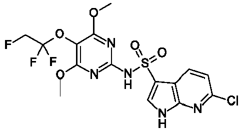 | 6-chloro-N-[4,6-dimethoxy-5-(1,1,2-trifluoroethoxy)pyrimidin-2-yl]-1H-pyrrolo[2,3-b]pyridine-3-sulfonamide | A5 | B58 | 468.1 |

### Example 172: 6-bromo-N-[5-(difluoromethoxy)-4,6-dimethoxy-pyrimidin-2-yl]-1H-pyrrolo[2,3-b]pyridine-3-sulfonamide

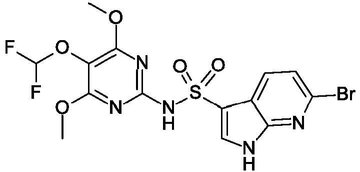

A solution of [5-(difluoromethoxy)-4,6-dimethoxy-pyrimidin-2-yl]amine (1.09 g, 4.44 mmol, intermediate B55) and n-ethyldiisopropylamine (877.48 mg, 1.16 mL, 6.65 mmol) in 1,2-dichloroethane (20 mL) was heated to 70 °C and stirred for 20 min. 6-bromo-1H-pyrrolo[2,3-b]pyridine-3-sulfonyl chloride (1.66 g, 5.32 mmol, intermediate A4) was added in 10 portions over 45 min. The stirring was continued at 70 °C for 30 min. The reaction mixture was concentrated in vacuo. The residue was purified by flash chromatography over silica gel using a gradient ethylacetate/heptane 0-60% to provide 260 mg of a solid. This product was purified again by flash chromatography on a C18 RediSepRf Gold column (30 g, 10% to 70% MeCN in water) to provide the title compound as a white solid (122 mg, 5.73 % yield). MS (ESI): m/z= 482.1 [M+H]+

The following examples 173-212 were prepared in analogy to example 172 by coupling the indicated sulfonylchloride intermediates A and amine intermediates B.

| Ex. | Structure | Name | Int. A | Int. B | MS (ESI): m/z, [M+H] + |
|---|---|---|---|---|---|
| 173 | 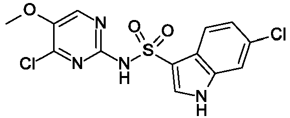 | 6-chloro-N-(4-chloro-5-methoxy-pyrimidin-2-yl)-1H-indole-3-sulfonamide | A1 | B23 | 373.1 |
| 174 | 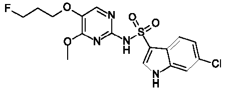 | 6-chloro-N-[5-(3-fluoropropoxy)-4-methoxy-pyrimidin-2-yl]-1H-indole-3-sulfonamide | A1 | B30 | 415.2 |
| 175 | 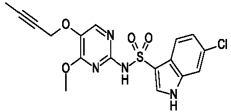 | N-(5-but-2-ynoxy-4-methoxy-pyrimidin-2-yl)-6-chloro-1H-indole-3-sulfonamide | A1 | B31 | 407.1 |
| 176 | 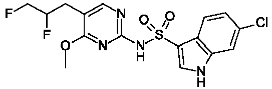 | 6-chloro-N-[5-(2,3-difluoropropyl)-4-methoxy-pyrimidin-2-yl]-1H-indole-3-sulfonamide | A1 | B43 | 417.2 |
| 177 | 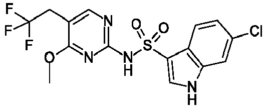 | 6-chloro-N-[4-methoxy-5-(2,2,2-trifluoroethyl)pyrimidin-2-yl]-1H-indole-3-sulfonamide | A1 | B44 | 421.2 |
| 178 | 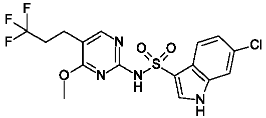 | 6-chloro-N-[4-methoxy-5-(3,3,3-trifluoropropyl)pyrimidin-2-yl]-1H-indole-3-sulfonamide | A1 | B45 | 435.1 |
| 179 | 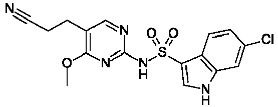 | 6-chloro-N-[5-(2-cyanoethyl)-4-methoxy-pyrimidin-2-yl]-1H-indole-3-sulfonamide | A1 | B48 | 392.1 |
| 180 | 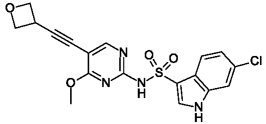 | 6-chloro-N-[4-methoxy-5-[2-(oxetan-3-yl)ethynyl]pyrimidin-2-yl]-1H-indole-3-sulfonamide | A1 | B52 | 419.0 |
| 181 | 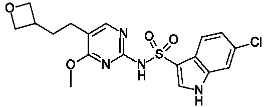 | 6-chloro-N-[4-methoxy-5-[2-(oxetan-3 -yl)ethyl]pyrimidin-2-yl]-1H-indole-3-sulfonamide | A1 | B53 | 423.0 |
| 182 | 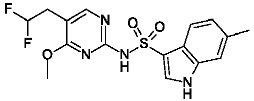 | N-[5-(2,2-difluoroethyl)-4-methoxy-pyrimidin-2-yl]-6-methyl-1H-indole-3-sulfonamide | A12 | B40 | 383.2 |
| 183 | 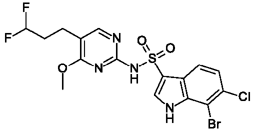 | 7-bromo-6-chloro-N-[5-(3,3-difluoropropyl)-4-methoxy-pyrimidin-2-yl]-1H-indole-3-sulfonamide | A13 | B42 | 495.1 |
| 184 | 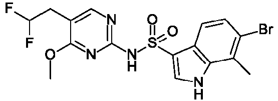 | 6-bromo-N-[5-(2,2-difluoroethyl)-4-methoxy-pyrimidin-2-yl]-7-methyl-1H-indole-3-sulfonamide | A14 | B40 | 463.2 |
| 185 | 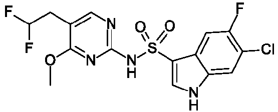 | 6-chloro-N-[5-(2,2-difluoroethyl)-4-methoxy-pyrimidin-2-yl]-5-fluoro-1H-indole-3-sulfonamide | A15 | B40 | 421.1 |
| 186 | 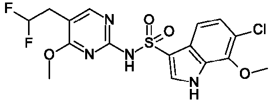 | 6-chloro-N-[5-(2,2-difluoroethyl)-4-methoxy-pyrimidin-2-yl]-7-methoxy-1H-indole-3-sulfonamide | A16 | B40 | 433.1 |
| 187 | 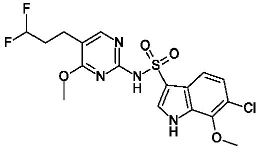 | 6-chloro-N-[5-(3,3-difluoropropyl)-4-methoxy-pyrimidin-2-yl]-7-methoxy-1H-indole-3-sulfonamide | A16 | B42 | 447.2 |
| 188 | 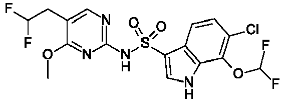 | 6-chloro-N-[5-(2,2-difluoroethyl)-4-methoxy-pyrimidin-2-yl]-7-(difluoromethoxy)-1H-indole-3-sulfonamide | A19 | B40 | 468.9 |
| 189 | 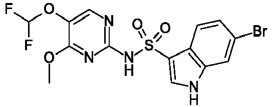 | 6-bromo-N-[5-(difluoromethoxy)-4-methoxy-pyrimidin-2-yl]-1H-indole-3-sulfonamide | A2 | B6 | 449.1 |
| 190 | 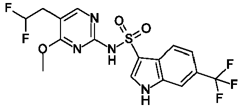 | N-[5-(2,2-difluoroethyl)-4-methoxy-pyrimidin-2-yl]-6-(trifluoromethyl)- 1H-indole-3-sulfonamide | A20 | B40 | 437.3 |
| 191 | 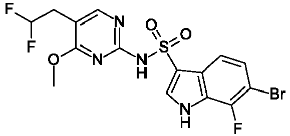 | 6-bromo-N-[5-(2,2-difluoroethyl)-4-methoxy-pyrimidin-2-yl]-7-fluoro-1H-indole-3-sulfonamide | A22 | B40 | 467.2 |
| 192 | 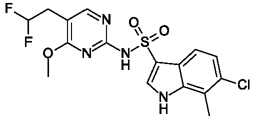 | 6-chloro-N-[5-(2,2-difluoroethyl)-4-methoxy-pyrimidin-2-yl]-7-methyl-1H-indole-3-sulfonamide | A23 | B40 | 417.2 |
| 193 | 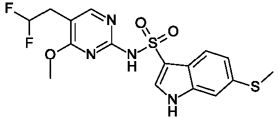 | N-[5-(2,2-difluoroethyl)-4-methoxy-pyrimidin-2-yl]-6-(methylthio)-1H-indole-3-sulfonamide | A25 | B40 | 412.1 |
| 194 | 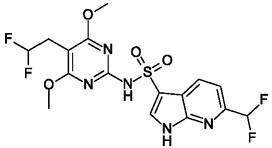 | N-[5-(2,2-difluoroethyl)-4,6-dimethoxy-pyrimidin-2-yl]-6-(difluoromethyl)-1H-pyrrolo[2,3-b]pyridine-3-sulfonamide | A26 | B63 | 450.2 |
| 195 | 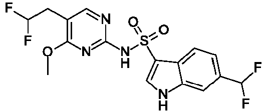 | N-[5-(2,2-difluoroethyl)-4-methoxy-pyrimidin-2-yl]-6-(difluoromethyl)-1H-indole-3-sulfonamide | A27 | B40 | 419.2 |
| 196 | 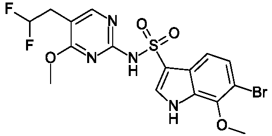 | 6-bromo-N-[5-(2,2-difluoroethyl)-4-methoxy-pyrimidin-2-yl]-7-methoxy-1H-indole-3-sulfonamide | A29 | B40 | 477.3 |
| 197 | 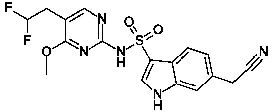 | 6-(cyanomethyl)-N-[5-(2,2-difluoroethyl)-4-methoxy-pyrimidin-2-yl]-1H-indole-3-sulfonamide | A31 | B40 | 408.1 |
| 198 | 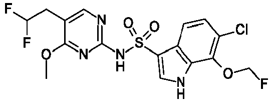 | 6-chloro-N-[5-(2,2-difluoroethyl)-4-methoxy-pyrimidin-2-yl]-7-(fluoromethoxy)-1H-indole-3-sulfonamide | A33 | B40 | 451.2 |
| 199 | 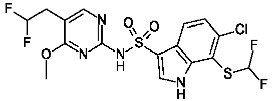 | 6-chloro-N-[5-(2,2-difluoroethyl)-4-methoxy-pyrimidin-2-yl]-7-(difluoromethylsulfanyl)-1H-indole-3-sulfonamide | A34 | B40 | 484.8 |
| 200 | 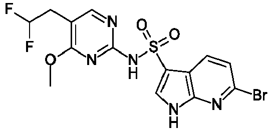 | 6-bromo-N-[5-(2,2-difluoroethyl)-4-methoxy-pyrimidin-2-yl]-1H-pyrrolo[2,3-b]pyridine-3-sulfonamide | A4 | B40 | 448.1 |
| 201 | 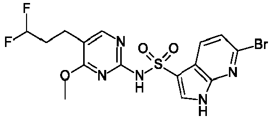 | 6-bromo-N-[5-(3,3-difluoropropyl)-4-methoxy-pyrimidin-2-yl]-1H-pyrrolo[2,3-b]pyridine-3-sulfonamide | A4 | B42 | 462.1 |
| 202 | 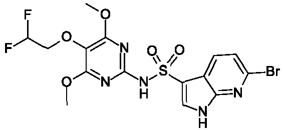 | 6-bromo-N-[5-(2,2-difluoroethoxy)-4,6-dimethoxy-pyrimidin-2-yl]-1H-pyrrolo[2,3-b]pyridine-3-sulfonamide | A4 | B57 | 494.1 |
| 203 | 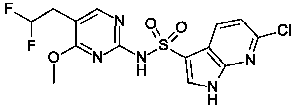 | 6-chloro-N-[5-(2,2-difluoroethyl)-4-methoxy-pyrimidin-2-yl]-1H-pyrrolo[2,3-b]pyridine-3-sulfonamide | A5 | B40 | 404.2 |
| 204 | 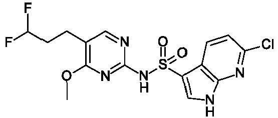 | 6-chloro-N-[5-(3,3-difluoropropyl)-4-methoxy-pyrimidin-2-yl]-1H-pyrrolo[2,3-b]pyridine-3-sulfonamide | A5 | B42 | 418.1 |
| 205 | 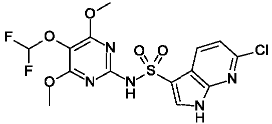 | 6-chloro-N-[5-(difluoromethoxy)-4,6-dimethoxy-pyrimidin-2-yl]-1H-pyrrolo[2,3-b]pyridine-3-sulfonamide | A5 | B55 | 436.2 |
| 206 | 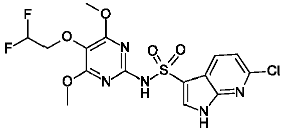 | 6-chloro-N-[5-(2,2-difluoroethoxy)-4,6-dimethoxy-pyrimidin-2-yl]-1H-pyrrolo[2,3-b]pyridine-3-sulfonamide | A5 | B57 | 450.2 |
| 207 | 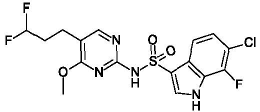 | 6-chloro-N-[5-(3,3-difluoropropyl)-4-methoxy-pyrimidin-2-yl]-7-fluoro-1H-indole-3-sulfonamide | A6 | B42 | 435.0 |
| 208 | 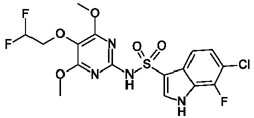 | 6-chloro-N-[5-(2,2-difluoroethoxy)-4,6-dimethoxy-pyrimidin-2-yl]-7-fluoro-1H-indole-3-sulfonamide | A6 | B57 | 467.2 |
| 209 | 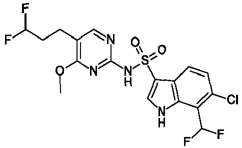 | 6-chloro-7-(difluoromethyl)-N-[5-(3,3-difluoropropyl)-4-methoxy-pyrimidin-2-yl]-1H-indole-3-sulfonamide | A7 | B42 | 467.1 |
| 210 | 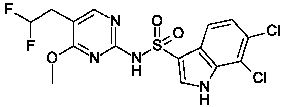 | 6,7-dichloro-N-[5-(2,2-difluoroethyl)-4-methoxy-pyrimidin-2-yl]-1H-indole-3-sulfonamide | A8 | B40 | 437.1 |
| 211 | 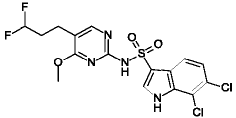 | 6,7-dichloro-N-[5-(3,3-difluoropropyl)-4-methoxy-pyrimidin-2-yl]-1H-indole-3-sulfonamide | A8 | B42 | 451.1 |
| 212 | 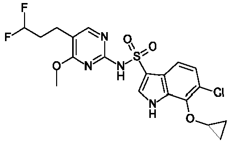 | 6-chloro-7-(cyclopropoxy)-N-[5-(3,3-difluoropropyl)-4-methoxy-pyrimidin-2-yl]-1H-indole-3-sulfonamide | A9 | B42 | 473.2 |

### Example 213: 6-bromo-7-fluoro-N-[5-(2-fluoroethoxy)-4-methoxy-pyrimidin-2-yl]-1H-indole-3-sulfonamide

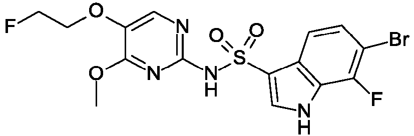

To a suspension of [5-(2-fluoroethoxy)-4-methoxy-pyrimidin-2-yl]amine (25 mg, 0.134 mmol, intermediate B3) and potassium phosphate tribasic (29.23 mg, 0.134 mmol) in acetonitrile (800 uL) was added 6-bromo-7-fluoro-1H-indole-3-sulfonyl chloride (60.3 mg, 0.174 mmol, intermediate A22). The reaction mixture was stirred at room temperature for 1 hr, poured into water and extracted twice with EtOAc. The organic layers were dried over sodiumsulfate, filtered and concentrated in vacuo. The residue was purified by flash chromatography over silica gel using a gradient ethylacetate/heptane 0-100% to provide the title compound as a white solid (17 mg, 27 % yield). MS (ESI): m/z= 465.1 [M+H]+

The following examples 214-267 were prepared in analogy to example 213 by coupling the indicated sulfonylchloride intermediates A and amine intermediates B.

| Ex. | Structure | Name | Int. A | Int. B | MS (ESI): m/z, [M+H] + |
|---|---|---|---|---|---|
| 214 | 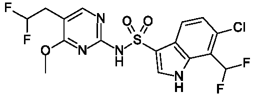 | 6-chloro-N-[5-(2,2-difluoroethyl)-4-methoxy-pyrimidin-2-yl]-7-(difluoromethyl)-1H-indole-3-sulfonamide | A7 | B40 | 453.1 |
| 215 | 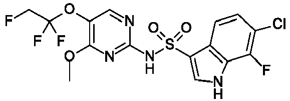 | 6-chloro-7-fluoro-N-[4-methoxy-5-(1,1,2-trifluoroethoxy)pyrimidin-2-yl]-1H-indole-3-sulfonamide | A6 | B34 | 455.2 |
| 216 | 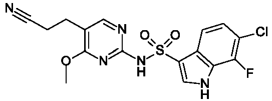 | 6-chloro-N-[5-(2-cyanoethyl)-4-methoxy-pyrimidin-2-yl]-7-fluoro-1H-indole-3-sulfonamide | A6 | B48 | 410.3 |
| 217 | 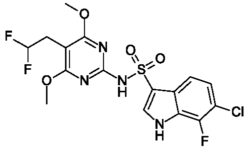 | 6-chloro-N-[5-(2,2-difluoroethyl)-4,6-dimethoxy-pyrimidin-2-yl]-7-fluoro-1H-indole-3-sulfonamide | A6 | B63 | 451.2 |
| 218 | 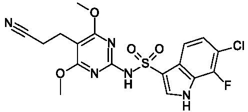 | 6-chloro-N-[5-(2-cyanoethyl)-4,6-dimethoxy-pyrimidin-2-yl]-7-fluoro-1H-indole-3-sulfonamide | A6 | B71 | 440.2 |
| 219 | 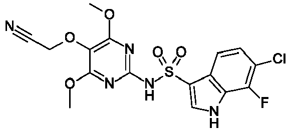 | 6-chloro-N-[5-(cyanomethoxy)-4,6-dimethoxy-pyrimidin-2-yl]-7-fluoro-1H-indole-3-sulfonamide | A6 | B89 | 442.1 |
| 220 | 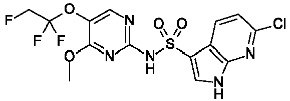 | 6-chloro-N-[4-methoxy-5-(1,1,2-trifluoroethoxy)pyrimidin-2-yl]-1H-pyrrolo[2,3-b]pyridine-3-sulfonamide | A5 | B34 | 438.1 |
| 221 | 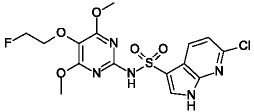 | 6-chloro-N-[5-(2-fluoroethoxy)-4,6-dimethoxy-pyrimidin-2-yl]-1H-pyrrolo[2,3-b]pyridine-3-sulfonamide | A5 | B56 | 432.0 |
| 222 | 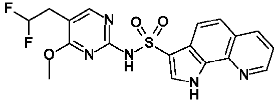 | N-[5-(2,2-difluoroethyl)-4-methoxy-pyrimidin-2-yl]-1H-pyrrolo[3,2-h]quinoline-3-sulfonamide | A46 | B40 | 420.1 |
| 223 | 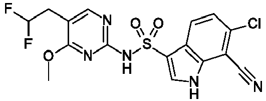 | 6-chloro-7-cyano-N-[5-(2,2-difluoroethyl)-4-methoxy-pyrimidin-2-yl]-1H-indole-3-sulfonamide | A43 | B40 | 428.1 |
| 224 | 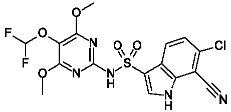 | 6-chloro-7-cyano-N-[5-(difluoromethoxy)-4,6-dimethoxy-pyrimidin-2-yl]-1H-indole-3-sulfonamide | A43 | B55 | 458.1 |
| 225 | 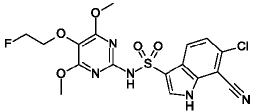 | 6-chloro-7-cyano-N-[5-(2-fluoroethoxy)-4,6-dimethoxy-pyrimidin-2-yl]-1H-indole-3-sulfonamide | A43 | B56 | 454.1 |
| 226 | 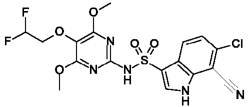 | 6-chloro-7-cyano-N-[5-(2,2-difluoroethoxy)-4,6-dimethoxy-pyrimidin-2-yl]-1H-indole-3-sulfonamide | A43 | B57 | 472.1 |
| 227 | 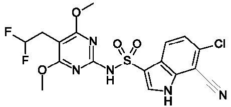 | 6-chloro-7-cyano-N-[5-(2,2-difluoroethyl)-4,6-dimethoxy-pyrimidin-2-yl]-1H-indole-3-sulfonamide | A43 | B63 | 456.1 |
| 228 | 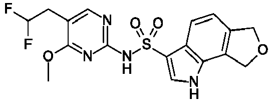 | N-[5-(2,2-difluoroethyl)-4-methoxy-pyrimidin-2-yl]-6,8-dihydro-1H-fur[3,4-g]indole-3-sulfonamide | A42 | B40 | 409.2 |
| 229 | 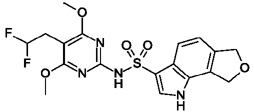 | N-[5-(2,2-difluoroethyl)-4,6-dimethoxy-pyrimidin-2-yl]-6,8-dihydro-1H-fur[3,4-g]indole-3-sulfonamide | A42 | B63 | 441.1 |
| 230 | 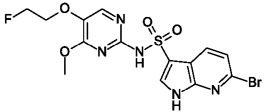 | 6-bromo-N-[5-(2-fluoroethoxy)-4-methoxy-pyrimidin-2-yl]-1H-pyrrolo[2,3-b]pyridine-3-sulfonamide | A4 | B3 | 448.3 |
| 231 | 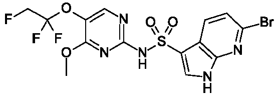 | 6-bromo-N-[4-methoxy-5-(1,1,2-trifluoroethoxy)pyrimidin-2-yl]-1H-pyrrolo[2,3-b]pyridine-3-sulfonamide | A4 | B34 | 484.0 |
| 232 | 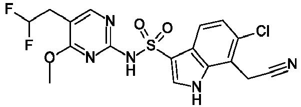 | 6-chloro-7-(cyanomethyl)-N-[5-(2,2-difluoroethyl)-4-methoxy-pyrimidin-2-yl]-1H-indole-3-sulfonamide | A36 | B40 | 442.2 |
| 233 | 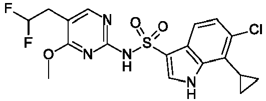 | 6-chloro-7-cyclopropyl-N-[5-(2,2-difluoroethyl)-4-methoxy-pyrimidin-2-yl]-1H-indole-3-sulfonamide | A35 | B40 | 443.2 |
| 234 | 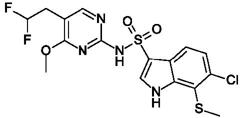 | 6-chloro-N-[5-(2,2-difluoroethyl)-4-methoxy-pyrimidin-2-yl]-7-(methylthio)-1H-indole-3-sulfonamide | A32 | B40 | 449.2 |
| 235 | 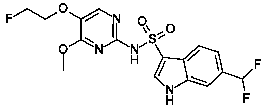 | 6-(difluoromethyl)-N-[5-(2-fluoroethoxy)-4-methoxy-pyrimidin-2-yl]-1H-indole-3-sulfonamide | A27 | B3 | 417.3 |
| 236 | 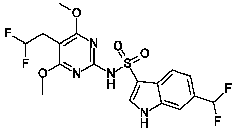 | N-[5-(2,2-difluoroethyl)-4,6-dimethoxy-pyrimidin-2-yl]-6-(difluoromethyl)-1H-indole-3-sulfonamide | A27 | B63 | 449.3 |
| 237 | 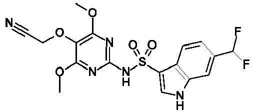 | N-[5-(cyanomethoxy)-4,6-dimethoxy-pyrimidin-2-yl]-6-(difluoromethyl)-1H-indole-3-sulfonamide | A27 | B89 | 440.2 |
| 238 | 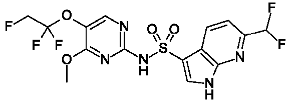 | 6-(difluoromethyl)-N-[4-methoxy-5-(1,1,2-trifluoroethoxy)pyrimidin-2-yl]-1H-pyrrolo[2,3-b]pyridine-3-sulfonamide | A26 | B34 | 454.1 |
| 239 | 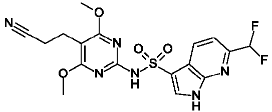 | N-[5-(2-cyanoethyl)-4,6-dimethoxy-pyrimidin-2-yl]-6-(difluoromethyl)-1H-pyrrolo[2,3-b]pyridine-3-sulfonamide | A26 | B71 | 439.3 |
| 240 | 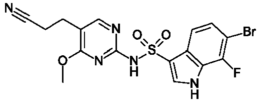 | 6-bromo-N-[5-(2-cyanoethyl)-4-methoxy-pyrimidin-2-yl]-7-fluoro-1H-indole-3-sulfonamide | A22 | B48 | 456.2 |
| 241 | 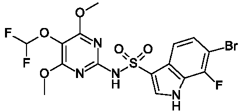 | 6-bromo-N-[5-(difluoromethoxy)-4,6-dimethoxy-pyrimidin-2-yl]-7-fluoro-1H-indole-3-sulfonamide | A22 | B55 | 497.0 |
| 242 | 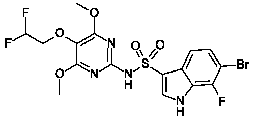 | 6-bromo-N-[5-(2,2-difluoroethoxy)-4,6-dimethoxy-pyrimidin-2-yl]-7-fluoro-1H-indole-3-sulfonamide | A22 | B57 | 513.2 |
| 243 | 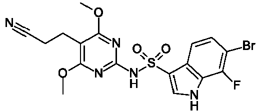 | 6-bromo-N-[5-(2-cyanoethyl)-4,6-dimethoxy-pyrimidin-2-yl]-7-fluoro-1H-indole-3-sulfonamide | A22 | B71 | 486.0 |
| 244 | 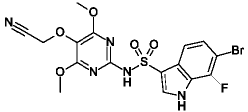 | 6-bromo-N-[5-(cyanomethoxy)-4,6-dimethoxy-pyrimidin-2-yl]-7-fluoro-1H-indole-3-sulfonamide | A22 | B89 | 486.1 |
| 245 | 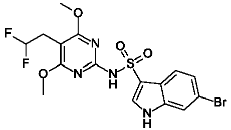 | 6-bromo-N-[5-(2,2-difluoroethyl)-4,6-dimethoxy-pyrimidin-2-yl]-1H-indole-3-sulfonamide | A2 | B63 | 479.0 |
| 246 | 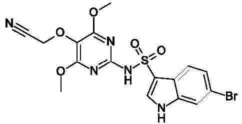 | 6-bromo-N-[5-(cyanomethoxy)-4,6-dimethoxy-pyrimidin-2-yl]-1H-indole-3-sulfonamide | A2 | B89 | 470.2 |
| 247 | 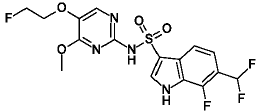 | 6-(difluoromethyl)-7-fluoro-N-[5-(2-fluoroethoxy)-4-methoxy-pyrimidin-2-yl]-1H-indole-3-sulfonamide | A18 | B3 | 435.0 |
| 248 | 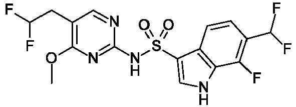 | N-[5-(2,2-difluoroethyl)-4-methoxy-pyrimidin-2-yl]-6-(difluoromethyl)-7-fluoro-1H-indole-3-sulfonamide | A18 | B40 | 437.5 |
| 249 | 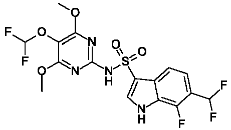 | N-[5-(difluoromethoxy)-4,6-dimethoxy-pyrimidin-2-yl]-6-(difluoromethyl)-7-fluoro-1H-indole-3-sulfonamide | A18 | B55 | 469.4 |
| 250 | 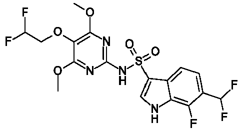 | N-[5-(2,2-difluoroethoxy)-4,6-dimethoxy-pyrimidin-2-yl]-6-(difluoromethyl)-7-fluoro-1H-indole-3-sulfonamide | A18 | B57 | 483.4 |
| 251 | 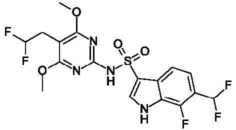 | N-[5-(2,2-difluoroethyl)-4,6-dimethoxy-pyrimidin-2-yl]-6-(difluoromethyl)-7-fluoro-1H-indole-3-sulfonamide | A18 | B63 | 467.3 |
| 252 | 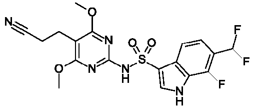 | N-[5-(2-cyanoethyl)-4,6-dimethoxy-pyrimidin-2-yl]-6-(difluoromethyl)-7-fluoro-1H-indole-3-sulfonamide | A18 | B71 | 456.3 |
| 253 | 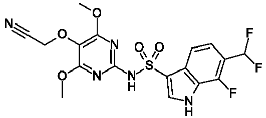 | N-[5-(cyanomethoxy)-4,6-dimethoxy-pyrimidin-2-yl]-6-(difluoromethyl)-7-fluoro-1H-indole-3-sulfonamide | A18 | B89 | 458.3 |
| 254 | 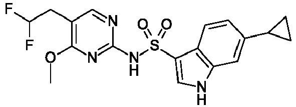 | 6-cyclopropyl-N-[5-(2,2-difluoroethyl)-4-methoxy-pyrimidin-2-yl]-1H-indole-3-sulfonamide | A17 | B40 | 409.3 |
| 255 | 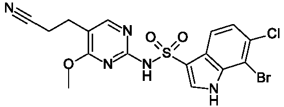 | 7-bromo-6-chloro-N-[5-(2-cyanoethyl)-4-methoxy-pyrimidin-2-yl]-1H-indole-3-sulfonamide | A13 | B48 | 472.2 |
| 256 | 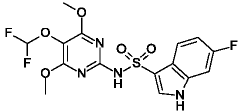 | N-[5-(difluoromethoxy)-4,6-dimethoxy-pyrimidin-2-yl]-6-fluoro-1H-indole-3-sulfonamide | A11 | B55 | 419.2 |
| 257 | 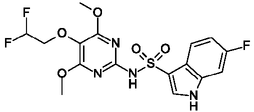 | N-[5-(2,2-difluoroethoxy)-4,6-dimethoxy-pyrimidin-2-yl]-6-fluoro-1H-indole-3-sulfonamide | A11 | B57 | 433.2 |
| 258 | 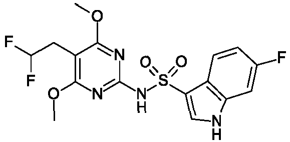 | N-[5-(2,2-difluoroethyl)-4,6-dimethoxy-pyrimidin-2-yl]-6-fluoro-1H-indole-3-sulfonamide | A11 | B63 | 417.2 |
| 259 | 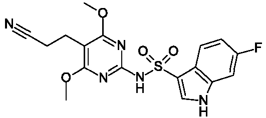 | N-[5-(2-cyanoethyl)-4,6-dimethoxy-pyrimidin-2-yl]-6-fluoro-1H-indole-3-sulfonamide | A11 | B71 | 406.2 |
| 260 | 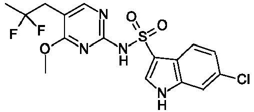 | 6-chloro-N-[5-(2,2-difluoropropyl)-4-methoxy-pyrimidin-2-yl]-1H-indole-3-sulfonamide | A1 | B41 | 417.2 |
| 261 | 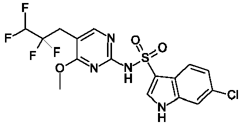 | 6-chloro-N-[4-methoxy-5-(2,2,3,3-tetrafluoropropyl)pyrimidin-2-yl]-1H-indole-3-sulfonamide | A1 | B47 | 453.2 |
| 262 | 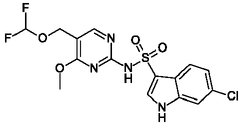 | 6-chloro-N-[5-(difluoromethoxymethyl)-4-methoxy-pyrimidin-2-yl]-1H-indole-3-sulfonamide | A1 | B50 | 419.2 |
| 263 | 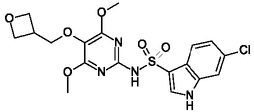 | 6-chloro-N-[4,6-dimethoxy-5-(oxetan-3-ylmethoxy)pyrimidin-2-yl]-1H-indole-3-sulfonamide | A1 | B87 | 455.2 |
| 264 | 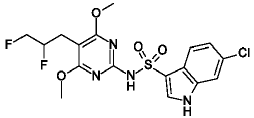 | 6-chloro-N-[5-(2,3-difluoropropyl)-4,6-dimethoxy-pyrimidin-2-yl]-1H-indole-3-sulfonamide | A1 | B88 | 447.1 |
| 265 | 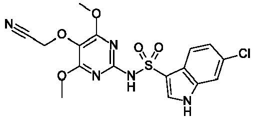 | 6-chloro-N-[5-(cyanomethoxy)-4,6-dimethoxy-pyrimidin-2-yl]-1H-indole-3-sulfonamide | A1 | B89 | 424.2 |
| 266 | 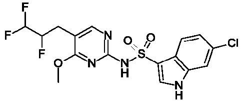 | 6-chloro-N-[4-methoxy-5-(2,3,3-trifluoropropyl)pyrimidin-2-yl]-1H-indole-3-sulfonamide | A1 | B90 | 435.0 |
| 267 | 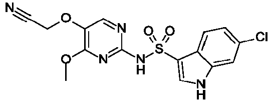 | 6-chloro-N-[5-(cyanomethoxy)-4-methoxy-pyrimidin-2-yl]-1H-indole-3-sulfonamide | A1 | B95 | 394.1 |

### Example 268: 6-bromo-N-[5-(difluoromethoxy)-4-methoxy-pyrimidin-2-yl]benzothiophene-3-sulfonamide

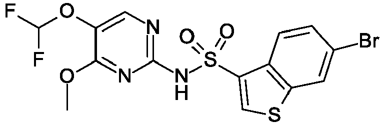

In a dried tube was added sodium hydride in dispersion in mineral oil (10.04 mg, 0.251 mmol), N,N-dimethylformamide (0.686 mL) and at 0 °C, [5-(difluoromethoxy)-4-methoxy-pyrimidin-2-yl]amine (40 mg, 0.209 mmol, intermediate B6). The mixture was stirred at 22 °C for 10 min, then 6-bromobenzothiophene-3-sulfonyl chloride (78.25 mg, 0.251 mmol, intermediate A37) was added in three portions. The mixture was stirred at 22 °C for 5 min, then quenched with ice water and acidified with citric acid 1M. The aqueous layer was extracted twice with ethyl acetate, the organic layers were combined, dried over sodium sulfate, filtered and concentrated in vacuo. The residue was purified by flash chromatography over silica gel using a gradient ethylacetate/heptane 0-70% to provide the title compound as a off-white solid (8 mg, 8.2 % yield). MS (ESI): m/z= 466.0 [M+H]+

### Example 269: 6-bromo-N-[5-(difluoromethoxy)-4,6-dimethoxy-pyrimidin-2-yl]thieno[2,3-b]pyridine-3-sulfonamide

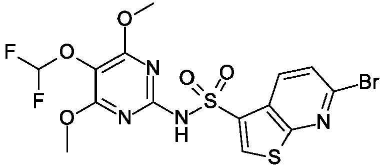

### Step 1: 6-bromothieno[2,3-b]pyridine-3-sulfonyl fluoride

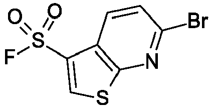

To a suspension of 6-bromothieno[2,3-b]pyridine-3-sulfonyl chloride (200 mg, 0.576 mmol, intermediate A21) in acetonitrile (0.576 mL) under argon, was added a solution of potassium hydrogen fluoride (112.44 mg, 1.44 mmol) in water (0.576 mL). The reaction mixture was stirred for 1 hour at room temperature. The reaction mixture was quenched with ethyl acetate and water and extracted twice with ethyl acetate. The combined organic phases were dried over sodium sulfate, filtered and concentrated in vacuo. The residue was purified by flash chromatography over silica gel using a gradient ethylacetate/heptane 0-100% to provide the title compound as a white solid (168.5 mg, 79 % yield). MS (ESI): m/z= 297.9 [M+H]+

### Step 2: 6-bromo-N-[5-(difluoromethoxy)-4,6-dimethoxy-pyrimidin-2-yl]thieno[2,3-b]pyridine-3-sulfonamide

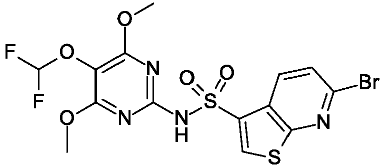

A solution of [5-(difluoromethoxy)-4,6-dimethoxy-pyrimidin-2-yl]amine (40 mg, 0.181 mmol, intermediate B55) in N,N-dimethylformamide (1 mL) was cooled to 0 °C. Sodium hydride (8.68 mg, 0.217 mmol) was added and the reaction mixture was stirred at rt for 20 min. The reaction mixture was cooled to 0 °C and 6-bromothieno[2,3-b]pyridine-3-sulfonyl fluoride (69.86 mg, 0.217 mmol) was added. The reaction mixture was allowed to warm to room temperature, stirred for 3 hours and quenched with water, poured into brine and extracted twice with ethyl acetate. The organic layers were dried over sodium sulfate, filtered and concentrated in vacuo. The residue was purified by flash chromatography over silica gel using a gradient ethylacetate/heptane 0-40% to provide the title compound as a white solid (6.2 mg, 6.9 % yield). MS (ESI): m/z= 499.0 [M+H]+

### Example 270: 6-bromo-N-[5-(3,3-difluoropropyl)-4-methoxy-pyrimidin-2-yl]thieno[2,3-b]pyridine-3-sulfonamide

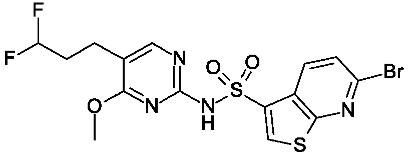

The title compound was prepared in analogy to Example 269 from 6-bromothieno[2,3-b]pyridine-3-sulfonyl fluoride ( example 269, step 1) and intermediate B42 as a white solid. MS (ESI): m/z= 479.0 [M+H]+

### Example 271: 6-chloro-N-[5-(difluoromethoxy)-4,6-dimethoxy-pyrimidin-2-yl]thieno[2,3-b]pyridine-3-sulfonamide

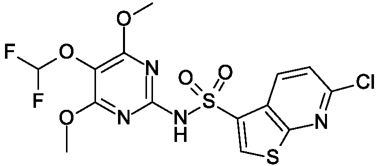

The title compound was prepared in analogy to Example 269 from 6-chlorothieno[2,3-b]pyridine-3-sulfonyl fluoride (obtained from the corresponding sulfonylchloride intermediate A10 in analogy to example 269, step 1) and intermediate B55 as a white solid. MS (ESI): m/z= 453.1 [M+H]+

### Example 272: N-[5-(2,2-difluoroethyl)-4-methoxy-pyrimidin-2-yl]-6-(oxetan-3-yl)-1H-indole-3-sulfonamide

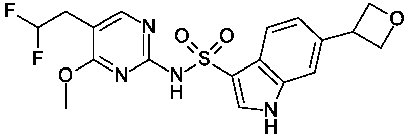

The corresponding volume of a stock solution of [4,4'-bis(1,1-dimethylethyl)-2,2'-bipyridine-n1,n1']bis[3,5-difluoro-2-[5-(trifluoromethyl)-2-pyridinyl-n]phenyl-c]iridium(III) hexafluorophosphate (1.22 mg, 0.001 mmol, CAS: 870987-63-6) in dichloromethane was added to a reaction vial and the solvent was evaporated. Then, sodium carbonate (23.08 mg, 0.218 mmol), 6-bromo-N-[5-(2,2-difluoroethyl)-4-methoxy-pyrimidin-2-yl]-1H-indole-3-sulfonamide (48.7 mg, 0.109 mmol, example 88), 3-bromooxetane (19.39 mg, 11.75 uL, 0.142 mmol), tris(trimethylsilyl)silane (36.28 mg, 45.01 uL, 0.146 mmol) and ethylene glycol dimethyl ether, extra dry (1.76 mL) were added. The reaction mixture was degassed with bubbling argon through for 2 min. Subsequently, the corresponding volume of a stock solution of 4,4'-di-tert-butyl-2,2'-dipyridyl (146.13 ug, 5.444E-04 mmol) and nickel(II) chloride ethylene glycol dimethyl (119.62 ug, 5.444E-04 mmol) in ethylene glycol dimethyl ether was added after sonication of the pastel green suspension. Finally, the mixture was again degassed by bubbling argon through for 5 min. The mixture was placed in the photoreactor, irradiated with 450 nm for 14 hours (stirring at 900 rpm, 100 % LED power) and concentrated in vacuo. The residue was purified by flash chromatography over silica gel using a gradient ethylacetate/heptane 0-100%, followed by preparative HPLC to provide the title compound as a white solid (8 mg, 17 % yield). MS (ESI): m/z= 425.1 [M+H]+

### Example 273: N-(4,6-dimethoxy-5-methyl-pyrimidin-2-yl)-6-methoxy-1H-indole-3-sulfonamide

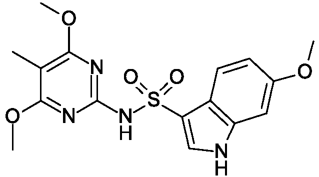

### Step 1: 1-(benzenesulfonyl)-6-methoxy-indole-3-sulfonyl chloride

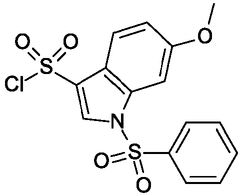

The title compound is known and has been prepared following procedures described in WO2018122232 from 6-methoxyindole (CAS: 3189-13-7).

### Step 2: 1-(benzenesulfonyl)-N-(4,6-dimethoxy-5-methyl-pyrimidin-2-yl)-6-methoxy-indole-3-sulfonamide

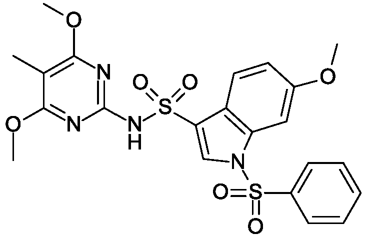

To a mixture of (4,6-dimethoxy-5-methyl-pyrimidin-2-yl)amine (100 mg, 0.591 mmol, intermediate B19) and 1-(benzenesulfonyl)-6-methoxy-indole-3-sulfonyl chloride (273.68 mg, 0.709 mmol) was added pyridine (2.5 mL). The reaction mixture was stirred at room temperature for 20 hours and concentrated in vacuo. The residue was purified by flash chromatography over silica gel using a gradient ethylacetate/heptane 0-40% to provide the title compound as an off-white solid (82 mg, 23 % yield). MS (ESI): m/z= 519.3 [M+H]+

### Step 3: N-(4,6-dimethoxy-5-methyl-pyrimidin-2-yl)-6-methoxy-1H-indole-3-sulfonamide

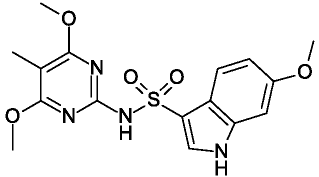

A mixture of 1-(benzenesulfonyl)-N-(4,6-dimethoxy-5-methyl-pyrimidin-2-yl)-6-methoxyindole-3-sulfonamide (80 mg, 0.131 mmol) and a 2 M solution of sodium carbonate (295.05 uL, 0.590 mmol) in methanol (2 mL) was stirred at 50 °C for 16 hours and at 60 °C for 3 hours. The reaction mixture was poured into water and extracted twice with EtOAc. The organic layers were dried over sodium sulfate, filtered and concentrated in vacuo. The residue was purified by flash chromatography over silica gel using a gradient ethylacetate/heptane 0-50% to provide the title compound as a white solid (3.6 mg, 7 % yield). MS (ESI): m/z= 379.2 [M+H]+

### Example 274: N-[5-(2,2-difluoroethyl)-4-methoxy-pyrimidin-2-yl]-6-(1-hydroxy-1-methylethyl)-1H-indole-3-sulfonamide

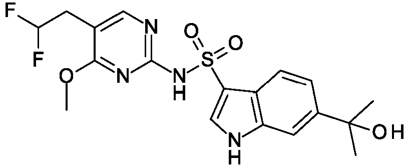

### Step 1: methyl 3-chlorosulfonyl-1H-indole-6-carboxylate

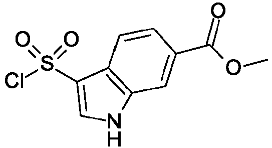

A solution of 1H-indole-6-carboxylic acid methyl ester (300 mg, 1.68 mmol, CAS: 50820-65-0) in acetonitrile (1.5 mL) was cooled to 0 °C. Chlorosulfonic acid (518.83 mg, 296.47 uL, 4.36 mmol) was added dropwise. The reaction mixture was stirred at 0 °C for 30 min, poured into ice/ethyl acetate and extracted twice with ethyl acetate. The organic layers were dried over sodium sulfate, filtered and concentrated in vacuo to provide the title compound as a light red solid (354 mg, 73 % yield). MS (ESI): m/z= 271.9 [M-H]⁻

### Step 2: methyl 3-[[5-(2,2-difluoroethyl)-4-methoxy-pyrimidin-2-ylisulfamoyl]-1H-indole-6-carboxylate

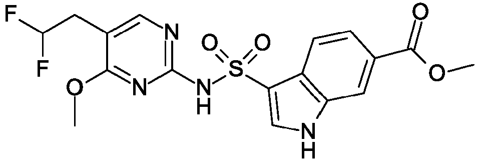

To a stirred solution of 5-(2,2-difluoroethyl)-4-methoxy-pyrimidin-2-amine (50 mg, 0.264 mmol, intermediate B40) and n-ethyldiisopropylamine (69.72 mg, 92.1 uL, 0.529 mmol) in dichloromethane (1 mL) was added methyl 3-chlorosulfonyl-1H-indole-6-carboxylate (114.23 mg, 0.396 mmol) portionwise at room temperature. The reaction mixture was stirred at room temperature for 10 min and concentrated in vacuo. The residue was purified by flash chromatography over silica gel using a gradient ethylacetate/heptane 0-100% to provide the title compound as a light yellow solid (14 mg, 11 % yield). MS (ESI): m/z= 427.1 [M+H]+

### Step 3: N-[5-(2,2-difluoroethyl)-4-methoxy-pyrimidin-2-yl]-6-(1-hydroxy-1-methyl-ethyl)-1H-indole-3-sulfonamide

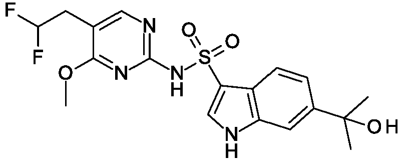

A solution of methyl 3-[[5-(2,2-difluoroethyl)-4-methoxy-pyrimidin-2-yl]sulfamoyl]-1H-indole-6-carboxylate (12 mg, 0.024 mmol) in tetrahydrofuran (200 uL) was cooled to 0 °C. 3 M methylmagnesium bromide in diethylether (41.75 mg, 40.34 uL, 0.121 mmol) was added dropwise. The reaction mixture was stirred at 0 °C for 30 min and at room temperature for 1 hour. 3 M methylmagnesium bromide in diethylether (41.75 mg, 40.34 uL, 0.121 mmol) was added dropwise and the stirring was continued at room temperature for 1 hour. The reaction mixture was quenched with a saturated solution of ammonium chloride and extracted twice with ethyl acetate. The organic layers were dried over sodium sulfate, filtered and concentrated in vacuo. The residue was purified by flash chromatography over silica gel using a gradient ethylacetate/heptane 0-100% to provide the title compound as an off-white solid (5.4 mg, 46 % yield). MS (ESI): m/z= 427.2 [M+H]+

### Example 275: 6-chloro-N-[5-(1,1-difluoro-2-hydroxy-ethoxy)-4,6-dimethoxy-pyrimidin-2-yl]-1H-indole-3-sulfonamide

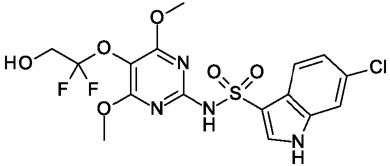

### Step 1: ethyl 2-(2-amino-4,6-dimethoxy-pyrimidin-5-yl)oxy-2,2-difluoro-acetate

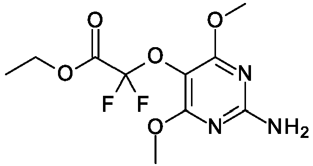

To a stirred solution of ethyl 2-[2-[bis[(4-methoxyphenyl)methyl]amino]-4,6-dimethoxy-pyrimidin-5-yl]oxy-2,2-difluoro-acetate (100 mg, 0.148 mmol, intermediate B58, step 1) in dichloromethane (100 uL) was added trifluoroacetic acid (1.01 g, 679.88 uL, 8.88 mmol). The reaction mixture was stirred at room temperature for 15 hours, at 60 °C for 7 hours and concentrated in vacuo. The residue was poured into sat. NaHCO3 and extracted twice with ethyl acetate. The organic layers were dried over sodium sulfate, filtered and concentrated in vacuo. The residue was purified by flash chromatography over silica gel using a gradient ethylacetate/heptane 0-50% to provide the title compound as a white solid (45 mg, 90 % yield). MS (ESI): m/z= 294.2 [M+H]+

### Step 2: ethyl 2-[2-[(6-chloro-1H-indol-3-y])sulfonylamino]-4.6-dimethoxy-pyrimidin-5-yl]oxy-2,2-difluoro-acetate

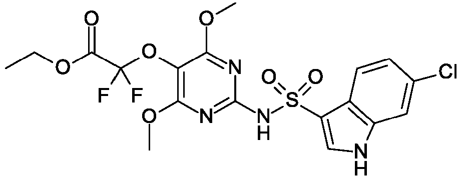

To a stirred solution of ethyl 2-(2-amino-4,6-dimethoxy-pyrimidin-5-yl)oxy-2,2-difluoro-acetate (39 mg, 0.116 mmol) and n,n-diisopropylethylamine (22.66 mg, 29.97 uL, 0.174 mmol) in dichloromethane (1.2 mL) was added 6-chloro-1H-indole-3-sulfonyl chloride (36.56 mg, 0.139 mmol, intermediate A1). The reaction mixture was stirred at room temperature for 40 min and concentrated in vacuo. The residue was purified by flash chromatography over silica gel using a gradient ethylacetate/heptane 0-60% to provide the title compound as a white solid (8 mg, 12 % yield). MS (ESI): m/z= 507.3 [M+H]+

### Step 3: 6-chloro-N-[5-(1,1-difluoro-2-hydroxy-ethoxy)-4,6-dimethoxy-pyrimidin-2-y1]-1H-indole-3-sulfonamide

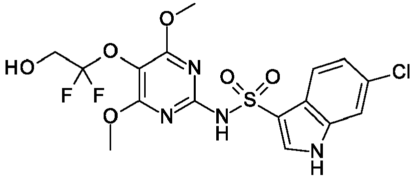

A solution of ethyl 2-[2-[(6-chloro-1H-indol-3-yl)sulfonylamino]-4,6-dimethoxy-pyrimidin-5-yl]oxy-2,2-difluoro-acetate (8 mg, 0.014 mmol) in tetrahydrofuran (200 uL) was cooled to 0 °C. 1 M lithium aluminium hydride solution in THF (21.31 uL, 0.021 mmol) was added dropwise. The reaction mixture was stirred at 0 °C for 1 hour, carefully quenched with a saturated solution of ammonium chloride and extracted twice with ethyl acetate. The organic layers were dried over sodium sulfate, filtered and concentrated in vacuo. The residue was purified by flash chromatography over silica gel using a gradient ethylacetate/heptane 0-100% to provide the title compound as a white solid (0.8 mg, 12 % yield). MS (ESI): m/z= 465.2 [M+H]+

### Example 276: 6-chloro-N-[5-(2,2-difluoroethyl)-4-methoxy-pyrimidin-2-yl]-7-methylsulfinyl-1H-indole-3-sulfonamide

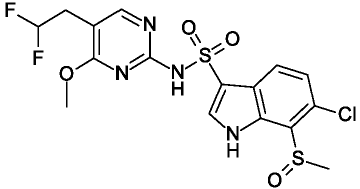

To a solution of 6-chloro-N-[5-(2,2-difluoroethyl)-4-methoxy-pyrimidin-2-yl]-7-methylsulfanyl-1H-indole-3-sulfonamide (20 mg, 0.045 mmol, example 234) in dichloromethane (300 uL) was added 3-chloroperoxybenzoic acid (11.98 mg, 0.053 mmol) at 0 °C. The reaction mixture was stirred at 0 °C for 15 min, poured into sat. NaHCO3 and extracted twice with ethyl acetate. The organic layers were dried over sodium sulfate, filtered and concentrated in vacuo. The residue was purified by flash chromatography over silica gel using a gradient ethylacetate/heptane 0-100% to provide the title compound as a white solid (10 mg, 47 % yield). MS (ESI): m/z= 465.1 [M+H]+

### Example 277: 6-chloro-N-[5-(2,2-difluoroethyl)-4-methoxy-pyrimidin-2-yl]-7-mesyl-1H-indole-3-sulfonamide

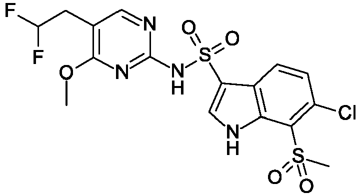

To a solution of 6-chloro-N-[5-(2,2-difluoroethyl)-4-methoxy-pyrimidin-2-yl]-7-methylsulfinyl-1H-indole-3-sulfonamide (5 mg, 0.011 mmol, example 276) in dichloromethane (81.36 uL) was added 3-chloroperoxybenzoic acid (4.64 mg, 0.027 mmol) at 0 °C. The reaction mixture was allowed to warm to room temperature and stirred for 1 hour. 3-chloroperoxybenzoic acid (4.64 mg, 0.027 mmol) was added again and the stirring was continued at room temperature for 1 hour. The reaction mixture was poured into sat. NaHCO3 and extracted twice with ethyl acetate. The organic layers were dried over sodium sulfate, filtered and concentrated in vacuo. The residue was purified by flash chromatography over silica gel using a gradient ethylacetate/heptane 0-100% to provide the title compound as a white solid (3 mg, 53 % yield). MS (ESI): m/z= 481.2 [M+H]+

### Example 278: 6-chloro-N-[5-(2-fluoroethoxy)-4,6-dimethoxy-pyrimidin-2-yl]-7-methylsulfinyl-1H-indole-3-sulfonamide

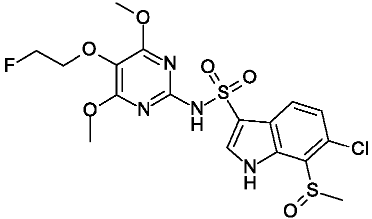

### Step 1: 6-chloro-N-[5-(2-fluoroethoxy)-4,6-dimethoxy-pyrimidin-2-yl]-7-methylsulfanyl-1H-indole-3-sulfonamide

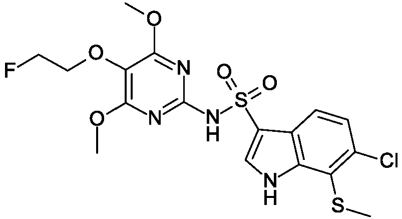

To a solution of 6-chloro-7-methylsulfanyl-1H-indole-3-sulfonyl chloride (400 mg, 0.810 mmol, intermediate A32) in acetonitrile (5 mL) were added 5-(2-fluoroethoxy)-4,6-dimethoxy-pyrimidin-2-amine (125.71 mg, 0.579 mmol, intermediate B56) and potassium phosphate tribasic (245.71 mg, 1.16 mmol). The reaction mixture was stirred at room temperature for 20 hours, diluted with water and extracted with ethyl acetate. The organic layers were washed with brine, dried over sodium sulfate, filtered and concentrated in vacuo. The residue was purified by flash chromatography over silica gel using a gradient ethylacetate/heptane 0-65% to provide the title compound as a light brown solid (229 mg, 56 % yield). MS (ESI): m/z= 477.1 [M+H]+

### Step 2: 6-chloro-N-[5-(2-fluoroethoxy)-4,6-dimethoxy-pyrimidin-2-yl]-7-methylsulfinyl-1H-indole-3-sulfonamide

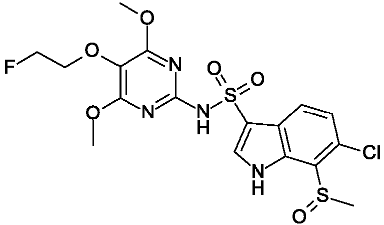

To a solution of 6-chloro-N-[5-(2-fluoroethoxy)-4,6-dimethoxy-pyrimidin-2-yl]-7-methylsulfanyl-1H-indole-3-sulfonamide (30 mg, 0.060 mmol) in dichloromethane (500 uL) was added 3-chloroperoxybenzoic acid (16.21 mg, 0.066 mmol) at 0 °C. The reaction mixture was stirred at room temperature for 15 hours, quenched with sat. NaHCO3 and extracted with ethyl acetate. The organic layers were washed with brine, dried over sodium sulfate, filtered and concentrated in vacuo. The residue was purified by RP flash chromatography over silica gel using a gradient acetonitrile/water 0-60% to provide the title compound as a light brown solid (26.2 mg, 85 % yield). MS (ESI): m/z= 493.1 [M+H]+

### Example 279: 6-chloro-N-[5-(2,2-difluoroethyl)-4,6-dimethoxy-pyrimidin-2-yl]-7-methylsulfinyl-1H-indole-3-sulfonamide

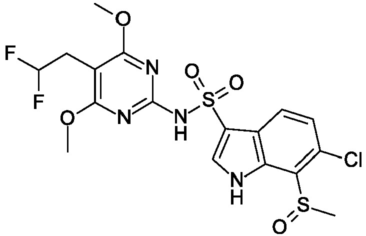

### Step 1: 6-chloro-N-[5-(2,2-difluoroethyl)-4,6-dimethoxy-pyrimidin-2-yl]-7-methylsulfanyl-1H-indole-3-sulfonamide

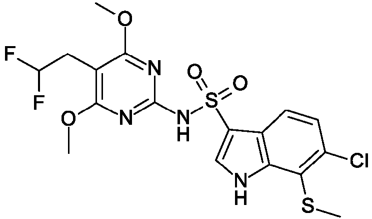

To a solution of 6-chloro-7-methylsulfanyl-1H-indole-3-sulfonyl chloride (400 mg, 0.810 mmol, intermediate A32) in acetonitrile (3.57 mL) were added 5-(2-fluoroethoxy)-4,6-dimethoxy-pyrimidin-2-amine (126.86 mg, 0.579 mmol, intermediate B63) and potassium phosphate tribasic (245.71 mg, 1.16 mmol). The reaction mixture was stirred at room temperature for 20 hours, diluted with water and extracted with ethyl acetate. The organic layers were washed with brine, dried over sodium sulfate, filtered and concentrated in vacuo. The residue was purified by flash chromatography over silica gel using a gradient ethylacetate/heptane 0-65% to provide the title compound as a light brown solid (138 mg, 47 % yield). MS (ESI): m/z= 479.1 [M+H]+

### Step 2: 6-chloro-N-[5-(2,2-difluoroethyl)-4,6-dimethoxy-pyrimidin-2-yl]-7-methylsulfinyl-1H-indole-3-sulfonamide

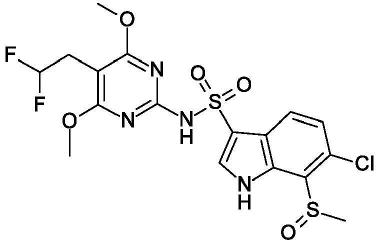

To a solution of 6-chloro-N-[5-(2,2-difluoroethyl)-4,6-dimethoxy-pyrimidin-2-yl]-7-methylsulfanyl-1H-indole-3-sulfonamide (30 mg, 0.060 mmol) in dichloromethane (475 uL) was added 3-chloroperoxybenzoic acid (16.14 mg, 0.065 mmol) at 0 °C. The reaction mixture was stirred at room temperature for 15 hours, quenched with sat. NaHCO3 and extracted with ethyl acetate. The organic layers were washed with brine, dried over sodium sulfate, filtered and concentrated in vacuo. The residue was purified by RP flash chromatography over silica gel using a gradient acetonitrile/water 0-50% to provide the title compound as a light brown solid (16 mg, 52 % yield). MS (ESI): m/z= 495.1 [M+H]+

### Example 280: 6-chloro-7-cyano-N-[5-(2-fluoroethoxy)-4-methoxy-pyrimidin-2-yl]-1H-indole-3-sulfonamide

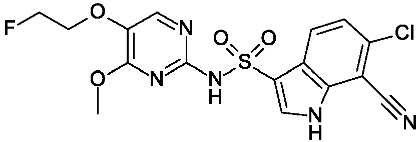

Zink cyanide (72.83 mg, 0.620 mmol), tetrakis(triphenylphosphine)palladium (0) (21.5 mg, 0.019 mmol) and 7-bromo-6-chloro-N-[5-(2-fluoroethoxy)-4-methoxy-pyrimidin-2-yl]-1H-indole-3-sulfonamide (59.5 mg, 0.124 mmol, example 141) were dissolved in N,N-dimethylformamide (0.900 mL). The mixture was stirred in a microwave at 160 °C for 20 min, diluted with brine and extracted three times with ethyl acetate. The organic layers were washed with brine, dried over sodium sulfate, filtered and concentrated in vacuo. The residue was purified by RP flash chromatography over silica gel using a gradient acetonitrile/water 0-100% to provide the title compound as a white solid (8.3 mg, 16 % yield). MS (ESI): m/z= 426.2 [M+H]+

### Example 281: 6-chloro-N-[4,6-dimethoxy-5-(2-methylsulfonylethyl)pyrimidin-2-yl]-1H-indole-3-sulfonamide

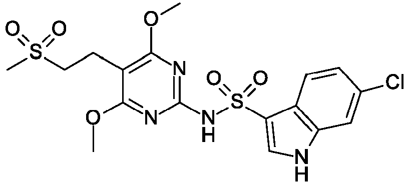

The title compound was prepared in analogy to Example 1 from intermediate A1 and intermediate B97 as a white solid. MS (ESI): m/z= 475.1 [M+H]+

### Example 282: N-[5-(2,2-difluoroethyl)-4-methoxy-pyrimidin-2-yl]-1H-pyrrolo[2,3-e][1,2]benzothiazole-3-sulfonamide

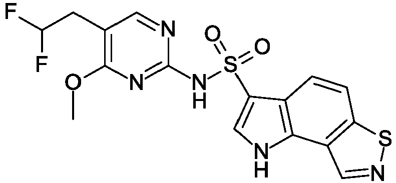

### Step 1: 7-bromo-1,2-benzothiazol-4-amine

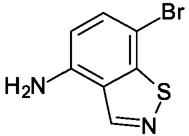

To a solution of 1,2-benzothiazol-4-amine (0.5 g, 3.33 mmol, CAS: 1547068-06-3) in N,N-dimethylformamide (10 mL) was added N-bromosuccinimide (533.29 mg, 3 mmol). The reaction mixture was stirred at 25 °C for 16 hours, diluted with water and extracted twice with ethyl acetate. The organic layers were washed twice with brine, dried over sodium sulfate, filtered and concentrated in vacuo to provide the title compound as a yellow solid (760 mg, 100 % yield). 1H NMR (400 MHz, CHLOROFORM-d) δ = 9.32 (s, 1H), 7.40 (d, J = 8.0 Hz, 1H), 6.58 - 6.47 (m, 3H)

### Step 2: 7-bromo-5-iodo-1,2-benzothiazol-4-amine

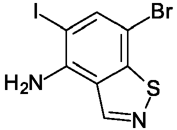

To a solution of 7-bromo-1,2-benzothiazol-4-amine (1.2 g, 5.24 mmol) in N,N-dimethylformamide (20 mL) was added N-iodosuccinimide (1.18 g, 5.24 mmol). The reaction mixture was stirred at 25 °C for 16 hours, poured into water and extracted twice with ethyl acetate. The organic layers were washed twice with brine, dried over sodium sulfate, filtered and concentrated in vacuo. The residue was purified by flash chromatography over silica gel using a gradient ethylacetate/petroleum ether 0-30% to provide the title compound as a brown solid (1.7 g, 91 % yield). NMR (400 MHz, DMSO-d6) δ = 9.44 (s, 1H), 7.83 (s, 1H), 6.57 (br s, 2H)

### Step 3: (5-bromo-1H-pyrrolo[2,3-e][1,2]benzothiazol-2-yl)-trimethyl-silane

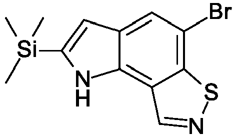

To a mixture of trimethylsilylacetylene (0.48 mL, 3.38 mmol), 7-bromo-5-iodo-1,2-benzothiazol-4-amine (1.0 g, 2.82 mmol), and potassium carbonate (777.49 mg, 5.63 mmol) in tetrahydrofuran (20 mL) was added XPhos (268.74 mg, 0.560 mmol) and tetrakis(triphenylphosphine)palladium(0) (325.36 mg, 0.280 mmol) under nitrogen. The reaction mixture was stirred at 25 °C for 16 hours and concentrated in vacuo. The residue was purified by flash chromatography over silica gel using a gradient ethylacetate/petroleum ether 0-50% to provide the title compound as a yellow solid (650 mg, 71 % yield). NMR (400 MHz, DMSO-d6) δ = 12.11 (br s, 1H), 9.54 (s, 1H), 8.06 (s, 1H), 6.86 (d, J = 2.0 Hz, 1H), 0.37 (s, 9H)

### Step 4: 5-bromo-2-trimethylsilyl-1H-pyrrolo[2,3-e][1,2]benzothiazole-3-sulfonyl chloride

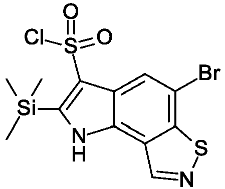

To a solution of (5-bromo-1H-pyrrolo[2,3-e][1,2]benzothiazol-2-yl)-trimethyl-silane (450.0 mg, 1.38 mmol) in acetonitrile (10 mL) was added chlorosulfonic acid (0.92 mL, 13.83 mmol) at 25 °C. The reaction mixture was stirred at 25 °C for 16 hours, diluted with water and extracted twice with ethyl acetate. The organic layers were washed twice with brine, dried over sodium sulfate, filtered and concentrated in vacuo to provide the title compound as a yellow solid (480 mg, 99 % yield). MS (ESI): m/z= 425.0 [M+H]+

### Step 5: 5-bromo-N-[5-(2,2-difluoroethyl)-4-methoxy-pyrimidin-2-yl]-2-trimethylsilyl-1H-pyrrolo[2,3-e][1,2]benzothiazole-3-sulfonamide

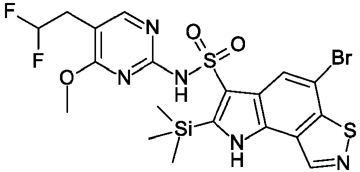

To a solution of 5-bromo-2-trimethylsilyl-1H-pyrrolo[2,3-e][1,2]benzothiazole-3-sulfonyl chloride (480.0 mg, 1.13 mmol) and potassium phosphate tribasic (448.3 mg, 2.11 mmol) in acetonitrile (10 mL) was added 5-(2,2-difluoroethyl)-4-methoxy-pyrimidin-2-amine (100.0 mg, 0.530 mmol, intermediate B40). The reaction mixture was stirred at 25 °C for 16 hours and concentrated in vacuo. The residue was diluted with water and extracted twice with ethyl acetate. The organic layers were washed twice with brine, dried over sodium sulfate, filtered and concentrated in vacuo. The residue was purified by preparative HPLC to provide the title compound as a yellow solid (70 mg, 23 % yield). MS (ESI) m/z: 578.1 [M+H]+

### Step 6: 5-bromo-N-[5-(2,2-difluoroethyl)-4-methoxy-pyrimidin-2-yl]-1H-pyrrolo[2,3-e][1,2]benzothiazole-3-sulfonamide

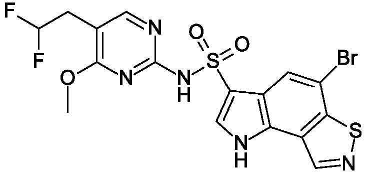

To a solution of 5-bromo-N-[5-(2,2-difluoroethyl)-4-methoxy-pyrimidin-2-yl]-2-trimethylsilyl-1H-pyrrolo[2,3-e][1,2]benzothiazole-3-sulfonamide (70.0 mg, 0.120 mmol) in N,N-dimethylformamide (2 mL) was added cesium fluoride (92.28 mg, 0.610 mmol). The reaction mixture was stirred at 60 °C for 1 hour, diluted with water and extracted twice with ethyl acetate. The organic layers were washed twice with brine, dried over sodium sulfate, filtered and and concentrated in vacuo. The residue was purified by flash chromatography over silica gel using a gradient ethylacetate/petroleum ether 0-50% to provide the title compound as a white solid (50 mg, 82 % yield). MS (ESI): m/z= 506.0 [M+H]+

### Step 7: N-[5-(2,2-difluoroethyl)-4-methoxy-pyrimidin-2-yl]-1H-pyrrolo[2,3-e][1,2]benzothiazole-3-sulfonamide

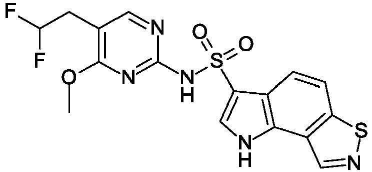

To a solution of 5-bromo-N-[5-(2,2-difluoroethyl)-4-methoxy-pyrimidin-2-yl]-1H-pyrrolo[2,3-e][1,2]benzothiazole-3-sulfonamide (40.0 mg, 0.080 mmol) in tetrahydrofuran (2 mL) and N,N-dimethylformamide (2 mL) was added palladium on activated charcoal (40.0 mg) and palladium hydroxide (40.0 mg). The reaction mixture was stirred under 1 atm. of hydrogen at 30 °C for 16 hours. The mixture was filtered through a pad of celite and washed three times with THF/DMF (1/1; 1 mL). To the filtrate was added palladium on activated charcoal (40.0 mg) and palladium hydroxide (40.0 mg). The reaction mixture was stirred under 1 atm. of hydrogen at 30 °C for 24 hours. Two more times was the reaction filtered, palladium on activated charcoal (40.0 mg) and palladium hydroxide (40.0 mg) were added, and the mixture stirred under 1 atm. of hydrogen at 30 °C for 24 hours. The reaction mixture was filtered through a pad of celite, washed three times with methanol and concentrated in vacuo. The residue was purified by preparative HPLC to provide the title compound as an off-white solid (11.5 mg, 32 % yield). MS (ESI) m/z: 426.1 [M+H]+

### Example 283: 7-bromo-6-chloro-N-[5-(2,2-difluoroethyl)-4,6-dimethoxy-pyrimidin-2-yl]-1H-indole-3-sulfonamide

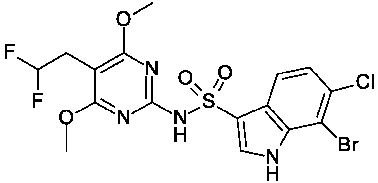

The title compound was prepared in analogy to Example 1 from intermediate A13 and intermediate B63 as an off-white solid. MS (ESI): m/z= 512.9 [M+H]+

### Example 284: N-[5-(cyanomethoxy)-4,6-dimethoxy-pyrimidin-2-yl]-6-methyl-1H-pyrrolo[2,3-b]pyridine-3-sulfonamide

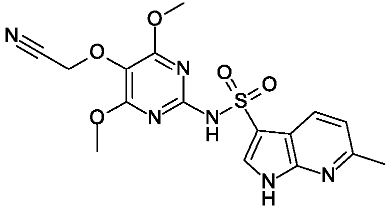

The title compound was prepared in analogy to Example 1 from intermediate A47 and intermediate B89 as a white lyoph powder. MS (ESI): m/z= 405.2 [M+H]+

### Example 285: N-[5-(2,2-difluoroethoxy)-4,6-dimethoxy-pyrimidin-2-yl]-6-methyl-1H-pyrrolo[2,3-b]pyridine-3-sulfonamide

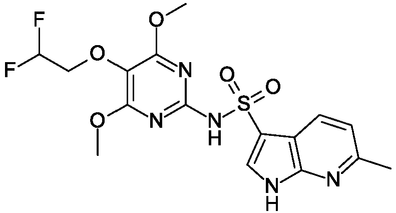

The title compound was prepared in analogy to Example 1 from intermediate A47 and intermediate B57 as a white lyoph powder. MS (ESI): m/z= 430.1 [M+H]+

### Example 286: N-[5-(difluoromethoxy)-4,6-dimethoxy-pyrimidin-2-yl]-6-methyl-1H-pyrrolo[2,3-b]pyridine-3-sulfonamide

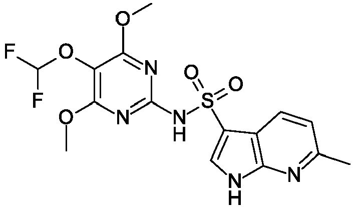

The title compound was prepared in analogy to Example 1 from intermediate A47 and intermediate B55 as a white lyoph powder. MS (ESI): m/z= 416.1 [M+H]+

### Example 287: 6-(difluoromethyl)-N-[5-(2,3-difluoropropyl)-4,6-dimethoxy-pyrimidin-2-yl]-1H-pyrrolo[2,3-b]pyridine-3-sulfonamide

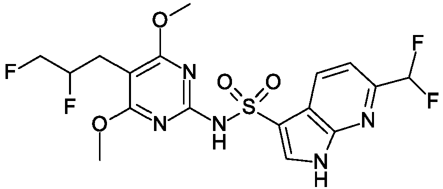

The title compound was prepared in analogy to Example 1 from intermediate A26 and intermediate B88 as a white solid. MS (ESI): m/z= 464.1 [M+H]+

### Example 288: 6-bromo-N-[5-(2,3-difluoropropyl)-4,6-dimethoxy-pyrimidin-2-yl]-1H-pyrrolo[2,3-b]pyridine-3-sulfonamide

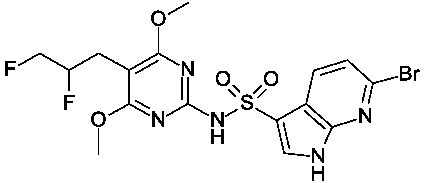

The title compound was prepared in analogy to Example 172 from intermediate A4 and intermediate B88 as a white solid. MS (ESI): m/z= 492.0 [M+H]+

### Example 289: 6-chloro-N-[5-(2,3-difluoropropyl)-4,6-dimethoxy-pyrimidin-2-yl]-1H-pyrrolo[2,3-b]pyridine-3-sulfonamide

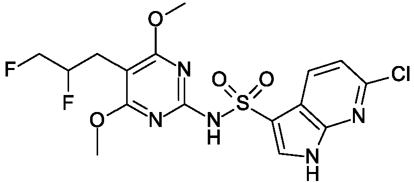

The title compound was prepared in analogy to Example 1 from intermediate A5 and intermediate B88 as a white solid. MS (ESI): m/z= 448.0 [M+H]+

### Example 290: N-[5-(2,2-difluoroethyl)-4-methoxy-pyrimidin-2-yl]-8H-pyrrolo[2,3-e][1,3]benzothiazole-6-sulfonamide

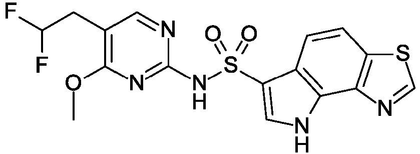

### Step 1: 7-bromo-1,3-benzothiazol-4-amine

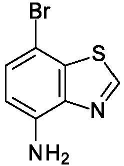

To a solution of 1,3-benzothiazol-4-amine (1.0 g, 6.66 mmol, CAS: 1123-51-9) in N,N-dimethylformamide (30 mL) was added N-bromosuccinimide (0.34 mL, 5.99 mmol). The reaction mixture was stirred at 25 °C for 2 hours, diluted with water and extracted three times with ethyl acetate. The organic layers were dried over sodium sulfate, filtered and and concentrated in vacuo. The residue was purified by flash chromatography over silica gel using a gradient ethylacetate/petroleum ether 0-20% to provide the title compound as a light yellow solid (1.3 g, 85 % yield). MS (ESI): m/z= 230.9 [M+H]+

### Step 2: 7-bromo-5-iodo-1,3-benzothiazol-4-amine

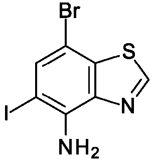

To a solution of 7-bromo-1,3-benzothiazol-4-amine (1.3 g, 5.67 mmol) in N,N-dimethylformamide (40 mL) was added N-iodosuccinimide (1276.62 mg, 5.67 mmol). The reaction mixture was stirred at 15 °C for 2 hours, diluted with water and extracted three times with ethyl acetate. The organic layers were washed with brine, dried over sodium sulfate, filtered and and concentrated in vacuo. The residue was purified by flash chromatography over silica gel using a gradient ethylacetate/petroleum ether 0-20% to provide the title compound as a yellow solid (2 g, 99 % yield). MS (ESI): m/z= 356.9 [M+H]+

### Step 3: (4-bromo-8H-pyrrolo[2,3-e][1,3]benzothiazol-7-yl)-trimethyl-silane

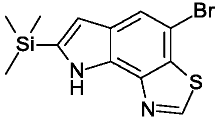

To a solution of trimethylsilylacetylene (0.96 mL, 6.76 mmol) and 7-bromo-5-iodo-1,3-benzothiazol-4-amine (2000.0 mg, 5.63 mmol) in tetrahydrofuran (50 mL) was added potassium carbonate (1554.97 mg, 11.27 mmol), XPhos (537.48 mg, 1.13 mmol) and tetrakis(triphenylphosphine)palladium(0) (650.72 mg, 0.560 mmol). The reaction mixture was stirred at 75 °C under nitrogen for 16 hours, filtered and concentrated in vacuo. The residue was purified by flash chromatography over silica gel using a gradient ethylacetate/petroleum ether 5-15%, followed by preparative HPLC to provide the title compound as a yellow solid (1000 mg, 55 % yield). MS (ESI): m/z= 327.0 [M+H]+

### Step 4: trimethyl(8H-pyrrolo[2,3-e][1,3]benzothiazol-7-yl)silane

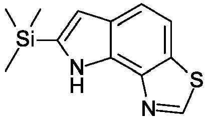

To a solution of (4-bromo-8H-pyrrolo[2,3-e][1,3]benzothiazol-7-yl)-trimethyl-silane (800.0 mg, 2.46 mmol) in tetrahydrofuran (16 mL) and N,N-dimethylformamide (16 mL) was added palladium on activated charcoal (400.0 mg) and palladium hydroxide (400.0 mg). The reaction mixture was stirred under 1 atm. of hydrogen at 30 °C for 16 hours. The mixture was filtered through a pad of celite and washed twice times with THF/DMF (1/1; 10 mL). To the filtrate was added palladium on activated charcoal (400.0 mg) and palladium hydroxide (400.0 mg). The reaction mixture was stirred under 1 atm. of hydrogen at 30 °C for 16 hours. One more time was the reaction filtered, palladium on activated charcoal (400.0 mg) and palladium hydroxide (400.0 mg) were added, and the mixture stirred under 1 atm. of hydrogen at 30 °C for 16 hours. The reaction mixture was filtered through a pad of celite, washed three times with THF/DMF (1/1; 10 mL) and concentrated in vacuo. The residue was purified by preparative HPLC to provide the title compound as a yellow gum (220 mg, 35 % yield). MS (ESI) m/z: 247.0 [M+H]+

### Step 5: 7-trimethylsilyl-8H-pyrrolo[2,3-e][1,3]benzothiazole-6-sulfonyl chloride

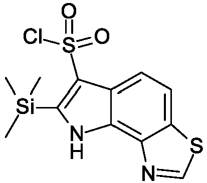

To a solution of trimethyl(8H-pyrrolo[2,3-e][1,3]benzothiazol-7-yl)silane (170.0 mg, 0.690 mmol) in acetonitrile (4 mL) was added chlorosulfonic acid (0.46 mL, 6.9 mmol) at 0 °C. The reaction mixture was warmed to 15 °C, stirred for 16 hours, poured into water and extracted with ethyl acetate. The organic layer was washed with brine, dried over sodium sulfate, filtered and and concentrated in vacuo to provide the title compound as a yellow solid (200 mg, 84 % yield). NMR (400 MHz, DMSO-d6) δ = 11.30 (s, 1H), 9.45 (s, 1H), 8.07 (d, J = 8.7 Hz, 1H), 7.72 (d, J = 8.7 Hz, 1H), 0.43 (s, 9H)

### Step 6: N-[5-(2,2-difluoroethyl)-4-methoxy-pyrimidin-2-yl]-7-trimethylsilyl-8H-pyrrolo[2,3-e][1,3]benzothiazole-6-sulfonamide

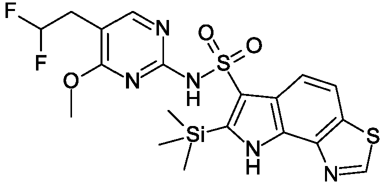

To a solution of 5-(2,2-difluoroethyl)-4-methoxy-pyrimidin-2-amine (70.0 mg, 0.370 mmol, intermediaire B40) and 7-trimethylsilyl-8H-pyrrolo[2,3-e][1,3]benzothiazole-6-sulfonyl chloride (191.45 mg, 0.560 mmol) in dichloromethane (3 mL) was added n-ethyldiisopropylamine (143.48 mg, 1.11 mmol) at 0 °C. The reaction mixture was warmed to 15 °C, stirred for 3 hours and concentrated in vacuo. The residue was purified by preparative HPLC to provide the title compound as a white solid (35 mg, 19 % yield). MS (ESI): m/z= 498.0 [M+H]+

### Step 7: N-[5-(2,2-difluoroethyl)-4-methoxy-pyrimidin-2-yl]-8H-pyrrolo[2,3-e][1,3]benzothiazole-6-sulfonamide

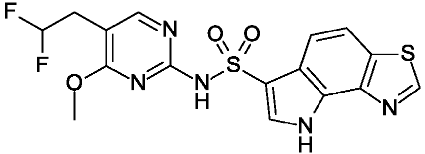

To a solution of N-[5-(2,2-difluoroethyl)-4-methoxy-pyrimidin-2-yl]-7-trimethylsilyl-8H-pyrrolo[2,3-e][1,3]benzothiazole-6-sulfonamide (30.0 mg, 0.060 mmol) in N,N-dimethylformamide (2 mL) was added cesium fluoride (45.79 mg, 0.300 mmol). The reaction mixture was warmed to 60 °C, stirred for 1 hour, filtered and concentrated in vacuo. The residue was purified by preparative HPLC to provide the title compound as a white solid (20.6 mg, 78 % yield). MS (ESI): m/z= 426.0 [M+H]+

### Example 291: 3-[[5-(2,2-difluoroethyl)-4-methoxy-pyrimidin-2-yl]sulfamoyl]-6-methyl-1H-indole-7-carboxylic acid methyl ester

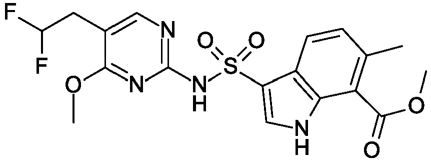

The title compound was prepared in analogy to Example 213 from intermediate A52 and intermediate B40 as an off-white solid. MS (ESI): m/z= 441.1 [M+H]+

### Example 292: N-[5-(2,2-difluoroethyl)-4-methoxy-pyrimidin-2-yl]-6-methyl-7-methylol-1H-indole-3-sulfonamide

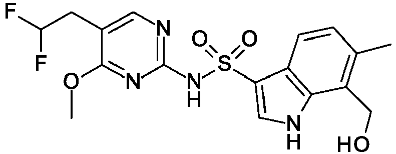

A suspension of 3-[[5-(2,2-difluoroethyl)-4-methoxy-pyrimidin-2-yl]sulfamoyl]-6-methyl-1H-indole-7-carboxylic acid methyl ester (35 mg, 0.079 mmol) in tetrahydrofuran (1 mL) was cooled to 0 °C. 1 M lithium aluminium hydride solution in THF (119.2 uL, 0.119 mmol) was added dropwise. The reaction mixture was stirred at 0 °C for 30 min. LC/MS indicated 15% sm left. The stirring was continued at 0 °C for 30 min. The reaction mixture was carefully quenched with a saturated solution of ammonium chloride, poured into water and extracted twice with ethyl acetate. The organic layers were washed twice with brine, dried over sodium sulfate, filtered and concentrated in vacuo to provide the title compound as a white solid (31 mg, 90 % yield). MS (ESI) m/z: 413.1 [M+H]+

### Example 293: N-[5-(2-fluoroethoxy)-4-methoxy-pyrimidin-2-yl]-9-keto-8-methyl-6,7-dihydro-1H-pyrrol[3,2-h]isoquinoline-3-sulfonamide

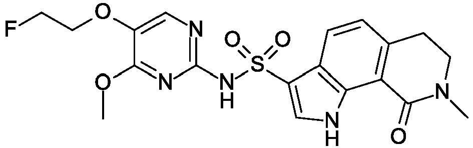

The title compound was prepared in analogy to Example 24 from intermediate A53 and intermediate B3 as a white solid. MS (ESI): m/z= 450.1 [M+H]+

### Example 294: 6-chloro-7-(cyanomethoxy)-N-[5-(2,2-difluoroethyl)-4,6-dimethoxy-pyrimidin-2-yl]-1H-indole-3-sulfonamide

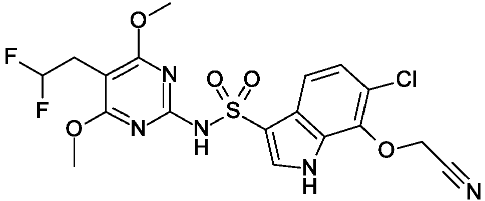

The title compound was prepared in analogy to Example 213 from intermediate A54 and intermediate B63 as a white solid. MS (ESI): m/z= 488.0 [M+H]+

### Reference Example RE-A:

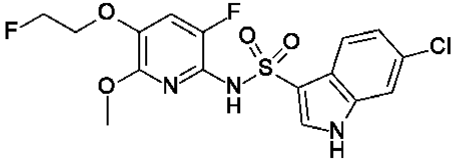

RE-A was synthesized starting from the OH precursor described in WO2019243398.

To a mixture of 6-[bis(p-anisyl)amino]-2,5-difluoro-pyridin-3-ol (1.19 g, 2.96 mmol, 1 eq) and potassium carbonate (825.49 mg, 5.91 mmol, 2 eq) in N,N-dimethylformamide (8 mL) was added 1-fluoro-2-iodo-ethane (1.13 g, 528.75 uL, 6.5 mmol, 2.2 eq). The reaction mixture was heated to 80 °C for 15 min before it was poured into water and extracted twice with EtOAc. The organic layers were washed twice with brine, dried over Na2SO4, filtered and concentrated in vacuo. The crude material was purified by flash chromatography on silica gel (80 g, 0% to 30% EtOAc in Heptane in 20 min) to afford [3,6-difluoro-5-(2-fluoroethoxy)-2-pyridyl]-bis(p-anisyl)amine (65 mg, 5.08%) as light yellow gum. MS (ESI) m/z: 433.3 [M+H]+

To a solution of [3,6-difluoro-5-(2-fluoroethoxy)-2-pyridyl]-bis(p-anisyl)amine (65 mg, 0.150 mmol, 1 eq) in dichloromethane (100 uL) at 0 °C trifluoroacetic acid (967.85 mg, 650 uL, 8.4 mmol, 55.91 eq) was added. The reaction mixture was stirred at 0 °C for 30 min and at rt for 1 h. before was poured into sat. NaHCO3 and extracted twice with EtOAc. The organic layers were dried over Na2SO4, filtered and concentrated in vacuo. The crude material was purified by flash chromatography on silica gel (4 g, 0% to 50% EtOAc in Heptane in 6 min) to afford [3,6-difluoro-5-(2-fluoroethoxy)-2-pyridyl]amine (27 mg, 93.49%) as off-white solid. MS (ESI) m/z: 193 [M+H]+

A mixture of 3,6-difluoro-5-(2-fluoroethoxy)pyridin-2-amine (27 mg, 0.141 mmol, 1 eq) and sodium methylate (31.96 mg, 0.562 mmol, 4 eq) in methanol (500 uL) was stirred at 100 °C until LCMS indicated full conversion (ca. 40 h) before it was concentrated in vacuo. The residue was poured into water and extracted twice with EtOAc. The organic layers were dried over Na₂SO₄, filtered and concentrated in vacuo to afford [3-fluoro-5-(2-fluoroethoxy)-6-methoxy-2-pyridyl]amine (27 mg, 90.34%) as light brown gum. MS (ESI) m/z: 205.1 [M+H]+

To a stirred solution of [3-fluoro-5-(2-fluoroethoxy)-6-methoxy-2-pyridyl]amine (25 mg, 0.118 mmol, 1 eq) and N-ethyldiisopropylamine (23.25 mg, 30.72 uL, 0.176 mmol, 1.5 eq) in dichloromethane (700 uL) was added 6-chloro-1H-indole-3-sulfonyl chloride (40.23 mg, 0.153 mmol, 1.3 eq). The reaction mixture was stirred at rt for 10 min before it was concentrated in vacuo. The crude material was purified by flash chromatography on silica gel (12 g, 0% to 60% EtOAc in Heptane in 15 min) to afford 6-chloro-N-[3-fluoro-5-(2-fluoroethoxy)-6-methoxy-2-pyridyl]-1H-indole-3-sulfonamide (13 mg, 26.47%) as off-white solid. MS (ESI) m/z: 418.1 [M+H]+.

### Reference Example RE-B:

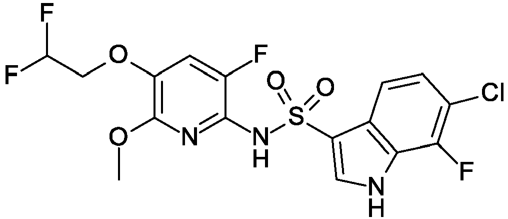

RE-B is Examples I-22 in WO2019243398 and was synthetized following the procedure described therein.

### Reference Example RE-C:

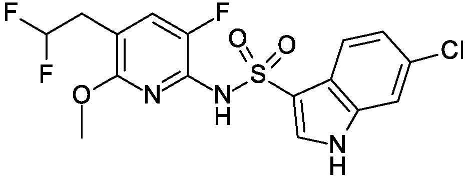

RE-C was synthesized starting from 5-bromo-3-fluoro-6-methoxy-pyridin-2-amine described in WO2018122232.

To a stirred solution of 5-bromo-3-fluoro-6-methoxy-pyridin-2-amine (200 mg, 0.905 mmol) in N,N-dimethylacetamide (3 mL) was added sodium hydride (108.57 mg, 2.71 mmol) at 0 °C. After stirring at 0 °C for 20 min, 4-methoxybenzyl chloride (289.2 mg, 251.48 uL, 1.81 mmol) was added. The reaction mixture was allowed to warm to room temperature, stirred for 30 min, carefully quenched with a saturated solution of ammonium chloride, poured into water and extracted twice with ethyl acetate. The organic layers were washed twice with brine, dried over Na2SO4, filtered and concentrated in vacuo. The residue was purified by flash chromatography over silica gel using a gradient ethylacetate/heptane 0-20% to provide 5-bromo-3-fluoro-6-methoxy-N,N-bis[(4-methoxyphenyl)methyl]pyridin-2-amine as a light yellow viscous oil (270 mg, 65 % yield). MS (ESI): m/z= 463.2 [M+H]+

A solution of 5-bromo-3-fluoro-6-methoxy-N,N-bis[(4-methoxyphenyl)methyl]pyridin-2-amine (300 mg, 0.650 mmol) in tetrahydrofuran (20 mL) was cooled to 0 °C. 1.3 M isopropylmagnesium chloride - lithium chloride complex, 1.3M solution in THF (2. mL, 2.6 mmol) was added. The ice-bath was removed and the reaction mixture was stirred at room temperature for 3 hours. The reaction mixture was cooled to 0 °C. N,N-dimethylformamide (380.25 mg, 402.81 uL, 5.2 mmol) was added. The ice-bath was removed and the reaction mixture was stirred at room temperature for 30 min, then quenched with 1 N KHSO4, poured into water and extracted twice with ethyl acetate. The organic layers were washed with brine, dried over sodium sulfate, filtered and concentrated in vacuo. The residue was purified by flash chromatography over silica gel using a gradient ethylacetate/heptane 0-30% to provide 6-[bis[(4-methoxyphenyl)methyl]amino]-5-fluoro-2-methoxy-pyridine-3-carbaldehyde as a colorless oil (205 mg, 75 % yield). MS (ESI): m/z= 411.3 [M+H]+

6-[bis[(4-methoxyphenyl)methyl]amino]-5-fluoro-2-methoxy-pyridine-3-carbaldehyde (205 mg, 0.484 mmol) was dissolved in N,N-dimethylformamide (8 mL) to give a colorless soltution. Triphenylphosphine (381.22 mg, 1.45 mmol) was added and the mixture was heated to 100 °C. Sodium chlorodifluoroacetate (221.59 mg, 1.45 mmol) was added in 3 portions of ~73 mg within 15 min. The reaction mixture was stirred at 100°C for 30 min, cooled to room temperature, then quenched with water and extracted twice with ethyl acetate. The organic layers were washed with brine, dried over sodium sulfate, filtered and concentrated in vacuo. The residue was purified by flash chromatography over silica gel using a gradient ethylacetate/heptane 0-20% to provide 5-(2,2-difluorovinyl)-3-fluoro-6-methoxy-N,N-bis[(4-methoxyphenyl)methyl]pyridin-2-amine as a colorless liquid (140 mg, 63 % yield). MS (ESI): m/z= 445.3 [M+H]+

A solution of 5-(2,2-difluorovinyl)-3-fluoro-6-methoxy-N,N-bis[(4-methoxyphenyl)methyl]pyridin-2-amine (140 mg, 0.315 mmol) in methanol (30 mL) was degassed with argon and palladium hydroxide on carbon (50 mg, 0.018 mmol) was added. The reaction mixture was stirred at room temperature under 1 atm. of hydrogen for 20 hours, filtered through a pad of celite and concentrated in vacuo to provide 5-(2,2-difluoroethyl)-3-fluoro-6-methoxy-pyridin-2-amine as a white semisolid (58 mg, 90 % yield). MS (ESI): m/z= 207.1 [M+H]+

To a solution of 5-(2,2-difluoroethyl)-3-fluoro-6-methoxy-pyridin-2-amine (58 mg, 0.281 mmol) in pyridine (1 mL) was added 6-chloro-1H-indole-3-sulfonyl chloride (77.39 mg, 0.309 mmol). The reaction mixture was stirred at room temperature for 1.5 hours and concentrated in vacuo. The residue was purified by flash chromatography over silica gel using a gradient ethylacetate/heptane 0-50%. The combined fractions were concentrated, transfered into a small flask with dichloromethane, heptane was added, then dichloromethane was evaporated. The precipitated solid was filtered and dried on high vacuo to provide 6-chloro-N-[5-(2,2-difluoroethyl)-3-fluoro-6-methoxy-2-pyridyl]-1H-indole-3-sulfonamide as a white solid (35 mg, 28 % yield). MS (ESI): m/z= 420.2 [M+H]+

### Reference Example RE-D:

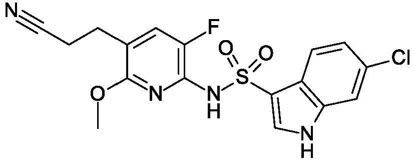

RE-D was synthesized starting from 5-bromo-3-fluoro-6-methoxy-pyridin-2-amine described in WO2018122232.

A mixture of 5-bromo-3-fluoro-6-methoxy-pyridin-2-amine (200 mg, 0.905 mmol), 3-(4,4,5,5-tetramethyl-1,3,2-dioxaborolan-2-yl)propionitrile (212.96 mg, 1.18 mmol), 1,4-dioxane (4 mL), 2 M sodium carbonate (1.36 mL, 2.71 mmol) and 1,1'-bis(diphenylphosphino)ferrocene-palladium(II)dichloride dichloromethane complex (74.81 mg, 0.090 mmol) was stirred at 90 °C for 18 hours, then poured into water and extracted twice with ethyl acetate. The organic layers were washed with brine, dried over sodium sulfate, filtered and concentrated in vacuo. The residue was purified by flash chromatography over silica gel using a gradient ethylacetate/heptane 0-50% to provide 3-(6-amino-5-fluoro-2-methoxy-3-pyridyl)propanenitrile as an off-white solid (28 mg, 12 % yield). MS (ESI): m/z= 196.1 [M+H]+

A mixture of 3-(6-amino-5-fluoro-2-methoxy-3-pyridyl)propanenitrile (28 mg, 0.143 mmol) and 6-chloro-1H-indole-3-sulfonyl chloride (39.46 mg, 0.158 mmol) in pyridine (0.500 mL) was stirred at room temperature for 2 hours, then poured into water and extracted twice with ethyl acetate. The organic layers were washed with brine, dried over sodium sulfate, filtered and concentrated in vacuo. The residue was purified by flash chromatography over silica gel using a gradient ethylacetate/heptane 0-80% to provide 6-chloro-N-[5-(2-cyanoethyl)-3-fluoro-6-methoxy-2-pyridyl]-1H-indole-3-sulfonamide as an off-white solid (11.6 mg, 19 % yield). MS (ESI): m/z= 409.1 [M+H]+

### Reference Example RE-E:

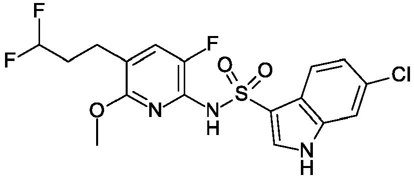

RE-D was synthesized starting from 5-bromo-3-fluoro-6-methoxy-pyridin-2-amine described in WO2018122232.

To a solution of 5-bromo-3-fluoro-6-methoxy-pyridin-2-amine (100 mg, 0.452) 1,4-dioxane (1.46 mL) was added (E)-3-(4,4,5,5-tetramethyl-1,3,2-dioxaborolan-2-yl)acrylic acid ethyl ester (132.97 mg, 131.65 uL, 0.588 mmol), cesium carbonate (442.23 mg, 1.36 mmol), 1,1'-bis(diphenylphosphino)ferrocene-palladium(II)dichloride dichloromethane complex (37.4 mg, 0.045 mmol) and water (0.146 mL). The reaction mixture was stirred at 100°C for 30 min, then poured into water and extracted with ethyl acetate. The organic layers were washed with brine, dried over sodium sulfate, filtered and concentrated in vacuo. The residue was purified by flash chromatography over silica gel using a gradient ethylacetate/heptane 0-50% to provide ethyl (E)-3-(6-amino-5-fluoro-2-methoxy-3-pyridyl)prop-2-enoate as a light brown solid (67.5 mg, 61 % yield). MS (ESI): m/z= 241.2 [M+H]+

A solution of ethyl (E)-3-(6-amino-5-fluoro-2-methoxy-3-pyridyl)prop-2-enoate (500 mg, 2.08 mmol) in methanol (10 mL) and ethyl acetate (5 mL) was degassed with argon and palladium activated charcoal (110.75 mg, 0.104 mmol) was added. The reaction mixture was stirred at room temperature under 1 atm. of hydrogen for 90 min, filtered through a pad of celite and concentrated in vacuo. The residue was purified by flash chromatography over silica gel using a gradient ethylacetate/heptane 0-15% to provide ethyl 3-(6-amino-5-fluoro-2-methoxy-3-pyridyl)propanoate as a colorless viscous oil (430 mg, 81 % yield). MS (ESI): m/z= 243.2 [M+H]+

To a solution of ethyl 3-(6-amino-5-fluoro-2-methoxy-3-pyridyl)propanoate (410 mg, 1.69 mmol ) in N,N-dimethylformamide (10 mL) at -10°C was added sodium hydride (169.25 mg, 4.23 mmol). The reaction was stirred for 30 min before 4-methoxybenzyl chloride (795.12 mg, 691.41 uL, 5.08 mmol) was added at -10°C. The reaction mixture was stirred at -10°C for 4 hours, then quenched with a saturated solution of ammonium chloride and extracted with ethyl acetate. The organic layers were dried over sodium sulfate, filtered and concentrated in vacuo. The residue was purified by flash chromatography over silica gel using a gradient ethylacetate/heptane 0-30% to provide ethyl 3-[6-[bis[(4-methoxyphenyl)methyl]amino]-5-fluoro-2-methoxy-3-pyridyl]propanoate as a light yellow viscous oil (565 mg, 59 % yield). MS (ESI): m/z= 483.3 [M+H]+

To a solution of ethyl 3-[6-[bis[(4-methoxyphenyl)methyl]amino]-5-fluoro-2-methoxy-3-pyridyl]propanoate (565 mg, 0.995 mmol) in tetrahydrofuran (10 mL) at -78 °C was added 1 M diisobutylaluminum hydride solution (1.99 mL, 1.99 mmol). The reaction was warmed to room temperature, stirred for 1 hour, cooled to -78 °C, quenched with a saturated solution of ammonium chloride and extracted with ethyl acetate. The organic layers were dried over sodium sulfate, filtered and concentrated in vacuo. The residue was purified by flash chromatography over silica gel using a gradient ethylacetate/heptane 0-60% to provide 3-[6-[bis[(4-methoxyphenyl)methyl]amino]-5-fluoro-2-methoxy-3-pyridyl]propan-1-ol as a colorless gum (350 mg, 68 % yield). MS (ESI): m/z= 441.4 [M+H]+

To a suspension of 3-[6-[bis[(4-methoxyphenyl)methyl]amino]-5-fluoro-2-methoxy-3-pyridyl]propan-1-ol (350 mg, 0.675 mmol) and sodium carbonate (143.16 mg, 1.35 mmol) in dichloromethane (10 mL) was added 1,1,1-tris(acetyloxy)-1,1-dihydro-1,2-benzodioxol-3-(1H)-one (315.09 mg, 0.743 mmol) at -10 °C. The reaction was stirred at -10°C for 3 hours, quenched with water and extracted with ethyl acetate. The organic layers were dried over sodium sulfate, filtered and concentrated in vacuo. The residue was purified by flash chromatography over silica gel using a gradient ethylacetate/heptane 0-15% to provide 3-[6-[bis[(4-methoxyphenyl)methyl]amino]-5-fluoro-2-methoxy-3-pyridyl]propanal as a colorless gum (160 mg, 51 % yield). MS (ESI): m/z= 439.4 [M+H]+

To a solution of 3-[6-[bis[(4-methoxyphenyl)methyl]amino]-5-fluoro-2-methoxy-3-pyridyl]propanal (160 mg, 0.365 mmol) in dichloromethane (5 mL) was added 2.7 M [bis(2-methoxyethyl)amino]sulfur trifluoride (356.78 mg, 297.32 uL, 0.803 mmol) at 0 °C. The reaction mixture was stirred at 0°C for 30 min, quenched with sat. NaHCO3 and extracted with ethyl acetate. The organic layers were dried over sodium sulfate, filtered and concentrated in vacuo. The residue was purified by flash chromatography over silica gel using a gradient ethylacetate/heptane 0-15% to provide 5-(3,3-difluoropropyl)-3-fluoro-6-methoxy-N,N-bis[(4-methoxyphenyl)methyl]pyridin-2-amine as a colorless gum (110 mg, 62 % yield). MS (ESI): m/z= 461.3 [M+H]+

A solution of 5-(3,3-difluoropropyl)-3-fluoro-6-methoxy-N,N-bis[(4-methoxyphenyl)methyl]pyridin-2-amine (110 mg, 0.239 mmol) in methanol (2 mL) was degassed with argon and palladium activated charcoal (12.71 mg, 0.012 mmol) was added. The reaction mixture was stirred at room temperature under 1 atm. of hydrogen for 90 min. Palladium hydroxide on carbon (30 mg, 0.011 mmol) was added. The reaction mixture was stirred at room temperature under 1 atm. of hydrogen for 3 days. Palladium hydroxide on carbon (67.1 mg, 0.024 mmol) was added. The reaction mixture was stirred at room temperature under 1 atm. of hydrogen for 2 hours, filtered through a pad of celite and concentrated in vacuo. The residue was purified by flash chromatography over silica gel using a gradient ethylacetate/heptane 0-20% to provide 5-(3,3-difluoropropyl)-3-fluoro-6-methoxy-pyridin-2-amine as a colorless gum (20 mg, 27 % yield). MS (ESI): m/z= 221.2 [M+H]+

To a solution of 5-(3,3-difluoropropyl)-3-fluoro-6-methoxy-pyridin-2-amine (20 mg, 0.064 mmol) in pyridine (0.300 mL) was added 6-chloro-1H-indole-3-sulfonyl chloride (19.08 mg, 0.076 mmol). The reaction mixture was stirred at room temperature for 1 hour and concentrated in vacuo. The residue was purified by flash chromatography over silica gel using a gradient ethylacetate/heptane 0-35%, followed by preparative HPLC to provide 6-chloro-N-[5-(3,3-difluoropropyl)-3-fluoro-6-methoxy-2-pyridyl]-1H-indole-3-sulfonamide as a colorless solid (4.5 mg, 16 % yield). MS (ESI): m/z= 434.2 [M+H]+

### Reference Example RE-F:

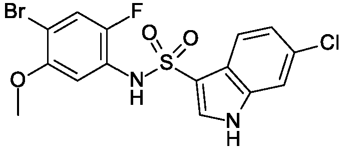

To a mixture of 4-bromo-2-fluoro-5-methoxy-aniline (30 mg, 0.136 mmol, CAS: 108310-38-9) and 6-chloro-1H-indole-3-sulfonyl chloride (46.66 mg, 0.177 mmol) was added pyridine (500 uL). The reaction mixture was stirred at room temperature for 10 min and concentrated in vacuo.

The residue was purified by preparative HPLC to provide N-(4-bromo-2-fluoro-5-methoxyphenyl)-6-chloro-1H-indole-3-sulfonamide as an off-white solid (36 mg, 61 % yield). MS (ESI): m/z= 433.0 [M-H]-

### Example A

A compound of formula I can be used in a manner known per se as the active ingredient for the production of tablets of the following composition:

### Per tablet

| | |
|---|---|
| Active ingredient | 200 mg |
| Microcrystalline cellulose | 155 mg |
| Corn starch | 25 mg |
| Talc | 25 mg |
| Hydroxypropylmethylcellulose | 20 mg |
| | 425 mg |

### Example B

A compound of formula I can be used in a manner known per se as the active ingredient for the production of capsules of the following composition:

### Per capsule

| | |
|---|---|
| Active ingredient | 100.0 mg |
| Corn starch | 20.0 mg |
| Lactose | 95.0 mg |
| Talc | 4.5 mg |
| Magnesium stearate | 0.5 mg |
| | 220.0 mg |

## Claims

1. Compounds of formula I 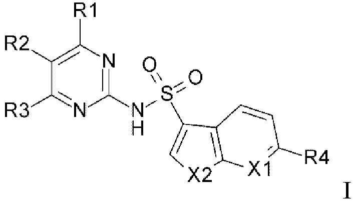 wherein
R¹ is H, halo, alkyl, alkoxy, alkoxyalkyl, alkoxyalkoxy, cyanoalkyl, haloalkyl, haloalkoxy, thiolalkyl, cycloalkyl, cycloalkylalkyl, cycloalkoxy, or heterocycloalkyl;
R² is cyano, halo, alkyl, alkenyl, alkynyl, alkoxy, alkenylalkyl, alkynylalkoxy, alkoxyalkyl, alkoxyalkoxy, haloalkyl, haloalkoxy, haloalkoxyalkyl, (haloalkoxy)alkoxy, cyanoalkyl, cyanoalkoxy, thiolalkyl, halothiolalkyl, halohydroxyalkoxy, alkylsulfonylalkyl, cycloalkyl, cycloalkylalkyl, cycloalkylalkoxy, cycloalkoxy, heterocycloalkyl, heterocycloalkylalkyl, heterocycloalkylalkynyl or heterocycloalkylalkoxy;
R³ is H, halo, alkyl, alkoxy, haloalkoxy, or thiolalkyl;
X₁ is CR⁵ or N;
X₂ is NH, -N-R⁶, S, -S(O)-, or -S(O₂)-;
R⁴ is H, halo, alkyl, hydroxyalkyl, thiolalkyl, cyanoalkyl, alkoxy, haloalkyl, haloalkoxy, cycloalkyl or heterocycloalkyl;
R⁵ is H, halo, cyano, alkyl, alkoxy, cyanoalkyl, cyanyalkoxy, N-alkyl, dialkylamino, haloalkyl, haloalkoxy, halothiolalkyl, alkylsulfinyl, alkylsulfonyl, alkylsulfone, thiolalkyl, hydroxyalkyl, alkoxycarbonyl, heterocycloalkyl, cycloalkyl or cycloalkoxy;
or R⁴ and R⁵ are connected to form a 5 or 6 member cycloalkyl, heterocycloalkyl, substituted heterocycloalkyl comprising one or two substituents independently selected from alkyl and oxo, or a heteroaromatic ring;
R⁶ is H, alkyl, alkylhydroxy, or haloalkyl;
and pharmaceutically acceptable salts.

2. A compound according to claim 1, wherein R¹ is H, halo, haloalkyl, haloalkoxy, alkyl, alkoxy, alkoxyalkoxy, alkoxyalkyl, thiolalkyl, cyanoalkyl, cycloalkoxy, cycloalkyl, cycloalkylalkyl, or heterocycloalkyl.

3. A compound according to claims 1-2, wherein R² is alkoxy, alkoxyalkoxy, alkoxyalkyl, alkylsulfonylalkyl, alkyl, alkenyl, alkynyl, alkynylalkoxy, cyano, cyanoalkyl, cycloalkyl, cycloalkoxy, cycloalkylalkyl, cyanoalkoxy, halo, haloalkoxy, (haloalkoxy)alkoxy, thiolalkyl, halothiolalkyl, halohydroxyalkoxy, haloalkyl, heterocycloalkyl, heterocycloalkylalkyl, heterocycloalkylalkynyl or heterocycloalkylalkoxy.

4. A compound according to claims 1-3, wherein R³ is H or alkoxy.

5. A compound according to claims 1-4, wherein X² is NH or S.

6. A compound according to claims 1-5, wherein R⁴ is H, halo, alkyl, alkoxy, thiolalkyl, cyanoalkyl, cycloalkyl, haloalkoxy, haloalkyl, heterocycloalkyl, or hydroxyalkyl, and R⁵ is H, halo, haloalkyl, haloalkoxy, halothiolalkyl, alkoxy, alkoxycarbonyl, alkyl, hydroxyalkyl, thiolalkyl, alkylsulfino, alkylsulfano, cyano, cyanoalkyl, cyanoalkoxy, dialkylamino, cycloalkyl, cycloalkoxy, or heterocycloalkyl, or R⁴ and R⁵ are connected to form a 5-membered heterocycloalkyl comprising a single O heteroatom, a 6-membered heterocycloalkyl comprising a single N heteroatom substituted by oxo and alkyl, a 5-membered heteroaromatic ring comprising one N heteroatom and one S heteroatom, or a 6-membered heteroaromatic ring comprising a single N heteroatom.

7. A compound according to claims 1-6, wherein wherein R⁴ is halo, alkyl or haloalkyl, and R⁵ is H, halo, haloalkyl, haloalkoxy, halothiolalkyl, alkoxy, alkyl, thiolalkyl, cyano, cyanoalkyl, cyanoalkoxy, dialkylamino or cycloalkoxy, or R⁴ and R⁵ are connected to form a 5-membered heterocycloalkyl comprising a single O heteroatom, or a 6-membered heteroaromatic ring comprising a single N heteroatom.

8. A compound according to claims 1-7, wherein R⁵ is H, halo, haloalkyl, haloalkoxy, halothiolalkyl, alkoxy, alkyl, thiolalkyl, cyano, cyanoalkyl, cyanoalkoxy, dialkylamino or cycloalkoxy.

9. A compound according to claims 1-8, wherein
R¹ is H, halo, haloalkyl, haloalkoxy, alkyl, alkoxy, alkoxyalkoxy, alkoxyalkyl, thiolalkyl, cyanoalkyl, cycloalkoxy, cycloalkyl, cycloalkylalkyl, or heterocycloalkyl;
R² is alkoxy, alkoxyalkoxy, alkoxyalkyl, alkylsulfonylalkyl, alkyl, alkenyl, alkynyl, alkynylalkoxy, cyano, cyanoalkyl, cycloalkyl, cycloalkoxy, cycloalkylalkyl, cyanoalkoxy, halo, haloalkoxy, (haloalkoxy)alkoxy, thiolalkyl, halothiolalkyl, halohydroxyalkoxy, haloalkyl, heterocycloalkyl, heterocycloalkylalkyl, heterocycloalkylalkynyl or heterocycloalkylalkoxy;
R³ is H, halo, alkyl, alkoxy, haloalkoxy, or thiolalkyl;
X₁ is CR⁵ or N;
X₂ is NH or S;
R⁴ is H, halo, alkyl, alkoxy, thiolalkyl, cyanoalkyl, cycloalkyl, haloalkoxy, haloalkyl, heterocycloalkyl, or hydroxyalkyl, and R⁵ is H, halo, haloalkyl, haloalkoxy, halothiolalkyl, alkoxy, alkyl, alkoxycarbonyl, hydroxyalkyl, thiolalkyl, alkylsulfino, alkylsulfano, cyano, cyanoalkyl, cyanoalkoxy, dialkylamino, cycloalkyl, cycloalkoxy, or heterocycloalkyl, or R⁴ and R⁵ are connected to form a 5-membered heterocycloalkyl comprising a single O heteroatom, a 6-membered heterocycloalkyl comprising a single N heteroatom substituted with alkyl and oxo, a 5-membered heteroaromatic ring comprising one N heteroatom and one S heteroatom, or a 6-membered heteroaromatic ring comprising a single N heteroatom;
and pharmaceutically acceptable salts.

10. A compound according to any one of claims 1 to 9, wherein
R¹ is H, alkoxy, cycloalkyl or haloalkoxy;
R² is alkyl, alkenyl, alkynyl, alkoxyalkyl, alkynyl, cyanoalkoxy, halo, haloalkoxy, haloalkyl or cycloalkyl;
R³ is H or alkoxy;
X₁ is CR⁵ or N;
X₂ is NH or S;
R⁴ is halo, alkyl or haloalkyl, and R⁵ is H, halo, haloalkyl, haloalkoxy, halothiolalkyl, alkoxy, alkyl, thiolalkyl, cyano, cyanoalkyl, cyanoalkoxy dialkylamino or cycloalkoxy, or R⁴ and R⁵ are connected to form a 5-membered heterocycloalkyl comprising a single O heteroatom or a 6-membered heteroaromatic ring comprising a single N heteroatom;
and pharmaceutically acceptable salts.

11. A compound according to any one of claims 1 to 10, selected from
6-chloro-N-[5-(2-fluoroethoxy)-4-methoxy-pyrimidin-2-yl]-1H-indole-3-sulfonamide;
6-chloro-7-fluoro-N-[5-(2-fluoroethoxy)-4-methoxy-pyrimidin-2-yl]-1H-indole-3-sulfonamide;
6,7-dichloro-N-[5-(2-fluoroethoxy)-4-methoxy-pyrimidin-2-yl]-1H-indole-3-sulfonamide;
6-chloro-7-(difluoromethyl)-N-[5-(2-fluoroethoxy)-4-methoxy-pyrimidin-2-yl]-1H-indole-3-sulfonamide;
6-chloro-7-(cyclopropoxy)-N-[5-(2-fluoroethoxy)-4-methoxy-pyrimidin-2-yl]-1H-indole-3-sulfonamide;
6-chloro-N-(5-ethoxy-4-methoxy-pyrimidin-2-yl)-1H-indole-3-sulfonamide;
6-chloro-N-[4-methoxy-5-(2-methoxyethoxy)pyrimidin-2-yl]-1H-indole-3-sulfonamide;
6-chloro-N-[5-(difluoromethoxy)-4-methoxy-pyrimidin-2-yl]-1H-indole-3-sulfonamide;
6-chloro-N-[5-[2-(difluoromethoxy)ethoxy]-4-methoxy-pyrimidin-2-yl]-1H-indole-3-sulfonamide;
6-chloro-N-[5-(2-fluoroethoxy)-4-methoxy-pyrimidin-2-yl]-1H-pyrrolo[2,3-b]pyridine-3-sulfonamide;
6-chloro-N-(5-chloro-4,6-dimethoxy-pyrimidin-2-yl)-1H-indole-3-sulfonamide;
N-(5-bromo-4,6-dimethoxy-pyrimidin-2-yl)-6-chloro-1H-indole-3-sulfonamide;
N-(5-bromo-4-ethoxy-6-methoxy-pyrimidin-2-yl)-6-chloro-1H-indole-3-sulfonamide;
N-[5-bromo-4-(2-fluoroethoxy)-6-methoxy-pyrimidin-2-yl]-6-chloro-1H-indole-3-sulfonamide;
6-chloro-N-(5-ethyl-4-methoxy-pyrimidin-2-yl)-1H-indole-3-sulfonamide;
6-chloro-N-(5-ethyl-4,6-dimethoxy-pyrimidin-2-yl)-1H-indole-3-sulfonamide;
6-chloro-N-(4,6-dimethoxy-5-methyl-pyrimidin-2-yl)-1H-indole-3-sulfonamide;
N-[5-bromo-4-(difluoromethoxy)-6-methoxy-pyrimidin-2-yl]-6-chloro-1H-indole-3-sulfonamide;
6-chloro-N-[4-cyclopropyl-5-(2,2-difluoroethoxy)-6-methoxy-pyrimidin-2-yl]-1H-indole-3-sulfonamide;
6-chloro-N-[5-(2,2-difluoroethoxy)-4-methoxy-6-methyl-pyrimidin-2-yl]-1H-indole-3-sulfonamide;
6-chloro-N-[5-(3-fluoropropyl)-4-methoxy-pyrimidin-2-yl]-1H-indole-3-sulfonamide;
N-(5-bromo-4-methoxy-pyrimidin-2-yl)-6-chloro-1H-indole-3-sulfonamide;
6-chloro-N-(5-cyano-4-methoxy-pyrimidin-2-yl)-1H-indole-3-sulfonamide;
6-chloro-N-[5-(2-fluoroethoxy)-4-methoxy-pyrimidin-2-yl]benzothiophene-3-sulfonamide;
6-bromo-N-[5-(2-fluoroethoxy)-4-methoxy-pyrimidin-2-yl]-1H-indole-3-sulfonamide;
6-chloro-N-(4-methoxy-5-methyl-pyrimidin-2-yl)-1H-indole-3-sulfonamide;
6-chloro-N-[5-(2-fluoroethoxy)-4-methoxy-pyrimidin-2-yl]thieno[2,3-b]pyridine-3-sulfonamide;
6-chloro-N-(5-cyclopropyl-4-methoxy-pyrimidin-2-yl)-1H-indole-3-sulfonamide;
6-chloro-N-(4-methoxy-5-prop-1-ynyl-pyrimidin-2-yl)-1H-indole-3-sulfonamide;
6-bromo-N-(5-bromo-4,6-dimethoxy-pyrimidin-2-yl)-1H-pyrrolo[2,3-b]pyridine-3-sulfonamide;
N-(5-bromo-4,6-dimethoxy-pyrimidin-2-yl)-6-chloro-1H-pyrrolo[2,3-b]pyridine-3-sulfonamide;
6-chloro-N-(5-cyano-4,6-dimethoxy-pyrimidin-2-yl)-1H-indole-3-sulfonamide;
N-(5-bromo-4,6-dimethyl-pyrimidin-2-yl)-6-chloro-1H-indole-3-sulfonamide;
6-chloro-N-[5-(2,2-difluoroethoxy)pyrimidin-2-yl]-1H-indole-3-sulfonamide;
6-chloro-N-[5-(2,2-difluoroethoxy)-4-methylsulfanyl-pyrimidin-2-yl]-1H-indole-3-sulfonamide;
6-chloro-N-(4-methoxy-5-methylsulfanyl-pyrimidin-2-yl)-1H-indole-3-sulfonamide;
6-chloro-N-[5-(difluoromethylthio)-4-methoxy-pyrimidin-2-yl]-1H-indole-3-sulfonamide;
6-chloro-N-[5-(cyclopropoxy)-4-methoxy-pyrimidin-2-yl]-1H-indole-3-sulfonamide;
6-chloro-N-[5-(3,3-difluoropropoxy)-4-methoxy-pyrimidin-2-yl]-1H-indole-3-sulfonamide;
6-chloro-N-[4-methoxy-5-(3,3,3-trifluoropropoxy)pyrimidin-2-yl]-1H-indole-3-sulfonamide;
6-chloro-N-[4-methoxy-5-(1,1,2-trifluoroethoxy)pyrimidin-2-yl]-1H-indole-3-sulfonamide;
6-chloro-N-[5-(1,1-difluoroethoxy)-4-methoxy-pyrimidin-2-yl]-1H-indole-3-sulfonamide;
6-chloro-N-[4-methoxy-5-(1,1,2,2-tetrafluoroethoxy)pyrimidin-2-yl]-1H-indole-3-sulfonamide;
6-chloro-N-[5-(2-chloro-1,1,2-trifluoro-ethoxy)-4-methoxy-pyrimidin-2-yl]-1H-indole-3-sulfonamide;
6-chloro-N-(4-methoxy-5-propyl-pyrimidin-2-yl)-1H-indole-3-sulfonamide;
6-chloro-N-[5-(cyclopropylmethyl)-4-methoxy-pyrimidin-2-yl]-1H-indole-3-sulfonamide;
6-chloro-N-[5-(2,2-difluoroethyl)-4-methoxy-pyrimidin-2-yl]-1H-indole-3-sulfonamide;
6-chloro-N-[5-(3,3-difluoropropyl)-4-methoxy-pyrimidin-2-yl]-1H-indole-3-sulfonamide;
6-chloro-N-[4-methoxy-5-(2-methoxyethyl)pyrimidin-2-yl]-1H-indole-3-sulfonamide;
6-chloro-N-[4-methoxy-5-(oxetan-3-yl)pyrimidin-2-yl]-1H-indole-3-sulfonamide;
6-chloro-N-[5-(fluoromethoxy)-4,6-dimethoxy-pyrimidin-2-yl]-1H-indole-3-sulfonamide;
6-chloro-N-[5-(difluoromethoxy)-4,6-dimethoxy-pyrimidin-2-yl]-1H-indole-3-sulfonamide;
6-chloro-N-[5-(2-fluoroethoxy)-4,6-dimethoxy-pyrimidin-2-yl]-1H-indole-3-sulfonamide;
6-chloro-N-[5-(2,2-difluoroethoxy)-4,6-dimethoxy-pyrimidin-2-yl]-1H-indole-3-sulfonamide;
6-chloro-N-[4,6-dimethoxy-5-(1,1,2-trifluoroethoxy)pyrimidin-2-yl]-1H-indole-3-sulfonamide;
N-(5-allyl-4,6-dimethoxy-pyrimidin-2-yl)-6-chloro-1H-indole-3-sulfonamide;
6-chloro-N-(4,6-dimethoxy-5-prop-1-ynyl-pyrimidin-2-yl)-1H-indole-3-sulfonamide;
6-chloro-N-(4,6-dimethoxy-5-propyl-pyrimidin-2-yl)-1H-indole-3-sulfonamide;
6-chloro-N-[5-(2-fluoroethyl)-4,6-dimethoxy-pyrimidin-2-yl]-1H-indole-3-sulfonamide;
6-chloro-N-[5-(2,2-difluoroethyl)-4,6-dimethoxy-pyrimidin-2-yl]-1H-indole-3-sulfonamide;
6-chloro-N-[5-(3-fluoropropyl)-4,6-dimethoxy-pyrimidin-2-yl]-1H-indole-3-sulfonamide;
6-chloro-N-[5-(3,3-difluoropropyl)-4,6-dimethoxy-pyrimidin-2-yl]-1H-indole-3-sulfonamide;
6-chloro-N-[5-(2,2-difluoropropyl)-4,6-dimethoxy-pyrimidin-2-yl]-1H-indole-3-sulfonamide;
6-chloro-N-[4,6-dimethoxy-5-(3,3,3-trifluoropropyl)pyrimidin-2-yl]-1H-indole-3-sulfonamide;
6-chloro-N-[4,6-dimethoxy-5-(methoxymethyl)pyrimidin-2-yl]-1H-indole-3-sulfonamide;
6-chloro-N-[4,6-dimethoxy-5-(2-methoxyethyl)pyrimidin-2-yl]-1H-indole-3-sulfonamide;
6-chloro-N-[5-(cyanomethyl)-4,6-dimethoxy-pyrimidin-2-yl]-1H-indole-3-sulfonamide;
6-chloro-N-[5-(2-cyanoethyl)-4,6-dimethoxy-pyrimidin-2-yl]-1H-indole-3-sulfonamide;
6-chloro-N-(5-cyclopropyl-4,6-dimethoxy-pyrimidin-2-yl)-1H-indole-3-sulfonamide;
N-[5-bromo-4-methoxy-6-(2-methoxyethoxy)pyrimidin-2-yl]-6-chloro-1H-indole-3-sulfonamide;
N-[5-bromo-4-(cyclopropoxy)-6-methoxy-pyrimidin-2-yl]-6-chloro-1H-indole-3-sulfonamide;
6-chloro-N-[4-(difluoromethoxy)-6-methoxy-5-methyl-pyrimidin-2-yl]-1H-indole-3-sulfonamide;
N-(5-bromo-4-fluoro-6-methoxy-pyrimidin-2-yl)-6-chloro-1H-indole-3-sulfonamide;
6-chloro-N-[5-(2,2-difluoroethoxy)-4-fluoro-6-methoxy-pyrimidin-2-yl]-1H-indole-3-sulfonamide;
6-chloro-N-(4-chloro-5,6-dimethoxy-pyrimidin-2-yl)-1H-indole-3-sulfonamide;
6-chloro-N-[4-chloro-5-(2,2-difluoroethoxy)pyrimidin-2-yl]-1H-indole-3-sulfonamide;
6-chloro-N-[5-(2,2-difluoroethoxy)-4-ethyl-6-methoxy-pyrimidin-2-yl]-1H-indole-3-sulfonamide;
6-chloro-N-[5-(2,2-difluoroethyl)-4-methoxy-6-methyl-pyrimidin-2-yl]-1H-indole-3-sulfonamide;
6-chloro-N-[4-cyclopropyl-5-(2,2-difluoroethyl)-6-methoxy-pyrimidin-2-yl]-1H-indole-3-sulfonamide;
N-[5-bromo-4-(cyclopropylmethyl)-6-methoxy-pyrimidin-2-yl]-6-chloro-1H-indole-3-sulfonamide;
N-[5-bromo-4-methoxy-6-(methoxymethyl)pyrimidin-2-yl]-6-chloro-1H-indole-3-sulfonamide;
N-[5-bromo-4-(1,1-difluoroethyl)-6-methoxy-pyrimidin-2-yl]-6-chloro-1H-indole-3-sulfonamide;
N-[5-bromo-4-(fluoromethoxy)-6-methoxy-pyrimidin-2-yl]-6-chloro-1H-indole-3-sulfonamide;
N-[5-bromo-4-(difluoromethyl)-6-methoxy-pyrimidin-2-yl]-6-chloro-1H-indole-3-sulfonamide;
N-[5-bromo-4-(cyanomethyl)-6-methoxy-pyrimidin-2-yl]-6-chloro-1H-indole-3-sulfonamide;
6-chloro-N-[5-(difluoromethoxy)-4-methoxy-6-methylsulfanyl-pyrimidin-2-yl]-1H-indole-3-sulfonamide;
N-[4,6-dimethoxy-5-(1,1,2-trifluoroethoxy)pyrimidin-2-yl]-6-fluoro-1H-indole-3-sulfonamide;
6-bromo-N-[5-(2,2-difluoroethyl)-4-methoxy-pyrimidin-2-yl]-1H-indole-3-sulfonamide;
6-bromo-N-[5-(2-cyanoethyl)-4-methoxy-pyrimidin-2-yl]-1H-indole-3-sulfonamide;
6-bromo-N-[5-(difluoromethoxy)-4,6-dimethoxy-pyrimidin-2-yl]-1H-indole-3-sulfonamide;
6-bromo-N-[5-(2-fluoroethoxy)-4,6-dimethoxy-pyrimidin-2-yl]-1H-indole-3-sulfonamide;
6-bromo-N-[5-(2,2-difluoroethoxy)-4,6-dimethoxy-pyrimidin-2-yl]-1H-indole-3-sulfonamide;
6-bromo-N-[5-(2-cyanoethyl)-4,6-dimethoxy-pyrimidin-2-yl]-1H-indole-3-sulfonamide;
N-[5-(difluoromethoxy)-4,6-dimethoxy-pyrimidin-2-yl]-6-(difluoromethyl)-1H-pyrrolo[2,3-b]pyridine-3-sulfonamide;
6-(difluoromethyl)-N-[5-(2-fluoroethoxy)-4,6-dimethoxy-pyrimidin-2-yl]-1H-pyrrolo[2,3-b]pyridine-3-sulfonamide;
N-[5-(2,2-difluoroethoxy)-4,6-dimethoxy-pyrimidin-2-yl]-6-(difluoromethyl)-1H-pyrrolo[2,3-b]pyridine-3-sulfonamide;
6-(difluoromethyl)-N-[4,6-dimethoxy-5-(1,1,2-trifluoroethoxy)pyrimidin-2-yl]-1H-pyrrolo[2,3-b]pyridine-3-sulfonamide;
6-(difluoromethyl)-N-[5-(3,3-difluoropropyl)-4,6-dimethoxy-pyrimidin-2-yl]-1H-pyrrolo[2,3-b]pyridine-3-sulfonamide;
N-[5-(cyanomethoxy)-4,6-dimethoxy-pyrimidin-2-yl]-6-(difluoromethyl)-1H-pyrrolo[2,3-b]pyridine-3-sulfonamide;
6-(difluoromethyl)-N-[4-methoxy-5-(1,1,2-trifluoroethoxy)pyrimidin-2-yl]-1H-indole-3-sulfonamide;
N-[5-(difluoromethoxy)-4,6-dimethoxy-pyrimidin-2-yl]-6-(difluoromethyl)-1H-indole-3-sulfonamide;
N-[5-(2,2-difluoroethoxy)-4,6-dimethoxy-pyrimidin-2-yl]-6-(difluoromethyl)-1H-indole-3-sulfonamide;
N-[5-(2-cyanoethyl)-4,6-dimethoxy-pyrimidin-2-yl]-6-(difluoromethyl)-1H-indole-3-sulfonamide;
N-[5-(2,2-difluoroethyl)-4-methoxy-pyrimidin-2-yl]-6-(difluoromethoxy)-1H-indole-3-sulfonamide;
6-chloro-N-[5-(2-cyanoethyl)-4-methoxy-pyrimidin-2-yl]-7-(difluoromethylthio)-1H-indole-3-sulfonamide;
6-chloro-7-(difluoromethylthio)-N-[5-(2-fluoroethoxy)-4,6-dimethoxy-pyrimidin-2-yl]-1H-indole-3-sulfonamide;
6-chloro-N-[5-(2-cyanoethyl)-4,6-dimethoxy-pyrimidin-2-yl]-7-(difluoromethylthio)-1H-indole-3-sulfonamide;
7-chloro-N-[5-(2,2-difluoroethyl)-4-methoxy-pyrimidin-2-yl]-1H-indole-3-sulfonamide;
6-bromo-N-[5-(2-fluoroethoxy)-4,6-dimethoxy-pyrimidin-2-yl]-1H-pyrrolo[2,3-b]pyridine-3-sulfonamide;
6-bromo-N-[4,6-dimethoxy-5-(1,1,2-trifluoroethoxy)pyrimidin-2-yl]-1H-pyrrolo[2,3-b]pyridine-3-sulfonamide;
6-bromo-N-[5-(2,2-difluoroethyl)-4,6-dimethoxy-pyrimidin-2-yl]-1H-pyrrolo[2,3-b]pyridine-3-sulfonamide;
6-bromo-N-[5-(3,3-difluoropropyl)-4,6-dimethoxy-pyrimidin-2-yl]-1H-pyrrolo[2,3-b]pyridine-3-sulfonamide;
6-bromo-N-[5-(2-cyanoethyl)-4,6-dimethoxy-pyrimidin-2-yl]-1H-pyrrolo[2,3-b]pyridine-3-sulfonamide;
6-bromo-N-[5-(cyanomethoxy)-4,6-dimethoxy-pyrimidin-2-yl]-1H-pyrrolo[2,3-b]pyridine-3-sulfonamide;
6-chloro-N-[5-(2,2-difluoroethyl)-4,6-dimethoxy-pyrimidin-2-yl]-7-(dimethylamino)-1H-indole-3-sulfonamide;
N-[5-(difluoromethoxy)-4,6-dimethoxy-pyrimidin-2-yl]-1H-pyrrolo[3,2-h]quinoline-3-sulfonamide;
N-[5-(2-fluoroethoxy)-4,6-dimethoxy-pyrimidin-2-yl]-1H-pyrrolo[3,2-h]quinoline-3-sulfonamide;
6-chloro-N-(4,6-dimethoxy-5-methyl-pyrimidin-2-yl)-1H-pyrrolo[2,3-b]pyridine-3-sulfonamide;
6-chloro-N-[5-(difluoromethoxy)-4-methoxy-pyrimidin-2-yl]-1H-pyrrolo[2,3-b]pyridine-3-sulfonamide;
6-chloro-N-[5-(2,2-difluoroethyl)-4,6-dimethoxy-pyrimidin-2-yl]-1H-pyrrolo[2,3-b]pyridine-3-sulfonamide;
6-chloro-N-[5-(3,3-difluoropropyl)-4,6-dimethoxy-pyrimidin-2-yl]-1H-pyrrolo[2,3-b]pyridine-3-sulfonamide;
6-chloro-N-[5-(2-cyanoethyl)-4,6-dimethoxy-pyrimidin-2-yl]-1H-pyrrolo[2,3-b]pyridine-3-sulfonamide;
6-chloro-N-[5-(2,2-difluoroethoxy)-4-methoxy-pyrimidin-2-yl]-7-fluoro-1H-indole-3-sulfonamide;
6-chloro-N-[5-(2,2-difluoroethyl)-4-methoxy-pyrimidin-2-yl]-7-fluoro-1H-indole-3-sulfonamide;
6-chloro-N-[5-(difluoromethoxy)-4,6-dimethoxy-pyrimidin-2-yl]-7-fluoro-1H-indole-3-sulfonamide;
6-chloro-N-[5-(difluoromethoxy)-4-methoxy-pyrimidin-2-yl]-7-fluoro-1H-indole-3-sulfonamide;
6-chloro-7-(cyclopropoxy)-N-[5-(2,2-difluoroethyl)-4-methoxy-pyrimidin-2-yl]-1H-indole-3-sulfonamide;
7-bromo-N-[5-(2,2-difluoroethyl)-4-methoxy-pyrimidin-2-yl]-6-fluoro-1H-indole-3-sulfonamide;
7-bromo-N-[5-(2,2-difluoroethyl)-4-methoxy-pyrimidin-2-yl]-1H-indole-3-sulfonamide;
7-chloro-N-[5-(2,2-difluoroethyl)-4-methoxy-pyrimidin-2-yl]-6-fluoro-1H-indole-3-sulfonamide;
N-[5-(2-fluoroethoxy)-4,6-dimethoxy-pyrimidin-2-yl]-6-methyl-1H-pyrrolo[2,3-b]pyridine-3-sulfonamide;
N-[5-(2,2-difluoroethyl)-4,6-dimethoxy-pyrimidin-2-yl]-6-methyl-1H-pyrrolo[2,3-b]pyridine-3-sulfonamide;
6-chloro-N-[5-(2,2-difluoroethyl)-4,6-dimethoxy-pyrimidin-2-yl]-7-morpholino-1H-indole-3-sulfonamide;
7-bromo-N-[5-(2,2-difluoroethyl)-4,6-dimethoxy-pyrimidin-2-yl]-1H-indole-3-sulfonamide;
6-chloro-N-[5-(2,2-difluoroethoxy)-4-methoxy-pyrimidin-2-yl]-1H-indole-3-sulfonamide;
6-chloro-N-[5-(1,1-difluoroethyl)-4-methoxy-pyrimidin-2-yl]-1H-indole-3-sulfonamide;
6-chloro-N-(4-ethyl-6-methoxy-5-methyl-pyrimidin-2-yl)-1H-indole-3-sulfonamide;
N-(5-bromo-4-methoxy-6-tetrahydropyran-4-yl-pyrimidin-2-yl)-6-chloro-1H-indole-3-sulfonamide;
6-chloro-N-[5-(2,2-difluoroethyl)-4-methoxy-pyrimidin-2-yl]thieno[2,3-b]pyridine-3-sulfonamide;
N-[5-(2,2-difluoroethyl)-4-methoxy-pyrimidin-2-yl]-6-fluoro-1H-indole-3-sulfonamide;
7-bromo-6-chloro-N-[5-(2-fluoroethoxy)-4-methoxy-pyrimidin-2-yl]-1H-indole-3-sulfonamide;
7-bromo-6-chloro-N-[5-(2,2-difluoroethyl)-4-methoxy-pyrimidin-2-yl]-1H-indole-3-sulfonamide;
6-bromo-N-[5-(2,2-difluoroethoxy)-4-methoxy-pyrimidin-2-yl]-1H-indole-3-sulfonamide;
6-bromo-N-(4,6-dimethoxy-5-methyl-pyrimidin-2-yl)thieno[2,3-b]pyridine-3-sulfonamide;
6-bromo-N-[5-(2,2-difluoroethoxy)-4-methoxy-pyrimidin-2-yl]thieno[2,3-b]pyridine-3-sulfonamide;
6-bromo-N-[5-(2-fluoroethoxy)-4-methoxy-pyrimidin-2-yl]thieno[2,3-b]pyridine-3-sulfonamide;
6-bromo-N-[4-methoxy-5-(1,1,2-trifluoroethoxy)pyrimidin-2-yl]thieno[2,3-b]pyridine-3-sulfonamide;
6-bromo-N-[5-(2,2-difluoroethyl)-4-methoxy-pyrimidin-2-yl]thieno[2,3-b]pyridine-3-sulfonamide;
6-bromo-N-[5-(2-cyanoethyl)-4-methoxy-pyrimidin-2-yl]thieno[2,3-b]pyridine-3-sulfonamide;
6-bromo-N-[5-(2,2-difluoroethyl)-4,6-dimethoxy-pyrimidin-2-yl]thieno[2,3-b]pyridine-3-sulfonamide;
6-bromo-N-[5-(2-cyanoethyl)-4,6-dimethoxy-pyrimidin-2-yl]thieno[2,3-b]pyridine-3-sulfonamide;
N-[5-(2,2-difluoroethyl)-4-methoxy-pyrimidin-2-yl]-6-ethyl-1H-indole-3-sulfonamide;
N-[5-(2,2-difluoroethyl)-4-methoxypyrimidin-2-yl]-6-propyl-1H-indole-3-sulfonamide;
6-chloro-N-[5-(2,2-difluoroethyl)-4-methoxy-pyrimidin-2-yl]benzothiophene-3-sulfonamide;
6-chloro-N-[5-(3,3-difluoropropyl)-4-methoxy-pyrimidin-2-yl]benzothiophene-3-sulfonamide;
6-chloro-N-[5-(2-cyanoethyl)-4-methoxy-pyrimidin-2-yl]benzothiophene-3-sulfonamide;
6-bromo-N-[5-(2,2-difluoroethoxy)-4-methoxy-pyrimidin-2-yl]benzothiophene-3-sulfonamide;
6-bromo-N-[5-(2-fluoroethoxy)-4-methoxy-pyrimidin-2-yl]benzothiophene-3-sulfonamide;
N-[5-(2-fluoroethoxy)-4-methoxy-pyrimidin-2-yl]-6-methyl-benzothiophene-3-sulfonamide;
6-bromo-N-(4,6-dimethoxy-5-methyl-pyrimidin-2-yl)-1H-pyrrolo[2,3-b]pyridine-3-sulfonamide;
6-bromo-N-[5-(2,2-difluoroethoxy)-4-methoxy-pyrimidin-2-yl]-1H-pyrrolo[2,3-b]pyridine-3-sulfonamide;
6-chloro-N-[5-(2,2-difluoroethyl)-4-methoxy-pyrimidin-2-yl]-7-fluoro-benzothiophene-3-sulfonamide;
6-chloro-N-[5-(2-cyanoethyl)-4-methoxy-pyrimidin-2-yl]-7-fluoro-benzothiophene-3-sulfonamide;
N-[5-(2,2-difluoroethyl)-4-methoxy-pyrimidin-2-yl]-6,7-difluoro-1H-indole-3-sulfonamide;
6-(difluoromethyl)-N-[5-(2-fluoroethoxy)-4-methoxy-pyrimidin-2-yl]thieno[2,3-b]pyridine-3-sulfonamide;
N-[5-(2,2-difluoroethyl)-4-methoxy-pyrimidin-2-yl]-6-(difluoromethyl)thieno[2,3-b]pyridine-3-sulfonamide;
N-[5-(2,2-difluoroethyl)-4,6-dimethoxy-pyrimidin-2-yl]-6-(difluoromethyl)thieno[2,3-b]pyridine-3-sulfonamide;
N-[5-(2,2-difluoroethoxy)-4,6-dimethoxy-pyrimidin-2-yl]-1H-pyrrolo[3,2-h]quinoline-3-sulfonamide;
N-[5-(2,2-difluoroethyl)-4,6-dimethoxy-pyrimidin-2-yl]-1H-pyrrolo[3,2-h]quinoline-3-sulfonamide;
6-chloro-N-[5-(2,2-difluoroethoxy)-4-methoxy-pyrimidin-2-yl]-1H-pyrrolo[2,3-b]pyridine-3-sulfonamide;
6-chloro-N-[4,6-dimethoxy-5-(1,1,2-trifluoroethoxy)pyrimidin-2-yl]-1H-pyrrolo[2,3-b]pyridine-3-sulfonamide;
6-bromo-N-[5-(difluoromethoxy)-4,6-dimethoxy-pyrimidin-2-yl]-1H-pyrrolo[2,3-b]pyridine-3-sulfonamide;
6-chloro-N-(4-chloro-5-methoxy-pyrimidin-2-yl)-1H-indole-3-sulfonamide; 6-chloro-N-[5-(3-fluoropropoxy)-4-methoxy-pyrimidin-2-yl]-1H-indole-3-sulfonamide;
N-(5-but-2-ynoxy-4-methoxy-pyrimidin-2-yl)-6-chloro-1H-indole-3-sulfonamide; 6-chloro-N-[5-(2,3-difluoropropyl)-4-methoxy-pyrimidin-2-yl]-1H-indole-3-sulfonamide;
6-chloro-N-[4-methoxy-5-(2,2,2-trifluoroethyl)pyrimidin-2-yl]-1H-indole-3-sulfonamide;
6-chloro-N-[4-methoxy-5-(3,3,3-trifluoropropyl)pyrimidin-2-yl]-1H-indole-3-sulfonamide;
6-chloro-N-[5-(2-cyanoethyl)-4-methoxy-pyrimidin-2-yl]-1H-indole-3-sulfonamide;
6-chloro-N-[4-methoxy-5-[2-(oxetan-3-yl)ethynyl]pyrimidin-2-yl]-1H-indole-3-sulfonamide;
6-chloro-N-[4-methoxy-5-[2-(oxetan-3-yl)ethyl]pyrimidin-2-yl]-1H-indole-3-sulfonamide;
N-[5-(2,2-difluoroethyl)-4-methoxy-pyrimidin-2-yl]-6-methyl-1H-indole-3-sulfonamide;
7-bromo-6-chloro-N-[5-(3,3-difluoropropyl)-4-methoxy-pyrimidin-2-yl]-1H-indole-3-sulfonamide;
6-bromo-N-[5-(2,2-difluoroethyl)-4-methoxy-pyrimidin-2-yl]-7-methyl-1H-indole-3-sulfonamide;
6-chloro-N-[5-(2,2-difluoroethyl)-4-methoxy-pyrimidin-2-yl]-7-methoxy-1H-indole-3-sulfonamide;
6-chloro-N-[5-(3,3-difluoropropyl)-4-methoxy-pyrimidin-2-yl]-7-methoxy-1H-indole-3-sulfonamide;
6-chloro-N-[5-(2,2-difluoroethyl)-4-methoxy-pyrimidin-2-yl]-7-(difluoromethoxy)-1H-indole-3-sulfonamide;
6-bromo-N-[5-(difluoromethoxy)-4-methoxy-pyrimidin-2-yl]-1H-indole-3-sulfonamide;
N-[5-(2,2-difluoroethyl)-4-methoxy-pyrimidin-2-yl]-6-(trifluoromethyl)-1H-indole-3-sulfonamide;
6-bromo-N-[5-(2,2-difluoroethyl)-4-methoxy-pyrimidin-2-yl]-7-fluoro-1H-indole-3-sulfonamide;
6-chloro-N-[5-(2,2-difluoroethyl)-4-methoxy-pyrimidin-2-yl]-7-methyl-1H-indole-3-sulfonamide;
N-[5-(2,2-difluoroethyl)-4-methoxy-pyrimidin-2-yl]-6-(methylthio)-1H-indole-3-sulfonamide;
N-[5-(2,2-difluoroethyl)-4,6-dimethoxy-pyrimidin-2-yl]-6-(difluoromethyl)-1H-pyrrolo[2,3-b]pyridine-3-sulfonamide;
N-[5-(2,2-difluoroethyl)-4-methoxy-pyrimidin-2-yl]-6-(difluoromethyl)-1H-indole-3-sulfonamide;
6-bromo-N-[5-(2,2-difluoroethyl)-4-methoxy-pyrimidin-2-yl]-7-methoxy-1H-indole-3-sulfonamide;
6-(cyanomethyl)-N-[5-(2,2-difluoroethyl)-4-methoxy-pyrimidin-2-yl]-1H-indole-3-sulfonamide;
6-chloro-N-[5-(2,2-difluoroethyl)-4-methoxy-pyrimidin-2-yl]-7-(fluoromethoxy)-1H-indole-3-sulfonamide;
6-chloro-N-[5-(2,2-difluoroethyl)-4-methoxy-pyrimidin-2-yl]-7-(difluoromethylsulfanyl)-1H-indole-3-sulfonamide;
6-bromo-N-[5-(2,2-difluoroethyl)-4-methoxy-pyrimidin-2-yl]-1H-pyrrolo[2,3-b]pyridine-3-sulfonamide;
6-bromo-N-[5-(3,3-difluoropropyl)-4-methoxy-pyrimidin-2-yl]-1H-pyrrolo[2,3-b]pyridine-3-sulfonamide;
6-bromo-N-[5-(2,2-difluoroethoxy)-4,6-dimethoxy-pyrimidin-2-yl]-1H-pyrrolo[2,3-b]pyridine-3-sulfonamide;
6-chloro-N-[5-(2,2-difluoroethyl)-4-methoxy-pyrimidin-2-yl]-1H-pyrrolo[2,3-b]pyridine-3-sulfonamide;
6-chloro-N-[5-(3,3-difluoropropyl)-4-methoxy-pyrimidin-2-yl]-1H-pyrrolo[2,3-b]pyridine-3-sulfonamide;
6-chloro-N-[5-(difluoromethoxy)-4,6-dimethoxy-pyrimidin-2-yl]-1H-pyrrolo[2,3-b]pyridine-3-sulfonamide;
6-chloro-N-[5-(2,2-difluoroethoxy)-4,6-dimethoxy-pyrimidin-2-yl]-1H-pyrrolo[2,3-b]pyridine-3-sulfonamide;
6-chloro-N-[5-(3,3-difluoropropyl)-4-methoxy-pyrimidin-2-yl]-7-fluoro-1H-indole-3-sulfonamide;
6-chloro-N-[5-(2,2-difluoroethoxy)-4,6-dimethoxy-pyrimidin-2-yl]-7-fluoro-1H-indole-3-sulfonamide;
6-chloro-7-(difluoromethyl)-N-[5-(3,3-difluoropropyl)-4-methoxy-pyrimidin-2-yl]-1H-indole-3-sulfonamide;
6,7-dichloro-N-[5-(2,2-difluoroethyl)-4-methoxy-pyrimidin-2-yl]-1H-indole-3-sulfonamide;
6,7-dichloro-N-[5-(3,3-difluoropropyl)-4-methoxy-pyrimidin-2-yl]-1H-indole-3-sulfonamide;
6-chloro-7-(cyclopropoxy)-N-[5-(3,3-difluoropropyl)-4-methoxy-pyrimidin-2-yl]-1H-indole-3-sulfonamide;
6-bromo-7-fluoro-N-[5-(2-fluoroethoxy)-4-methoxy-pyrimidin-2-yl]-1H-indole-3-sulfonamide;
6-chloro-N-[5-(2,2-difluoroethyl)-4-methoxy-pyrimidin-2-yl]-7-(difluoromethyl)-1H-indole-3-sulfonamide;
6-chloro-7-fluoro-N-[4-methoxy-5-(1,1,2-trifluoroethoxy)pyrimidin-2-yl]-1H-indole-3-sulfonamide;
6-chloro-N-[5-(2-cyanoethyl)-4-methoxy-pyrimidin-2-yl]-7-fluoro-1H-indole-3-sulfonamide;
6-chloro-N-[5-(2,2-difluoroethyl)-4,6-dimethoxy-pyrimidin-2-yl]-7-fluoro-1H-indole-3-sulfonamide;
6-chloro-N-[5-(2-cyanoethyl)-4,6-dimethoxy-pyrimidin-2-yl]-7-fluoro-1H-indole-3-sulfonamide;
6-chloro-N-[5-(cyanomethoxy)-4,6-dimethoxy-pyrimidin-2-yl]-7-fluoro-1H-indole-3-sulfonamide;
6-chloro-N-[4-methoxy-5-(1,1,2-trifluoroethoxy)pyrimidin-2-yl]-1H-pyrrolo[2,3-b]pyridine-3-sulfonamide;
6-chloro-N-[5-(2-fluoroethoxy)-4,6-dimethoxy-pyrimidin-2-yl]-1H-pyrrolo[2,3-b]pyridine-3-sulfonamide;
N-[5-(2,2-difluoroethyl)-4-methoxy-pyrimidin-2-yl]-1H-pyrrolo[3,2-h]quinoline-3-sulfonamide;
6-chloro-7-cyano-N-[5-(2,2-difluoroethyl)-4-methoxy-pyrimidin-2-yl]-1H-indole-3-sulfonamide;
6-chloro-7-cyano-N-[5-(difluoromethoxy)-4,6-dimethoxy-pyrimidin-2-yl]-1H-indole-3-sulfonamide;
6-chloro-7-cyano-N-[5-(2-fluoroethoxy)-4,6-dimethoxy-pyrimidin-2-yl]-1H-indole-3-sulfonamide;
6-chloro-7-cyano-N-[5-(2,2-difluoroethoxy)-4,6-dimethoxy-pyrimidin-2-yl]-1H-indole-3-sulfonamide;
6-chloro-7-cyano-N-[5-(2,2-difluoroethyl)-4,6-dimethoxy-pyrimidin-2-yl]-1H-indole-3-sulfonamide;
N-[5-(2,2-difluoroethyl)-4-methoxy-pyrimidin-2-yl]-6,8-dihydro-1H-fur[3,4-g]indole-3-sulfonamide;
N-[5-(2,2-difluoroethyl)-4,6-dimethoxy-pyrimidin-2-yl]-6,8-dihydro-1H-fur[3,4-g]indole-3-sulfonamide;
6-bromo-N-[5-(2-fluoroethoxy)-4-methoxy-pyrimidin-2-yl]-1H-pyrrolo[2,3-b]pyridine-3-sulfonamide;
6-bromo-N-[4-methoxy-5-(1,1,2-trifluoroethoxy)pyrimidin-2-yl]-1H-pyrrolo[2,3-b]pyridine-3-sulfonamide;
6-chloro-7-(cyanomethyl)-N-[5-(2,2-difluoroethyl)-4-methoxy-pyrimidin-2-yl]-1H-indole-3-sulfonamide;
6-chloro-7-cyclopropyl-N-[5-(2,2-difluoroethyl)-4-methoxy-pyrimidin-2-yl]-1H-indole-3-sulfonamide;
6-chloro-N-[5-(2,2-difluoroethyl)-4-methoxy-pyrimidin-2-yl]-7-(methylthio)-1H-indole-3-sulfonamide;
6-(difluoromethyl)-N-[5-(2-fluoroethoxy)-4-methoxy-pyrimidin-2-yl]-1H-indole-3-sulfonamide;
N-[5-(2,2-difluoroethyl)-4,6-dimethoxy-pyrimidin-2-yl]-6-(difluoromethyl)-1H-indole-3-sulfonamide;
N-[5-(cyanomethoxy)-4,6-dimethoxy-pyrimidin-2-yl]-6-(difluoromethyl)-1H-indole-3-sulfonamide;
6-(difluoromethyl)-N-[4-methoxy-5-(1,1,2-trifluoroethoxy)pyrimidin-2-yl]-1H-pyrrolo[2,3-b]pyridine-3-sulfonamide;
N-[5-(2-cyanoethyl)-4,6-dimethoxy-pyrimidin-2-yl]-6-(difluoromethyl)-1H-pyrrolo[2,3-b]pyridine-3-sulfonamide;
6-bromo-N-[5-(2-cyanoethyl)-4-methoxy-pyrimidin-2-yl]-7-fluoro-1H-indole-3-sulfonamide;
6-bromo-N-[5-(difluoromethoxy)-4,6-dimethoxy-pyrimidin-2-yl]-7-fluoro-1H-indole-3-sulfonamide;
6-bromo-N-[5-(2,2-difluoroethoxy)-4,6-dimethoxy-pyrimidin-2-yl]-7-fluoro-1H-indole-3-sulfonamide;
6-bromo-N-[5-(2-cyanoethyl)-4,6-dimethoxy-pyrimidin-2-yl]-7-fluoro-1H-indole-3-sulfonamide;
6-bromo-N-[5-(cyanomethoxy)-4,6-dimethoxy-pyrimidin-2-yl]-7-fluoro-1H-indole-3-sulfonamide;
6-bromo-N-[5-(2,2-difluoroethyl)-4,6-dimethoxy-pyrimidin-2-yl]-1H-indole-3-sulfonamide;
6-bromo-N-[5-(cyanomethoxy)-4,6-dimethoxy-pyrimidin-2-yl]-1H-indole-3-sulfonamide;
6-(difluoromethyl)-7-fluoro-N-[5-(2-fluoroethoxy)-4-methoxy-pyrimidin-2-yl]-1H-indole-3-sulfonamide;
N-[5-(2,2-difluoroethyl)-4-methoxy-pyrimidin-2-yl]-6-(difluoromethyl)-7-fluoro-1H-indole-3-sulfonamide;
N-[5-(difluoromethoxy)-4,6-dimethoxy-pyrimidin-2-yl]-6-(difluoromethyl)-7-fluoro-1H-indole-3-sulfonamide;
N-[5-(2,2-difluoroethoxy)-4,6-dimethoxy-pyrimidin-2-yl]-6-(difluoromethyl)-7-fluoro-1H-indole-3-sulfonamide;
N-[5-(2,2-difluoroethyl)-4,6-dimethoxy-pyrimidin-2-yl]-6-(difluoromethyl)-7-fluoro-1H-indole-3-sulfonamide;
N-[5-(2-cyanoethyl)-4,6-dimethoxy-pyrimidin-2-yl]-6-(difluoromethyl)-7-fluoro-1H-indole-3-sulfonamide;
N-[5-(cyanomethoxy)-4,6-dimethoxy-pyrimidin-2-yl]-6-(difluoromethyl)-7-fluoro-1H-indole-3-sulfonamide;
6-cyclopropyl-N-[5-(2,2-difluoroethyl)-4-methoxy-pyrimidin-2-yl]-1H-indole-3-sulfonamide;
7-bromo-6-chloro-N-[5-(2-cyanoethyl)-4-methoxy-pyrimidin-2-yl]-1H-indole-3-sulfonamide;
N-[5-(difluoromethoxy)-4,6-dimethoxy-pyrimidin-2-yl]-6-fluoro-1H-indole-3-sulfonamide;
N-[5-(2,2-difluoroethoxy)-4,6-dimethoxy-pyrimidin-2-yl]-6-fluoro-1H-indole-3-sulfonamide;
N-[5-(2,2-difluoroethyl)-4,6-dimethoxy-pyrimidin-2-yl]-6-fluoro-1H-indole-3-sulfonamide;
N-[5-(2-cyanoethyl)-4,6-dimethoxy-pyrimidin-2-yl]-6-fluoro-1H-indole-3-sulfonamide;
6-chloro-N-[5-(2,2-difluoropropyl)-4-methoxy-pyrimidin-2-yl]-1H-indole-3-sulfonamide;
6-chloro-N-[4-methoxy-5-(2,2,3,3-tetrafluoropropyl)pyrimidin-2-yl]-1H-indole-3-sulfonamide;
6-chloro-N-[5-(difluoromethoxymethyl)-4-methoxy-pyrimidin-2-yl]-1H-indole-3-sulfonamide;
6-chloro-N-[4,6-dimethoxy-5-(oxetan-3-ylmethoxy)pyrimidin-2-yl]-1H-indole-3-sulfonamide;
6-chloro-N-[5-(2,3-difluoropropyl)-4,6-dimethoxy-pyrimidin-2-yl]-1H-indole-3-sulfonamide;
6-chloro-N-[5-(cyanomethoxy)-4,6-dimethoxy-pyrimidin-2-yl]-1H-indole-3-sulfonamide;
6-chloro-N-[4-methoxy-5-(2,3,3-trifluoropropyl)pyrimidin-2-yl]-1H-indole-3-sulfonamide;
6-chloro-N-[5-(cyanomethoxy)-4-methoxy-pyrimidin-2-yl]-1H-indole-3-sulfonamide;
6-bromo-N-[5-(difluoromethoxy)-4-methoxy-pyrimidin-2-yl]benzothiophene-3-sulfonamide;
6-bromo-N-[5-(difluoromethoxy)-4,6-dimethoxy-pyrimidin-2-yl]thieno[2,3-b]pyridine-3-sulfonamide;
6-bromo-N-[5-(3,3-difluoropropyl)-4-methoxy-pyrimidin-2-yl]thieno[2,3-b]pyridine-3-sulfonamide;
6-chloro-N-[5-(difluoromethoxy)-4,6-dimethoxy-pyrimidin-2-yl]thieno[2,3-b]pyridine-3-sulfonamide;
N-[5-(2,2-difluoroethyl)-4-methoxy-pyrimidin-2-yl]-6-(oxetan-3-yl)-1H-indole-3-sulfonamide;
N-(4,6-dimethoxy-5-methyl-pyrimidin-2-yl)-6-methoxy-1H-indole-3-sulfonamide;
N-[5-(2,2-difluoroethyl)-4-methoxy-pyrimidin-2-yl]-6-(1-hydroxy-1-methylethyl)-1H-indole-3-sulfonamide;
6-chloro-N-[5-(1,1-difluoro-2-hydroxy-ethoxy)-4,6-dimethoxy-pyrimidin-2-yl]-1H-indole-3-sulfonamide;
6-chloro-N-[5-(2,2-difluoroethyl)-4-methoxy-pyrimidin-2-yl]-7-methylsulfinyl-1H-indole-3-sulfonamide;
6-chloro-N-[5-(2,2-difluoroethyl)-4-methoxy-pyrimidin-2-yl]-7-mesyl-1H-indole-3-sulfonamide;
6-chloro-N-[5-(2-fluoroethoxy)-4,6-dimethoxy-pyrimidin-2-yl]-7-methylsulfinyl-1H-indole-3-sulfonamide;
6-chloro-N-[5-(2,2-difluoroethyl)-4,6-dimethoxy-pyrimidin-2-yl]-7-methylsulfinyl-1H-indole-3-sulfonamide;
6-chloro-7-cyano-N-[5-(2-fluoroethoxy)-4-methoxy-pyrimidin-2-yl]-1H-indole-3-sulfonamide;
6-chloro-N-[4,6-dimethoxy-5-(2-methylsulfonylethyl)pyrimidin-2-yl]-1H-indole-3-sulfonamide;
N-[5-(2,2-difluoroethyl)-4-methoxy-pyrimidin-2-yl]-1H-pyrrolo[2,3-e][1,2]benzothiazole-3-sulfonamide;
7-bromo-6-chloro-N-[5-(2,2-difluoroethyl)-4,6-dimethoxy-pyrimidin-2-yl]-1H-indole-3-sulfonamide;
N-[5-(cyanomethoxy)-4,6-dimethoxy-pyrimidin-2-yl]-6-methyl-1H-pyrrolo[2,3-b]pyridine-3-sulfonamide;
N-[5-(2,2-difluoroethoxy)-4,6-dimethoxy-pyrimidin-2-yl]-6-methyl-1H-pyrrolo[2,3-b]pyridine-3-sulfonamide;
N-[5-(difluoromethoxy)-4,6-dimethoxy-pyrimidin-2-yl]-6-methyl-1H-pyrrolo[2,3-b]pyridine-3-sulfonamide;
6-(difluoromethyl)-N-[5-(2,3-difluoropropyl)-4,6-dimethoxy-pyrimidin-2-yl]-1H-pyrrolo[2,3-b]pyridine-3-sulfonamide;
6-bromo-N-[5-(2,3-difluoropropyl)-4,6-dimethoxy-pyrimidin-2-yl]-1H-pyrrolo[2,3-b]pyridine-3-sulfonamide;
6-chloro-N-[5-(2,3-difluoropropyl)-4,6-dimethoxy-pyrimidin-2-yl]-1H-pyrrolo[2,3-b]pyridine-3-sulfonamide;
N-[5-(2,2-difluoroethyl)-4-methoxy-pyrimidin-2-yl]-8H-pyrrolo[2,3-e][1,3]benzothiazole-6-sulfonamide;
3-[[5-(2,2-difluoroethyl)-4-methoxy-pyrimidin-2-yl]sulfamoyl]-6-methyl-1H-indole-7-carboxylic acid methyl ester
N-[5-(2,2-difluoroethyl)-4-methoxy-pyrimidin-2-yl]-6-methyl-7-methylol-1H-indole-3-sulfonamide;
N-[5-(2-fluoroethoxy)-4-methoxy-pyrimidin-2-yl]-9-keto-8-methyl-6,7-dihydro-1H-pyrrol[3,2-h]isoquinoline-3-sulfonamide;
6-chloro-7-(cyanomethoxy)-N-[5-(2,2-difluoroethyl)-4,6-dimethoxy-pyrimidin-2-yl]-1H-indole-3-sulfonamide;
and pharmaceutically acceptable salts thereof.

12. A compound according to any of claims 1 to 11, selected from
6-chloro-7-fluoro-N-[5-(2-fluoroethoxy)-4-methoxy-pyrimidin-2-yl]-1H-indole-3-sulfonamide;
6,7-dichloro-N-[5-(2-fluoroethoxy)-4-methoxy-pyrimidin-2-yl]-1H-indole-3-sulfonamide;
6-chloro-7-(difluoromethyl)-N-[5-(2-fluoroethoxy)-4-methoxy-pyrimidin-2-yl]-1H-indole-3-sulfonamide;
N-(5-bromo-4,6-dimethoxy-pyrimidin-2-yl)-6-chloro-1H-indole-3-sulfonamide;
6-chloro-N-(5-ethyl-4,6-dimethoxy-pyrimidin-2-yl)-1H-indole-3-sulfonamide;
6-chloro-N-(4,6-dimethoxy-5-methyl-pyrimidin-2-yl)-1H-indole-3-sulfonamide;
6-chloro-N-[4-cyclopropyl-5-(2,2-difluoroethoxy)-6-methoxy-pyrimidin-2-yl]-1H-indole-3-sulfonamide;
6-chloro-N-[5-(3-fluoropropyl)-4-methoxy-pyrimidin-2-yl]-1H-indole-3-sulfonamide;
6-bromo-N-[5-(2-fluoroethoxy)-4-methoxy-pyrimidin-2-yl]-1H-indole-3-sulfonamide;
6-bromo-N-(5-bromo-4,6-dimethoxy-pyrimidin-2-yl)-1H-pyrrolo[2,3-b]pyridine-3-sulfonamide;
6-chloro-N-[4-methoxy-5-(1,1,2-trifluoroethoxy)pyrimidin-2-yl]-1H-indole-3-sulfonamide;
6-chloro-N-[5-(2,2-difluoroethyl)-4-methoxy-pyrimidin-2-yl]-1H-indole-3-sulfonamide;
6-chloro-N-[5-(3,3-difluoropropyl)-4-methoxy-pyrimidin-2-yl]-1H-indole-3-sulfonamide;
6-chloro-N-[5-(difluoromethoxy)-4,6-dimethoxy-pyrimidin-2-yl]-1H-indole-3-sulfonamide;
6-chloro-N-[5-(2-fluoroethoxy)-4,6-dimethoxy-pyrimidin-2-yl]-1H-indole-3-sulfonamide;
6-chloro-N-[5-(2,2-difluoroethoxy)-4,6-dimethoxy-pyrimidin-2-yl]-1H-indole-3-sulfonamide;
6-chloro-N-[4,6-dimethoxy-5-(1,1,2-trifluoroethoxy)pyrimidin-2-yl]-1H-indole-3-sulfonamide;
N-(5-allyl-4,6-dimethoxy-pyrimidin-2-yl)-6-chloro-1H-indole-3-sulfonamide;
6-chloro-N-(4,6-dimethoxy-5-prop-1-ynyl-pyrimidin-2-yl)-1H-indole-3-sulfonamide;
6-chloro-N-(4,6-dimethoxy-5-propyl-pyrimidin-2-yl)-1H-indole-3-sulfonamide;
6-chloro-N-[5-(2-fluoroethyl)-4,6-dimethoxy-pyrimidin-2-yl]-1H-indole-3-sulfonamide;
6-chloro-N-[5-(2,2-difluoroethyl)-4,6-dimethoxy-pyrimidin-2-yl]-1H-indole-3-sulfonamide;
6-chloro-N-[5-(3-fluoropropyl)-4,6-dimethoxy-pyrimidin-2-yl]-1H-indole-3-sulfonamide;
6-chloro-N-[5-(3,3-difluoropropyl)-4,6-dimethoxy-pyrimidin-2-yl]-1H-indole-3-sulfonamide;
6-chloro-N-[4,6-dimethoxy-5-(3,3,3-trifluoropropyl)pyrimidin-2-yl]-1H-indole-3-sulfonamide;
6-chloro-N-[4,6-dimethoxy-5-(2-methoxyethyl)pyrimidin-2-yl]-1H-indole-3-sulfonamide;
6-chloro-N-[5-(2-cyanoethyl)-4,6-dimethoxy-pyrimidin-2-yl]-1H-indole-3-sulfonamide;
6-chloro-N-(5-cyclopropyl-4,6-dimethoxy-pyrimidin-2-yl)-1H-indole-3-sulfonamide;
6-chloro-N-[4-(difluoromethoxy)-6-methoxy-5-methyl-pyrimidin-2-yl]-1H-indole-3-sulfonamide;
6-bromo-N-[5-(2,2-difluoroethyl)-4-methoxy-pyrimidin-2-yl]-1H-indole-3-sulfonamide;
6-bromo-N-[5-(difluoromethoxy)-4,6-dimethoxy-pyrimidin-2-yl]-1H-indole-3-sulfonamide;
6-bromo-N-[5-(2-fluoroethoxy)-4,6-dimethoxy-pyrimidin-2-yl]-1H-indole-3-sulfonamide;
6-bromo-N-[5-(2,2-difluoroethoxy)-4,6-dimethoxy-pyrimidin-2-yl]-1H-indole-3-sulfonamide;
6-bromo-N-[5-(2-cyanoethyl)-4,6-dimethoxy-pyrimidin-2-yl]-1H-indole-3-sulfonamide;
N-[5-(2,2-difluoroethoxy)-4,6-dimethoxy-pyrimidin-2-yl]-6-(difluoromethyl)-1H-pyrrolo[2,3-b]pyridine-3-sulfonamide;
6-(difluoromethyl)-N-[4,6-dimethoxy-5-(1,1,2-trifluoroethoxy)pyrimidin-2-yl]-1H-pyrrolo[2,3-b]pyridine-3-sulfonamide;
6-(difluoromethyl)-N-[5-(3,3-difluoropropyl)-4,6-dimethoxy-pyrimidin-2-yl]-1H-pyrrolo[2,3-b]pyridine-3-sulfonamide;
N-[5-(difluoromethoxy)-4,6-dimethoxy-pyrimidin-2-yl]-6-(difluoromethyl)-1H-indole-3-sulfonamide;
N-[5-(2,2-difluoroethoxy)-4,6-dimethoxy-pyrimidin-2-yl]-6-(difluoromethyl)-1H-indole-3-sulfonamide;
N-[5-(2-cyanoethyl)-4,6-dimethoxy-pyrimidin-2-yl]-6-(difluoromethyl)-1H-indole-3-sulfonamide;
6-chloro-N-[5-(2-cyanoethyl)-4-methoxy-pyrimidin-2-yl]-7-(difluoromethylthio)-1H-indole-3-sulfonamide;
6-chloro-7-(difluoromethylthio)-N-[5-(2-fluoroethoxy)-4,6-dimethoxy-pyrimidin-2-yl]-1H-indole-3-sulfonamide;
6-chloro-N-[5-(2-cyanoethyl)-4,6-dimethoxy-pyrimidin-2-yl]-7-(difluoromethylthio)-1H-indole-3-sulfonamide;
6-bromo-N-[5-(2-fluoroethoxy)-4,6-dimethoxy-pyrimidin-2-yl]-1H-pyrrolo[2,3-b]pyridine-3-sulfonamide;
6-bromo-N-[4,6-dimethoxy-5-(1,1,2-trifluoroethoxy)pyrimidin-2-yl]-1H-pyrrolo[2,3-b]pyridine-3-sulfonamide;
6-bromo-N-[5-(2,2-difluoroethyl)-4,6-dimethoxy-pyrimidin-2-yl]-1H-pyrrolo[2,3-b]pyridine-3-sulfonamide;
6-bromo-N-[5-(3,3-difluoropropyl)-4,6-dimethoxy-pyrimidin-2-yl]-1H-pyrrolo[2,3-b]pyridine-3-sulfonamide;
6-bromo-N-[5-(2-cyanoethyl)-4,6-dimethoxy-pyrimidin-2-yl]-1H-pyrrolo[2,3-b]pyridine-3-sulfonamide;
6-bromo-N-[5-(cyanomethoxy)-4,6-dimethoxy-pyrimidin-2-yl]-1H-pyrrolo[2,3-b]pyridine-3-sulfonamide;
6-chloro-N-[5-(2,2-difluoroethyl)-4,6-dimethoxy-pyrimidin-2-yl]-7-(dimethylamino)-1H-indole-3-sulfonamide;
N-[5-(difluoromethoxy)-4,6-dimethoxy-pyrimidin-2-yl]-1H-pyrrolo[3,2-h]quinoline-3-sulfonamide;
N-[5-(2-fluoroethoxy)-4,6-dimethoxy-pyrimidin-2-yl]-1H-pyrrolo[3,2-h]quinoline-3-sulfonamide;
6-chloro-N-(4,6-dimethoxy-5-methyl-pyrimidin-2-yl)-1H-pyrrolo[2,3-b]pyridine-3-sulfonamide;
6-chloro-N-[5-(2,2-difluoroethyl)-4,6-dimethoxy-pyrimidin-2-yl]-1H-pyrrolo[2,3-b]pyridine-3-sulfonamide;
6-chloro-N-[5-(3,3-difluoropropyl)-4,6-dimethoxy-pyrimidin-2-yl]-1H-pyrrolo[2,3-b]pyridine-3-sulfonamide;
6-chloro-N-[5-(2-cyanoethyl)-4,6-dimethoxy-pyrimidin-2-yl]-1H-pyrrolo[2,3-b]pyridine-3-sulfonamide;
6-chloro-N-[5-(2,2-difluoroethyl)-4-methoxy-pyrimidin-2-yl]-7-fluoro-1H-indole-3-sulfonamide;
6-chloro-N-[5-(difluoromethoxy)-4,6-dimethoxy-pyrimidin-2-yl]-7-fluoro-1H-indole-3-sulfonamide;
6-chloro-7-(cyclopropoxy)-N-[5-(2,2-difluoroethyl)-4-methoxy-pyrimidin-2-yl]-1H-indole-3-sulfonamide;
N-[5-(2,2-difluoroethyl)-4,6-dimethoxy-pyrimidin-2-yl]-6-methyl-1H-pyrrolo[2,3-b]pyridine-3-sulfonamide;
N-[5-(2,2-difluoroethyl)-4-methoxy-pyrimidin-2-yl]-6-fluoro-1H-indole-3-sulfonamide;
7-bromo-6-chloro-N-[5-(2-fluoroethoxy)-4-methoxy-pyrimidin-2-yl]-1H-indole-3-sulfonamide;
7-bromo-6-chloro-N-[5-(2,2-difluoroethyl)-4-methoxy-pyrimidin-2-yl]-1H-indole-3-sulfonamide;
6-bromo-N-(4,6-dimethoxy-5-methyl-pyrimidin-2-yl)thieno[2,3-b]pyridine-3-sulfonamide;
6-bromo-N-[5-(2,2-difluoroethyl)-4-methoxy-pyrimidin-2-yl]thieno[2,3-b]pyridine-3-sulfonamide;
6-bromo-N-[5-(2,2-difluoroethyl)-4,6-dimethoxy-pyrimidin-2-yl]thieno[2,3-b]pyridine-3-sulfonamide;
6-bromo-N-[5-(2-cyanoethyl)-4,6-dimethoxy-pyrimidin-2-yl]thieno[2,3-b]pyridine-3-sulfonamide;
6-chloro-N-[5-(2,2-difluoroethyl)-4-methoxy-pyrimidin-2-yl]benzothiophene-3-sulfonamide;
6-bromo-N-[5-(2-fluoroethoxy)-4-methoxy-pyrimidin-2-yl]benzothiophene-3-sulfonamide;
6-bromo-N-(4,6-dimethoxy-5-methyl-pyrimidin-2-yl)-1H-pyrrolo[2,3-b]pyridine-3-sulfonamide;
N-[5-(2,2-difluoroethoxy)-4,6-dimethoxy-pyrimidin-2-yl]-1H-pyrrolo[3,2-h]quinoline-3-sulfonamide;
N-[5-(2,2-difluoroethyl)-4,6-dimethoxy-pyrimidin-2-yl]-1H-pyrrolo[3,2-h]quinoline-3-sulfonamide;
6-bromo-N-[5-(difluoromethoxy)-4,6-dimethoxy-pyrimidin-2-yl]-1H-pyrrolo[2,3-b]pyridine-3-sulfonamide;
6-chloro-N-[5-(2,3-difluoropropyl)-4-methoxy-pyrimidin-2-yl]-1H-indole-3-sulfonamide;
6-chloro-N-[5-(2-cyanoethyl)-4-methoxy-pyrimidin-2-yl]-1H-indole-3-sulfonamide;
7-bromo-6-chloro-N-[5-(3,3-difluoropropyl)-4-methoxy-pyrimidin-2-yl]-1H-indole-3-sulfonamide;
6-bromo-N-[5-(2,2-difluoroethyl)-4-methoxy-pyrimidin-2-yl]-7-methyl-1H-indole-3-sulfonamide;
6-chloro-N-[5-(2,2-difluoroethyl)-4-methoxy-pyrimidin-2-yl]-7-methoxy-1H-indole-3-sulfonamide;
6-chloro-N-[5-(3,3-difluoropropyl)-4-methoxy-pyrimidin-2-yl]-7-methoxy-1H-indole-3-sulfonamide;
6-chloro-N-[5-(2,2-difluoroethyl)-4-methoxy-pyrimidin-2-yl]-7-(difluoromethoxy)-1H-indole-3-sulfonamide;
6-bromo-N-[5-(2,2-difluoroethyl)-4-methoxy-pyrimidin-2-yl]-7-fluoro-1H-indole-3-sulfonamide;
6-chloro-N-[5-(2,2-difluoroethyl)-4-methoxy-pyrimidin-2-yl]-7-methyl-1H-indole-3-sulfonamide;
N-[5-(2,2-difluoroethyl)-4,6-dimethoxy-pyrimidin-2-yl]-6-(difluoromethyl)-1H-pyrrolo[2,3-b]pyridine-3-sulfonamide;
6-bromo-N-[5-(2,2-difluoroethyl)-4-methoxy-pyrimidin-2-yl]-7-methoxy-1H-indole-3-sulfonamide;
6-chloro-N-[5-(2,2-difluoroethyl)-4-methoxy-pyrimidin-2-yl]-7-(fluoromethoxy)-1H-indole-3-sulfonamide;
6-chloro-N-[5-(2,2-difluoroethyl)-4-methoxy-pyrimidin-2-yl]-7-(difluoromethylsulfanyl)-1H-indole-3-sulfonamide;
6-bromo-N-[5-(3,3-difluoropropyl)-4-methoxy-pyrimidin-2-yl]-1H-pyrrolo[2,3-b]pyridine-3-sulfonamide;
6-bromo-N-[5-(2,2-difluoroethoxy)-4,6-dimethoxy-pyrimidin-2-yl]-1H-pyrrolo[2,3-b]pyridine-3-sulfonamide;
6-chloro-N-[5-(3,3-difluoropropyl)-4-methoxy-pyrimidin-2-yl]-1H-pyrrolo[2,3-b]pyridine-3-sulfonamide;
6-chloro-N-[5-(difluoromethoxy)-4,6-dimethoxy-pyrimidin-2-yl]-1H-pyrrolo[2,3-b]pyridine-3-sulfonamide;
6-chloro-N-[5-(3,3-difluoropropyl)-4-methoxy-pyrimidin-2-yl]-7-fluoro-1H-indole-3-sulfonamide;
6-chloro-N-[5-(2,2-difluoroethoxy)-4,6-dimethoxy-pyrimidin-2-yl]-7-fluoro-1H-indole-3-sulfonamide;
6,7-dichloro-N-[5-(2,2-difluoroethyl)-4-methoxy-pyrimidin-2-yl]-1H-indole-3-sulfonamide;
6,7-dichloro-N-[5-(3,3-difluoropropyl)-4-methoxy-pyrimidin-2-yl]-1H-indole-3-sulfonamide;
6-chloro-7-(cyclopropoxy)-N-[5-(3,3-difluoropropyl)-4-methoxy-pyrimidin-2-yl]-1H-indole-3-sulfonamide;
6-bromo-7-fluoro-N-[5-(2-fluoroethoxy)-4-methoxy-pyrimidin-2-yl]-1H-indole-3-sulfonamide;
6-chloro-N-[5-(2,2-difluoroethyl)-4-methoxy-pyrimidin-2-yl]-7-(difluoromethyl)-1H-indole-3-sulfonamide;
6-chloro-7-fluoro-N-[4-methoxy-5-(1,1,2-trifluoroethoxy)pyrimidin-2-yl]-1H-indole-3-sulfonamide;
6-chloro-N-[5-(2-cyanoethyl)-4-methoxy-pyrimidin-2-yl]-7-fluoro-1H-indole-3-sulfonamide;
6-chloro-N-[5-(2,2-difluoroethyl)-4,6-dimethoxy-pyrimidin-2-yl]-7-fluoro-1H-indole-3-sulfonamide;
6-chloro-N-[5-(2-cyanoethyl)-4,6-dimethoxy-pyrimidin-2-yl]-7-fluoro-1H-indole-3-sulfonamide;
6-chloro-N-[5-(cyanomethoxy)-4,6-dimethoxy-pyrimidin-2-yl]-7-fluoro-1H-indole-3-sulfonamide;
6-chloro-N-[4-methoxy-5-(1,1,2-trifluoroethoxy)pyrimidin-2-yl]-1H-pyrrolo[2,3-b]pyridine-3-sulfonamide;
6-chloro-N-[5-(2-fluoroethoxy)-4,6-dimethoxy-pyrimidin-2-yl]-1H-pyrrolo[2,3-b]pyridine-3-sulfonamide;
6-chloro-7-cyano-N-[5-(2,2-difluoroethyl)-4-methoxy-pyrimidin-2-yl]-1H-indole-3-sulfonamide;
6-chloro-7-cyano-N-[5-(difluoromethoxy)-4,6-dimethoxy-pyrimidin-2-yl]-1H-indole-3-sulfonamide;
6-chloro-7-cyano-N-[5-(2-fluoroethoxy)-4,6-dimethoxy-pyrimidin-2-yl]-1H-indole-3-sulfonamide;
6-chloro-7-cyano-N-[5-(2,2-difluoroethoxy)-4,6-dimethoxy-pyrimidin-2-yl]-1H-indole-3-sulfonamide;
6-chloro-7-cyano-N-[5-(2,2-difluoroethyl)-4,6-dimethoxy-pyrimidin-2-yl]-1H-indole-3-sulfonamide;
N-[5-(2,2-difluoroethyl)-4,6-dimethoxy-pyrimidin-2-yl]-6,8-dihydro-1H-fur[3,4-g]indole-3-sulfonamide;
6-bromo-N-[5-(2-fluoroethoxy)-4-methoxy-pyrimidin-2-yl]-1H-pyrrolo[2,3-b]pyridine-3-sulfonamide;
6-bromo-N-[4-methoxy-5-(1,1,2-trifluoroethoxy)pyrimidin-2-yl]-1H-pyrrolo[2,3-b]pyridine-3-sulfonamide;
6-chloro-7-(cyanomethyl)-N-[5-(2,2-difluoroethyl)-4-methoxy-pyrimidin-2-yl]-1H-indole-3-sulfonamide;
6-chloro-N-[5-(2,2-difluoroethyl)-4-methoxy-pyrimidin-2-yl]-7-(methylthio)-1H-indole-3-sulfonamide;
6-(difluoromethyl)-N-[5-(2-fluoroethoxy)-4-methoxy-pyrimidin-2-yl]-1H-indole-3-sulfonamide;
N-[5-(2,2-difluoroethyl)-4,6-dimethoxy-pyrimidin-2-yl]-6-(difluoromethyl)-1H-indole-3-sulfonamide;
N-[5-(cyanomethoxy)-4,6-dimethoxy-pyrimidin-2-yl]-6-(difluoromethyl)-1H-indole-3-sulfonamide;
N-[5-(2-cyanoethyl)-4,6-dimethoxy-pyrimidin-2-yl]-6-(difluoromethyl)-1H-pyrrolo[2,3-b]pyridine-3-sulfonamide;
6-bromo-N-[5-(2-cyanoethyl)-4-methoxy-pyrimidin-2-yl]-7-fluoro-1H-indole-3-sulfonamide;
6-bromo-N-[5-(difluoromethoxy)-4,6-dimethoxy-pyrimidin-2-yl]-7-fluoro-1H-indole-3-sulfonamide;
6-bromo-N-[5-(2,2-difluoroethoxy)-4,6-dimethoxy-pyrimidin-2-yl]-7-fluoro-1H-indole-3-sulfonamide;
6-bromo-N-[5-(2-cyanoethyl)-4, 6-dimethoxy-pyrimidin-2-yl]-7-fluoro-1H-indole-3-sulfonamide;
6-bromo-N-[5-(cyanomethoxy)-4,6-dimethoxy-pyrimidin-2-yl]-7-fluoro-1H-indole-3-sulfonamide;
6-bromo-N-[5-(2,2-difluoroethyl)-4,6-dimethoxy-pyrimidin-2-yl]-1H-indole-3-sulfonamide;
6-bromo-N-[5-(cyanomethoxy)-4,6-dimethoxy-pyrimidin-2-yl]-1H-indole-3-sulfonamide;
6-(difluoromethyl)-7-fluoro-N-[5-(2-fluoroethoxy)-4-methoxy-pyrimidin-2-yl]-1H-indole-3-sulfonamide;
N-[5-(difluoromethoxy)-4,6-dimethoxy-pyrimidin-2-yl]-6-(difluoromethyl)-7-fluoro-1H-indole-3-sulfonamide;
N-[5-(2,2-difluoroethoxy)-4,6-dimethoxy-pyrimidin-2-yl]-6-(difluoromethyl)-7-fluoro-1H-indole-3-sulfonamide;
N-[5-(2,2-difluoroethyl)-4,6-dimethoxy-pyrimidin-2-yl]-6-(difluoromethyl)-7-fluoro-1H-indole-3-sulfonamide;
N-[5-(2-cyanoethyl)-4,6-dimethoxy-pyrimidin-2-yl]-6-(difluoromethyl)-7-fluoro-1H-indole-3-sulfonamide;
N-[5-(cyanomethoxy)-4,6-dimethoxy-pyrimidin-2-yl]-6-(difluoromethyl)-7-fluoro-1H-indole-3-sulfonamide;
7-bromo-6-chloro-N-[5-(2-cyanoethyl)-4-methoxy-pyrimidin-2-yl]-1H-indole-3-sulfonamide;
N-[5-(2,2-difluoroethyl)-4,6-dimethoxy-pyrimidin-2-yl]-6-fluoro-1H-indole-3-sulfonamide;
N-[5-(2-cyanoethyl)-4,6-dimethoxy-pyrimidin-2-yl]-6-fluoro-1H-indole-3-sulfonamide;
6-chloro-N-[5-(difluoromethoxymethyl)-4-methoxy-pyrimidin-2-yl]-1H-indole-3-sulfonamide;
6-chloro-N-[5-(2,3-difluoropropyl)-4,6-dimethoxy-pyrimidin-2-yl]-1H-indole-3-sulfonamide;
6-chloro-N-[5-(cyanomethoxy)-4,6-dimethoxy-pyrimidin-2-yl]-1H-indole-3-sulfonamide;
6-chloro-N-[5-(cyanomethoxy)-4-methoxy-pyrimidin-2-yl]-1H-indole-3-sulfonamide;
7-bromo-6-chloro-N-[5-(2,2-difluoroethyl)-4,6-dimethoxy-pyrimidin-2-yl]-1H-indole-3-sulfonamide;
6-chloro-7-(cyanomethoxy)-N-[5-(2,2-difluoroethyl)-4,6-dimethoxy-pyrimidin-2-yl]-1H-indole-3-sulfonamide;
and pharmaceutically acceptable salts thereof.

13. A compound according to claim 11 or claim 12, wherein the compound is 6-bromo-N-[5-(difluoromethoxy)-4,6-dimethoxy-pyrimidin-2-yl]-1H-pyrrolo[2,3-b]pyridine-3-sulfonamide or a pharmaceutically acceptable salt thereof.

14. A compound according to any of claims 11 to 13, wherein the compound is 6-bromo-N-[5-(difluoromethoxy)-4,6-dimethoxy-pyrimidin-2-yl]-1H-pyrrolo[2,3-b]pyridine-3-sulfonamide.

15. A process to prepare a compound according to any one of claims 1 to 14 comprising the reaction of a compound of II 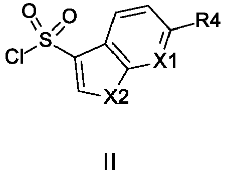
with a compound of formula III 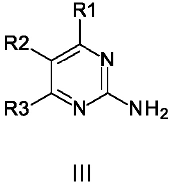
in the presence of a base selected from N,N-diisopropylethylamine or pyridine, wherein R¹, R², R³, R⁴, X₁ and X₂ are as defined above.

16. A compound according to any one of claims 1 to 14 for use as therapeutically active substance.

17. A pharmaceutical composition comprising a compound according to any one of claims 1 to 14 and a therapeutically inert carrier.

18. A compound according to any one of claims 1 to 14 for use in the treatment or prophylaxis of conditions resulting from direct damage to myelin sheaths (including but not limited central pontine and extra-pontine myelinolysis, carbon monoxide poisoning, nutritional deficiency, and virus-induced demyelination), demyelinating disorders (including but not limited to multiple sclerosis, acute and multiphasic disseminated encephalomyelitis, neuromyelitis optica spectrum disorders, and leukodystrophies), CNS disorders associated with myelin loss (including but not limited to Alzheimer's disease, schizophrenia, Parkinson's disease, Huntington's disease, Amyotrophic lateral sclerosis, and Ischemia due to stroke), and inflammation in the CNS for instance following encephalitis, primary angiitis, meningitis and obesity.

19. A compound according to any one of claims 1 to 14 for use in the treatment or prophylaxis of multiple sclerosis.

## Patentansprüche

1. Verbindungen der Formel I 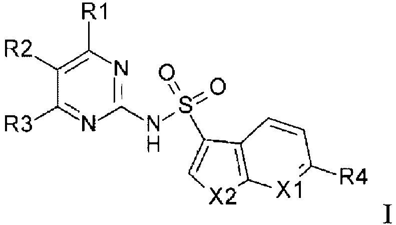 wobei
R¹ H, Halogen, Alkyl, Alkoxy, Alkoxyalkyl, Alkoxyalkoxy, Cyanoalkyl, Halogenalkyl, Halogenalkoxy, Thiolalkyl, Cycloalkyl, Cycloalkylalkyl, Cycloalkoxy oder Heterocycloalkyl ist;
R² Cyano, Halogen, Alkyl, Alkenyl, Alkinyl, Alkoxy, Alkenylalkyl, Alkinylalkoxy, Alkoxyalkyl, Alkoxyalkoxy, Halogenalkyl, Halogenalkoxy, Halogenalkoxyalkyl, (Halogenalkoxy)alkoxy, Cyanoalkyl, Cyanoalkoxy, Thiolalkyl, Halogenthiolalkyl, Halogenhydroxyalkoxy, Alkylsulfonylalkyl, Cycloalkyl, Cycloalkylalkyl, Cycloalkylalkoxy, Cycloalkoxy, Heterocycloalkyl, Heterocycloalkylalkyl, Heterocycloalkylalkinyl oder Heterocycloalkylalkoxy ist;
R³ H, Halogen, Alkyl, Alkoxy, Halogenalkoxy oder Thiolalkyl ist;
X₁ CR⁵ oder N ist;
X₂ NH, -N-R⁶, S, -S(O)- oder -S(O₂)- ist;
R⁴ H, Halogen, Alkyl, Hydroxyalkyl, Thiolalkyl, Cyanoalkyl, Alkoxy, Halogenalkyl, Halogenalkoxy, Cycloalkyl oder Heterocycloalkyl ist;
R⁵ H, Halogen, Cyano, Alkyl, Alkoxy, Cyanoalkyl, Cyanoalkoxy, N-Alkyl, Dialkylamino,
Halogenalkyl, Halogenalkoxy, Halogenthiolalkyl, Alkylsulfinyl, Alkylsulfonyl, Alkylsulfon, Thiolalkyl, Hydroxyalkyl, Alkoxycarbonyl, Heterocycloalkyl, Cycloalkyl oder Cycloalkoxy ist;
oder R⁴ und R⁵ verbunden sind, um ein 5- oder 6-gliedriges Cycloalkyl, Heterocycloalkyl, substituiertes Heterocycloalkyl, umfassend einen oder zwei Substituenten, die unabhängig voneinander ausgewählt sind aus Alkyl und Oxo, oder einen heteroaromatischen Ring zu bilden;
R⁶ H, Alkyl, Alkylhydroxy oder Halogenalkyl ist;
und pharmazeutisch verträgliche Salze.

2. Verbindung nach Anspruch 1, wobei R¹ H, Halogen, Halogenalkyl, Halogenalkoxy, Alkyl, Alkoxy, Alkoxyalkoxy, Alkoxyalkyl, Thiolalkyl, Cyanoalkyl, Cycloalkoxy, Cycloalkyl, Cycloalkylalkyl oder Heterocycloalkyl ist.

3. Verbindung nach den Ansprüchen 1-2, wobei R² Alkoxy, Alkoxyalkoxy, Alkoxyalkyl, Alkylsulfonylalkyl, Alkyl, Alkenyl, Alkinyl, Alkinylalkoxy, Cyano, Cyanoalkyl, Cycloalkyl, Cycloalkoxy, Cycloalkylalkyl, Cyanoalkoxy, Halogen, Halogenalkoxy, (Halogenalkoxy)alkoxy, Thiolalkyl, Halogenthiolalkyl, Halogenhydroxyalkoxy, Halogenalkyl, Heterocycloalkyl, Heterocycloalkylalkyl, Heterocycloalkylalkinyl oder Heterocycloalkylalkoxy ist.

4. Verbindung nach den Ansprüchen 1-3, wobei R³ H oder Alkoxy ist.

5. Verbindung nach den Ansprüchen 1-4, wobei X² NH oder S ist.

6. Verbindung nach den Ansprüchen 1-5, wobei R⁴ H, Halogen, Alkyl, Alkoxy, Thiolalkyl, Cyanoalkyl, Cycloalkyl, Halogenalkoxy, Halogenalkyl, Heterocycloalkyl oder Hydroxyalkyl ist und R⁵ H, Halogen, Halogenalkyl, Halogenalkoxy, Halogenthiolalkyl, Alkoxy, Alkoxycarbonyl, Alkyl, Hydroxyalkyl, Thiolalkyl, Alkylsulfino, Alkylsulfano, Cyano, Cyanoalkyl, Cyanoalkoxy, Dialkylamino, Cycloalkyl, Cycloalkoxy oder Heterocycloalkyl ist, oder R⁴ und R⁵ verbunden sind, um ein 5-gliedriges Heterocycloalkyl, umfassend ein einzelnes O-Heteroatom, ein 6-gliedriges Heterocycloalkyl, umfassend ein einzelnes N-Heteroatom, substituiert mit Oxo und Alkyl, einen 5-gliedrigen heteroaromatischen Ring, umfassend ein N-Heteroatom und ein S-Heteroatom, oder einen 6-gliedrigen heteroaromatischen Ring, umfassend ein einzelnes N-Heteroatom, zu bilden.

7. Verbindung nach den Ansprüchen 1-6, wobei R⁴ Halogen, Alkyl oder Halogenalkyl ist und R⁵ H, Halogen, Halogenalkyl, Halogenalkoxy, Halogenthiolalkyl, Alkoxy, Alkyl, Thiolalkyl, Cyano, Cyanoalkyl, Cyanoalkoxy, Dialkylamino oder Cycloalkoxy ist oder R⁴ und R⁵ verbunden sind, um ein 5-gliedriges Heterocycloalkyl, umfassend ein einzelnes O-Heteroatom, oder einen 6-gliedrigen heteroaromatischen Ring, umfassend ein einzelnes N-Heteroatom, zu bilden.

8. Verbindung nach den Ansprüchen 1-7, wobei R⁵ H, Halogen, Halogenalkyl, Halogenalkoxy, Halogenthiolalkyl, Alkoxy, Alkyl, Thiolalkyl, Cyano, Cyanoalkyl, Cyanoalkoxy, Dialkylamino oder Cycloalkoxy ist.

9. Verbindung nach den Ansprüchen 1-8, wobei
R¹ H, Halogen, Halogenalkyl, Halogenalkoxy, Alkyl, Alkoxy, Alkoxyalkoxy, Alkoxyalkyl, Thiolalkyl, Cyanoalkyl, Cycloalkoxy, Cycloalkyl, Cycloalkylalkyl oder Heterocycloalkyl ist;
R² Alkoxy, Alkoxyalkoxy, Alkoxyalkyl, Alkylsulfonylalkyl, Alkyl, Alkenyl, Alkinyl, Alkinylalkoxy, Cyano, Cyanoalkyl, Cycloalkyl, Cycloalkoxy, Cycloalkylalkyl, Cyanoalkoxy, Halogen, Halogenalkoxy, (Halogenalkoxy)alkoxy, Thiolalkyl, Halogenthiolalkyl, Halogenhydroxyalkoxy, Halogenalkyl, Heterocycloalkyl, Heterocycloalkylalkyl, Heterocycloalkylalkinyl oder Heterocycloalkylalkoxy ist;
R³ H, Halogen, Alkyl, Alkoxy, Halogenalkoxy oder Thiolalkyl ist;
X₁ CR⁵ oder N ist;
X₂ NH oder S ist;
R⁴ H, Halogen, Alkyl, Alkoxy, Thiolalkyl, Cyanoalkyl, Cycloalkyl, Halogenalkoxy, Halogenalkyl, Heterocycloalkyl oder Hydroxyalkyl ist und R⁵ H, Halogen, Halogenalkyl, Halogenalkoxy, Halogenthiolalkyl, Alkoxy, Alkyl, Alkoxycarbonyl, Hydroxyalkyl, Thiolalkyl, Alkylsulfino, Alkylsulfano, Cyano, Cyanoalkyl, Cyanoalkoxy, Dialkylamino, Cycloalkyl, Cycloalkoxy oder Heterocycloalkyl ist, oder R⁴ und R⁵ verbunden sind, um ein 5-gliedriges Heterocycloalkyl, umfassend ein einzelnes O-Heteroatom, ein 6-gliedriges Heterocycloalkyl, umfassend ein einzelnes N-Heteroatom, substituiert mit Alkyl und Oxo, einen 5-gliedrigen heteroaromatischen Ring, umfassend ein N-Heteroatom und ein S-Heteroatom, oder einen 6-gliedrigen heteroaromatischen Ring, umfassend ein einzelnes N-Heteroatom, zu bilden;
und pharmazeutisch verträgliche Salze.

10. Verbindung nach einem der Ansprüche 1 bis 9, wobei
R¹ H, Alkoxy, Cycloalkyl oder Halogenalkoxy ist;
R² Alkyl, Alkenyl, Alkinyl, Alkoxyalkyl, Alkinyl, Cyanoalkoxy, Halogen, Halogenalkoxy, Halogenalkyl oder Cycloalkyl ist;
R³ H oder Alkoxy ist;
X₁ CR⁵ oder N ist;
X₂ NH oder S ist;
R⁴ Halogen, Alkyl oder Halogenalkyl ist und R⁵ H, Halogen, Halogenalkyl, Halogenalkoxy, Halogenthiolalkyl, Alkoxy, Alkyl, Thiolalkyl, Cyano, Cyanoalkyl, Cyanoalkoxy, Dialkylamino oder Cycloalkoxy ist, oder R⁴ und R⁵ verbunden sind, um ein 5-gliedriges Heterocycloalkyl, umfassend ein einzelnes O-Heteroatom, oder einen 6-gliedrigen heteroaromatischen Ring, umfassend ein einzelnes N-Heteroatom, zu bilden;
und pharmazeutisch verträgliche Salze.

11. Verbindung nach einem der Ansprüche 1 bis 10, ausgewählt aus
6-Chlor-N-[5-(2-fluorethoxy)-4-methoxypyrimidin-2-yl]-1H-indol-3-sulfonamid;
6-Chlor-7-fluor-N-[5-(2-fluorethoxy)-4-methoxypyrimidin-2-yl]-1H-indol-3-sulfonamid;
6,7-Dichlor-N-[5-(2-fluorethoxy)-4-methoxypyrimidin-2-yl]-1H-indol-3-sulfonamid;
6-Chlor-7-(difluormethyl)-N-[5-(2-fluorethoxy)-4-methoxypyrimidin-2-yl]-1H-indol-3-sulfonamid;
6-Chlor-7-(cyclopropoxy)-N-[5-(2-fluorethoxy)-4-methoxypyrimidin-2-yl]-1H-indol-3-sulfonamid;
6-Chlor-N-(5-ethoxy-4-methoxypyrimidin-2-yl)-1H-indol-3-sulfonamid;
6-Chlor-N-[4-methoxy-5-(2-methoxyethoxy)pyrimidin-2-yl]-1H-indol-3-sulfonamid;
6-Chlor-N-[5-(difluormethoxy)-4-methoxypyrimidin-2-yl]-1H-indol-3-sulfonamid;
6-Chlor-N-[5-[2-(difluormethoxy)ethoxy]-4-methoxypyrimidin-2-yl]-1H-indol-3-sulfonamid;
6-Chlor-N-[5-(2-fluorethoxy)-4-methoxypyrimidin-2-yl]-1H-pyrrolo[2,3-b]pyridin-3-sulfonamid;
6-Chlor-N-(5-chlor-4,6-dimethoxypyrimidin-2-yl)-1H-indol-3-sulfonamid;
N-(5-Brom-4,6-dimethoxypyrimidin-2-yl)-6-chlor-1H-indol-3-sulfonamid;
N-(5-Brom-4-ethoxy-6-methoxypyrimidin-2-yl)-6-chlor-1H-indol-3-sulfonamid;
N-[5-Brom-4-(2-fluorethoxy)-6-methoxypyrimidin-2-yl]-6-chlor-1H-indol-3-sulfonamid;
6-Chlor-N-(5-ethyl-4-methoxypyrimidin-2-yl)-1H-indol-3-sulfonamid;
6-Chlor-N-(5-ethyl-4,6-dimethoxypyrimidin-2-yl)-1H-indol-3-sulfonamid;
6-Chlor-N-(4,6-dimethoxy-5-methylpyrimidin-2-yl)-1H-indol-3-sulfonamid;
N-[5-Brom-4-(difluormethoxy)-6-methoxypyrimidin-2-yl]-6-chlor-1H-indol-3-sulfonamid;
6-Chlor-N-[4-cyclopropyl-5-(2,2-difluorethoxy)-6-methoxypyrimidin-2-yl]-1H-indol-3-sulfonamid;
6-Chlor-N-[5-(2,2-difluorethoxy)-4-methoxy-6-methylpyrimidin-2-yl]-1H-indol-3-sulfonamid;
6-Chlor-N-[5-(3-fluorpropyl)-4-methoxypyrimidin-2-yl]-1H-indol-3-sulfonamid;
N-(5-Brom-4-methoxypyrimidin-2-yl)-6-chlor-1H-indol-3-sulfonamid;
6-Chlor-N-(5-cyano-4-methoxypyrimidin-2-yl)-1H-indol-3-sulfonamid;
6-Chlor-N-[5-(2-fluorethoxy)-4-methoxypyrimidin-2-yl]benzothiophen-3-sulfonamid;
6-Brom-N-[5-(2-fluorethoxy)-4-methoxypyrimidin-2-yl]-1H-indol-3-sulfonamid;
6-Chlor-N-(4-methoxy-5-methylpyrimidin-2-yl)-1H-indol-3-sulfonamid;
6-Chlor-N-[5-(2-fluorethoxy)-4-methoxypyrimidin-2-yl]thieno[2,3-b]pyridin-3-sulfonamid;
6-Chlor-N-(5-cyclopropyl-4-methoxypyrimidin-2-yl)-1H-indol-3-sulfonamid;
6-Chlor-N-(4-methoxy-5-prop-1-inylpyrimidin-2-y1)-1H-indol-3-sulfonamid;
6-Brom-N-(5-brom-4,6-dimethoxypyrimidin-2-yl)-1H-pyrrolo[2,3-b]pyridin-3-sulfonamid;
N-(5-Brom-4,6-dimethoxypyrimidin-2-yl)-6-chlor-1H-pyrrolo[2,3-b]pyridin-3-sulfonamid;
6-Chlor-N-(5-cyano-4,6-dimethoxypyrimidin-2-yl)-1H-indol-3-sulfonamid;
N-(5-Brom-4,6-dimethylpyrimidin-2-yl)-6-chlor-1H-indol-3-sulfonamid;
6-Chlor-N-[5-(2,2-difluorethoxy)pyrimidin-2-yl]-1H-indol-3-sulfonamid;
6-Chlor-N-[5-(2,2-difluorethoxy)-4-methylsulfanylpyrimidin-2-yl]-1H-indol-3-sulfonamid;
6-Chlor-N-(4-methoxy-5-methylsulfanylpyrimidin-2-yl)-1H-indol-3-sulfonamid;
6-Chlor-N-[5-(difluormethylthio)-4-methoxypyrimidin-2-yl]-1H-indol-3-sulfonamid;
6-Chlor-N-[5-(cyclopropoxy)-4-methoxypyrimidin-2-yl]-1H-indol-3-sulfonamid;
6-Chlor-N-[5-(3,3-difluorpropoxy)-4-methoxypyrimidin-2-yl]-1H-indol-3-sulfonamid;
6-Chlor-N-[4-methoxy-5-(3,3,3-trifluorpropoxy)pyrimidin-2-yl]-1H-indol-3-sulfonamid;
6-Chlor-N-[4-methoxy-5-(1,1,2-trifluorethoxy)pyrimidin-2-yl]-1H-indol-3-sulfonamid;
6-Chlor-N-[5-(1,1-difluorethoxy)-4-methoxypyrimidin-2-yl]-1H-indol-3-sulfonamid;
6-Chlor-N-[4-methoxy-5-(1,1,2,2-tetrafluorethoxy)pyrimidin-2-yl]-1H-indol-3-sulfonamid;
6-Chlor-N-[5-(2-chlor-1,1,2-trifluorethoxy)-4-methoxypyrimidin-2-yl]-1H-indol-3-sulfonamid;
6-Chlor-N-(4-methoxy-5-propylpyrimidin-2-yl)-1H-indol-3-sulfonamid;
6-Chlor-N-[5-(cyclopropylmethyl)-4-methoxypyrimidin-2-yl]-1H-indol-3-sulfonamid;
6-Chlor-N-[5-(2,2-difluorethyl)-4-methoxypyrimidin-2-yl]-1H-indol-3-sulfonamid;
6-Chlor-N-[5-(3,3-difluorpropyl)-4-methoxypyrimidin-2-yl]-1H-indol-3-sulfonamid;
6-Chlor-N-[4-methoxy-5-(2-methoxyethyl)pyrimidin-2-yl]-1H-indol-3-sulfonamid;
6-Chlor-N-[4-methoxy-5-(oxetan-3-yl)pyrimidin-2-yl]-1H-indol-3-sulfonamid;
6-Chlor-N-[5-(fluormethoxy)-4,6-dimethoxypyrimidin-2-yl]-1H-indol-3-sulfonamid;
6-Chlor-N-[5-(difluormethoxy)-4,6-dimethoxypyrimidin-2-yl]-1H-indol-3-sulfonamid;
6-Chlor-N-[5-(2-fluorethoxy)-4,6-dimethoxypyrimidin-2-yl]-1H-indol-3-sulfonamid;
6-Chlor-N-[5-(2,2-difluorethoxy)-4,6-dimethoxypyrimidin-2-yl]-1H-indol-3-sulfonamid;
6-Chlor-N-[4,6-dimethoxy-5-(1,1,2-trifluorethoxy)pyrimidin-2-yl]-1H-indol-3-sulfonamid;
N-(5-Allyl-4,6-dimethoxypyrimidin-2-yl)-6-chlor-1H-indol-3-sulfonamid;
6-Chlor-N-(4,6-dimethoxy-5-prop-1-inylpyrimidin-2-yl)-1H-indol-3-sulfonamid;
6-Chlor-N-(4,6-dimethoxy-5-propylpyrimidin-2-yl)-1H-indol-3-sulfonamid;
6-Chlor-N-[5-(2-fluorethyl)-4,6-dimethoxypyrimidin-2-yl]-1H-indol-3-sulfonamid;
6-Chlor-N-[5-(2,2-difluorethyl)-4,6-dimethoxypyrimidin-2-yl]-1H-indol-3-sulfonamid;
6-Chlor-N-[5-(3-fluorpropyl)-4,6-dimethoxypyrimidin-2-yl]-1H-indol-3-sulfonamid;
6-Chlor-N-[5-(3,3-difluorpropyl)-4,6-dimethoxypyrimidin-2-yl]-1H-indol-3-sulfonamid;
6-Chlor-N-[5-(2,2-difluorpropyl)-4,6-dimethoxypyrimidin-2-yl]-1H-indol-3-sulfonamid;
6-Chlor-N-[4,6-dimethoxy-5-(3,3,3-trifluorpropyl)pyrimidin-2-yl]-1H-indol-3-sulfonamid;
6-Chlor-N-[4,6-dimethoxy-5-(methoxymethyl)pyrimidin-2-yl]-1H-indol-3-sulfonamid;
6-Chlor-N-[4,6-dimethoxy-5-(2-methoxyethyl)pyrimidin-2-yl]-1H-indol-3-sulfonamid;
6-Chlor-N-[5-(cyanomethyl)-4,6-dimethoxypyrimidin-2-yl]-1H-indol-3-sulfonamid;
6-Chlor-N-[5-(2-cyanoethyl)-4,6-dimethoxypyrimidin-2-yl]-1H-indol-3-sulfonamid;
6-Chlor-N-(5-cyclopropyl-4,6-dimethoxypyrimidin-2-yl)-1H-indol-3-sulfonamid;
N-[5-Brom-4-methoxy-6-(2-methoxyethoxy)pyrimidin-2-yl]-6-chlor-1H-indol-3-sulfonamid;
N-[5-Brom-4-(cyclopropoxy)-6-methoxypyrimidin-2-yl]-6-chlor-1H-indol-3-sulfonamid;
6-Chlor-N-[4-(difluormethoxy)-6-methoxy-5-methylpyrimidin-2-yl]-1H-indol-3-sulfonamid;
N-(5-Brom-4-fluor-6-methoxypyrimidin-2-yl)-6-chlor-1H-indol-3-sulfonamid;
6-Chlor-N-[5-(2,2-difluorethoxy)-4-fluor-6-methoxypyrimidin-2-yl]-1H-indol-3-sulfonamid;
6-Chlor-N-(4-chlor-5,6-dimethoxypyrimidin-2-yl)-1H-indol-3-sulfonamid;
6-Chlor-N-[4-chlor-5-(2,2-difluorethoxy)pyrimidin-2-yl]-1H-indol-3-sulfonamid;
6-Chlor-N-[5-(2,2-difluorethoxy)-4-ethyl-6-methoxypyrimidin-2-yl]-1H-indol-3-sulfonamid;
6-Chlor-N-[5-(2,2-difluorethyl)-4-methoxy-6-methylpyrimidin-2-yl]-1H-indol-3-sulfonamid;
6-Chlor-N-[4-cyclopropyl-5-(2,2-difluorethyl)-6-methoxypyrimidin-2-yl]-1H-indol-3-sulfonamid;
N-[5-Brom-4-(cyclopropylmethyl)-6-methoxypyrimidin-2-yl]-6-chlor-1H-indol-3-sulfonamid;
N-[5-Brom-4-methoxy-6-(methoxymethyl)pyrimidin-2-yl]-6-chlor-1H-indol-3-sulfonamid;
N-[5-Brom-4-(1,1-difluorethyl)-6-methoxypyrimidin-2-yl]-6-chlor-1H-indol-3-sulfonamid;
N-[5-Brom-4-(fluormethoxy)-6-methoxypyrimidin-2-yl]-6-chlor-1H-indol-3-sulfonamid;
N-[5-Brom-4-(difluormethyl)-6-methoxypyrimidin-2-yl]-6-chlor-1H-indol-3-sulfonamid;
N-[5-Brom-4-(cyanomethyl)-6-methoxypyrimidin-2-yl]-6-chlor-1H-indol-3-sulfonamid;
6-Chlor-N-[5-(difluormethoxy)-4-methoxy-6-methylsulfanylpyrimidin-2-yl]-1H-indol-3-sulfonamid;
N-[4,6-Dimethoxy-5-(1,1,2-trifluorethoxy)pyrimidin-2-yl]-6-fluor-1H-indol-3-sulfonamid;
6-Brom-N-[5-(2,2-difluorethyl)-4-methoxypyrimidin-2-yl]-1H-indol-3-sulfonamid;
6-Brom-N-[5-(2-cyanoethyl)-4-methoxypyrimidin-2-yl]-1H-indol-3-sulfonamid;
6-Brom-N-[5-(difluormethoxy)-4,6-dimethoxypyrimidin-2-yl]-1H-indol-3-sulfonamid;
6-Brom-N-[5-(2-fluorethoxy)-4,6-dimethoxypyrimidin-2-yl]-1H-indol-3-sulfonamid;
6-Brom-N-[5-(2,2-difluorethoxy)-4,6-dimethoxypyrimidin-2-yl]-1H-indol-3-sulfonamid;
6-Brom-N-[5-(2-cyanoethyl)-4,6-dimethoxypyrimidin-2-yl]-1H-indol-3-sulfonamid;
N-[5-(Difluormethoxy)-4,6-dimethoxypyrimidin-2-yl]-6-(difluormethyl)-1H-pyrrolo[2,3-b]pyridin-3-sulfonamid;
6-(Difluormethyl)-N-[5-(2-fluorethoxy)-4,6-dimethoxypyrimidin-2-yl]-1H-pyrrolo[2,3-b]pyridin-3-sulfonamid;
N-[5-(2,2-Difluorethoxy)-4,6-dimethoxypyrimidin-2-yl]-6-(difluormethyl)-1H-pyrrolo[2,3-b]pyridin-3-sulfonamid;
6-(Difluormethyl)-N-[4,6-dimethoxy-5-(1,1,2-trifluorethoxy)pyrimidin-2-yl]-1H-pyrrolo[2,3-b]pyridin-3-sulfonamid;
6-(Difluormethyl)-N-[5-(3,3-difluorpropyl)-4,6-dimethoxypyrimidin-2-yl]-1H-pyrrolo[2,3-b]pyridin-3-sulfonamid;
N-[5-(Cyanomethoxy)-4,6-dimethoxypyrimidin-2-yl]-6-(difluormethyl)-1H-pyrrolo[2,3-b]pyridin-3-sulfonamid;
6-(Difluormethyl)-N-[4-methoxy-5-(1,1,2-trifluorethoxy)pyrimidin-2-yl]-1H-indol-3-sulfonamid;
N-[5-(Difluormethoxy)-4,6-dimethoxypyrimidin-2-yl]-6-(difluormethyl)-1H-indol-3-sulfonamid;
N-[5-(2,2-Difluorethoxy)-4,6-dimethoxypyrimidin-2-yl]-6-(difluormethyl)-1H-indol-3-sulfonamid;
N-[5-(2-Cyanoethyl)-4,6-dimethoxypyrimidin-2-yl]-6-(difluormethyl)-1H-indol-3-sulfonamid;
N-[5-(2,2-Difluorethyl)-4-methoxypyrimidin-2-yl]-6-(difluormethoxy)-1H-indol-3-sulfonamid;
6-Chlor-N-[5-(2-cyanoethyl)-4-methoxypyrimidin-2-yl]-7-(difluormethylthio)-1H-indol-3-sulfonamid;
6-Chlor-7-(difluormethylthio)-N-[5-(2-fluorethoxy)-4,6-dimethoxypyrimidin-2-yl]-1H-indol-3-sulfonamid;
6-Chlor-N-[5-(2-cyanoethyl)-4,6-dimethoxypyrimidin-2-yl]-7-(difluormethylthio)-1H-indol-3-sulfonamid;
7-Chlor-N-[5-(2,2-difluorethyl)-4-methoxypyrimidin-2-yl]-1H-indol-3-sulfonamid;
6-Brom-N-[5-(2-fluorethoxy)-4,6-dimethoxypyrimidin-2-yl]-1H-pyrrolo[2,3-b]pyridin-3-sulfonamid;
6-Brom-N-[4,6-dimethoxy-5-(1,1,2-trifluorethoxy)pyrimidin-2-yl]-1H-pyrrolo[2,3-b]pyridin-3-sulfonamid;
6-Brom-N-[5-(2,2-difluorethyl)-4,6-dimethoxypyrimidin-2-yl]-1H-pyrrolo[2,3-b]pyridin-3-sulfonamid;
6-Brom-N-[5-(3,3-difluorpropyl)-4,6-dimethoxypyrimidin-2-yl]-1H-pyrrolo[2,3-b]pyridin-3-sulfonamid;
6-Brom-N-[5-(2-cyanoethyl)-4,6-dimethoxypyrimidin-2-yl]-1H-pyrrolo[2,3-b]pyridin-3-sulfonamid;
6-Brom-N-[5-(cyanomethoxy)-4,6-dimethoxypyrimidin-2-yl]-1H-pyrrolo[2,3-b]pyridin-3-sulfonamid;
6-Chlor-N-[5-(2,2-difluorethyl)-4,6-dimethoxypyrimidin-2-yl]-7-(dimethylamino)-1H-indol-3-sulfonamid;
N-[5-(Difluormethoxy)-4,6-dimethoxypyrimidin-2-yl]-1H-pyrrolo[3,2-h]chinolin-3-sulfonamid;
N-[5-(2-Fluorethoxy)-4,6-dimethoxypyrimidin-2-yl]-1H-pyrrolo[3,2-h]chinolin-3-sulfonamid;
6-Chlor-N-(4,6-dimethoxy-5-methylpyrimidin-2-yl)-1H-pyrrolo[2,3-b]pyridin-3-sulfonamid;
6-Chlor-N-[5-(difluormethoxy)-4-methoxypyrimidin-2-yl]-1H-pyrrolo[2,3-b]pyridin-3-sulfonamid;
6-Chlor-N-[5-(2,2-difluorethyl)-4,6-dimethoxypyrimidin-2-yl]-1H-pyrrolo[2,3-b]pyridin-3-sulfonamid;
6-Chlor-N-[5-(3,3-difluorpropyl)-4,6-dimethoxypyrimidin-2-yl]-1H-pyrrolo[2,3-b]pyridin-3-sulfonamid;
6-Chlor-N-[5-(2-cyanoethyl)-4,6-dimethoxypyrimidin-2-yl]-1H-pyrrolo[2,3-b]pyridin-3-sulfonamid;
6-Chlor-N-[5-(2,2-difluorethoxy)-4-methoxypyrimidin-2-yl]-7-fluor-1H-indol-3-sulfonamid;
6-Chlor-N-[5-(2,2-difluorethyl)-4-methoxypyrimidin-2-yl]-7-fluor-1H-indol-3-sulfonamid;
6-Chlor-N-[5-(difluormethoxy)-4,6-dimethoxypyrimidin-2-yl]-7-fluor-1H-indol-3-sulfonamid;
6-Chlor-N-[5-(difluormethoxy)-4-methoxypyrimidin-2-yl]-7-fluor-1H-indol-3-sulfonamid;
6-Chlor-7-(cyclopropoxy)-N-[5-(2,2-difluorethyl)-4-methoxypyrimidin-2-yl]-1H-indol-3-sulfonamid;
7-Brom-N-[5-(2,2-difluorethyl)-4-methoxypyrimidin-2-yl]-6-fluor-1H-indol-3-sulfonamid;
7-Brom-N-[5-(2,2-difluorethyl)-4-methoxypyrimidin-2-yl]-1H-indol-3-sulfonamid;
7-Chlor-N-[5-(2,2-difluorethyl)-4-methoxypyrimidin-2-yl]-6-fluor-1H-indol-3-sulfonamid;
N-[5-(2-Fluorethoxy)-4,6-dimethoxypyrimidin-2-yl]-6-methyl-1H-pyrrolo[2,3-b]pyridin-3-sulfonamid;
N-[5-(2,2-Difluorethyl)-4,6-dimethoxypyrimidin-2-yl]-6-methyl-1H-pyrrolo[2,3-b]pyridin-3-sulfonamid;
6-Chlor-N-[5-(2,2-difluorethyl)-4,6-dimethoxypyrimidin-2-yl]-7-morpholino-1H-indol-3-sulfonamid;
7-Brom-N-[5-(2,2-difluorethyl)-4,6-dimethoxypyrimidin-2-yl]-1H-indol-3-sulfonamid;
6-Chlor-N-[5-(2,2-difluorethoxy)-4-methoxypyrimidin-2-yl]-1H-indol-3-sulfonamid;
6-Chlor-N-[5-(1,1-difluorethyl)-4-methoxypyrimidin-2-yl]-1H-indol-3-sulfonamid;
6-Chlor-N-(4-ethyl-6-methoxy-5-methylpyrimidin-2-yl)-1H-indol-3-sulfonamid;
N-(5-Brom-4-methoxy-6-tetrahydropyran-4-ylpyrimidin-2-yl)-6-chlor-1H-indol-3-sulfonamid;
6-Chlor-N-[5-(2,2-difluorethyl)-4-methoxypyrimidin-2-yl]thieno[2,3-b]pyridin-3-sulfonamid;
N-[5-(2,2-Difluorethyl)-4-methoxypyrimidin-2-yl]-6-fluor-1H-indol-3-sulfonamid;
7-Brom-6-chlor-N-[5-(2-fluorethoxy)-4-methoxypyrimidin-2-yl]-1H-indol-3-sulfonamid;
7-Brom-6-chlor-N-[5-(2,2-difluorethyl)-4-methoxypyrimidin-2-yl]-1H-indol-3-sulfonamid;
6-Brom-N-[5-(2,2-difluorethoxy)-4-methoxypyrimidin-2-yl]-1H-indol-3-sulfonamid;
6-Brom-N-(4,6-dimethoxy-5-methylpyrimidin-2-yl)thieno[2,3-b]pyridin-3-sulfonamid;
6-Brom-N-[5-(2,2-difluorethoxy)-4-methoxypyrimidin-2-yl]thieno[2,3-b]pyridin-3-sulfonamid;
6-Brom-N-[5-(2-fluorethoxy)-4-methoxypyrimidin-2-yl]thieno[2,3-b]pyridin-3-sulfonamid;
6-Brom-N-[4-methoxy-5-(1,1,2-trifluorethoxy)pyrimidin-2-yl]thieno[2,3-b]pyridin-3-sulfonamid;
6-Brom-N-[5-(2,2-difluorethyl)-4-methoxypyrimidin-2-yl]thieno[2,3-b]pyridin-3-sulfonamid;
6-Brom-N-[5-(2-cyanoethyl)-4-methoxypyrimidin-2-yl]thieno[2,3-b]pyridin-3-sulfonamid;
6-Brom-N-[5-(2,2-difluorethyl)-4,6-dimethoxypyrimidin-2-yl]thieno[2,3-b]pyridin-3-sulfonamid;
6-Brom-N-[5-(2-cyanoethyl)-4,6-dimethoxypyrimidin-2-yl]thieno[2,3-b]pyridin-3-sulfonamid;
N-[5-(2,2-Difluorethyl)-4-methoxypyrimidin-2-yl]-6-ethyl-1H-indol-3-sulfonamid;
N-[5-(2,2-Difluorethyl)-4-methoxypyrimidin-2-yl]-6-propyl-1H-indol-3-sulfonamid;
6-Chlor-N-[5-(2,2-difluorethyl)-4-methoxypyrimidin-2-yl]benzothiophen-3-sulfonamid;
6-Chlor-N-[5-(3,3-difluorpropyl)-4-methoxypyrimidin-2-yl]benzothiophen-3-sulfonamid;
6-Chlor-N-[5-(2-cyanoethyl)-4-methoxypyrimidin-2-yl]benzothiophen-3-sulfonamid;
6-Brom-N-[5-(2,2-difluorethoxy)-4-methoxypyrimidin-2-yl]benzothiophen-3-sulfonamid;
6-Brom-N-[5-(2-fluorethoxy)-4-methoxypyrimidin-2-yl]benzothiophen-3-sulfonamid;
N-[5-(2-Fluorethoxy)-4-methoxypyrimidin-2-yl]-6-methylbenzothiophen-3-sulfonamid;
6-Brom-N-(4,6-dimethoxy-5-methylpyrimidin-2-yl)-1H-pyrrolo[2,3-b]pyridin-3-sulfonamid;
6-Brom-N-[5-(2,2-difluorethoxy)-4-methoxypyrimidin-2-yl]-1H-pyrrolo[2,3-b]pyridin-3-sulfonamid;
6-Chlor-N-[5-(2,2-difluorethyl)-4-methoxypyrimidin-2-yl]-7-fluorbenzothiophen-3-sulfonamid;
6-Chlor-N-[5-(2-cyanoethyl)-4-methoxypyrimidin-2-yl]-7-fluorbenzothiophen-3-sulfonamid;
N-[5-(2,2-Difluorethyl)-4-methoxypyrimidin-2-yl]-6,7-difluor-1H-indol-3-sulfonamid;
6-(Difluormethyl)-N-[5-(2-fluorethoxy)-4-methoxypyrimidin-2-yl]thieno[2,3-b]pyridin-3-sulfonamid;
N-[5-(2,2-Difluorethyl)-4-methoxypyrimidin-2-yl]-6-(difluormethyl)thieno[2,3-b]pyridin-3-sulfonamid;
N-[5-(2,2-Difluorethyl)-4,6-dimethoxypyrimidin-2-yl]-6-(difluormethyl)thieno[2,3-b]pyridin-3-sulfonamid;
N-[5-(2,2-Difluorethoxy)-4,6-dimethoxypyrimidin-2-yl]-1H-pyrrolo[3,2-h]chinolin-3-sulfonamid;
N-[5-(2,2-Difluorethyl)-4,6-dimethoxypyrimidin-2-yl]-1H-pyrrolo[3,2-h]chinolin-3-sulfonamid;
6-Chlor-N-[5-(2,2-difluorethoxy)-4-methoxypyrimidin-2-yl]-1H-pyrrolo[2,3-b]pyridin-3-sulfonamid;
6-Chlor-N-[4,6-dimethoxy-5-(1,1,2-trifluorethoxy)pyrimidin-2-yl]-1H-pyrrolo[2,3-b]pyridin-3-sulfonamid;
6-Brom-N-[5-(difluormethoxy)-4,6-dimethoxypyrimidin-2-yl]-1H-pyrrolo[2,3-b]pyridin-3-sulfonamid;
6-Chlor-N-(4-chlor-5-methoxypyrimidin-2-yl)-1H-indol-3-sulfonamid;
6-Chlor-N-[5-(3-fluorpropoxy)-4-methoxypyrimidin-2-yl]-1H-indol-3-sulfonamid;
N-(5-But-2-inoxy-4-methoxypyrimidin-2-yl)-6-chlor-1H-indol-3-sulfonamid;
6-Chlor-N-[5-(2,3-difluorpropyl)-4-methoxypyrimidin-2-yl]-1H-indol-3-sulfonamid;
6-Chlor-N-[4-methoxy-5-(2,2,2-trifluorethyl)pyrimidin-2-yl]-1H-indol-3-sulfonamid;
6-Chlor-N-[4-methoxy-5-(3,3,3-trifluorpropyl)pyrimidin-2-yl]-1H-indol-3-sulfonamid;
6-Chlor-N-[5-(2-cyanoethyl)-4-methoxypyrimidin-2-yl]-1H-indol-3-sulfonamid;
6-Chlor-N-[4-methoxy-5-[2-(oxetan-3-yl)ethinyl]pyrimidin-2-yl]-1H-indol-3-sulfonamid;
6-Chlor-N-[4-methoxy-5-[2-(oxetan-3-yl)ethyl]pyrimidin-2-yl]-1H-indol-3-sulfonamid;
N-[5-(2,2-Difluorethyl)-4-methoxypyrimidin-2-yl]-6-methyl-1H-indol-3-sulfonamid;
7-Brom-6-chlor-N-[5-(3,3-difluorpropyl)-4-methoxypyrimidin-2-yl]-1H-indol-3-sulfonamid;
6-Brom-N-[5-(2,2-difluorethyl)-4-methoxypyrimidin-2-yl]-7-methyl-1H-indol-3-sulfonamid;
6-Chlor-N-[5-(2,2-difluorethyl)-4-methoxypyrimidin-2-yl]-7-methoxy-1H-indol-3-sulfonamid;
6-Chlor-N-[5-(3,3-difluorpropyl)-4-methoxypyrimidin-2-yl]-7-methoxy-1H-indol-3-sulfonamid;
6-Chlor-N-[5-(2,2-difluorethyl)-4-methoxypyrimidin-2-yl]-7-(difluormethoxy)-1H-indol-3-sulfonamid;
6-Brom-N-[5-(difluormethoxy)-4-methoxypyrimidin-2-yl]-1H-indol-3-sulfonamid;
N-[5-(2,2-Difluorethyl)-4-methoxypyrimidin-2-yl]-6-(trifluormethyl)-1H-indol-3-sulfonamid;
6-Brom-N-[5-(2,2-difluorethyl)-4-methoxypyrimidin-2-yl]-7-fluor-1H-indol-3-sulfonamid;
6-Chlor-N-[5-(2,2-difluorethyl)-4-methoxypyrimidin-2-yl]-7-methyl-1H-indol-3-sulfonamid;
N-[5-(2,2-Difluorethyl)-4-methoxypyrimidin-2-yl]-6-(methylthio)-1H-indol-3-sulfonamid;
N-[5-(2,2-Difluorethyl)-4,6-dimethoxypyrimidin-2-yl]-6-(difluormethyl)-1H-pyrrolo[2,3-b]pyridin-3-sulfonamid;
N-[5-(2,2-Difluorethyl)-4-methoxypyrimidin-2-yl]-6-(difluormethyl)-1H-indol-3-sulfonamid;
6-Brom-N-[5-(2,2-difluorethyl)-4-methoxypyrimidin-2-yl]-7-methoxy-1H-indol-3-sulfonamid;
6-(Cyanomethyl)-N-[5-(2,2-difluorethyl)-4-methoxypyrimidin-2-yl]-1H-indol-3-sulfonamid;
6-Chlor-N-[5-(2,2-difluorethyl)-4-methoxypyrimidin-2-yl]-7-(fluormethoxy)-1H-indol-3-sulfonamid;
6-Chlor-N-[5-(2,2-difluorethyl)-4-methoxypyrimidin-2-yl]-7-(difluormethylsulfanyl)-1H-indol-3-sulfonamid;
6-Brom-N-[5-(2,2-difluorethyl)-4-methoxypyrimidin-2-yl]-1H-pyrrolo[2,3-b]pyridin-3-sulfonamid;
6-Brom-N-[5-(3,3-difluorpropyl)-4-methoxypyrimidin-2-yl]-1H-pyrrolo[2,3-b]pyridin-3-sulfonamid;
6-Brom-N-[5-(2,2-difluorethoxy)-4,6-dimethoxypyrimidin-2-yl]-1H-pyrrolo[2,3-b]pyridin-3-sulfonamid;
6-Chlor-N-[5-(2,2-difluorethyl)-4-methoxypyrimidin-2-yl]-1H-pyrrolo[2,3-b]pyridin-3-sulfonamid;
6-Chlor-N-[5-(3,3-difluorpropyl)-4-methoxypyrimidin-2-yl]-1H-pyrrolo[2,3-b]pyridin-3-sulfonamid;
6-Chlor-N-[5-(difluormethoxy)-4,6-dimethoxypyrimidin-2-yl]-1H-pyrrolo[2,3-b]pyridin-3-sulfonamid;
6-Chlor-N-[5-(2,2-difluorethoxy)-4,6-dimethoxypyrimidin-2-yl]-1H-pyrrolo[2,3-b]pyridin-3-sulfonamid;
6-Chlor-N-[5-(3,3-difluorpropyl)-4-methoxypyrimidin-2-yl]-7-fluor-1H-indol-3-sulfonamid;
6-Chlor-N-[5-(2,2-difluorethoxy)-4,6-dimethoxypyrimidin-2-yl]-7-fluor-1H-indol-3-sulfonamid;
6-Chlor-7-(difluormethyl)-N-[5-(3,3-difluorpropyl)-4-methoxypyrimidin-2-yl]-1H-indol-3-sulfonamid;
6,7-Dichlor-N-[5-(2,2-difluorethyl)-4-methoxypyrimidin-2-yl]-1H-indol-3-sulfonamid;
6,7-Dichlor-N-[5-(3,3-difluorpropyl)-4-methoxypyrimidin-2-yl]-1H-indol-3-sulfonamid;
6-Chlor-7-(cyclopropoxy)-N-[5-(3,3-difluorpropyl)-4-methoxypyrimidin-2-yl]-1H-indol-3-sulfonamid;
6-Brom-7-fluor-N-[5-(2-fluorethoxy)-4-methoxypyrimidin-2-yl]-1H-indol-3-sulfonamid;
6-Chlor-N-[5-(2,2-difluorethyl)-4-methoxypyrimidin-2-yl]-7-(difluormethyl)-1H-indol-3-sulfonamid;
6-Chlor-7-fluor-N-[4-methoxy-5-(1,1,2-trifluorethoxy)pyrimidin-2-yl]-1H-indol-3-sulfonamid;
6-Chlor-N-[5-(2-cyanoethyl)-4-methoxypyrimidin-2-yl]-7-fluor-1H-indol-3-sulfonamid;
6-Chlor-N-[5-(2,2-difluorethyl)-4,6-dimethoxypyrimidin-2-yl]-7-fluor-1H-indol-3-sulfonamid;
6-Chlor-N-[5-(2-cyanoethyl)-4,6-dimethoxypyrimidin-2-yl]-7-fluor-1H-indol-3-sulfonamid;
6-Chlor-N-[5-(cyanomethoxy)-4,6-dimethoxypyrimidin-2-yl]-7-fluor-1H-indol-3-sulfonamid;
6-Chlor-N-[4-methoxy-5-(1,1,2-trifluorethoxy)pyrimidin-2-yl]-1H-pyrrolo[2,3-b]pyridin-3-sulfonamid;
6-Chlor-N-[5-(2-fluorethoxy)-4,6-dimethoxypyrimidin-2-yl]-1H-pyrrolo[2,3-b]pyridin-3-sulfonamid;
N-[5-(2,2-Difluorethyl)-4-methoxypyrimidin-2-yl]-1H-pyrrolo[3,2-h]chinolin-3-sulfonamid;
6-Chlor-7-cyano-N-[5-(2,2-difluorethyl)-4-methoxypyrimidin-2-yl]-1H-indol-3-sulfonamid;
6-Chlor-7-cyano-N-[5-(difluormethoxy)-4,6-dimethoxypyrimidin-2-yl]-1H-indol-3-sulfonamid;
6-Chlor-7-cyano-N-[5-(2-fluorethoxy)-4,6-dimethoxypyrimidin-2-yl]-1H-indol-3-sulfonamid;
6-Chlor-7-cyano-N-[5-(2,2-difluorethoxy)-4,6-dimethoxypyrimidin-2-yl]-1H-indol-3-sulfonamid;
6-Chlor-7-cyano-N-[5-(2,2-difluorethyl)-4,6-dimethoxypyrimidin-2-yl]-1H-indol-3-sulfonamid;
N-[5-(2,2-Difluorethyl)-4-methoxypyrimidin-2-yl]-6,8-dihydro-1H-fur[3,4-g]indol-3-sulfonamid;
N-[5-(2,2-Difluorethyl)-4,6-dimethoxypyrimidin-2-yl]-6,8-dihydro-1H-fur[3,4-g]indol-3-sulfonamid;
6-Brom-N-[5-(2-fluorethoxy)-4-methoxypyrimidin-2-yl]-1H-pyrrolo[2,3-b]pyridin-3-sulfonamid;
6-Brom-N-[4-methoxy-5-(1,1,2-trifluorethoxy)pyrimidin-2-yl]-1H-pyrrolo[2,3-b]pyridin-3-sulfonamid;
6-Chlor-7-(cyanomethyl)-N-[5-(2,2-difluorethyl)-4-methoxypyrimidin-2-yl]-1H-indol-3-sulfonamid;
6-Chlor-7-cyclopropyl-N-[5-(2,2-difluorethyl)-4-methoxypyrimidin-2-yl]-1H-indol-3-sulfonamid;
6-Chlor-N-[5-(2,2-difluorethyl)-4-methoxypyrimidin-2-yl]-7-(methylthio)-1H-indol-3-sulfonamid;
6-(Difluormethyl)-N-[5-(2-fluorethoxy)-4-methoxypyrimidin-2-yl]-1H-indol-3-sulfonamid;
N-[5-(2,2-Difluorethyl)-4,6-dimethoxypyrimidin-2-yl]-6-(difluormethyl)-1H-indol-3-sulfonamid;
N-[5-(Cyanomethoxy)-4,6-dimethoxypyrimidin-2-yl]-6-(difluormethyl)-1H-indol-3-sulfonamid;
6-(Difluormethyl)-N-[4-methoxy-5-(1,1,2-trifluorethoxy)pyrimidin-2-yl]-1H-pyrrolo[2,3-b]pyridin-3-sulfonamid;
N-[5-(2-Cyanoethyl)-4,6-dimethoxypyrimidin-2-yl]-6-(difluormethyl)-1H-pyrrolo[2,3-b]pyridin-3-sulfonamid;
6-Brom-N-[5-(2-cyanoethyl)-4-methoxypyrimidin-2-yl]-7-fluor-1H-indol-3-sulfonamid;
6-Brom-N-[5-(difluormethoxy)-4,6-dimethoxypyrimidin-2-yl]-7-fluor-1H-indol-3-sulfonamid;
6-Brom-N-[5-(2,2-difluorethoxy)-4,6-dimethoxypyrimidin-2-yl]-7-fluor-1H-indol-3-sulfonamid;
6-Brom-N-[5-(2-cyanoethyl)-4,6-dimethoxypyrimidin-2-yl]-7-fluor-1H-indol-3-sulfonamid;
6-Brom-N-[5-(cyanomethoxy)-4,6-dimethoxypyrimidin-2-yl]-7-fluor-1H-indol-3-sulfonamid;
6-Brom-N-[5-(2,2-difluorethyl)-4,6-dimethoxypyrimidin-2-yl]-1H-indol-3-sulfonamid;
6-Brom-N-[5-(cyanomethoxy)-4,6-dimethoxypyrimidin-2-yl]-1H-indol-3-sulfonamid;
6-(Difluormethyl)-7-fluor-N-[5-(2-fluorethoxy)-4-methoxypyrimidin-2-yl]-1H-indol-3-sulfonamid;
N-[5-(2,2-Difluorethyl)-4-methoxypyrimidin-2-yl]-6-(difluormethyl)-7-fluor-1H-indol-3-sulfonamid;
N-[5-(Difluormethoxy)-4,6-dimethoxypyrimidin-2-yl]-6-(difluormethyl)-7-fluor-1H-indol-3-sulfonamid;
N-[5-(2,2-Difluorethoxy)-4,6-dimethoxypyrimidin-2-yl]-6-(difluormethyl)-7-fluor-1H-indol-3-sulfonamid;
N-[5-(2,2-Difluorethyl)-4,6-dimethoxypyrimidin-2-yl]-6-(difluormethyl)-7-fluor-1H-indol-3-sulfonamid;
N-[5-(2-Cyanoethyl)-4,6-dimethoxypyrimidin-2-yl]-6-(difluormethyl)-7-fluor-1H-indol-3-sulfonamid;
N-[5-(Cyanomethoxy)-4,6-dimethoxypyrimidin-2-yl]-6-(difluormethyl)-7-fluor-1H-indol-3-sulfonamid;
6-Cyclopropyl-N-[5-(2,2-difluorethyl)-4-methoxypyrimidin-2-yl]-1H-indol-3-sulfonamid;
7-Brom-6-chlor-N-[5-(2-cyanoethyl)-4-methoxypyrimidin-2-yl]-1H-indol-3-sulfonamid;
N-[5-(Difluormethoxy)-4,6-dimethoxypyrimidin-2-yl]-6-fluor-1H-indol-3-sulfonamid;
N-[5-(2,2-Difluorethoxy)-4,6-dimethoxypyrimidin-2-yl]-6-fluor-1H-indol-3-sulfonamid;
N-[5-(2,2-Difluorethyl)-4,6-dimethoxypyrimidin-2-yl]-6-fluor-1H-indol-3-sulfonamid;
N-[5-(2-Cyanoethyl)-4,6-dimethoxypyrimidin-2-yl]-6-fluor-1H-indol-3-sulfonamid;
6-Chlor-N-[5-(2,2-difluorpropyl)-4-methoxypyrimidin-2-yl]-1H-indol-3-sulfonamid;
6-Chlor-N-[4-methoxy-5-(2,2,3,3-tetrafluorpropyl)pyrimidin-2-yl]-1H-indol-3-sulfonamid;
6-Chlor-N-[5-(difluormethoxymethyl)-4-methoxypyrimidin-2-yl]-1H-indol-3-sulfonamid;
6-Chlor-N-[4,6-dimethoxy-5-(oxetan-3-ylmethoxy)pyrimidin-2-yl]-1H-indol-3-sulfonamid;
6-Chlor-N-[5-(2,3-difluorpropyl)-4,6-dimethoxypyrimidin-2-yl]-1H-indol-3-sulfonamid;
6-Chlor-N-[5-(cyanomethoxy)-4,6-dimethoxypyrimidin-2-yl]-1H-indol-3-sulfonamid;
6-Chlor-N-[4-methoxy-5-(2,3,3-trifluorpropyl)pyrimidin-2-yl]-1H-indol-3-sulfonamid;
6-Chlor-N-[5-(cyanomethoxy)-4-methoxypyrimidin-2-yl]-1H-indol-3-sulfonamid,
6-Brom-N-[5-(difluormethoxy)-4-methoxypyrimidin-2-yl]benzothiophen-3-sulfonamid;
6-Brom-N-[5-(difluormethoxy)-4,6-dimethoxypyrimidin-2-yl]thieno[2,3-b]pyridin-3-sulfonamid;
6-Brom-N-[5-(3,3-difluorpropyl)-4-methoxypyrimidin-2-yl]thieno[2,3-b]pyridin-3-sulfonamid;
6-Chlor-N-[5-(difluormethoxy)-4,6-dimethoxypyrimidin-2-yl]thieno[2,3-b]pyridin-3-sulfonamid;
N-[5-(2,2-Difluorethyl)-4-methoxypyrimidin-2-yl]-6-(oxetan-3-yl)-1H-indol-3-sulfonamid;
N-(4,6-Dimethoxy-5-methylpyrimidin-2-yl)-6-methoxy-1H-indol-3-sulfonamid;
N-[5-(2,2-Difluorethyl)-4-methoxypyrimidin-2-yl]-6-(1-hydroxy-1-methylethyl)-1H-indol-3-sulfonamid;
6-Chlor-N-[5-(1,1-difluor-2-hydroxyethoxy)-4,6-dimethoxypyrimidin-2-yl]-1H-indol-3-sulfonamid;
6-Chlor-N-[5-(2,2-difluorethyl)-4-methoxypyrimidin-2-yl]-7-methylsulfinyl-1H-indol-3-sulfonamid;
6-Chlor-N-[5-(2,2-difluorethyl)-4-methoxypyrimidin-2-yl]-7-methyl-1H-indol-3-sulfonamid;
6-Chlor-N-[5-(2-fluorethoxy)-4,6-dimethoxypyrimidin-2-yl]-7-methylsulfinyl-1H-indol-3-sulfonamid;
6-Chlor-N-[5-(2,2-difluorethyl)-4,6-dimethoxypyrimidin-2-yl]-7-methylsulfinyl-1H-indol-3-sulfonamid;
6-Chlor-7-cyano-N-[5-(2-fluorethoxy)-4-methoxypyrimidin-2-yl]-1H-indol-3-sulfonamid;
6-Chlor-N-[4,6-dimethoxy-5-(2-methylsulfonylethyl)pyrimidin-2-yl]-1H-indol-3-sulfonamid;
N-[5-(2,2-Difluorethyl)-4-methoxypyrimidin-2-yl]-1H-pyrrolo[2,3-e][1,2]benzothiazol-3-sulfonamid;
7-Brom-6-chlor-N-[5-(2,2-difluorethyl)-4,6-dimethoxypyrimidin-2-yl]-1H-indol-3-sulfonamid;
N-[5-(Cyanomethoxy)-4,6-dimethoxypyrimidin-2-yl]-6-methyl-1H-pyrrolo[2,3-b]pyridin-3-sulfonamid;
N-[5-(2,2-Difluorethoxy)-4,6-dimethoxypyrimidin-2-yl]-6-methyl-1H-pyrrolo[2,3-b]pyridin-3-sulfonamid;
N-[5-(Difluormethoxy)-4,6-dimethoxypyrimidin-2-yl]-6-methyl-1H-pyrrolo[2,3-b]pyridin-3-sulfonamid;
6-(Difluormethyl)-N-[5-(2,3-difluorpropyl)-4,6-dimethoxypyrimidin-2-yl]-1H-pyrrolo[2,3-b]pyridin-3-sulfonamid;
6-Brom-N-[5-(2,3-difluorpropyl)-4,6-dimethoxypyrimidin-2-yl]-1H-pyrrolo[2,3-b]pyridin-3-sulfonamid;
6-Chlor-N-[5-(2,3-difluorpropyl)-4,6-dimethoxypyrimidin-2-yl]-1H-pyrrolo[2,3-b]pyridin-3-sulfonamid;
N-[5-(2,2-Difluorethyl)-4-methoxypyrimidin-2-yl]-8H-pyrrolo[2,3-e][1,3]benzothiazol-6-sulfonamid;
3-[[5-(2,2-Difluorethyl)-4-methoxypyrimidin-2-yl]sulfamoyl]-6-methyl-1H-indol-7-carbonsäuremethylester;
N-[5-(2,2-Difluorethyl)-4-methoxypyrimidin-2-yl]-6-methyl-7-methylol-1H-indol-3-sulfonamid;
N-[5-(2-Fluorethoxy)-4-methoxypyrimidin-2-yl]-9-keto-8-methyl-6,7-dihydro-1H-pyrrol[3,2-h]isochinolin-3-sulfonamid;
6-Chlor-7-(cyanomethoxy)-N-[5-(2,2-difluorethyl)-4,6-dimethoxypyrimidin-2-yl]-1H-indol-3-sulfonamid;
und pharmazeutisch verträgliche Salze davon.

12. Verbindung nach einem der Ansprüche 1 bis 11, ausgewählt aus
6-Chlor-7-fluor-N-[5-(2-fluorethoxy)-4-methoxypyrimidin-2-yl]-1H-indol-3-sulfonamid;
6,7-Dichlor-N-[5-(2-fluorethoxy)-4-methoxypyrimidin-2-yl]-1H-indol-3-sulfonamid;
6-Chlor-7-(difluormethyl)-N-[5-(2-fluorethoxy)-4-methoxypyrimidin-2-yl]-1H-indol-3-sulfonamid;
N-(5-Brom-4,6-dimethoxypyrimidin-2-yl)-6-chlor-1H-indol-3-sulfonamid;
6-Chlor-N-(5-ethyl-4,6-dimethoxypyrimidin-2-yl)-1H-indol-3-sulfonamid;
6-Chlor-N-(4,6-dimethoxy-5-methylpyrimidin-2-yl)-1H-indol-3-sulfonamid;
6-Chlor-N-[4-cyclopropyl-5-(2,2-difluorethoxy)-6-methoxypyrimidin-2-yl]-1H-indol-3-sulfonamid;
6-Chlor-N-[5-(3-fluorpropyl)-4-methoxypyrimidin-2-yl]-1H-indol-3-sulfonamid;
6-Brom-N-[5-(2-fluorethoxy)-4-methoxypyrimidin-2-yl]-1H-indol-3-sulfonamid;
6-Brom-N-(5-brom-4,6-dimethoxypyrimidin-2-yl)-1H-pyrrolo[2,3-b]pyridin-3-sulfonamid;
6-Chlor-N-[4-methoxy-5-(1,1,2-trifluorethoxy)pyrimidin-2-yl]-1H-indol-3-sulfonamid;
6-Chlor-N-[5-(2,2-difluorethyl)-4-methoxypyrimidin-2-yl]-1H-indol-3-sulfonamid;
6-Chlor-N-[5-(3,3-difluorpropyl)-4-methoxypyrimidin-2-yl]-1H-indol-3-sulfonamid;
6-Chlor-N-[5-(difluormethoxy)-4,6-dimethoxypyrimidin-2-yl]-1H-indol-3-sulfonamid;
6-Chlor-N-[5-(2-fluorethoxy)-4,6-dimethoxypyrimidin-2-yl]-1H-indol-3-sulfonamid;
6-Chlor-N-[5-(2,2-difluorethoxy)-4,6-dimethoxypyrimidin-2-yl]-1H-indol-3-sulfonamid;
6-Chlor-N-[4,6-dimethoxy-5-(1,1,2-trifluorethoxy)pyrimidin-2-yl]-1H-indol-3-sulfonamid;
N-(5-Allyl-4,6-dimethoxypyrimidin-2-yl)-6-chlor-1H-indol-3-sulfonamid;
6-Chlor-N-(4,6-dimethoxy-5-prop-1-inylpyrimidin-2-yl)-1H-indol-3-sulfonamid;
6-Chlor-N-(4,6-dimethoxy-5-propylpyrimidin-2-yl)-1H-indol-3-sulfonamid;
6-Chlor-N-[5-(2-fluorethyl)-4,6-dimethoxypyrimidin-2-yl]-1H-indol-3-sulfonamid;
6-Chlor-N-[5-(2,2-difluorethyl)-4,6-dimethoxypyrimidin-2-yl]-1H-indol-3-sulfonamid;
6-Chlor-N-[5-(3-fluorpropyl)-4,6-dimethoxypyrimidin-2-yl]-1H-indol-3-sulfonamid;
6-Chlor-N-[5-(3,3-difluorpropyl)-4,6-dimethoxypyrimidin-2-yl]-1H-indol-3-sulfonamid;
6-Chlor-N-[4,6-dimethoxy-5-(3,3,3-trifluorpropyl)pyrimidin-2-yl]-1H-indol-3-sulfonamid;
6-Chlor-N-[4,6-dimethoxy-5-(2-methoxyethyl)pyrimidin-2-yl]-1H-indol-3-sulfonamid;
6-Chlor-N-[5-(2-cyanoethyl)-4,6-dimethoxypyrimidin-2-yl]-1H-indol-3-sulfonamid;
6-Chlor-N-(5-cyclopropyl-4,6-dimethoxypyrimidin-2-yl)-1H-indol-3-sulfonamid;
6-Chlor-N-[4-(difluormethoxy)-6-methoxy-5-methylpyrimidin-2-yl]-1H-indol-3-sulfonamid;
6-Brom-N-[5-(2,2-difluorethyl)-4-methoxypyrimidin-2-yl]-1H-indol-3-sulfonamid;
6-Brom-N-[5-(difluormethoxy)-4,6-dimethoxypyrimidin-2-yl]-1H-indol-3-sulfonamid;
6-Brom-N-[5-(2-fluorethoxy)-4,6-dimethoxypyrimidin-2-yl]-1H-indol-3-sulfonamid;
6-Brom-N-[5-(2,2-difluorethoxy)-4,6-dimethoxypyrimidin-2-yl]-1H-indol-3-sulfonamid;
6-Brom-N-[5-(2-cyanoethyl)-4,6-dimethoxypyrimidin-2-yl]-1H-indol-3-sulfonamid;
N-[5-(2,2-Difluorethoxy)-4,6-dimethoxypyrimidin-2-yl]-6-(difluormethyl)-1H-pyrrolo[2,3-b]pyridin-3-sulfonamid;
6-(Difluormethyl)-N-[4,6-dimethoxy-5-(1,1,2-trifluorethoxy)pyrimidin-2-yl]-1H-pyrrolo[2,3-b]pyridin-3-sulfonamid;
6-(Difluormethyl)-N-[5-(3,3-difluorpropyl)-4,6-dimethoxypyrimidin-2-yl]-1H-pyrrolo[2,3-b]pyridin-3-sulfonamid;
N-[5-(Difluormethoxy)-4,6-dimethoxypyrimidin-2-yl]-6-(difluormethyl)-1H-indol-3-sulfonamid;
N-[5-(2,2-Difluorethoxy)-4,6-dimethoxypyrimidin-2-yl]-6-(difluormethyl)-1H-indol-3-sulfonamid;
N-[5-(2-Cyanoethyl)-4,6-dimethoxypyrimidin-2-yl]-6-(difluormethyl)-1H-indol-3-sulfonamid;
6-Chlor-N-[5-(2-cyanoethyl)-4-methoxypyrimidin-2-yl]-7-(difluormethylthio)-1H-indol-3-sulfonamid;
6-Chlor-7-(difluormethylthio)-N-[5-(2-fluorethoxy)-4,6-dimethoxypyrimidin-2-yl]-1H-indol-3-sulfonamid;
6-Chlor-N-[5-(2-cyanoethyl)-4,6-dimethoxypyrimidin-2-yl]-7-(difluormethylthio)- 1H-indol-3-sulfonamid;
6-Brom-N-[5-(2-fluorethoxy)-4,6-dimethoxypyrimidin-2-yl]-1H-pyrrolo[2,3-b]pyridin-3-sulfonamid;
6-Brom-N-[4,6-dimethoxy-5-(1,1,2-trifluorethoxy)pyrimidin-2-yl]-1H-pyrrolo[2,3-b]pyridin-3-sulfonamid;
6-Brom-N-[5-(2,2-difluorethyl)-4,6-dimethoxypyrimidin-2-yl]-1H-pyrrolo[2,3-b]pyridin-3-sulfonamid;
6-Brom-N-[5-(3,3-difluorpropyl)-4,6-dimethoxypyrimidin-2-yl]-1H-pyrrolo[2,3-b]pyridin-3-sulfonamid;
6-Brom-N-[5-(2-cyanoethyl)-4,6-dimethoxypyrimidin-2-yl]-1H-pyrrolo[2,3-b]pyridin-3-sulfonamid;
6-Brom-N-[5-(cyanomethoxy)-4,6-dimethoxypyrimidin-2-yl]-1H-pyrrolo[2,3-b]pyridin-3-sulfonamid;
6-Chlor-N-[5-(2,2-difluorethyl)-4,6-dimethoxypyrimidin-2-yl]-7-(dimethylamino)- 1H-indol-3-sulfonamid;
N-[5-(Difluormethoxy)-4,6-dimethoxypyrimidin-2-yl]-1H-pyrrolo[3,2-h]chinolin-3-sulfonamid;
N-[5-(2-Fluorethoxy)-4,6-dimethoxypyrimidin-2-yl]-1H-pyrrolo[3,2-h]chinolin-3-sulfonamid;
6-Chlor-N-(4,6-dimethoxy-5-methylpyrimidin-2-yl)-1H-pyrrolo[2,3-b]pyridin-3-sulfonamid;
6-Chlor-N-[5-(2,2-difluorethyl)-4,6-dimethoxypyrimidin-2-yl]-1H-pyrrolo[2,3-b]pyridin-3-sulfonamid;
6-Chlor-N-[5-(3,3-difluorpropyl)-4,6-dimethoxypyrimidin-2-yl]-1H-pyrrolo[2,3-b]pyridin-3-sulfonamid;
6-Chlor-N-[5-(2-cyanoethyl)-4,6-dimethoxypyrimidin-2-yl]-1H-pyrrolo[2,3-b]pyridin-3-sulfonamid;
6-Chlor-N-[5-(2,2-difluorethyl)-4-methoxypyrimidin-2-yl]-7-fluor-1H-indol-3-sulfonamid;
6-Chlor-N-[5-(difluormethoxy)-4,6-dimethoxypyrimidin-2-yl]-7-fluor-1H-indol-3-sulfonamid;
6-Chlor-7-(cyclopropoxy)-N-[5-(2,2-difluorethyl)-4-methoxypyrimidin-2-yl]-1H-indol-3-sulfonamid;
N-[5-(2,2-Difluorethyl)-4,6-dimethoxypyrimidin-2-yl]-6-methyl-1H-pyrrolo[2,3-b]pyridin-3-sulfonamid;
N-[5-(2,2-Difluorethyl)-4-methoxypyrimidin-2-yl]-6-fluor-1H-indol-3-sulfonamid;
7-Brom-6-chlor-N-[5-(2-fluorethoxy)-4-methoxypyrimidin-2-yl]-1H-indol-3-sulfonamid;
7-Brom-6-chlor-N-[5-(2,2-difluorethyl)-4-methoxypyrimidin-2-yl]-1H-indol-3-sulfonamid;
6-Brom-N-(4,6-dimethoxy-5-methylpyrimidin-2-yl)thieno[2,3-b]pyridin-3-sulfonamid;
6-Brom-N-[5-(2,2-difluorethyl)-4-methoxypyrimidin-2-yl]thieno[2,3-b]pyridin-3-sulfonamid;
6-Brom-N-[5-(2,2-difluorethyl)-4,6-dimethoxypyrimidin-2-yl]thieno[2,3-b]pyridin-3-sulfonamid;
6-Brom-N-[5-(2-cyanoethyl)-4,6-dimethoxypyrimidin-2-yl]thieno[2,3-b]pyridin-3-sulfonamid;
6-Chlor-N-[5-(2,2-difluorethyl)-4-methoxypyrimidin-2-yl]benzothiophen-3-sulfonamid;
6-Brom-N-[5-(2-fluorethoxy)-4-methoxypyrimidin-2-yl]benzothiophen-3-sulfonamid;
6-Brom-N-(4,6-dimethoxy-5-methylpyrimidin-2-yl)-1H-pyrrolo[2,3-b]pyridin-3-sulfonamid;
N-[5-(2,2-Difluorethoxy)-4,6-dimethoxypyrimidin-2-yl]-1H-pyrrolo[3,2-h]chinolin-3-sulfonamid;
N-[5-(2,2-Difluorethyl)-4,6-dimethoxypyrimidin-2-yl]-1H-pyrrolo[3,2-h]chinolin-3-sulfonamid;
6-Brom-N-[5-(difluormethoxy)-4,6-dimethoxypyrimidin-2-yl]-1H-pyrrolo[2,3-b]pyridin-3-sulfonamid;
6-Chlor-N-[5-(2,3-difluorpropyl)-4-methoxypyrimidin-2-yl]-1H-indol-3-sulfonamid;
6-Chlor-N-[5-(2-cyanoethyl)-4-methoxypyrimidin-2-yl]-1H-indol-3-sulfonamid; 7-Brom-6-chlor-N-[5-(3,3-difluorpropyl)-4-methoxypyrimidin-2-yl]-1H-indol-3-sulfonamid;
6-Brom-N-[5-(2,2-difluorethyl)-4-methoxypyrimidin-2-yl]-7-methyl-1H-indol-3-sulfonamid;
6-Chlor-N-[5-(2,2-difluorethyl)-4-methoxypyrimidin-2-yl]-7-methoxy-1H-indol-3-sulfonamid;
6-Chlor-N-[5-(3,3-difluorpropyl)-4-methoxypyrimidin-2-yl]-7-methoxy-1H-indol-3-sulfonamid;
6-Chlor-N-[5-(2,2-difluorethyl)-4-methoxypyrimidin-2-yl]-7-(difluormethoxy)-1H-indol-3-sulfonamid;
6-Brom-N-[5-(2,2-difluorethyl)-4-methoxypyrimidin-2-yl]-7-fluor-1H-indol-3-sulfonamid;
6-Chlor-N-[5-(2,2-difluorethyl)-4-methoxypyrimidin-2-yl]-7-methyl-1H-indol-3-sulfonamid;
N-[5-(2,2-Difluorethyl)-4,6-dimethoxypyrimidin-2-yl]-6-(difluormethyl)-1H-pyrrolo[2,3-b]pyridin-3-sulfonamid;
6-Brom-N-[5-(2,2-difluorethyl)-4-methoxypyrimidin-2-yl]-7-methoxy-1H-indol-3-sulfonamid;
6-Chlor-N-[5-(2,2-difluorethyl)-4-methoxypyrimidin-2-yl]-7-(fluormethoxy)-1H-indol-3-sulfonamid;
6-Chlor-N-[5-(2,2-difluorethyl)-4-methoxypyrimidin-2-yl]-7-(difluormethylsulfanyl)-1H-indol-3-sulfonamid;
6-Brom-N-[5-(3,3-difluorpropyl)-4-methoxypyrimidin-2-yl]-1H-pyrrolo[2,3-b]pyridin-3-sulfonamid;
6-Brom-N-[5-(2,2-difluorethoxy)-4,6-dimethoxypyrimidin-2-yl]-1H-pyrrolo[2,3-b]pyridin-3-sulfonamid;
6-Chlor-N-[5-(3,3-difluorpropyl)-4-methoxypyrimidin-2-yl]-1H-pyrrolo[2,3-b]pyridin-3-sulfonamid;
6-Chlor-N-[5-(difluormethoxy)-4,6-dimethoxypyrimidin-2-yl]-1H-pyrrolo[2,3-b]pyridin-3-sulfonamid;
6-Chlor-N-[5-(3,3-difluorpropyl)-4-methoxypyrimidin-2-yl]-7-fluor-1H-indol-3-sulfonamid;
6-Chlor-N-[5-(2,2-difluorethoxy)-4,6-dimethoxypyrimidin-2-yl]-7-fluor-1H-indol-3-sulfonamid;
6,7-Dichlor-N-[5-(2,2-difluorethyl)-4-methoxypyrimidin-2-yl]-1H-indol-3-sulfonamid;
6,7-Dichlor-N-[5-(3,3-difluorpropyl)-4-methoxypyrimidin-2-yl]-1H-indol-3-sulfonamid;
6-Chlor-7-(cyclopropoxy)-N-[5-(3,3-difluorpropyl)-4-methoxypyrimidin-2-yl]-1H-indol-3-sulfonamid;
6-Brom-7-fluor-N-[5-(2-fluorethoxy)-4-methoxypyrimidin-2-yl]-1H-indol-3-sulfonamid;
6-Chlor-N-[5-(2,2-difluorethyl)-4-methoxypyrimidin-2-yl]-7-(difluormethyl)-1H-indol-3-sulfonamid;
6-Chlor-7-fluor-N-[4-methoxy-5-(1,1,2-trifluorethoxy)pyrimidin-2-yl]-1H-indol-3-sulfonamid;
6-Chlor-N-[5-(2-cyanoethyl)-4-methoxypyrimidin-2-yl]-7-fluor-1H-indol-3-sulfonamid;
6-Chlor-N-[5-(2,2-difluorethyl)-4,6-dimethoxypyrimidin-2-yl]-7-fluor-1H-indol-3-sulfonamid;
6-Chlor-N-[5-(2-cyanoethyl)-4,6-dimethoxypyrimidin-2-yl]-7-fluor-1H-indol-3-sulfonamid;
6-Chlor-N-[5-(cyanomethoxy)-4,6-dimethoxypyrimidin-2-yl]-7-fluor-1H-indol-3-sulfonamid;
6-Chlor-N-[4-methoxy-5-(1,1,2-trifluorethoxy)pyrimidin-2-yl]-1H-pyrrolo[2,3-b]pyridin-3-sulfonamid;
6-Chlor-N-[5-(2-fluorethoxy)-4,6-dimethoxypyrimidin-2-yl]-1H-pyrrolo[2,3-b]pyridin-3-sulfonamid;
6-Chlor-7-cyano-N-[5-(2,2-difluorethyl)-4-methoxypyrimidin-2-yl]-1H-indol-3-sulfonamid;
6-Chlor-7-cyano-N-[5-(difluormethoxy)-4,6-dimethoxypyrimidin-2-yl]-1H-indol-3-sulfonamid;
6-Chlor-7-cyano-N-[5-(2-fluorethoxy)-4,6-dimethoxypyrimidin-2-yl]-1H-indol-3-sulfonamid;
6-Chlor-7-cyano-N-[5-(2,2-difluorethoxy)-4,6-dimethoxypyrimidin-2-yl]-1H-indol-3-sulfonamid;
6-Chlor-7-cyano-N-[5-(2,2-difluorethyl)-4,6-dimethoxypyrimidin-2-yl]-1H-indol-3-sulfonamid;
N-[5-(2,2-Difluorethyl)-4,6-dimethoxypyrimidin-2-yl]-6,8-dihydro-1H-fur[3,4-g]indol-3-sulfonamid;
6-Brom-N-[5-(2-fluorethoxy)-4-methoxypyrimidin-2-yl]-1H-pyrrolo[2,3-b]pyridin-3-sulfonamid;
6-Brom-N-[4-methoxy-5-(1,1,2-trifluorethoxy)pyrimidin-2-yl]-1H-pyrrolo[2,3-b]pyridin-3-sulfonamid;
6-Chlor-7-(cyanomethyl)-N-[5-(2,2-difluorethyl)-4-methoxypyrimidin-2-yl]-1H-indol-3-sulfonamid;
6-Chlor-N-[5-(2,2-difluorethyl)-4-methoxypyrimidin-2-yl]-7-(methylthio)-1H-indol-3-sulfonamid;
6-(Difluormethyl)-N-[5-(2-fluorethoxy)-4-methoxypyrimidin-2-yl]-1H-indol-3-sulfonamid;
N-[5-(2,2-Difluorethyl)-4,6-dimethoxypyrimidin-2-yl]-6-(difluormethyl)-1H-indol-3-sulfonamid;
N-[5-(Cyanomethoxy)-4,6-dimethoxypyrimidin-2-yl]-6-(difluormethyl)-1H-indol-3-sulfonamid;
N-[5-(2-Cyanoethyl)-4,6-dimethoxypyrimidin-2-yl]-6-(difluormethyl)-1H-pyrrolo[2,3-b]pyridin-3-sulfonamid;
6-Brom-N-[5-(2-cyanoethyl)-4-methoxypyrimidin-2-yl]-7-fluor-1H-indol-3-sulfonamid;
6-Brom-N-[5-(difluormethoxy)-4,6-dimethoxypyrimidin-2-yl]-7-fluor-1H-indol-3-sulfonamid;
6-Brom-N-[5-(2,2-difluorethoxy)-4,6-dimethoxypyrimidin-2-yl]-7-fluor-1H-indol-3-sulfonamid;
6-Brom-N-[5-(2-cyanoethyl)-4,6-dimethoxypyrimidin-2-yl]-7-fluor-1H-indol-3-sulfonamid;
6-Brom-N-[5-(cyanomethoxy)-4,6-dimethoxypyrimidin-2-yl]-7-fluor-1H-indol-3-sulfonamid;
6-Brom-N-[5-(2,2-difluorethyl)-4,6-dimethoxypyrimidin-2-yl]-1H-indol-3-sulfonamid;
6-Brom-N-[5-(cyanomethoxy)-4,6-dimethoxypyrimidin-2-yl]-1H-indol-3-sulfonamid;
6-(Difluormethyl)-7-fluor-N-[5-(2-fluorethoxy)-4-methoxypyrimidin-2-yl]-1H-indol-3-sulfonamid;
N-[5-(Difluormethoxy)-4,6-dimethoxypyrimidin-2-yl]-6-(difluormethyl)-7-fluor-1H-indol-3-sulfonamid;
N-[5-(2,2-Difluorethoxy)-4,6-dimethoxypyrimidin-2-yl]-6-(difluormethyl)-7-fluor-1H-indol-3-sulfonamid;
N-[5-(2,2-Difluorethyl)-4,6-dimethoxypyrimidin-2-yl]-6-(difluormethyl)-7-fluor-1H-indol-3-sulfonamid;
N-[5-(2-Cyanoethyl)-4,6-dimethoxypyrimidin-2-yl]-6-(difluormethyl)-7-fluor-1H-indol-3-sulfonamid;
N-[5-(Cyanomethoxy)-4,6-dimethoxypyrimidin-2-yl]-6-(difluormethyl)-7-fluor-1H-indol-3-sulfonamid;
7-Brom-6-chlor-N-[5-(2-cyanoethyl)-4-methoxypyrimidin-2-yl]-1H-indol-3-sulfonamid;
N-[5-(2,2-Difluorethyl)-4,6-dimethoxypyrimidin-2-yl]-6-fluor-1H-indol-3-sulfonamid;
N-[5-(2-Cyanoethyl)-4,6-dimethoxypyrimidin-2-yl]-6-fluor-1H-indol-3-sulfonamid;
6-Chlor-N-[5-(difluormethoxymethyl)-4-methoxypyrimidin-2-yl]-1H-indol-3-sulfonamid;
6-Chlor-N-[5-(2,3-difluorpropyl)-4,6-dimethoxypyrimidin-2-yl]-1H-indol-3-sulfonamid;
6-Chlor-N-[5-(cyanomethoxy)-4,6-dimethoxypyrimidin-2-yl]-1H-indol-3-sulfonamid;
6-Chlor-N-[5-(cyanomethoxy)-4-methoxypyrimidin-2-yl]-1H-indol-3-sulfonamid; 7-Brom-6-chlor-N-[5-(2,2-difluorethyl)-4,6-dimethoxypyrimidin-2-yl]-1H-indol-3-sulfonamid;
6-Chlor-7-(cyanomethoxy)-N-[5-(2,2-difluorethyl)-4,6-dimethoxypyrimidin-2-yl]-1H-indol-3-sulfonamid;
und pharmazeutisch verträgliche Salze davon.

13. Verbindung nach Anspruch 11 oder Anspruch 12, wobei die Verbindung 6-Brom-N-[5-(difluormethoxy)-4,6-dimethoxypyrimidin-2-yl]-1H-pyrrolo[2,3-b]pyridin-3-sulfonamid oder ein pharmazeutisch verträgliches Salz davon ist.

14. Verbindung nach einem der Ansprüche 11 bis 13, wobei die Verbindung 6-Brom-N-[5-(difluormethoxy)-4,6-dimethoxypyrimidin-2-yl]-1H-pyrrolo[2,3-b]pyridin-3-sulfonamid ist.

15. Verfahren zur Herstellung einer Verbindung nach einem der Ansprüche 1 bis 14, umfassend die Umsetzung einer Verbindung der Formel II 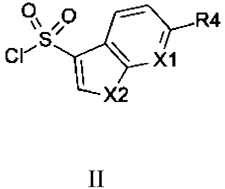
mit einer Verbindung der Formel III 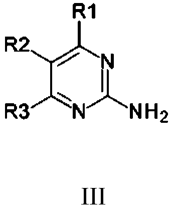
in Gegenwart einer Base, ausgewählt aus N,N-Diisopropylethylamin oder Pyridin, wobei R¹, R², R³, R⁴, X₁ und X₂ wie oben definiert sind.

16. Verbindung nach einem der Ansprüche 1 bis 14 zur Verwendung als therapeutisch aktive Substanz.

17. Pharmazeutische Zusammensetzung, umfassend eine Verbindung nach einem der Ansprüche 1 bis 14 und einen therapeutisch inerten Träger.

18. Verbindung nach einem der Ansprüche 1 bis 14 zur Verwendung bei der Behandlung oder Prophylaxe von Erkrankungen, die aus direkter Schädigung von Myelinscheiden resultieren (einschließlich, aber nicht beschränkt auf zentrale Pontin- und Extra-Pontin-Myelinolyse, Kohlenmonoxidvergiftung, Ernährungsmangel und virusinduzierte Demyelinisierung), demyelinisierenden Störungen (einschließlich, aber nicht beschränkt auf Multiple Sklerose, akute und mehrphasige disseminierte Enzephalomyelitis, Neuromyelitis-optica-Spektrum-Störungen und Leukodystrophien), Störungen des ZNS, die mit Myelinverlust assoziiert sind (einschließlich, aber nicht beschränkt auf Alzheimer-Krankheit, Schizophrenie, Parkinson-Krankheit, Huntington-Krankheit, amyotrophe Lateralsklerose und Ischämie aufgrund von Schlaganfall), und Entzündung im ZNS, zum Beispiel nach Enzephalitis, primärer Angiitis, Meningitis und Fettleibigkeit.

19. Verbindung nach einem der Ansprüche 1 bis 14 zur Verwendung bei der Behandlung oder Prophylaxe von Multipler Sklerose.

## Revendications

1. Composés de formule I 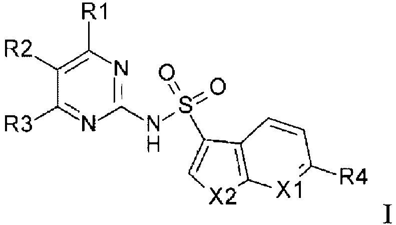 dans lesquels
R¹ représente H, un halogène, un alkyle, un alcoxy, un alcoxyalkyle, un alcoxyalcoxy, un cyanoalkyle, un halogénoalkyle, un halogénoalcoxy, un thiolalkyle, un cycloalkyle, un cycloalkylalkyle, un cycloalcoxy ou un hétérocycloalkyle ;
R² représente un cyano, un halogène, un alkyle, un alcényle, un alcynyle, un alcoxy, un alcénylalkyle, un alcynylalcoxy, un alcoxyalkyle, un alcoxyalcoxy, un halogénoalkyle, un halogénoalcoxy, un halogénoalcoxyalkyle, un (halogénoalcoxy)alcoxy, un cyanoalkyle, un cyanoalcoxy, un thiolalkyle, un halogénothiolalkyle, un halogénohydroxyalcoxy, un alkylsulfonylalkyle, un cycloalkyle, un cycloalkylalkyle, un cycloalkylalcoxy, un cycloalcoxy, un hétérocycloalkyle, un hétérocycloalkylalkyle, un hétérocycloalkylalcynyle ou un hétérocycloalkylalcoxy ;
R³ représente H, un halogène, un alkyle, un alcoxy, un halogénoalcoxy ou un thiolalkyle ;
X₁ représente CR⁵ ou N ;
X₂ représente NH, -N-R⁶, S, -S(O)- ou -S(O₂)- ;
R⁴ représente H, un halogène, un alkyle, un hydroxyalkyle, un thiolalkyle, un cyanoalkyle, un alcoxy, un halogénoalkyle, un halogénoalcoxy, un cycloalkyle ou un hétérocycloalkyle ;
R⁵ représente H, un halogène, un cyano, un alkyle, un alcoxy, un cyanoalkyle, un cyanoalcoxy, un N-alkyle, un dialkylamino, un halogénoalkyle, un halogénoalcoxy, un halogénothiolalkyle, un alkylsulfinyle, un alkylsulfonyle, une alkylsulfone, un thiolalkyle, un hydroxyalkyle, un alcoxycarbonyle, un hétérocycloalkyle, un cycloalkyle ou un cycloalcoxy ;
ou R⁴ et R⁵ sont liés pour former un cycloalkyle à 5 ou 6 chaînons, un hétérocycloalkyle, un hétérocycloalkyle substitué comprenant un ou deux substituants indépendamment choisis parmi un alkyle et un oxo ou un cycle hétéroaromatique ;
R⁶ représente H, un alkyle, un alkylhydroxy ou un halogénoalkyle ;
et des sels pharmaceutiquement acceptables.

2. Composé selon la revendication 1, dans lequel R¹ représente H, un halogène, un halogénoalkyle, un halogénoalcoxy, un alkyle, un alcoxy, un alcoxyalcoxy, un alcoxyalkyle, un thiolalkyle, un cyanoalkyle, un cycloalcoxy, un cycloalkyle, un cycloalkylalkyle ou un hétérocycloalkyle.

3. Composé selon les revendications 1 à 2, dans lequel R² représente un alcoxy, un alcoxyalcoxy, un alcoxyalkyle, un alkylsulfonylalkyle, un alkyle, un alcényle, un alcynyle, un alcynylalcoxy, un cyano, un cyanoalkyle, un cycloalkyle, un cycloalcoxy, un cycloalkylalkyle, un cyanoalcoxy, un halogène, un halogénoalcoxy, un (halogénoalcoxy)alcoxy, un thiolalkyle, un halogénothiolalkyle, un halogénohydroxyalcoxy, un halogénoalkyle, un hétérocycloalkyle, un hétérocycloalkylalkyle, un hétérocycloalkylalcynyle ou un hétérocycloalkylalcoxy.

4. Composé selon les revendications 1 à 3, dans lequel R³ représente H ou un alcoxy.

5. Composé selon les revendications 1 à 4, dans lequel X² représente NH ou S.

6. Composé selon les revendications 1 à 5, dans lequel R⁴ représente H, un halogène, un alkyle, un alcoxy, un thiolalkyle, un cyanoalkyle, un cycloalkyle, un halogénoalcoxy, un halogénoalkyle, un hétérocycloalkyle ou un hydroxyalkyle, et R⁵ représente H, un halogène, un halogénoalkyle, un halogénoalcoxy, un halogénothiolalkyle, un alcoxy, un alcoxycarbonyle, un alkyle, un hydroxyalkyle, un thiolalkyle, un alkylsulfino, un alkylsulfano, un cyano, un cyanoalkyle, un cyanoalcoxy, un dialkylamino, un cycloalkyle, un cycloalcoxy ou un hétérocycloalkyle, ou R⁴ et R⁵ sont liés pour former un hétérocycloalkyle à 5 chaînons comprenant un seul hétéroatome O, un hétérocycloalkyle à 6 chaînons comprenant un seul hétéroatome N substitué par un oxo et un alkyle, un cycle hétéroaromatique à 5 chaînons comprenant un hétéroatome N et un hétéroatome S ou un cycle hétéroaromatique à 6 chaînons comprenant un seul hétéroatome N.

7. Composé selon les revendications 1 à 6, dans lequel R⁴ représente un halogène, un alkyle ou un halogénoalkyle, et R⁵ représente H, un halogène, un halogénoalkyle, un halogénoalcoxy, un halogénothiolalkyle, un alcoxy, un alkyle, un thiolalkyle, un cyano, un cyanoalkyle, un cyanoalcoxy, un dialkylamino ou un cycloalcoxy, ou R⁴ et R⁵ sont liés pour former un hétérocycloalkyle à 5 chaînons comprenant un seul hétéroatome O ou un cycle hétéroaromatique à 6 chaînons comprenant un seul hétéroatome N.

8. Composé selon les revendications 1 à 7, dans lequel R⁵ représente H, un halogène, un halogénoalkyle, un halogénoalcoxy, un halogénothiolalkyle, un alcoxy, un alkyle, un thiolalkyle, un cyano, un cyanoalkyle, un cyanoalcoxy, un dialkylamino ou un cycloalcoxy.

9. Composé selon les revendications 1 à 8, dans lequel
R¹ représente H, un halogène, un halogénoalkyle, un halogénoalcoxy, un alkyle, un alcoxy, un alcoxyalcoxy, un alcoxyalkyle, un thiolalkyle, un cyanoalkyle, un cycloalcoxy, un cycloalkyle, un cycloalkylalkyle ou un hétérocycloalkyle ;
R² représente un alcoxy, un alcoxyalcoxy, un alcoxyalkyle, un alkylsulfonylalkyle, un alkyle, un alcényle, un alcynyle, un alcynylalcoxy, un cyano, un cyanoalkyle, un cycloalkyle, un cycloalcoxy, un cycloalkylalkyle, un cyanoalcoxy, un halogène, un halogénoalcoxy, un (halogénoalcoxy)alcoxy, un thiolalkyle, un halogénothiolalkyle, un halogénohydroxyalcoxy, un halogénoalkyle, un hétérocycloalkyle, un hétérocycloalkylalkyle, un hétérocycloalkylalcynyle ou un hétérocycloalkylalcoxy ;
R³ représente H, un halogène, un alkyle, un alcoxy, un halogénoalcoxy ou un thiolalkyle ;
X₁ représente CR⁵ ou N ;
X₂ représente NH ou S ;
R⁴ représente H, un halogène, un alkyle, un alcoxy, un thiolalkyle, un cyanoalkyle, un cycloalkyle, un halogénoalcoxy, un halogénoalkyle, un hétérocycloalkyle ou un hydroxyalkyle, et R⁵ représente H, un halogène, un halogénoalkyle, un halogénoalcoxy, un halogénothiolalkyle, un alcoxy, un alkyle, un alcoxycarbonyle, un hydroxyalkyle, un thiolalkyle, un alkylsulfino, un alkylsulfano, un cyano, un cyanoalkyle, un cyanoalcoxy, un dialkylamino, un cycloalkyle, un cycloalcoxy ou un hétérocycloalkyle, ou R⁴ et R⁵ sont liés pour former un hétérocycloalkyle à 5 chaînons comprenant un seul hétéroatome O, un hétérocycloalkyle à 6 chaînons comprenant un seul hétéroatome N substitué par un alkyle et un oxo, un cycle hétéroaromatique à 5 chaînons comprenant un hétéroatome N et un hétéroatome S ou un cycle hétéroaromatique à 6 chaînons comprenant un seul hétéroatome N ;
et des sels pharmaceutiquement acceptables.

10. Composé selon l'une quelconque des revendications 1 à 9, dans lequel
R¹ représente H, un alcoxy, un cycloalkyle ou un halogénoalcoxy ;
R² représente un alkyle, un alcényle, un alcynyle, un alcoxyalkyle, un alcynyle, un cyanoalcoxy, un halogène, un halogénoalcoxy, un halogénoalkyle ou un cycloalkyle ;
R³ représente H ou un alcoxy ;
X₁ représente CR⁵ ou N ;
X₂ représente NH ou S ;
R⁴ représente un halogène, un alkyle ou un halogénoalkyle, et R⁵ représente H, un halogène, un halogénoalkyle, un halogénoalcoxy, un halogénothiolalkyle, un alcoxy, un alkyle, un thiolalkyle, un cyano, un cyanoalkyle, un cyanoalcoxy, un dialkylamino ou un cycloalcoxy, ou R⁴ et R⁵ sont liés pour former un hétérocycloalkyle à 5 chaînons comprenant un seul hétéroatome O ou un cycle hétéroaromatique à 6 chaînons comprenant un seul hétéroatome N ;
et des sels pharmaceutiquement acceptables.

11. Composé selon l'une quelconque des revendications 1 à 10, choisi parmi
le 6-chloro-N-[5-(2-fluoroéthoxy)-4-méthoxy-pyrimidin-2-yl]-1H-indole-3-sulfonamide ;
le 6-chloro-7-fluoro-N-[5-(2-fluoroéthoxy)-4-méthoxy-pyrimidin-2-yl]-1H-indole-3-sulfonamide ;
le 6,7-dichloro-N-[5-(2-fluoroéthoxy)-4-méthoxy-pyrimidin-2-yl]-1H-indole-3-sulfonamide ;
le 6-chloro-7-(difluorométhyl)-N-[5-(2-fluoroéthoxy)-4-méthoxy-pyrimidin-2-yl]-1H-indole-3-sulfonamide ;
le 6-chloro-7-(cyclopropoxy)-N-[5-(2-fluoroéthoxy)-4-méthoxy-pyrimidin-2-yl]-1H-indole-3-sulfonamide ;
le 6-chloro-N-(5-éthoxy-4-méthoxy-pyrimidin-2-yl)-1H-indole-3-sulfonamide ;
le 6-chloro-N-[4-méthoxy-5-(2-méthoxyéthoxy)pyrimidin-2-yl]-1H-indole-3-sulfonamide ;
le 6-chloro-N-[5-(difluorométhoxy)-4-méthoxy-pyrimidin-2-yl]-1H-indole-3-sulfonamide ;
le 6-chloro-N-[5-[2-(difluorométhoxy)éthoxy]-4-méthoxy-pyrimidin-2-yl]-1H-indole-3-sulfonamide ;
le 6-chloro-N-[5-(2-fluoroéthoxy)-4-méthoxy-pyrimidin-2-yl]-1H-pyrrolo[2,3-b]pyridine-3-sulfonamide ;
le 6-chloro-N-(5-chloro-4,6-diméthoxy-pyrimidin-2-yl)-1H-indole-3-sulfonamide ;
le N-(5-bromo-4,6-diméthoxy-pyrimidin-2-yl)-6-chloro-1H-indole-3-sulfonamide ;
le N-(5-bromo-4-éthoxy-6-méthoxy-pyrimidin-2-yl)-6-chloro-1H-indole-3-sulfonamide ;
le N-[5-bromo-4-(2-fluoroéthoxy)-6-méthoxy-pyrimidin-2-yl]-6-chloro-1H-indole-3-sulfonamide ;
le 6-chloro-N-(5-éthyl-4-méthoxy-pyrimidin-2-yl)-1H-indole-3-sulfonamide ;
le 6-chloro-N-(5-éthyl-4,6-diméthoxy-pyrimidin-2-yl)-1H-indole-3-sulfonamide ;
le 6-chloro-N-(4,6-diméthoxy-5-méthyl-pyrimidin-2-yl)-1H-indole-3-sulfonamide ;
le N-[5-bromo-4-(difluorométhoxy)-6-méthoxy-pyrimidin-2-yl]-6-chloro-1H-indole-3-sulfonamide ;
le 6-chloro-N-[4-cyclopropyl-5-(2,2-difluoroéthoxy)-6-méthoxy-pyrimidin-2-yl]-1H-indole-3-sulfonamide ;
le 6-chloro-N-[5-(2,2-difluoroéthoxy)-4-méthoxy-6-méthyl-pyrimidin-2-yl]-1H-indole-3-sulfonamide ;
le 6-chloro-N-[5-(3-fluoropropyl)-4-méthoxy-pyrimidin-2-yl]-1H-indole-3-sulfonamide ;
le N-(5-bromo-4-méthoxy-pyrimidin-2-yl)-6-chloro-1H-indole-3-sulfonamide ;
le 6-chloro-N-(5-cyano-4-méthoxy-pyrimidin-2-yl)-1H-indole-3-sulfonamide ;
le 6-chloro-N-[5-(2-fluoroéthoxy)-4-méthoxy-pyrimidin-2-yl]benzothiophène-3-sulfonamide ;
le 6-bromo-N-[5-(2-fluoroéthoxy)-4-méthoxy-pyrimidin-2-yl]-1H-indole-3-sulfonamide ;
le 6-chloro-N-(4-méthoxy-5-méthyl-pyrimidin-2-yl)-1H-indole-3-sulfonamide ;
le 6-chloro-N-[5-(2-fluoroéthoxy)-4-méthoxy-pyrimidin-2-yl]thiéno[2,3-b]pyridine-3-sulfonamide ;
le 6-chloro-N-(5-cyclopropyl-4-méthoxy-pyrimidin-2-yl)-1H-indole-3-sulfonamide ;
le 6-chloro-N-(4-méthoxy-5-prop-1-ynyl-pyrimidin-2-yl)-1H-indole-3-sulfonamide ;
le 6-bromo-N-(5-bromo-4,6-diméthoxy-pyrimidin-2-yl)-1H-pyrrolo[2,3-b]pyridine-3-sulfonamide ;
le N-(5-bromo-4,6-diméthoxy-pyrimidin-2-yl)-6-chloro-1H-pyrrolo[2,3-b]pyridine-3-sulfonamide ;
le 6-chloro-N-(5-cyano-4,6-diméthoxy-pyrimidin-2-yl)-1H-indole-3-sulfonamide ;
le N-(5-bromo-4,6-diméthyl-pyrimidin-2-yl)-6-chloro-1H-indole-3-sulfonamide ;
le 6-chloro-N-[5-(2,2-difluoroéthoxy)pyrimidin-2-yl]-1H-indole-3-sulfonamide ;
le 6-chloro-N-[5-(2,2-difluoroéthoxy)-4-méthylsulfanyl-pyrimidin-2-yl]-1H-indole-3-sulfonamide ;
le 6-chloro-N-(4-méthoxy-5-méthylsulfanyl-pyrimidin-2-yl)-1H-indole-3-sulfonamide ;
le 6-chloro-N-[5-(difluorométhylthio)-4-méthoxy-pyrimidin-2-yl]-1H-indole-3-sulfonamide ;
le 6-chloro-N-[5-(cyclopropoxy)-4-méthoxy-pyrimidin-2-yl]-1H-indole-3-sulfonamide ;
le 6-chloro-N-[5-(3,3-difluoropropoxy)-4-méthoxy-pyrimidin-2-yl]-1H-indole-3-sulfonamide ;
le 6-chloro-N-[4-méthoxy-5-(3,3,3-trifluoropropoxy)pyrimidin-2-yl]-1H-indole-3-sulfonamide ;
le 6-chloro-N-[4-méthoxy-5-(1,1,2-trifluoroéthoxy)pyrimidin-2-yl]-1H-indole-3-sulfonamide ;
le 6-chloro-N-[5-(1,1-difluoroéthoxy)-4-méthoxy-pyrimidin-2-yl]-1H-indole-3-sulfonamide ;
le 6-chloro-N-[4-méthoxy-5-(1,1,2,2-tétrafluoroéthoxy)pyrimidin-2-yl]-1H-indole-3-sulfonamide ;
le 6-chloro-N-[5-(2-chloro-1,1,2-trifluoro-éthoxy)-4-méthoxy-pyrimidin-2-yl]-1H-indole-3-sulfonamide ;
le 6-chloro-N-(4-méthoxy-5-propyl-pyrimidin-2-yl)-1H-indole-3-sulfonamide ;
le 6-chloro-N-[5-(cyclopropylméthyl)-4-méthoxy-pyrimidin-2-yl]-1H-indole-3-sulfonamide ;
le 6-chloro-N-[5-(2,2-difluoroéthyl)-4-méthoxy-pyrimidin-2-yl]-1H-indole-3-sulfonamide ;
le 6-chloro-N-[5-(3,3-difluoropropyl)-4-méthoxy-pyrimidin-2-yl]-1H-indole-3-sulfonamide ;
le 6-chloro-N-[4-méthoxy-5-(2-méthoxyéthyl)pyrimidin-2-yl]-1H-indole-3-sulfonamide ;
le 6-chloro-N-[4-méthoxy-5-(oxétan-3-yl)pyrimidin-2-yl]-1H-indole-3-sulfonamide ;
le 6-chloro-N-[5-(fluorométhoxy)-4,6-diméthoxy-pyrimidin-2-yl]-1H-indole-3-sulfonamide ;
le 6-chloro-N-[5-(difluorométhoxy)-4,6-diméthoxy-pyrimidin-2-yl]-1H-indole-3-sulfonamide ;
le 6-chloro-N-[5-(2-fluoroéthoxy)-4,6-diméthoxy-pyrimidin-2-yl]-1H-indole-3-sulfonamide ;
le 6-chloro-N-[5-(2,2-difluoroéthoxy)-4,6-diméthoxy-pyrimidin-2-yl]-1H-indole-3-sulfonamide ;
le 6-chloro-N-[4,6-diméthoxy-5-(1,1,2-trifluoroéthoxy)pyrimidin-2-yl]-1H-indole-3-sulfonamide ;
le N-(5-allyl-4,6-diméthoxy-pyrimidin-2-yl)-6-chloro-1H-indole-3-sulfonamide ;
le 6-chloro-N-(4,6-diméthoxy-5-prop-1-ynyl-pyrimidin-2-yl)-1H-indole-3-sulfonamide ;
le 6-chloro-N-(4,6-diméthoxy-5-propyl-pyrimidin-2-yl)-1H-indole-3-sulfonamide ;
le 6-chloro-N-[5-(2-fluoroéthyl)-4,6-diméthoxy-pyrimidin-2-yl]-1H-indole-3-sulfonamide ;
le 6-chloro-N-[5-(2,2-difluoroéthyl)-4,6-diméthoxy-pyrimidin-2-yl]-1H-indole-3-sulfonamide ;
le 6-chloro-N-[5-(3-fluoropropyl)-4,6-diméthoxy-pyrimidin-2-yl]-1H-indole-3-sulfonamide ;
le 6-chloro-N-[5-(3,3-difluoropropyl)-4,6-diméthoxy-pyrimidin-2-yl]-1H-indole-3-sulfonamide ;
le 6-chloro-N-[5-(2,2-difluoropropyl)-4,6-diméthoxy-pyrimidin-2-yl]-1H-indole-3-sulfonamide ;
le 6-chloro-N-[4,6-diméthoxy-5-(3,3,3-trifluoropropyl)pyrimidin-2-yl]-1H-indole-3-sulfonamide ;
le 6-chloro-N-[4,6-diméthoxy-5-(méthoxyméthyl)pyrimidin-2-yl]-1H-indole-3-sulfonamide ;
le 6-chloro-N-[4,6-diméthoxy-5-(2-méthoxyéthyl)pyrimidin-2-yl]-1H-indole-3-sulfonamide ;
le 6-chloro-N-[5-(cyanométhyl)-4,6-diméthoxy-pyrimidin-2-yl]-1H-indole-3-sulfonamide ;
le 6-chloro-N-[5-(2-cyanoéthyl)-4,6-diméthoxy-pyrimidin-2-yl]-1H-indole-3-sulfonamide ;
le 6-chloro-N-(5-cyclopropyl-4,6-diméthoxy-pyrimidin-2-yl)-1H-indole-3-sulfonamide ;
le N-[5-bromo-4-méthoxy-6-(2-méthoxyéthoxy)pyrimidin-2-yl]-6-chloro-1H-indole-3-sulfonamide ;
le N-[5-bromo-4-(cyclopropoxy)-6-méthoxy-pyrimidin-2-yl]-6-chloro-1H-indole-3-sulfonamide ;
le 6-chloro-N-[4-(difluorométhoxy)-6-méthoxy-5-méthyl-pyrimidin-2-yl]-1H-indole-3-sulfonamide ;
le N-(5-bromo-4-fluoro-6-méthoxy-pyrimidin-2-yl)-6-chloro-1H-indole-3-sulfonamide ;
le 6-chloro-N-[5-(2,2-difluoroéthoxy)-4-fluoro-6-méthoxy-pyrimidin-2-yl]-1H-indole-3-sulfonamide ;
le 6-chloro-N-(4-chloro-5,6-diméthoxy-pyrimidin-2-yl)-1H-indole-3-sulfonamide ;
le 6-chloro-N-[4-chloro-5-(2,2-difluoroéthoxy)pyrimidin-2-yl]-1H-indole-3-sulfonamide ;
le 6-chloro-N-[5-(2,2-difluoroéthoxy)-4-éthyl-6-méthoxy-pyrimidin-2-yl]-1H-indole-3-sulfonamide ;
le 6-chloro-N-[5-(2,2-difluoroéthyl)-4-méthoxy-6-méthyl-pyrimidin-2-yl]-1H-indole-3-sulfonamide ;
le 6-chloro-N-[4-cyclopropyl-5-(2,2-difluoroéthyl)-6-méthoxy-pyrimidin-2-yl]-1H-indole-3-sulfonamide ;
le N-[5-bromo-4-(cyclopropylméthyl)-6-méthoxy-pyrimidin-2-yl]-6-chloro-1H-indole-3-sulfonamide ;
le N-[5-bromo-4-méthoxy-6-(méthoxyméthyl)pyrimidin-2-yl]-6-chloro-1H-indole-3-sulfonamide ;
le N-[5-bromo-4-(1,1-difluoroéthyl)-6-méthoxy-pyrimidin-2-yl]-6-chloro-1H-indole-3-sulfonamide ;
le N-[5-bromo-4-(fluorométhoxy)-6-méthoxy-pyrimidin-2-yl]-6-chloro-1H-indole-3-sulfonamide ;
le N-[5-bromo-4-(difluorométhyl)-6-méthoxy-pyrimidin-2-yl]-6-chloro-1H-indole-3-sulfonamide ;
le N-[5-bromo-4-(cyanométhyl)-6-méthoxy-pyrimidin-2-yl]-6-chloro-1H-indole-3-sulfonamide ;
le 6-chloro-N-[5-(difluorométhoxy)-4-méthoxy-6-méthylsulfanyl-pyrimidin-2-yl]-1H-indole-3-sulfonamide ;
le N-[4,6-diméthoxy-5-(1,1,2-trifluoroéthoxy)pyrimidin-2-yl]-6-fluoro-1H-indole-3-sulfonamide ;
le 6-bromo-N-[5-(2,2-difluoroéthyl)-4-méthoxy-pyrimidin-2-yl]-1H-indole-3-sulfonamide ;
le 6-bromo-N-[5-(2-cyanoéthyl)-4-méthoxy-pyrimidin-2-yl]-1H-indole-3-sulfonamide ;
le 6-bromo-N-[5-(difluorométhoxy)-4,6-diméthoxy-pyrimidin-2-yl]-1H-indole-3-sulfonamide ;
le 6-bromo-N-[5-(2-fluoroéthoxy)-4,6-diméthoxy-pyrimidin-2-yl]-1H-indole-3-sulfonamide ;
le 6-bromo-N-[5-(2,2-difluoroéthoxy)-4,6-diméthoxy-pyrimidin-2-yl]-1H-indole-3-sulfonamide ;
le 6-bromo-N-[5-(2-cyanoéthyl)-4,6-diméthoxy-pyrimidin-2-yl]-1H-indole-3-sulfonamide ;
le N-[5-(difluorométhoxy)-4,6-diméthoxy-pyrimidin-2-yl]-6-(difluorométhyl)-1H-pyrrolo[2,3-b]pyridine-3-sulfonamide ;
le 6-(difluorométhyl)-N-[5-(2-fluoroéthoxy)-4,6-diméthoxy-pyrimidin-2-yl]-1H-pyrrolo[2,3-b]pyridine-3-sulfonamide ;
le N-[5-(2,2-difluoroéthoxy)-4,6-diméthoxy-pyrimidin-2-yl]-6-(difluorométhyl)-1H-pyrrolo[2,3-b]pyridine-3-sulfonamide ;
le 6-(difluorométhyl)-N-[4,6-diméthoxy-5-(1,1,2-trifluoroéthoxy)pyrimidin-2-yl]-1H-pyrrolo[2,3-b]pyridine-3-sulfonamide ;
le 6-(difluorométhyl)-N-[5-(3,3-difluoropropyl)-4,6-diméthoxy-pyrimidin-2-yl]-1H-pyrrolo[2,3-b]pyridine-3-sulfonamide ;
le N-[5-(cyanométhoxy)-4,6-diméthoxy-pyrimidin-2-yl]-6-(difluorométhyl)-1H-pyrrolo[2,3-b]pyridine-3-sulfonamide ;
le 6-(difluorométhyl)-N-[4-méthoxy-5-(1,1,2-trifluoroéthoxy)pyrimidin-2-yl]-1H-indole-3-sulfonamide ;
le N-[5-(difluorométhoxy)-4,6-diméthoxy-pyrimidin-2-yl]-6-(difluorométhyl)-1H-indole-3-sulfonamide ;
le N-[5-(2,2-difluoroéthoxy)-4,6-diméthoxy-pyrimidin-2-yl]-6-(difluorométhyl)-1H-indole-3-sulfonamide ;
le N-[5-(2-cyanoéthyl)-4,6-diméthoxy-pyrimidin-2-yl]-6-(difluorométhyl)-1H-indole-3-sulfonamide ;
le N-[5-(2,2-difluoroéthyl)-4-méthoxy-pyrimidin-2-yl]-6-(difluorométhoxy)-1H-indole-3-sulfonamide ;
le 6-chloro-N-[5-(2-cyanoéthyl)-4-méthoxy-pyrimidin-2-yl]-7-(difluorométhylthio)-1H-indole-3-sulfonamide ;
le 6-chloro-7-(difluorométhylthio)-N-[5-(2-fluoroéthoxy)-4,6-diméthoxy-pyrimidin-2-yl]-1H-indole-3-sulfonamide ;
le 6-chloro-N-[5-(2-cyanoéthyl)-4,6-diméthoxy-pyrimidin-2-yl]-7-(difluorométhylthio)-1H-indole-3-sulfonamide ;
le 7-chloro-N-[5-(2,2-difluoroéthyl)-4-méthoxy-pyrimidin-2-yl]-1H-indole-3-sulfonamide ;
le 6-bromo-N-[5-(2-fluoroéthoxy)-4,6-diméthoxy-pyrimidin-2-yl]-1H-pyrrolo[2,3-b]pyridine-3-sulfonamide ;
le 6-bromo-N-[4,6-diméthoxy-5-(1,1,2-trifluoroéthoxy)pyrimidin-2-yl]-1H-pyrrolo[2,3-b]pyridine-3-sulfonamide ;
le 6-bromo-N-[5-(2,2-difluoroéthyl)-4,6-diméthoxy-pyrimidin-2-yl]-1H-pyrrolo[2,3-b]pyridine-3-sulfonamide ;
le 6-bromo-N-[5-(3,3-difluoropropyl)-4,6-diméthoxy-pyrimidin-2-yl]-1H-pyrrolo[2,3-b]pyridine-3-sulfonamide ;
le 6-bromo-N-[5-(2-cyanoéthyl)-4,6-diméthoxy-pyrimidin-2-yl]-1H-pyrrolo[2,3-b]pyridine-3-sulfonamide ;
le 6-bromo-N-[5-(cyanométhoxy)-4,6-diméthoxy-pyrimidin-2-yl]-1H-pyrrolo[2,3-b]pyridine-3-sulfonamide ;
le 6-chloro-N-[5-(2,2-difluoroéthyl)-4,6-diméthoxy-pyrimidin-2-yl]-7-(diméthylamino)-1H-indole-3-sulfonamide ;
le N-[5-(difluorométhoxy)-4,6-diméthoxy-pyrimidin-2-yl]-1H-pyrrolo[3,2-h]quinoléine-3-sulfonamide ;
le N-[5-(2-fluoroéthoxy)-4,6-diméthoxy-pyrimidin-2-yl]-1H-pyrrolo[3,2-h]quinoléine-3-sulfonamide ;
le 6-chloro-N-(4,6-diméthoxy-5-méthyl-pyrimidin-2-yl)-1H-pyrrolo[2,3-b]pyridine-3-sulfonamide ;
le 6-chloro-N-[5-(difluorométhoxy)-4-méthoxy-pyrimidin-2-yl]-1H-pyrrolo[2,3-b]pyridine-3-sulfonamide ;
le 6-chloro-N-[5-(2,2-difluoroéthyl)-4,6-diméthoxy-pyrimidin-2-yl]-1H-pyrrolo[2,3-b]pyridine-3-sulfonamide ;
le 6-chloro-N-[5-(3,3-difluoropropyl)-4,6-diméthoxy-pyrimidin-2-yl]-1H-pyrrolo[2,3-b]pyridine-3-sulfonamide ;
le 6-chloro-N-[5-(2-cyanoéthyl)-4,6-diméthoxy-pyrimidin-2-yl]-1H-pyrrolo[2,3-b]pyridine-3-sulfonamide ;
le 6-chloro-N-[5-(2,2-difluoroéthoxy)-4-méthoxy-pyrimidin-2-yl]-7-fluoro-1H-indole-3-sulfonamide ;
le 6-chloro-N-[5-(2,2-difluoroéthyl)-4-méthoxy-pyrimidin-2-yl]-7-fluoro-1H-indole-3-sulfonamide ;
le 6-chloro-N-[5-(difluorométhoxy)-4,6-diméthoxy-pyrimidin-2-yl]-7-fluoro-1H-indole-3-sulfonamide ;
le 6-chloro-N-[5-(difluorométhoxy)-4-méthoxy-pyrimidin-2-yl]-7-fluoro-1H-indole-3-sulfonamide ;
le 6-chloro-7-(cyclopropoxy)-N-[5-(2,2-difluoroéthyl)-4-méthoxy-pyrimidin-2-yl]-1H-indole-3-sulfonamide ;
le 7-bromo-N-[5-(2,2-difluoroéthyl)-4-méthoxy-pyrimidin-2-yl]-6-fluoro-1H-indole-3-sulfonamide ;
le 7-bromo-N-[5-(2,2-difluoroéthyl)-4-méthoxy-pyrimidin-2-yl]-1H-indole-3-sulfonamide ;
le 7-chloro-N-[5-(2,2-difluoroéthyl)-4-méthoxy-pyrimidin-2-yl]-6-fluoro-1H-indole-3-sulfonamide ;
le N-[5-(2-fluoroéthoxy)-4,6-diméthoxy-pyrimidin-2-yl]-6-méthyl-1H-pyrrolo[2,3-b]pyridine-3-sulfonamide ;
le N-[5-(2,2-difluoroéthyl)-4,6-diméthoxy-pyrimidin-2-yl]-6-méthyl-1H-pyrrolo[2,3-b]pyridine-3-sulfonamide ;
le 6-chloro-N-[5-(2,2-difluoroéthyl)-4,6-diméthoxy-pyrimidin-2-yl]-7-morpholino-1H-indole-3-sulfonamide ;
le 7-bromo-N-[5-(2,2-difluoroéthyl)-4,6-diméthoxy-pyrimidin-2-yl]-1H-indole-3-sulfonamide ;
le 6-chloro-N-[5-(2,2-difluoroéthoxy)-4-méthoxy-pyrimidin-2-yl]-1H-indole-3-sulfonamide ;
le 6-chloro-N-[5-(1,1-difluoroéthyl)-4-méthoxy-pyrimidin-2-yl]-1H-indole-3-sulfonamide ;
le 6-chloro-N-(4-éthyl-6-méthoxy-5-méthyl-pyrimidin-2-yl)-1H-indole-3-sulfonamide ;
le N-(5-bromo-4-méthoxy-6-tétrahydropyran-4-yl-pyrimidin-2-yl)-6-chloro-1H-indole-3-sulfonamide ;
le 6-chloro-N-[5-(2,2-difluoroéthyl)-4-méthoxy-pyrimidin-2-yl]thiéno[2,3-b]pyridine-3-sulfonamide ;
le N-[5-(2,2-difluoroéthyl)-4-méthoxy-pyrimidin-2-yl]-6-fluoro-1H-indole-3-sulfonamide ;
le 7-bromo-6-chloro-N-[5-(2-fluoroéthoxy)-4-méthoxy-pyrimidin-2-yl]-1H-indole-3-sulfonamide ;
le 7-bromo-6-chloro-N-[5-(2,2-difluoroéthyl)-4-méthoxy-pyrimidin-2-yl]-1H-indole-3-sulfonamide ;
le 6-bromo-N-[5-(2,2-difluoroéthoxy)-4-méthoxy-pyrimidin-2-yl]-1H-indole-3-sulfonamide ;
le 6-bromo-N-(4,6-diméthoxy-5-méthyl-pyrimidin-2-yl)thiéno[2,3-b]pyridine-3-sulfonamide ;
le 6-bromo-N-[5-(2,2-difluoroéthoxy)-4-méthoxy-pyrimidin-2-yl]thiéno[2,3-b]pyridine-3-sulfonamide ;
le 6-bromo-N-[5-(2-fluoroéthoxy)-4-méthoxy-pyrimidin-2-yl]thiéno[2,3-b]pyridine-3-sulfonamide ;
le 6-bromo-N-[4-méthoxy-5-(1,1,2-trifluoroéthoxy)pyrimidin-2-yl]thiéno[2,3-b]pyridine-3-sulfonamide ;
le 6-bromo-N-[5-(2,2-difluoroéthyl)-4-méthoxy-pyrimidin-2-yl]thiéno[2,3-b]pyridine-3-sulfonamide ;
le 6-bromo-N-[5-(2-cyanoéthyl)-4-méthoxy-pyrimidin-2-yl]thiéno[2,3-b]pyridine-3-sulfonamide ;
le 6-bromo-N-[5-(2,2-difluoroéthyl)-4,6-diméthoxy-pyrimidin-2-yl]thiéno[2,3-b]pyridine-3-sulfonamide ;
le 6-bromo-N-[5-(2-cyanoéthyl)-4,6-diméthoxy-pyrimidin-2-yl]thiéno[2,3-b]pyridine-3-sulfonamide ;
le N-[5-(2,2-difluoroéthyl)-4-méthoxy-pyrimidin-2-yl]-6-éthyl-1H-indole-3-sulfonamide ;
le N-[5-(2,2-difluoroéthyl)-4-méthoxypyrimidin-2-yl]-6-propyl-1H-indole-3-sulfonamide ;
le 6-chloro-N-[5-(2,2-difluoroéthyl)-4-méthoxy-pyrimidin-2-yl]benzothiophène-3-sulfonamide ;
le 6-chloro-N-[5-(3,3-difluoropropyl)-4-méthoxy-pyrimidin-2-yl]benzothiophène-3-sulfonamide ;
le 6-chloro-N-[5-(2-cyanoéthyl)-4-méthoxy-pyrimidin-2-yl]benzothiophène-3-sulfonamide ;
le 6-bromo-N-[5-(2,2-difluoroéthoxy)-4-méthoxy-pyrimidin-2-yl]benzothiophène-3-sulfonamide ;
le 6-bromo-N-[5-(2-fluoroéthoxy)-4-méthoxy-pyrimidin-2-yl]benzothiophène-3-sulfonamide ;
le N-[5-(2-fluoroéthoxy)-4-méthoxy-pyrimidin-2-yl]-6-méthyl-benzothiophène-3-sulfonamide ;
le 6-bromo-N-(4,6-diméthoxy-5-méthyl-pyrimidin-2-yl)-1H-pyrrolo[2,3-b]pyridine-3-sulfonamide ;
le 6-bromo-N-[5-(2,2-difluoroéthoxy)-4-méthoxy-pyrimidin-2-yl]-1H-pyrrolo[2,3-b]pyridine-3-sulfonamide ;
le 6-chloro-N-[5-(2,2-difluoroéthyl)-4-méthoxy-pyrimidin-2-yl]-7-fluoro-benzothiophène-3-sulfonamide ;
le 6-chloro-N-[5-(2-cyanoéthyl)-4-méthoxy-pyrimidin-2-yl]-7-fluoro-benzothiophène-3-sulfonamide ;
le N-[5-(2,2-difluoroéthyl)-4-méthoxy-pyrimidin-2-yl]-6,7-difluoro-1H-indole-3-sulfonamide ;
le 6-(difluorométhyl)-N-[5-(2-fluoroéthoxy)-4-méthoxy-pyrimidin-2-yl]thiéno[2,3-b]pyridine-3-sulfonamide ;
le N-[5-(2,2-difluoroéthyl)-4-méthoxy-pyrimidin-2-yl]-6-(difluorométhyl)thiéno[2,3-b]pyridine-3-sulfonamide ;
le N-[5-(2,2-difluoroéthyl)-4,6-diméthoxy-pyrimidin-2-yl]-6-(difluorométhyl)thiéno[2,3-b]pyridine-3-sulfonamide ;
le N-[5-(2,2-difluoroéthoxy)-4,6-diméthoxy-pyrimidin-2-yl]-1H-pyrrolo[3,2-h]quinoléine-3-sulfonamide ;
le N-[5-(2,2-difluoroéthyl)-4,6-diméthoxy-pyrimidin-2-yl]-1H-pyrrolo[3,2-h]quinoléine-3-sulfonamide ;
le 6-chloro-N-[5-(2,2-difluoroéthoxy)-4-méthoxy-pyrimidin-2-yl]-1H-pyrrolo[2,3-b]pyridine-3-sulfonamide ;
le 6-chloro-N-[4,6-diméthoxy-5-(1,1,2-trifluoroéthoxy)pyrimidin-2-yl]-1H-pyrrolo[2,3-b]pyridine-3-sulfonamide ;
le 6-bromo-N-[5-(difluorométhoxy)-4,6-diméthoxy-pyrimidin-2-yl]-1H-pyrrolo[2,3-b]pyridine-3-sulfonamide ;
le 6-chloro-N-(4-chloro-5-méthoxy-pyrimidin-2-yl)-1H-indole-3-sulfonamide ;
le 6-chloro-N-[5-(3-fluoropropoxy)-4-méthoxy-pyrimidin-2-yl]-1H-indole-3-sulfonamide ;
le N-(5-but-2-ynoxy-4-méthoxy-pyrimidin-2-yl)-6-chloro-1H-indole-3-sulfonamide ;
le 6-chloro-N-[5-(2,3-difluoropropyl)-4-méthoxy-pyrimidin-2-yl]-1H-indole-3-sulfonamide ;
le 6-chloro-N-[4-méthoxy-5-(2,2,2-trifluoroéthyl)pyrimidin-2-yl]-1H-indole-3-sulfonamide ;
le 6-chloro-N-[4-méthoxy-5-(3,3,3-trifluoropropyl)pyrimidin-2-yl]-1H-indole-3-sulfonamide ;
le 6-chloro-N-[5-(2-cyanoéthyl)-4-méthoxy-pyrimidin-2-yl]-1H-indole-3-sulfonamide ;
le 6-chloro-N-[4-méthoxy-5-[2-(oxétan-3-yl)éthynyl]pyrimidin-2-yl]-1H-indole-3-sulfonamide ;
le 6-chloro-N-[4-méthoxy-5-[2-(oxétan-3-yl)éthyl]pyrimidin-2-yl]-1H-indole-3-sulfonamide ;
le N-[5-(2,2-difluoroéthyl)-4-méthoxy-pyrimidin-2-yl]-6-méthyl-1H-indole-3-sulfonamide ;
le 7-bromo-6-chloro-N-[5-(3,3-difluoropropyl)-4-méthoxy-pyrimidin-2-yl]-1H-indole-3-sulfonamide ;
le 6-bromo-N-[5-(2,2-difluoroéthyl)-4-méthoxy-pyrimidin-2-yl]-7-méthyl-1H-indole-3-sulfonamide ;
le 6-chloro-N-[5-(2,2-difluoroéthyl)-4-méthoxy-pyrimidin-2-yl]-7-méthoxy-1H-indole-3-sulfonamide ;
le 6-chloro-N-[5-(3,3-difluoropropyl)-4-méthoxy-pyrimidin-2-yl]-7-méthoxy-1H-indole-3-sulfonamide ;
le 6-chloro-N-[5-(2,2-difluoroéthyl)-4-méthoxy-pyrimidin-2-yl]-7-(difluorométhoxy)-1H-indole-3-sulfonamide ;
le 6-bromo-N-[5-(difluorométhoxy)-4-méthoxy-pyrimidin-2-yl]-1H-indole-3-sulfonamide ;
le N-[5-(2,2-difluoroéthyl)-4-méthoxy-pyrimidin-2-yl]-6-(trifluorométhyl)-1H-indole-3-sulfonamide ;
le 6-bromo-N-[5-(2,2-difluoroéthyl)-4-méthoxy-pyrimidin-2-yl]-7-fluoro- 1H-indole-3-sulfonamide ;
le 6-chloro-N-[5-(2,2-difluoroéthyl)-4-méthoxy-pyrimidin-2-yl]-7-méthyl-1H-indole-3-sulfonamide ;
le N-[5-(2,2-difluoroéthyl)-4-méthoxy-pyrimidin-2-yl]-6-(méthylthio)-1H-indole-3-sulfonamide ;
le N-[5-(2,2-difluoroéthyl)-4,6-diméthoxy-pyrimidin-2-yl]-6-(difluorométhyl)-1H-pyrrolo[2,3-b]pyridine-3-sulfonamide ;
le N-[5-(2,2-difluoroéthyl)-4-méthoxy-pyrimidin-2-yl]-6-(difluorométhyl)-1H-indole-3-sulfonamide ;
le 6-bromo-N-[5-(2,2-difluoroéthy1)-4-méthoxy-pyrimidin-2-y1]-7-méthoxy-1H-indole-3-sulfonamide ;
le 6-(cyanométhyl)-N-[5-(2,2-difluoroéthyl)-4-méthoxy-pyrimidin-2-yl]-1H-indole-3-sulfonamide ;
le 6-chloro-N-[5-(2,2-difluoroéthyl)-4-méthoxy-pyrimidin-2-yl]-7-(fluorométhoxy)-1H-indole-3-sulfonamide ;
le 6-chloro-N-[5-(2,2-difluoroéthyl)-4-méthoxy-pyrimidin-2-yl]-7-(difluorométhylsulfanyl)-1H-indole-3-sulfonamide ;
le 6-bromo-N-[5-(2,2-difluoroéthyl)-4-méthoxy-pyrimidin-2-yl]-1H-pyrrolo[2,3-b]pyridine-3-sulfonamide ;
le 6-bromo-N-[5-(3,3-difluoropropyl)-4-méthoxy-pyrimidin-2-yl]-1H-pyrrolo[2,3-b]pyridine-3-sulfonamide ;
le 6-bromo-N-[5-(2,2-difluoroéthoxy)-4,6-diméthoxy-pyrimidin-2-yl]- 1H-pyrrolo[2,3-b]pyridine-3-sulfonamide ;
le 6-chloro-N-[5-(2,2-difluoroéthyl)-4-méthoxy-pyrimidin-2-yl]-1H-pyrrolo[2,3-b]pyridine-3-sulfonamide ;
le 6-chloro-N-[5-(3,3-difluoropropyl)-4-méthoxy-pyrimidin-2-yl]-1H-pyrrolo[2,3-b]pyridine-3-sulfonamide ;
le 6-chloro-N-[5-(difluorométhoxy)-4,6-diméthoxy-pyrimidin-2-yl]-1H-pyrrolo[2,3-b]pyridine-3-sulfonamide ;
le 6-chloro-N-[5-(2,2-difluoroéthoxy)-4,6-diméthoxy-pyrimidin-2-yl]-1H-pyrrolo[2,3-b]pyridine-3-sulfonamide ;
le 6-chloro-N-[5-(3,3-difluoropropyl)-4-méthoxy-pyrimidin-2-yl]-7-fluoro-1H-indole-3-sulfonamide ;
le 6-chloro-N-[5-(2,2-difluoroéthoxy)-4,6-diméthoxy-pyrimidin-2-yl]-7-fluoro-1H-indole-3-sulfonamide;
le 6-chloro-7-(difluorométhyl)-N-[5-(3,3-difluoropropyl)-4-méthoxy-pyrimidin-2-yl]-1H-indole-3-sulfonamide ;
le 6,7-dichloro-N-[5-(2,2-difluoroéthyl)-4-méthoxy-pyrimidin-2-yl]-1H-indole-3-sulfonamide ;
le 6,7-dichloro-N-[5-(3,3-difluoropropyl)-4-méthoxy-pyrimidin-2-yl]-1H-indole-3-sulfonamide ;
le 6-chloro-7-(cyclopropoxy)-N-[5-(3,3-difluoropropyl)-4-méthoxy-pyrimidin-2-yl]-1H-indole-3-sulfonamide ;
le 6-bromo-7-fluoro-N-[5-(2-fluoroéthoxy)-4-méthoxy-pyrimidin-2-yl]-1H-indole-3-sulfonamide ;
le 6-chloro-N-[5-(2,2-difluoroéthyl)-4-méthoxy-pyrimidin-2-yl]-7-(difluorométhyl)-1H-indole-3-sulfonamide ;
le 6-chloro-7-fluoro-N-[4-méthoxy-5-(1,1,2-trifluoroéthoxy)pyrimidin-2-yl]-1H-indole-3-sulfonamide ;
le 6-chloro-N-[5-(2-cyanoéthyl)-4-méthoxy-pyrimidin-2-yl]-7-fluoro-1H-indole-3-sulfonamide ;
le 6-chloro-N-[5-(2,2-difluoroéthyl)-4,6-diméthoxy-pyrimidin-2-yl]-7-fluoro-1H-indole-3-sulfonamide ;
le 6-chloro-N-[5-(2-cyanoéthyl)-4,6-diméthoxy-pyrimidin-2-yl]-7-fluoro-1H-indole-3-sulfonamide ;
le 6-chloro-N-[5-(cyanométhoxy)-4,6-diméthoxy-pyrimidin-2-yl]-7-fluoro-1H-indole-3-sulfonamide ;
le 6-chloro-N-[4-méthoxy-5-(1,1,2-trifluoroéthoxy)pyrimidin-2-yl]-1H-pyrrolo[2,3-b]pyridine-3-sulfonamide ;
le 6-chloro-N-[5-(2-fluoroéthoxy)-4,6-diméthoxy-pyrimidin-2-yl]-1H-pyrrolo[2,3-b]pyridine-3-sulfonamide ;
le N-[5-(2,2-difluoroéthyl)-4-méthoxy-pyrimidin-2-yl]-1H-pyrrolo[3,2-h]quinoléine-3-sulfonamide ;
le 6-chloro-7-cyano-N-[5-(2,2-difluoroéthyl)-4-méthoxy-pyrimidin-2-yl]-1H-indole-3-sulfonamide ;
le 6-chloro-7-cyano-N-[5-(difluorométhoxy)-4,6-diméthoxy-pyrimidin-2-yl]-1H-indole-3-sulfonamide ;
le 6-chloro-7-cyano-N-[5-(2-fluoroéthoxy)-4,6-diméthoxy-pyrimidin-2-yl]-1H-indole-3-sulfonamide ;
le 6-chloro-7-cyano-N-[5-(2,2-difluoroéthoxy)-4,6-diméthoxy-pyrimidin-2-yl]-1H-indole-3-sulfonamide;
le 6-chloro-7-cyano-N-[5-(2,2-difluoroéthyl)-4,6-diméthoxy-pyrimidin-2-yl]-1H-indole-3-sulfonamide ;
le N-[5-(2,2-difluoroéthyl)-4-méthoxy-pyrimidin-2-yl]-6,8-dihydro-1H-fur[3,4-g]indole-3-sulfonamide ;
le N-[5-(2,2-difluoroéthyl)-4,6-diméthoxy-pyrimidin-2-yl]-6,8-dihydro-1H-fur[3,4-g]indole-3-sulfonamide ;
le 6-bromo-N-[5-(2-fluoroéthoxy)-4-méthoxy-pyrimidin-2-yl]-1H-pyrrolo[2,3-b]pyridine-3-sulfonamide ;
le 6-bromo-N-[4-méthoxy-5-(1,1,2-trifluoroéthoxy)pyrimidin-2-yl]-1H-pyrrolo[2,3-b]pyridine-3-sulfonamide ;
le 6-chloro-7-(cyanométhyl)-N-[5-(2,2-difluoroéthyl)-4-méthoxy-pyrimidin-2-yl]-1H-indole-3-sulfonamide;
le 6-chloro-7-cyclopropyl-N-[5-(2,2-difluoroéthyl)-4-méthoxy-pyrimidin-2-yl]-1H-indole-3-sulfonamide;
le 6-chloro-N-[5-(2,2-difluoroéthyl)-4-méthoxy-pyrimidin-2-yl]-7-(méthylthio)-1H-indole-3-sulfonamide;
le 6-(difluorométhyl)-N-[5-(2-fluoroéthoxy)-4-méthoxy-pyrimidin-2-yl]-1H-indole-3-sulfonamide ;
le N-[5-(2,2-difluoroéthyl)-4,6-diméthoxy-pyrimidin-2-yl]-6-(difluorométhyl)-1H-indole-3-sulfonamide ;
le N-[5-(cyanométhoxy)-4,6-diméthoxy-pyrimidin-2-yl]-6-(difluorométhyl)-1H-indole-3-sulfonamide ;
le 6-(difluorométhyl)-N-[4-méthoxy-5-(1,1,2-trifluoroéthoxy)pyrimidin-2-yl]-1H-pyrrolo[2,3-b]pyridine-3-sulfonamide ;
le N-[5-(2-cyanoéthyl)-4,6-diméthoxy-pyrimidin-2-yl]-6-(difluorométhyl)-1H-pyrrolo[2,3-b]pyridine-3-sulfonamide ;
le 6-bromo-N-[5-(2-cyanoéthyl)-4-méthoxy-pyrimidin-2-yl]-7-fluoro-1H-indole-3-sulfonamide ;
le 6-bromo-N-[5-(difluorométhoxy)-4,6-diméthoxy-pyrimidin-2-yl]-7-fluoro-1H-indole-3-sulfonamide ;
le 6-bromo-N-[5-(2,2-difluoroéthoxy)-4,6-diméthoxy-pyrimidin-2-yl]-7-fluoro-1H-indole-3-sulfonamide;
le 6-bromo-N-[5-(2-cyanoéthyl)-4,6-diméthoxy-pyrimidin-2-yl]-7-fluoro-1H-indole-3-sulfonamide ;
le 6-bromo-N-[5-(cyanométhoxy)-4,6-diméthoxy-pyrimidin-2-yl]-7-fluoro-1H-indole-3-sulfonamide ;
le 6-bromo-N-[5-(2,2-difluoroéthyl)-4,6-diméthoxy-pyrimidin-2-yl]-1H-indole-3-sulfonamide ;
le 6-bromo-N-[5-(cyanométhoxy)-4,6-diméthoxy-pyrimidin-2-yl]-1H-indole-3-sulfonamide ;
le 6-(difluorométhyl)-7-fluoro-N-[5-(2-fluoroéthoxy)-4-méthoxy-pyrimidin-2-yl]-1H-indole-3-sulfonamide;
le N-[5-(2,2-difluoroéthyl)-4-méthoxy-pyrimidin-2-yl]-6-(difluorométhyl)-7-fluoro-1H-indole-3-sulfonamide ;
le N-[5-(difluorométhoxy)-4,6-diméthoxy-pyrimidin-2-yl]-6-(difluorométhyl)-7-fluoro-1H-indole-3-sulfonamide ;
le N-[5-(2,2-difluoroéthoxy)-4,6-diméthoxy-pyrimidin-2-yl]-6-(difluorométhyl)-7-fluoro-1H-indole-3-sulfonamide ;
le N-[5-(2,2-difluoroéthyl)-4,6-diméthoxy-pyrimidin-2-yl]-6-(difluorométhyl)-7-fluoro-1H-indole-3-sulfonamide ;
le N-[5-(2-cyanoéthyl)-4,6-diméthoxy-pyrimidin-2-yl]-6-(difluorométhyl)-7-fluoro-1H-indole-3-sulfonamide ;
le N-[5-(cyanométhoxy)-4,6-diméthoxy-pyrimidin-2-yl]-6-(difluorométhyl)-7-fluoro-1H-indole-3-sulfonamide ;
le 6-cyclopropyl-N-[5-(2,2-difluoroéthyl)-4-méthoxy-pyrimidin-2-yl]-1H-indole-3-sulfonamide ;
le 7-bromo-6-chloro-N-[5-(2-cyanoéthyl)-4-méthoxy-pyrimidin-2-yl]-1H-indole-3-sulfonamide ;
le N-[5-(difluorométhoxy)-4,6-diméthoxy-pyrimidin-2-yl]-6-fluoro-1H-indole-3-sulfonamide ;
le N-[5-(2,2-difluoroéthoxy)-4,6-diméthoxy-pyrimidin-2-yl]-6-fluoro-1H-indole-3-sulfonamide ;
le N-[5-(2,2-difluoroéthyl)-4,6-diméthoxy-pyrimidin-2-yl]-6-fluoro-1H-indole-3-sulfonamide ;
le N-[5-(2-cyanoéthyl)-4,6-diméthoxy-pyrimidin-2-yl]-6-fluoro-1H-indole-3-sulfonamide ;
le 6-chloro-N-[5-(2,2-difluoropropyl)-4-méthoxy-pyrimidin-2-yl]-1H-indole-3-sulfonamide ;
le 6-chloro-N-[4-méthoxy-5-(2,2,3,3-tétrafluoropropyl)pyrimidin-2-yl]-1H-indole-3-sulfonamide ;
le 6-chloro-N-[5-(difluorométhoxyméthyl)-4-méthoxy-pyrimidin-2-yl]-1H-indole-3-sulfonamide ;
le 6-chloro-N-[4,6-diméthoxy-5-(oxétan-3-ylméthoxy)pyrimidin-2-yl]-1H-indole-3-sulfonamide ;
le 6-chloro-N-[5-(2,3-difluoropropyl)-4,6-diméthoxy-pyrimidin-2-yl]-1H-indole-3-sulfonamide ;
le 6-chloro-N-[5-(cyanométhoxy)-4,6-diméthoxy-pyrimidin-2-yl]-1H-indole-3-sulfonamide ;
le 6-chloro-N-[4-méthoxy-5-(2,3,3-trifluoropropyl)pyrimidin-2-yl]-1H-indole-3-sulfonamide ;
le 6-chloro-N-[5-(cyanométhoxy)-4-méthoxy-pyrimidin-2-yl]-1H-indole-3-sulfonamide ;
le 6-bromo-N-[5-(difluorométhoxy)-4-méthoxy-pyrimidin-2-yl]benzothiophène-3-sulfonamide ;
le 6-bromo-N-[5-(difluorométhoxy)-4,6-diméthoxy-pyrimidin-2-yl]thiéno[2,3-b]pyridine-3-sulfonamide ;
le 6-bromo-N-[5-(3,3-difluoropropyl)-4-méthoxy-pyrimidin-2-yl]thiéno[2,3-b]pyridine-3-sulfonamide ;
le 6-chloro-N-[5-(difluorométhoxy)-4,6-diméthoxy-pyrimidin-2-yl]thiéno[2,3-b]pyridine-3-sulfonamide ;
le N-[5-(2,2-difluoroéthyl)-4-méthoxy-pyrimidin-2-yl]-6-(oxétan-3-yl)-1H-indole-3-sulfonamide ;
le N-(4,6-diméthoxy-5-méthyl-pyrimidin-2-yl)-6-méthoxy-1H-indole-3-sulfonamide ;
le N-[5-(2,2-difluoroéthyl)-4-méthoxy-pyrimidin-2-yl]-6-(1-hydroxy-1-méthyl-éthyl)-1H-indole-3-sulfonamide ;
le 6-chloro-N-[5-(1,1-difluoro-2-hydroxy-éthoxy)-4,6-diméthoxy-pyrimidin-2-yl]-1H-indole-3-sulfonamide;
le 6-chloro-N-[5-(2,2-difluoroéthyl)-4-méthoxy-pyrimidin-2-yl]-7-méthylsulfinyl-1H-indole-3-sulfonamide;
le 6-chloro-N-[5-(2,2-difluoroéthyl)-4-méthoxy-pyrimidin-2-yl]-7-mésyl-1H-indole-3-sulfonamide ;
le 6-chloro-N-[5-(2-fluoroéthoxy)-4,6-diméthoxy-pyrimidin-2-yl]-7-méthylsulfinyl-1H-indole-3-sulfonamide ;
le 6-chloro-N-[5-(2,2-difluoroéthyl)-4,6-diméthoxy-pyrimidin-2-yl]-7-méthylsulfinyl-1H-indole-3-sulfonamide ;
le 6-chloro-7-cyano-N-[5-(2-fluoroéthoxy)-4-méthoxy-pyrimidin-2-yl]-1H-indole-3-sulfonamide ;
le 6-chloro-N-[4,6-diméthoxy-5-(2-méthylsulfonyléthyl)pyrimidin-2-yl]-1H-indole-3-sulfonamide ;
le N-[5-(2,2-difluoroéthyl)-4-méthoxy-pyrimidin-2-yl]-1H-pyrrolo[2,3-e][1,2]benzothiazole-3-sulfonamide ;
le 7-bromo-6-chloro-N-[5-(2,2-difluoroéthyl)-4,6-diméthoxy-pyrimidin-2-yl]-1H-indole-3-sulfonamide ;
le N-[5-(cyanométhoxy)-4,6-diméthoxy-pyrimidin-2-yl]-6-méthyl-1H-pyrrolo[2,3-b]pyridine-3-sulfonamide ;
le N-[5-(2,2-difluoroéthoxy)-4,6-diméthoxy-pyrimidin-2-yl]-6-méthyl-1H-pyrrolo[2,3-b]pyridine-3-sulfonamide ;
le N-[5-(difluorométhoxy)-4,6-diméthoxy-pyrimidin-2-yl]-6-méthyl-1H-pyrrolo[2,3-b]pyridine-3-sulfonamide ;
le 6-(difluorométhyl)-N-[5-(2,3-difluoropropyl)-4,6-diméthoxy-pyrimidin-2-yl]-1H-pyrrolo[2,3-b]pyridine-3-sulfonamide ;
le 6-bromo-N-[5-(2,3-difluoropropyl)-4,6-diméthoxy-pyrimidin-2-yl]-1H-pyrrolo[2,3-b]pyridine-3-sulfonamide ;
le 6-chloro-N-[5-(2,3-difluoropropyl)-4,6-diméthoxy-pyrimidin-2-yl]-1H-pyrrolo[2,3-b]pyridine-3-sulfonamide ;
le N-[5-(2,2-difluoroéthyl)-4-méthoxy-pyrimidin-2-yl]-8H-pyrrolo[2,3-e][1,3]benzothiazole-6-sulfonamide ;
l'ester méthylique de l'acide 3-[[5-(2,2-difluoroéthyl)-4-méthoxy-pyrimidin-2-yl]sulfamoyl]-6-méthyl-1H-indole-7-carboxylique ;
le N-[5-(2,2-difluoroéthyl)-4-méthoxy-pyrimidin-2-yl]-6-méthyl-7-méthylol-1H-indole-3-sulfonamide ;
le N-[5-(2-fluoroéthoxy)-4-méthoxy-pyrimidin-2-yl]-9-céto-8-méthyl-6,7-dihydro-1H-pyrrol[3,2-h]isoquinoléine-3-sulfonamide ;
le 6-chloro-7-(cyanométhoxy)-N-[5-(2,2-difluoroéthyl)-4,6-diméthoxy-pyrimidin-2-yl]-1H-indole-3-sulfonamide ;
et des sels pharmaceutiquement acceptables de ceux-ci.

12. Composé selon l'une quelconque des revendications 1 à 11, choisi parmi
le 6-chloro-7-fluoro-N-[5-(2-fluoroéthoxy)-4-méthoxy-pyrimidin-2-yl]-1H-indole-3-sulfonamide ;
le 6,7-dichloro-N-[5-(2-fluoroéthoxy)-4-méthoxy-pyrimidin-2-yl]-1H-indole-3-sulfonamide ;
le 6-chloro-7-(difluorométhyl)-N-[5-(2-fluoroéthoxy)-4-méthoxy-pyrimidin-2-yl]-1H-indole-3-sulfonamide ;
le N-(5-bromo-4,6-diméthoxy-pyrimidin-2-y1l)-6-chloro-1H-indole-3-sulfonamide ;
le 6-chloro-N-(5-éthyl-4,6-diméthoxy-pyrimidin-2-yl)-1H-indole-3-sulfonamide ;
le 6-chloro-N-(4,6-diméthoxy-5-méthyl-pyrimidin-2-yl)-1H-indole-3-sulfonamide ;
le 6-chloro-N-[4-cyclopropyl-5-(2,2-difluoroéthoxy)-6-méthoxy-pyrimidin-2-yl]-1H-indole-3-sulfonamide;
le 6-chloro-N-[5-(3-fluoropropyl)-4-méthoxy-pyrimidin-2-yl]-1H-indole-3-sulfonamide ;
le 6-bromo-N-[5-(2-fluoroéthoxy)-4-méthoxy-pyrimidin-2-yl]-1H-indole-3-sulfonamide ;
le 6-bromo-N-(5-bromo-4,6-diméthoxy-pyrimidin-2-yl)-1H-pyrrolo[2,3-b]pyridine-3-sulfonamide ;
le 6-chloro-N-[4-méthoxy-5-(1,1,2-trifluoroéthoxy)pyrimidin-2-yl]-1H-indole-3-sulfonamide ;
le 6-chloro-N-[5-(2,2-difluoroéthyl)-4-méthoxy-pyrimidin-2-yl]-1H-indole-3-sulfonamide ;
le 6-chloro-N-[5-(3,3-difluoropropyl)-4-méthoxy-pyrimidin-2-yl]-1H-indole-3-sulfonamide ;
le 6-chloro-N-[5-(difluorométhoxy)-4,6-diméthoxy-pyrimidin-2-yl]-1H-indole-3-sulfonamide ;
le 6-chloro-N-[5-(2-fluoroéthoxy)-4,6-diméthoxy-pyrimidin-2-yl]-1H-indole-3-sulfonamide ;
le 6-chloro-N-[5-(2,2-difluoroéthoxy)-4,6-diméthoxy-pyrimidin-2-yl]-1H-indole-3-sulfonamide ;
le 6-chloro-N-[4,6-diméthoxy-5-(1,1,2-trifluoroéthoxy)pyrimidin-2-yl]-1H-indole-3-sulfonamide ;
le N-(5-allyl-4,6-diméthoxy-pyrimidin-2-yl)-6-chloro-1H-indole-3-sulfonamide ;
le 6-chloro-N-(4,6-diméthoxy-5-prop-1-ynyl-pyrimidin-2-yl)-1H-indole-3-sulfonamide ;
le 6-chloro-N-(4,6-diméthoxy-5-propyl-pyrimidin-2-yl)-1H-indole-3-sulfonamide ;
le 6-chloro-N-[5-(2-fluoroéthyl)-4,6-diméthoxy-pyrimidin-2-yl]-1H-indole-3-sulfonamide ;
le 6-chloro-N-[5-(2,2-difluoroéthyl)-4,6-diméthoxy-pyrimidin-2-yl]-1H-indole-3-sulfonamide ;
le 6-chloro-N-[5-(3-fluoropropyl)-4,6-diméthoxy-pyrimidin-2-yl]-1H-indole-3-sulfonamide ;
le 6-chloro-N-[5-(3,3-difluoropropyl)-4,6-diméthoxy-pyrimidin-2-yl]-1H-indole-3-sulfonamide ;
le 6-chloro-N-[4,6-diméthoxy-5-(3,3,3-trifluoropropyl)pyrimidin-2-yl]-1H-indole-3-sulfonamide ;
le 6-chloro-N-[4,6-diméthoxy-5-(2-méthoxyéthyl)pyrimidin-2-yl]-1H-indole-3-sulfonamide ;
le 6-chloro-N-[5-(2-cyanoéthyl)-4,6-diméthoxy-pyrimidin-2-yl]-1H-indole-3-sulfonamide ;
le 6-chloro-N-(5-cyclopropyl-4,6-diméthoxy-pyrimidin-2-yl)-1H-indole-3-sulfonamide ;
le 6-chloro-N-[4-(difluorométhoxy)-6-méthoxy-5-méthyl-pyrimidin-2-yl]-1H-indole-3-sulfonamide ;
le 6-bromo-N-[5-(2,2-difluoroéthyl)-4-méthoxy-pyrimidin-2-yl]-1H-indole-3-sulfonamide ;
le 6-bromo-N-[5-(difluorométhoxy)-4,6-diméthoxy-pyrimidin-2-yl]-1H-indole-3-sulfonamide ;
le 6-bromo-N-[5-(2-fluoroéthoxy)-4,6-diméthoxy-pyrimidin-2-yl]-1H-indole-3-sulfonamide ;
le 6-bromo-N-[5-(2,2-difluoroéthoxy)-4,6-diméthoxy-pyrimidin-2-yl]-1H-indole-3-sulfonamide ;
le 6-bromo-N-[5-(2-cyanoéthyl)-4,6-diméthoxy-pyrimidin-2-yl]-1H-indole-3-sulfonamide ;
le N-[5-(2,2-difluoroéthoxy)-4,6-diméthoxy-pyrimidin-2-yl]-6-(difluorométhyl)-1H-pyrrolo[2,3-b]pyridine-3-sulfonamide ;
le 6-(difluorométhyl)-N-[4,6-diméthoxy-5-(1,1,2-trifluoroéthoxy)pyrimidin-2-yl]-1H-pyrrolo[2,3-b]pyridine-3-sulfonamide ;
le 6-(difluorométhyl)-N-[5-(3,3-difluoropropyl)-4,6-diméthoxy-pyrimidin-2-yl]-1H-pyrrolo[2,3-b]pyridine-3-sulfonamide ;
le N-[5-(difluorométhoxy)-4,6-diméthoxy-pyrimidin-2-yl]-6-(difluorométhyl)-1H-indole-3-sulfonamide ;
le N-[5-(2,2-difluoroéthoxy)-4,6-diméthoxy-pyrimidin-2-yl]-6-(difluorométhyl)-1H-indole-3-sulfonamide;
le N-[5-(2-cyanoéthyl)-4,6-diméthoxy-pyrimidin-2-yl]-6-(difluorométhyl)-1H-indole-3-sulfonamide ;
le 6-chloro-N-[5-(2-cyanoéthyl)-4-méthoxy-pyrimidin-2-yl]-7-(difluorométhylthio)-1H-indole-3-sulfonamide ;
le 6-chloro-7-(difluorométhylthio)-N-[5-(2-fluoroéthoxy)-4,6-diméthoxy-pyrimidin-2-yl]-1H-indole-3-sulfonamide ;
le 6-chloro-N-[5-(2-cyanoéthyl)-4,6-diméthoxy-pyrimidin-2-yl]-7-(difluorométhylthio)-1H-indole-3-sulfonamide ;
le 6-bromo-N-[5-(2-fluoroéthoxy)-4,6-diméthoxy-pyrimidin-2-yl]-1H-pyrrolo[2,3-b]pyridine-3-sulfonamide ;
le 6-bromo-N-[4,6-diméthoxy-5-(1,1,2-trifluoroéthoxy)pyrimidin-2-yl]-1H-pyrrolo[2,3-b]pyridine-3-sulfonamide ;
le 6-bromo-N-[5-(2,2-difluoroéthyl)-4,6-diméthoxy-pyrimidin-2-yl]-1H-pyrrolo[2,3-b]pyridine-3-sulfonamide ;
le 6-bromo-N-[5-(3,3-difluoropropyl)-4,6-diméthoxy-pyrimidin-2-yl]-1H-pyrrolo[2,3-b]pyridine-3-sulfonamide ;
le 6-bromo-N-[5-(2-cyanoéthyl)-4,6-diméthoxy-pyrimidin-2-yl]-1H-pyrrolo[2,3-b]pyridine-3-sulfonamide ;
le 6-bromo-N-[5-(cyanométhoxy)-4,6-diméthoxy-pyrimidin-2-yl]-1H-pyrrolo[2,3-b]pyridine-3-sulfonamide ;
le 6-chloro-N-[5-(2,2-difluoroéthyl)-4,6-diméthoxy-pyrimidin-2-yl]-7-(diméthylamino)-1H-indole-3-sulfonamide;
le N-[5-(difluorométhoxy)-4,6-diméthoxy-pyrimidin-2-yl]-1H-pyrrolo[3,2-h]quinoléine-3-sulfonamide ;
le N-[5-(2-fluoroéthoxy)-4,6-diméthoxy-pyrimidin-2-yl]-1H-pyrrolo[3,2-h]quinoléine-3-sulfonamide ;
le 6-chloro-N-(4,6-diméthoxy-5-méthyl-pyrimidin-2-yl)-1H-pyrrolo[2,3-b]pyridine-3-sulfonamide ;
le 6-chloro-N-[5-(2,2-difluoroéthyl)-4,6-diméthoxy-pyrimidin-2-yl]-1H-pyrrolo[2,3-b]pyridine-3-sulfonamide ;
le 6-chloro-N-[5-(3,3-difluoropropyl)-4,6-diméthoxy-pyrimidin-2-yl]-1H-pyrrolo[2,3-b]pyridine-3-sulfonamide ;
le 6-chloro-N-[5-(2-cyanoéthyl)-4,6-diméthoxy-pyrimidin-2-yl]-1H-pyrrolo[2,3-b]pyridine-3-sulfonamide ;
le 6-chloro-N-[5-(2,2-difluoroéthyl)-4-méthoxy-pyrimidin-2-yl]-7-fluoro-1H-indole-3-sulfonamide ;
le 6-chloro-N-[5-(difluorométhoxy)-4,6-diméthoxy-pyrimidin-2-yl]-7-fluoro-1H-indole-3-sulfonamide ;
le 6-chloro-7-(cyclopropoxy)-N-[5-(2,2-difluoroéthyl)-4-méthoxy-pyrimidin-2-yl]-1H-indole-3-sulfonamide ;
le N-[5-(2,2-difluoroéthyl)-4,6-diméthoxy-pyrimidin-2-yl]-6-méthyl-1H-pyrrolo[2,3-b]pyridine-3-sulfonamide ;
le N-[5-(2,2-difluoroéthyl)-4-méthoxy-pyrimidin-2-yl]-6-fluoro-1H-indole-3-sulfonamide ;
le 7-bromo-6-chloro-N-[5-(2-fluoroéthoxy)-4-méthoxy-pyrimidin-2-yl]-1H-indole-3-sulfonamide ;
le 7-bromo-6-chloro-N-[5-(2,2-difluoroéthyl)-4-méthoxy-pyrimidin-2-yl]-1H-indole-3-sulfonamide ;
le 6-bromo-N-(4,6-diméthoxy-5-méthyl-pyrimidin-2-yl)thiéno[2,3-b]pyridine-3-sulfonamide ;
le 6-bromo-N-[5-(2,2-difluoroéthyl)-4-méthoxy-pyrimidin-2-yl]thiéno[2,3-b]pyridine-3-sulfonamide ;
le 6-bromo-N-[5-(2,2-difluoroéthyl)-4,6-diméthoxy-pyrimidin-2-yl]thiéno[2,3-b]pyridine-3-sulfonamide ;
le 6-bromo-N-[5-(2-cyanoéthyl)-4,6-diméthoxy-pyrimidin-2-yl]thiéno[2,3-b]pyridine-3-sulfonamide ;
le 6-chloro-N-[5-(2,2-difluoroéthyl)-4-méthoxy-pyrimidin-2-yl]benzothiophène-3-sulfonamide ;
le 6-bromo-N-[5-(2-fluoroéthoxy)-4-méthoxy-pyrimidin-2-yl]benzothiophène-3-sulfonamide ;
le 6-bromo-N-(4,6-diméthoxy-5-méthyl-pyrimidin-2-yl)-1H-pyrrolo[2,3-b]pyridine-3-sulfonamide ;
le N-[5-(2,2-difluoroéthoxy)-4,6-diméthoxy-pyrimidin-2-yl]-1H-pyrrolo[3,2-h]quinoléine-3-sulfonamide ;
le N-[5-(2,2-difluoroéthyl)-4,6-diméthoxy-pyrimidin-2-yl]-1H-pyrrolo[3,2-h]quinoléine-3-sulfonamide ;
le 6-bromo-N-[5-(difluorométhoxy)-4,6-diméthoxy-pyrimidin-2-yl]-1H-pyrrolo[2,3-b]pyridine-3-sulfonamide ;
le 6-chloro-N-[5-(2,3-difluoropropyl)-4-méthoxy-pyrimidin-2-yl]-1H-indole-3-sulfonamide ;
le 6-chloro-N-[5-(2-cyanoéthyl)-4-méthoxy-pyrimidin-2-yl]-1H-indole-3-sulfonamide ;
le 7-bromo-6-chloro-N-[5-(3,3-difluoropropyl)-4-méthoxy-pyrimidin-2-yl]-1H-indole-3-sulfonamide ;
le 6-bromo-N-[5S-(2,2-difluoroéthyl)-4-méthoxy-pyrimidin-2-yl]-7-méthyl-1H-indole-3-sulfonamide ;
le 6-chloro-N-[5-(2,2-difluoroéthyl)-4-méthoxy-pyrimidin-2-yl]-7-méthoxy-1H-indole-3-sulfonamide ;
le 6-chloro-N-[5-(3,3-difluoropropyl)-4-méthoxy-pyrimidin-2-yl]-7-méthoxy-1H-indole-3-sulfonamide ;
le 6-chloro-N-[5-(2,2-difluoroéthyl)-4-méthoxy-pyrimidin-2-yl]-7-(difluorométhoxy)-1H-indole-3-sulfonamide ;
le 6-bromo-N-[5-(2,2-difluoroéthyl)-4-méthoxy-pyrimidin-2-yl]-7-fluoro- 1H-indole-3-sulfonamide ;
le 6-chloro-N-[5-(2,2-difluoroéthyl)-4-méthoxy-pyrimidin-2-yl]-7-méthyl-1H-indole-3-sulfonamide ;
le N-[5-(2,2-difluoroéthyl)-4,6-diméthoxy-pyrimidin-2-yl]-6-(difluorométhyl)-1H-pyrrolo[2,3-b]pyridine-3-sulfonamide ;
le 6-bromo-N-[5-(2,2-difluoroéthyl)-4-méthoxy-pyrimidin-2-yl]-7-méthoxy-1H-indole-3-sulfonamide ;
le 6-chloro-N-[5-(2,2-difluoroéthyl)-4-méthoxy-pyrimidin-2-yl]-7-(fluorométhoxy)-1H-indole-3-sulfonamide ;
le 6-chloro-N-[5-(2,2-difluoroéthyl)-4-méthoxy-pyrimidin-2-yl]-7-(difluorométhylsulfanyl)-1H-indole-3-sulfonamide ;
le 6-bromo-N-[5-(3,3-difluoropropyl)-4-méthoxy-pyrimidin-2-yl]-1H-pyrrolo[2,3-b]pyridine-3-sulfonamide ;
le 6-bromo-N-[5-(2,2-difluoroéthoxy)-4,6-diméthoxy-pyrimidin-2-yl]-1H-pyrrolo[2,3-b]pyridine-3-sulfonamide ;
le 6-chloro-N-[5-(3,3-difluoropropyl)-4-méthoxy-pyrimidin-2-yl]-1H-pyrrolo[2,3-b]pyridine-3-sulfonamide ;
le 6-chloro-N-[5-(difluorométhoxy)-4,6-diméthoxy-pyrimidin-2-yl]-1H-pyrrolo[2,3-b]pyridine-3-sulfonamide ;
le 6-chloro-N-[5-(3,3-difluoropropyl)-4-méthoxy-pyrimidin-2-yl]-7-fluoro-1H-indole-3-sulfonamide ;
le 6-chloro-N-[5-(2,2-difluoroéthoxy)-4,6-diméthoxy-pyrimidin-2-yl]-7-fluoro-1H-indole-3-sulfonamide;
le 6,7-dichloro-N-[5-(2,2-difluoroéthyl)-4-méthoxy-pyrimidin-2-yl]-1H-indole-3-sulfonamide ;
le 6,7-dichloro-N-[5-(3,3-difluoropropyl)-4-méthoxy-pyrimidin-2-yl]-1H-indole-3-sulfonamide ;
le 6-chloro-7-(cyclopropoxy)-N-[5-(3,3-difluoropropyl)-4-méthoxy-pyrimidin-2-yl]-1H-indole-3-sulfonamide ;
le 6-bromo-7-fluoro-N-[5-(2-fluoroéthoxy)-4-méthoxy-pyrimidin-2-yl]-1H-indole-3-sulfonamide ;
le 6-chloro-N-[5-(2,2-difluoroéthyl)-4-méthoxy-pyrimidin-2-yl]-7-(difluorométhyl)-1H-indole-3-sulfonamide ;
le 6-chloro-7-fluoro-N-[4-méthoxy-5-(1,1,2-trifluoroéthoxy)pyrimidin-2-yl]-1H-indole-3-sulfonamide ;
le 6-chloro-N-[5-(2-cyanoéthyl)-4-méthoxy-pyrimidin-2-yl]-7-fluoro-1H-indole-3-sulfonamide ;
le 6-chloro-N-[5-(2,2-difluoroéthyl)-4,6-diméthoxy-pyrimidin-2-y1]-7-fluoro-1 H-indole-3-sulfonamide ;
le 6-chloro-N-[5-(2-cyanoéthyl)-4,6-diméthoxy-pyrimidin-2-y1]-7-fluoro-1H-indole-3-sulfonamide ;
le 6-chloro-N-[5-(cyanométhoxy)-4,6-diméthoxy-pyrimidin-2-y1]-7-fluoro-1H-indole-3-sulfonamide ;
le 6-chloro-N-[4-méthoxy-5-(1,1,2-trifluoroéthoxy)pyrimidin-2-yl]-1H-pyrrolo[2,3-b]pyridine-3-sulfonamide ;
le 6-chloro-N-[5-(2-fluoroéthoxy)-4,6-diméthoxy-pyrimidin-2-yl]-1H-pyrrolo[2,3-b]pyridine-3-sulfonamide ;
le 6-chloro-7-cyano-N-[5-(2,2-difluoroéthyl)-4-méthoxy-pyrimidin-2-yl]-1H-indole-3-sulfonamide ;
le 6-chloro-7-cyano-N-[5-(difluorométhoxy)-4,6-diméthoxy-pyrimidin-2-yl]-1H-indole-3-sulfonamide ;
le 6-chloro-7-cyano-N-[5-(2-fluoroéthoxy)-4,6-diméthoxy-pyrimidin-2-yl]-1H-indole-3-sulfonamide ;
le 6-chloro-7-cyano-N-[5-(2,2-difluoroéthoxy)-4,6-diméthoxy-pyrimidin-2-yl]-1H-indole-3-sulfonamide;
le 6-chloro-7-cyano-N-[5-(2,2-difluoroéthyl)-4,6-diméthoxy-pyrimidin-2-yl]-1H-indole-3-sulfonamide ;
le N-[5-(2,2-difluoroéthyl)-4,6-diméthoxy-pyrimidin-2-yl]-6,8-dihydro-1H-fur[3,4-g]indole-3-sulfonamide ;
le 6-bromo-N-[5-(2-fluoroéthoxy)-4-méthoxy-pyrimidin-2-yl]-1H-pyrrolo[2,3-b]pyridine-3-sulfonamide ;
le 6-bromo-N-[4-méthoxy-5-(1,1,2-trifluoroéthoxy)pyrimidin-2-yl]-1H-pyrrolo[2,3-b]pyridine-3-sulfonamide ;
le 6-chloro-7-(cyanométhyl)-N-[5-(2,2-difluoroéthyl)-4-méthoxy-pyrimidin-2-yl]-1H-indole-3-sulfonamide;
le 6-chloro-N-[5-(2,2-difluoroéthyl)-4-méthoxy-pyrimidin-2-yl]-7-(méthylthio)-1H-indole-3-sulfonamide;
le 6-(difluorométhyl)-N-[5-(2-fluoroéthoxy)-4-méthoxy-pyrimidin-2-yl]-1H-indole-3-sulfonamide ;
le N-[5-(2,2-difluoroéthyl)-4,6-diméthoxy-pyrimidin-2-yl]-6-(difluorométhyl)-1H-indole-3-sulfonamide ;
le N-[5-(cyanométhoxy)-4,6-diméthoxy-pyrimidin-2-yl]-6-(difluorométhyl)-1H-indole-3-sulfonamide ;
le N-[5-(2-cyanoéthyl)-4,6-diméthoxy-pyrimidin-2-yl]-6-(difluorométhyl)-1H-pyrrolo[2,3-b]pyridine-3-sulfonamide ;
le 6-bromo-N-[5-(2-cyanoéthyl)-4-méthoxy-pyrimidin-2-yl]-7-fluoro-1H-indole-3-sulfonamide ;
le 6-bromo-N-[5-(difluorométhoxy)-4,6-diméthoxy-pyrimidin-2-yl]-7-fluoro-1H-indole-3-sulfonamide ;
le 6-bromo-N-[5-(2,2-difluoroéthoxy)-4,6-diméthoxy-pyrimidin-2-yl]-7-fluoro-1H-indole-3-sulfonamide;
le 6-bromo-N-[5-(2-cyanoéthyl)-4,6-diméthoxy-pyrimidin-2-yl]-7-fluoro-1H-indole-3-sulfonamide ;
le 6-bromo-N-[5-(cyanométhoxy)-4,6-diméthoxy-pyrimidin-2-y1]-7-fluoro-1H-indole-3-sulfonamide ;
le 6-bromo-N-[5-(2,2-difluoroéthyl)-4,6-diméthoxy-pyrimidin-2-yl]-1H-indole-3-sulfonamide ;
le 6-bromo-N-[5-(cyanométhoxy)-4,6-diméthoxy-pyrimidin-2-yl]-1H-indole-3-sulfonamide ;
le 6-(difluorométhyl)-7-fluoro-N-[5-(2-fluoroéthoxy)-4-méthoxy-pyrimidin-2-yl]-1H-indole-3-sulfonamide;
le N-[5-(difluorométhoxy)-4,6-diméthoxy-pyrimidin-2-yl]-6-(difluorométhyl)-7-fluoro-1H-indole-3-sulfonamide ;
le N-[5-(2,2-difluoroéthoxy)-4,6-diméthoxy-pyrimidin-2-yl]-6-(difluorométhyl)-7-fluoro-1H-indole-3-sulfonamide ;
le N-[5-(2,2-difluoroéthyl)-4,6-diméthoxy-pyrimidin-2-yl]-6-(difluorométhyl)-7-fluoro-1H-indole-3-sulfonamide ;
le N-[5-(2-cyanoéthyl)-4,6-diméthoxy-pyrimidin-2-yl]-6-(difluorométhyl)-7-fluoro-1H-indole-3-sulfonamide ;
le N-[5-(cyanométhoxy)-4,6-diméthoxy-pyrimidin-2-yl]-6-(difluorométhyl)-7-fluoro-1H-indole-3-sulfonamide ;
le 7-bromo-6-chloro-N-[5-(2-cyanoéthyl)-4-méthoxy-pyrimidin-2-yl]-1H-indole-3-sulfonamide ;
le N-[5-(2,2-difluoroéthyl)-4,6-diméthoxy-pyrimidin-2-yl]-6-fluoro-1H-indole-3-sulfonamide ;
le N-[5-(2-cyanoéthyl)-4,6-diméthoxy-pyrimidin-2-yl]-6-fluoro-1H-indole-3-sulfonamide ;
le 6-chloro-N-[5-(difluorométhoxyméthyl)-4-méthoxy-pyrimidin-2-yl]-1H-indole-3-sulfonamide ;
le 6-chloro-N-[5-(2,3-difluoropropyl)-4,6-diméthoxy-pyrimidin-2-yl]-1H-indole-3-sulfonamide ;
le 6-chloro-N-[5-(cyanométhoxy)-4,6-diméthoxy-pyrimidin-2-yl]-1H-indole-3-sulfonamide ;
le 6-chloro-N-[5-(cyanométhoxy)-4-méthoxy-pyrimidin-2-yl]-1H-indole-3-sulfonamide ;
le 7-bromo-6-chloro-N-[5-(2,2-difluoroéthyl)-4,6-diméthoxy-pyrimidin-2-yl]-1H-indole-3-sulfonamide ;
le 6-chloro-7-(cyanométhoxy)-N-[5-(2,2-difluoroéthyl)-4,6-diméthoxy-pyrimidin-2-yl]-1H-indole-3-sulfonamide ;
et des sels pharmaceutiquement acceptables de ceux-ci.

13. Composé selon la revendication 11 ou la revendication 12, dans lequel le composé est le 6-bromo-N-[5-(difluorométhoxy)-4,6-diméthoxy-pyrimidin-2-yl]-1H-pyrrolo[2,3-b]pyridine-3-sulfonamide ou un sel pharmaceutiquement acceptable de celui-ci.

14. Composé selon l'une quelconque des revendications 11 à 13, dans lequel le composé est le 6-bromo-N-[5-(difluorométhoxy)-4,6-diméthoxy-pyrimidin-2-yl]-1H-pyrrolo[2,3-b]pyridine-3-sulfonamide.

15. Procédé de préparation d'un composé selon l'une quelconque des revendications 1 à 14 comprenant la réaction d'un composé de formule II 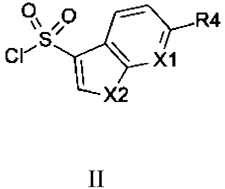
avec un composé de formule III 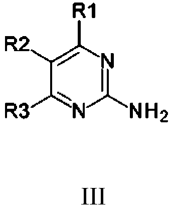
en présence d'une base choisie parmi la N,N-diisopropyléthylamine ou la pyridine, dans lequel R¹, R², R³, R⁴, X₁ et X₂ sont tels que définis ci-dessus.

16. Composé selon l'une quelconque des revendications 1 à 14 pour une utilisation comme substance thérapeutiquement active.

17. Composition pharmaceutique comprenant un composé selon l'une quelconque des revendications 1 à 14 et un véhicule thérapeutiquement inerte.

18. Composé selon l'une quelconque des revendications 1 à 14 pour une utilisation dans le traitement ou la prophylaxie d'affections résultant d'une lésion directe de gaines de myéline (notamment mais sans s'y limiter une myélinolyse centropontine et extrapontine, un empoisonnement au monoxyde de carbone, une carence nutritionnelle et une démyélinisation induite par un virus), des troubles de démyélinisation (notamment mais sans s'y limiter une sclérose en plaques, une encéphalomyélite disséminée aigüe et multiphasique, des troubles du spectre de la neuromyélite optique et des leucodystrophies), des troubles du SNC associés à une perte de myéline (notamment mais sans s'y limiter la maladie d'Alzheimer, une schizophrénie, la maladie de Parkinson, la maladie d'Huntington, une sclérose latérale amyotrophique et une ischémie due à un accident vasculaire cérébral) et une inflammation dans le SNC, par exemple suite à une encéphalite, une angéite primaire, une méningite et une obésité.

19. Composé selon l'une quelconque des revendications 1 à 14 pour une utilisation dans le traitement ou la prophylaxie d'une sclérose en plaques.
